# EUROPEAN PATENT APPLICATION

(11) **EP 3 590 957 A1**
(43) Date of publication of application: **08.01.2020**
(21) Application number: 17898509.9
(22) Date of filing: 05.09.2017
(51) Int. Cl.: C07K 14/415, A23J 3/16, A23L 11/30, A61K 39/35, A61P 37/08, G01N 33/50

(54) **ALLERGY ANTIGEN AND EPITOPE THEREOF**

(30) Priority: 03.03.2017 WO PCT/JP2017/008593
(71) Applicant: Hoyu Co., Ltd., Nagoya-shi Aichi, 461-8650 (JP)
(72) Inventor: MATSUNAGA, Kayoko, Toyoake-shi Aichi 470-1192 (JP); YAGAMI, Akiko, Toyoake-shi Aichi 470-1192 (JP); SHIMOJO, Naoshi, Nagakute-shi Aichi 480-1136 (JP); OHNO, Fumiaki, Nagakute-shi Aichi 480-1136 (JP)
(74) Representative: Mewburn Ellis LLP
(86) International application number: PCT/JP2017/032005
(87) International publication number: WO 2018/158985

(57) **Abstract**

The present invention provides novel antigens of an allergy to soybean, methods and kits for diagnosing an allergy to soybean, pharmaceutical compositions comprising such an antigen, soybeans or processed products of soybean in which such an antigen is eliminated, and a tester for determining the presence or absence of a soybean antigen in an object of interest. The present invention also relates to polypeptides comprising an epitope of an allergen, kits, compositions and methods for diagnosing an allergy, comprising such a polypeptide, pharmaceutical compositions comprising such a polypeptide, and food raw materials or edible processed products in which an antigen comprising such a polypeptide is eliminated or reduced, or the polypeptide is cleaved or removed. The present invention further relates to a tester for determining the presence or absence of an antigen in an object of interest.

## Description

### TECHNICAL FIELD

The present invention relates to a novel antigen of an allergy to soybean. The present invention also relates to a kit, a composition, and a method for diagnosing allergy to soybean. The present invention also relates to a pharmaceutical composition comprising such an antigen and soybean or processed products of soybean in which such an antigen is eliminated or reduced. The present invention further relates to a tester for determining the presence or absence of a soybean antigen in an object of interest.

The present invention also relates to a polypeptide comprising an epitope of an allergen. The present invention also relates to a kit, a composition and a method for diagnosing an allergy, comprising such a polypeptide. The present invention also relates to a pharmaceutical composition comprising such a polypeptide, and a food raw material or an edible processed product in which an antigen comprising such a polypeptide is eliminated or reduced, or such a polypeptide is cleaved or removed. The present invention further relates to a method for producing an edible processed product in which such an antigen is eliminated or reduced. The present invention further relates to a tester for determining the presence or absence of an antigen comprising such a polypeptide in an object of interest.

### BACKGROUND ART

In blood and tissues of allergic patients, IgE antibodies specific to particular antigens are produced. Physiological consequences caused by interaction between such IgE antibodies and such particular antigens elicit allergic reactions.

In the process of production of conventional allergy testing agents, antigen reagents are commonly prepared simply by grinding a candidate allergenic food, material or the like (Patent Literature 1). As seen above, conventional antigen reagents do not necessarily contain only a particular antigenic protein inducing an allergic reaction (allergen component), and rather contain different types of protein components. Thus, conventional antigen reagents contain varied amounts of allergen components. For this reason, the only case where conventional allergy tests have permitted detection of a positive allergic reaction is when in a conventional antigen reagent containing many types of proteins, a particular protein acting as an allergen component is present in an amount exceeding a threshold that allows determination of a positive reaction for binding to an IgE antibody. However, no determination of a positive reaction was possible and diagnosis efficiency was not sufficiently high when using a conventional allergy testing agent in patients possessing an IgE antibody binding to an allergen component present in small amounts in an allergen such as food.

The severity and symptoms of an allergic reaction do not necessarily correlate with the content of an allergen component. Even when a patient's IgE antibody reacts with an allergen component present in trace amounts in a candidate allergic food and material, the allergic reaction may develop allergic symptoms or may affect the severity of those symptoms.

An attempt to increase the diagnostic efficiency is being made by examining IgE antibodies to components composing a crude antigen to distinguish sensitization that directly contributes to a diagnosis from sensitization based on cross-antigenicity by a pan-allergen or the like. 8 soybean allergens are currently registered to the World Health Organization/International Union of Immunological Societies (WHO/IUIS).

However, while it is necessary to exhaustively identify allergen components in candidate allergic foods and materials in order to enhance the reliability of allergy tests, the patient detection rate by the measurement of such allergenic components is far from sufficient. Identification of novel allergens in soybean is very important not only for increasing the precision of diagnosis, but also for determining targets of therapeutic agents and low allergenic foods.

Meanwhile, in the field of protein separation and purification, various efforts have conventionally been made to develop methods for separating and purifying a protein or nucleic acid of interest from cell extracts or the like. Such methods may well be exemplified by dialysis based on salt concentration, and centrifugal separation.

Other efforts have been made to develop many purification methods based on electric charges of protein or nucleic acid residues or on the difference in molecular weight. Electric charge-based purification methods can be exemplified by column chromatography using ion exchange resins, and isoelectric focusing. Purifications based on molecular weight difference can be exemplified by centrifugal separation, molecular-sieve column chromatography, and SDS-PAGE (sodium dodecyl sulfate-polyacrylamide gel electrophoresis).

In recent years, a method for separating and purifying many different proteins from a small amount of sample has been used, which is more specifically a two-dimensional electrophoresis consisting of isoelectric focusing in the first dimension, followed by SDS-PAGE in the second dimension. The present applicant has conventionally developed some 2D electrophoresis methods with high separation ability (Patent Literature 2-5).

Allergen-specific IgE antibodies recognize and bind to epitopes that are particular amino acid sequences in allergen components. However, only a slight number of analyses have been made on epitopes as to the allergen components (Non Patent Literature 1), but such analyses are still totally quite rare. Furthermore, any kit for diagnosing an allergy using a polypeptide comprising an epitope has not yet emerged in the market.

### CITATION LIST

### PATENT LITERATURE

PTL1: Japanese Patent Application Publication No. JP 2002-286716
PTL2: Japanese Patent Application Publication No. JP 2011-33544
PTL3: Japanese Patent Application Publication No. JP 2011-33546
PTL4: Japanese Patent Application Publication No. JP 2011-33547
PTL5: Japanese Patent Application Publication No. JP 2011-33548

### NON PATENT LITERATURE

NPL 1: Matsuo, H., et al., J. Biol. Chem., (2004), Vol.279, No.13, pp.12135-12140
NPL 2: Zhao, L., et al., PLoS One. 2017 Aug 11; 12(8):e0182935. doi: 10.1371/journal.pone.0182935. eCollection 2017.
NPL 3: Cong, Y., et al., J Dairy Sci. 2013; 96(11):6870-6. doi: 10.3168/jds.2013-6880. Epub 2013 Sep 12.
NPL 4: Beezhold, D. H., et al., J Allergy Clin Immunol. 2001 Jun; 107(6):1069-76.

### SUMMARY OF INVENTION

### TECHNICAL PROBLEM

The present invention provides novel antigens of an allergy to soybean. The present invention also provides methods and kits for diagnosing allergy to soybean. The present invention also provides pharmaceutical compositions comprising such an antigen and soybean or processed products of soybean in which such an antigen is eliminated or reduced. The present invention further provides testers for determining the presence or absence of a soybean antigen in an object of interest.

The present invention also provides polypeptides comprising an epitope of an allergen. The present invention also provides kits, compositions and methods for diagnosing an allergy. The present invention also provides pharmaceutical compositions comprising such a polypeptide, and food raw materials or edible processed products in which an antigen comprising such a polypeptide is eliminated or reduced, or such a polypeptide is cleaved or removed. The present invention further relates to methods for producing an edible processed product in which such an antigen is eliminated or reduced. The present invention further relates to testers for determining the presence or absence of an antigen comprising such a polypeptide in an object of interest.

### SOLUTION TO PROBLEM

In order to solve the aforementioned problems, the present inventors had made intensive studies to identify causative antigens of an allergy. As a result, the inventors succeeded in identifying novel antigens to which an IgE antibody in the serum of an allergic patient specifically binds. The present invention has been completed based on this finding.

Thus, in one embodiment, the present invention can be as defined below.
[1] A kit for diagnosing an allergy to a soybean, the kit comprising, as an antigen, at least one of proteins defined below in any one of (1α) to (55α):
   (1α)
      (1αA1) endoplasmin homolog isoform X2 or a variant thereof, which is defined below in any of (1αA1-a) to (1αA1-e):
         (1αA1-a) a protein comprising an amino acid sequence with deletion, substitution, insertion or addition of one or several amino acids in SEQ ID NO: 70;
         (1αA1-b) a protein comprising an amino acid sequence having at least 70% identity to the amino acid sequence of SEQ ID NO: 70;
         (laAl-c) a protein comprising an amino acid sequence encoded by a nucleotide sequence with deletion, substitution, insertion or addition of one or several nucleotides in SEQ ID NO: 69;
         (1αA1-d) a protein comprising an amino acid sequence encoded by a nucleotide sequence having at least 70% identity to the nucleotide sequence of SEQ ID NO: 69; or
         (1αA1-e) a protein comprising an amino acid sequence encoded by a nucleic acid that hybridizes under stringent conditions with a nucleic acid having a nucleotide sequence complementary to the nucleotide sequence of SEQ ID NO: 69;
         (1αB1) a protein comprising at least one amino acid sequence selected from the group consisting of SEQ ID NOs: 71-87,
      (1αA2) endoplasmin homolog isoform X2 or a variant thereof, which is defined below in any of (1αA2-a) to (1αA2-e):
         (1αA2-a) a protein comprising an amino acid sequence with deletion, substitution, insertion or addition of one or several amino acids in SEQ ID NO: 89;
         (1αA2-b) a protein comprising an amino acid sequence having at least 70% identity to the amino acid sequence of SEQ ID NO: 89;
         (1αA2-c) a protein comprising an amino acid sequence encoded by a nucleotide sequence with deletion, substitution, insertion or addition of one or several nucleotides in SEQ ID NO: 88;
         (1αA2-d) a protein comprising an amino acid sequence encoded by a nucleotide sequence having at least 70% identity to the nucleotide sequence of SEQ ID NO: 88; or
         (1αA2-e) a protein comprising an amino acid sequence encoded by a nucleic acid that hybridizes under stringent conditions with a nucleic acid having a nucleotide sequence complementary to the nucleotide sequence of SEQ ID NO: 88; or
      (1αB2) a protein comprising at least one amino acid sequence selected from the group consisting of SEQ ID NOs: 90-104;
   (2α)
      (2αA1) heat shock cognate protein 80 or a variant thereof, which is defined below in any of (2αA1-a) to (2αA1-e):
         (2αA1-a) a protein comprising an amino acid sequence with deletion, substitution, insertion or addition of one or several amino acids in SEQ ID NO: 106;
         (2αA1-b) a protein comprising an amino acid sequence having at least 70% identity to the amino acid sequence of SEQ ID NO: 106;
         (2αA1-c) a protein comprising an amino acid sequence encoded by a nucleotide sequence with deletion, substitution, insertion or addition of one or several nucleotides in SEQ ID NO: 105;
         (2αA1-d) a protein comprising an amino acid sequence encoded by a nucleotide sequence having at least 70% identity to the nucleotide sequence of SEQ ID NO: 105; or
         (2αA1-e) a protein comprising an amino acid sequence encoded by a nucleic acid that hybridizes under stringent conditions with a nucleic acid having a nucleotide sequence complementary to the nucleotide sequence of SEQ ID NO: 105; or
         (2αB1) a protein comprising at least one amino acid sequence selected from the group consisting of SEQ ID NOs: 107-120;
      (2αA2) heat shock protein 90-1 or a variant thereof, which is defined below in any of (2αA2-a) to (2αA2-e):
         (2αA2-a) a protein comprising an amino acid sequence with deletion, substitution, insertion or addition of one or several amino acids in SEQ ID NO: 122;
         (2αA2-b) a protein comprising an amino acid sequence having at least 70% identity to the amino acid sequence of SEQ ID NO: 122;
         (2αA2-c) a protein comprising an amino acid sequence encoded by a nucleotide sequence with deletion, substitution, insertion or addition of one or several nucleotides in SEQ ID NO: 121;
         (2αA2-d) a protein comprising an amino acid sequence encoded by a nucleotide sequence having at least 70% identity to the nucleotide sequence of SEQ ID NO: 121; or
         (2αA2-e) a protein comprising an amino acid sequence encoded by a nucleic acid that hybridizes under stringent conditions with a nucleic acid having a nucleotide sequence complementary to the nucleotide sequence of SEQ ID NO: 121; or
      (2αB2) a protein comprising at least one amino acid sequence selected from the group consisting of SEQ ID NOs: 123-137;
      (2αA3) hypothetical protein GLYMA_02G302500 or a variant thereof, which is defined below in any of (2αA3-a) to (2αA3-e):
         (2αA3-a) a protein comprising an amino acid sequence with deletion, substitution, insertion or addition of one or several amino acids in SEQ ID NO: 139;
         (2αA3-b) a protein comprising an amino acid sequence having at least 70% identity to the amino acid sequence of SEQ ID NO: 139;
         (2αA3-c) a protein comprising an amino acid sequence encoded by a nucleotide sequence with deletion, substitution, insertion or addition of one or several nucleotides in SEQ ID NO: 138;
         (2αA3-d) a protein comprising an amino acid sequence encoded by a nucleotide sequence having at least 70% identity to the nucleotide sequence of SEQ ID NO: 138; or
         (2αA3-e) a protein comprising an amino acid sequence encoded by a nucleic acid that hybridizes under stringent conditions with a nucleic acid having a nucleotide sequence complementary to the nucleotide sequence of SEQ ID NO: 138; or
      (2αB3) a protein comprising at least one amino acid sequence selected from the group consisting of SEQ ID NOs: 140-153;
   (4α)
      (4αA) embryo-specific urease isoform X1 or a variant thereof, which is defined below in any of (4αA-a) to (4αA-e):
         (4αA-a) a protein comprising an amino acid sequence with deletion, substitution, insertion or addition of one or several amino acids in SEQ ID NO: 155;
         (4αA-b) a protein comprising an amino acid sequence having at least 70% identity to the amino acid sequence of SEQ ID NO: 155;
         (4αA-c) a protein comprising an amino acid sequence encoded by a nucleotide sequence with deletion, substitution, insertion or addition of one or several nucleotides in SEQ ID NO: 154;
         (4αA-d) a protein comprising an amino acid sequence encoded by a nucleotide sequence having at least 70% identity to the nucleotide sequence of SEQ ID NO: 154; or
         (4αA-e) a protein comprising an amino acid sequence encoded by a nucleic acid that hybridizes under stringent conditions with a nucleic acid having a nucleotide sequence complementary to the nucleotide sequence of SEQ ID NO: 154; or
      (4αB) a protein comprising at least one amino acid sequence selected from the group consisting of SEQ ID NOs: 156 and 157;
   (5α)
      (5αA) alpha-1,4 glucan phosphorylase L isozyme, chloroplastic/amyloplastic or a variant thereof, which is defined below in any of (5αA-a) to (5αA-e):
         (5αA-a) a protein comprising an amino acid sequence with deletion, substitution, insertion or addition of one or several amino acids in SEQ ID NO: 159;
         (5αA-b) a protein comprising an amino acid sequence having at least 70% identity to the amino acid sequence of SEQ ID NO: 159;
         (5αA-c) a protein comprising an amino acid sequence encoded by a nucleotide sequence with deletion, substitution, insertion or addition of one or several nucleotides in SEQ ID NO: 158;
         (5αA-d) a protein comprising an amino acid sequence encoded by a nucleotide sequence having at least 70% identity to the nucleotide sequence of SEQ ID NO: 158; or
         (5αA-e) a protein comprising an amino acid sequence encoded by a nucleic acid that hybridizes under stringent conditions with a nucleic acid having a nucleotide sequence complementary to the nucleotide sequence of SEQ ID NO: 158; or
      (5αB) a protein comprising at least one amino acid sequence selected from the group consisting of SEQ ID NOs: 160 and 161;
   (6α)
      (6αA1) aconitate hydratase 1 or a variant thereof, which is defined below in any of (6aA1-a) to (6αA1-e):
         (6αA1-a) a protein comprising an amino acid sequence with deletion, substitution, insertion or addition of one or several amino acids in SEQ ID NO: 163;
         (6αA1-b) a protein comprising an amino acid sequence having at least 70% identity to the amino acid sequence of SEQ ID NO: 163;
         (6αA1-c) a protein comprising an amino acid sequence encoded by a nucleotide sequence with deletion, substitution, insertion or addition of one or several nucleotides in SEQ ID NO: 162;
         (6αA1-d) a protein comprising an amino acid sequence encoded by a nucleotide sequence having at least 70% identity to the nucleotide sequence of SEQ ID NO: 162; or
         (6αA1-e) a protein comprising an amino acid sequence encoded by a nucleic acid that hybridizes under stringent conditions with a nucleic acid having a nucleotide sequence complementary to the nucleotide sequence of SEQ ID NO: 162;
      (6αB1) a protein comprising at least one amino acid sequence selected from the group consisting of SEQ ID NOs: 164-167; or
      (6αA2) aconitate hydratase 1 or a variant thereof, which is defined below in any of (6αA2-a) to (6αA2-e):
         (6αA2-a) a protein comprising an amino acid sequence with deletion, substitution, insertion or addition of one or several amino acids in SEQ ID NO: 169;
         (6αA2-b) a protein comprising an amino acid sequence having at least 70% identity to the amino acid sequence of SEQ ID NO: 169;
         (6αA2-c) a protein comprising an amino acid sequence encoded by a nucleotide sequence with deletion, substitution, insertion or addition of one or several nucleotides in SEQ ID NO: 168;
         (6αA2-d) a protein comprising an amino acid sequence encoded by a nucleotide sequence having at least 70% identity to the nucleotide sequence of SEQ ID NO: 168; or
         (6αA2-e) a protein comprising an amino acid sequence encoded by a nucleic acid that hybridizes under stringent conditions with a nucleic acid having a nucleotide sequence complementary to the nucleotide sequence of SEQ ID NO: 168; or
      (6αB2) a protein comprising at least one amino acid sequence selected from the group consisting of SEQ ID NOs: 170-172;
   (7α)
      (7αA) methionine synthase or a variant thereof, which is defined below in any of (7αA-a) to (7αA-e):
         (7αA-a) a protein comprising an amino acid sequence with deletion, substitution, insertion or addition of one or several amino acids in SEQ ID NO: 174;
         (7αA-b) a protein comprising an amino acid sequence having at least 70% identity to the amino acid sequence of SEQ ID NO: 174;
         (7αA-c) a protein comprising an amino acid sequence encoded by a nucleotide sequence with deletion, substitution, insertion or addition of one or several nucleotides in SEQ ID NO: 173;
         (7αA-d) a protein comprising an amino acid sequence encoded by a nucleotide sequence having at least 70% identity to the nucleotide sequence of SEQ ID NO: 173; or
         (7αA-e) a protein comprising an amino acid sequence encoded by a nucleic acid that hybridizes under stringent conditions with a nucleic acid having a nucleotide sequence complementary to the nucleotide sequence of SEQ ID NO: 173; or
      (7αB) a protein comprising at least one amino acid sequence selected from the group consisting of SEQ ID NOs: 175-187;
   (8α)
      (8αA) transketolase, chloroplastic or a variant thereof, which is defined below in any of (8αA-a) to (8αA-e):
         (8αA-a) a protein comprising an amino acid sequence with deletion, substitution, insertion or addition of one or several amino acids in SEQ ID NO: 189;
         (8αA-b) a protein comprising an amino acid sequence having at least 70% identity to the amino acid sequence of SEQ ID NO: 189;
         (8αA-c) a protein comprising an amino acid sequence encoded by a nucleotide sequence with deletion, substitution, insertion or addition of one or several nucleotides in SEQ ID NO: 188;
         (8αA-d) a protein comprising an amino acid sequence encoded by a nucleotide sequence having at least 70% identity to the nucleotide sequence of SEQ ID NO: 188; or
         (8αA-e) a protein comprising an amino acid sequence encoded by a nucleic acid that hybridizes under stringent conditions with a nucleic acid having a nucleotide sequence complementary to the nucleotide sequence of SEQ ID NO: 188; or
      (8αB) a protein comprising at least one amino acid sequence selected from the group consisting of SEQ ID NOs: 190-199;
   (9α)
      (9αA) lysine-tRNA ligase, cytoplasmic-like isoform X2 or a variant thereof, which is defined below in any of (9αA-a) to (9αA-e):
         (9αA-a) a protein comprising an amino acid sequence with deletion, substitution, insertion or addition of one or several amino acids in SEQ ID NO: 201;
         (9αA-b) a protein comprising an amino acid sequence having at least 70% identity to the amino acid sequence of SEQ ID NO: 201;
         (9αA-c) a protein comprising an amino acid sequence encoded by a nucleotide sequence with deletion, substitution, insertion or addition of one or several nucleotides in SEQ ID NO: 200;
         (9αA-d) a protein comprising an amino acid sequence encoded by a nucleotide sequence having at least 70% identity to the nucleotide sequence of SEQ ID NO: 200; or
         (9αA-e) a protein comprising an amino acid sequence encoded by a nucleic acid that hybridizes under stringent conditions with a nucleic acid having a nucleotide sequence complementary to the nucleotide sequence of SEQ ID NO: 200; or
      (9αB) a protein comprising an amino acid sequence of SEQ ID NO: 202;
   (10α)
      (10αA1) chaperonin CPN60-2, mitochondrial or a variant thereof, which is defined below in any of (10αA1-a) to (10αA1-e):
         (10αA1-a) a protein comprising an amino acid sequence with deletion, substitution, insertion or addition of one or several amino acids in SEQ ID NO: 204;
         (10αA1-b) a protein comprising an amino acid sequence having at least 70% identity to the amino acid sequence of SEQ ID NO: 204;
         (10αA1-c) a protein comprising an amino acid sequence encoded by a nucleotide sequence with deletion, substitution, insertion or addition of one or several nucleotides in SEQ ID NO: 203;
         (10αA1-d) a protein comprising an amino acid sequence encoded by a nucleotide sequence having at least 70% identity to the nucleotide sequence of SEQ ID NO: 203; or
         (10αA1-e) a protein comprising an amino acid sequence encoded by a nucleic acid that hybridizes under stringent conditions with a nucleic acid having a nucleotide sequence complementary to the nucleotide sequence of SEQ ID NO: 203;
      (10αB1) a protein comprising at least one amino acid sequence selected from the group consisting of SEQ ID NOs: 205-218;
      (10αA2) chaperonin CPN60-2, mitochondrial-like isoform X1 or a variant thereof, which is defined below in any of (10αA2-a) to (10αA2-e):
         (10αA2-a) a protein comprising an amino acid sequence with deletion, substitution, insertion or addition of one or several amino acids in SEQ ID NO: 220;
         (10αA2-b) a protein comprising an amino acid sequence having at least 70% identity to the amino acid sequence of SEQ ID NO: 220;
         (10αA2-c) a protein comprising an amino acid sequence encoded by a nucleotide sequence with deletion, substitution, insertion or addition of one or several nucleotides in SEQ ID NO: 219;
         (10αA2-d) a protein comprising an amino acid sequence encoded by a nucleotide sequence having at least 70% identity to the nucleotide sequence of SEQ ID NO: 219; or
         (10αA2-e) a protein comprising an amino acid sequence encoded by a nucleic acid that hybridizes under stringent conditions with a nucleic acid having a nucleotide sequence complementary to the nucleotide sequence of SEQ ID NO: 219; or
      (10αB2) a protein comprising at least one amino acid sequence selected from the group consisting of SEQ ID NOs: 221-231;
   (11α)
      (11αA) polyadenylate-binding protein 8 isoform X3 or a variant thereof, which is defined below in any of (11αA-a) to (11αA-e):
         (11αA-a) a protein comprising an amino acid sequence with deletion, substitution, insertion or addition of one or several amino acids in SEQ ID NO: 233;
         (11αA-b) a protein comprising an amino acid sequence having at least 70% identity to the amino acid sequence of SEQ ID NO: 233;
         (11αA-c) a protein comprising an amino acid sequence encoded by a nucleotide sequence with deletion, substitution, insertion or addition of one or several nucleotides in SEQ ID NO: 232;
         (11αA-d) a protein comprising an amino acid sequence encoded by a nucleotide sequence having at least 70% identity to the nucleotide sequence of SEQ ID NO: 232; or
         (11αA-e) a protein comprising an amino acid sequence encoded by a nucleic acid that hybridizes under stringent conditions with a nucleic acid having a nucleotide sequence complementary to the nucleotide sequence of SEQ ID NO: 232; or
      (11αB) a protein comprising at least one amino acid sequence selected from the group consisting of SEQ ID NOs: 234-237;
   (12α)
      (12αA) pyrophosphate-fructose 6-phosphate 1-phosphotransferase subunit alpha-like or a variant thereof, which is defined below in any of (12αA-a) to (12αA-e):
         (12αA-a) a protein comprising an amino acid sequence with deletion, substitution, insertion or addition of one or several amino acids in SEQ ID NO: 239;
         (12αA-b) a protein comprising an amino acid sequence having at least 70% identity to the amino acid sequence of SEQ ID NO: 239;
         (12αA-c) a protein comprising an amino acid sequence encoded by a nucleotide sequence with deletion, substitution, insertion or addition of one or several nucleotides in SEQ ID NO: 238;
         (12αA-d) a protein comprising an amino acid sequence encoded by a nucleotide sequence having at least 70% identity to the nucleotide sequence of SEQ ID NO: 238; or
         (12αA-e) a protein comprising an amino acid sequence encoded by a nucleic acid that hybridizes under stringent conditions with a nucleic acid having a nucleotide sequence complementary to the nucleotide sequence of SEQ ID NO: 238; or
      (12αB) a protein comprising an amino acid sequence of SEQ ID NO: 240;
   (13α)
      (13αA1) protein disulfide isomerase-like protein precursor or a variant thereof, which is defined below in any of (13αA1-a) to (13αA1-e):
         (13αA1-a) a protein comprising an amino acid sequence with deletion, substitution, insertion or addition of one or several amino acids in SEQ ID NO: 242;
         (13αA1-b) a protein comprising an amino acid sequence having at least 70% identity to the amino acid sequence of SEQ ID NO: 242;
         (13αA1-c) a protein comprising an amino acid sequence encoded by a nucleotide sequence with deletion, substitution, insertion or addition of one or several nucleotides in SEQ ID NO: 241;
         (13αA1-d) a protein comprising an amino acid sequence encoded by a nucleotide sequence having at least 70% identity to the nucleotide sequence of SEQ ID NO: 241; or
         (13αA1-e) a protein comprising an amino acid sequence encoded by a nucleic acid that hybridizes under stringent conditions with a nucleic acid having a nucleotide sequence complementary to the nucleotide sequence of SEQ ID NO: 241;
      (13αB1) a protein comprising at least one amino acid sequence selected from the group consisting of SEQ ID NOs: 243-252;
      (13αA2) protein disulfide-isomerase or a variant thereof, which is defined below in any of (13αA2-a) to (13αA2-e):
         (13αA2-a) a protein comprising an amino acid sequence with deletion, substitution, insertion or addition of one or several amino acids in SEQ ID NO: 254;
         (13αA2-b) a protein comprising an amino acid sequence having at least 70% identity to the amino acid sequence of SEQ ID NO: 254;
         (13αA2-c) a protein comprising an amino acid sequence encoded by a nucleotide sequence with deletion, substitution, insertion or addition of one or several nucleotides in SEQ ID NO: 253;
         (13αA2-d) a protein comprising an amino acid sequence encoded by a nucleotide sequence having at least 70% identity to the nucleotide sequence of SEQ ID NO: 253; or
         (13αA2-e) a protein comprising an amino acid sequence encoded by a nucleic acid that hybridizes under stringent conditions with a nucleic acid having a nucleotide sequence complementary to the nucleotide sequence of SEQ ID NO: 253; or
      (13αB2) a protein comprising at least one amino acid sequence selected from the group consisting of SEQ ID NOs: 255-278;
   (14α)
      (14αA1) V-type proton ATPase subunit B 2 or a variant thereof, which is defined below in any of (14αA1-a) to (14αA1-e):
         (14αA1-a) a protein comprising an amino acid sequence with deletion, substitution, insertion or addition of one or several amino acids in SEQ ID NO: 280;
         (14αA1-b) a protein comprising an amino acid sequence having at least 70% identity to the amino acid sequence of SEQ ID NO: 280;
         (14αA1-c) a protein comprising an amino acid sequence encoded by a nucleotide sequence with deletion, substitution, insertion or addition of one or several nucleotides in SEQ ID NO: 279;
         (14αA1-d) a protein comprising an amino acid sequence encoded by a nucleotide sequence having at least 70% identity to the nucleotide sequence of SEQ ID NO: 279; or
         (14αA1-e) a protein comprising an amino acid sequence encoded by a nucleic acid that hybridizes under stringent conditions with a nucleic acid having a nucleotide sequence complementary to the nucleotide sequence of SEQ ID NO: 279;
      (14αB1) a protein comprising at least one amino acid sequence selected from the group consisting of SEQ ID NOs: 281-284;
      (14αA2) V-type proton ATPase subunit B 2 or a variant thereof, which is defined below in any of (14αA2-a) to (14αA2-e):
         (14αA2-a) a protein comprising an amino acid sequence with deletion, substitution, insertion or addition of one or several amino acids in SEQ ID NO: 286;
         (14αA2-b) a protein comprising an amino acid sequence having at least 70% identity to the amino acid sequence of SEQ ID NO: 286;
         (14αA2-c) a protein comprising an amino acid sequence encoded by a nucleotide sequence with deletion, substitution, insertion or addition of one or several nucleotides in SEQ ID NO: 285;
         (14αA2-d) a protein comprising an amino acid sequence encoded by a nucleotide sequence having at least 70% identity to the nucleotide sequence of SEQ ID NO: 285; or
         (14αA2-e) a protein comprising an amino acid sequence encoded by a nucleic acid that hybridizes under stringent conditions with a nucleic acid having a nucleotide sequence complementary to the nucleotide sequence of SEQ ID NO: 285; or
      (14αB2) a protein comprising at least one amino acid sequence selected from the group consisting of SEQ ID NOs: 287-289;
   (15, 16α)
      (15, 16αA1) UTP-glucose-1 -phosphate uridylyltransferase or a variant thereof, which is defined below in any of (15, 16αA1-a) to (15, 16αA1-e):
         (15, 16αA1-a) a protein comprising an amino acid sequence with deletion, substitution, insertion or addition of one or several amino acids in SEQ ID NO: 291;
         (15, 16αA1-b) a protein comprising an amino acid sequence having at least 70% identity to the amino acid sequence of SEQ ID NO: 291;
         (15, 16αA1-c) a protein comprising an amino acid sequence encoded by a nucleotide sequence with deletion, substitution, insertion or addition of one or several nucleotides in SEQ ID NO: 290;
         (15, 16αA1-d) a protein comprising an amino acid sequence encoded by a nucleotide sequence having at least 70% identity to the nucleotide sequence of SEQ ID NO: 290; or
         (15, 16αA1-e) a protein comprising an amino acid sequence encoded by a nucleic acid that hybridizes under stringent conditions with a nucleic acid having a nucleotide sequence complementary to the nucleotide sequence of SEQ ID NO: 290;
      (15, 16αB1) a protein comprising at least one amino acid sequence selected from the group consisting of SEQ ID NOs: 292-304;
      (15, 16αA2) hypothetical protein GLYMA_02G241100 or a variant thereof, which is defined below in any of (15, 16αA2-a) to (15, 16αA2-e):
         (15, 16αA2-a) a protein comprising an amino acid sequence with deletion, substitution, insertion or addition of one or several amino acids in SEQ ID NO: 306;
         (15, 16αA2-b) a protein comprising an amino acid sequence having at least 70% identity to the amino acid sequence of SEQ ID NO: 306;
         (15, 16αA2-c) a protein comprising an amino acid sequence encoded by a nucleotide sequence with deletion, substitution, insertion or addition of one or several nucleotides in SEQ ID NO: 305;
         (15, 16αA2-d) a protein comprising an amino acid sequence encoded by a nucleotide sequence having at least 70% identity to the nucleotide sequence of SEQ ID NO: 305; or
         (15, 16αA2-e) a protein comprising an amino acid sequence encoded by a nucleic acid that hybridizes under stringent conditions with a nucleic acid having a nucleotide sequence complementary to the nucleotide sequence of SEQ ID NO: 305; or
      (15, 16αB2) a protein comprising at least one amino acid sequence selected from the group consisting of SEQ ID NOs: 307-318;
   (17α)
      (17αA1) guanosine nucleotide diphosphate dissociation inhibitor 2 or a variant thereof, which is defined below in any of (17αA1-a) to (17αA1-e):
         (17αA1-a) a protein comprising an amino acid sequence with deletion, substitution, insertion or addition of one or several amino acids in SEQ ID NO: 320;
         (17αA1-b) a protein comprising an amino acid sequence having at least 70% identity to the amino acid sequence of SEQ ID NO: 320;
         (17αA1-c) a protein comprising an amino acid sequence encoded by a nucleotide sequence with deletion, substitution, insertion or addition of one or several nucleotides in SEQ ID NO: 319;
         (17αA1-d) a protein comprising an amino acid sequence encoded by a nucleotide sequence having at least 70% identity to the nucleotide sequence of SEQ ID NO: 319; or
         (17αA1-e) a protein comprising an amino acid sequence encoded by a nucleic acid that hybridizes under stringent conditions with a nucleic acid having a nucleotide sequence complementary to the nucleotide sequence of SEQ ID NO: 319;
      (17αB1) a protein comprising at least one amino acid sequence selected from the group consisting of SEQ ID NOs: 321-325;
      (17αA2) rab GDP dissociation inhibitor alpha-like or a variant thereof, which is defined below in any of (17αA2-a) to (17αA2-e):
         (17αA2-a) a protein comprising an amino acid sequence with deletion, substitution, insertion or addition of one or several amino acids in SEQ ID NO: 327;
         (17αA2-b) a protein comprising an amino acid sequence having at least 70% identity to the amino acid sequence of SEQ ID NO: 327;
         (17αA2-c) a protein comprising an amino acid sequence encoded by a nucleotide sequence with deletion, substitution, insertion or addition of one or several nucleotides in SEQ ID NO: 326;
         (17αA2-d) a protein comprising an amino acid sequence encoded by a nucleotide sequence having at least 70% identity to the nucleotide sequence of SEQ ID NO: 326; or
         (17αA2-e) a protein comprising an amino acid sequence encoded by a nucleic acid that hybridizes under stringent conditions with a nucleic acid having a nucleotide sequence complementary to the nucleotide sequence of SEQ ID NO: 326; or
      (17αB2) a protein comprising at least one amino acid sequence selected from the group consisting of SEQ ID NOs: 328-332;
   (18α)
      (18αA) hypothetical protein GLYMA_10G028300 or a variant thereof, which is defined below in any of (18αA-a) to (18αA-e):
         (18αA-a) a protein comprising an amino acid sequence with deletion, substitution, insertion or addition of one or several amino acids in SEQ ID NO: 334;
         (18αA-b) a protein comprising an amino acid sequence having at least 70% identity to the amino acid sequence of SEQ ID NO: 334;
         (18αA-c) a protein comprising an amino acid sequence encoded by a nucleotide sequence with deletion, substitution, insertion or addition of one or several nucleotides in SEQ ID NO: 333;
         (18αA-d) a protein comprising an amino acid sequence encoded by a nucleotide sequence having at least 70% identity to the nucleotide sequence of SEQ ID NO: 333; or
         (18αA-e) a protein comprising an amino acid sequence encoded by a nucleic acid that hybridizes under stringent conditions with a nucleic acid having a nucleotide sequence complementary to the nucleotide sequence of SEQ ID NO: 333; or
      (18αB) a protein comprising at least one amino acid sequence selected from the group consisting of SEQ ID NOs: 335-339;
   (19α)
      (19αA) sucrose binding protein homolog S-64 or a variant thereof, which is defined below in any of (19αA-a) to (19αA-e):
         (19αA-a) a protein comprising an amino acid sequence with deletion, substitution, insertion or addition of one or several amino acids in SEQ ID NO: 341;
         (19αA-b) a protein comprising an amino acid sequence having at least 70% identity to the amino acid sequence of SEQ ID NO: 341;
         (19αA-c) a protein comprising an amino acid sequence encoded by a nucleotide sequence with deletion, substitution, insertion or addition of one or several nucleotides in SEQ ID NO: 340;
         (19αA-d) a protein comprising an amino acid sequence encoded by a nucleotide sequence having at least 70% identity to the nucleotide sequence of SEQ ID NO: 340; or
         (19αA-e) a protein comprising an amino acid sequence encoded by a nucleic acid that hybridizes under stringent conditions with a nucleic acid having a nucleotide sequence complementary to the nucleotide sequence of SEQ ID NO: 340; or
      (19αB) a protein comprising at least one amino acid sequence selected from the group consisting of SEQ ID NOs: 342-346;
   (25α)
      (25αA1) succinyl-CoA ligase [ADP-forming] subunit beta, mitochondrial or a variant thereof, which is defined below in any of (25αA1-a) to (25αA1-e):
         (25αA1-a) a protein comprising an amino acid sequence with deletion, substitution, insertion or addition of one or several amino acids in SEQ ID NO: 348;
         (25αA1-b) a protein comprising an amino acid sequence having at least 70% identity to the amino acid sequence of SEQ ID NO: 348;
         (25αA1-c) a protein comprising an amino acid sequence encoded by a nucleotide sequence with deletion, substitution, insertion or addition of one or several nucleotides in SEQ ID NO: 347;
         (25αA1-d) a protein comprising an amino acid sequence encoded by a nucleotide sequence having at least 70% identity to the nucleotide sequence of SEQ ID NO: 347; or
         (25αA1-e) a protein comprising an amino acid sequence encoded by a nucleic acid that hybridizes under stringent conditions with a nucleic acid having a nucleotide sequence complementary to the nucleotide sequence of SEQ ID NO: 347;
      (25αB1) a protein comprising at least one amino acid sequence selected from the group consisting of SEQ ID NOs: 349-356;
      (25αA2) succinyl-CoA ligase [ADP-forming] subunit beta, mitochondrial or a variant thereof, which is defined below in any of (25αA2-a) to (25αA2-e):
         (25αA2-a) a protein comprising an amino acid sequence with deletion, substitution, insertion or addition of one or several amino acids in SEQ ID NO: 358;
         (25αA2-b) a protein comprising an amino acid sequence having at least 70% identity to the amino acid sequence of SEQ ID NO: 358;
         (25αA2-c) a protein comprising an amino acid sequence encoded by a nucleotide sequence with deletion, substitution, insertion or addition of one or several nucleotides in SEQ ID NO: 357;
         (25αA2-d) a protein comprising an amino acid sequence encoded by a nucleotide sequence having at least 70% identity to the nucleotide sequence of SEQ ID NO: 357; or
         (25αA2-e) a protein comprising an amino acid sequence encoded by a nucleic acid that hybridizes under stringent conditions with a nucleic acid having a nucleotide sequence complementary to the nucleotide sequence of SEQ ID NO: 357; or
      (25αB2) a protein comprising at least one amino acid sequence selected from the group consisting of SEQ ID NOs: 359-365;
   (26α)
      (26αA1) phosphoglycerate kinase, cytosolic or a variant thereof, which is defined below in any of (26αA1-a) to (26αA1-e):
         (26αA1-a) a protein comprising an amino acid sequence with deletion, substitution, insertion or addition of one or several amino acids in SEQ ID NO: 367;
         (26αA1-b) a protein comprising an amino acid sequence having at least 70% identity to the amino acid sequence of SEQ ID NO: 367;
         (26αA1-c) a protein comprising an amino acid sequence encoded by a nucleotide sequence with deletion, substitution, insertion or addition of one or several nucleotides in SEQ ID NO: 366;
         (26αA1-d) a protein comprising an amino acid sequence encoded by a nucleotide sequence having at least 70% identity to the nucleotide sequence of SEQ ID NO: 366; or
         (26αA1-e) a protein comprising an amino acid sequence encoded by a nucleic acid that hybridizes under stringent conditions with a nucleic acid having a nucleotide sequence complementary to the nucleotide sequence of SEQ ID NO: 366;
      (26αB1) a protein comprising at least one amino acid sequence selected from the group consisting of SEQ ID NOs: 368-382;
      (26αA2) phosphoglycerate kinase, cytosolic or a variant thereof, which is defined below in any of (26αA2-a) to (26αA2-e):
         (26αA2-a) a protein comprising an amino acid sequence with deletion, substitution, insertion or addition of one or several amino acids in SEQ ID NO: 384;
         (26αA2-b) a protein comprising an amino acid sequence having at least 70% identity to the amino acid sequence of SEQ ID NO: 384;
         (26αA2-c) a protein comprising an amino acid sequence encoded by a nucleotide sequence with deletion, substitution, insertion or addition of one or several nucleotides in SEQ ID NO: 383;
         (26αA2-d) a protein comprising an amino acid sequence encoded by a nucleotide sequence having at least 70% identity to the nucleotide sequence of SEQ ID NO: 383; or
         (26αA2-e) a protein comprising an amino acid sequence encoded by a nucleic acid that hybridizes under stringent conditions with a nucleic acid having a nucleotide sequence complementary to the nucleotide sequence of SEQ ID NO: 383;
      (26αB2) a protein comprising at least one amino acid sequence selected from the group consisting of SEQ ID NOs: 385-392;
      (26αA3) phosphoglycerate kinase, cytosolic or a variant thereof, which is defined below in any of (26αA3-a) to (26αA3-e):
         (26αA3-a) a protein comprising an amino acid sequence with deletion, substitution, insertion or addition of one or several amino acids in SEQ ID NO: 394;
         (26αA3-b) a protein comprising an amino acid sequence having at least 70% identity to the amino acid sequence of SEQ ID NO: 394;
         (26αA3-c) a protein comprising an amino acid sequence encoded by a nucleotide sequence with deletion, substitution, insertion or addition of one or several nucleotides in SEQ ID NO: 393;
         (26αA3-d) a protein comprising an amino acid sequence encoded by a nucleotide sequence having at least 70% identity to the nucleotide sequence of SEQ ID NO: 393; or
         (26αA3-e) a protein comprising an amino acid sequence encoded by a nucleic acid that hybridizes under stringent conditions with a nucleic acid having a nucleotide sequence complementary to the nucleotide sequence of SEQ ID NO: 393; or
      (26αB3) a protein comprising at least one amino acid sequence selected from the group consisting of SEQ ID NOs: 395-409;
   (27α)
      (27αA1) alcohol dehydrogenase 1 or a variant thereof, which is defined below in any of (27αA1-a) to (27αA1-e):
         (27αA1-a) a protein comprising an amino acid sequence with deletion, substitution, insertion or addition of one or several amino acids in SEQ ID NO: 411;
         (27αA1-b) a protein comprising an amino acid sequence having at least 70% identity to the amino acid sequence of SEQ ID NO: 411;
         (27αA1-c) a protein comprising an amino acid sequence encoded by a nucleotide sequence with deletion, substitution, insertion or addition of one or several nucleotides in SEQ ID NO: 410;
         (27αA1-d) a protein comprising an amino acid sequence encoded by a nucleotide sequence having at least 70% identity to the nucleotide sequence of SEQ ID NO: 410; or
         (27αA1-e) a protein comprising an amino acid sequence encoded by a nucleic acid that hybridizes under stringent conditions with a nucleic acid having a nucleotide sequence complementary to the nucleotide sequence of SEQ ID NO: 410;
      (27αB1) a protein comprising at least one amino acid sequence selected from the group consisting of SEQ ID NOs: 412-416;
      (27αA2) alcohol dehydrogenase 1 or a variant thereof, which is defined below in any of (27αA2-a) to (27αA2-e):
         (27αA2-a) a protein comprising an amino acid sequence with deletion, substitution, insertion or addition of one or several amino acids in SEQ ID NO: 418;
         (27αA2-b) a protein comprising an amino acid sequence having at least 70% identity to the amino acid sequence of SEQ ID NO: 418;
         (27αA2-c) a protein comprising an amino acid sequence encoded by a nucleotide sequence with deletion, substitution, insertion or addition of one or several nucleotides in SEQ ID NO: 417;
         (27αA2-d) a protein comprising an amino acid sequence encoded by a nucleotide sequence having at least 70% identity to the nucleotide sequence of SEQ ID NO: 417; or
         (27αA2-e) a protein comprising an amino acid sequence encoded by a nucleic acid that hybridizes under stringent conditions with a nucleic acid having a nucleotide sequence complementary to the nucleotide sequence of SEQ ID NO: 417;
      (27αB2) a protein comprising at least one amino acid sequence selected from the group consisting of SEQ ID NOs: 419 and 420;
      (27αA3) alcohol dehydrogenase 1-like or a variant thereof, which is defined below in any of (27αA3-a) to (27αA3-e):
         (27αA3-a) a protein comprising an amino acid sequence with deletion, substitution, insertion or addition of one or several amino acids in SEQ ID NO: 422;
         (27αA3-b) a protein comprising an amino acid sequence having at least 70% identity to the amino acid sequence of SEQ ID NO: 422;
         (27αA3-c) a protein comprising an amino acid sequence encoded by a nucleotide sequence with deletion, substitution, insertion or addition of one or several nucleotides in SEQ ID NO: 421;
         (27αA3-d) a protein comprising an amino acid sequence encoded by a nucleotide sequence having at least 70% identity to the nucleotide sequence of SEQ ID NO: 421; or
         (27αA3-e) a protein comprising an amino acid sequence encoded by a nucleic acid that hybridizes under stringent conditions with a nucleic acid having a nucleotide sequence complementary to the nucleotide sequence of SEQ ID NO: 421; or
      (27αB3) a protein comprising at least one amino acid sequence selected from the group consisting of SEQ ID NOs: 423-427;
   (28α)
      (28αA) 35 kDa seed maturation protein isoform X1 or a variant thereof, which is defined below in any of (28αA-a) to (28αA-e):
         (28αA-a) a protein comprising an amino acid sequence with deletion, substitution, insertion or addition of one or several amino acids in SEQ ID NO: 429;
         (28αA-b) a protein comprising an amino acid sequence having at least 70% identity to the amino acid sequence of SEQ ID NO: 429;
         (28αA-c) a protein comprising an amino acid sequence encoded by a nucleotide sequence with deletion, substitution, insertion or addition of one or several nucleotides in SEQ ID NO: 428;
         (28αA-d) a protein comprising an amino acid sequence encoded by a nucleotide sequence having at least 70% identity to the nucleotide sequence of SEQ ID NO: 428; or
         (28αA-e) a protein comprising an amino acid sequence encoded by a nucleic acid that hybridizes under stringent conditions with a nucleic acid having a nucleotide sequence complementary to the nucleotide sequence of SEQ ID NO: 428; or
      (28αB) a protein comprising at least one amino acid sequence selected from the group consisting of SEQ ID NOs: 430-433;
   (29-33α)
      (29-33αA1) glyceraldehyde-3-phosphate dehydrogenase isoform X1 or a variant thereof, which is defined below in any of (29-33αA1-a) to (29-33αA1-e):
         (29-33αA1-a) a protein comprising an amino acid sequence with deletion, substitution, insertion or addition of one or several amino acids in SEQ ID NO: 435;
         (29-33αA1-b) a protein comprising an amino acid sequence having at least 70% identity to the amino acid sequence of SEQ ID NO: 435;
         (29-33αA1-c) a protein comprising an amino acid sequence encoded by a nucleotide sequence with deletion, substitution, insertion or addition of one or several nucleotides in SEQ ID NO: 434;
         (29-33αA1-d) a protein comprising an amino acid sequence encoded by a nucleotide sequence having at least 70% identity to the nucleotide sequence of SEQ ID NO: 434; or
         (29-33αA1-e) a protein comprising an amino acid sequence encoded by a nucleic acid that hybridizes under stringent conditions with a nucleic acid having a nucleotide sequence complementary to the nucleotide sequence of SEQ ID NO: 434;
      (29-33αB1) a protein comprising at least one amino acid sequence selected from the group consisting of SEQ ID NOs: 436-439;
      (29-33αA2) glyceraldehyde-3-phosphate dehydrogenase, cytosolic or a variant thereof, which is defined below in any of (29-33αA2-a) to (29-33αA2-e):
         (29-33αA2-a) a protein comprising an amino acid sequence with deletion, substitution, insertion or addition of one or several amino acids in SEQ ID NO: 441;
         (29-33αA2-b) a protein comprising an amino acid sequence having at least 70% identity to the amino acid sequence of SEQ ID NO: 441;
         (29-33αA2-c) a protein comprising an amino acid sequence encoded by a nucleotide sequence with deletion, substitution, insertion or addition of one or several nucleotides in SEQ ID NO: 440;
         (29-33αA2-d) a protein comprising an amino acid sequence encoded by a nucleotide sequence having at least 70% identity to the nucleotide sequence of SEQ ID NO: 440; or
         (29-33αA2-e) a protein comprising an amino acid sequence encoded by a nucleic acid that hybridizes under stringent conditions with a nucleic acid having a nucleotide sequence complementary to the nucleotide sequence of SEQ ID NO: 440;
      (29-33αB2) a protein comprising at least one amino acid sequence selected from the group consisting of SEQ ID NOs: 442-445;
      (29-33αA3) uncharacterized protein LOC100782924 or a variant thereof, which is defined below in any of (29-33αA3-a) to (29-33αA3-e):
         (29-33αA3-a) a protein comprising an amino acid sequence with deletion, substitution, insertion or addition of one or several amino acids in SEQ ID NO: 447;
         (29-33αA3-b) a protein comprising an amino acid sequence having at least 70% identity to the amino acid sequence of SEQ ID NO: 447;
         (29-33αA3-c) a protein comprising an amino acid sequence encoded by a nucleotide sequence with deletion, substitution, insertion or addition of one or several nucleotides in SEQ ID NO: 446;
         (29-33αA3-d) a protein comprising an amino acid sequence encoded by a nucleotide sequence having at least 70% identity to the nucleotide sequence of SEQ ID NO: 446; or
         (29-33αA3-e) a protein comprising an amino acid sequence encoded by a nucleic acid that hybridizes under stringent conditions with a nucleic acid having a nucleotide sequence complementary to the nucleotide sequence of SEQ ID NO: 446; or
      (29-33αB3) a protein comprising at least one amino acid sequence selected from the group consisting of SEQ ID NOs: 448-451,
   (34α)
      (34αA1) bifunctional UDP-glucose 4-epimerase and UDP-xylose 4-epimerase 1-like or a variant thereof, which is defined below in any of (34αA1-a) to (34αA1-e):
         (34αA1-a) a protein comprising an amino acid sequence with deletion, substitution, insertion or addition of one or several amino acids in SEQ ID NO: 453;
         (34αA1-b) a protein comprising an amino acid sequence having at least 70% identity to the amino acid sequence of SEQ ID NO: 453;
         (34αA1-c) a protein comprising an amino acid sequence encoded by a nucleotide sequence with deletion, substitution, insertion or addition of one or several nucleotides in SEQ ID NO: 452;
         (34αA1-d) a protein comprising an amino acid sequence encoded by a nucleotide sequence having at least 70% identity to the nucleotide sequence of SEQ ID NO: 452; or
         (34αA1-e) a protein comprising an amino acid sequence encoded by a nucleic acid that hybridizes under stringent conditions with a nucleic acid having a nucleotide sequence complementary to the nucleotide sequence of SEQ ID NO: 452;
      (34αB1) a protein comprising an amino acid sequence of SEQ ID NO: 454;
      (34αA2) uncharacterized protein LOC100803483 or a variant thereof, which is defined below in any of (34αA2-a) to (34αA2-e):
         (34αA2-a) a protein comprising an amino acid sequence with deletion, substitution, insertion or addition of one or several amino acids in SEQ ID NO: 456;
         (34αA2-b) a protein comprising an amino acid sequence having at least 70% identity to the amino acid sequence of SEQ ID NO: 456;
         (34αA2-c) a protein comprising an amino acid sequence encoded by a nucleotide sequence with deletion, substitution, insertion or addition of one or several nucleotides in SEQ ID NO: 455;
         (34αA2-d) a protein comprising an amino acid sequence encoded by a nucleotide sequence having at least 70% identity to the nucleotide sequence of SEQ ID NO: 455; or
         (34αA2-e) a protein comprising an amino acid sequence encoded by a nucleic acid that hybridizes under stringent conditions with a nucleic acid having a nucleotide sequence complementary to the nucleotide sequence of SEQ ID NO: 455; or
      (34αB2) a protein comprising at least one amino acid sequence selected from the group consisting of SEQ ID NOs: 457 and 458;
   (35α)
      (35αA1) elongation factor 1-alpha or a variant thereof, which is defined below in any of (35αA1-a) to (35αA1-e):
         (35αA1-a) a protein comprising an amino acid sequence with deletion, substitution, insertion or addition of one or several amino acids in SEQ ID NO: 460;
         (35αA1-b) a protein comprising an amino acid sequence having at least 70% identity to the amino acid sequence of SEQ ID NO: 460;
         (35αA1-c) a protein comprising an amino acid sequence encoded by a nucleotide sequence with deletion, substitution, insertion or addition of one or several nucleotides in SEQ ID NO: 459;
         (35αA1-d) a protein comprising an amino acid sequence encoded by a nucleotide sequence having at least 70% identity to the nucleotide sequence of SEQ ID NO: 459; or
         (35αA1-e) a protein comprising an amino acid sequence encoded by a nucleic acid that hybridizes under stringent conditions with a nucleic acid having a nucleotide sequence complementary to the nucleotide sequence of SEQ ID NO: 459;
      (35αB1) a protein comprising at least one amino acid sequence selected from the group consisting of SEQ ID NOs: 461-466;
      (35αA2) elongation factor 1-alpha or a variant thereof, which is defined below in any of (35αA2-a) to (35αA2-e):
         (35αA2-a) a protein comprising an amino acid sequence with deletion, substitution, insertion or addition of one or several amino acids in SEQ ID NO: 468;
         (35αA2-b) a protein comprising an amino acid sequence having at least 70% identity to the amino acid sequence of SEQ ID NO: 468;
         (35αA2-c) a protein comprising an amino acid sequence encoded by a nucleotide sequence with deletion, substitution, insertion or addition of one or several nucleotides in SEQ ID NO: 467;
         (35αA2-d) a protein comprising an amino acid sequence encoded by a nucleotide sequence having at least 70% identity to the nucleotide sequence of SEQ ID NO: 467; or
         (35αA2-e) a protein comprising an amino acid sequence encoded by a nucleic acid that hybridizes under stringent conditions with a nucleic acid having a nucleotide sequence complementary to the nucleotide sequence of SEQ ID NO: 467;
      (35αB2) a protein comprising at least one amino acid sequence selected from the group consisting of SEQ ID NOs: 469-471;
      (35αA3) elongation factor-1A isoform X1 or a variant thereof, which is defined below in any of (35αA3-a) to (35αA3-e):
         (35αA3-a) a protein comprising an amino acid sequence with deletion, substitution, insertion or addition of one or several amino acids in SEQ ID NO: 473;
         (35αA3-b) a protein comprising an amino acid sequence having at least 70% identity to the amino acid sequence of SEQ ID NO: 473;
         (35αA3-c) a protein comprising an amino acid sequence encoded by a nucleotide sequence with deletion, substitution, insertion or addition of one or several nucleotides in SEQ ID NO: 472;
         (35αA3-d) a protein comprising an amino acid sequence encoded by a nucleotide sequence having at least 70% identity to the nucleotide sequence of SEQ ID NO: 472; or
         (35αA3-e) a protein comprising an amino acid sequence encoded by a nucleic acid that hybridizes under stringent conditions with a nucleic acid having a nucleotide sequence complementary to the nucleotide sequence of SEQ ID NO: 472; or
      (35αB3) a protein comprising at least one amino acid sequence selected from the group consisting of SEQ ID NOs: 474-477;
   (36, 37α)
      (36, 37αA1) seed maturation protein PM34 or a variant thereof, which is defined below in any of (36, 37αA1-a) to (36, 37αA1-e):
         (36, 37αA1-a) a protein comprising an amino acid sequence with deletion, substitution, insertion or addition of one or several amino acids in SEQ ID NO: 479;
         (36, 37αA1-b) a protein comprising an amino acid sequence having at least 70% identity to the amino acid sequence of SEQ ID NO: 479;
         (36, 37αA1-c) a protein comprising an amino acid sequence encoded by a nucleotide sequence with deletion, substitution, insertion or addition of one or several nucleotides in SEQ ID NO: 478;
         (36, 37αA1-d) a protein comprising an amino acid sequence encoded by a nucleotide sequence having at least 70% identity to the nucleotide sequence of SEQ ID NO: 478; or
         (36, 37αA1-e) a protein comprising an amino acid sequence encoded by a nucleic acid that hybridizes under stringent conditions with a nucleic acid having a nucleotide sequence complementary to the nucleotide sequence of SEQ ID NO: 478;
      (36, 37αB1) a protein comprising at least one amino acid sequence selected from the group consisting of SEQ ID NOs: 480-483;
      (36, 37αA2) uncharacterized protein LOC100809384 or a variant thereof, which is defined below in any of (36, 37αA2-a) to (36, 37αA2-e):
         (36, 37αA2-a) a protein comprising an amino acid sequence with deletion, substitution, insertion or addition of one or several amino acids in SEQ ID NO: 485;
         (36, 37αA2-b) a protein comprising an amino acid sequence having at least 70% identity to the amino acid sequence of SEQ ID NO: 485;
         (36, 37αA2-c) a protein comprising an amino acid sequence encoded by a nucleotide sequence with deletion, substitution, insertion or addition of one or several nucleotides in SEQ ID NO: 484;
         (36, 37αA2-d) a protein comprising an amino acid sequence encoded by a nucleotide sequence having at least 70% identity to the nucleotide sequence of SEQ ID NO: 484; or
         (36, 37αA2-e) a protein comprising an amino acid sequence encoded by a nucleic acid that hybridizes under stringent conditions with a nucleic acid having a nucleotide sequence complementary to the nucleotide sequence of SEQ ID NO: 484;
      (36, 37αB2) a protein comprising at least one amino acid sequence selected from the group consisting of SEQ ID NOs: 486 and 487;
      (36, 37αA3) hypothetical protein GLYMA_10G155300 or a variant thereof, which is defined below in any of (36, 37αA3-a) to (36, 37αA3-e):
         (36, 37αA3-a) a protein comprising an amino acid sequence with deletion, substitution, insertion or addition of one or several amino acids in SEQ ID NO: 489;
         (36, 37αA3-b) a protein comprising an amino acid sequence having at least 70% identity to the amino acid sequence of SEQ ID NO: 489;
         (36, 37αA3-c) a protein comprising an amino acid sequence encoded by a nucleotide sequence with deletion, substitution, insertion or addition of one or several nucleotides in SEQ ID NO: 488;
         (36, 37αA3-d) a protein comprising an amino acid sequence encoded by a nucleotide sequence having at least 70% identity to the nucleotide sequence of SEQ ID NO: 488; or
         (36, 37αA3-e) a protein comprising an amino acid sequence encoded by a nucleic acid that hybridizes under stringent conditions with a nucleic acid having a nucleotide sequence complementary to the nucleotide sequence of SEQ ID NO: 488; or
      (36, 37αB3) a protein comprising at least one amino acid sequence selected from the group consisting of SEQ ID NOs: 490-493;
   (38α)
      (38αA) seed biotin-containing protein SBP65-like isoform X1 or a variant thereof, which is defined below in any of (38αA-a) to (38αA-e):
         (38αA-a) a protein comprising an amino acid sequence with deletion, substitution, insertion or addition of one or several amino acids in SEQ ID NO: 495;
         (38αA-b) a protein comprising an amino acid sequence having at least 70% identity to the amino acid sequence of SEQ ID NO: 495;
         (38αA-c) a protein comprising an amino acid sequence encoded by a nucleotide sequence with deletion, substitution, insertion or addition of one or several nucleotides in SEQ ID NO: 494;
         (38αA-d) a protein comprising an amino acid sequence encoded by a nucleotide sequence having at least 70% identity to the nucleotide sequence of SEQ ID NO: 494; or
         (38αA-e) a protein comprising an amino acid sequence encoded by a nucleic acid that hybridizes under stringent conditions with a nucleic acid having a nucleotide sequence complementary to the nucleotide sequence of SEQ ID NO: 494; or
      (38αB) a protein comprising at least one amino acid sequence selected from the group consisting of SEQ ID NOs: 496-513;
   (39α)
      (39αA) ATP synthase subunit O, mitochondrial-like or a variant thereof, which is defined below in any of (39αA-a) to (39αA-e):
         (39αA-a) a protein comprising an amino acid sequence with deletion, substitution, insertion or addition of one or several amino acids in SEQ ID NO: 515;
         (39αA-b) a protein comprising an amino acid sequence having at least 70% identity to the amino acid sequence of SEQ ID NO: 515;
         (39αA-c) a protein comprising an amino acid sequence encoded by a nucleotide sequence with deletion, substitution, insertion or addition of one or several nucleotides in SEQ ID NO: 514;
         (39αA-d) a protein comprising an amino acid sequence encoded by a nucleotide sequence having at least 70% identity to the nucleotide sequence of SEQ ID NO: 514; or
         (39αA-e) a protein comprising an amino acid sequence encoded by a nucleic acid that hybridizes under stringent conditions with a nucleic acid having a nucleotide sequence complementary to the nucleotide sequence of SEQ ID NO: 514; or
      (39αB) a protein comprising an amino acid sequence of SEQ ID NO: 516; (40, 56α) (40, 56αA) P24 oleosin isoform A or a variant thereof, which is defined below in any of (40, 56αA-a) to (40, 56αA-e):
         (40, 56αA-a) a protein comprising an amino acid sequence with deletion, substitution, insertion or addition of one or several amino acids in SEQ ID NO: 518;
         (40, 56αA-b) a protein comprising an amino acid sequence having at least 70% identity to the amino acid sequence of SEQ ID NO: 518;
         (40, 56αA-c) a protein comprising an amino acid sequence encoded by a nucleotide sequence with deletion, substitution, insertion or addition of one or several nucleotides in SEQ ID NO: 517;
         (40, 56αA-d) a protein comprising an amino acid sequence encoded by a nucleotide sequence having at least 70% identity to the nucleotide sequence of SEQ ID NO: 517; or
         (40, 56αA-e) a protein comprising an amino acid sequence encoded by a nucleic acid that hybridizes under stringent conditions with a nucleic acid having a nucleotide sequence complementary to the nucleotide sequence of SEQ ID NO: 517; or
      (40, 56αB) a protein comprising at least one amino acid sequence selected from the group consisting of SEQ ID NOs: 519 and 520;
   (41α)
      (41αA) uncharacterized protein At3g49720-like isoform X2 or a variant thereof, which is defined below in any of (41αA-a) to (41αA-e):
         (41αA-a) a protein comprising an amino acid sequence with deletion, substitution, insertion or addition of one or several amino acids in SEQ ID NO: 522;
         (41αA-b) a protein comprising an amino acid sequence having at least 70% identity to the amino acid sequence of SEQ ID NO: 522;
         (41αA-c) a protein comprising an amino acid sequence encoded by a nucleotide sequence with deletion, substitution, insertion or addition of one or several nucleotides in SEQ ID NO: 521;
         (41αA-d) a protein comprising an amino acid sequence encoded by a nucleotide sequence having at least 70% identity to the nucleotide sequence of SEQ ID NO: 521; or
         (41αA-e) a protein comprising an amino acid sequence encoded by a nucleic acid that hybridizes under stringent conditions with a nucleic acid having a nucleotide sequence complementary to the nucleotide sequence of SEQ ID NO: 521; or
      (41αB) a protein comprising an amino acid sequence of SEQ ID NO: 523;
   (42, 43α)
      (42, 43αA1) superoxide dismutase [Mn], mitochondrial or a variant thereof, which is defined below in any of (42, 43αA1-a) to (42, 43αA1-e):
         (42, 43αA1-a) a protein comprising an amino acid sequence with deletion, substitution, insertion or addition of one or several amino acids in SEQ ID NO: 525;
         (42, 43αA1-b) a protein comprising an amino acid sequence having at least 70% identity to the amino acid sequence of SEQ ID NO: 525;
         (42, 43αA1-c) a protein comprising an amino acid sequence encoded by a nucleotide sequence with deletion, substitution, insertion or addition of one or several nucleotides in SEQ ID NO: 524;
         (42, 43αA1-d) a protein comprising an amino acid sequence encoded by a nucleotide sequence having at least 70% identity to the nucleotide sequence of SEQ ID NO: 524; or
         (42, 43αA1-e) a protein comprising an amino acid sequence encoded by a nucleic acid that hybridizes under stringent conditions with a nucleic acid having a nucleotide sequence complementary to the nucleotide sequence of SEQ ID NO: 524;
      (42, 43αB1) a protein comprising at least one amino acid sequence selected from the group consisting of SEQ ID NOs: 526-531;
      (42, 43αA2) hypothetical protein GLYMA_04G221300 or a variant thereof, which is defined below in any of (42, 43αA2-a) to (42, 43αA2-e):
         (42, 43αA2-a) a protein comprising an amino acid sequence with deletion, substitution, insertion or addition of one or several amino acids in SEQ ID NO: 533;
         (42, 43αA2-b) a protein comprising an amino acid sequence having at least 70% identity to the amino acid sequence of SEQ ID NO: 533;
         (42, 43αA2-c) a protein comprising an amino acid sequence encoded by a nucleotide sequence with deletion, substitution, insertion or addition of one or several nucleotides in SEQ ID NO: 532;
         (42, 43αA2-d) a protein comprising an amino acid sequence encoded by a nucleotide sequence having at least 70% identity to the nucleotide sequence of SEQ ID NO: 532; or
         (42, 43αA2-e) a protein comprising an amino acid sequence encoded by a nucleic acid that hybridizes under stringent conditions with a nucleic acid having a nucleotide sequence complementary to the nucleotide sequence of SEQ ID NO: 532; or
      (42, 43αB2) a protein comprising at least one amino acid sequence selected from the group consisting of SEQ ID NOs: 534-539;
   (45α)
      (45αA1) uncharacterized protein LOC100499771 or a variant thereof, which is defined below in any of (45αA1-a) to (45αA1-e):
         (45αA1-a) a protein comprising an amino acid sequence with deletion, substitution, insertion or addition of one or several amino acids in SEQ ID NO: 541;
         (45αA1-b) a protein comprising an amino acid sequence having at least 70% identity to the amino acid sequence of SEQ ID NO: 541;
         (45αA1-c) a protein comprising an amino acid sequence encoded by a nucleotide sequence with deletion, substitution, insertion or addition of one or several nucleotides in SEQ ID NO: 540;
         (45αA1-d) a protein comprising an amino acid sequence encoded by a nucleotide sequence having at least 70% identity to the nucleotide sequence of SEQ ID NO: 540; or
         (45αA1-e) a protein comprising an amino acid sequence encoded by a nucleic acid that hybridizes under stringent conditions with a nucleic acid having a nucleotide sequence complementary to the nucleotide sequence of SEQ ID NO: 540;
      (45αB1) a protein comprising at least one amino acid sequence selected from the group consisting of SEQ ID NOs: 542-545;
      (45αA2) hypothetical protein GLYMA_09G192800 or a variant thereof, which is defined below in any of (45αA2-a) to (45αA2-e):
         (45αA2-a) a protein comprising an amino acid sequence with deletion, substitution, insertion or addition of one or several amino acids in SEQ ID NO: 547;
         (45αA2-b) a protein comprising an amino acid sequence having at least 70% identity to the amino acid sequence of SEQ ID NO: 547;
         (45αA2-c) a protein comprising an amino acid sequence encoded by a nucleotide sequence with deletion, substitution, insertion or addition of one or several nucleotides in SEQ ID NO: 546;
         (45αA2-d) a protein comprising an amino acid sequence encoded by a nucleotide sequence having at least 70% identity to the nucleotide sequence of SEQ ID NO: 546; or
         (45αA2-e) a protein comprising an amino acid sequence encoded by a nucleic acid that hybridizes under stringent conditions with a nucleic acid having a nucleotide sequence complementary to the nucleotide sequence of SEQ ID NO: 546; or
      (45αB2) a protein comprising at least one amino acid sequence selected from the group consisting of SEQ ID NOs: 548-551;
   (46α)
      (46αA) seed maturation protein PM22 or a variant thereof, which is defined below in any of (46αA-a) to (46αA-e):
         (46αA-a) a protein comprising an amino acid sequence with deletion, substitution, insertion or addition of one or several amino acids in SEQ ID NO: 553;
         (46αA-b) a protein comprising an amino acid sequence having at least 70% identity to the amino acid sequence of SEQ ID NO: 553;
         (46αA-c) a protein comprising an amino acid sequence encoded by a nucleotide sequence with deletion, substitution, insertion or addition of one or several nucleotides in SEQ ID NO: 552;
         (46αA-d) a protein comprising an amino acid sequence encoded by a nucleotide sequence having at least 70% identity to the nucleotide sequence of SEQ ID NO: 552; or
         (46αA-e) a protein comprising an amino acid sequence encoded by a nucleic acid that hybridizes under stringent conditions with a nucleic acid having a nucleotide sequence complementary to the nucleotide sequence of SEQ ID NO: 552; or
      (46αB) a protein comprising an amino acid sequence of SEQ ID NO: 554;
   (47α)
      (47αA1) eukaryotic translation initiation factor 5A-2 or a variant thereof, which is defined below in any of (47αA1-a) to (47αA1-e):
         (47αA1-a) a protein comprising an amino acid sequence with deletion, substitution, insertion or addition of one or several amino acids in SEQ ID NO: 556;
         (47αA1-b) a protein comprising an amino acid sequence having at least 70% identity to the amino acid sequence of SEQ ID NO: 556;
         (47αA1-c) a protein comprising an amino acid sequence encoded by a nucleotide sequence with deletion, substitution, insertion or addition of one or several nucleotides in SEQ ID NO: 555;
         (47αA1-d) a protein comprising an amino acid sequence encoded by a nucleotide sequence having at least 70% identity to the nucleotide sequence of SEQ ID NO: 555; or
         (47αA1-e) a protein comprising an amino acid sequence encoded by a nucleic acid that hybridizes under stringent conditions with a nucleic acid having a nucleotide sequence complementary to the nucleotide sequence of SEQ ID NO: 555;
      (47αB1) a protein comprising at least one amino acid sequence selected from the group consisting of SEQ ID NOs: 557 and 558;
      (47αA2) uncharacterized protein LOC100305510 or a variant thereof, which is defined below in any of (47αA2-a) to (47αA2-e):
         (47αA2-a) a protein comprising an amino acid sequence with deletion, substitution, insertion or addition of one or several amino acids in SEQ ID NO: 560;
         (47αA2-b) a protein comprising an amino acid sequence having at least 70% identity to the amino acid sequence of SEQ ID NO: 560;
         (47αA2-c) a protein comprising an amino acid sequence encoded by a nucleotide sequence with deletion, substitution, insertion or addition of one or several nucleotides in SEQ ID NO: 559;
         (47αA2-d) a protein comprising an amino acid sequence encoded by a nucleotide sequence having at least 70% identity to the nucleotide sequence of SEQ ID NO: 559; or
         (47αA2-e) a protein comprising an amino acid sequence encoded by a nucleic acid that hybridizes under stringent conditions with a nucleic acid having a nucleotide sequence complementary to the nucleotide sequence of SEQ ID NO: 559; or
      (47αB2) a protein comprising at least one amino acid sequence selected from the group consisting of SEQ ID NOs: 561-563;
   (48α)
      (48αA) uncharacterized protein LOC100306570 or a variant thereof, which is defined below in any of (48αA-a) to (48αA-e):
         (48αA-a) a protein comprising an amino acid sequence with deletion, substitution, insertion or addition of one or several amino acids in SEQ ID NO: 565;
         (48αA-b) a protein comprising an amino acid sequence having at least 70% identity to the amino acid sequence of SEQ ID NO: 565;
         (48αA-c) a protein comprising an amino acid sequence encoded by a nucleotide sequence with deletion, substitution, insertion or addition of one or several nucleotides in SEQ ID NO: 564;
         (48αA-d) a protein comprising an amino acid sequence encoded by a nucleotide sequence having at least 70% identity to the nucleotide sequence of SEQ ID NO: 564; or
         (48αA-e) a protein comprising an amino acid sequence encoded by a nucleic acid that hybridizes under stringent conditions with a nucleic acid having a nucleotide sequence complementary to the nucleotide sequence of SEQ ID NO: 564; or
      (48αB) a protein comprising at least one amino acid sequence selected from the group consisting of SEQ ID NOs: 566-570;
   (50α)
      (50αA) uncharacterized protein LOC100813859 or a variant thereof, which is defined below in any of (50αA-a) to (50αA-e):
         (50αA-a) a protein comprising an amino acid sequence with deletion, substitution, insertion or addition of one or several amino acids in SEQ ID NO: 572;
         (50αA-b) a protein comprising an amino acid sequence having at least 70% identity to the amino acid sequence of SEQ ID NO: 572;
         (50αA-c) a protein comprising an amino acid sequence encoded by a nucleotide sequence with deletion, substitution, insertion or addition of one or several nucleotides in SEQ ID NO: 571;
         (50αA-d) a protein comprising an amino acid sequence encoded by a nucleotide sequence having at least 70% identity to the nucleotide sequence of SEQ ID NO: 571; or
         (50αA-e) a protein comprising an amino acid sequence encoded by a nucleic acid that hybridizes under stringent conditions with a nucleic acid having a nucleotide sequence complementary to the nucleotide sequence of SEQ ID NO: 571; or
      (50αB) a protein comprising at least one amino acid sequence selected from the group consisting of SEQ ID NOs: 573-576;
   (51α)
      (51αA) 14-3-3-like protein or a variant thereof, which is defined below in any of (51αA-a) to (51αA-e):
         (51αA-a) a protein comprising an amino acid sequence with deletion, substitution, insertion or addition of one or several amino acids in SEQ ID NO: 578;
         (51αA-b) a protein comprising an amino acid sequence having at least 70% identity to the amino acid sequence of SEQ ID NO: 578;
         (51αA-c) a protein comprising an amino acid sequence encoded by a nucleotide sequence with deletion, substitution, insertion or addition of one or several nucleotides in SEQ ID NO: 577;
         (51αA-d) a protein comprising an amino acid sequence encoded by a nucleotide sequence having at least 70% identity to the nucleotide sequence of SEQ ID NO: 577; or
         (51αA-e) a protein comprising an amino acid sequence encoded by a nucleic acid that hybridizes under stringent conditions with a nucleic acid having a nucleotide sequence complementary to the nucleotide sequence of SEQ ID NO: 577; or
      (51αB) a protein comprising at least one amino acid sequence selected from the group consisting of SEQ ID NOs: 579-582;
   (52α)
      (52αA) probable fructokinase-4 or a variant thereof, which is defined below in any of (52αA-a) to (52αA-e):
         (52αA-a) a protein comprising an amino acid sequence with deletion, substitution, insertion or addition of one or several amino acids in SEQ ID NO: 584;
         (52αA-b) a protein comprising an amino acid sequence having at least 70% identity to the amino acid sequence of SEQ ID NO: 584;
         (52αA-c) a protein comprising an amino acid sequence encoded by a nucleotide sequence with deletion, substitution, insertion or addition of one or several nucleotides in SEQ ID NO: 583;
         (52αA-d) a protein comprising an amino acid sequence encoded by a nucleotide sequence having at least 70% identity to the nucleotide sequence of SEQ ID NO: 583; or
         (52αA-e) a protein comprising an amino acid sequence encoded by a nucleic acid that hybridizes under stringent conditions with a nucleic acid having a nucleotide sequence complementary to the nucleotide sequence of SEQ ID NO: 583; or
      (52αB) a protein comprising at least one amino acid sequence selected from the group consisting of SEQ ID NOs: 585-588;
   (53α)
      (53αA1) triosephosphate isomerase isoform X1 or a variant thereof, which is defined below in any of (53αA1-a) to (53αA1-e):
         (53αA1-a) a protein comprising an amino acid sequence with deletion, substitution, insertion or addition of one or several amino acids in SEQ ID NO: 590;
         (53αA1-b) a protein comprising an amino acid sequence having at least 70% identity to the amino acid sequence of SEQ ID NO: 590;
         (53αA1-c) a protein comprising an amino acid sequence encoded by a nucleotide sequence with deletion, substitution, insertion or addition of one or several nucleotides in SEQ ID NO: 589;
         (53αA1-d) a protein comprising an amino acid sequence encoded by a nucleotide sequence having at least 70% identity to the nucleotide sequence of SEQ ID NO: 589; or
         (53αA1-e) a protein comprising an amino acid sequence encoded by a nucleic acid that hybridizes under stringent conditions with a nucleic acid having a nucleotide sequence complementary to the nucleotide sequence of SEQ ID NO: 589;
      (53αB1) a protein comprising at least one amino acid sequence selected from the group consisting of SEQ ID NOs: 591-597;
      (53αA2) triosephosphate isomerase, cytosolic or a variant thereof, which is defined below in any of (53αA2-a) to (53αA2-e):
         (53αA2-a) a protein comprising an amino acid sequence with deletion, substitution, insertion or addition of one or several amino acids in SEQ ID NO: 599;
         (53αA2-b) a protein comprising an amino acid sequence having at least 70% identity to the amino acid sequence of SEQ ID NO: 599;
         (53αA2-c) a protein comprising an amino acid sequence encoded by a nucleotide sequence with deletion, substitution, insertion or addition of one or several nucleotides in SEQ ID NO: 598;
         (53αA2-d) a protein comprising an amino acid sequence encoded by a nucleotide sequence having at least 70% identity to the nucleotide sequence of SEQ ID NO: 598; or
         (53αA2-e) a protein comprising an amino acid sequence encoded by a nucleic acid that hybridizes under stringent conditions with a nucleic acid having a nucleotide sequence complementary to the nucleotide sequence of SEQ ID NO: 598; or
      (53αB2) a protein comprising at least one amino acid sequence selected from the group consisting of SEQ ID NOs: 600-604;
   (54α)
      (54αA1) superoxide dismutase [Fe], chloroplastic isoform X1 or a variant thereof, which is defined below in any of (54αA1-a) to (54αA1-e):
         (54αA1-a) a protein comprising an amino acid sequence with deletion, substitution, insertion or addition of one or several amino acids in SEQ ID NO: 606;
         (54αA1-b) a protein comprising an amino acid sequence having at least 70% identity to the amino acid sequence of SEQ ID NO: 606;
         (53αA1-c) a protein comprising an amino acid sequence encoded by a nucleotide sequence with deletion, substitution, insertion or addition of one or several nucleotides in SEQ ID NO: 605;
         (54αA1-d) a protein comprising an amino acid sequence encoded by a nucleotide sequence having at least 70% identity to the nucleotide sequence of SEQ ID NO: 605; or
         (54αA1-e) a protein comprising an amino acid sequence encoded by a nucleic acid that hybridizes under stringent conditions with a nucleic acid having a nucleotide sequence complementary to the nucleotide sequence of SEQ ID NO: 605;
      (54αB1) a protein comprising at least one amino acid sequence selected from the group consisting of SEQ ID NOs: 607-611;
      (54αA2) iron-superoxide dismutase or a variant thereof, which is defined below in any of (54αA2-a) to (54αA2-e):
         (54αA2-a) a protein comprising an amino acid sequence with deletion, substitution, insertion or addition of one or several amino acids in SEQ ID NO: 613;
         (54αA2-b) a protein comprising an amino acid sequence having at least 70% identity to the amino acid sequence of SEQ ID NO: 613;
         (54αA2-c) a protein comprising an amino acid sequence encoded by a nucleotide sequence with deletion, substitution, insertion or addition of one or several nucleotides in SEQ ID NO: 612;
         (54αA2-d) a protein comprising an amino acid sequence encoded by a nucleotide sequence having at least 70% identity to the nucleotide sequence of SEQ ID NO: 612; or
         (54αA2-e) a protein comprising an amino acid sequence encoded by a nucleic acid that hybridizes under stringent conditions with a nucleic acid having a nucleotide sequence complementary to the nucleotide sequence of SEQ ID NO: 612; or
      (54αB2) a protein comprising at least one amino acid sequence selected from the group consisting of SEQ ID NOs: 614-616; or
   (55α)
      (55αA1) 51 kDa seed maturation protein precursor or a variant thereof, which is defined below in any of (55αA1-a) to (55αA1-e):
         (55αA1-a) a protein comprising an amino acid sequence with deletion, substitution, insertion or addition of one or several amino acids in SEQ ID NO: 618;
         (55αA1-b) a protein comprising an amino acid sequence having at least 70% identity to the amino acid sequence of SEQ ID NO: 618;
         (55αA1-c) a protein comprising an amino acid sequence encoded by a nucleotide sequence with deletion, substitution, insertion or addition of one or several nucleotides in SEQ ID NO: 617;
         (55αA1-d) a protein comprising an amino acid sequence encoded by a nucleotide sequence having at least 70% identity to the nucleotide sequence of SEQ ID NO: 617; or
         (55αA1-e) a protein comprising an amino acid sequence encoded by a nucleic acid that hybridizes under stringent conditions with a nucleic acid having a nucleotide sequence complementary to the nucleotide sequence of SEQ ID NO: 617;
      (55αB1) a protein comprising at least one amino acid sequence selected from the group consisting of SEQ ID NOs: 619-635;
      (55αA2) Lea protein precursor or a variant thereof, which is defined below in any of (55αA2-a) to (55αA2-e):
         (55αA2-a) a protein comprising an amino acid sequence with deletion, substitution, insertion or addition of one or several amino acids in SEQ ID NO: 637;
         (55αA2-b) a protein comprising an amino acid sequence having at least 70% identity to the amino acid sequence of SEQ ID NO: 637;
         (55αA2-c) a protein comprising an amino acid sequence encoded by a nucleotide sequence with deletion, substitution, insertion or addition of one or several nucleotides in SEQ ID NO: 636;
         (55αA2-d) a protein comprising an amino acid sequence encoded by a nucleotide sequence having at least 70% identity to the nucleotide sequence of SEQ ID NO: 636; or
         (55αA2-e) a protein comprising an amino acid sequence encoded by a nucleic acid that hybridizes under stringent conditions with a nucleic acid having a nucleotide sequence complementary to the nucleotide sequence of SEQ ID NO: 636; or
      (55αB2) a protein comprising at least one amino acid sequence selected from the group consisting of SEQ ID NOs: 638-649.
[2] A composition for diagnosing an allergy to a soybean, comprising, as an antigen, at least one of proteins as defined above in any of (1α) to (55α) of [1].
[3] A method for providing an indicator for diagnosing an allergy to a soybean in a subject, the method comprising the steps of:
   (i) contacting a sample obtained from the subject with an antigen, wherein the sample is a solution comprising an Ig antibody;
   (ii) detecting binding between the IgE antibody present in the sample from the subject and the antigen; and
   (iii) when the binding between the IgE antibody in the subject and the antigen is detected, an indicator of the fact that the subject is allergic to a soybean is provided;
      wherein the antigen is at least one of proteins as defined above in any of (1α) to (55α) of [1].
[4] A pharmaceutical composition comprising at least one of proteins as defined above in any of (1α) to (55α) of [1].
[5] The pharmaceutical composition as set forth in [4], wherein the pharmaceutical composition is intended for the treatment of an allergy to a soybean.
[6] A soybean or processed products of soybean in which an antigen is eliminated or reduced, wherein the antigen is at least one of proteins as defined above in any of (1α) to (55α) of [1].
[7] A method for producing processed products of soybean in which an antigen is eliminated or reduced, the method comprising the step of confirming that the antigen is eliminated or reduced in a production process of the processed products, wherein the antigen is at least one of proteins as defined above in any of (1α) to (55α) of [1].
[8] A tester for determining the presence or absence of a soybean antigen in an object of interest, comprising an antibody that binds to at least one of proteins as defined above in any of (1α) to (55α) of [1].
[9] A soybean-derived antigen which is at least one of proteins as defined above in any of (1α) to (55α) of [1] and is causative of an allergy to soybean.
   The present inventors also succeeded in finding epitopes as to the novel antigens.
   As mentioned above, interaction between IgE antibodies from allergic patients and particular antigens elicit allergic reactions. These IgE antibodies recognize and bind to epitopes that are particular amino acid sequences in allergen components. Since the epitopes have a relatively short amino acid sequence, the IgE antibodies are capable of binding to different allergen components if the same amino acid sequence is present in the different allergen components. When different allergen components have a common epitope so that IgE antibodies from allergic patients bind to both of them, the allergens have cross-reactivity (or also called cross-antigenicity). Thus, the epitopes defined in the present invention enable diagnosis or treatment of an allergy including cross-reactivity, and detection of a plurality of allergen components comprising the epitopes, etc.
   The present invention has been completed based on this finding. Thus, in another embodiment, the present invention can be as defined below.
[10] A polypeptide specifically binding to an IgE antibody from an allergic patient, the polypeptide being any one of the following:
   (1β) a polypeptide comprising at least one amino acid sequence selected from the group consisting of SEQ ID NOs: 650-660;
   (2β) a polypeptide comprising at least one amino acid sequence selected from the group consisting of SEQ ID NOs: 661-692;
   (4β) a polypeptide comprising at least one amino acid sequence selected from the group consisting of SEQ ID NOs: 717-731;
   (5β) a polypeptide comprising at least one amino acid sequence selected from the group consisting of SEQ ID NOs: 732-738;
   (6β) a polypeptide comprising at least one amino acid sequence selected from the group consisting of SEQ ID NOs: 739 and 740;
   (7β) a polypeptide comprising at least one amino acid sequence selected from the group consisting of SEQ ID NOs: 741-744;
   (9β) a polypeptide comprising at least one amino acid sequence selected from the group consisting of SEQ ID NOs: 745-749;
   (11β) a polypeptide comprising at least one amino acid sequence selected from the group consisting of SEQ ID NOs: 751-754;
   (12β) a polypeptide comprising the amino acid sequence of SEQ ID NO: 755;
   (13β) a polypeptide comprising at least one amino acid sequence selected from the group consisting of SEQ ID NOs: 756-758;
   (14β) a polypeptide comprising at least one amino acid sequence selected from the group consisting of SEQ ID NOs: 759-764;
   (15β) a polypeptide comprising at least one amino acid sequence selected from the group consisting of SEQ ID NOs: 765 and 766;
   (17β) a polypeptide comprising the amino acid sequence of SEQ ID NO: 767;
   (18β) a polypeptide comprising at least one amino acid sequence selected from the group consisting of SEQ ID NOs: 750 and 768-775;
   (19β) a polypeptide comprising at least one amino acid sequence selected from the group consisting of SEQ ID NOs: 776-787;
   (25β) a polypeptide comprising at least one amino acid sequence selected from the group consisting of SEQ ID NOs: 814 and 815;
   (26β) a polypeptide comprising at least one amino acid sequence selected from the group consisting of SEQ ID NOs: 816-819;
   (27β) a polypeptide comprising the amino acid sequence of SEQ ID NO: 820;
   (28β) a polypeptide comprising at least one amino acid sequence selected from the group consisting of SEQ ID NOs: 821-835;
   (29β) a polypeptide comprising at least one amino acid sequence selected from the group consisting of SEQ ID NOs: 836-839;
   (34β) a polypeptide comprising at least one amino acid sequence selected from the group consisting of SEQ ID NOs: 840-846;
   (35β) a polypeptide comprising at least one amino acid sequence selected from the group consisting of SEQ ID NOs: 847-856;
   (36β) a polypeptide comprising at least one amino acid sequence selected from the group consisting of SEQ ID NOs: 857-861;
   (38β) a polypeptide comprising at least one amino acid sequence selected from the group consisting of SEQ ID NOs: 862-892;
   (39β) a polypeptide comprising at least one amino acid sequence selected from the group consisting of SEQ ID NOs: 893-895;
   (40β) a polypeptide comprising at least one amino acid sequence selected from the group consisting of SEQ ID NOs: 896-902;
   (41β) a polypeptide comprising the amino acid sequence of SEQ ID NO: 903;
   (42β) a polypeptide comprising at least one amino acid sequence selected from the group consisting of SEQ ID NOs: 904-906;
   (45β) a polypeptide comprising at least one amino acid sequence selected from the group consisting of SEQ ID NOs: 910-912;
   (46β) a polypeptide comprising at least one amino acid sequence selected from the group consisting of SEQ ID NOs: 913-915;
   (47β) a polypeptide comprising at least one amino acid sequence selected from the group consisting of SEQ ID NOs: 916-918;
   (48β) a polypeptide comprising at least one amino acid sequence selected from the group consisting of SEQ ID NOs: 919-922;
   (50β) a polypeptide comprising at least one amino acid sequence selected from the group consisting of SEQ ID NOs: 924-927;
   (51β) a polypeptide comprising at least one amino acid sequence selected from the group consisting of SEQ ID NOs: 928-930;
   (52β) a polypeptide comprising at least one amino acid sequence selected from the group consisting of SEQ ID NOs: 931-938;
   (53β) a polypeptide comprising the amino acid sequence of SEQ ID NO: 939;
   (54β) a polypeptide comprising at least one amino acid sequence selected from the group consisting of SEQ ID NOs: 940-943; and
   (55β) a polypeptide comprising at least one amino acid sequence selected from the group consisting of SEQ ID NOs: 944-952.
[11] A polypeptide specifically binding to an IgE antibody from an allergic patient, the polypeptide being any one of the following:
   (3β) a polypeptide comprising at least one amino acid sequence selected from the group consisting of SEQ ID NOs: 693-716;
   (20β) a polypeptide comprising at least one amino acid sequence selected from the group consisting of SEQ ID NOs: 788-792;
   (21β) a polypeptide comprising at least one amino acid sequence selected from the group consisting of SEQ ID NOs: 793-802;
   (22β) a polypeptide comprising at least one amino acid sequence selected from the group consisting of SEQ ID NOs: 803-808;
   (23β) a polypeptide comprising at least one amino acid sequence selected from the group consisting of SEQ ID NOs: 809-812;
   (24β) a polypeptide comprising the amino acid sequence of SEQ ID NO: 813;
   (44β) a polypeptide comprising at least one amino acid sequence selected from the group consisting of SEQ ID NOs: 907-909;
   (49β) a polypeptide comprising the amino acid sequence of SEQ ID NO: 923;
   (57β) a polypeptide comprising at least one amino acid sequence selected from the group consisting of SEQ ID NOs: 953-955;
   (58β) a polypeptide comprising at least one amino acid sequence selected from the group consisting of SEQ ID NOs: 956-960;
   (59β) a polypeptide comprising at least one amino acid sequence selected from the group consisting of SEQ ID NOs: 961 and 962; and
   (60β) a polypeptide comprising the amino acid sequence of SEQ ID NO: 963.
[12] The polypeptide according to the above [10] or [11], wherein the number of amino acid residues is 500 or less.
[13] A kit for diagnosing an allergy, comprising at least one of polypeptides according to any one of the above [10] to [12].
[14] A composition for diagnosing an allergy, the composition comprising at least one of polypeptides according to any one of the above [10] to [12] as an antigen.
[15] A method for providing an indicator for diagnosing an allergy in a subject, the method comprising the steps of:
   (i) contacting a sample obtained from the subject with an antigen, wherein the sample is a solution comprising an Ig antibody;
   (ii) detecting binding between the IgE antibody present in the sample obtained from the subject and the antigen; and
   (iii) when the binding between the IgE antibody in the subject and the antigen is detected, an indicator of the fact that the subject is allergic is provided;
      wherein the antigen is at least one of polypeptides according to any one of the above [10] to [12].
[16] A pharmaceutical composition comprising at least one of polypeptides according to any one of the above [10] to [12].
[17] The pharmaceutical composition according to the above [16], wherein the pharmaceutical composition is intended for the treatment of an allergy.
[18] A tester for determining the presence or absence of an antigen in an object of interest, the tester comprising an antibody that binds to at least one of polypeptides according to any one of the above [10] to [12].
[19] A food raw material or an edible processed product in which an antigen is eliminated or reduced, or a polypeptide is cleaved or removed, wherein the antigen is at least one of polypeptides according to any one of the above [10] to [12].
[20] A method for producing an edible processed product in which an antigen is eliminated or reduced, or a polypeptide is cleaved or removed, the method comprising the step of confirming that the antigen is eliminated or reduced, or the polypeptide is cleaved or removed, in a production process of the processed product, wherein the antigen is at least one of polypeptides according to any one of the above [10] to [12].

### ADVANTAGEOUS EFFECTS OF INVENTION

The present invention can provide novel antigens of an allergy to soybean. Since the novel antigens (allergen components) that trigger a soybean allergy were identified according to this invention, this invention can provide highly sensitive methods and kits for diagnosing an allergy to soybean, pharmaceutical compositions comprising such an antigen, soybean or processed products of soybean in which such an antigen is eliminated or reduced.

The present invention can provide novel polypeptides comprising an epitope of an allergen. Use of the polypeptide of the present invention enables provision of highly sensitive methods and kits for diagnosing an allergy, comprising such a polypeptide, pharmaceutical compositions comprising such a polypeptide, food raw materials or edible processed products in which an antigen comprising such a polypeptide is eliminated or reduced, or such a polypeptide is cleaved or removed, and a method for producing the edible processed products.

### BRIEF DESCRIPTION OF DRAWINGS

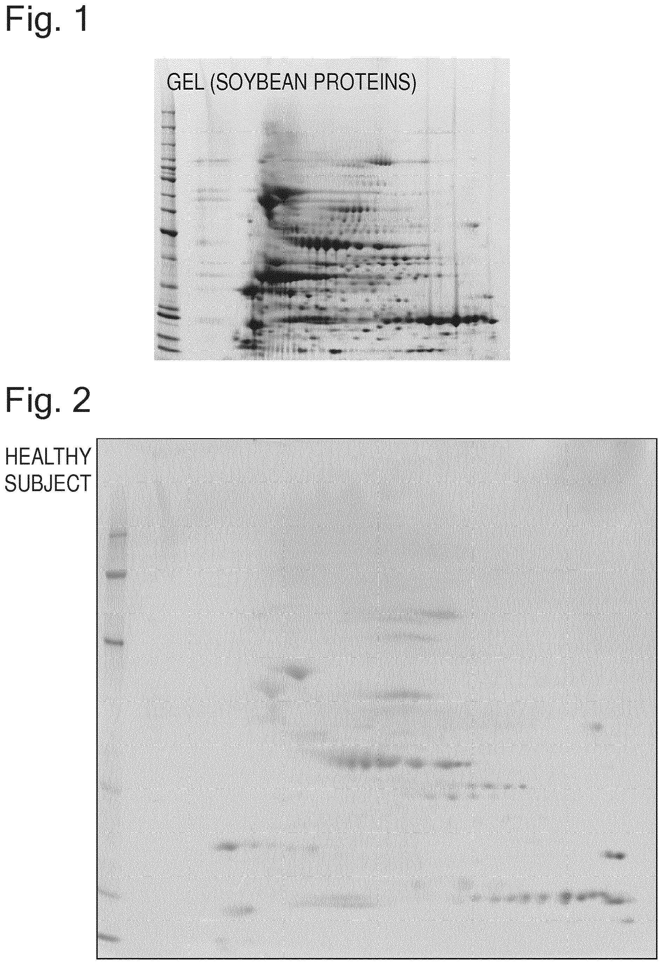

[Fig. 1] Fig. 1 is a photograph of a gel showing a protein electrophoretic pattern in two-dimensional electrophoresis of proteins contained in soybean. The bands at the left of the photograph are bands of molecular weight markers.
[Fig. 2] Fig. 2 is a photograph of an immunoblot of a two-dimensional electrophoretic pattern of proteins contained in soybean stained with serum of a healthy subject.

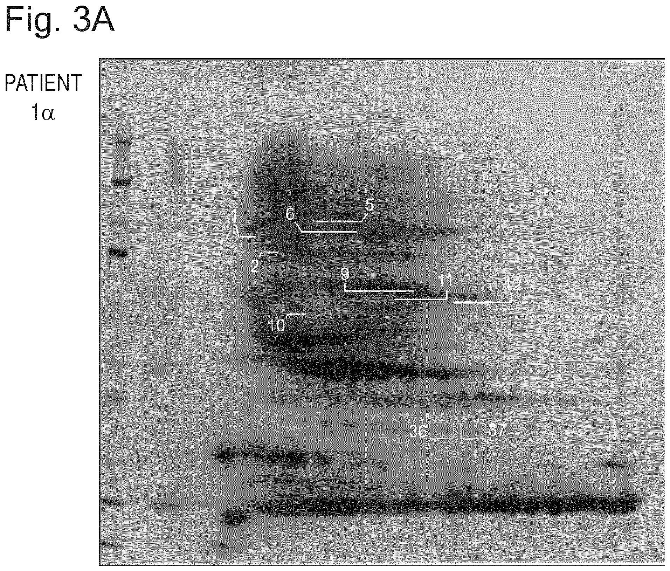

[Fig. 3A] Fig. 3A is a photograph of an immunoblot of a two-dimensional electrophoretic pattern of proteins contained in soybean stained with serum of Soybean-allergic patient 1α. The spots indicated with a white line or enclosed in a square are spot where the serum of the soybean-allergic patient specifically reacted. The numbers (n) indicated are the numbers of the spots and correspond to the spots indicated with "nα" herein.

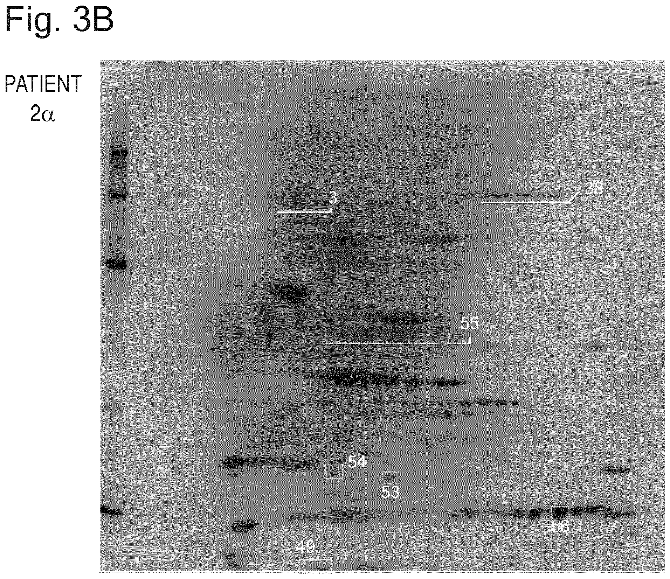

[Fig. 3B] Fig. 3B is a photograph of an immunoblot of a two-dimensional electrophoretic pattern of proteins contained in soybean stained with serum of Soybean-allergic patient 2α. The spots indicated with a white line or enclosed in a square are spot where the serum of the soybean-allergic patient specifically reacted. The numbers (n) indicated are the numbers of the spots and correspond to the spots indicated with "nα" herein.

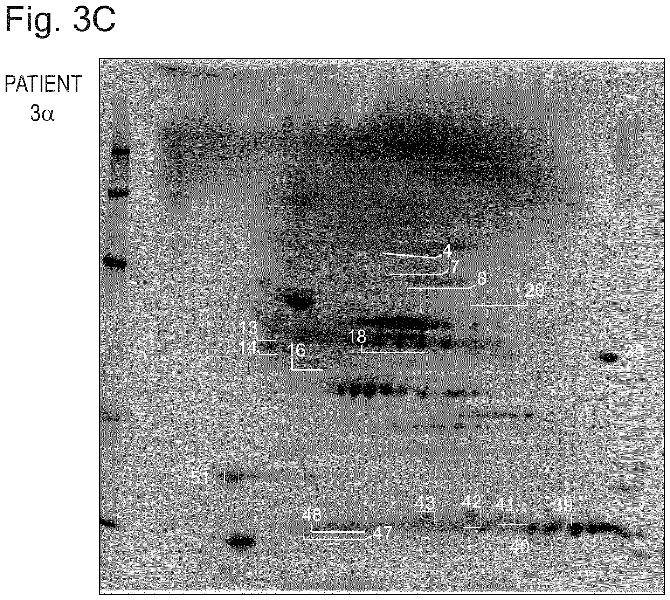

[Fig. 3C] Fig. 3C is a photograph of an immunoblot of a two-dimensional electrophoretic pattern of proteins contained in soybean stained with serum of Soybean-allergic patient 3α. The spots indicated with a white line or enclosed in a square are spot where the serum of the soybean-allergic patient specifically reacted. The numbers (n) indicated are the numbers of the spots and correspond to the spots indicated with "nα" herein.

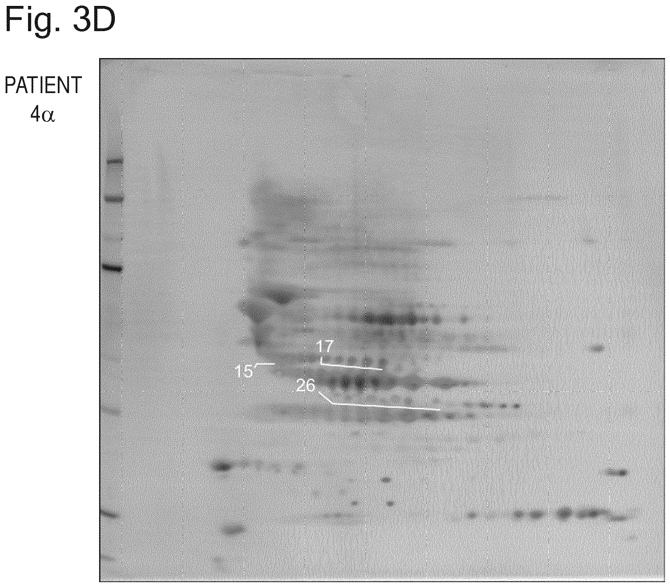

[Fig. 3D] Fig. 3D is a photograph of an immunoblot of a two-dimensional electrophoretic pattern of proteins contained in soybean stained with serum of Soybean-allergic patient 4α. The spots indicated with a while line are spots where the serum of the soybean-allergic patient specifically reacted. The numbers (n) indicated are the numbers of the spots and correspond to the spots indicated with "nα" herein.

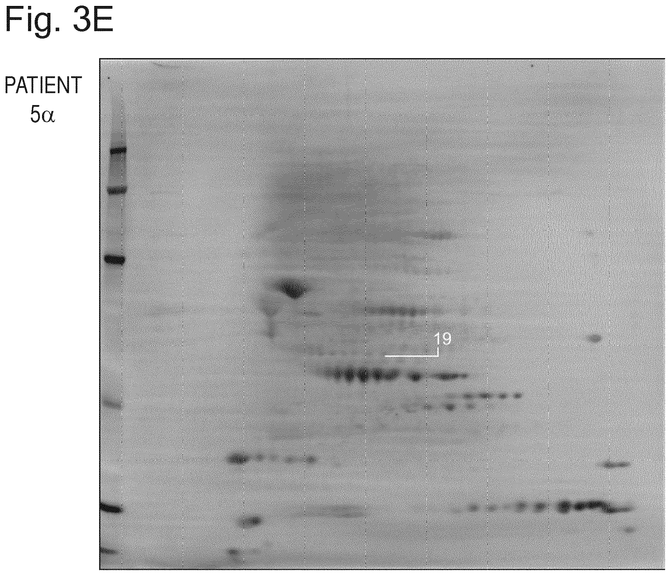

[Fig. 3E] Fig. 3E is a photograph of an immunoblot of a two-dimensional electrophoretic pattern of proteins contained in soybean stained with serum of Soybean-allergic patient 5α. The spots indicated with a while line are spots where the serum of the soybean-allergic patient specifically reacted. The numbers (n) indicated are the numbers of the spots and correspond to the spots indicated with "nα" herein.

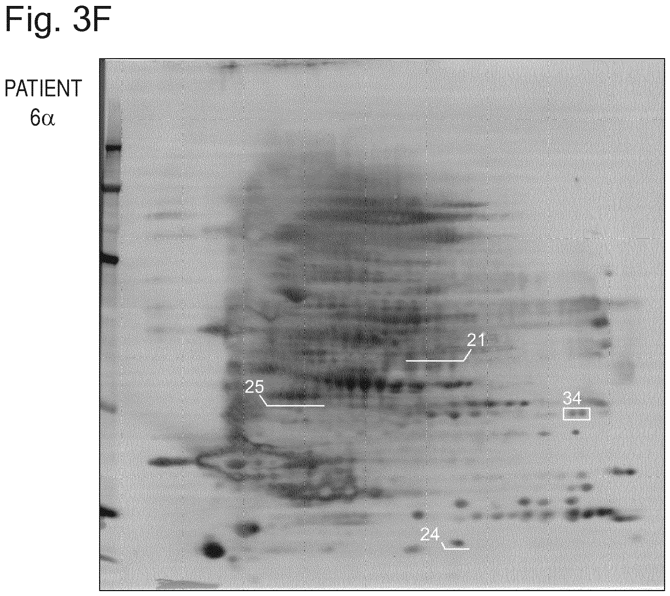

[Fig. 3F] Fig. 3F is a photograph of an immunoblot of a two-dimensional electrophoretic pattern of proteins contained in soybean stained with serum of Soybean-allergic patient 6α. The spots indicated with a white line or enclosed in a square are spot where the serum of the soybean-allergic patient specifically reacted. The numbers (n) indicated are the numbers of the spots and correspond to the spots indicated with "nα" herein.

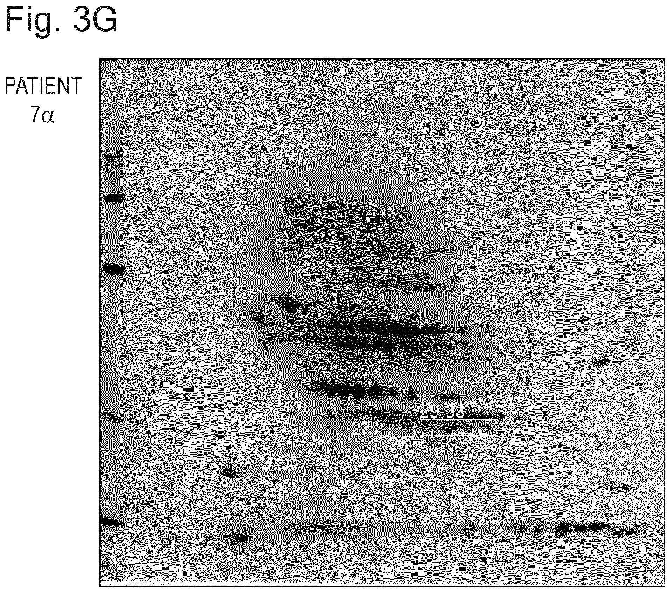

[Fig. 3G] Fig. 3G is a photograph of an immunoblot of a two-dimensional electrophoretic pattern of proteins contained in soybean stained with serum of Soybean-allergic patient 7α. The spots enclosed in a square are spots where the serum of the soybean-allergic patient specifically reacted. The numbers (n) indicated are the numbers of the spots and correspond to the spots indicated with "nα" herein.

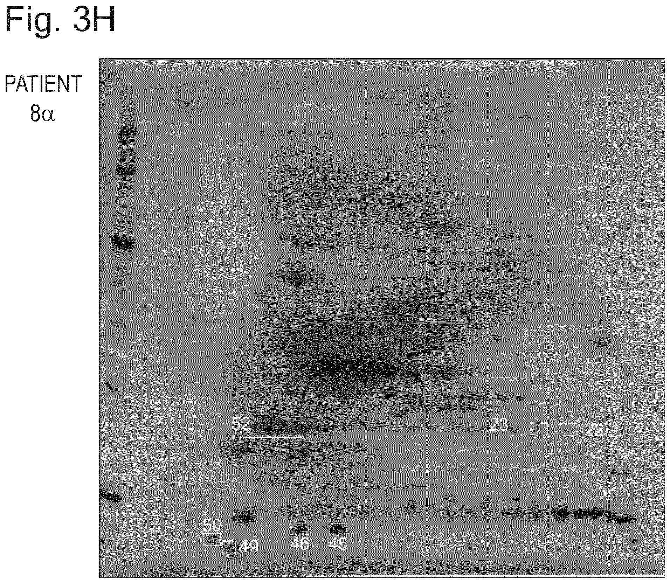

[Fig. 3H] Fig. 3H is a photograph of an immunoblot of a two-dimensional electrophoretic pattern of proteins contained in soybean stained with serum of Soybean-allergic patient 8α. The spots indicated with a white line or enclosed in a square are spot where the serum of the soybean-allergic patient specifically reacted. The numbers (n) indicated are the numbers of the spots and correspond to the spots indicated with "nα" herein.

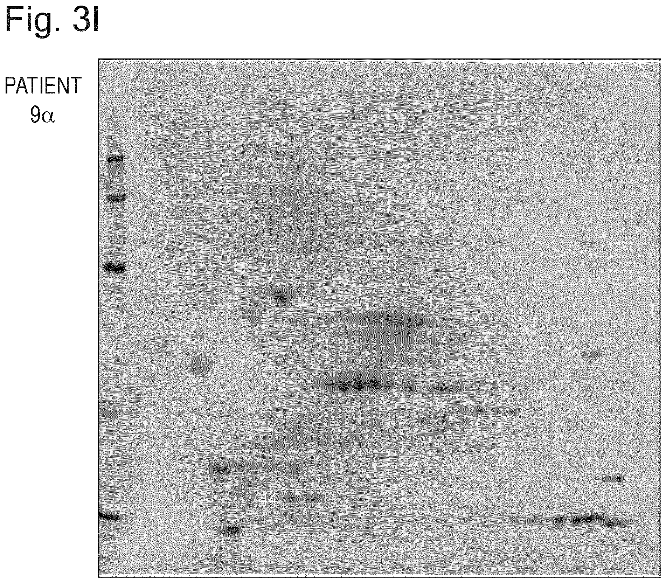

[Fig. 3I] Fig. 3I is a photograph of an immunoblot of a two-dimensional electrophoretic pattern of proteins contained in soybean stained with serum of Soybean-allergic patient 9α. The spots indicated with a white line or enclosed in a square are spot where the serum of the soybean-allergic patient specifically reacted. The numbers (n) indicated are the numbers of the spots and correspond to the spots indicated with "nα" herein.

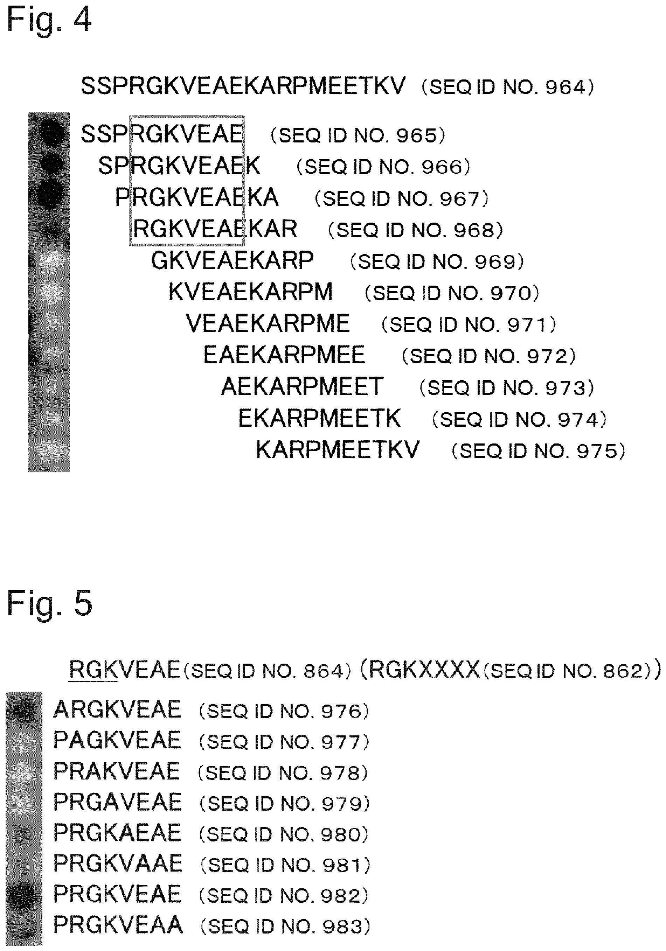

[Fig. 4] Fig. 4 is a diagram showing one example of results of determining peptides having the minimum number of amino acids functioning as an epitope by an overlapping technique.
[Fig. 5] Fig. 5 is a diagram showing one example of results of studying positions important for exertion of antigenicity as to the identified epitopes by an alanine scanning technique.

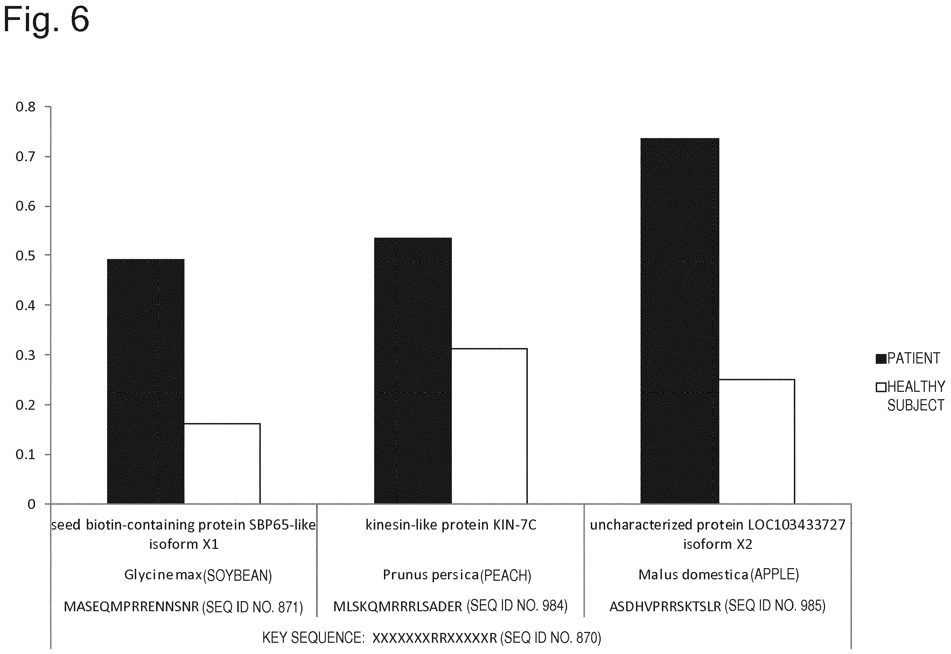

[Fig. 6] Fig. 6 is a graph showing results of studying cross-reactivity.

### DESCRIPTION OF EMBODIMENTS

The present invention will be described in detail below, but the present invention is not limited to them.

Unless otherwise defined herein, all scientific and technical terms used in relation to the present invention shall have meanings commonly understood by those skilled in the art.

Amino acid sequences herein are represented by one-letter amino acid symbols well known to those skilled in the art and the left end corresponds to the amino terminal and the right end corresponds to the carboxy terminal.

As referred to herein, the "allergy" refers to the state in which, when a certain antigen enters the body of a living individual sensitized to said antigen, the living individual shows a hypersensitive reaction detrimental to him/her. In blood and tissues of individuals with many food-allergic diseases, IgE antibodies specific to antigens are produced. IgE antibodies bind to mast cells or basophils. When an antigen specific to such an IgE antibody enters again the body of a patient with an allergic disease, said antigen combines with the IgE antibody bound to mast cells or basophils, and the IgE antibody crosslinks said antigen on the cell surface, resulting in physiological effects of IgE antibody-antigen interaction. Examples of such physiological effects include release of histamine, serotonin, heparin, eosinophil chemotactic factors, leucotrienes, or the like. These released substances provoke an allergic reaction resulting from the combination of an IgE antibody with particular antigens. Such allergic reactions caused by particular antigens occur through the aforementioned pathway.

In the present invention, the allergy of interest is not particularly limited as long as it is an allergy to an allergen comprising an epitope to be used. Such an allergy may include allergies to plants of the family *Malvaceae,* the family *Rubiaceae,* the family *Brassicaceae,* the family *Poaceae,* the family *Cucurbitaceae,* the family *Compositae,* the family *Asparagaceae,* the family *Juglandaceae,* the family *Rhamnaceae,* the family *Pedaliaceae,* the family *Umbelliferae,* the family *Euphorbiaceae,* the family *Solanaceae,* the family *Bromeliaceae,* the family *Caricaceae,* the family *Rosaceae,* the family *Amaranthaceae,* the family *Vitaceae,* the family *Leguminosae,* the family *Lythraceae* and/or the family *Betulaceae.* Examples of the plant of the family *Malvaceae* include nalta jute (Jew's mallow, scientific name: *Corchorus olitorius*) and cacao (scientific name: *Theobroma cacao*)*.* Examples of the plant of the family *Rubiaceae* include Robusta coffee (scientific name: *Coffea canephora*)*.* Examples of the plant of the family *Brassicaceae* include Qing geng cai (scientific name: *Brassica rapa*), radish (scientific name: *Raphanus sativus*), and rapeseed (scientific name: *Brassica napus*). Examples of the plant of the family *Poaceae* include great millet (scientific name: *Sorghum bicolor*), corn (scientific name: *Zea mays*), and bread wheat (scientific name: *Triticum aestivum*). Examples of the plant of the family *Cucurbitaceae* include melon (scientific name: *Cucumis melo*), bitter melon (bitter gourd, scientific name: *Momordica charantia*)*,* and cucumber (scientific name: *Cucumis sativus*). Examples of the plant of the family *Compositae* include sunflower (scientific name: *Helianthus annuus*)*.* Examples of the plant of the family *Asparagaceae* include asparagus (scientific name: *Asparagus officinalis*). Examples of the plant of the family *Juglandaceae* include Persian walnut (scientific name: *Juglans regia*)*.* Examples of the plant of the family *Rhamnaceae* include Chinese date (scientific name: *Ziziphus jujuba*)*.* Examples of the plant of the family *Pedaliaceae* include sesame (scientific name: *Sesamum indicum*)*.* Examples of the plant of the family *Umbelliferae* include carrot (scientific name: *Daucus carota* subsp. *Sativus*). Examples of the plant of the family *Euphorbiaceae* include cassava (scientific name: *Manihot esculenta*)*.* Examples of the plant of the family *Solanaceae* include red pepper (scientific name: *Capsicum annuum*) and tomato (scientific name: *Solanum lycopersicum*)*.* Examples of the plant of the family *Bromeliaceae* include pineapple (scientific name: *Ananas comosus*)*.* Examples of the plant of the family *Caricaceae* include papaya (scientific name: *Carica papaya*). Examples of the plant of the family *Rosaceae* include apple (scientific name: *Malus domestica*)*,* wild cherry (scientific name: *Prunus avium),* and *peach* (scientific name: *Prunus persica*)*.* Examples of the plant of the family *Amaranthaceae* include quinua (scientific name: *Chenopodium quinoa*)*,* spinach (scientific name: *Spinacia oleracea*), and beet (scientific name: *Beta vulgaris* subsp. *vulgaris*)*.* Examples of the plant of the family *Vitaceae* include common grape (scientific name: *Vitis vinifera*). Examples of the plant of the family *Leguminosae* include pigeon pea (scientific name: *Cajanus cajan*)*,* adzuki bean (scientific name: *Vigna angularis*)*,* wild soybean (scientific name: *Glycine soja*)*,* and soybean (scientific name: *Glycine max*). Examples of the plant of the family *Lythraceae* include pomegranate (scientific name: *Punica granatum*)*.* Examples of the plant of the family *Betulaceae* include white birch (scientific name: *Betula platyphylla*)*.* The allergy can produce an allergic reaction upon contact with an antigen or consumption of the antigen. Here, the contact refers to touch to an object and, particularly, as for the human body, refers to attachment to the skin, the mucosa (eyes, lips, etc.) or the like. The consumption refers to incorporation into the body and refers to incorporation by inhalation or through an oral route. In general, allergic reactions caused by consumption of foods are particularly referred to as food allergies. In a preferred mode, the allergy may be a food allergy.

As referred to herein, the "allergy to soybean" refers to the state in which an individual has an allergic reaction caused by proteins, etc. present in soybean which act as an antigen.

As referred to herein, the "antigen" refers to a substance that provokes an allergic reaction, and is also referred to as an "allergen component". The antigen is preferably a protein.

As referred to herein, the "protein" refers to a molecule having a structure in which naturally occurring amino acids are joined together by peptide bond. The number of amino acids present in a protein is not particularly limited. As referred to herein, the term "polypeptide" also means a molecule having a structure in which naturally occurring amino acids are joined together by peptide bond. The number of amino acids present in a polypeptide is not particularly limited. The "polypeptide" conceptually includes the "protein". Also, polypeptides having about 2 to 50 amino acids joined together by peptide bond are in some cases called "peptides", especially. In the case where amino acids can form different enantiomers, the amino acids are understood to form an L-enantiomer, unless otherwise indicated. The amino acid sequences of proteins, polypeptides, or peptides as used herein are represented by one-letter symbols of amino acids in accordance with standard usage and the notational convention commonly used in the art. The leftward direction represents the amino-terminal direction, and the rightward direction represents the carboxy-terminal direction. In the one-letter symbols of amino acids, X can be any substance having an amino group and a carboxyl group that can bind to amino acids at both ends, and particularly represents that any of 20 types of naturally occurring amino acids are acceptable.

### Identification of antigens

Proteins contained in soybean were analyzed by the aforementioned technique to identify causative antigens of an allergy to soybean. To be specific, soybean proteins were subjected to two-dimensional electrophoresis under the conditions described below.

The electrophoresis in the first dimension was isoelectric focusing, which was performed using isoelectric focusing gels with a gel-strip length of 5 to 10 cm and a gel pH range of 3 to 10. The pH gradient of the gels in the direction of electrophoresis was as follows: when the total gel-strip length is taken as 1, the gel-strip length up to pH 5 is taken as "a", the gel-strip length from pH 5 to 7 is taken as "b", and the gel-strip length above pH 7 is taken as "c", "a" is in the range of 0.15 to 0.3, "b" is in the range of 0.4 to 0.7, and "c" is in the range of 0.15 to 0.3. More specifically, the isoelectric focusing was performed using the IPG gels, Immobiline Drystrip (pH3-10NL), produced by GE Healthcare Bio-Sciences Corporation (hereinafter abbreviated as "GE"). The electrophoresis system used was IPGphor produced by GE. The maximum current of the electrophoresis system was limited to 75 µA per gel strip. The voltage program adopted to perform the first-dimensional isoelectric focusing was as follows: (1) a constant voltage step was performed at a constant voltage of 300 V until the volt-hours reached 750 Vhr (the current variation width during electrophoresis for 30 minutes before the end of this step was 5 µA); (2) the voltage was increased gradually to 1000 V for 300 Vhr; (3) the voltage was further increased gradually to 5000 V for 4500 Vhr; and then (4) the voltage was held at a constant voltage of 5000 V until the total Vhr reached 12000.

The electrophoresis in the second dimension was SDS-PAGE, which was performed using polyacrylamide gels whose gel concentration at the distal end in the direction of electrophoresis was set to 3 to 6% and whose gel concentration at the proximal end was set to a higher value than that at the distal end. More specifically, the SDS-PAGE was performed using NuPAGE 4-12% Bris-Tris Gels (IPG well, Mini, 1 mm) produced by Life Technologies. The electrophoresis system used was XCell SureLock Mini-Cell produced by Life Technologies. The electrophoresis was run at a constant voltage of 200 V for about 45 minutes using an electrophoresis buffer composed of 50 mM MOPS, 50 mM Tris base, 0.1% (w/v) SDS and 1mM EDTA.

As a result, the following spots in a two-dimensional electrophoresis gel run under the conditions described above for proteins in soybean have been revealed to exhibit specific binding to IgE antibodies from soybean-allergic patients.
Spot 1α: Molecular weight 70 to 120 kDa, pI 2.5 to 6.5
Spot 2α: Molecular weight 50 to 120 kDa, pI 2.5 to 6.5
Spot 4α: Molecular weight 70 to 120 kDa, pI 3.5 to 7.5
Spot 5α: Molecular weight 70 to 170 kDa, pI 3.5 to 7.5
Spot 6α: Molecular weight 70 to 120 kDa, pI 3.5 to 7.5
Spot 7α: Molecular weight 50 to 120 kDa, pI 3.5 to 7.5
Spot 8α: Molecular weight 50 to 120 kDa, pI 3.5 to 8.5
Spot 9α: Molecular weight 50 to 120 kDa, pI 3.5 to 8.5
Spot 10α: Molecular weight 30 to 90 kDa, pI 3.5 to 7.5
Spot 11α: Molecular weight 30 to 90 kDa, pI 3.5 to 8.5
Spot 12α: Molecular weight 30 to 90 kDa, pI 3.5 to 8.5
Spot 13α: Molecular weight 30 to 90 kDa, pI 3.5 to 7.5
Spot 14α: Molecular weight 30 to 90 kDa, pI 2.5 to 6.5
Spot 15α, 16α: Molecular weight 20 to 90 kDa, pI 3.5 to 7.5
Spot 17α: Molecular weight 20 to 90 kDa, pI 3.5 to 7.5
Spot 18α: Molecular weight 30 to 90 kDa, pI 3.5 to 8.5
Spot 19α: Molecular weight 30 to 90 kDa, pI 3.5 to 8.5
Spot 25α: Molecular weight 20 to 90 kDa, pI 3.5 to 7.5
Spot 26α: Molecular weight 20 to 90 kDa, pI 3.5 to 7.5
Spot 27α: Molecular weight 20 to 90 kDa, pI 3.5 to 7.5
Spot 28α: Molecular weight 10 to 70 kDa, pI 3.5 to 7.5
Spot 29α, 30α, 31α, 32α, 33α: Molecular weight 10 to 70 kDa, pI 3.5 to 8.5
Spot 34α: Molecular weight 10 to 70 kDa, pI 5.5 to 10.5
Spot 35α: Molecular weight 20 to 90 kDa, pI 5.5 to 10.5
Spot 36α, 37α: Molecular weight 10 to 70 kDa, pI 3.5 to 8.5
Spot 38α: Molecular weight 70 to 170 kDa, pI 5.5 to 10.5
Spot 39α: Molecular weight 5 to 60 kDa, pI 5.5 to 10.5
Spot 40α, 56α: Molecular weight 5 to 60 kDa, pI 5.5 to 10.5
Spot 41α: Molecular weight 5 to 60 kDa, pI 5.5 to 10.5
Spot 42α, 43α: Molecular weight 5 to 60 kDa, pI 5.5 to 10.5
Spot 45α: Molecular weight 2 to 50 kDa, pI 3.5 to 7.5
Spot 46α: Molecular weight 2 to 50 kDa, pI 3.5 to 7.5
Spot 47α: Molecular weight 5 to 50 kDa, pI 3.5 to 7.5
Spot 48α: Molecular weight 2 to 50 kDa, pI 3.5 to 8.5
Spot 50α: Molecular weight 2 to 50 kDa, pI 2.5 to 6.5
Spot 51α: Molecular weight 5 to 60 kDa, pI 2.5 to 6.5
Spot 52α: Molecular weight 10 to 70 kDa, pI 3.5 to 7.5
Spot 53α: Molecular weight 5 to 60 kDa, pI 3.5 to 8.5
Spot 54α: Molecular weight 5 to 60 kDa, pI 3.5 to 8.5
Spot 55α: Molecular weight 30 to 90 kDa, pI 5.5 to 10.5

### Antigen

### (1α) Antigen in spot 1α

As the result of sequence identification of the antigen in spot 1α by mass spectroscopy, the amino acid sequences of SEQ ID NOs: 7-104 were detected.

Also, the mass spectroscopic data obtained for spot 1α on a mass spectrometer was analyzed by comparing the data against the NCBI protein data, and as a result, endoplasmin homolog isoform X2 (amino acid sequence: SEQ ID NO: 70, encoding nucleotide sequence: SEQ ID NO: 69) was identified as a protein comprising any of SEQ ID NOs: 71 to 87 and endoplasmin homolog isoform X2 (amino acid sequence: SEQ ID NO: 89, encoding nucleotide sequence: SEQ ID NO: 88) was identified as a protein comprising any of SEQ ID NOs: 90 to 104.

Accordingly, the antigen in spot 1α in the present invention can be any of proteins selected from the group consisting of the proteins as defined below in (1αA1-a) to (1αA1-e), (1αB1), (1αA2-a) to (1αA2-e), and (1αB2):
(1αA1-a) a protein comprising an amino acid sequence with deletion, substitution, insertion or addition of one or several amino acids in SEQ ID NO: 70;
(1αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 70;
(1αA1-c) a protein comprising an amino acid sequence encoded by a nucleotide sequence with deletion, substitution, insertion or addition of one or several nucleotides in SEQ ID NO: 69;
(1αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 69;
(1αA1-e) a protein comprising an amino acid sequence encoded by a nucleic acid that hybridizes under stringent conditions with a nucleic acid having a nucleotide sequence complementary to the nucleotide sequence of SEQ ID NO: 69;
(1αB1) a protein comprising at least one amino acid sequence selected from the group consisting of SEQ ID NOs: 71-87, preferably a protein comprising at least 2, 3, 4, 5, 6, 7, 8, 9, 10, 11, 12, 13, 14, 15, or 16 or all sequences of the amino acid sequences. As referred to above, the amino acid sequence of any of SEQ ID NOs: 71 to 87 may be an amino acid sequence derived therefrom by deletion, substitution, insertion or addition of one or several amino acids;
(1αA2-a) a protein comprising an amino acid sequence with deletion, substitution, insertion or addition of one or several amino acids in SEQ ID NO: 89;
(1αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 89;
(laA2-c) a protein comprising an amino acid sequence encoded by a nucleotide sequence with deletion, substitution, insertion or addition of one or several nucleotides in SEQ ID NO: 88;
(1αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 88;
(1αA2-e) a protein comprising an amino acid sequence encoded by a nucleic acid that hybridizes under stringent conditions with a nucleic acid having a nucleotide sequence complementary to the nucleotide sequence of SEQ ID NO: 88;
(1αB2) a protein comprising at least one amino acid sequence selected from the group consisting of SEQ ID NOs: 90-104, preferably a protein comprising at least 2, 3, 4, 5, 6, 7, 8, 9, 10, 11, 12, 13, or 14 or all sequences of the amino acid sequences. As referred to above, the amino acid sequence of any of SEQ ID NOs: 71 to 87 may be an amino acid sequence derived therefrom by deletion, substitution, insertion or addition of one or several amino acids.

The protein selected from the group consisting of the proteins as defined above in (1αA1-a) to (1αA1-e), (1αB1), (1αA2-a) to (1αA2-e), and (1αB2) can be proteins that are found in a protein spot with a molecular weight of around 70 to 120 kDa, preferably around 80 to 110 kDa, and an isoelectric point of 2.5 to 6.5, preferably 3.0 to 6.0 on gels used in the two-dimensional electrophoresis performed under the conditions described above in the subsection titled "Identification of antigens".

Epitopes of the aforementioned antigens (1αA1-a) to (1αA1-e) and (1αB1) reside in amino acids 546-553 (SEQ ID NO: 652), amino acids 653-661 (SEQ ID NO: 655), amino acids 781-795 (SEQ ID NO: 657), amino acids 252-266 (SEQ ID NO: 658), amino acids 771-785 (SEQ ID NO: 659), and amino acids 493-502 (SEQ ID NO: 660) of SEQ ID NO: 70. When the antigen in spot 1α contains a variation in the amino acid sequence of SEQ ID NO: 70, amino acids at positions corresponding to the region of at least one of these epitopes may retain the sequence in SEQ ID NO: 70.

From the viewpoint of improving sensitivity (a degree to which a patient can be diagnosed as being positive) or specificity (a degree to which a healthy subject is not diagnosed as being positive), the antigen in spot 1α may be the following variant.

In the epitope having the sequence of SEQ ID NO: 652, sites of amino acids other than X in SEQ ID NO: 650 or 651 are important for binding to IgE antibodies from allergic patients. Thus, in another preferred mode, when the antigen in spot 1α contains a variation in the amino acids of SEQ ID NO: 70, amino acids 546-553 of SEQ ID NO: 70 in this antigen may have the amino acid sequence of SEQ ID NO: 650 or 651.

In the epitope having the sequence of SEQ ID NO: 655, sites of amino acids other than X in SEQ ID NO: 653 or 654 are important for binding to IgE antibodies from allergic patients. Thus, in another preferred mode, when the antigen in spot 1α contains a variation in the amino acid sequence of SEQ ID NO: 70, amino acids 653-661 of SEQ ID NO: 70 in this antigen may have the amino acid sequence of SEQ ID NO: 653 or 654.

In the epitope having the sequence of SEQ ID NO: 657, sites of amino acids other than X in SEQ ID NO: 656 are important for binding to IgE antibodies from allergic patients. Thus, in another preferred mode, when the antigen in spot 1α contains a variation in the amino acid sequence of SEQ ID NO: 70, amino acids 781-795 of SEQ ID NO: 70 in this antigen may have the amino acid sequence of SEQ ID NO: 656.

Preferably, a protein that is an antigen in spot 1α has activity equivalent to that of the protein having the amino acid sequence of SEQ ID NO: 70 or 89 (endoplasmin homolog isoform X2) or causative of an allergy to soybean.

### (2α) Antigen in spot 2α

As the result of sequence identification of the antigen in spot 2α by mass spectroscopy, the amino acid sequences of SEQ ID NOs: 107-153 were detected.

Also, the mass spectroscopic data obtained for spot 2α on a mass spectrometer was analyzed by comparing the data against the NCBI protein data, and as a result, heat shock cognate protein 80 (amino acid sequence: SEQ ID NO: 106, encoding nucleotide sequence: SEQ ID NO: 105) was identified as a protein comprising any of SEQ ID NOs: 107 to 120, heat shock protein 90-1 (amino acid sequence: SEQ ID NO: 122, encoding nucleotide sequence: SEQ ID NO: 121) was identified as a protein comprising any of SEQ ID NOs: 123 to 137, and hypothetical protein GLYMA_02G302500 was identified as a protein comprising any of SEQ ID NOs: 140 to 153.

Accordingly, the antigen in spot 1α in the present invention can be any of proteins selected from the group consisting of the proteins as defined below in (2αA1-a) to (2αA1-e), (2αB1), (2αA2-a) to (2αA2-e), (2αB2), (2αA3-a) to (2αA3-e), and (2αB3):
(2αA1-a) a protein comprising an amino acid sequence with deletion, substitution, insertion or addition of one or several amino acids in SEQ ID NO: 106;
(2αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 106;
(2αA1-c) a protein comprising an amino acid sequence encoded by a nucleotide sequence with deletion, substitution, insertion or addition of one or several nucleotides in SEQ ID NO: 105;
(2αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 105;
(2αA1-e) a protein comprising an amino acid sequence encoded by a nucleic acid that hybridizes under stringent conditions with a nucleic acid having a nucleotide sequence complementary to the nucleotide sequence of SEQ ID NO: 105;
(2αB1) a protein comprising at least one amino acid sequence selected from the group consisting of SEQ ID NOs: 107-120, preferably a protein comprising at least 2, 3, 4, 5, 6, 7, 8, 9, 10, 11, 12, or 13 or all sequences of the amino acid sequences. As referred to above, the amino acid sequence of any of SEQ ID NOs: 107 to 120 may be an amino acid sequence derived therefrom by deletion, substitution, insertion or addition of one or several amino acids;
(2αA2-a) a protein comprising an amino acid sequence with deletion, substitution, insertion or addition of one or several amino acids in SEQ ID NO: 122;
(2αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 122;
(2αA2-c) a protein comprising an amino acid sequence encoded by a nucleotide sequence with deletion, substitution, insertion or addition of one or several nucleotides in SEQ ID NO: 121;
(2αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 121;
(2αA2-e) a protein comprising an amino acid sequence encoded by a nucleic acid that hybridizes under stringent conditions with a nucleic acid having a nucleotide sequence complementary to the nucleotide sequence of SEQ ID NO: 121;
(2αB2) a protein comprising at least one amino acid sequence selected from the group consisting of SEQ ID NOs: 123-137, preferably a protein comprising at least 2, 3, 4, 5, 6, 7, 8, 9, 10, 11, 12, 13, or 14 or all sequences of the amino acid sequences. As referred to above, the amino acid sequence of any of SEQ ID NOs: 123 to 137 may be an amino acid sequence derived therefrom by deletion, substitution, insertion or addition of one or several amino acids.
(2αA3-a) a protein comprising an amino acid sequence with deletion, substitution, insertion or addition of one or several amino acids in SEQ ID NO: 139;
(2αA3-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 139;
(2αA3-c) a protein comprising an amino acid sequence encoded by a nucleotide sequence with deletion, substitution, insertion or addition of one or several nucleotides in SEQ ID NO: 138;
(2αA3-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 138;
(2αA3-e) a protein comprising an amino acid sequence encoded by a nucleic acid that hybridizes under stringent conditions with a nucleic acid having a nucleotide sequence complementary to the nucleotide sequence of SEQ ID NO: 138;
(2αB3) a protein comprising at least one amino acid sequence selected from the group consisting of SEQ ID NOs: 140-153, preferably a protein comprising at least 2, 3, 4, 5, 6, 7, 8, 9, 10, 11, 12, or 13 or all sequences of the amino acid sequences. As referred to above, the amino acid sequence of any of SEQ ID NOs: 140 to 153 may be an amino acid sequence derived therefrom by deletion, substitution, insertion or addition of one or several amino acids.

The protein selected from the group consisting of the proteins as defined above in (2αA1-a) to (2αA1-e), (2αB1), (2αA2-a) to (2αA2-e), (2αB2), (2αA3-a) to (2αA3-e), and (2αB3) can be proteins that are found in a protein spot with a molecular weight of around 50 to 120 kDa, preferably around 60 to 110 kDa, and an isoelectric point of 2.5 to 6.5, preferably 3.0 to 6.0 on gels used in the two-dimensional electrophoresis performed under the conditions described above in the subsection titled "Identification of antigens".

Epitopes of the aforementioned antigens (2αA1-a) to (2αA1-e) and (2αB1) reside in amino acids 42-50 (SEQ ID NO: 663), amino acids 51-65 (SEQ ID NO: 666), amino acids 69-73 (SEQ ID NO: 669), amino acids 221-235 (SEQ ID NO: 670), amino acids 351-365 (SEQ ID NO: 671), amino acids 361-375 (SEQ ID NO: 672), amino acids 411-425 (SEQ ID NO: 675), amino acids 551-565 (SEQ ID NO: 676), amino acids 671-685 (SEQ ID NO: 677), amino acids 681-695 (SEQ ID NO: 678), amino acids 41-55 (SEQ ID NO: 681), amino acids 451-465 (SEQ ID NO: 684), amino acids 271-285 (SEQ ID NO: 689), amino acids 51-59 (SEQ ID NO: 690), amino acids 65-72 (SEQ ID NO: 691),and amino acids 453-460 (SEQ ID NO: 692) of SEQ ID NO: 106. When the antigen in spot 2α contains a variation in the amino acid sequence of SEQ ID NO: 106, amino acids at positions corresponding to the region of at least one of these epitopes may retain the sequence in SEQ ID NO: 106.

From the viewpoint of improving sensitivity (a degree to which a patient can be diagnosed as being positive) or specificity (a degree to which a healthy subject is not diagnosed as being positive), the antigen in spot 2α may be the following variant.

In the epitope having the sequence of SEQ ID NO: 663, sites of amino acids other than X in SEQ ID NO: 661 or 662 are important for binding to IgE antibodies from allergic patients. Thus, in another preferred mode, when the antigen in spot 2α contains a variation in the amino acid sequence of SEQ ID NO: 106, amino acids 42-50 of SEQ ID NO: 106 in this antigen may have the amino acid sequence of SEQ ID NO: 661 or 662.

In the epitope having the sequence of SEQ ID NO: 666, sites of amino acids other than X in SEQ ID NO: 664 or 665 are important for binding to IgE antibodies from allergic patients. Thus, in another preferred mode, when the antigen in spot 2α contains a variation in the amino acid sequence of SEQ ID NO: 106, amino acids 51-65 of SEQ ID NO: 106 in this antigen may have the amino acid sequence of SEQ ID NO: 664 or 665.

In the epitope having the sequence of SEQ ID NO: 669, sites of amino acids other than X in SEQ ID NO: 667 or 668 are important for binding to IgE antibodies from allergic patients. Thus, in another preferred mode, when the antigen in spot 2α contains a variation in the amino acid sequence of SEQ ID NO: 106, amino acids 69-73 of SEQ ID NO: 106 in this antigen may have the amino acid sequence of SEQ ID NO: 667 or 668.

In the epitope having the sequence of SEQ ID NO: 675, sites of amino acids other than X in SEQ ID NO: 673 or 674 are important for binding to IgE antibodies from allergic patients. Thus, in another preferred mode, when the antigen in spot 2α contains a variation in the amino acid sequence of SEQ ID NO: 106, amino acids 411-425 of SEQ ID NO: 106 in this antigen may have the amino acid sequence of SEQ ID NO: 673 or 674.

In the epitope having the sequence of SEQ ID NO: 681, sites of amino acids other than X in SEQ ID NO: 679 or 680 are important for binding to IgE antibodies from allergic patients. Thus, in another preferred mode, when the antigen in spot 2α contains a variation in the amino acid sequence of SEQ ID NO: 106, amino acids 41-55 of SEQ ID NO: 106 in this antigen may have the amino acid sequence of SEQ ID NO: 679 or 680.

In the epitope having the sequence of SEQ ID NO: 684, sites of amino acids other than X in SEQ ID NO: 682 or 685 are important for binding to IgE antibodies from allergic patients. Thus, in another preferred mode, when the antigen in spot 2α contains a variation in the amino acid sequence of SEQ ID NO: 106, amino acids 451-465 of SEQ ID NO: 106 in this antigen may have the amino acid sequence of SEQ ID NO: 682 or 685.

In the epitope having the sequence of SEQ ID NO: 689, sites of amino acids other than X in SEQ ID NO: 688 are important for binding to IgE antibodies from allergic patients. Thus, in another preferred mode, when the antigen in spot 2α contains a variation in the amino acid sequence of SEQ ID NO: 106, amino acids 271-285 of SEQ ID NO: 106 in this antigen may have the amino acid sequence of SEQ ID NO: 688.

Preferably, a protein that is an antigen in spot 2α has activity equivalent to that of the protein having the amino acid sequence of SEQ ID NO: 106 (heat shock cognate protein 80), the protein having the amino acid sequence of SEQ ID NO: 122 (heat shock protein 90-1), or the protein having the amino acid sequence of SEQ ID NO: 139 (hypothetical protein GLYMA_02G302500), or causative of an allergy to soybean.

### (4α) Antigen in spot 4α

As the result of sequence identification of the antigen in spot 4α by mass spectroscopy, the amino acid sequences of SEQ ID NOs: 156 and 157 were detected.

Also, the mass spectroscopic data obtained for spot 4α on a mass spectrometer was analyzed by comparing the data against the NCBI protein data, and as a result, embryo-specific urease isoform X1 (amino acid sequence: SEQ ID NO: 155, encoding nucleotide sequence: SEQ ID NO: 154) was identified as a protein comprising SEQ ID NO: 156 or 157.

Accordingly, the antigens in spot 4α in the present invention can be any protein selected from the group consisting of the proteins as defined below in (4αA-a) to (4αA-e) and (4αB):
(4αA-a) a protein comprising an amino acid sequence with deletion, substitution, insertion or addition of one or several amino acids in SEQ ID NO: 155;
(4αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 155;
(4αA-c) a protein comprising an amino acid sequence encoded by a nucleotide sequence with deletion, substitution, insertion or addition of one or several nucleotides in SEQ ID NO: 154;
(4αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 154;
(4αA-e) a protein comprising an amino acid sequence encoded by a nucleic acid that hybridizes under stringent conditions with a nucleic acid having a nucleotide sequence complementary to the nucleotide sequence of SEQ ID NO: 154;
(4αB) a protein comprising at least one amino acid sequence selected from the group consisting of SEQ ID NOs: 156 and 157, preferably a protein comprising both the amino acid sequences. As referred to above, the amino acid sequence of any of SEQ ID NOs: 156 and 157 may be an amino acid sequence derived therefrom by deletion, substitution, insertion or addition of one or several amino acids.

The proteins selected from the group consisting of (4αA-a) to (4αA-e) and (4αB) as defined above can be proteins that are found in a protein spot with a molecular weight of around 70 to 120 kDa, preferably around 80 to 110 kDa and an isoelectric point of 3.5 to 7.5, preferably 4.0 to 7.0 on gels used in the two-dimensional electrophoresis performed under the conditions described above in the subsection titled "Identification of antigens".

Epitopes of the antigen in spot 4α reside in amino acids 121-130 (SEQ ID NO: 719), amino acids 289-295 (SEQ ID NO: 722), amino acids 293-302 (SEQ ID NO: 725), amino acids 501-515 (SEQ ID NO: 726), amino acids 571-585 (SEQ ID NO: 727), amino acids 591-605 (SEQ ID NO: 728), and amino acids 771-785 (SEQ ID NO: 731) of SEQ ID NO: 155. The antigen in spot 4α may retain the sequence in SEQ ID NO: 155 as to amino acids at positions corresponding to the region of at least one of these epitopes.

From the viewpoint of improving sensitivity (a degree to which a patient can be diagnosed as being positive) or specificity (a degree to which a healthy subject is not diagnosed as being positive), the antigen in spot 4α may be the following variant.

In the epitope having the sequence of SEQ ID NO: 719, sites of amino acids other than X in SEQ ID NO: 717 or 718 are important for binding to IgE antibodies from allergic patients. Thus, in another preferred mode, amino acids 121-130 of SEQ ID NO: 155 in the antigen in spot 4α may have the amino acid sequence of SEQ ID NO: 717 or 718.

In the epitope having the sequence of SEQ ID NO: 722, sites of amino acids other than X in SEQ ID NO: 720 or 721 are important for binding to IgE antibodies from allergic patients. Thus, in another preferred mode, amino acids 289-295 of SEQ ID NO: 155 in the antigen in spot 4α may have the amino acid sequence of SEQ ID NO: 720 or 721.

In the epitope having the sequence of SEQ ID NO: 725, sites of amino acids other than X in SEQ ID NO: 723 or 724 are important for binding to IgE antibodies from allergic patients. Thus, in another preferred mode, amino acids 293-302 of SEQ ID NO: 155 in the antigen in spot 4α may have the amino acid sequence of SEQ ID NO: 723 or 724.

In the epitope having the sequence of SEQ ID NO: 731, sites of amino acids other than X in SEQ ID NO: 729 or 730 are important for binding to IgE antibodies from allergic patients. Thus, in another preferred mode, amino acids 771-785 of SEQ ID NO: 155 in the antigen in spot 4α may have the amino acid sequence of SEQ ID NO: 729 or 730.

Preferably, a protein that is an antigen in spot 4α has activity equivalent to that of the protein having the amino acid sequence of SEQ ID NO: 155 (embryo-specific urease isoform X1) or causative of an allergy to soybean.

### (5α) Antigen in spot 5α

As the result of sequence identification of the antigen in spot 5α by mass spectroscopy, the amino acid sequences of SEQ ID NOs: 160 and 161 were detected.

Also, the mass spectroscopic data obtained for spot 5α on a mass spectrometer was analyzed by comparing the data against the NCBI protein data, and as a result, alpha-1,4 glucan phosphorylase L isozyme, chloroplastic/amyloplastic (amino acid sequence: SEQ ID NO: 159, encoding nucleotide sequence: SEQ ID NO: 158) was identified as a protein comprising SEQ ID NO: 160 or 161.

Accordingly, the antigens in spot 5α in the present invention can be any protein selected from the group consisting of the proteins as defined below in (5αA-a) to (5αA-e) and (5αB):
(5αA-a) a protein comprising an amino acid sequence with deletion, substitution, insertion or addition of one or several amino acids in SEQ ID NO: 159;
(5αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 159;
(5αA-c) a protein comprising an amino acid sequence encoded by a nucleotide sequence with deletion, substitution, insertion or addition of one or several nucleotides in SEQ ID NO: 158;
(5αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 158;
(5αA-e) a protein comprising an amino acid sequence encoded by a nucleic acid that hybridizes under stringent conditions with a nucleic acid having a nucleotide sequence complementary to the nucleotide sequence of SEQ ID NO: 158;
(5αB) a protein comprising at least one amino acid sequence selected from the group consisting of SEQ ID NOs: 160 and 161, preferably a protein comprising both the amino acid sequences. As referred to above, the amino acid sequence of any of SEQ ID NOs: 160 and 161 may be an amino acid sequence derived therefrom by deletion, substitution, insertion or addition of one or several amino acids.

The proteins selected from the group consisting of (5αA-a) to (5αA-e) and (5αB) as defined above can be proteins that are found in a protein spot with a molecular weight of around 70 to 170 kDa, preferably around 80 to 160 kDa and an isoelectric point of 3.5 to 7.5, preferably 4.0 to 7.0 on gels used in the two-dimensional electrophoresis performed under the conditions described above in the subsection titled "Identification of antigens".

Epitopes of the antigen in spot 5α reside in amino acids 861-875 (SEQ ID NO: 734), amino acids 871-885 (SEQ ID NO: 735), amino acids 891-905 (SEQ ID NO: 736), amino acids 901-915 (SEQ ID NO: 737), and amino acids 951-965 (SEQ ID NO: 738) of SEQ ID NO: 159. The antigen in spot 5α may retain the sequence in SEQ ID NO: 159 as to amino acids at positions corresponding to the region of at least one of these epitopes.

From the viewpoint of improving sensitivity (a degree to which a patient can be diagnosed as being positive) or specificity (a degree to which a healthy subject is not diagnosed as being positive), the antigen in spot 5α may be the following variant.

In the epitope having the sequence of SEQ ID NO: 734, sites of amino acids other than X in SEQ ID NO: 732 or 733 are important for binding to IgE antibodies from allergic patients. Thus, in another preferred mode, amino acids 861-875 of SEQ ID NO: 159 in the antigen in spot 5α may have the amino acid sequence of SEQ ID NO: 732 or 733.

Preferably, a protein that is an antigen in spot 5α has activity equivalent to that of the protein having the amino acid sequence of SEQ ID NO: 159 (alpha-1,4 glucan phosphorylase L isozyme, chloroplastic/amyloplastic) or causative of an allergy to soybean.

### (6α) Antigen in spot 6α

As the result of sequence identification of the antigen in spot 6α by mass spectroscopy, the amino acid sequences of SEQ ID NOs: 164-172 were detected.

Also, the mass spectroscopic data obtained for spot 6α on a mass spectrometer was analyzed by comparing the data against the NCBI protein data, and as a result, aconitate hydratase 1 (amino acid sequence: SEQ ID NO: 163, encoding nucleotide sequence: SEQ ID NO: 162) was identified as a protein comprising any of SEQ ID NOs: 164 to 167 and aconitate hydratase 1 (amino acid sequence: SEQ ID NO: 169, encoding nucleotide sequence: SEQ ID NO: 168) was identified as a protein comprising any of SEQ ID NOs: 170 to 172.

Accordingly, the antigen in spot 6α in the present invention can be any of proteins selected from the group consisting of the proteins as defined below in (6αA1-a) to (6αA1-e), (6αB1), (6αA2-a) to (6αA2-e), and (6αB2):
(6αA1-a) a protein comprising an amino acid sequence with deletion, substitution, insertion or addition of one or several amino acids in SEQ ID NO: 163;
(6αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 163;
(6αA1-c) a protein comprising an amino acid sequence encoded by a nucleotide sequence with deletion, substitution, insertion or addition of one or several nucleotides in SEQ ID NO: 162;
(6αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 162;
(6αA1-e) a protein comprising an amino acid sequence encoded by a nucleic acid that hybridizes under stringent conditions with a nucleic acid having a nucleotide sequence complementary to the nucleotide sequence of SEQ ID NO: 162;
(6αB1) a protein comprising at least one amino acid sequence selected from the group consisting of SEQ ID NOs: 164-167, preferably a protein comprising at least 2, 3, or all sequences of the amino acid sequences. As referred to above, the amino acid sequence of any of SEQ ID NOs: 164 to 167 may be an amino acid sequence derived therefrom by deletion, substitution, insertion or addition of one or several amino acids;
(6αA2-a) a protein comprising an amino acid sequence with deletion, substitution, insertion or addition of one or several amino acids in SEQ ID NO: 169;
(6αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 169;
(6αA2-c) a protein comprising an amino acid sequence encoded by a nucleotide sequence with deletion, substitution, insertion or addition of one or several nucleotides in SEQ ID NO: 168;
(6αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 168;
(6αA2-e) a protein comprising an amino acid sequence encoded by a nucleic acid that hybridizes under stringent conditions with a nucleic acid having a nucleotide sequence complementary to the nucleotide sequence of SEQ ID NO: 168;
(6αB2) a protein comprising at least one amino acid sequence selected from the group consisting of SEQ ID NOs: 170-172, preferably a protein comprising at least 2, or all sequences of the amino acid sequences. As referred to above, the amino acid sequence of any of SEQ ID NOs: 170 to 172 may be an amino acid sequence derived therefrom by deletion, substitution, insertion or addition of one or several amino acids.

The protein selected from the group consisting of the proteins as defined above in (6αA1-a) to (6αA1-e), (6αB1), (6αA2-a) to (6αA2-e), and (6αB2) can be proteins that are found in a protein spot with a molecular weight of around 70 to 120 kDa, preferably around 80 to 110 kDa, and an isoelectric point of 3.5 to 7.5, preferably 4.0 to 7.0 on gels used in the two-dimensional electrophoresis performed under the conditions described above in the subsection titled "Identification of antigens".

Epitopes of the aforementioned antigens (6αA1-a) to (6αA1-e) and (6αB1) reside in amino acids 761-775 (SEQ ID NO: 739), amino acids 831-845 (SEQ ID NO: 740) of SEQ ID NO: 163. When the antigen in spot 6α contains a variation in the amino acid sequence of SEQ ID NO: 163, amino acids at positions corresponding to the region of at least one of these epitopes may retain the sequence in SEQ ID NO: 163.

Preferably, a protein that is an antigen in spot 6α has activity equivalent to that of the protein having the amino acid sequence of SEQ ID NO: 163 or 169 (aconitate hydratase 1) or causative of an allergy to soybean.

### (7α) Antigen in spot 7α

As the result of sequence identification of the antigen in spot 7α by mass spectroscopy, the amino acid sequences of SEQ ID NOs: 175-187 were detected.

Also, the mass spectroscopic data obtained for spot 7α on a mass spectrometer was analyzed by comparing the data against the NCBI protein data, and as a result, methionine synthase (amino acid sequence: SEQ ID NO: 174, encoding nucleotide sequence: SEQ ID NO: 173) was identified as a protein comprising any of SEQ ID NOs: 175 to 187.

Accordingly, the antigens in spot 7α in the present invention can be any protein selected from the group consisting of the proteins as defined below in (7αA-a) to (7αA-e) and (7αB):
(7αA-a) a protein comprising an amino acid sequence with deletion, substitution, insertion or addition of one or several amino acids in SEQ ID NO: 174;
(7αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 174;
(7αA-c) a protein comprising an amino acid sequence encoded by a nucleotide sequence with deletion, substitution, insertion or addition of one or several nucleotides in SEQ ID NO: 173;
(7αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 173;
(7αA-e) a protein comprising an amino acid sequence encoded by a nucleic acid that hybridizes under stringent conditions with a nucleic acid having a nucleotide sequence complementary to the nucleotide sequence of SEQ ID NO: 173;
(7αB) a protein comprising at least one amino acid sequence selected from the group consisting of SEQ ID NOs: 175-187, preferably a protein comprising at least 2, 3, 4, 5, 6, 7, 8, 9, 10, 11 or 12 or all sequences of the amino acid sequences. As referred to above, the amino acid sequence of any of SEQ ID NOs: 175-187 may be an amino acid sequence derived therefrom by deletion, substitution, insertion or addition of one or several amino acids.

The proteins selected from the group consisting of (7αA-a) to (7αA-e) and (7αB) as defined above can be proteins that are found in a protein spot with a molecular weight of around 50 to 120 kDa, preferably around 60 to 110 kDa and an isoelectric point of 3.5 to 7.5, preferably 4.0 to 7.0 on gels used in the two-dimensional electrophoresis performed under the conditions described above in the subsection titled "Identification of antigens".

Epitopes of the antigen in spot 7α reside in amino acids 471-485 (SEQ ID NO: 741), amino acids 581-595 (SEQ ID NO: 744) of SEQ ID NO: 174. The antigen in spot 7α may retain the sequence in SEQ ID NO: 174 as to amino acids at positions corresponding to the region of at least one of these epitopes.

From the viewpoint of improving sensitivity (a degree to which a patient can be diagnosed as being positive) or specificity (a degree to which a healthy subject is not diagnosed as being positive), the antigen in spot 7α may be the following variant.

In the epitope having the sequence of SEQ ID NO: 744, sites of amino acids other than X in SEQ ID NO: 742 or 743 are important for binding to IgE antibodies from allergic patients. Thus, in another preferred mode, amino acids 581-595 of SEQ ID NO: 174 in the antigen in spot 7α may have the amino acid sequence of SEQ ID NO: 742 or 743.

Preferably, a protein that is an antigen in spot 7α has activity equivalent to that of the protein having the amino acid sequence of SEQ ID NO: 174 (methionine synthase) or causative of an allergy to soybean.

### (8α) Antigen in spot 8α

As the result of sequence identification of the antigen in spot 8α by mass spectroscopy, the amino acid sequences of SEQ ID NOs: 190-199 were detected.

Also, the mass spectroscopic data obtained for spot 8α on a mass spectrometer was analyzed by comparing the data against the NCBI protein data, and as a result, transketolase, chloroplastic (amino acid sequence: SEQ ID NO: 189, encoding nucleotide sequence: SEQ ID NO: 188) was identified as a protein comprising any of SEQ ID NOs: 190 to 199.

Accordingly, the antigens in spot 8α in the present invention can be any protein selected from the group consisting of the proteins as defined below in (8αA-a) to (8αA-e) and (8αB):
(8αA-a) a protein comprising an amino acid sequence with deletion, substitution, insertion or addition of one or several amino acids in SEQ ID NO: 189;
(8αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 189;
(8αA-c) a protein comprising an amino acid sequence encoded by a nucleotide sequence with deletion, substitution, insertion or addition of one or several nucleotides in SEQ ID NO: 188;
(8αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 188;
(8αA-e) a protein comprising an amino acid sequence encoded by a nucleic acid that hybridizes under stringent conditions with a nucleic acid having a nucleotide sequence complementary to the nucleotide sequence of SEQ ID NO: 188;
(8αB) a protein comprising at least one amino acid sequence selected from the group consisting of SEQ ID NOs: 190-199, preferably a protein comprising at least 2, 3, 4, 5, 6, 7, 8 or 9 or all sequences of the amino acid sequences. As referred to above, the amino acid sequence of any of SEQ ID NOs: 190-199 may be an amino acid sequence derived therefrom by deletion, substitution, insertion or addition of one or several amino acids.

The proteins selected from the group consisting of (8αA-a) to (8αA-e) and (8αB) as defined above can be proteins that are found in a protein spot with a molecular weight of around 50 to 120 kDa, preferably around 60 to 110 kDa and an isoelectric point of 3.5 to 8.5, preferably 4.0 to 8.0 on gels used in the two-dimensional electrophoresis performed under the conditions described above in the subsection titled "Identification of antigens".

Preferably, a protein that is an antigen in spot 8α has activity equivalent to that of the protein having the amino acid sequence of SEQ ID NO: 189 (transketolase, chloroplastic) or causative of an allergy to soybean.

### (9α) Antigen in spot 9α

As the result of sequence identification of the antigen in spot 9α by mass spectroscopy, the amino acid sequence of SEQ ID NO: 202 was detected.

Also, the mass spectroscopic data obtained for spot 9α on a mass spectrometer was analyzed by comparing the data against the NCBI protein data, and as a result, lysine-tRNA ligase, cytoplasmic-like isoform X2 (amino acid sequence: SEQ ID NO: 201, encoding nucleotide sequence: SEQ ID NO: 200) was identified as a protein comprising SEQ ID NO: 202.

Accordingly, the antigens in spot 9α in the present invention can be any protein selected from the group consisting of the proteins as defined below in (9αA-a) to (9αA-e) and (9αB):
(9αA-a) a protein comprising an amino acid sequence with deletion, substitution, insertion or addition of one or several amino acids in SEQ ID NO: 201;
(9αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 201;
(9αA-c) a protein comprising an amino acid sequence encoded by a nucleotide sequence with deletion, substitution, insertion or addition of one or several nucleotides in SEQ ID NO: 200;
(9αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 200;
(9αA-e) a protein comprising an amino acid sequence encoded by a nucleic acid that hybridizes under stringent conditions with a nucleic acid having a nucleotide sequence complementary to the nucleotide sequence of SEQ ID NO: 200;
(9αB) a protein comprising an amino acid sequence of SEQ ID NO: 202. As referred to above, the amino acid sequence(s) of SEQ ID NO: 202 may be an amino acid sequence(s) derived therefrom by deletion, substitution, insertion or addition of one or several amino acids.

The proteins selected from the group consisting of (9αA-a) to (9αA-e) and (9αB) as defined above can be proteins that are found in a protein spot with a molecular weight of around 50 to 120 kDa, preferably around 60 to 110 kDa and an isoelectric point of 3.5 to 8.5, preferably 4.0 to 8.0 on gels used in the two-dimensional electrophoresis performed under the conditions described above in the subsection titled "Identification of antigens".

Epitopes of the antigen in spot 9α reside in amino acids 236-245 (SEQ ID NO: 747), amino acids 283-290 (SEQ ID NO: 749) of SEQ ID NO: 201. The antigen in spot 9α may retain the sequence in SEQ ID NO: 201 as to amino acids at positions corresponding to the region of at least one of these epitopes.

From the viewpoint of improving sensitivity (a degree to which a patient can be diagnosed as being positive) or specificity (a degree to which a healthy subject is not diagnosed as being positive), the antigen in spot 9α may be the following variant.

In the epitope having the sequence of SEQ ID NO: 747, sites of amino acids other than X in SEQ ID NO: 745 or 746 are important for binding to IgE antibodies from allergic patients. Thus, in another preferred mode, amino acids 236-245 of SEQ ID NO: 201 in the antigen in spot 9α may have the amino acid sequence of SEQ ID NO: 745 or 746.

In the epitope having the sequence of SEQ ID NO: 749, sites of amino acids other than X in SEQ ID NO: 748 are important for binding to IgE antibodies from allergic patients. Thus, in another preferred mode, amino acids 283-290 of SEQ ID NO: 201 in the antigen in spot 9α may have the amino acid sequence of SEQ ID NO: 748.

Preferably, a protein that is an antigen in spot 9α has activity equivalent to that of the protein having the amino acid sequence of SEQ ID NO: 201 (lysine-tRNA ligase, cytoplasmic-like isoform X2) or causative of an allergy to soybean.

### (10α) Antigen in spot 10α

As the result of sequence identification of the antigen in spot 10α by mass spectroscopy, the amino acid sequences of SEQ ID NOs: 205-231 were detected.

Also, the mass spectroscopic data obtained for spot 10α on a mass spectrometer was analyzed by comparing the data against the NCBI protein data, and as a result, chaperonin CPN60-2, mitochondrial (amino acid sequence: SEQ ID NO: 204, encoding nucleotide sequence: SEQ ID NO: 203) was identified as a protein comprising any of SEQ ID NOs: 205 to 218 and chaperonin CPN60-2, mitochondrial-like isoform X1 (amino acid sequence: SEQ ID NO: 220, encoding nucleotide sequence: SEQ ID NO: 219) was identified as a protein comprising any of SEQ ID NOs: 221 to 231.

Accordingly, the antigen in spot 10α in the present invention can be any of proteins selected from the group consisting of the proteins as defined below in (10αA1-a) to (10aA1-e), (10αB1), (10αA2-a) to (10αA2-e), and (10αB2):
(10αA1-a) a protein comprising an amino acid sequence with deletion, substitution, insertion or addition of one or several amino acids in SEQ ID NO: 204;
(10αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 204;
(10αA1-c) a protein comprising an amino acid sequence encoded by a nucleotide sequence with deletion, substitution, insertion or addition of one or several nucleotides in SEQ ID NO: 203;
(10αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 203;
(10αA1-e) a protein comprising an amino acid sequence encoded by a nucleic acid that hybridizes under stringent conditions with a nucleic acid having a nucleotide sequence complementary to the nucleotide sequence of SEQ ID NO: 203;
(10αB1) a protein comprising at least one amino acid sequence selected from the group consisting of SEQ ID NOs: 205-218, preferably a protein comprising at least 2, 3, 4, 5, 6, 7, 8, 9, 10, 11, 12, or 13 or all sequences of the amino acid sequences. As referred to above, the amino acid sequence of any of SEQ ID NOs: 205 to 218 may be an amino acid sequence derived therefrom by deletion, substitution, insertion or addition of one or several amino acids;
(10αA2-a) a protein comprising an amino acid sequence with deletion, substitution, insertion or addition of one or several amino acids in SEQ ID NO: 220;
(10αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 220;
(10αA2-c) a protein comprising an amino acid sequence encoded by a nucleotide sequence with deletion, substitution, insertion or addition of one or several nucleotides in SEQ ID NO: 219;
(10αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 219;
(10αA2-e) a protein comprising an amino acid sequence encoded by a nucleic acid that hybridizes under stringent conditions with a nucleic acid having a nucleotide sequence complementary to the nucleotide sequence of SEQ ID NO: 219;
(10αB2) a protein comprising at least one amino acid sequence selected from the group consisting of SEQ ID NOs: 221-231, preferably a protein comprising at least 2, 3, 4, 5, 6, 7, 8, 9, or 10, or all sequences of the amino acid sequences. As referred to above, the amino acid sequence of any of SEQ ID NOs: 221 to 231 may be an amino acid sequence derived therefrom by deletion, substitution, insertion or addition of one or several amino acids.

The protein selected from the group consisting of the proteins as defined above in (10αA1-a) to (10αA1-e), (10αB1), (10αA2-a) to (10αA2-e), and (10αB2) can be proteins that are found in a protein spot with a molecular weight of around 30 to 90 kDa, preferably around 40 to 80 kDa, and an isoelectric point of 3.5 to 7.5, preferably 4.0 to 7.0 on gels used in the two-dimensional electrophoresis performed under the conditions described above in the subsection titled "Identification of antigens".

Preferably, a protein that is an antigen in spot 10α has activity equivalent to that of the protein having the amino acid sequence of SEQ ID NO: 204 (haperonin CPN60-2, mitochondrial) or the protein having the amino acid sequence of SEQ ID NO: 220 (chaperonin CPN60-2, mitochondrial-like isoform X1) or causative of an allergy to soybean.

### (11α) Antigen in spot 11α

As the result of sequence identification of the antigen in spot 11α by mass spectroscopy, the amino acid sequences of SEQ ID NOs: 234-237 were detected.

Also, the mass spectroscopic data obtained for spot 11α on a mass spectrometer was analyzed by comparing the data against the NCBI protein data, and as a result, polyadenylate-binding protein 8 isoform X3 (amino acid sequence: SEQ ID NO: 233, encoding nucleotide sequence: SEQ ID NO: 232) was identified as a protein comprising any of SEQ ID NOs: 234 to 237.

Accordingly, the antigens in spot 11α in the present invention can be any protein selected from the group consisting of the proteins as defined below in (11αA-a) to (11αA-e) and (11αB):
(11αA-a) a protein comprising an amino acid sequence with deletion, substitution, insertion or addition of one or several amino acids in SEQ ID NO: 233;
(11αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 233;
(11αA-c) a protein comprising an amino acid sequence encoded by a nucleotide sequence with deletion, substitution, insertion or addition of one or several nucleotides in SEQ ID NO: 232;
(11αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 232;
(11αA-e) a protein comprising an amino acid sequence encoded by a nucleic acid that hybridizes under stringent conditions with a nucleic acid having a nucleotide sequence complementary to the nucleotide sequence of SEQ ID NO: 232;
(11αB) a protein comprising at least one amino acid sequence selected from the group consisting of SEQ ID NOs: 234-237, preferably a protein comprising at least 2 or 3 or all sequences of the amino acid sequences. As referred to above, the amino acid sequence of any of SEQ ID NOs: 234-237 may be an amino acid sequence derived therefrom by deletion, substitution, insertion or addition of one or several amino acids.

The proteins selected from the group consisting of (11αA-a) to (11αA-e) and (11αB) as defined above can be proteins that are found in a protein spot with a molecular weight of around 30 to 90 kDa, preferably around 40 to 80 kDa and an isoelectric point of 3.5 to 8.5, preferably 4.0 to 8.0 on gels used in the two-dimensional electrophoresis performed under the conditions described above in the subsection titled "Identification of antigens".

Epitopes of the antigen in spot 11α reside in amino acids 454-463 (SEQ ID NO: 753), amino acids 363-370 (SEQ ID NO: 754) of SEQ ID NO: 233. The antigen in spot 11α may retain the sequence in SEQ ID NO: 233 as to amino acids at positions corresponding to the region of at least one of these epitopes.

From the viewpoint of improving sensitivity (a degree to which a patient can be diagnosed as being positive) or specificity (a degree to which a healthy subject is not diagnosed as being positive), the antigen in spot 11α may be the following variant.

In the epitope having the sequence of SEQ ID NO: 753, sites of amino acids other than X in SEQ ID NO: 751 or 752 are important for binding to IgE antibodies from allergic patients. Thus, in another preferred mode, amino acids 454-463 of SEQ ID NO: 223 in the antigen in spot 11α may have the amino acid sequence of SEQ ID NO: 751 or 752.

Preferably, a protein that is an antigen in spot 11α has activity equivalent to that of the protein having the amino acid sequence of SEQ ID NO: 233 (polyadenylate-binding protein 8 isoform X3) or causative of an allergy to soybean.

### (12α) Antigen in spot 12α

As the result of sequence identification of the antigen in spot 12α by mass spectroscopy, the amino acid sequence of SEQ ID NO: 240 was detected.

Also, the mass spectroscopic data obtained for spot 12α on a mass spectrometer was analyzed by comparing the data against the NCBI protein data, and as a result, pyrophosphate-fructose 6-phosphate 1-phosphotransferase subunit alpha-like (amino acid sequence: SEQ ID NO: 239, encoding nucleotide sequence: SEQ ID NO: 238) was identified as a protein comprising SEQ ID NO: 240.

Accordingly, the antigens in spot 12α in the present invention can be any protein selected from the group consisting of the proteins as defined below in (12αA-a) to (12αA-e) and (12αB):
(12αA-a) a protein comprising an amino acid sequence with deletion, substitution, insertion or addition of one or several amino acids in SEQ ID NO: 239;
(12αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 239;
(12αA-c) a protein comprising an amino acid sequence encoded by a nucleotide sequence with deletion, substitution, insertion or addition of one or several nucleotides in SEQ ID NO: 238;
(12αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 238;
(12αA-e) a protein comprising an amino acid sequence encoded by a nucleic acid that hybridizes under stringent conditions with a nucleic acid having a nucleotide sequence complementary to the nucleotide sequence of SEQ ID NO: 238;
(12αB) a protein comprising an amino acid sequence of SEQ ID NO: 240. As referred to above, the amino acid sequence(s) of SEQ ID NO: 240 may be an amino acid sequence(s) derived therefrom by deletion, substitution, insertion or addition of one or several amino acids.

The proteins selected from the group consisting of (12αA-a) to (12αA-e) and (12αB) as defined above can be proteins that are found in a protein spot with a molecular weight of around 30 to 90 kDa, preferably around 40 to 80 kDa and an isoelectric point of 3.5 to 8.5, preferably 4.0 to 8.0 on gels used in the two-dimensional electrophoresis performed under the conditions described above in the subsection titled "Identification of antigens".

An epitope of the antigen in spot 12α resides in amino acids 551-565 (SEQ ID NO: 755) of SEQ ID NO: 239. The antigen in spot 11α may retain the sequence in SEQ ID NO: 239 as to amino acids at positions corresponding to the region of this epitope.

Preferably, a protein that is an antigen in spot 12α has activity equivalent to that of the protein having the amino acid sequence of SEQ ID NO: 239 (pyrophosphate-fructose 6-phosphate 1-phosphotransferase subunit alpha-like) or causative of an allergy to soybean.

### (13α) Antigen in spot 13α

As the result of sequence identification of the antigen in spot 13α by mass spectroscopy, the amino acid sequences of SEQ ID NOs: 243-278 were detected.

Also, the mass spectroscopic data obtained for spot 13α on a mass spectrometer was analyzed by comparing the data against the NCBI protein data, and as a result, protein disulfide isomerase-like protein precursor (amino acid sequence: SEQ ID NO: 242, encoding nucleotide sequence: SEQ ID NO: 241) was identified as a protein comprising any of SEQ ID NOs: 243 to 252 and protein disulfide-isomerase (amino acid sequence: SEQ ID NO: 254, encoding nucleotide sequence: SEQ ID NO: 253) was identified as a protein comprising any of SEQ ID NOs: 255 to 278.

Accordingly, the antigen in spot 13α in the present invention can be any of proteins selected from the group consisting of the proteins as defined below in (13αA1-a) to (13αA1-e), (13αB1), (13αA2-a) to (13αA2-e), and (13αB2):
(13αA1-a) a protein comprising an amino acid sequence with deletion, substitution, insertion or addition of one or several amino acids in SEQ ID NO: 242;
(13αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 242;
(13αA1-c) a protein comprising an amino acid sequence encoded by a nucleotide sequence with deletion, substitution, insertion or addition of one or several nucleotides in SEQ ID NO: 241;
(13αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 241;
(13αA1-e) a protein comprising an amino acid sequence encoded by a nucleic acid that hybridizes under stringent conditions with a nucleic acid having a nucleotide sequence complementary to the nucleotide sequence of SEQ ID NO: 241;
(13αB1) a protein comprising at least one amino acid sequence selected from the group consisting of SEQ ID NOs: 243-252, preferably a protein comprising at least 2, 3, 4, 5, 6, 7, 8, or 9, or all sequences of the amino acid sequences. As referred to above, the amino acid sequence of any of SEQ ID NOs: 205 to 218 may be an amino acid sequence derived therefrom by deletion, substitution, insertion or addition of one or several amino acids;
(13αA2-a) a protein comprising an amino acid sequence with deletion, substitution, insertion or addition of one or several amino acids in SEQ ID NO: 254;
(13αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 254;
(13αA2-c) a protein comprising an amino acid sequence encoded by a nucleotide sequence with deletion, substitution, insertion or addition of one or several nucleotides in SEQ ID NO: 253;
(13αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 253;
(13αA2-e) a protein comprising an amino acid sequence encoded by a nucleic acid that hybridizes under stringent conditions with a nucleic acid having a nucleotide sequence complementary to the nucleotide sequence of SEQ ID NO: 253;
(13αB2) a protein comprising at least one amino acid sequence selected from the group consisting of SEQ ID NOs: 255-278, preferably a protein comprising at least 2, 3, 4, 5, 6, 7, 8, 9, 10, 11, 12, 13, 14, 15, 16, 17, 18, 19, 20, 21, 22, or 23, or all sequences of the amino acid sequences. As referred to above, the amino acid sequence of any of SEQ ID NOs: 221 to 231 may be an amino acid sequence derived therefrom by deletion, substitution, insertion or addition of one or several amino acids.

The protein selected from the group consisting of the proteins as defined above in (13αA1-a) to (13αA1-e), (13αB1), (13αA2-a) to (13αA2-e), and (13αB2) can be proteins that are found in a protein spot with a molecular weight of around 30 to 90 kDa, preferably around 40 to 80 kDa, and an isoelectric point of 3.5 to 7.5, preferably 4.0 to 7.0 on gels used in the two-dimensional electrophoresis performed under the conditions described above in the subsection titled "Identification of antigens".

Epitopes of the aforementioned antigens (13αA1-a) to (13αA1-e) and (13αB1) reside in amino acids 471-485 (SEQ ID NO: 758) of SEQ ID NO: 242. When the antigen in spot 13α contains a variation in the amino acid sequence of SEQ ID NO: 242, this antigen may retain the sequence in SEQ ID NO: 242 as to amino acids at positions corresponding to the region of at least one of these epitopes.

From the viewpoint of improving sensitivity (a degree to which a patient can be diagnosed as being positive) or specificity (a degree to which a healthy subject is not diagnosed as being positive), the antigen in spot 13α may be the following variant.

In the epitope having the sequence of SEQ ID NO: 758, sites of amino acids other than X in SEQ ID NO: 756 or 757 are important for binding to IgE antibodies from allergic patients. Thus, in another preferred mode, when the antigen in spot 13α contains a variation in the amino acid sequence of SEQ ID NO: 242, amino acids 471-485 of SEQ ID NO: 242 in this antigen may have the amino acid sequence of SEQ ID NO: 756 or 757.

Preferably, a protein that is an antigen in spot 13α has activity equivalent to that of the protein having the amino acid sequence of SEQ ID NO: 242 (protein disulfide isomerase-like protein precursor) or the protein having the amino acid sequence of SEQ ID NO: 254 (protein disulfide-isomerase) or causative of an allergy to soybean.

### (14α) Antigen in spot 14α

As the result of sequence identification of the antigen in spot 14α by mass spectroscopy, the amino acid sequences of SEQ ID NOs: 281-289 were detected.

Also, the mass spectroscopic data obtained for spot 14α on a mass spectrometer was analyzed by comparing the data against the NCBI protein data, and as a result, V-type proton ATPase subunit B2 (amino acid sequence: SEQ ID NO: 280, encoding nucleotide sequence: SEQ ID NO: 279) was identified as a protein comprising any of SEQ ID NOs: 281 to 284 and V-type proton ATPase subunit B2 (amino acid sequence: SEQ ID NO: 286, encoding nucleotide sequence: SEQ ID NO: 285) was identified as a protein comprising any of SEQ ID NOs: 287 to 289.

Accordingly, the antigen in spot 14α in the present invention can be any of proteins selected from the group consisting of the proteins as defined below in (14αA1-a) to (14αA1-e), (14αB1), (14αA2-a) to (14αA2-e), and (14αB2):
(14αA1-a) a protein comprising an amino acid sequence with deletion, substitution, insertion or addition of one or several amino acids in SEQ ID NO: 280;
(14αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 280;
(14αA1-c) a protein comprising an amino acid sequence encoded by a nucleotide sequence with deletion, substitution, insertion or addition of one or several nucleotides in SEQ ID NO: 279;
(14αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 279;
(14αA1-e) a protein comprising an amino acid sequence encoded by a nucleic acid that hybridizes under stringent conditions with a nucleic acid having a nucleotide sequence complementary to the nucleotide sequence of SEQ ID NO: 279;
(14αB1) a protein comprising at least one amino acid sequence selected from the group consisting of SEQ ID NOs: 281-284, preferably a protein comprising at least 2, 3, or all sequences of the amino acid sequences. As referred to above, the amino acid sequence of any of SEQ ID NOs: 281 to 284 may be an amino acid sequence derived therefrom by deletion, substitution, insertion or addition of one or several amino acids;
(14αA2-a) a protein comprising an amino acid sequence with deletion, substitution, insertion or addition of one or several amino acids in SEQ ID NO: 286;
(14αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 286;
(14αA2-c) a protein comprising an amino acid sequence encoded by a nucleotide sequence with deletion, substitution, insertion or addition of one or several nucleotides in SEQ ID NO: 285;
(14αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 285;
(14αA2-e) a protein comprising an amino acid sequence encoded by a nucleic acid that hybridizes under stringent conditions with a nucleic acid having a nucleotide sequence complementary to the nucleotide sequence of SEQ ID NO: 285;
(14αB2) a protein comprising at least one amino acid sequence selected from the group consisting of SEQ ID NOs: 287-289, preferably a protein comprising at least 2, or all sequences of the amino acid sequences. As referred to above, the amino acid sequence of any of SEQ ID NOs: 287 to 289 may be an amino acid sequence derived therefrom by deletion, substitution, insertion or addition of one or several amino acids.

The protein selected from the group consisting of the proteins as defined above in (14αA1-a) to (14αA1-e), (14αB1), (14αA2-a) to (14αA2-e), and (14αB2) can be proteins that are found in a protein spot with a molecular weight of around 30 to 90 kDa, preferably around 40 to 80 kDa, and an isoelectric point of 2.5 to 6.5, preferably 3.0 to 6.0 on gels used in the two-dimensional electrophoresis performed under the conditions described above in the subsection titled "Identification of antigens".

Epitopes of the aforementioned antigens (14αA1-a) to (14αA1-e) and (14αB1) reside in amino acids 191-205 (SEQ ID NO: 761) and amino acids 391-405 (SEQ ID NO: 764) of SEQ ID NO: 280. When the antigen in spot 14α contains a variation in the amino acid sequence of SEQ ID NO: 280, this antigen may retain the sequence in SEQ ID NO: 280 as to amino acids at positions corresponding to the region of at least one of these epitopes.

From the viewpoint of improving sensitivity (a degree to which a patient can be diagnosed as being positive) or specificity (a degree to which a healthy subject is not diagnosed as being positive), the antigen in spot 14α may be the following variant.

In the epitope having the sequence of SEQ ID NO: 761, sites of amino acids other than X in SEQ ID NO: 759 or 760 are important for binding to IgE antibodies from allergic patients. Thus, in another preferred mode, when the antigen in spot 14α contains a variation in the amino acid sequence of SEQ ID NO: 280, amino acids 191-205 of SEQ ID NO: 280 in this antigen may each independently have the amino acid sequence of SEQ ID NO: 759 or 760.

In the epitope having the sequence of SEQ ID NO: 764, sites of amino acids other than X in SEQ ID NO: 762 or 763 are important for binding to IgE antibodies from allergic patients. Thus, in another preferred mode, when the antigen in spot 14α contains a variation in the amino acid sequence of SEQ ID NO: 280, amino acids 391-405 of SEQ ID NO: 280 in this antigen may each independently have the amino acid sequence of SEQ ID NO: 762 or 763.

Preferably, a protein that is an antigen in spot 14α has activity equivalent to that of the protein having the amino acid sequence of SEQ ID NO: 280 or 286 (V-type proton ATPase subunit B2) or causative of an allergy to soybean.

### (15α, 16α) Antigens in spots 15α, 16α

As the result of sequence identification of the antigens in spots 15α and 16α by mass spectroscopy, the amino acid sequences of SEQ ID NOs: 292-318 were detected.

Also, the mass spectroscopic data obtained for spots 15α, 16α on a mass spectrometer was analyzed by comparing the data against the NCBI protein data, and as a result, UTP-glucose-1-phosphate uridylyltransferase (amino acid sequence: SEQ ID NO: 291, encoding nucleotide sequence: SEQ ID NO: 290) was identified as a protein comprising any of SEQ ID NOs: 292 to 304 and the hypothetical protein GLYMA_02G241100 (amino acid sequence: SEQ ID NO: 306, encoding nucleotide sequence: SEQ ID NO: 305) was identified as a protein comprising any of SEQ ID NOs: 307 to 318.

Accordingly, the antigen in spot 15α, 16α in the present invention can be any of proteins selected from the group consisting of the proteins as defined below in (15, 16αA1-a) to (15, 16αA1-e), (15, 16αB1), (15, 16αA2-a) to (15, 16αA2-e), and (15, 16αB2):
(15, 16αA1-a) a protein comprising an amino acid sequence with deletion, substitution, insertion or addition of one or several amino acids in SEQ ID NO: 291;
(15, 16αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 291;
(15, 16αA1-c) a protein comprising an amino acid sequence encoded by a nucleotide sequence with deletion, substitution, insertion or addition of one or several nucleotides in SEQ ID NO: 290;
(15, 16αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 290;
(15, 16αA1-e) a protein comprising an amino acid sequence encoded by a nucleic acid that hybridizes under stringent conditions with a nucleic acid having a nucleotide sequence complementary to the nucleotide sequence of SEQ ID NO: 290;
(15, 16αB1) a protein comprising at least one amino acid sequence selected from the group consisting of SEQ ID NOs: 292-304, preferably a protein comprising at least 2, 3, 4, 5, 6, 7, 8, 9, 10, 11, or 12 or all sequences of the amino acid sequences. As referred to above, the amino acid sequence of any of SEQ ID NOs: 292 to 304 may be an amino acid sequence derived therefrom by deletion, substitution, insertion or addition of one or several amino acids;
(15, 16αA2-a) a protein comprising an amino acid sequence with deletion, substitution, insertion or addition of one or several amino acids in SEQ ID NO: 306;
(15, 16αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 306;
(15, 16αA2-c) a protein comprising an amino acid sequence encoded by a nucleotide sequence with deletion, substitution, insertion or addition of one or several nucleotides in SEQ ID NO: 305;
(15, 16αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 305;
(15, 16αA2-e) a protein comprising an amino acid sequence encoded by a nucleic acid that hybridizes under stringent conditions with a nucleic acid having a nucleotide sequence complementary to the nucleotide sequence of SEQ ID NO: 305;
(15, 16αB2) a protein comprising at least one amino acid sequence selected from the group consisting of SEQ ID NOs: 307-318, preferably a protein comprising at least 2, 3, 4, 5, 6, 7, 8, 9, 10, or 11 or all sequences of the amino acid sequences. As referred to above, the amino acid sequence of any of SEQ ID NOs: 307 to 318 may be an amino acid sequence derived therefrom by deletion, substitution, insertion or addition of one or several amino acids.

The protein selected from the group consisting of the proteins as defined above in (15, 16αA1-a) to (15, 16αA1-e), (15, 16αB1), (15, 16αA2-a) to (15, 16αA2-e), and (15, 16αB2) can be proteins that are found in a protein spot with a molecular weight of around 20 to 90 kDa, preferably around 30 to 80 kDa, and an isoelectric point of 3.5 to 7.5, preferably 4.0 to 7.0 on gels used in the two-dimensional electrophoresis performed under the conditions described above in the subsection titled "Identification of antigens".

Epitopes of the aforementioned antigens (15, 16αA1-a) to (15, 16αA1-e) and (15, 16αB1) reside in amino acids 211-225 (SEQ ID NO: 766) of SEQ ID NO: 291. When the antigen in spot 15α, 16α contains a variation in the amino acid sequence of SEQ ID NO: 291, this antigen may retain the sequence in SEQ ID NO: 291 as to amino acids at positions corresponding to the region of at least one of these epitopes.

From the viewpoint of improving sensitivity (a degree to which a patient can be diagnosed as being positive) or specificity (a degree to which a healthy subject is not diagnosed as being positive), the antigen in spot 15α, 16α may be the following variant.

In the epitope having the sequence of SEQ ID NO: 766, sites of amino acids other than X in SEQ ID NO: 765 are important for binding to IgE antibodies from allergic patients. Thus, in another preferred mode, when the antigen in spot 15α, 16α contains a variation in the amino acid sequence of SEQ ID NO: 291, amino acids 211-225 of SEQ ID NO: 291 in this antigen may have the amino acid sequence of SEQ ID NO: 765.

Preferably, a protein that is an antigen in spot 15α or 16α has activity equivalent to that of the protein having the amino acid sequence of SEQ ID NO: 291 (UTP-glucose-1-phosphate uridylyltransferase) or the protein having the amino acid sequence of SEQ ID NO: 306 (hypothetical protein GLYMA_02G241100) or causative of an allergy to soybean.

### (17α) Antigen in spot 17α

As the result of sequence identification of the antigen in spot 17α by mass spectroscopy, the amino acid sequences of SEQ ID NOs: 321-332 were detected.

Also, the mass spectroscopic data obtained for spot 17α on a mass spectrometer was analyzed by comparing the data against the NCBI protein data, and as a result, guanosine nucleotide diphosphate dissociation inhibitor 2 (amino acid sequence: SEQ ID NO: 320, encoding nucleotide sequence: SEQ ID NO: 319) was identified as a protein comprising any of SEQ ID NOs: 321 to 325 and rab GDP dissociation inhibitor alpha-like (amino acid sequence: SEQ ID NO: 327, encoding nucleotide sequence: SEQ ID NO: 326) was identified as a protein comprising any of SEQ ID NOs: 328 to 332.

Accordingly, the antigen in spot 17α in the present invention can be any of proteins selected from the group consisting of the proteins as defined below in (17αA1-a) to (17αA1-e), (17αB1), (17αA2-a) to (17αA2-e), and (17αB2):
(17αA1-a) a protein comprising an amino acid sequence with deletion, substitution, insertion or addition of one or several amino acids in SEQ ID NO: 320;
(17αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 320;
(17αA1-c) a protein comprising an amino acid sequence encoded by a nucleotide sequence with deletion, substitution, insertion or addition of one or several nucleotides in SEQ ID NO: 319;
(17αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 319;
(17αA1-e) a protein comprising an amino acid sequence encoded by a nucleic acid that hybridizes under stringent conditions with a nucleic acid having a nucleotide sequence complementary to the nucleotide sequence of SEQ ID NO: 319;
(17αB1) a protein comprising at least one amino acid sequence selected from the group consisting of SEQ ID NOs: 321-325, preferably a protein comprising at least 2, 3, or 4 or all sequences of the amino acid sequences. As referred to above, the amino acid sequence of any of SEQ ID NOs: 321 to 325 may be an amino acid sequence derived therefrom by deletion, substitution, insertion or addition of one or several amino acids.
(17αA2-a) a protein comprising an amino acid sequence with deletion, substitution, insertion or addition of one or several amino acids in SEQ ID NO: 327;
(17αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 327;
(17αA2-c) a protein comprising an amino acid sequence encoded by a nucleotide sequence with deletion, substitution, insertion or addition of one or several nucleotides in SEQ ID NO: 326;
(17αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 326;
(17αA2-e) a protein comprising an amino acid sequence encoded by a nucleic acid that hybridizes under stringent conditions with a nucleic acid having a nucleotide sequence complementary to the nucleotide sequence of SEQ ID NO: 326;
(17αB2) a protein comprising at least one amino acid sequence selected from the group consisting of SEQ ID NOs: 328-332, preferably a protein comprising at least 2, 3, or 4 or all sequences of the amino acid sequences. As referred to above, the amino acid sequence of any of SEQ ID NOs: 328 to 332 may be an amino acid sequence derived therefrom by deletion, substitution, insertion or addition of one or several amino acids;

The protein selected from the group consisting of the proteins as defined above in (17αA1-a) to (17αA1-e), (17αB1), (17αA2-a) to (17αA2-e), and (17αB2) can be proteins that are found in a protein spot with a molecular weight of around 20 to 90 kDa, preferably around 30 to 80 kDa, and an isoelectric point of 3.5 to 7.5, preferably 4.0 to 7.0 on gels used in the two-dimensional electrophoresis performed under the conditions described above in the subsection titled "Identification of antigens".

Epitopes of the aforementioned antigens (17αA1-a) to (17αA1-e) and (17αB1) reside in amino acids 171-185 (SEQ ID NO: 767) of SEQ ID NO: 320. When the antigen in spot 17α contains a variation in the amino acid sequence of SEQ ID NO: 320, this antigen may retain the sequence in SEQ ID NO: 320 as to amino acids at positions corresponding to the region of at least one of these epitopes.

Preferably, a protein that is an antigen in spot 17α has activity equivalent to that of the protein having the amino acid sequence of SEQ ID NO: 320 (guanosine nucleotide diphosphate dissociation inhibitor 2) or the protein having the amino acid sequence of SEQ ID NO: 327 (rab GDP dissociation inhibitor alpha-like) or causative of an allergy to soybean.

### (18α) Antigen in spot 18α

As the result of sequence identification of the antigen in spot 18α by mass spectroscopy, the amino acid sequences of SEQ ID NOs: 335-339 were detected.

Also, the mass spectroscopic data obtained for spot 18α on a mass spectrometer was analyzed by comparing the data against the NCBI protein data, and as a result, hypothetical protein GLYMA_10G028300 (amino acid sequence: SEQ ID NO: 334, encoding nucleotide sequence: SEQ ID NO: 333) was identified as a protein comprising any of SEQ ID NOs: 335 to 339.

Accordingly, the antigens in spot 18α in the present invention can be any protein selected from the group consisting of the proteins as defined below in (18αA-a) to (18αA-e) and (18αB):
(18αA-a) a protein comprising an amino acid sequence with deletion, substitution, insertion or addition of one or several amino acids in SEQ ID NO: 334;
(18αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 334;
(18αA-c) a protein comprising an amino acid sequence encoded by a nucleotide sequence with deletion, substitution, insertion or addition of one or several nucleotides in SEQ ID NO: 333;
(18αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 333;
(18αA-e) a protein comprising an amino acid sequence encoded by a nucleic acid that hybridizes under stringent conditions with a nucleic acid having a nucleotide sequence complementary to the nucleotide sequence of SEQ ID NO: 333;
(18αB) a protein comprising at least one amino acid sequence selected from the group consisting of SEQ ID NOs: 335-339, preferably a protein comprising at least 2, 3 or 4 or all sequences of the amino acid sequences. As referred to above, the amino acid sequence of any of SEQ ID NOs: 335-339 may be an amino acid sequence derived therefrom by deletion, substitution, insertion or addition of one or several amino acids.

The proteins selected from the group consisting of (18αA-a) to (18αA-e) and (18αB) as defined above can be proteins that are found in a protein spot with a molecular weight of around 30 to 90 kDa, preferably around 40 to 80 kDa and an isoelectric point of 3.5 to 8.5, preferably 4.0 to 8.0 on gels used in the two-dimensional electrophoresis performed under the conditions described above in the subsection titled "Identification of antigens".

Epitopes of the antigen in spot 18α reside in amino acids 52-60 (SEQ ID NO: 750), amino acids 81-95 (SEQ ID NO: 768), amino acids 101-115 (SEQ ID NO: 771), amino acids 281-295 (SEQ ID NO: 772), amino acids 491-500 (SEQ ID NO: 775) of SEQ ID NO: 334. The antigen in spot 18α may retain the sequence in SEQ ID NO: 334 as to amino acids at positions corresponding to the region of at least one of these epitopes.

From the viewpoint of improving sensitivity (a degree to which a patient can be diagnosed as being positive) or specificity (a degree to which a healthy subject is not diagnosed as being positive), the antigen in spot 18α may be the following variant.

In the epitope having the sequence of SEQ ID NO: 771, sites of amino acids other than X in SEQ ID NO: 769 or 770 are important for binding to IgE antibodies from allergic patients. Thus, in another preferred mode, amino acids 101-115 of SEQ ID NO: 334 in the antigen in spot 18α may have the amino acid sequence of SEQ ID NO: 769 or 770.

In the epitope having the sequence of SEQ ID NO: 775, sites of amino acids other than X in SEQ ID NO: 773 or 774 are important for binding to IgE antibodies from allergic patients. Thus, in another preferred mode, amino acids 491-500 of SEQ ID NO: 334 in the antigen in spot 18α may have the amino acid sequence of SEQ ID NO: 773 or 774.

Preferably, a protein that is an antigen in spot 18α has activity equivalent to that of the protein having the amino acid sequence of SEQ ID NO: 334 (hypothetical protein GLYMA_10G028300) or causative of an allergy to soybean.

### (19a) Antigen in spot 19α

As the result of sequence identification of the antigen in spot 19α by mass spectroscopy, the amino acid sequences of SEQ ID NOs: 342-346 were detected.

Also, the mass spectroscopic data obtained for spot 19α on a mass spectrometer was analyzed by comparing the data against the NCBI protein data, and as a result, sucrose binding protein homolog S-64 (amino acid sequence: SEQ ID NO: 341, encoding nucleotide sequence: SEQ ID NO: 340) was identified as a protein comprising any of SEQ ID NOs: 342 to 346.

Accordingly, the antigens in spot 19α in the present invention can be any protein selected from the group consisting of the proteins as defined below in (19αA-a) to (19αA-e) and (19αB):
(19αA-a) a protein comprising an amino acid sequence with deletion, substitution, insertion or addition of one or several amino acids in SEQ ID NO: 341;
(19αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 341;
(19αA-c) a protein comprising an amino acid sequence encoded by a nucleotide sequence with deletion, substitution, insertion or addition of one or several nucleotides in SEQ ID NO: 340;
(19αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 340;
(19αA-e) a protein comprising an amino acid sequence encoded by a nucleic acid that hybridizes under stringent conditions with a nucleic acid having a nucleotide sequence complementary to the nucleotide sequence of SEQ ID NO: 340;
(19αB) a protein comprising at least one amino acid sequence selected from the group consisting of SEQ ID NOs: 342-346, preferably a protein comprising at least 2, 3 or 4 or all sequences of the amino acid sequences. As referred to above, the amino acid sequence of any of SEQ ID NOs: 342-346 may be an amino acid sequence derived therefrom by deletion, substitution, insertion or addition of one or several amino acids.

The proteins selected from the group consisting of (19αA-a) to (19αA-e) and (19αB) as defined above can be proteins that are found in a protein spot with a molecular weight of around 30 to 90 kDa, preferably around 40 to 80 kDa and an isoelectric point of 3.5 to 8.5, preferably 4.0 to 8.0 on gels used in the two-dimensional electrophoresis performed under the conditions described above in the subsection titled "Identification of antigens".

Epitopes of the antigen in spot 19α reside in amino acids 191-205 (SEQ ID NO: 778), amino acids 475-489 (SEQ ID NO: 781), amino acids 81-95 (SEQ ID NO: 784), amino acids 436-450 (SEQ ID NO: 786), amino acids 21-30 (SEQ ID NO: 787) of SEQ ID NO: 341. The antigen in spot 19α may retain the sequence in SEQ ID NO: 341 as to amino acids at positions corresponding to the region of at least one of these epitopes.

From the viewpoint of improving sensitivity (a degree to which a patient can be diagnosed as being positive) or specificity (a degree to which a healthy subject is not diagnosed as being positive), the antigen in spot 19α may be the following variant.

In the epitope having the sequence of SEQ ID NO: 778, sites of amino acids other than X in SEQ ID NO: 776 or 778 are important for binding to IgE antibodies from allergic patients. Thus, in another preferred mode, amino acids 191-205 of SEQ ID NO: 341 in the antigen in spot 19α may have the amino acid sequence of SEQ ID NO: 776 or 778.

In the epitope having the sequence of SEQ ID NO: 781, sites of amino acids other than X in SEQ ID NO: 779 or 780 are important for binding to IgE antibodies from allergic patients. Thus, in another preferred mode, amino acids 475-489 of SEQ ID NO: 341 in the antigen in spot 19α may have the amino acid sequence of SEQ ID NO: 779 or 780.

In the epitope having the sequence of SEQ ID NO: 784, sites of amino acids other than X in SEQ ID NO: 782 or 783 are important for binding to IgE antibodies from allergic patients. Thus, in another preferred mode, amino acids 81-95 of SEQ ID NO: 341 in the antigen in spot 19α may have the amino acid sequence of SEQ ID NO: 782 or 783.

Preferably, a protein that is an antigen in spot 19α has activity equivalent to that of the protein having the amino acid sequence of SEQ ID NO: 341 (sucrose binding protein homolog S-64) or causative of an allergy to soybean.

### (25α) Antigen in spot 25α

As the result of sequence identification of the antigen in spot 25α by mass spectroscopy, the amino acid sequences of SEQ ID NOs: 349-365 were detected.

Also, the mass spectroscopic data obtained for spot 25α on a mass spectrometer was analyzed by comparing the data against the NCBI protein data, and as a result, succinyl-CoA ligase [ADP-forming] subunit beta, mitochondrial (amino acid sequence: SEQ ID NO: 348, encoding nucleotide sequence: SEQ ID NO: 347) was identified as a protein comprising any of SEQ ID NOs: 349 to 356 and succinyl-CoA ligase [ADP-forming] subunit beta, mitochondrial (amino acid sequence: SEQ ID NO: 358, encoding nucleotide sequence: SEQ ID NO: 357) was identified as a protein comprising any of SEQ ID NOs: 359 to 365.

Accordingly, the antigen in spot 25α in the present invention can be any of proteins selected from the group consisting of the proteins as defined below in (25αA1-a) to (25αA1-e), (25αB1), (25αA2-a) to (25αA2-e), and (25αB2):
(25αA1-a) a protein comprising an amino acid sequence with deletion, substitution, insertion or addition of one or several amino acids in SEQ ID NO: 348;
(25αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 348;
(25αA1-c) a protein comprising an amino acid sequence encoded by a nucleotide sequence with deletion, substitution, insertion or addition of one or several nucleotides in SEQ ID NO: 347;
(25αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 347;
(25αA1-e) a protein comprising an amino acid sequence encoded by a nucleic acid that hybridizes under stringent conditions with a nucleic acid having a nucleotide sequence complementary to the nucleotide sequence of SEQ ID NO: 347;
(25αB1) a protein comprising at least one amino acid sequence selected from the group consisting of SEQ ID NOs: 349-356, preferably a protein comprising at least 2, 3, 4, 5, 6, or 7 or all sequences of the amino acid sequences. As referred to above, the amino acid sequence of any of SEQ ID NOs: 349 to 356 may be an amino acid sequence derived therefrom by deletion, substitution, insertion or addition of one or several amino acids; (25αA2-a) a protein comprising an amino acid sequence with deletion, substitution, insertion or addition of one or several amino acids in SEQ ID NO: 358;
(25αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 358;
(25αA2-c) a protein comprising an amino acid sequence encoded by a nucleotide sequence with deletion, substitution, insertion or addition of one or several nucleotides in SEQ ID NO: 357;
(25αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 357;
(25αA2-e) a protein comprising an amino acid sequence encoded by a nucleic acid that hybridizes under stringent conditions with a nucleic acid having a nucleotide sequence complementary to the nucleotide sequence of SEQ ID NO: 357;
(25αB2) a protein comprising at least one amino acid sequence selected from the group consisting of SEQ ID NOs: 359-365, preferably a protein comprising at least 2, 3, 4, 5, or 6 or all sequences of the amino acid sequences. As referred to above, the amino acid sequence of any of SEQ ID NOs: 359 to 365 may be an amino acid sequence derived therefrom by deletion, substitution, insertion or addition of one or several amino acids.

The protein selected from the group consisting of the proteins as defined above in (25αA1-a) to (25αA1-e), (25αB1), (25αA2-a) to (25αA2-e), and (25αB2) can be proteins that are found in a protein spot with a molecular weight of around 20 to 90 kDa, preferably around 30 to 80 kDa, and an isoelectric point of 3.5 to 7.5, preferably 4.0 to 7.0 on gels used in the two-dimensional electrophoresis performed under the conditions described above in the subsection titled "Identification of antigens".

Epitopes of the aforementioned antigens (25αA1-a) to (25αA1-e) and (25αB1) reside in amino acids 241-255 (SEQ ID NO: 814) and amino acids 321-335 (SEQ ID NO: 815) of SEQ ID NO: 348. When the antigen in spot 25α contains a variation in the amino acid sequence of SEQ ID NO: 348, this antigen may retain the sequence in SEQ ID NO: 348 as to amino acids at positions corresponding to the region of at least one of these epitopes.

Preferably, a protein that is an antigen in spot 25α has activity equivalent to that of the protein having the amino acid sequence of SEQ ID NO: 348 or 358 (succinyl-CoA ligase [ADP-forming] subunit beta, mitochondrial) or causative of an allergy to soybean.

### (26α) Antigen in spot 26α

As the result of sequence identification of the antigen in spot 26α by mass spectroscopy, the amino acid sequences of SEQ ID NOs: 368-409 were detected.

Also, the mass spectroscopic data obtained for spot 26α on a mass spectrometer was analyzed by comparing the data against the NCBI protein data, and as a result, phosphoglycerate kinase, cytosolic (amino acid sequence: SEQ ID NO: 367, encoding nucleotide sequence: SEQ ID NO: 366) was identified as a protein comprising any of SEQ ID NOs: 368 to 382, phosphoglycerate kinase, cytosolic (amino acid sequence: SEQ ID NO: 384, encoding nucleotide sequence: SEQ ID NO: 383) was identified as a protein comprising any of SEQ ID NOs: 385 to 392, and phosphoglycerate kinase, cytosolic (amino acid sequence: SEQ ID NO: 394, encoding nucleotide sequence: SEQ ID NO: 393) was identified as a protein comprising any of SEQ ID NOs: 395 to 409.

Accordingly, the antigen in spot 26α in the present invention can be any of proteins selected from the group consisting of the proteins as defined below in (26αA1-a) to (26αA1-e), (26αB1), (26αA2-a) to (26αA2-e), (26αB2), (26αA3-a) to (26αA3-e), and (26αB3):
(26αA1-a) a protein comprising an amino acid sequence with deletion, substitution, insertion or addition of one or several amino acids in SEQ ID NO: 367;
(26αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 367;
(26αA1-c) a protein comprising an amino acid sequence encoded by a nucleotide sequence with deletion, substitution, insertion or addition of one or several nucleotides in SEQ ID NO: 366;
(26αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 366;
(26αA1-e) a protein comprising an amino acid sequence encoded by a nucleic acid that hybridizes under stringent conditions with a nucleic acid having a nucleotide sequence complementary to the nucleotide sequence of SEQ ID NO: 366;
(26αB1) a protein comprising at least one amino acid sequence selected from the group consisting of SEQ ID NOs: 368-382, preferably a protein comprising at least 2, 3, 4, 5, 6, 7, 8, 9, 10, 11, 12, 13, or 14 or all sequences of the amino acid sequences. As referred to above, the amino acid sequence of any of SEQ ID NOs: 368 to 382 may be an amino acid sequence derived therefrom by deletion, substitution, insertion or addition of one or several amino acids;
(26αA2-a) a protein comprising an amino acid sequence with deletion, substitution, insertion or addition of one or several amino acids in SEQ ID NO: 384;
(26αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 384;
(26αA2-c) a protein comprising an amino acid sequence encoded by a nucleotide sequence with deletion, substitution, insertion or addition of one or several nucleotides in SEQ ID NO: 383;
(26αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 383;
(26αA2-e) a protein comprising an amino acid sequence encoded by a nucleic acid that hybridizes under stringent conditions with a nucleic acid having a nucleotide sequence complementary to the nucleotide sequence of SEQ ID NO: 383;
(26αB2) a protein comprising at least one amino acid sequence selected from the group consisting of SEQ ID NOs: 385-392, preferably a protein comprising at least 2, 3, 4, 5, 6, or 7 or all sequences of the amino acid sequences. As referred to above, the amino acid sequence of any of SEQ ID NOs: 385 to 392 may be an amino acid sequence derived therefrom by deletion, substitution, insertion or addition of one or several amino acids.
(26αA3-a) a protein comprising an amino acid sequence with deletion, substitution, insertion or addition of one or several amino acids in SEQ ID NO: 394;
(26αA3-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 394;
(26αA3-c) a protein comprising an amino acid sequence encoded by a nucleotide sequence with deletion, substitution, insertion or addition of one or several nucleotides in SEQ ID NO: 393;
(26αA3-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 393;
(26αA3-e) a protein comprising an amino acid sequence encoded by a nucleic acid that hybridizes under stringent conditions with a nucleic acid having a nucleotide sequence complementary to the nucleotide sequence of SEQ ID NO: 393;
(26αB3) a protein comprising at least one amino acid sequence selected from the group consisting of SEQ ID NOs: 395-409, preferably a protein comprising at least 2, 3, 4, 5, 6, 7, 8, 9, 10, 11, 12, 13, or 14 or all sequences of the amino acid sequences. As referred to above, the amino acid sequence of any of SEQ ID NOs: 395 to 409 may be an amino acid sequence derived therefrom by deletion, substitution, insertion or addition of one or several amino acids.

The protein selected from the group consisting of the proteins as defined above in (26αA1-a) to (26αA1-e), (26αB1), (26αA2-a) to (26αA2-e), (26αB2), (26αA3-a) to (26αA3-e), and (26αB3) can be proteins that are found in a protein spot with a molecular weight of around 20 to 90 kDa, preferably around 30 to 80 kDa, and an isoelectric point of 3.5 to 7.5, preferably 4.0 to 7.0 on gels used in the two-dimensional electrophoresis performed under the conditions described above in the subsection titled "Identification of antigens". Epitopes of the aforementioned antigens (26αA1-a) to (26αA1-e) and (26αB1) reside in amino acids 121-135 (SEQ ID NO: 816) and amino acids 329-335 (SEQ ID NO: 819) of SEQ ID NO: 367. When the antigen in spot 26α contains a variation in the amino acid sequence of SEQ ID NO: 367, this antigen may retain the sequence in SEQ ID NO: 361 as to amino acids at positions corresponding to the region of at least one of these epitopes.

From the viewpoint of improving sensitivity (a degree to which a patient can be diagnosed as being positive) or specificity (a degree to which a healthy subject is not diagnosed as being positive), the antigen in spot 26α may be the following variant.

In the epitope having the sequence of SEQ ID NO: 819, sites of amino acids other than X in SEQ ID NO: 817 or 818 are important for binding to IgE antibodies from allergic patients. Thus, in another preferred mode, when the antigen in spot 26α contains a variation in the amino acid sequence of SEQ ID NO: 367, amino acids 329-335 of SEQ ID NO: 367 in this antigen may have the amino acid sequence of SEQ ID NO: 817 or 818.

Preferably, a protein that is an antigen in spot 25α has activity equivalent to that of the protein having the amino acid sequence of SEQ ID NO: 367, 384, or 394 (phosphoglycerate kinase, cytosolic) or causative of an allergy to soybean.

### (27a) Antigen in spot 27α

As the result of sequence identification of the antigen in spot 27α by mass spectroscopy, the amino acid sequences of SEQ ID NOs: 412-427 were detected.

Also, the mass spectroscopic data obtained for spot 27α on a mass spectrometer was analyzed by comparing the data against the NCBI protein data, and as a result, alcohol dehydrogenase 1 (amino acid sequence: SEQ ID NO: 411, encoding nucleotide sequence: SEQ ID NO: 410) was identified as a protein comprising any of SEQ ID NOs: 412 to 416, alcohol dehydrogenase 1 (amino acid sequence: SEQ ID NO: 418, encoding nucleotide sequence: SEQ ID NO: 417) was identified as a protein comprising any of SEQ ID NOs: 419 and 420, and alcohol dehydrogenase 1-like (amino acid sequence: SEQ ID NO: 422, encoding nucleotide sequence: SEQ ID NO: 421) was identified as a protein comprising any of SEQ ID NOs: 423 to 427.

Accordingly, the antigen in spot 27α in the present invention can be any of proteins selected from the group consisting of the proteins as defined below in (27αA1-a) to (27αA1-e), (27αB1), (27αA2-a) to (27αA2-e), (27αB2), (27αA3-a) to (27αA3-e), and (27αB3):
(27αA1-a) a protein comprising an amino acid sequence with deletion, substitution, insertion or addition of one or several amino acids in SEQ ID NO: 411;
(27αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 411;
(27αA1-c) a protein comprising an amino acid sequence encoded by a nucleotide sequence with deletion, substitution, insertion or addition of one or several nucleotides in SEQ ID NO: 410;
(27αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 410;
(27αA1-e) a protein comprising an amino acid sequence encoded by a nucleic acid that hybridizes under stringent conditions with a nucleic acid having a nucleotide sequence complementary to the nucleotide sequence of SEQ ID NO: 410;
(27αB1) a protein comprising at least one amino acid sequence selected from the group consisting of SEQ ID NOs: 412-416, preferably a protein comprising at least 2, 3, or 4 or all sequences of the amino acid sequences. As referred to above, the amino acid sequence of any of SEQ ID NOs: 412 to 416 may be an amino acid sequence derived therefrom by deletion, substitution, insertion or addition of one or several amino acids;
(27αA2-a) a protein comprising an amino acid sequence with deletion, substitution, insertion or addition of one or several amino acids in SEQ ID NO: 418;
(27αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 418;
(27αA2-c) a protein comprising an amino acid sequence encoded by a nucleotide sequence with deletion, substitution, insertion or addition of one or several nucleotides in SEQ ID NO: 417;
(27αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 417;
(27αA2-e) a protein comprising an amino acid sequence encoded by a nucleic acid that hybridizes under stringent conditions with a nucleic acid having a nucleotide sequence complementary to the nucleotide sequence of SEQ ID NO: 417;
(27αB2) a protein comprising at least one amino acid sequence selected from the group consisting of SEQ ID NOs: 419 and 420, preferably a protein comprising both the amino acid sequences. As referred to above, the amino acid sequence of any of SEQ ID NOs: 419, 420 may be an amino acid sequence derived therefrom by deletion, substitution, insertion or addition of one or several amino acids.
(27αA3-a) a protein comprising an amino acid sequence with deletion, substitution, insertion or addition of one or several amino acids in SEQ ID NO: 422;
(27αA3-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 422;
(27αA3-c) a protein comprising an amino acid sequence encoded by a nucleotide sequence with deletion, substitution, insertion or addition of one or several nucleotides in SEQ ID NO: 421;
(27αA3-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 421;
(27αA3-e) a protein comprising an amino acid sequence encoded by a nucleic acid that hybridizes under stringent conditions with a nucleic acid having a nucleotide sequence complementary to the nucleotide sequence of SEQ ID NO: 421;
(27αB3) a protein comprising at least one amino acid sequence selected from the group consisting of SEQ ID NOs: 423-427, preferably a protein comprising at least 2, 3, or 4 or all sequences of the amino acid sequences. As referred to above, the amino acid sequence of any of SEQ ID NOs: 423 to 427 may be an amino acid sequence derived therefrom by deletion, substitution, insertion or addition of one or several amino acids.

The protein selected from the group consisting of the proteins as defined above in (27αA1-a) to (27αA1-e), (27αB1), (27αA2-a) to (27αA2-e), (27αB2), (27αA3-a) to (27αA3-e), and (27αB3) can be proteins that are found in a protein spot with a molecular weight of around 20 to 90 kDa, preferably around 30 to 80 kDa, and an isoelectric point of 3.5 to 7.5, preferably 4.0 to 7.0 on gels used in the two-dimensional electrophoresis performed under the conditions described above in the subsection titled "Identification of antigens".

Epitopes of the aforementioned antigens (27αA1-a) to (27αA1-e) and (27αB1) reside in amino acids 21-35 (SEQ ID NO: 820) of SEQ ID NO: 411. When the antigen in spot 27α contains a variation in the amino acid sequence of SEQ ID NO: 411, this antigen may retain the sequence in SEQ ID NO: 411 as to amino acids at positions corresponding to the region of this epitope.

Preferably, a protein that is an antigen in spot 27α has activity equivalent to that of the protein having the amino acid sequence of SEQ ID NO: 411 or 418 (alcohol dehydrogenase 1) or the protein having the amino acid sequence of SEQ ID NO: 422 (alcohol dehydrogenase 1-like) or causative of an allergy to soybean.

### (28α) Antigen in spot 28α

As the result of sequence identification of the antigen in spot 28α by mass spectroscopy, the amino acid sequences of SEQ ID NOs: 430-433 were detected.

Also, the mass spectroscopic data obtained for spot 28α on a mass spectrometer was analyzed by comparing the data against the NCBI protein data, and as a result, 35 kDa seed maturation protein isoform X1 (amino acid sequence: SEQ ID NO: 429, encoding nucleotide sequence: SEQ ID NO: 428) was identified as a protein comprising any of SEQ ID NO: 430 to 433.

Accordingly, the antigens in spot 28α in the present invention can be any protein selected from the group consisting of the proteins as defined below in (28αA-a) to (28αA-e) and (28αB):
(28αA-a) a protein comprising an amino acid sequence with deletion, substitution, insertion or addition of one or several amino acids in SEQ ID NO: 429;
(28αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 429;
(28αA-c) a protein comprising an amino acid sequence encoded by a nucleotide sequence with deletion, substitution, insertion or addition of one or several nucleotides in SEQ ID NO: 428;
(28αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 428;
(28αA-e) a protein comprising an amino acid sequence encoded by a nucleic acid that hybridizes under stringent conditions with a nucleic acid having a nucleotide sequence complementary to the nucleotide sequence of SEQ ID NO: 428;
(28αB) a protein comprising at least one amino acid sequence selected from the group consisting of SEQ ID NOs: 430-433, preferably a protein comprising at least 2 or 3 or all sequences of the amino acid sequences. As referred to above, the amino acid sequence of any of SEQ ID NOs: 430-433 may be an amino acid sequence derived therefrom by deletion, substitution, insertion or addition of one or several amino acids.

The proteins selected from the group consisting of (28αA-a) to (28αA-e) and (28αB) as defined above can be proteins that are found in a protein spot with a molecular weight of around 10 to 70 kDa, preferably around 20 to 60 kDa and an isoelectric point of 3.5 to 7.5, preferably 4.0 to 7.0 on gels used in the two-dimensional electrophoresis performed under the conditions described above in the subsection titled "Identification of antigens".

Epitopes of the antigen in spot 28α reside in amino acids 76-85(SEQ ID NO: 820), amino acids 191-205 (SEQ ID NO: 826), amino acids 231-245 (SEQ ID NO: 829), amino acids 111-125 (SEQ ID NO: 830), amino acids 131-145 (SEQ ID NO: 831), amino acids 161-175 (SEQ ID NO: 832), amino acids 181-195 (SEQ ID NO: 833), amino acids 291-305 (SEQ ID NO: 834), amino acids 295-309 (SEQ ID NO: 835) of SEQ ID NO: 429. The antigen in spot 28α may retain the sequence in SEQ ID NO: 429 as to amino acids at positions corresponding to the region of at least one of these epitopes.

From the viewpoint of improving sensitivity (a degree to which a patient can be diagnosed as being positive) or specificity (a degree to which a healthy subject is not diagnosed as being positive), the antigen in spot 28α may be the following variant.

In the epitope having the sequence of SEQ ID NO: 823, sites of amino acids other than X in SEQ ID NO: 821 or 822 are important for binding to IgE antibodies from allergic patients. Thus, in another preferred mode, amino acids 76-85 of SEQ ID NO: 429 in the antigen in spot 28α may have the amino acid sequence of SEQ ID NO: 821 or 822.

In the epitope having the sequence of SEQ ID NO: 826, sites of amino acids other than X in SEQ ID NO: 824 or 825 are important for binding to IgE antibodies from allergic patients. Thus, in another preferred mode, amino acids 191-205 of SEQ ID NO: 429 in the antigen in spot 28α may have the amino acid sequence of SEQ ID NO: 824 or 825.

In the epitope having the sequence of SEQ ID NO: 829, sites of amino acids other than X in SEQ ID NO: 827 or 828 are important for binding to IgE antibodies from allergic patients. Thus, in another preferred mode, amino acids 231-2455 of SEQ ID NO: 429 in the antigen in spot 28α may have the amino acid sequence of SEQ ID NO: 827 or 828.

Preferably, a protein that is an antigen in spot 28α has activity equivalent to that of the protein having the amino acid sequence of SEQ ID NO: 422 (35 kDa seed maturation protein isoform X1) or causative of an allergy to soybean.

### (29α, 30α, 31α, 32α, 33α) Antigens in spots 29α to 33α

As the result of sequence identification of the antigens in spots 29α to 33α by mass spectroscopy, the amino acid sequences of SEQ ID NOs: 436-451 were detected.

Also, the mass spectroscopic data obtained for spots 29α to 33α on a mass spectrometer was analyzed by comparing the data against the NCBI protein data, and as a result, glyceraldehyde-3-phosphate dehydrogenase isoform X1 (amino acid sequence: SEQ ID NO: 435, encoding nucleotide sequence: SEQ ID NO: 434) was identified as a protein comprising any of SEQ ID NO: 436 to 439, glyceraldehyde-3-phosphate dehydrogenase, cytosolic (amino acid sequence: SEQ ID NO: 441, encoding nucleotide sequence: SEQ ID NO: 440) was identified as a protein comprising any of SEQ ID NO: 442 to 445, and uncharacterized protein LOC100782924 (amino acid sequence: SEQ ID NO: 447, encoding nucleotide sequence: SEQ ID NO: 446) was identified as a protein comprising any of SEQ ID NO: 448 to 451.

Accordingly, the antigen in spot 29α to 33α in the present invention can be any of proteins selected from the group consisting of the proteins as defined below in (29-33αA1-a) to (29-33αA1-e), (29-33αB1), (29-33αA2-a) to (29-33αA2-e), (29-33αB2), (29-33αA3-a) to (29-33αA3-e), and (29-33αB3):
(29-33αA1-a) a protein comprising an amino acid sequence with deletion, substitution, insertion or addition of one or several amino acids in SEQ ID NO: 435;
(29-33αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 435;
(29-33αA1-c) a protein comprising an amino acid sequence encoded by a nucleotide sequence with deletion, substitution, insertion or addition of one or several nucleotides in SEQ ID NO: 434;
(29-33αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 434;
(29-33αA1-e) a protein comprising an amino acid sequence encoded by a nucleic acid that hybridizes under stringent conditions with a nucleic acid having a nucleotide sequence complementary to the nucleotide sequence of SEQ ID NO: 434;
(29-33αB1) a protein comprising at least one amino acid sequence selected from the group consisting of SEQ ID NOs: 436-439, preferably a protein comprising at least 2, 3, or all sequences of the amino acid sequences. As referred to above, the amino acid sequence of any of SEQ ID NOs: 436 to 439 may be an amino acid sequence derived therefrom by deletion, substitution, insertion or addition of one or several amino acids;
(29-33αA2-a) a protein comprising an amino acid sequence with deletion, substitution, insertion or addition of one or several amino acids in SEQ ID NO: 441;
(29-33αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 441;
(29-33αA2-c) a protein comprising an amino acid sequence encoded by a nucleotide sequence with deletion, substitution, insertion or addition of one or several nucleotides in SEQ ID NO: 440;
(29-33αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 440;
(29-33αA2-e) a protein comprising an amino acid sequence encoded by a nucleic acid that hybridizes under stringent conditions with a nucleic acid having a nucleotide sequence complementary to the nucleotide sequence of SEQ ID NO: 440;
(29-33αB2) a protein comprising at least one amino acid sequence selected from the group consisting of SEQ ID NOs: 442-445, preferably a protein comprising at least 2, 3, or all sequences of the amino acid sequences. As referred to above, the amino acid sequence of any of SEQ ID NOs: 442 to 445 may be an amino acid sequence derived therefrom by deletion, substitution, insertion or addition of one or several amino acids.
(29-33αA3-a) a protein comprising an amino acid sequence with deletion, substitution, insertion or addition of one or several amino acids in SEQ ID NO: 447;
(29-33αA3-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 447;
(29-33αA3-c) a protein comprising an amino acid sequence encoded by a nucleotide sequence with deletion, substitution, insertion or addition of one or several nucleotides in SEQ ID NO: 446;
(29-33αA3-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 446;
(29-33αA3-e) a protein comprising an amino acid sequence encoded by a nucleic acid that hybridizes under stringent conditions with a nucleic acid having a nucleotide sequence complementary to the nucleotide sequence of SEQ ID NO: 446;
(29-33αB3) a protein comprising at least one amino acid sequence selected from the group consisting of SEQ ID NOs: 448-451, preferably a protein comprising at least 2, 3, or all sequences of the amino acid sequences. As referred to above, the amino acid sequence of any of SEQ ID NOs: 448 to 451 may be an amino acid sequence derived therefrom by deletion, substitution, insertion or addition of one or several amino acids.

The protein selected from the group consisting of the proteins as defined above in (29-33αA1-a) to (29-33αA1-e), (29-33αB1), (29-33αA2-a) to (29-33αA2-e), (29-33αB2), (29-33αA3-a) to (29-33αA3-e), and (29-33αB3) can be proteins that are found in a protein spot with a molecular weight of around 10 to 70 kDa, preferably around 20 to 60 kDa, and an isoelectric point of 3.5 to 8.5, preferably 4.0 to 8.0 on gels used in the two-dimensional electrophoresis performed under the conditions described above in the subsection titled "Identification of antigens".

Epitopes of the aforementioned antigens (29-33αA1-a) to (29-33αA1-e) and (29-33αB1) reside in amino acids 66-75 (SEQ ID NO: 838), amino acids 301-315 (SEQ ID NO: 839) of SEQ ID NO: 435. When the antigen in spot 29-33α contains a variation in the amino acid sequence of SEQ ID NO: 435, this antigen may retain the sequence in SEQ ID NO: 435 as to amino acids at positions corresponding to the region of at least one of these epitopes.

From the viewpoint of improving sensitivity (a degree to which a patient can be diagnosed as being positive) or specificity (a degree to which a healthy subject is not diagnosed as being positive), the antigen in spot 29-33α may be the following variant.

In the epitope having the sequence of SEQ ID NO: 838, sites of amino acids other than X in SEQ ID NO: 836 or 837 are important for binding to IgE antibodies from allergic patients. Thus, in another preferred mode, when the antigen in spot 29-33α contains a variation in the amino acid sequence of SEQ ID NO: 435, amino acids 66-75 of SEQ ID NO: 435 in this antigen may have the amino acid sequence of SEQ ID NO: 836 or 837.

Preferably, a protein that is an antigen in any of spots 29α to 33α has activity equivalent to that of the protein having the amino acid sequence of SEQ ID NO: 435 (glyceraldehyde-3-phosphate dehydrogenase isoform X1), the protein having the amino acid sequence of SEQ ID NO: 441 (glyceraldehyde-3-phosphate dehydrogenase, cytosolic), or the protein having the amino acid sequence of SEQ ID NO: 447 (uncharacterized protein LOC100782924) or causative of an allergy to soybean.

### (34α) Antigen in spot 34α

As the result of sequence identification of the antigen in spot 34α by mass spectroscopy, the amino acid sequences of SEQ ID NOs: 454-458 were detected.

Also, the mass spectroscopic data obtained for spot 34α on a mass spectrometer was analyzed by comparing the data against the NCBI protein data, and as a result, bifunctional UDP-glucose 4-epimerase and UDP-xylose 4-epimerase 1-like (amino acid sequence: SEQ ID NO: 453, encoding nucleotide sequence: SEQ ID NO: 452) was identified as a protein comprising SEQ ID NO: 454 and uncharacterized protein LOC100803483 (amino acid sequence: SEQ ID NO: 456, encoding nucleotide sequence: SEQ ID NO: 455) was identified as a protein comprising SEQ ID NO: 457 or 458.

Accordingly, the antigen in spot 34α in the present invention can be any of proteins selected from the group consisting of the proteins as defined below in (34αA1-a) to (34αA1-e), (34αB1), (34αA2-a) to (34αA2-e), and (34αB2):
(34αA1-a) a protein comprising an amino acid sequence with deletion, substitution, insertion or addition of one or several amino acids in SEQ ID NO: 453;
(34αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 453;
(34αA1-c) a protein comprising an amino acid sequence encoded by a nucleotide sequence with deletion, substitution, insertion or addition of one or several nucleotides in SEQ ID NO: 452;
(34αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 452;
(34αA1-e) a protein comprising an amino acid sequence encoded by a nucleic acid that hybridizes under stringent conditions with a nucleic acid having a nucleotide sequence complementary to the nucleotide sequence of SEQ ID NO: 452;
(34αB1) a protein comprising an amino acid sequence of SEQ ID NOs: 454. As referred to above, the amino acid sequence of any of SEQ ID NOs: 464 may be an amino acid sequence derived therefrom by deletion, substitution, insertion or addition of one or several amino acids;
(34αA2-a) a protein comprising an amino acid sequence with deletion, substitution, insertion or addition of one or several amino acids in SEQ ID NO: 456;
(34αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 456;
(34αA2-c) a protein comprising an amino acid sequence encoded by a nucleotide sequence with deletion, substitution, insertion or addition of one or several nucleotides in SEQ ID NO: 455;
(34αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 455;
(34αA2-e) a protein comprising an amino acid sequence encoded by a nucleic acid that hybridizes under stringent conditions with a nucleic acid having a nucleotide sequence complementary to the nucleotide sequence of SEQ ID NO: 455;
(34αB2) a protein comprising at least one amino acid sequence selected from the group consisting of SEQ ID NOs: 457 and 458, preferably a protein comprising both the amino acid sequences. As referred to above, the amino acid sequence of any of SEQ ID NOs: 457, 458 may be an amino acid sequence derived therefrom by deletion, substitution, insertion or addition of one or several amino acids.

The protein selected from the group consisting of the proteins as defined above in (34αA1-a) to (34αA1-e), (34αB1), (34αA2-a) to (34αA2-e), and (34αB2) can be proteins that are found in a protein spot with a molecular weight of around 10 to 70 kDa, preferably around 20 to 60 kDa, and an isoelectric point of 5.5 to 10.5, preferably 6.0 to 10.0 on gels used in the two-dimensional electrophoresis performed under the conditions described above in the subsection titled "Identification of antigens".

Epitopes of the aforementioned antigens (34αA1-a) to (34αA1-e) and (34αB1) reside in amino acids 61-75 (SEQ ID NO: 842), amino acids 31-45 (SEQ ID NO: 843), amino acids 141-155 (SEQ ID NO: 844), amino acids 221-235 (SEQ ID NO: 845), amino acids 231-245 (SEQ ID NO: 846) of SEQ ID NO: 454. When the antigen in spot 34α contains a variation in the amino acid sequence of SEQ ID NO: 454, this antigen may retain the sequence in SEQ ID NO: 454 as to amino acids at positions corresponding to the region of at least one of these epitopes.

From the viewpoint of improving sensitivity (a degree to which a patient can be diagnosed as being positive) or specificity (a degree to which a healthy subject is not diagnosed as being positive), the antigen in spot 34α may be the following variant.

In the epitope having the sequence of SEQ ID NO: 842, sites of amino acids other than X in SEQ ID NO: 840 or 841 are important for binding to IgE antibodies from allergic patients. Thus, in another preferred mode, when the antigen in spot 34α contains a variation in the amino acid sequence of SEQ ID NO: 454, amino acids 61-75 of SEQ ID NO: 454 in this antigen may have the amino acid sequence of SEQ ID NO: 840 or 841.

Preferably, a protein that is an antigen in spot 34α has activity equivalent to that of the protein having the amino acid sequence of SEQ ID NO: 453 (bifunctional UDP-glucose 4-epimerase and UDP-xylose 4-epimerase 1-like) or the protein having the amino acid sequence of SEQ ID NO: 456 (uncharacterized protein LOC100803483) or causative of an allergy to soybean.

### (35α) Antigen in spot 35α

As the result of sequence identification of the antigen in spot 35α by mass spectroscopy, the amino acid sequences of SEQ ID NOs: 461-477 were detected.

Also, the mass spectroscopic data obtained for spot 35α on a mass spectrometer was analyzed by comparing the data against the NCBI protein data, and as a result, elongation factor 1-alpha (amino acid sequence: SEQ ID NO: 460, encoding nucleotide sequence: SEQ ID NO: 459) was identified as a protein comprising any of SEQ ID NOs: 461 to 466, elongation factor 1-alpha (amino acid sequence: SEQ ID NO: 468, encoding nucleotide sequence: SEQ ID NO: 467) was identified as a protein comprising any of SEQ ID NOs: 469 to 471, and elongation factor-lA isoform X1 (amino acid sequence: SEQ ID NO: 473, encoding nucleotide sequence: SEQ ID NO: 472) was identified as a protein comprising any of SEQ ID NOs: 474 to 477.

Accordingly, the antigen in spot 35α in the present invention can be any of proteins selected from the group consisting of the proteins as defined below in (35αA1-a) to (35αA1-e), (35αB1), (35αA2-a) to (35αA2-e), (35αB2), (35αA3-a) to (35αA3-e), and (35αB3):
(35αA1-a) a protein comprising an amino acid sequence with deletion, substitution, insertion or addition of one or several amino acids in SEQ ID NO: 460;
(35αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 460;
(35αA1-c) a protein comprising an amino acid sequence encoded by a nucleotide sequence with deletion, substitution, insertion or addition of one or several nucleotides in SEQ ID NO: 459;
(35αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 459;
(35αA1-e) a protein comprising an amino acid sequence encoded by a nucleic acid that hybridizes under stringent conditions with a nucleic acid having a nucleotide sequence complementary to the nucleotide sequence of SEQ ID NO: 459;
(35αB1) a protein comprising at least one amino acid sequence selected from the group consisting of SEQ ID NOs: 461-466, preferably a protein comprising at least 2, 3, 4, or 5 or all sequences of the amino acid sequences. As referred to above, the amino acid sequence of any of SEQ ID NOs: 412 to 416 may be an amino acid sequence derived therefrom by deletion, substitution, insertion or addition of one or several amino acids;
(35αA2-a) a protein comprising an amino acid sequence with deletion, substitution, insertion or addition of one or several amino acids in SEQ ID NO: 468;
(35αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 468;
(35αA2-c) a protein comprising an amino acid sequence encoded by a nucleotide sequence with deletion, substitution, insertion or addition of one or several nucleotides in SEQ ID NO: 467;
(35αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 467;
(35αA2-e) a protein comprising an amino acid sequence encoded by a nucleic acid that hybridizes under stringent conditions with a nucleic acid having a nucleotide sequence complementary to the nucleotide sequence of SEQ ID NO: 467;
(35αB2) a protein comprising at least one amino acid sequence selected from the group consisting of SEQ ID NOs: 469-471, preferably a protein comprising at least 2, or all sequences of the amino acid sequences. As referred to above, the amino acid sequence of any of SEQ ID NOs: 469 to 471 may be an amino acid sequence derived therefrom by deletion, substitution, insertion or addition of one or several amino acids.
(35αA3-a) a protein comprising an amino acid sequence with deletion, substitution, insertion or addition of one or several amino acids in SEQ ID NO: 473;
(35αA3-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 473;
(35αA3-c) a protein comprising an amino acid sequence encoded by a nucleotide sequence with deletion, substitution, insertion or addition of one or several nucleotides in SEQ ID NO: 472;
(35αA3-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 472;
(35αA3-e) a protein comprising an amino acid sequence encoded by a nucleic acid that hybridizes under stringent conditions with a nucleic acid having a nucleotide sequence complementary to the nucleotide sequence of SEQ ID NO: 472;
(35αB3) a protein comprising at least one amino acid sequence selected from the group consisting of SEQ ID NOs: 474-477, preferably a protein comprising at least 2, 3, or all sequences of the amino acid sequences. As referred to above, the amino acid sequence of any of SEQ ID NOs: 474 to 477 may be an amino acid sequence derived therefrom by deletion, substitution, insertion or addition of one or several amino acids.

The protein selected from the group consisting of the proteins as defined above in (35αA1-a) to (35αA1-e), (35αB1), (35αA2-a) to (35αA2-e), (35αB2), (35αA3-a) to (35αA3-e), and (35αB3) can be proteins that are found in a protein spot with a molecular weight of around 20 to 90 kDa, preferably around 30 to 80 kDa, and an isoelectric point of 5.5 to 10.5, preferably 6.0 to 10.0 on gels used in the two-dimensional electrophoresis performed under the conditions described above in the subsection titled "Identification of antigens".

Epitopes of the aforementioned antigens (35αA1-a) to (35αA1-e) and (35αB1) reside in amino acids 31-45 (SEQ ID NO: 849), amino acids 121-135 (SEQ ID NO: 850), amino acids 192-220 (SEQ ID NO: 853), amino acids 216-225 (SEQ ID NO: 856) of SEQ ID NO: 460. When the antigen in spot 35α contains a variation in the amino acid sequence of SEQ ID NO: 460, amino acids at positions corresponding to the region of at least one of these epitopes may retain the sequence in SEQ ID NO: 460.

From the viewpoint of improving sensitivity (a degree to which a patient can be diagnosed as being positive) or specificity (a degree to which a healthy subject is not diagnosed as being positive), the antigen in spot 35α may be the following variant.

In the epitope having the sequence of SEQ ID NO: 849, sites of amino acids other than X in SEQ ID NO: 847 or 848 are important for binding to IgE antibodies from allergic patients. Thus, in another preferred mode, when the antigen in spot 35α contains a variation in the amino acid sequence of SEQ ID NO: 460, amino acids 31-45 of SEQ ID NO: 460 in this antigen may have the amino acid sequence of SEQ ID NO: 847 or 848.

In the epitope having the sequence of SEQ ID NO: 853, sites of amino acids other than X in SEQ ID NO: 851 or 852 are important for binding to IgE antibodies from allergic patients. Thus, in another preferred mode, when the antigen in spot 35α contains a variation in the amino acid sequence of SEQ ID NO: 460, amino acids 192-220 of SEQ ID NO: 460 in this antigen may have the amino acid sequence of SEQ ID NO: 851 or 852.

In the epitope having the sequence of SEQ ID NO: 856, sites of amino acids other than X in SEQ ID NO: 854 or 855 are important for binding to IgE antibodies from allergic patients. Thus, in another preferred mode, when the antigen in spot 35α contains a variation in the amino acid sequence of SEQ ID NO: 460, amino acids 216-225 of SEQ ID NO: 460 in this antigen may have the amino acid sequence of SEQ ID NO: 854 or 855.

Preferably, a protein that is an antigen in spot 35α has activity equivalent to that of the protein having the amino acid sequence of SEQ ID NO: 460 or 468 (elongation factor 1-alpha) or the protein having the amino acid sequence of SEQ ID NO: 473 (elongation factor-1A isoform X1) or causative of an allergy to soybean.

### (36α, 37α) Antigens in spots 36α, 37α

As the result of sequence identification of the antigens in spots 36α and 37α by mass spectroscopy, the amino acid sequences of SEQ ID NOs: 480-493 were detected.

Also, the mass spectroscopic data obtained for spots 36α and 37α on a mass spectrometer was analyzed by comparing the data against the NCBI protein data, and as a result, seed maturation protein PM34 (amino acid sequence: SEQ ID NO: 479, encoding nucleotide sequence: SEQ ID NO: 478) was identified as a protein comprising any of SEQ ID NOs: 480 to 483, uncharacterized protein LOC100809384 (amino acid sequence: SEQ ID NO: 485, encoding nucleotide sequence: SEQ ID NO: 484) was identified as a protein comprising SEQ ID NO: 486 or 487, and hypothetical protein GLYMA_10G155300 (amino acid sequence: SEQ ID NO: 489, encoding nucleotide sequence: SEQ ID NO: 488) was identified as a protein comprising any of SEQ ID NOs: 4490 to 493.

Accordingly, the antigen in spot 36α, 37α in the present invention can be any of proteins selected from the group consisting of the proteins as defined below in (36, 37αA1-a) to (36, 37αA1-e), (36, 37αB1), (36, 37αA2-a) to (36, 37αA2-e), (36, 37αB2), (36, 37αA3-a) to (36, 37αA3-e), and (36, 37αB3):
(36, 37αA1-a) a protein comprising an amino acid sequence with deletion, substitution, insertion or addition of one or several amino acids in SEQ ID NO: 479;
(36, 37αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 479;
(36, 37αA1-c) a protein comprising an amino acid sequence encoded by a nucleotide sequence with deletion, substitution, insertion or addition of one or several nucleotides in SEQ ID NO: 478;
(36, 37αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 478;
(36, 37αA1-e) a protein comprising an amino acid sequence encoded by a nucleic acid that hybridizes under stringent conditions with a nucleic acid having a nucleotide sequence complementary to the nucleotide sequence of SEQ ID NO: 478;
(36, 37αB1) a protein comprising at least one amino acid sequence selected from the group consisting of SEQ ID NOs: 480-483, preferably a protein comprising at least 2, 3, or all sequences of the amino acid sequences. As referred to above, the amino acid sequence of any of SEQ ID NOs: 480 to 483 may be an amino acid sequence derived therefrom by deletion, substitution, insertion or addition of one or several amino acids;
(36, 37αA2-a) a protein comprising an amino acid sequence with deletion, substitution, insertion or addition of one or several amino acids in SEQ ID NO: 485;
(36, 37αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 485;
(36, 37αA2-c) a protein comprising an amino acid sequence encoded by a nucleotide sequence with deletion, substitution, insertion or addition of one or several nucleotides in SEQ ID NO: 484;
(36, 37αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 484;
(36, 37αA2-e) a protein comprising an amino acid sequence encoded by a nucleic acid that hybridizes under stringent conditions with a nucleic acid having a nucleotide sequence complementary to the nucleotide sequence of SEQ ID NO: 484;
(36, 37αB2) a protein comprising at least one amino acid sequence selected from the group consisting of SEQ ID NOs: 486 and 487, preferably a protein comprising both the amino acid sequences. As referred to above, the amino acid sequence of any of SEQ ID NOs: 486, 487 may be an amino acid sequence derived therefrom by deletion, substitution, insertion or addition of one or several amino acids.
(36, 37αA3-a) a protein comprising an amino acid sequence with deletion, substitution, insertion or addition of one or several amino acids in SEQ ID NO: 489;
(36, 37αA3-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 489;
(36, 37αA3-c) a protein comprising an amino acid sequence encoded by a nucleotide sequence with deletion, substitution, insertion or addition of one or several nucleotides in SEQ ID NO: 488;
(36, 37αA3-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 488;
(36, 37αA3-e) a protein comprising an amino acid sequence encoded by a nucleic acid that hybridizes under stringent conditions with a nucleic acid having a nucleotide sequence complementary to the nucleotide sequence of SEQ ID NO: 488;
(36, 37αB3) a protein comprising at least one amino acid sequence selected from the group consisting of SEQ ID NOs: 490-493, preferably a protein comprising at least 2, 3, or all sequences of the amino acid sequences. As referred to above, the amino acid sequence of any of SEQ ID NOs: 490 to 493 may be an amino acid sequence derived therefrom by deletion, substitution, insertion or addition of one or several amino acids.

The protein selected from the group consisting of the proteins as defined above in (36, 37αA1-a) to (36, 37αA1-e), (36, 37αB1), (36, 37αA2-a) to (36, 37αA2-e), (36, 37αB2), (36, 37αA3-a) to (36, 37αA3-e), and (36, 37αB3) can be proteins that are found in a protein spot with a molecular weight of around 10 to 70 kDa, preferably around 20 to 60 kDa, and an isoelectric point of 3.5 to 8.5, preferably 4.0 to 8.0 on gels used in the two-dimensional electrophoresis performed under the conditions described above in the subsection titled "Identification of antigens".

Epitopes of the aforementioned antigens (36, 37αA1-a) to (36, 37αA1-e) and (36, 37αB1) reside in amino acids 141-155 (SEQ ID NO: 857), amino acids 231-245 (SEQ ID NO: 858), amino acids 7-14 (SEQ ID NO: 861) of SEQ ID NO: 479. When the antigen in spot 36α, 37α contains a variation in the amino acid sequence of SEQ ID NO: 479, this antigen may retain the sequence in SEQ ID NO: 479 as to amino acids at positions corresponding to the region of at least one of these epitopes.

From the viewpoint of improving sensitivity (a degree to which a patient can be diagnosed as being positive) or specificity (a degree to which a healthy subject is not diagnosed as being positive), the antigen in spot 36α,37α may be the following variant.

In the epitope having the sequence of SEQ ID NO: 861, sites of amino acids other than X in SEQ ID NO: 859 or 860 are important for binding to IgE antibodies from allergic patients. Thus, in another preferred mode, when the antigen in spot 36α,37α contains a variation in the amino acid sequence of SEQ ID NO: 479, amino acids 7-14 of SEQ ID NO: 479 in this antigen may have the amino acid sequence of SEQ ID NO: 859 or 860.

Preferably, a protein that is an antigen in spot 36α or 37α has activity equivalent to that of the protein having the amino acid sequence of SEQ ID NO: 479 (seed maturation protein PM34), the protein having the amino acid sequence of SEQ ID NO: 485 (uncharacterized protein LOC100809384), or the protein having the amino acid sequence of SEQ ID NO: 489 (hypothetical protein GLYMA_10G155300) or causative of an allergy to soybean.

### (38α) Antigen in spot 38α

As the result of sequence identification of the antigen in spot 38α by mass spectroscopy, the amino acid sequences of SEQ ID NOs: 496-513 were detected.

Also, the mass spectroscopic data obtained for spot 38α on a mass spectrometer was analyzed by comparing the data against the NCBI protein data, and as a result, seed biotin-containing protein SBP65-like isoform X1 (amino acid sequence: SEQ ID NO: 495, encoding nucleotide sequence: SEQ ID NO: 494) was identified as a protein comprising any of SEQ ID NOs: 496 to 513.

Accordingly, the antigens in spot 38α in the present invention can be any protein selected from the group consisting of the proteins as defined below in (38αA-a) to (38αA-e) and (38αB):
(38αA-a) a protein comprising an amino acid sequence with deletion, substitution, insertion or addition of one or several amino acids in SEQ ID NO: 495;
(38αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 495;
(38αA-c) a protein comprising an amino acid sequence encoded by a nucleotide sequence with deletion, substitution, insertion or addition of one or several nucleotides in SEQ ID NO: 494;
(38αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 494;
(38αA-e) a protein comprising an amino acid sequence encoded by a nucleic acid that hybridizes under stringent conditions with a nucleic acid having a nucleotide sequence complementary to the nucleotide sequence of SEQ ID NO: 494;
(38αB) a protein comprising at least one amino acid sequence selected from the group consisting of SEQ ID NOs: 496-513, preferably a protein comprising at least 2, 3, 4, 5, 6, 7, 8, 9, 10, 11, 12, 13, 14, 15, 16 or 17 or all sequences of the amino acid sequences. As referred to above, the amino acid sequence of any of SEQ ID NOs: 496-513 may be an amino acid sequence derived therefrom by deletion, substitution, insertion or addition of one or several amino acids.

The proteins selected from the group consisting of (38αA-a) to (38αA-e) and (38αB) as defined above can be proteins that are found in a protein spot with a molecular weight of around 70 to 170 kDa, preferably around 80 to 160 kDa and an isoelectric point of 5.5 to 10.5, preferably 6.0 to 10.0 on gels used in the two-dimensional electrophoresis performed under the conditions described above in the subsection titled "Identification of antigens".

Epitopes of the antigen in spot 38α reside in amino acids 440-446 or 574-580 (SEQ ID NO: 864), amino acids 71-80 (SEQ ID NO: 867), amino acids 521-535 (SEQ ID NO: 869), amino acids 1-15 (SEQ ID NO: 871), amino acids 31-45 (SEQ ID NO: 874), amino acids 221-235 (SEQ ID NO: 877), and amino acids 461-470 (SEQ ID NO: 882), and amino acids 481-490 (SEQ ID NO: 884), and amino acids 821-835 (SEQ ID NO: 887), and amino acids 861-870 (SEQ ID NO: 889), and amino acids 1031-1040 (SEQ ID NO: 892) of SEQ ID NO: 495. The antigen in spot 38α may retain the sequence in SEQ ID NO: 495 as to amino acids at positions corresponding to the region of at least one of these epitopes.

From the viewpoint of improving sensitivity (a degree to which a patient can be diagnosed as being positive) or specificity (a degree to which a healthy subject is not diagnosed as being positive), the antigen in spot 38α may be the following variant.

In the epitope having the sequence of SEQ ID NO: 864, sites of amino acids other than X in SEQ ID NO: 862 are important for binding to IgE antibodies from allergic patients. Thus, in another preferred mode, amino acids 440-446 and / or 574-580 of SEQ ID NO: 495 in the antigen in spot 38α may have the amino acid sequence of SEQ ID NO: 862.

In the epitope having the sequence of SEQ ID NO: 867, sites of amino acids other than X in SEQ ID NO: 865 or 866 are important for binding to IgE antibodies from allergic patients. Thus, in another preferred mode, amino acids 71-80 of SEQ ID NO: 495 in the antigen in spot 38α may have the amino acid sequence of SEQ ID NO: 865 or 866.

In the epitope having the sequence of SEQ ID NO: 869, sites of amino acids other than X in SEQ ID NO: 868 are important for binding to IgE antibodies from allergic patients. Thus, in another preferred mode, amino acids 521-535 of SEQ ID NO: 495 in the antigen in spot 38α may have the amino acid sequence of SEQ ID NO: 868.

In the epitope having the sequence of SEQ ID NO: 871, sites of amino acids other than X in SEQ ID NO: 870 are important for binding to IgE antibodies from allergic patients. Thus, in another preferred mode, amino acids 1-15 of SEQ ID NO: 495 in the antigen in spot 38α may have the amino acid sequence of SEQ ID NO: 870.

In the epitope having the sequence of SEQ ID NO: 874, sites of amino acids other than X in SEQ ID NO: 872 or 873 are important for binding to IgE antibodies from allergic patients. Thus, in another preferred mode, amino acids 31-45 of SEQ ID NO: 495 in the antigen in spot 38α may have the amino acid sequence of SEQ ID NO: 872 or 873.

In the epitope having the sequence of SEQ ID NO: 877, sites of amino acids other than X in SEQ ID NO: 875 or 876 are important for binding to IgE antibodies from allergic patients. Thus, in another preferred mode, amino acids 221-235 of SEQ ID NO: 495 in the antigen in spot 38α may have the amino acid sequence of SEQ ID NO: 875 or 876.

In the epitope having the sequence of SEQ ID NO: 880, sites of amino acids other than X in SEQ ID NO: 878 or 879 are important for binding to IgE antibodies from allergic patients. Thus, in another preferred mode, amino acids 311-325 of SEQ ID NO: 495 in the antigen in spot 38α may have the amino acid sequence of SEQ ID NO: 878 or 879.

In the epitope having the sequence of SEQ ID NO: 882, sites of amino acids other than X in SEQ ID NO: 881 are important for binding to IgE antibodies from allergic patients. Thus, in another preferred mode, amino acids 461-470 of SEQ ID NO: 495 in the antigen in spot 38α may have the amino acid sequence of SEQ ID NO: 881.

In the epitope having the sequence of SEQ ID NO: 884, sites of amino acids other than X in SEQ ID NO: 883 are important for binding to IgE antibodies from allergic patients. Thus, in another preferred mode, amino acids 481-490 of SEQ ID NO: 495 in the antigen in spot 38α may have the amino acid sequence of SEQ ID NO: 883.

In the epitope having the sequence of SEQ ID NO: 887, sites of amino acids other than X in SEQ ID NO: 885 or 886 are important for binding to IgE antibodies from allergic patients. Thus, in another preferred mode, amino acids 821-835 of SEQ ID NO: 495 in the antigen in spot 38α may have the amino acid sequence of SEQ ID NO: 885 or 886.

In the epitope having the sequence of SEQ ID NO: 889, sites of amino acids other than X in SEQ ID NO: 888 are important for binding to IgE antibodies from allergic patients. Thus, in another preferred mode, amino acids 861-870 of SEQ ID NO: 495 in the antigen in spot 38α may have the amino acid sequence of SEQ ID NO: 888.

In the epitope having the sequence of SEQ ID NO: 892, sites of amino acids other than X in SEQ ID NO: 890 or 891 are important for binding to IgE antibodies from allergic patients. Thus, in another preferred mode, amino acids 1031-1040 of SEQ ID NO: 495 in the antigen in spot 38α may have the amino acid sequence of SEQ ID NO: 890 or 891.

Preferably, a protein that is an antigen in spot 38α has activity equivalent to that of the protein having the amino acid sequence of SEQ ID NO: 495 (seed biotin-containing protein SBP65-like isoform X1) or causative of an allergy to soybean.

### (39α) Antigen in spot 39α

As the result of sequence identification of the antigen in spot 39α by mass spectroscopy, the amino acid sequence of SEQ ID NO: 516 was detected.

Also, the mass spectroscopic data obtained for spot 39α on a mass spectrometer was analyzed by comparing the data against the NCBI protein data, and as a result, ATP synthase subunit O, mitochondrial-like (amino acid sequence: SEQ ID NO: 515, encoding nucleotide sequence: SEQ ID NO: 514) was identified as a protein comprising SEQ ID NO: 516.

Accordingly, the antigens in spot 39α in the present invention can be any protein selected from the group consisting of the proteins as defined below in (39αA-a) to (39αA-e) and (39αB):
(39αA-a) a protein comprising an amino acid sequence with deletion, substitution, insertion or addition of one or several amino acids in SEQ ID NO: 515;
(39αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 515;
(39αA-c) a protein comprising an amino acid sequence encoded by a nucleotide sequence with deletion, substitution, insertion or addition of one or several nucleotides in SEQ ID NO: 514;
(39αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 514;
(39αA-e) a protein comprising an amino acid sequence encoded by a nucleic acid that hybridizes under stringent conditions with a nucleic acid having a nucleotide sequence complementary to the nucleotide sequence of SEQ ID NO: 514;
(39αB) a protein comprising an amino acid sequence of SEQ ID NO: 516. As referred to above, the amino acid sequence(s) of SEQ ID NO: 516 may be an amino acid sequence(s) derived therefrom by deletion, substitution, insertion or addition of one or several amino acids.

The proteins selected from the group consisting of (39αA-a) to (39αA-e) and (39αB) as defined above can be proteins that are found in a protein spot with a molecular weight of around 5 to 60 kDa, preferably around 10 to 50 kDa and an isoelectric point of 5.5 to 10.5, preferably 6.0 to 10.0 on gels used in the two-dimensional electrophoresis performed under the conditions described above in the subsection titled "Identification of antigens".

An epitope of the antigen in spot 39α resides in amino acids 71-85 (SEQ ID NO: 895) of SEQ ID NO: 515. The antigen in spot 39α may retain the sequence in SEQ ID NO: 515 as to amino acids at positions corresponding to the region of this epitope.

From the viewpoint of improving sensitivity (a degree to which a patient can be diagnosed as being positive) or specificity (a degree to which a healthy subject is not diagnosed as being positive), the antigen in spot 39α may be the following variant.

In the epitope having the sequence of SEQ ID NO: 895, sites of amino acids other than X in SEQ ID NO: 893 or 894 are important for binding to IgE antibodies from allergic patients. Thus, in another preferred mode, amino acids 71-85 of SEQ ID NO: 515 in the antigen in spot 39α may have the amino acid sequence of SEQ ID NO: 893 or 894.

Preferably, a protein that is an antigen in spot 39α has activity equivalent to that of the protein having the amino acid sequence of SEQ ID NO: 515 (ATP synthase subunit O, mitochondrial-like) or causative of an allergy to soybean.

### (40α, 56α) Antigens in spots 40α, 56α

As the result of sequence identification of the antigens in spots 40α and 56α by mass spectroscopy, the amino acid sequences of SEQ ID NOs: 519 and 520 were detected.

Also, the mass spectroscopic data obtained for spots 40α and 56α on a mass spectrometer was analyzed by comparing the data against the NCBI protein data, and as a result, P24 oleosin isoform A (amino acid sequence: SEQ ID NO: 518, encoding nucleotide sequence: SEQ ID NO: 517) was identified as a protein comprising SEQ ID NO: 519 or 520.

Accordingly, the antigens in spot 40α, 56α in the present invention can be any protein selected from the group consisting of the proteins as defined below in (40, 56αA-a) to (40, 56αA-e) and (40, 56αB):
(40, 56αA-a) a protein comprising an amino acid sequence with deletion, substitution, insertion or addition of one or several amino acids in SEQ ID NO: 518;
(40, 56αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 518;
(40, 56αA-c) a protein comprising an amino acid sequence encoded by a nucleotide sequence with deletion, substitution, insertion or addition of one or several nucleotides in SEQ ID NO: 517;
(40, 56αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 517;
(40, 56αA-e) a protein comprising an amino acid sequence encoded by a nucleic acid that hybridizes under stringent conditions with a nucleic acid having a nucleotide sequence complementary to the nucleotide sequence of SEQ ID NO: 517;
(40, 56αB) a protein comprising at least one amino acid sequence selected from the group consisting of SEQ ID NOs: 519 and 520, preferably a protein comprising both the amino acid sequences. As referred to above, the amino acid sequence of any of SEQ ID NOs: 519 and 520 may be an amino acid sequence derived therefrom by deletion, substitution, insertion or addition of one or several amino acids.

The proteins selected from the group consisting of (40, 56αA-a) to (40, 56αA-e) and (40, 56αB) as defined above can be proteins that are found in a protein spot with a molecular weight of around 5 to 60 kDa, preferably around 10 to 50 kDa and an isoelectric point of 5.5 to 10.5, preferably 6.0 to 10.0 on gels used in the two-dimensional electrophoresis performed under the conditions described above in the subsection titled "Identification of antigens".

Epitopes of the antigens in spots 40α and 56α reside in amino acids 35-39 (SEQ ID NO: 898), amino acids 191-205 (SEQ ID NO: 899) and amino acids 214-222 (SEQ ID NO: 902) of SEQ ID NO: 518. The antigens in spots 40α and 56α may retain the sequence in SEQ ID NO: 518 as to amino acids at positions corresponding to the region of these epitopes.

From the viewpoint of improving sensitivity (a degree to which a patient can be diagnosed as being positive) or specificity (a degree to which a healthy subject is not diagnosed as being positive), the antigen in spot 40α, 56α may be the following variant.

In the epitope having the sequence of SEQ ID NO: 898, sites of amino acids other than X in SEQ ID NO: 896 or 897 are important for binding to IgE antibodies from allergic patients. Thus, in another preferred mode, amino acids 35-39 of SEQ ID NO: 518 in the antigen in spot 40α, 56α may have the amino acid sequence of SEQ ID NO: 896 or 897.

In the epitope having the sequence of SEQ ID NO: 902, sites of amino acids other than X in SEQ ID NO: 900 or 901 are important for binding to IgE antibodies from allergic patients. Thus, in another preferred mode, amino acids 214-2229 of SEQ ID NO: 518 in the antigen in spot 40α, 56α may have the amino acid sequence of SEQ ID NO: 900 or 901.

Preferably, a protein that is an antigen in spot 40α or 56α has activity equivalent to that of the protein having the amino acid sequence of SEQ ID NO: 518 (P24 oleosin isoform A) or causative of an allergy to soybean.

### (41α) Antigen in spot 41α

As the result of sequence identification of the antigen in spot 41α by mass spectroscopy, the amino acid sequence of SEQ ID NO: 523 was detected.

Also, the mass spectroscopic data obtained for spot 41α on a mass spectrometer was analyzed by comparing the data against the NCBI protein data, and as a result, uncharacterized protein At3g49720-like isoform X2 (amino acid sequence: SEQ ID NO: 522, encoding nucleotide sequence: SEQ ID NO: 521) was identified as a protein comprising SEQ ID NO: 523.

Accordingly, the antigens in spot 41α in the present invention can be any protein selected from the group consisting of the proteins as defined below in (41αA-a) to (41αA-e) and (41αB):
(41αA-a) a protein comprising an amino acid sequence with deletion, substitution, insertion or addition of one or several amino acids in SEQ ID NO: 522;
(41αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 522;
(41αA-c) a protein comprising an amino acid sequence encoded by a nucleotide sequence with deletion, substitution, insertion or addition of one or several nucleotides in SEQ ID NO: 521;
(41αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 521;
(41αA-e) a protein comprising an amino acid sequence encoded by a nucleic acid that hybridizes under stringent conditions with a nucleic acid having a nucleotide sequence complementary to the nucleotide sequence of SEQ ID NO: 521;
(41αB) a protein comprising an amino acid sequence of SEQ ID NO: 523. As referred to above, the amino acid sequence of SEQ ID NO: 523 may be an amino acid sequence derived therefrom by deletion, substitution, insertion or addition of one or several amino acids.

The proteins selected from the group consisting of (41αA-a) to (41αA-e) and (41αB) as defined above can be proteins that are found in a protein spot with a molecular weight of around 5 to 60 kDa, preferably around 10 to 50 kDa and an isoelectric point of 5.5 to 10.5, preferably 6.0 to 10.0 on gels used in the two-dimensional electrophoresis performed under the conditions described above in the subsection titled "Identification of antigens".

An epitope of the antigen in spot 41α resides in amino acids 111-125 (SEQ ID NO: 903) of SEQ ID NO: 522. The antigen in spot 41α may retain the sequence in SEQ ID NO: 522 as to amino acids at positions corresponding to the region of this epitope.

Preferably, a protein that is an antigen in spot 41α has activity equivalent to that of the protein having the amino acid sequence of SEQ ID NO: 515 (ATP synthase subunit O, mitochondrial-like) or causative of an allergy to soybean.

### (42α, 43α) Antigen in spots 42α, 43α

As the result of sequence identification of the antigens in spots 42α and 43α by mass spectroscopy, the amino acid sequences of SEQ ID NOs: 526-539 were detected.

Also, the mass spectroscopic data obtained for spots 42α and 43α on a mass spectrometer was analyzed by comparing the data against the NCBI protein data, and as a result, superoxide dismutase [Mn], mitochondrial (amino acid sequence: SEQ ID NO: 525, encoding nucleotide sequence: SEQ ID NO: 524) was identified as a protein comprising any of SEQ ID NOs: 526 to 531 and hypothetical protein GLYMA_04G221300 (amino acid sequence: SEQ ID NO: 533, encoding nucleotide sequence: SEQ ID NO: 532) was identified as a protein comprising any of SEQ ID NOs: 534 to 539.

Accordingly, the antigen in spot 42α, 43α in the present invention can be any of proteins selected from the group consisting of the proteins as defined below in (42, 43αA1-a) to (42, 43αA1-e), (42, 43αB1), (42, 43αA2-a) to (42, 43αA2-e), and (42, 43αB2):
(42, 43αA1-a) a protein comprising an amino acid sequence with deletion, substitution, insertion or addition of one or several amino acids in SEQ ID NO: 525;
(42, 43αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 525;
(42, 43αA1-c) a protein comprising an amino acid sequence encoded by a nucleotide sequence with deletion, substitution, insertion or addition of one or several nucleotides in SEQ ID NO: 524;
(42, 43αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 524;
(42, 43αA1-e) a protein comprising an amino acid sequence encoded by a nucleic acid that hybridizes under stringent conditions with a nucleic acid having a nucleotide sequence complementary to the nucleotide sequence of SEQ ID NO: 524;
(42, 43αB1) a protein comprising at least one amino acid sequence selected from the group consisting of SEQ ID NOs: 526-531, preferably a protein comprising at least 2, 3, 4, or 5 or all sequences of the amino acid sequences. As referred to above, the amino acid sequence of any of SEQ ID NOs: 526 to 531 may be an amino acid sequence derived therefrom by deletion, substitution, insertion or addition of one or several amino acids;
(42, 43αA2-a) a protein comprising an amino acid sequence with deletion, substitution, insertion or addition of one or several amino acids in SEQ ID NO: 533;
(42, 43αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 533;
(42, 43αA2-c) a protein comprising an amino acid sequence encoded by a nucleotide sequence with deletion, substitution, insertion or addition of one or several nucleotides in SEQ ID NO: 532;
(42, 43αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 532;
(42, 43αA2-e) a protein comprising an amino acid sequence encoded by a nucleic acid that hybridizes under stringent conditions with a nucleic acid having a nucleotide sequence complementary to the nucleotide sequence of SEQ ID NO: 532;
(42, 43αB2) a protein comprising at least one amino acid sequence selected from the group consisting of SEQ ID NOs: 534-539, preferably a protein comprising at least 2, 3, 4, or 5 or all sequences of the amino acid sequences. As referred to above, the amino acid sequence of any of SEQ ID NOs: 534 to 539 may be an amino acid sequence derived therefrom by deletion, substitution, insertion or addition of one or several amino acids.

The protein selected from the group consisting of the proteins as defined above in (42, 43αA1-a) to (42, 43αA1-e), (42, 43αB1), (42, 43αA2-a) to (42, 43αA2-e), and (42, 43αB2) can be proteins that are found in a protein spot with a molecular weight of around 5 to 60 kDa, preferably around 10 to 50 kDa, and an isoelectric point of 5.5 to 10.5, preferably 6.0 to 10.0 on gels used in the two-dimensional electrophoresis performed under the conditions described above in the subsection titled "Identification of antigens".

Epitopes of the aforementioned antigens (42, 43αA1-a) to (42, 43αA1-e) and (42, 43αB1) reside in amino acids 227-241 (SEQ ID NO: 906) of SEQ ID NO: 525. When the antigen in spot 42α, 43α contains a variation in the amino acid sequence of SEQ ID NO: 525, this antigen may retain the sequence in SEQ ID NO: 525 as to amino acids at positions corresponding to the region of at least one of these epitopes.

From the viewpoint of improving sensitivity (a degree to which a patient can be diagnosed as being positive) or specificity (a degree to which a healthy subject is not diagnosed as being positive), the antigen in spot 42α, 43α may be the following variant.

In the epitope having the sequence of SEQ ID NO: 906, sites of amino acids other than X in SEQ ID NO: 904 or 905 are important for binding to IgE antibodies from allergic patients. Thus, in another preferred mode, when the antigen in spot 42α, 43α contains a variation in the amino acid sequence of SEQ ID NO: 525, amino acids 227-241 of SEQ ID NO: 525 in this antigen may have the amino acid sequence of SEQ ID NO: 904 or 905.

Preferably, a protein that is an antigen in spot 42α or 43α has activity equivalent to that of the protein having the amino acid sequence of SEQ ID NO: 525 (superoxide dismutase [Mn], mitochondrial) or the protein having the amino acid sequence of SEQ ID NO: 533 (hypothetical protein GLYMA_04G221300) or causative of an allergy to soybean.

### (45α) Antigen in spot 45α

As the result of sequence identification of the antigen in spot 45α by mass spectroscopy, the amino acid sequences of SEQ ID NOs: 542-551 were detected.

Also, the mass spectroscopic data obtained for spot 45α on a mass spectrometer was analyzed by comparing the data against the NCBI protein data, and as a result, uncharacterized protein LOC100499771 (amino acid sequence: SEQ ID NO: 541, encoding nucleotide sequence: SEQ ID NO: 540) was identified as a protein comprising any of SEQ ID NOs: 542 to 545 and hypothetical protein GLYMA_09G192800 (amino acid sequence: SEQ ID NO: 547, encoding nucleotide sequence: SEQ ID NO: 546) was identified as a protein comprising any of SEQ ID NOs: 548 to 551.

Accordingly, the antigen in spot 45α in the present invention can be any of proteins selected from the group consisting of the proteins as defined below in (45αA1-a) to (45αA1-e), (45αB1), (45αA2-a) to (45αA2e), and (45αB2):
(45αA1-a) a protein comprising an amino acid sequence with deletion, substitution, insertion or addition of one or several amino acids in SEQ ID NO: 541;
(45αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 541;
(45αA1-c) a protein comprising an amino acid sequence encoded by a nucleotide sequence with deletion, substitution, insertion or addition of one or several nucleotides in SEQ ID NO: 540;
(45αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 540;
(45αA1-e) a protein comprising an amino acid sequence encoded by a nucleic acid that hybridizes under stringent conditions with a nucleic acid having a nucleotide sequence complementary to the nucleotide sequence of SEQ ID NO: 540;
(45αB1) a protein comprising at least one amino acid sequence selected from the group consisting of SEQ ID NOs: 542-545, preferably a protein comprising at least 2, 3, or all sequences of the amino acid sequences. As referred to above, the amino acid sequence of any of SEQ ID NOs: 542 to 545 may be an amino acid sequence derived therefrom by deletion, substitution, insertion or addition of one or several amino acids;
(45αA2-a) a protein comprising an amino acid sequence with deletion, substitution, insertion or addition of one or several amino acids in SEQ ID NO: 547;
(45αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 547;
(45αA2-c) a protein comprising an amino acid sequence encoded by a nucleotide sequence with deletion, substitution, insertion or addition of one or several nucleotides in SEQ ID NO: 546;
(45αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 546;
(45αA2-e) a protein comprising an amino acid sequence encoded by a nucleic acid that hybridizes under stringent conditions with a nucleic acid having a nucleotide sequence complementary to the nucleotide sequence of SEQ ID NO: 546;
(45αB2) a protein comprising at least one amino acid sequence selected from the group consisting of SEQ ID NOs: 548-551, preferably a protein comprising at least 2, 3, or all sequences of the amino acid sequences. As referred to above, the amino acid sequence of any of SEQ ID NOs: 548 to 551 may be an amino acid sequence derived therefrom by deletion, substitution, insertion or addition of one or several amino acids.

The protein selected from the group consisting of the proteins as defined above in (45αA1-a) to (45αA1-e), (45αB1), (45αA2-a) to (45αA2-e), and (45αB2) can be proteins that are found in a protein spot with a molecular weight of around 2 to 50 kDa, preferably around 5 to 40 kDa, and an isoelectric point of 3.5 to 7.5, preferably 4.0 to 7.0 on gels used in the two-dimensional electrophoresis performed under the conditions described above in the subsection titled "Identification of antigens".

Epitopes of the aforementioned antigens (45αA1-a) to (45αA1-e) and (45αB1) reside in amino acids 11-25 (SEQ ID NO: 910), amino acids 51-65 (SEQ ID NO: 911), amino acids 111-125 (SEQ ID NO: 912) of SEQ ID NO: 541. When the antigen in spot 45α contains a variation in the amino acid sequence of SEQ ID NO: 541, amino acids at positions corresponding to the region of at least one of these epitopes may retain the sequence in SEQ ID NO: 541.

Preferably, a protein that is an antigen in spot 45α has activity equivalent to that of the protein having the amino acid sequence of SEQ ID NO: 541 (uncharacterized protein LOC100499771) or the protein having the amino acid sequence of SEQ ID NO: 547 (hypothetical protein GLYMA_09G192800) or causative of an allergy to soybean.

### (46α) Antigen in spot 46α

As the result of sequence identification of the antigen in spot 46α by mass spectroscopy, the amino acid sequence of SEQ ID NOs: 554 was detected.

Also, the mass spectroscopic data obtained for spot 46α on a mass spectrometer was analyzed by comparing the data against the NCBI protein data, and as a result, seed maturation protein PM22 (amino acid sequence: SEQ ID NO: 553, encoding nucleotide sequence: SEQ ID NO: 552) was identified as a protein comprising SEQ ID NO: 554.

Accordingly, the antigens in spot 46α in the present invention can be any protein selected from the group consisting of the proteins as defined below in (46αA-a) to (46αA-e) and (46αB):
(46αA-a) a protein comprising an amino acid sequence with deletion, substitution, insertion or addition of one or several amino acids in SEQ ID NO: 553;
(46αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 553;
(46αA-c) a protein comprising an amino acid sequence encoded by a nucleotide sequence with deletion, substitution, insertion or addition of one or several nucleotides in SEQ ID NO: 552;
(46αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 552;
(46αA-e) a protein comprising an amino acid sequence encoded by a nucleic acid that hybridizes under stringent conditions with a nucleic acid having a nucleotide sequence complementary to the nucleotide sequence of SEQ ID NO: 552;
(46αB) a protein comprising an amino acid sequence of SEQ ID NO: 554. As referred to above, the amino acid sequence(s) of SEQ ID NO: 554 may be an amino acid sequence(s) derived therefrom by deletion, substitution, insertion or addition of one or several amino acids.

The proteins selected from the group consisting of (46αA-a) to (46αA-e) and (46αB) as defined above can be proteins that are found in a protein spot with a molecular weight of around 2 to 50 kDa, preferably around 5 to 40 kDa and an isoelectric point of 3.5 to 7.5, preferably 4.0 to 7.0 on gels used in the two-dimensional electrophoresis performed under the conditions described above in the subsection titled "Identification of antigens".

An epitope of the antigen in spot 46α resides in amino acids 112-120 (SEQ ID NO: 915) of SEQ ID NO: 553. The antigen in spot 46α may retain the sequence in SEQ ID NO: 553 as to amino acids at positions corresponding to the region of this epitope.

From the viewpoint of improving sensitivity (a degree to which a patient can be diagnosed as being positive) or specificity (a degree to which a healthy subject is not diagnosed as being positive), the antigen in spot 46α may be the following variant.

In the epitope having the sequence of SEQ ID NO: 915, sites of amino acids other than X in SEQ ID NO: 913 or 914 are important for binding to IgE antibodies from allergic patients. Thus, in another preferred mode, amino acids 112-120 of SEQ ID NO: 553 in the antigen in spot 46α may have the amino acid sequence of SEQ ID NO: 913 or 914.

Preferably, a protein that is an antigen in spot 46α has activity equivalent to that of the protein having the amino acid sequence of SEQ ID NO: 553 (seed maturation protein PM22) or causative of an allergy to soybean.

### (47α) Antigen in spot 47α

As the result of sequence identification of the antigen in spot 47α by mass spectroscopy, the amino acid sequences of SEQ ID NOs: 557-563 were detected.

Also, the mass spectroscopic data obtained for spot 47α on a mass spectrometer was analyzed by comparing the data against the NCBI protein data, and as a result, eukaryotic translation initiation factor 5A-2 (amino acid sequence: SEQ ID NO: 556, encoding nucleotide sequence: SEQ ID NO: 555) was identified as a protein comprising SEQ ID NO: 557 or 558 and uncharacterized protein LOC100305510 (amino acid sequence: SEQ ID NO: 560, encoding nucleotide sequence: SEQ ID NO: 559) was identified as a protein comprising any of SEQ ID NOs: 561 to 563.

Accordingly, the antigen in spot 47α in the present invention can be any of proteins selected from the group consisting of the proteins as defined below in (47αA1-a) to (47αA1-e), (47αB1), (47αA2-a) to (47αA2-e), and (47αB2):
(47αA1-a) a protein comprising an amino acid sequence with deletion, substitution, insertion or addition of one or several amino acids in SEQ ID NO: 556;
(47αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 556;
(47αA1-c) a protein comprising an amino acid sequence encoded by a nucleotide sequence with deletion, substitution, insertion or addition of one or several nucleotides in SEQ ID NO: 555;
(47αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 555;
(47αA1-e) a protein comprising an amino acid sequence encoded by a nucleic acid that hybridizes under stringent conditions with a nucleic acid having a nucleotide sequence complementary to the nucleotide sequence of SEQ ID NO: 555;
(47αB1) a protein comprising at least one amino acid sequence selected from the group consisting of SEQ ID NOs: 557 and 558, preferably a protein comprising both the amino acid sequences. As referred to above, the amino acid sequence of any of SEQ ID NOs: 557, 558 may be an amino acid sequence derived therefrom by deletion, substitution, insertion or addition of one or several amino acids;
(47αA2-a) a protein comprising an amino acid sequence with deletion, substitution, insertion or addition of one or several amino acids in SEQ ID NO: 560;
(47αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 560;
(47αA2-c) a protein comprising an amino acid sequence encoded by a nucleotide sequence with deletion, substitution, insertion or addition of one or several nucleotides in SEQ ID NO: 559;
(47αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 559;
(47αA2-e) a protein comprising an amino acid sequence encoded by a nucleic acid that hybridizes under stringent conditions with a nucleic acid having a nucleotide sequence complementary to the nucleotide sequence of SEQ ID NO: 559;
(47αB2) a protein comprising at least one amino acid sequence selected from the group consisting of SEQ ID NOs: 561-563, preferably a protein comprising at least 2, or all sequences of the amino acid sequences. As referred to above, the amino acid sequence of any of SEQ ID NOs: 548 to 551 may be an amino acid sequence derived therefrom by deletion, substitution, insertion or addition of one or several amino acids.

The protein selected from the group consisting of the proteins as defined above in (47αA1-a) to (47αA1-e), (47αB1), (47αA2-a) to (47αA2-e), and (47αB2) can be proteins that are found in a protein spot with a molecular weight of around 2 to 50 kDa, preferably around 5 to 40 kDa, and an isoelectric point of 3.5 to 7.5, preferably 4.0 to 7.0 on gels used in the two-dimensional electrophoresis performed under the conditions described above in the subsection titled "Identification of antigens".

Epitopes of the aforementioned antigens (47αA1-a) to (47αA1-e) and (47αB1) reside in amino acids 11-25 (SEQ ID NO: 916), amino acids 81-95 (SEQ ID NO: 917), amino acids 131-145 (SEQ ID NO: 918) of SEQ ID NO: 556. When the antigen in spot 47α contains a variation in the amino acid sequence of SEQ ID NO: 556, amino acids at positions corresponding to the region of at least one of these epitopes may retain the sequence in SEQ ID NO: 556.

Preferably, a protein that is an antigen in spot 47α has activity equivalent to that of the protein having the amino acid sequence of SEQ ID NO: 556 (eukaryotic translation initiation factor 5A-2) or the protein having the amino acid sequence of SEQ ID NO: 560 (uncharacterized protein LOC100305510) or causative of an allergy to soybean.

### (48α) Antigen in spot 48α

As the result of sequence identification of the antigen in spot 48α by mass spectroscopy, the amino acid sequences of SEQ ID NOs: 566-570 were detected.

Also, the mass spectroscopic data obtained for spot 48α on a mass spectrometer was analyzed by comparing the data against the NCBI protein data, and as a result, uncharacterized protein LOC100306570 (amino acid sequence: SEQ ID NO: 565, encoding nucleotide sequence: SEQ ID NO: 564) was identified as a protein comprising any of SEQ ID NOs: 566 to 570.

Accordingly, the antigens in spot 48α in the present invention can be any protein selected from the group consisting of the proteins as defined below in (48αA-a) to (48αA-e) and (48αB):
(48αA-a) a protein comprising an amino acid sequence with deletion, substitution, insertion or addition of one or several amino acids in SEQ ID NO: 565;
(48αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 565;
(48αA-c) a protein comprising an amino acid sequence encoded by a nucleotide sequence with deletion, substitution, insertion or addition of one or several nucleotides in SEQ ID NO: 564;
(48αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 564;
(48αA-e) a protein comprising an amino acid sequence encoded by a nucleic acid that hybridizes under stringent conditions with a nucleic acid having a nucleotide sequence complementary to the nucleotide sequence of SEQ ID NO: 564;
(48αB) a protein comprising at least one amino acid sequence selected from the group consisting of SEQ ID NOs: 566-570, preferably a protein comprising at least 2, 3 or 4 or all sequences of the amino acid sequences. As referred to above, the amino acid sequence of any of SEQ ID NOs: 566-570 may be an amino acid sequence derived therefrom by deletion, substitution, insertion or addition of one or several amino acids.

The proteins selected from the group consisting of (48αA-a) to (48αA-e) and (48αB) as defined above can be proteins that are found in a protein spot with a molecular weight of around 2 to 50 kDa, preferably around 5 to 40 kDa and an isoelectric point of 3.5 to 8.5, preferably 4.0 to 8.0 on gels used in the two-dimensional electrophoresis performed under the conditions described above in the subsection titled "Identification of antigens".

Epitopes of the antigen in spot 48α reside in amino acids 94-102 (SEQ ID NO: 921) and amino acids 131-145 (SEQ ID NO: 922) of SEQ ID NO: 565. The antigen in spot 48α may retain the sequence in SEQ ID NO: 565 as to amino acids at positions corresponding to the region of these epitopes.

From the viewpoint of improving sensitivity (a degree to which a patient can be diagnosed as being positive) or specificity (a degree to which a healthy subject is not diagnosed as being positive), the antigen in spot 48α may be the following variant.

In the epitope having the sequence of SEQ ID NO: 921, sites of amino acids other than X in SEQ ID NO: 919 or 918 are important for binding to IgE antibodies from allergic patients. Thus, in another preferred mode, amino acids 94-102 of SEQ ID NO: 565 in the antigen in spot 48α may have the amino acid sequence of SEQ ID NO: 919 or 918.

Preferably, a protein that is an antigen in spot 48α has activity equivalent to that of the protein having the amino acid sequence of SEQ ID NO: 565 (uncharacterized protein LOC100306570) or causative of an allergy to soybean.

### (50α) Antigen in spot 50α

As the result of sequence identification of the antigen in spot 50α by mass spectroscopy, the amino acid sequences of SEQ ID NOs: 573-576 were detected.

Also, the mass spectroscopic data obtained for spot 50α on a mass spectrometer was analyzed by comparing the data against the NCBI protein data, and as a result, uncharacterized protein LOC100813859 (amino acid sequence: SEQ ID NO: 572, encoding nucleotide sequence: SEQ ID NO: 571) was identified as a protein comprising any of SEQ ID NOs: 573 to 576.

Accordingly, the antigens in spot 50α in the present invention can be any protein selected from the group consisting of the proteins as defined below in (50αA-a) to (50αA-e) and (50αB):
(50αA-a) a protein comprising an amino acid sequence with deletion, substitution, insertion or addition of one or several amino acids in SEQ ID NO: 572;
(50αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 572;
(50αA-c) a protein comprising an amino acid sequence encoded by a nucleotide sequence with deletion, substitution, insertion or addition of one or several nucleotides in SEQ ID NO: 571;
(50αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 571;
(50αA-e) a protein comprising an amino acid sequence encoded by a nucleic acid that hybridizes under stringent conditions with a nucleic acid having a nucleotide sequence complementary to the nucleotide sequence of SEQ ID NO: 571;
(50αB) a protein comprising at least one amino acid sequence selected from the group consisting of SEQ ID NOs: 573-576, preferably a protein comprising at least 2 or 3 or all sequences of the amino acid sequences. As referred to above, the amino acid sequence of any of SEQ ID NOs: 573-576 may be an amino acid sequence derived therefrom by deletion, substitution, insertion or addition of one or several amino acids.

The proteins selected from the group consisting of (50αA-a) to (50αA-e) and (50αB) as defined above can be proteins that are found in a protein spot with a molecular weight of around 2 to 50 kDa, preferably around 5 to 40 kDa and an isoelectric point of 2.5 to 6.5, preferably 3.0 to 6.0 on gels used in the two-dimensional electrophoresis performed under the conditions described above in the subsection titled "Identification of antigens".

Epitopes of the antigen in spot 50α reside in amino acids 91-105 (SEQ ID NO: 924) and amino acids 62-70 (SEQ ID NO: 927) of SEQ ID NO: 572. The antigen in spot 50α may retain the sequence in SEQ ID NO: 572 as to amino acids at positions corresponding to the region of these epitopes.

From the viewpoint of improving sensitivity (a degree to which a patient can be diagnosed as being positive) or specificity (a degree to which a healthy subject is not diagnosed as being positive), the antigen in spot 50α may be the following variant.

In the epitope having the sequence of SEQ ID NO: 927, sites of amino acids other than X in SEQ ID NO: 925 or 926 are important for binding to IgE antibodies from allergic patients. Thus, in another preferred mode, amino acids 62-70 of SEQ ID NO: 572 in the antigen in spot 50α may have the amino acid sequence of SEQ ID NO: 925 or 926.

Preferably, a protein that is an antigen in spot 50α has activity equivalent to that of the protein having the amino acid sequence of SEQ ID NO: 572 (uncharacterized protein LOC100813859) or causative of an allergy to soybean.

### (51α) Antigen in spot 51α

As the result of sequence identification of the antigen in spot 51α by mass spectroscopy, the amino acid sequences of SEQ ID NOs: 579-582 were detected.

Also, the mass spectroscopic data obtained for spot 51α on a mass spectrometer was analyzed by comparing the data against the NCBI protein data, and as a result, 14-3-3-like protein (amino acid sequence: SEQ ID NO: 578, encoding nucleotide sequence: SEQ ID NO: 577) was identified as a protein comprising any of SEQ ID NOs: 579 to 582.

Accordingly, the antigens in spot 51α in the present invention can be any protein selected from the group consisting of the proteins as defined below in (51αA-a) to (51αA-e) and (51αB):
(51αA-a) a protein comprising an amino acid sequence with deletion, substitution, insertion or addition of one or several amino acids in SEQ ID NO: 578;
(51αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 578;
(51αA-c) a protein comprising an amino acid sequence encoded by a nucleotide sequence with deletion, substitution, insertion or addition of one or several nucleotides in SEQ ID NO: 577;
(51αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 577;
(51αA-e) a protein comprising an amino acid sequence encoded by a nucleic acid that hybridizes under stringent conditions with a nucleic acid having a nucleotide sequence complementary to the nucleotide sequence of SEQ ID NO: 577;
(51αB) a protein comprising at least one amino acid sequence selected from the group consisting of SEQ ID NOs: 579-582, preferably a protein comprising at least 2 or 3 or all sequences of the amino acid sequences. As referred to above, the amino acid sequence of any of SEQ ID NOs: 579-582 may be an amino acid sequence derived therefrom by deletion, substitution, insertion or addition of one or several amino acids.

The proteins selected from the group consisting of (51αA-a) to (51αA-e) and (51αB) as defined above can be proteins that are found in a protein spot with a molecular weight of around 5 to 60 kDa, preferably around 10 to 50 kDa and an isoelectric point of 2.5 to 6.5, preferably 3.0 to 6.0 on gels used in the two-dimensional electrophoresis performed under the conditions described above in the subsection titled "Identification of antigens".

Epitopes of the antigen in spot 51α reside in amino acids 71-85 (SEQ ID NO: 928), amino acids 141-155 (SEQ ID NO: 929) and amino acids 211-225 (SEQ ID NO: 930) of SEQ ID NO: 578. The antigen in spot 51α may retain the sequence in SEQ ID NO: 578 as to amino acids at positions corresponding to the region of these epitopes.

Preferably, a protein that is an antigen in spot 51α has activity equivalent to that of the protein having the amino acid sequence of SEQ ID NO: 578 (14-3-3-like protein) or causative of an allergy to soybean.

### (52α) Antigen in spot 52α

As the result of sequence identification of the antigen in spot 52α by mass spectroscopy, the amino acid sequences of SEQ ID NOs: 585-588 were detected.

Also, the mass spectroscopic data obtained for spot 52α on a mass spectrometer was analyzed by comparing the data against the NCBI protein data, and as a result, probable fructokinase-4 (amino acid sequence: SEQ ID NO: 584, encoding nucleotide sequence: SEQ ID NO: 583) was identified as a protein comprising any of SEQ ID NOs: 585 to 588.

Accordingly, the antigens in spot 52α in the present invention can be any protein selected from the group consisting of the proteins as defined below in (52αA-a) to (52αA-e) and (52αB):
(52αA-a) a protein comprising an amino acid sequence with deletion, substitution, insertion or addition of one or several amino acids in SEQ ID NO: 584;
(52αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 584;
(52αA-c) a protein comprising an amino acid sequence encoded by a nucleotide sequence with deletion, substitution, insertion or addition of one or several nucleotides in SEQ ID NO: 583;
(52αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 583;
(52αA-e) a protein comprising an amino acid sequence encoded by a nucleic acid that hybridizes under stringent conditions with a nucleic acid having a nucleotide sequence complementary to the nucleotide sequence of SEQ ID NO: 583;
(52αB) a protein comprising at least one amino acid sequence selected from the group consisting of SEQ ID NOs: 585-588, preferably a protein comprising at least 2 or 3 or all sequences of the amino acid sequences. As referred to above, the amino acid sequence of any of SEQ ID NOs: 585-588 may be an amino acid sequence derived therefrom by deletion, substitution, insertion or addition of one or several amino acids.

The proteins selected from the group consisting of (52αA-a) to (52αA-e) and (52αB) as defined above can be proteins that are found in a protein spot with a molecular weight of around 5 to 60 kDa, preferably around 10 to 50 kDa and an isoelectric point of 3.5 to 7.5, preferably 4.0 to 7.0 on gels used in the two-dimensional electrophoresis performed under the conditions described above in the subsection titled "Identification of antigens".

Epitopes of the antigen in spot 52α reside in amino acids 11-25 (SEQ ID NO: 933), amino acids 71-85 (SEQ ID NO: 934), amino acids 121-135 (SEQ ID NO: 935), amino acids 201-215 (SEQ ID NO: 936), amino acids 211-225 (SEQ ID NO: 937), and amino acids 281-295 (SEQ ID NO: 938) of SEQ ID NO: 584. The antigen in spot 52α may retain the sequence in SEQ ID NO: 584 as to amino acids at positions corresponding to the region of these epitopes.

From the viewpoint of improving sensitivity (a degree to which a patient can be diagnosed as being positive) or specificity (a degree to which a healthy subject is not diagnosed as being positive), the antigen in spot 52α may be the following variant.

In the epitope having the sequence of SEQ ID NO: 933, sites of amino acids other than X in SEQ ID NO: 931 or 932 are important for binding to IgE antibodies from allergic patients. Thus, in another preferred mode, amino acids 11-25 of SEQ ID NO: 584 in the antigen in spot 52α may have the amino acid sequence of SEQ ID NO: 931 or 932.

Preferably, a protein that is an antigen in spot 52α has activity equivalent to that of the protein having the amino acid sequence of SEQ ID NO: 584 (probable fructokinase-4) or causative of an allergy to soybean.

### (53α) Antigen in spot 53α

As the result of sequence identification of the antigen in spot 53α by mass spectroscopy, the amino acid sequences of SEQ ID NOs: 591-604 were detected.

Also, the mass spectroscopic data obtained for spot 53α on a mass spectrometer was analyzed by comparing the data against the NCBI protein data, and as a result, triosephosphate isomerase isoform X1 (amino acid sequence: SEQ ID NO: 590, encoding nucleotide sequence: SEQ ID NO: 589) was identified as a protein comprising any of SEQ ID NOs: 591 to 597 and triosephosphate isomerase, cytosolic (amino acid sequence: SEQ ID NO: 599, encoding nucleotide sequence: SEQ ID NO: 598) was identified as a protein comprising any of SEQ ID NOs: 600 to 604.

Accordingly, the antigen in spot 53α in the present invention can be any of proteins selected from the group consisting of the proteins as defined below in (53αA1-a) to (53αA1-e), (53αB1), (53αA2-a) to (53αA2-e), and (53αB2):
(53αA1-a) a protein comprising an amino acid sequence with deletion, substitution, insertion or addition of one or several amino acids in SEQ ID NO: 590;
(53αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 590;
(53αA1-c) a protein comprising an amino acid sequence encoded by a nucleotide sequence with deletion, substitution, insertion or addition of one or several nucleotides in SEQ ID NO: 589;
(53αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 589;
(53αA1-e) a protein comprising an amino acid sequence encoded by a nucleic acid that hybridizes under stringent conditions with a nucleic acid having a nucleotide sequence complementary to the nucleotide sequence of SEQ ID NO: 589;
(53αB1) a protein comprising at least one amino acid sequence selected from the group consisting of SEQ ID NOs: 591-597, preferably a protein comprising at least 2, 3, 4, 5, or 6 or all sequences of the amino acid sequences. As referred to above, the amino acid sequence of any of SEQ ID NOs: 591 to 597 may be an amino acid sequence derived therefrom by deletion, substitution, insertion or addition of one or several amino acids; (53αA2-a) a protein comprising an amino acid sequence with deletion, substitution, insertion or addition of one or several amino acids in SEQ ID NO: 599;
(53αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 599;
(53αA2-c) a protein comprising an amino acid sequence encoded by a nucleotide sequence with deletion, substitution, insertion or addition of one or several nucleotides in SEQ ID NO: 598;
(53αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 598;
(53αA2-e) a protein comprising an amino acid sequence encoded by a nucleic acid that hybridizes under stringent conditions with a nucleic acid having a nucleotide sequence complementary to the nucleotide sequence of SEQ ID NO: 598;
(53αB2) a protein comprising at least one amino acid sequence selected from the group consisting of SEQ ID NOs: 600-604, preferably a protein comprising at least 2, 3, or 4 or all sequences of the amino acid sequences. As referred to above, the amino acid sequence of any of SEQ ID NOs: 600 to 604 may be an amino acid sequence derived therefrom by deletion, substitution, insertion or addition of one or several amino acids.

The protein selected from the group consisting of the proteins as defined above in (53αA1-a) to (53αA1-e), (53αB1), (53αA2-a) to (53αA2-e), and (53αB2) can be proteins that are found in a protein spot with a molecular weight of around 5 to 60 kDa, preferably around 10 to 50 kDa, and an isoelectric point of 3.5 to 8.5, preferably 4.0 to 8.0 on gels used in the two-dimensional electrophoresis performed under the conditions described above in the subsection titled "Identification of antigens".

Epitopes of the aforementioned antigens (53αA1-a) to (53αA1-e) and (53αB1) reside in amino acids 71-85 (SEQ ID NO: 939) of SEQ ID NO: 590. When the antigen in spot 53α contains a variation in the amino acid sequence of SEQ ID NO: 590, amino acids at positions corresponding to the region of at least one of these epitopes may retain the sequence in SEQ ID NO: 590.

Preferably, a protein that is an antigen in spot 53α has activity equivalent to that of the protein having the amino acid sequence of SEQ ID NO: 590 (triosephosphate isomerase isoform X1) or the protein having the amino acid sequence of SEQ ID NO: 599 (triosephosphate isomerase, cytosolic) or causative of an allergy to soybean.

### (54α) Antigen in spot 54α

As the result of sequence identification of the antigen in spot 54α by mass spectroscopy, the amino acid sequences of SEQ ID NOs: 607-616 were detected.

Also, the mass spectroscopic data obtained for spot 54α on a mass spectrometer was analyzed by comparing the data against the NCBI protein data, and as a result, superoxide dismutase [Fe], chloroplastic isoform X1 (amino acid sequence: SEQ ID NO: 606, encoding nucleotide sequence: SEQ ID NO: 605) as a protein comprising any of SEQ ID NOs: 607 to 611 and iron-superoxide dismutase (amino acid sequence: SEQ ID NO: 613, encoding nucleotide sequence: SEQ ID NO: 612) was identified as a protein comprising any of SEQ ID NOs: 614 to 616.

Accordingly, the antigen in spot 54α in the present invention can be any of proteins selected from the group consisting of the proteins as defined below in (54αA1-a) to (54αA1-e), (54αB1), (54αA2-a) to (54αA2-e), and (54αB2):
(54αA1-a) a protein comprising an amino acid sequence with deletion, substitution, insertion or addition of one or several amino acids in SEQ ID NO: 606;
(54αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 606;
(54αA1-c) a protein comprising an amino acid sequence encoded by a nucleotide sequence with deletion, substitution, insertion or addition of one or several nucleotides in SEQ ID NO: 605;
(54αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 605;
(54αA1-e) a protein comprising an amino acid sequence encoded by a nucleic acid that hybridizes under stringent conditions with a nucleic acid having a nucleotide sequence complementary to the nucleotide sequence of SEQ ID NO: 605;
(54αB1) a protein comprising at least one amino acid sequence selected from the group consisting of SEQ ID NOs: 607-611, preferably a protein comprising at least 2, 3, or 4 or all sequences of the amino acid sequences. As referred to above, the amino acid sequence of any of SEQ ID NOs: 591 to 597 may be an amino acid sequence derived therefrom by deletion, substitution, insertion or addition of one or several amino acids;
(54αA2-a) a protein comprising an amino acid sequence with deletion, substitution, insertion or addition of one or several amino acids in SEQ ID NO: 613;
(54αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 613;
(54αA2-c) a protein comprising an amino acid sequence encoded by a nucleotide sequence with deletion, substitution, insertion or addition of one or several nucleotides in SEQ ID NO: 612;
(54αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 612;
(54αA2-e) a protein comprising an amino acid sequence encoded by a nucleic acid that hybridizes under stringent conditions with a nucleic acid having a nucleotide sequence complementary to the nucleotide sequence of SEQ ID NO: 612;
(54αB2) a protein comprising at least one amino acid sequence selected from the group consisting of SEQ ID NOs: 614-616, preferably a protein comprising at least 2, or all sequences of the amino acid sequences. As referred to above, the amino acid sequence of any of SEQ ID NOs: 614 to 616 may be an amino acid sequence derived therefrom by deletion, substitution, insertion or addition of one or several amino acids.

The protein selected from the group consisting of the proteins as defined above in (54αA1-a) to (54αA1-e), (54αB1), (54αA2-a) to (54αA2-e), and (54αB2) can be proteins that are found in a protein spot with a molecular weight of around 5 to 60 kDa, preferably around 10 to 50 kDa, and an isoelectric point of 3.5 to 8.5, preferably 4.0 to 8.0 on gels used in the two-dimensional electrophoresis performed under the conditions described above in the subsection titled "Identification of antigens".

Epitopes of the aforementioned antigens (54αA1-a) to (54αA1-e) and (54αB1) reside in amino acids 121-135 (SEQ ID NO: 940), amino acids 148-154 (SEQ ID NO: 943) of SEQ ID NO: 606. When the antigen in spot 54α contains a variation in the amino acid sequence of SEQ ID NO: 606, amino acids at positions corresponding to the region of at least one of these epitopes may retain the sequence in SEQ ID NO: 606.

From the viewpoint of improving sensitivity (a degree to which a patient can be diagnosed as being positive) or specificity (a degree to which a healthy subject is not diagnosed as being positive), the antigen in spot 54α may be the following variant.

In the epitope having the sequence of SEQ ID NO: 943, sites of amino acids other than X in SEQ ID NO: 941 or 942 are important for binding to IgE antibodies from allergic patients. Thus, in another preferred mode, when the antigen in spot 54α contains a variation in the amino acid sequence of SEQ ID NO: 606, amino acids 148-154 of SEQ ID NO: 606 in this antigen may have the amino acid sequence of SEQ ID NO: 941 or 942.

Preferably, a protein that is an antigen in spot 54α has activity equivalent to that of the protein having the amino acid sequence of SEQ ID NO: 606 (superoxide dismutase [Fe], chloroplastic isoform X1) or the protein having the amino acid sequence of SEQ ID NO: 613 (iron-superoxide dismutase) or causative of an allergy to soybean.

### (55α) Antigen in spot 55α

As the result of sequence identification of the antigen in spot 55α by mass spectroscopy, the amino acid sequences of SEQ ID NOs: 619-649 were detected.

Also, the mass spectroscopic data obtained for spot 55α on a mass spectrometer was analyzed by comparing the data against the NCBI protein data, and as a result, 51 kDa seed maturation protein precursor (amino acid sequence: SEQ ID NO: 618, encoding nucleotide sequence: SEQ ID NO: 617) was identified as a protein comprising any of SEQ ID NOs: 619 to 635 and Lea protein precursor (amino acid sequence: SEQ ID NO: 637, encoding nucleotide sequence: SEQ ID NO: 636) was identified as a protein comprising any of SEQ ID NOs: 638 to 649.

Accordingly, the antigen in spot 55α in the present invention can be any of proteins selected from the group consisting of the proteins as defined below in (55αA1-a) to (55αA1-e), (55αB1), (55αA2-a) to (55αA2-e), and (55αB2):
(55αA1-a) a protein comprising an amino acid sequence with deletion, substitution, insertion or addition of one or several amino acids in SEQ ID NO: 618;
(55αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 618;
(55αA1-c) a protein comprising an amino acid sequence encoded by a nucleotide sequence with deletion, substitution, insertion or addition of one or several nucleotides in SEQ ID NO: 617;
(55αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 617;
(55αA1-e) a protein comprising an amino acid sequence encoded by a nucleic acid that hybridizes under stringent conditions with a nucleic acid having a nucleotide sequence complementary to the nucleotide sequence of SEQ ID NO: 617;
(55αB1) a protein comprising at least one amino acid sequence selected from the group consisting of SEQ ID NOs: 619-635, preferably a protein comprising at least 2, 3, 4, 5, 6, 7, 8, 9, 10, 11, 12, 13, 14, 15, or 16 or all sequences of the amino acid sequences. As referred to above, the amino acid sequence of any of SEQ ID NOs: 619 to 635 may be an amino acid sequence derived therefrom by deletion, substitution, insertion or addition of one or several amino acids;
(55αA2-a) a protein comprising an amino acid sequence with deletion, substitution, insertion or addition of one or several amino acids in SEQ ID NO: 637;
(55αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 637;
(55αA2-c) a protein comprising an amino acid sequence encoded by a nucleotide sequence with deletion, substitution, insertion or addition of one or several nucleotides in SEQ ID NO: 636;
(55αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 636;
(55αA2-e) a protein comprising an amino acid sequence encoded by a nucleic acid that hybridizes under stringent conditions with a nucleic acid having a nucleotide sequence complementary to the nucleotide sequence of SEQ ID NO: 636;
(55αB2) a protein comprising at least one amino acid sequence selected from the group consisting of SEQ ID NOs: 638-649, preferably a protein comprising at least 2, 3, 4, 5, 6, 7, 8, 9, 10, or 11 or all sequences of the amino acid sequences. As referred to above, the amino acid sequence of any of SEQ ID NOs: 638 to 649 may be an amino acid sequence derived therefrom by deletion, substitution, insertion or addition of one or several amino acids.

The protein selected from the group consisting of the proteins as defined above in (55αA1-a) to (55αA1-e), (55αB1), (55αA2-a) to (55αA2-e), and (55αB2) can be proteins that are found in a protein spot with a molecular weight of around 30 to 90 kDa, preferably around 40 to 80 kDa, and an isoelectric point of 5.5 to 10.5, preferably 6.0 to 10.0 on gels used in the two-dimensional electrophoresis performed under the conditions described above in the subsection titled "Identification of antigens".

Epitopes of the aforementioned antigens (55αA1-a) to (55αA1-e) and (55αB1) reside in amino acids 201-209 (SEQ ID NO: 946), amino acids 318-325 (SEQ ID NO: 949), amino acids 202-210 (SEQ ID NO: 952) of SEQ ID NO: 618. When the antigen in spot 55α contains a variation in the amino acid sequence of SEQ ID NO: 618, amino acids at positions corresponding to the region of at least one of these epitopes may retain the sequence in SEQ ID NO: 618.

From the viewpoint of improving sensitivity (a degree to which a patient can be diagnosed as being positive) or specificity (a degree to which a healthy subject is not diagnosed as being positive), the antigen in spot 55α may be the following variant.

In the epitope having the sequence of SEQ ID NO: 946, sites of amino acids other than X in SEQ ID NO: 944 or 945 are important for binding to IgE antibodies from allergic patients. Thus, in another preferred mode, when the antigen in spot 55α contains a variation in the amino acid sequence of SEQ ID NO: 618, amino acids 201-209 of SEQ ID NO: 618 in this antigen may have the amino acid sequence of SEQ ID NO: 944 or 945.

In the epitope having the sequence of SEQ ID NO: 949, sites of amino acids other than X in SEQ ID NO: 947 or 948 are important for binding to IgE antibodies from allergic patients. Thus, in another preferred mode, when the antigen in spot 55α contains a variation in the amino acid sequence of SEQ ID NO: 618, amino acids 318-325 of SEQ ID NO: 618 in this antigen may have the amino acid sequence of SEQ ID NO: 947 or 948.

In the epitope having the sequence of SEQ ID NO: 952, sites of amino acids other than X in SEQ ID NO: 950 or 951 are important for binding to IgE antibodies from allergic patients. Thus, in another preferred mode, when the antigen in spot 55α contains a variation in the amino acid sequence of SEQ ID NO: 618, amino acids 202-210 of SEQ ID NO: 618 in this antigen may have the amino acid sequence of SEQ ID NO: 950 or 951.

Preferably, a protein that is an antigen in spot 55α has activity equivalent to that of the protein having the amino acid sequence of SEQ ID NO: 618 (51 kDa seed maturation protein precursor) or the protein having the amino acid sequence of SEQ ID NO: 637 (Lea protein precursor) or causative of an allergy to soybean.

The proteins that are the aforementioned antigens (1α) to (55α) include those proteins whose amino acid residues are modified by phosphorylation, sugar chain modification, aminoacylation, ring-opening, deamination or the like.

By stating herein "deletion, substitution, insertion or addition of one or several amino acids" in relation to amino acid sequence, it is meant that in an amino acid sequence of interest, one or several amino acids (e.g., 30%, preferably 25%, 20%, 15%, 10%, 5%, 3%, 2% or 1%, of amino acids with respect to the total length of the amino acid sequence, or e.g., 1, 2, 3, 4, 5, 6, 7, 8, 9 or 10 amino acids) are deleted, one or several amino acids are substituted by any other amino acids, any other amino acids are inserted, and/or any other amino acids are added.

Among the aforementioned modifications, substitution is preferably conservative substitution. The "conservative substitution" refers to the substitution of a certain amino acid residue by a different amino acid residue having similar physicochemical characteristics, and can be any type of substitution as long as it does not substantially change the characteristics of the structure of the original sequence for example, any type of substitution is acceptable as long as any substituted amino acids do not disrupt the helical structure of the original sequence or other secondary structures that characterize the original sequence. The following gives examples of separate groups of amino acid residues that are conservatively substitutable with each other, but substitutable amino acid residues are not limited to the examples given below.
Group A: leucine, isoleucine, valine, alanine, methionine
Group B: aspartic acid, glutamic acid
Group C: asparagine, glutamine
Group D: lysine, arginine
Group E: serine, threonine
Group F: phenylalanine, tyrosine

In the case of non-conservative substitution, one member belonging to one of the aforementioned groups can be replaced with a member belong to any other group. For example, in order to eliminate the possibility of unwanted sugar-chain modification, amino acids of group B, D or E as listed above may be substituted by those of any other group. Also, cysteines may be deleted or substituted by any other amino acids to prevent them from being folded into a protein in its tertiary structure. Also, in order to maintain the balance between hydrophilicity and hydrophobicity or to increase hydrophilicity for the purpose of facilitating synthesis, any amino acids may be substituted in consideration of the hydropathy scales of amino acids, which are a measure of the hydrophilic and hydrophobic properties of amino acids (J. Kyte and R. Doolittle, J. Mol. Biol., Vol.157, p.105-132, 1982).

As referred to herein, the percent identity between two amino acid sequences can be determined by visual inspection and mathematical calculation. Alternatively, the percent identity can be determined using a computer program. Examples of such computer programs include BLAST, FASTA (Altschul, et al., J. Mol. Biol., 215: 403-410 (1990)), and ClustalW. In particular, various conditions (parameters) for identity searches with the BLAST program are described in Altschul, et al. (Nucl. Acids. Res., 25, p.3389-3402, 1997), and are publicly available on the websites of the National Center for Biotechnology Information (NCBI) and DNA Data Bank of Japan (DDBJ) (Altschul, et al., BLAST Manual, Altschul, et al., NCB/NLM/NIH Bethesda, MD 20894). Also, the percent identity can be determined using a genetic information processing software program, such as GENETYX Ver.7 (Genetyx Corporation), DNASIS Pro (Hitachi Software Engineering Co., Ltd.), or Vector NTI (Infomax Inc.).

By stating herein "deletion, substitution, insertion or addition of one or several nucleotides" in relation to nucleotide sequence, it is meant that in a nucleotide sequence of interest, one or several nucleotides (e.g., 30%, preferably 25%, 20%, 15%, 10%, 5%, 3%, 2% or 1%, of nucleotides with respect to the total length of the nucleotide sequence, or e.g., 1, 5, 10, 15, 20, 25 or 30 nucleotides) are deleted, one or several nucleotides are substituted by any other nucleotides, any other nucleotides are inserted, and/or any other nucleotides are added. It is preferable that such a nucleotide deletion, substitution, insertion or addition should not give rise to a frame shift in an amino acid coding sequence.

As referred to herein, the percent identity between two nucleotide sequences can be determined by visual inspection and mathematical calculation. Alternatively, the percent identity can be determined using a computer program. Examples of such sequence comparison computer programs include the BLASTN program, version 2.2.7, available on the website of the National Library of Medicine (http://www.ncbi.nlm.nih.gov/blast/bl2seq/bls.html) (Altschul, et al. (1990) J. Mol. Biol., 215: 403-10), or the WU-BLAST 2.0 algorithm. Standard default parameter settings for WU-BLAST 2.0 are found and available on the following website: http://blast.wustl.edu.

As referred to herein, "under stringent conditions" means that hybridization takes place under moderately or highly stringent conditions. To be specific, the moderately stringent conditions can be easily determined by those having ordinary skill in the art on the basis of, for example, the length of DNA. Basic conditions are described in Sambrook, et al., Molecular Cloning: A Laboratory Manual, 3rd ed., ch.6-7, Cold Spring Harbor Laboratory Press, 2001. The moderately stringent conditions include hybridization under the conditions of preferably 1×SSC to 6×SSC at 42°C to 55°C, more preferably 1×SSC to 3×SSC at 45°C to 50°C, most preferably 2×SSC at 50°C. In the case of using a hybridization solution containing, for example, about 50% formamide, a temperature around 5 to 15°C lower than the foregoing should be adopted. Washing is also carried out under the conditions of 0.5×SSC to 6×SSC at 40°C to 60°C. In the process of hybridization and washing, generally 0.05% to 0.2% SDS, preferably about 0.1% SDS, may be added. Likewise, the highly stringent conditions can be easily determined by those having ordinary skill in the art on the basis of, for example, the length of DNA. Generally, the highly stringent (high stringent) conditions include hybridization and/or washing at a higher temperature and/or a lower salt concentration than those adopted under the moderately stringent conditions. For example, hybridization is carried out under the conditions of O.1×SSC to 2×SSC at 55°C to 65°C, more preferably O.1×SSC to 1×SSC at 60°C to 65°C, most preferably 0.2×SSC at 63°C. Washing is carried out under the conditions of 0.2×SSC to 2×SSC at 50°C to 68°C, more preferably 0.2×SSC at 60 to 65°C.

The residue of X as to the epitopes of the aforementioned antigens (1α) to (55α) is an amino acid residue at a site where binding activity against IgE antibodies from allergic patients was maintained even after substitution by alanine in alanine scanning described in Example 4. It is well known to those skilled in the art that even when such a site is substituted by any other amino acids, it is highly probable that this binding activity against IgE antibodies is maintained.

Antigens may be obtained by separating and purifying them from soybean using a combination of protein purification methods well known to those skilled in the art. Also, antigens may be obtained by expressing them as recombinant proteins using a genetic recombination technique well known to those skilled in the art and by separating and purifying them using protein purification methods well known to those skilled in the art.

Exemplary protein purification methods include: solubility-based purification methods such as salt precipitation and solvent precipitation; purification methods based on molecular weight difference, such as dialysis, ultrafiltration, gel filtration and SDS-PAGE; charge-based purification methods such as ion exchange chromatography and hydroxylapatite chromatography; specific affinity-based purification methods such as affinity chromatography; purification methods based on hydrophobicity difference, such as reverse-phase high-performance liquid chromatography; and purification methods based on isoelectric point difference, such as isoelectric focusing.

Preparation of a protein by a genetic recombination technique is carried out by preparing an expression vector comprising an antigen-encoding nucleic acid, introducing the expression vector into appropriate host cells by gene transfer or genetic transformation, culturing the host cells under suitable conditions for expression of a recombinant protein, and recovering the recombinant protein expressed in the host cells.

The "vector" refers to a nucleic acid that can be used to introduce a nucleic acid attached thereto into host cells. The "expression vector" is a vector that can induce the expression of a protein encoded by a nucleic acid introduced therethrough. Exemplary vectors include plasmid vectors and viral vectors. Those skilled in the art can select an appropriate expression vector for the expression of a recombinant protein depending on the type of host cells to be used.

The "host cells" refers to cells that undergo gene transfer or genetic transformation by a vector. The host cells can be appropriately selected by those skilled in the art depending on the type of the vector to be used. The host cells can be derived from prokaryotes such as E. coli. When prokaryotic cells like E coli. are used as host cells, the antigen of the present invention may be designed to contain an N-terminal methionine residue in order to facilitate the expression of a recombinant protein in the prokaryotic cells. The N-terminal methionine can be cleaved from the recombinant protein after expression. Also, the host cells may be cells derived from eukaryotes, such as single-cell eukaryotes like yeast, plant cells and animal cells (e.g., human cells, monkey cells, hamster cells, rat cells, murine cells or insect cells) or silkworm.

Gene transfer or genetic transformation of an expression vector into host cells can be carried out as appropriate by following a technique known to those skilled in the art. Those skilled in the art can make possible the expression of a recombinant protein by selecting suitable conditions for the expression of the recombinant protein as appropriate depending on the type of host cells and culturing the host cells under the selected conditions. Then, those skilled in the art can homogenize the host cells having the expressed recombinant protein, and separate and purify an antigen expressed as the recombinant protein from the resulting homogenate by using an appropriate combination of such protein purification methods as mentioned above.

### Diagnosis kit and method (1)

The present invention provides a method for providing an indicator for diagnosing an allergy to a soybean in a subject, the method comprising the steps of:
(i) contacting a sample obtained from the subject with an antigen, wherein the sample is a solution comprising an Ig antibody;
(ii) detecting binding between an IgE antibody present in the sample from the subject and the antigen; and
(iii) when the binding between the IgE antibody in the subject and the antigen is detected, an indicator of the fact that the subject is allergic to a soybean is provided;
   wherein the antigen is at least one of proteins as defined above in any of (1α) to (55 α).

The sample obtained from a subject is a solution containing an Ig antibody, preferably an IgE antibody, as collected from the subject. Examples of such solutions include blood, saliva, sputum, snivel, urine, sweat, and tear. The sample obtained from the subject may be subjected to pretreatment for increasing the concentration of an Ig antibody in the sample before being contacted with an antigen. The pretreatment of a sample may involve, for example, collection of the serum or the plasma from the blood. Furthermore, a Fab moiety that is an antigen-binding moiety may be purified. In a particularly preferred mode, the step (i) mentioned above is carried out by contacting an IgE antibody present in the serum obtained from a subject with an antigen.

The IgE antibody may be the IgE antibody itself or may be mast cells or the like bound to IgE antibodies.

Detection of contact and binding between the sample obtained from a subject and an antigen can be carried out by using a known method. Examples of such methods that can be used include detection by ELISA (Enzyme-Linked Immunosorbent Assay), sandwich immunoassay, immunoblotting, immunoprecipitation, or immunochromatography. These are all techniques for detecting binding between an antigen and an IgE antibody from a subject by contacting and binding the IgE antibody from a subject with the antigen, allowing an enzymatically labelled secondary antibody to act on the IgE antibody specifically bound to the antigen, and adding an enzyme substrate (generally, chromogenic or luminescent reagent) to detect an enzymatic reaction product. Also, a method for detecting a fluorescently labeled secondary antibody can be used. Alternatively, detection by a measurement method capable of evaluating binding between an antigen and an IgE antibody, such as surface plasmon resonance (SPR), can also be used. A plurality of antigen-specific IgE antibodies may be mixed.

The antigen may be provided as an isolated antigen in a state immobilized on a carrier. In this case, the steps (i) and (ii) mentioned above can be carried out using ELISA, sandwich immunoassay, immunochromatography, surface plasmon resonance, or the like. Also, the step (i) mentioned above can be carried out by contacting the sample obtained from a subject with an antigen-immobilized surface. The isolated antigen may be obtained by separating and purifying it from soybean using a combination of protein purification methods well known to those skilled in the art, or by preparing it using a genetic recombination technique. The IgE antibody from the subject may be used in a state immobilized on a carrier, and binding to the antigen may be detected by the aforementioned technique.

The antigen may be in a state unimmobilized on a carrier. In this case, flow cytometry or the like can be used in the aforementioned steps (i) and (ii), and the presence of the antigen bound to the antibody can be confirmed with laser beam. Examples include a basophil activation test (BAT). Another example includes a histamine release test (HRT) which examines whether histamine is released by further contacting an antigen with blood cells in a sample.

The antigen may be detected by immunoblotting in a state separated by two-dimensional electrophoresis. The two-dimensional electrophoresis is a technique for separating a protein sample by performing isoelectric focusing in the first dimension and performing SDS-PAGE (SDS-polyacrylamide gel electrophoresis) in the second dimension. The conditions for two-dimensional electrophoresis are not particularly limited as long as the conditions permit the separation of the antigen of the present invention. For example, the conditions for two-dimensional electrophoresis as described above in the subsection titled "Identification of antigens" can be adopted. Also, the electrophoresis conditions may be defined by reference to the descriptions in Patent Literatures 1 to 4 mentioned above. For example, two-dimensional electrophoresis can be carried out under the conditions that satisfy at least one selected from the group consisting of the following requirements:
(A) the isoelectric focusing gels used in the first dimension should have a gel-strip length of 5 to 10 cm and a gel pH range of 3 to 10, and the pH gradient of the gels in the direction of electrophoresis should be as follows: where the gel-strip length up to pH 5 is taken as "a", that length from pH 5 to 7 as "b", and that length above pH 7 as "c", the relations "a < b" and "b > c" are satisfied;
(B) in the case of (A), when the total gel-strip length is taken as 1, "a" should be in the range of 0.15 to 0.3, "b" should be in the range of 0.4 to 0.7, and "c" should be in the range of 0.15 to 0.3;
(C) in the first dimensional isoelectric focusing, a constant voltage step should be performed by applying a constant voltage ranging from 100 V to 600 V per gel strip containing a sample, and after the electrophoresis variation width during electrophoresis for 30 minutes falls within the range of 5 µA, a voltage-increasing step should be started at which the voltage is increased from the aforementioned constant voltage;
(D) in the case of (C), the final voltage at the voltage-increasing step should be in the range of 3000 V to 6000 V;
(E) the isoelectric focusing gels used in the first dimension should have a longitudinal gel-strip length of 5 to 10 cm, and the electrophoresis gels used in the second dimension should have a gel concentration at the distal end in the direction of electrophoresis, which is in the range of 3 to 6%; and
(F) in the case of (E), the electrophoresis gels used in the second dimension should have a gel concentration at the proximal end in the direction of electrophoresis, which is set to a higher value than that at the distal end in the direction of electrophoresis.

The aforementioned antigens (1α) to (55α) are antigens that specifically bind to IgE antibodies from patients with allergy to soybean. Therefore, when binding between an IgE antibody from a subject and the antigen is detected, an indicator of the fact that the subject is allergic to soybean is provided.

The present invention further provides a kit for diagnosing an allergy to a soybean, comprising at least one of the aforementioned antigens (1α) to (55α). The diagnosis kit of this invention may be used in the aforementioned method for providing an indicator for diagnosing an allergy to a soybean or in a diagnosis method as described later. The diagnosis kit of this invention may comprise not only the at least one of the aforementioned antigens (1α) to (55α), but also an anti-IgE antibody labeled with an enzyme and a chromogenic or luminescent substrate serving as a substrate for the enzyme. Also, a fluorescent-labeled anti-IgE antibody may be used. In the diagnosis kit of this invention, the antigen may be provided in a state immobilized on a carrier. The diagnosis kit of this invention may also be provided together with instructions on the procedure for diagnosis or a package containing said instructions.

In another mode, the aforementioned diagnosis kit comprises a companion diagnostic agent for an allergy to a soybean. The companion diagnostic agent is used for identifying patients expected to respond to pharmaceutical products or identifying patients having the risk of severe adverse reactions to pharmaceutical products, or for studying the reactivity of pharmaceutical products in order to optimize treatment using the pharmaceutical products. Here, the optimization of treatment includes, for example, determination of dosage and administration, judgment regarding discontinuation of administration, and confirmation of an allergen component that is used to cause immunological tolerance.

The present invention further provides a composition for diagnosing an allergy to a soybean, comprising at least one of the aforementioned antigens (1α) to (55α). The diagnosis composition of this invention can be used in a diagnosis method as described below. The diagnosis composition of this invention may further comprise a pharmaceutically acceptable carrier and/or additives commonly used with the antigen of this invention depending on the need.

In one mode, the present invention provides a method for diagnosing an allergy to a soybean in a subject, the method comprising:
(i) contacting a sample obtained from the subject with an antigen;
(ii) detecting binding between an IgE antibody present in the sample from the subject and the antigen; and
(iii) when the binding between the IgE antibody in the subject and the antigen is detected, diagnosing the subject as being allergic to a soybean;
   wherein the antigen is at least one of proteins as defined above in any of (1α) to (55α). In this method, the steps (i) and (ii) are performed as described above regarding the corresponding steps of the method for providing an indicator for diagnosing an allergy to a soybean.

In another mode, the present invention provides a method for diagnosing an allergy to a soybean in a subject, the method comprising administering to the subject at least one of the aforementioned antigens (1α) to (55α). This method may be performed in the form of a skin test characterized by applying the antigen onto the skin. Examples of the skin test include various forms of tests, such as: a prick test in which a diagnosis composition is applied onto the skin and then a tiny prick to such an extent as not to provoke bleeding is made in the skin to allow an antigen to penetrate the skin, thereby observing a skin reaction; a scratch test in which a diagnosis composition is applied onto the skin and then the skin is lightly scratched to observe a reaction; a patch test in which a diagnosis composition in the form of cream, ointment, etc. is applied onto the skin to observe a reaction; and an intracutaneous test in which an antigen is administered intracutaneously to observe a reaction. If a skin reaction such as swelling occurs in a skin portion to which the antigen has been applied, the subject of interest is diagnosed as having an allergy to a soybean. The amount of the antigen to be applied to the skin in such tests can be, for example, not more than 100 µg per dose.

In the process of allergy diagnosis, a load test aiming to identify an antigen is often adopted. At least one of the aforementioned antigens (1α) to (55α) can be used as an active ingredient for a load test to diagnose an allergy to a soybean. Here, the antigen protein to be used in the load test may be a protein that has been expressed and purified and may be a protein that has been expressed in food or cooking ingredients, such as rice-based vaccine expressing pollen allergens which are obtained by transforming rice with a gene of a cedar pollen antigen and expressing the antigen protein in the rice.

In yet another mode, the present invention provides at least one of the aforementioned antigens (1α) to (55α), intended for use in the diagnosis of an allergy to a soybean. This also includes the provision of at least one of the aforementioned antigens (1α) to (55α) mixed with a known antigen.

In still another mode, the present invention provides use of at least one of the aforementioned antigens (1α) to (55α) for the production of a diagnostic agent for an allergy to a soybean.

### Pharmaceutical composition and treatment method (1)

The present invention provides a pharmaceutical composition comprising at least one of the aforementioned antigens (1α) to (55α). In one mode, the aforementioned pharmaceutical composition is used for the treatment and diagnosis of an allergy to a soybean.

The present invention also provides a method for treating an allergy to a soybean, the method comprising administering at least one of the aforementioned antigens (1α) to (55α) to a patient in need of a treatment for an allergy to a soybean.

In another mode, the present invention provides at least one of the aforementioned antigens (1α) to (55α), intended for use in the treatment for an allergy to a soybean. In yet another mode, the present invention provides use of at least one of the aforementioned antigens (1α) to (55α) for the production of a therapeutic agent for an allergy to a soybean.

In the process of allergy treatment, a hyposensitization therapy aiming to induce immunological tolerance by administering an antigen to a patient is often adopted. The at least one of the aforementioned antigens (1α) to (55α) can be used as an active ingredient for a hyposensitization therapy for an allergy to a soybean. Here, the antigen protein to be used in the hyposensitization therapy may be a protein that has been expressed and purified and may be a protein that has been expressed in food or cooking ingredients, such as rice-based vaccine expressing pollen allergens which are obtained by transforming rice with a gene of a cedar pollen antigen and expressing the antigen protein in the rice.

The pharmaceutical composition of the present invention can be administered by common administration routes. Examples of common administration routes include oral, sublingual, percutaneous, intracutaneous, subcutaneous, intravascular, intranasal, intramuscular, intraperitoneal, and intrarectal administrations.

The pharmaceutical composition of the present invention can be used as a pharmaceutical composition to which a commonly used pharmaceutically acceptable adjuvant or excipient or any other additives (e.g., stabilizer, solubilizer, emulsifier, buffer, preservative, colorant) are added by a conventional method together with the antigen of this invention depending on the need. The dosage form of the pharmaceutical composition can be selected by those skilled in the art as appropriate depending on the administration route. The pharmaceutical composition can be in the form of, for example, tablet, capsule, troche, sublingual tablet, parenteral injection, intranasal spray, poultice, solution, cream, or lotion. The administration dose, frequency and/or period of the pharmaceutical composition of this invention can be selected by a physician as appropriate depending on the administration route and the patient's condition and characteristics such as age and body weight. For example, the pharmaceutical composition may be administered to an adult patient at a dose of not more than 100 µg per dose. The administration interval can be, for example, once daily, once weekly, twice weekly, once per three months or so. The administration period can be, for example, several weeks to several years. The pharmaceutical composition may be administered in such a manner that the dose is increased in incremental steps over the administration period.

### Tester (1)

The present invention provides a tester comprising an antibody for at least one of the aforementioned antigens (1α) to (55α).

The antibody can be prepared by a conventional method. For example, the antibody may be prepared by immunizing a mammal such as rabbit with one of the aforementioned antigens (1α) to (55α). The antibody may be an Ig antibody, a polyclonal antibody, a monoclonal antibody, or an antigen-binding fragment thereof (e.g., Fab, F(ab')₂, Fab').

Further, in the aforementioned tester, the antibody may be provided in a form bound to a carrier. The type of the carrier is not particularly limited as long as it is usable for detection of binding between an antibody and an antigen. Any given carrier known to those skilled in the art can be used.

Examples of a method for determining the presence or absence of an antigen include the following methods:
a method in which a prepared tester comprising an IgE antibody is contacted with a sample obtained from a food, a cooking ingredient, etc., ELISA or the like method is used to detect whether there is a binding between the IgE antibody and an antigen in the sample, and if the binding between the IgE antibody and the antigen is detected, it is determined that the antibody remains in the food, the cooking ingredient, etc. of interest; and
a method in which filter paper is impregnated with a food or a cooking ingredient and reacted with an antibody solution so as to detect an antigen contained therein.

Another mode of the present invention includes a tester for determining the presence or absence of an antigen of an allergy to a soybean in an object of interest, the tester comprising a primer having a nucleotide sequence complementary to a portion of at least one of the nucleotide sequences of SEQ ID NOs: 1, 3, 5, 7, 9, 11, 13, 15, 69, 88, 105, 121, 138, 154, 158, 162, 168, 173, 188, 200, 203, 219, 232, 238, 241, 253, 279, 285, 290, 305, 319, 326, 333, 340, 347, 357, 366, 383, 393, 410, 417, 421, 428, 434, 440, 446, 452, 455, 459, 467, 472, 478, 484, 488, 494, 514, 517, 521, 524, 532, 540, 546, 552, 555, 559, 564, 571, 577, 583, 589, 598, 605, 612, 617 and 636. The primer has, for example, but not limited to, a nucleotide sequence complementary to, preferably, 12 residues, 15 bases, 20 bases, or 25 bases, in a 3'-terminal or central sequence in a partial sequence of at least one of the nucleotide sequences of SEQ ID NOs defined above in this paragraph. Particularly, when mRNA is of interest, the tester has a complementary primer of a poly-A tail. In a preferred mode, the tester comprising the primer mentioned above may further comprise a primer comprising a 5'-terminal nucleotide sequence, preferably a nucleotide sequence of 12 bases, 15 bases, 20 bases, or 25 bases, of at least one of the nucleotide sequences of SEQ ID NOs defined above in this paragraph.

For example, cDNA is amplified by PCR (Polymerase Chain Reaction) including RT-PCR using templated DNA or mRNA obtained from a soybean and the aforementioned complementary primer, and the sequence of the amplified cDNA is compared with the nucleotide sequences of SEQ ID NOs defined above in the previous paragraph to determine the presence or absence of the antigen. Amplification methods by PCR can be exemplified by RACE. In this respect, even if there exists a point mutation encoding the same amino acid in the comparison of the amplified cDNA with the nucleotide sequences of SEQ ID NOs defined above in the previous paragraph, or even if the nucleotide sequence of the amplified cDNA has insertion, deletion, substitution or addition of bases in the nucleotide sequences of SEQ ID NOs defined above in the previous paragraph, it is determined that the antigen is present when the amino acid sequence encoded by the cDNA has at least 70%, preferably at least 80, 90, 95, 98, or 99% identity to the amino acid sequence of SEQ ID NO: 2, 4, 6, 8, 10, 12, 14, 16, 70, 89, 106, 122, 139, 155, 159, 163, 169, 174, 189, 201, 204, 220, 233, 239, 242, 254, 280, 286, 291, 306, 320, 327, 334, 341, 348, 358, 367, 384, 394, 411, 418, 422, 429, 435, 441, 447, 453, 456, 460, 468, 473, 479, 485, 489, 495, 515, 518, 522, 525, 533, 541, 547, 553, 556, 560, 565, 572, 578, 584, 590, 599, 606, 613, 618 or 637.

In one mode, the aforementioned tester is used to determine the presence or absence of an antigen in cooking ingredients or in products of interest in a food production line. The tester may also be used for quality inspection of production lines and pre-shipment products by manufacturers, or may be used for self-checking of the presence or absence of an antigen in a food or cooking ingredients of interest by consumers.

### Allergen-free food and the like (1)

The present invention provides a soybean or processed product of soybean in which at least one of the aforementioned antigens (1α) to (55α) is eliminated or reduced.

The method for eliminating or reducing the antigen of the present invention in a soybean or processed products of soybean is not limited. The elimination or reduction of the antigen may be conducted by any method, as long as the method permits the elimination or reduction of the antigen.

For example, the soybean of the present invention whose antigen is eliminated or reduced may be obtained by preparing a soybean in which the expression of the antigen of the present invention is knocked out, using a gene knock-out technique. Any technique known to those skilled in the art such as genetic modification can be used as the gene knock-out technique.

An antigen of the present invention may be the artefact that an antigen of the present invention assumed the removal or a reduced soybean raw material as for the removal or the reduced processed products of soybean. In the case of using an ordinary soybean as a source ingredient, a treatment for removing or reducing the antigen of this invention is performed before or after preparation of a processed product of soybean. The methods for removing or reducing the antigen of the present invention in the processed products of soybean which assumed an ordinary soybean raw material include a method to remove protein component in food such as a high pressure treatment and elution with the neutral salt solution or the high temperature steam, and a method to perform hydrolysis, denaturation, or amino acid alteration (chemical modification, elimination, or the like of a side chain) by heat treatment and acid treatment.

### Method for producing antigen-free processed product of soybean

The present invention provides a method for producing a processed product of soybean in which an antigen is eliminated or reduced, the method comprising the step of confirming that the antigen is eliminated or reduced, in a production process of the processed product, wherein the antigen is at least one of the aforementioned antigens (1α) to (55α).

The step of confirming that the antigen is eliminated or reduced, in a production process of the processed product of soybean in which an antigen is eliminated or reduced may be performed by confirming the presence or absence of an antigen by the method described above in the subsection titled "Tester (1)".

The production of the processed product of soybean in which an antigen is eliminated or reduced may be performed by the method described above in the subsection titled "Allergen-free food and the like (1)".

### Epitope of antigen

Epitopes and amino acids important for binding activity against IgE antibodies from allergic patients within the epitopes were identified as shown in Examples 4 and 5 as to antigens identified as shown in Example 3 and known antigens Gly m 4, Gly m 5, Gly m 6 and Gly m 8.

The present invention provides polypeptides of the following (1β) to (60β) as polypeptides comprising an amino acid sequence specifically binding to an IgE antibody from an allergic patient.

### (1β) Epitope of endoplasmin homolog isoform X2 (spot 1α)

In the present invention, the polypeptide (1β) can be any polypeptide selected from the group consisting of (1β-1) to (1β-4) as defined below:
(1β-1) a polypeptide comprising at least one amino acid sequence selected from the group consisting of SEQ ID NOs: 652, 655 and 657-660;
(1β-2) a polypeptide comprising the amino acid sequence of XXKXEXXX (SEQ ID NO: 650), preferably a polypeptide comprising the amino acid sequence of XTKXEXXX (SEQ ID NO: 651), more preferably a polypeptide comprising an amino acid sequence in which any 1, 2, 3, 4 or 5 of the amino acids at positions 1, 2, 4, and 6-8 of SEQ ID NO: 652 are substituted by alanine, further preferably a polypeptide comprising an amino acid sequence in which any 1, 2, 3 or 4 of the amino acids at positions 1, 4, and 6-8 of SEQ ID NO: 652 are substituted by other amino acids;
(1β-3) a polypeptide comprising the amino acid sequence of XXXKDNVXX (SEQ ID NO: 653), preferably a polypeptide comprising the amino acid sequence of XLXKDNVXX (SEQ ID NO: 654), more preferably a polypeptide comprising an amino acid sequence in which any 1, 2, 3, 4 or 5 of the amino acids at positions 1-3, 8, and 9 of SEQ ID NO: 655 are substituted by alanine, further preferably a polypeptide comprising an amino acid sequence in which any 1, 2, 3 or 4 of the amino acids at positions 1, 3, 8, and 9 of SEQ ID NO: 655 are substituted by other amino acids; and
(1β-4) a polypeptide comprising the amino acid sequence of EEEDDXXXXXEXXXX (SEQ ID NO: 656), more preferably a polypeptide comprising an amino acid sequence in which any 1, 2, 3, 4, 5, 6, 7, 8 or 9 of the amino acids at positions 6-10 and 12-15 of SEQ ID NO: 657 are substituted by alanine, further preferably a polypeptide comprising an amino acid sequence in which any 1, 2, 3, 4, 5, 6, 7, 8 or 9 of the amino acids at positions 6-10 and 12-15 of SEQ ID NO: 657 are substituted by other amino acids.

### (2β) Epitope of heat shock cognate protein 80 (spot 2α)

In the present invention, the polypeptide (2β) can be any polypeptide selected from the group consisting of 2β-1) to (2β-9) as defined below:
(2β-1) a polypeptide comprising at least one amino acid sequence selected from the group consisting of SEQ ID NOs: 663, 666, 669, 670-672, 675, 676-678, 681, 684, 687, 689 and 690-692;
(2β-2) a polypeptide comprising the amino acid sequence of DXXDKXXXX (SEQ ID NO: 661), preferably a polypeptide comprising the amino acid sequence of DXXDKXRXX (SEQ ID NO: 662), more preferably a polypeptide comprising an amino acid sequence in which any 1, 2, 3, 4, 5 or 6 of the amino acids at positions 2, 3, and 6-9 of SEQ ID NO: 663 are substituted by alanine, further preferably a polypeptide comprising an amino acid sequence in which any 1, 2, 3, 4 or 5 of the amino acids at positions 2, 3, 6, 8, and 9 of SEQ ID NO: 663 are substituted by other amino acids;
(2β-3) a polypeptide comprising the amino acid sequence of XXXXKSKLXXXXXXX (SEQ ID NO: 664), preferably a polypeptide comprising the amino acid sequence of XXXXKSKLXXXPXXX (SEQ ID NO: 665), more preferably a polypeptide comprising an amino acid sequence in which any 1, 2, 3, 4, 5, 6, 7, 8, 9, 10 or 11 of the amino acids at positions 1-4, and 9-15 of SEQ ID NO: 666 are substituted by alanine, further preferably a polypeptide comprising an amino acid sequence in which any 1, 2, 3, 4, 5, 6, 7, 8, 9 or 10 of the amino acids at positions 1-4, 9-11, and 13-15 of SEQ ID NO: 666 are substituted by other amino acids;
(2β-4) a polypeptide comprising the amino acid sequence of XPDKX (SEQ ID NO: 667), preferably a polypeptide comprising the amino acid sequence of IPDKX (SEQ ID NO: 668), more preferably a polypeptide comprising an amino acid sequence in which any 1 or 2 of the amino acids at positions 1 and 5 of SEQ ID NO: 669 are substituted by alanine, further preferably a polypeptide comprising an amino acid sequence in which any 1 or 2 of the amino acids at positions 1 and 5 of SEQ ID NO: 669 are substituted by other amino acids; (2β-5) a polypeptide comprising the amino acid sequence of XXFSKXXXXXXXEDX (SEQ ID NO: 673), preferably a polypeptide comprising the amino acid sequence of XXFSKXXXXXXXEDS (SEQ ID NO: 674), more preferably a polypeptide comprising an amino acid sequence in which any 1, 2, 3, 4, 5, 6, 7, 8, 9 or 10 of the amino acids at positions 1, 2, 6-12 and 15 of SEQ ID NO: 675 are substituted by alanine, further preferably a polypeptide comprising an amino acid sequence in which any 1, 2, 3, 4, 5, 6, 7, 8, or 9 of the amino acids at positions 1, 2 and 6-12 of SEQ ID NO: 675 are substituted by other amino acids;
(2β-6) a polypeptide comprising the amino acid sequence of XXXXDXXXXEXXXDX (SEQ ID NO: 679), preferably a polypeptide comprising the amino acid sequence of XXXXDKIRXEXXXDK (SEQ ID NO: 680), more preferably a polypeptide comprising an amino acid sequence in which any 1, 2, 3, 4, 5, 6, 7, 8, 9, 10, 11 or 12 of the amino acids at positions 1-4, 6-9, 11-13 and 15 of SEQ ID NO: 681 are substituted by alanine, further preferably a polypeptide comprising an amino acid sequence in which any 1, 2, 3, 4, 5, 6, 7 or 8 of the amino acids at positions 1-4, 9, 11-13 of SEQ ID NO: 681 are substituted by other amino acids;
(2β-7) a polypeptide comprising the amino acid sequence of XYXXRXXXXQNXXXX (SEQ ID NO: 682), preferably a polypeptide comprising an amino acid sequence in which any 1, 2, 3, 4, 5, 6, 7, 8, 9, 10 or 11 of the amino acids at positions 1, 3, 4, 6-9, and 12-15 of SEQ ID NO: 684 are substituted by alanine, more preferably a polypeptide comprising an amino acid sequence in which any 1, 2, 3, 4, 5, 6, 7, 8, 9, 10 or 11 of the amino acids at positions 1, 3, 4, 6-9, and 12-15 of SEQ ID NO: 684 are substituted by other amino acids; (2β-8) a polypeptide comprising the amino acid sequence of DXXXRXXXXQNXXXX (SEQ ID NO: 685), preferably a polypeptide comprising an amino acid sequence in which any 1, 2, 3, 4, 5, 6, 7, 8, 9, 10 or 11 of the amino acids at positions 2-4, 6-9, and 12-15 of SEQ ID NO: 687 are substituted by alanine, more preferably a polypeptide comprising an amino acid sequence in which any 1, 2, 3, 4, 5, 6, 7, 8, 9, 10 or 11 of the amino acids at positions 2-4, 6-9, and 12-15 of SEQ ID NO: 687 are substituted by other amino acids; (2β-9) a polypeptide comprising the amino acid sequence of RKPXXXXXXXXAAXX (SEQ ID NO: 688), preferably a polypeptide comprising an amino acid sequence in which any 1, 2, 3, 4, 5, 6, 7, 8, 9, or 10 of the amino acids at positions 4-11, 14 and 15 of SEQ ID NO: 689 are substituted by alanine, more preferably a polypeptide comprising an amino acid sequence in which any 1, 2, 3, 4, 5, 6, 7, 8, 9, or 10 of the amino acids at positions 4-11, 14 and 15 of SEQ ID NO: 689 are substituted by other amino acids.

### (3β) Epitope of Cell division cycle protein 48 homolog (spot 3α)

In the present invention, the polypeptide (3β) can be any polypeptide selected from the group consisting of (3β-1) to (3β-4) as defined below:
(3β-1) a polypeptide comprising at least one amino acid sequence selected from the group consisting of SEQ ID NOs: 695, 697, 699 and 700-716;
(3β-2) a polypeptide comprising the amino acid sequence of XXXXXXXXXXRLXEX (SEQ ID NO: 693), preferably a polypeptide comprising the amino acid sequence of XXXLKREDEERLDEV (SEQ ID NO: 694), more preferably a polypeptide comprising an amino acid sequence in which any 1, 2, 3, 4, 5, 6, 7, 8, 9, 10, 11 or 12 of the amino acids at positions 1-10, 13 and 15 of SEQ ID NO: 695 are substituted by alanine, further preferably a polypeptide comprising an amino acid sequence in which any 1, 2 or 3 of the amino acids at positions 1-3 of SEQ ID NO: 695 are substituted by other amino acids;
(3β-3) a polypeptide comprising the amino acid sequence of XXXXXXXXXXXXXDE (SEQ ID NO: 696), preferably a polypeptide comprising an amino acid sequence in which any 1, 2, 3, 4, 5, 6, 7, 8, 9, 10, 11, 12 or 13 of the amino acids at positions 1-13 of SEQ ID NO: 697 are substituted by alanine, more preferably a polypeptide comprising an amino acid sequence in which any 1, 2, 3, 4, 5, 6, 7, 8, 9, 10, 11, 12 or 13 of the amino acids at positions 1-13 of SEQ ID NO: 697 are substituted by other amino acids;
(3β-4) a polypeptide comprising the amino acid sequence of XXXXGDR (SEQ ID NO: 698), preferably a polypeptide comprising an amino acid sequence in which any 1, 2, 3 or 4 of the amino acids at positions 1-4 of SEQ ID NO: 699 are substituted by alanine, more preferably a polypeptide comprising an amino acid sequence in which any 1, 2, 3 or 4 of the amino acids at positions 1-4 of SEQ ID NO: 699 are substituted by other amino acids.

In one mode, the polypeptide (3β) may not comprise the full-length amino acid sequence (SEQ ID NO: 16) of Cell division cycle protein 48 homolog or a variant or a homolog having 100% or at least 90%, at least 80% or at least 70% identity to the total length of this amino acid sequence.

SEQ ID NOs: 695, 697, 699 and 700-716 correspond to amino acids 191-205 (SEQ ID NO: 695), amino acids 21-35 (SEQ ID NO: 697), amino acids 779-785 (SEQ ID NO: 699), amino acids 8-14 (SEQ ID NO: 700), amino acids 49-56 (SEQ ID NO: 701), amino acids 101-107 (SEQ ID NO: 702), amino acids 161-175 (SEQ ID NO: 703), amino acids 291-305 (SEQ ID NO: 704), amino acids 316-323 (SEQ ID NO: 705), amino acids 327-333 (SEQ ID NO: 706), amino acids 341-355 (SEQ ID NO: 707), amino acids 361-369 (SEQ ID NO: 708), amino acids 431-445 (SEQ ID NO: 709), amino acids 451-465 (SEQ ID NO: 710), amino acids 501-515 (SEQ ID NO: 711), amino acids 565-572 (SEQ ID NO: 712), amino acids 631-645 (SEQ ID NO: 713), amino acids 703-712 (SEQ ID NO: 714), amino acids 711-715 (SEQ ID NO: 715), and amino acids 721-735 (SEQ ID NO: 716), respectively, of SEQ ID NO: 16.

### (4β) Epitope of embryo-specific urease isoform X1 (spot 4α)

In the present invention, the polypeptide (4β) can be any polypeptide selected from the group consisting of (4β-1) to (4β-5) as defined below:
(4β-1) a polypeptide comprising at least one amino acid sequence selected from the group consisting of SEQ ID NOs: 719, 722,725, 726-728 and 731;
(4β-2) a polypeptide comprising the amino acid sequence of XXXXXXXKED (SEQ ID NO: 717), preferably a polypeptide comprising the amino acid sequence of XXXXXEXKED (SEQ ID NO: 718), more preferably a polypeptide comprising an amino acid sequence in which any 1, 2, 3, 4, 5, 6 or 7 of the amino acids at positions 1-7 of SEQ ID NO: 719 are substituted by alanine, further preferably a polypeptide comprising an amino acid sequence in which any 1, 2, 3, 4, 5 or 6 of the amino acids at positions 1-5 and 7 of SEQ ID NO: 719 are substituted by other amino acids;
(4β-3) a polypeptide comprising the amino acid sequence of KXXLGDX (SEQ ID NO: 720), preferably a polypeptide comprising the amino acid sequence of KIXLGDX (SEQ ID NO: 721), more preferably a polypeptide comprising an amino acid sequence in which any 1, 2 or 3 of the amino acids at positions 2, 3 and 7 of SEQ ID NO: 722 are substituted by alanine, further preferably a polypeptide comprising an amino acid sequence in which any 1, 2 or 3 of the amino acids at positions 3 and 7 of SEQ ID NO: 722 are substituted by other amino acids; (4β-4) a polypeptide comprising the amino acid sequence of XXXDXFXKXE (SEQ ID NO: 723), preferably a polypeptide comprising the amino acid sequence of XXXDXFXKIE (SEQ ID NO: 724), more preferably a polypeptide comprising an amino acid sequence in which any 1, 2, 3, 4, 5 or 6 of the amino acids at positions 1-3, 5, 7 and 9 of SEQ ID NO: 725 are substituted by alanine, further preferably a polypeptide comprising an amino acid sequence in which any 1, 2, 3, 4 or 5 of the amino acids at positions 1-3, 5 and 7 of SEQ ID NO: 725 are substituted by other amino acids;
(4β-5) a polypeptide comprising the amino acid sequence of XXXHXLXXXNKXXEA (SEQ ID NO: 729), preferably a polypeptide comprising the amino acid sequence of XXVHXLXXXNKXXEA (SEQ ID NO: 730), more preferably a polypeptide comprising an amino acid sequence in which any 1, 2, 3, 4, 5, 6, 7, 8 or 9 of the amino acids at positions 1-3, 5, 7-9,12 and 13 of SEQ ID NO: 731 are substituted by alanine, further preferably a polypeptide comprising an amino acid sequence in which any 1, 2, 3, 4, 5, 6, 7 or 8 of the amino acids at positions 1, 2, 5, 7-9, 12 and 13 of SEQ ID NO: 731 are substituted by other amino acids.

### (5β) alpha-1,4 glucan phosphorylase L isozyme, chloroplastic/amyloplastic (spot 5α)

In the present invention, the polypeptide (5β) can be any polypeptide selected from the group consisting of (5β-1) to (5β-2) as defined below:
(5β-1) a polypeptide comprising at least one amino acid sequence selected from the group consisting of SEQ ID NOs: 734 and 735-738;
(5β-2) a polypeptide comprising the amino acid sequence of XXXXXXGLXKXRXEX (SEQ ID NO: 732), preferably a polypeptide comprising the amino acid sequence of XXXXXXGLXKXRXEG (SEQ ID NO: 733), more preferably a polypeptide comprising an amino acid sequence in which any 1, 2, 3, 4, 5, 6, 8, 9 or 10 of the amino acids at positions 1-6, 9, 11, 13 and 15 of SEQ ID NO: 734 are substituted by alanine, further preferably a polypeptide comprising an amino acid sequence in which any 1, 2, 3, 4, 5, 6, 7, 8 or 9 of the amino acids at positions 1-6, 9, 11 and 13 of SEQ ID NO: 734 are substituted by other amino acids.

### (6β) Epitope of aconitate hydratase 1 (spot 6α)

In the present invention, the polypeptide (6β) can be a polypeptide comprising at least one amino acid sequence selected from the group consisting of SEQ ID NOs: 739 and 740.

### (7β) Epitope of methionine synthase (spot 7α)

In the present invention, the polypeptide (7β) can be any polypeptide selected from the group consisting of (7β-1) to (7β-2) as defined below:
(7β-1) a polypeptide comprising at least one amino acid sequence selected from the group consisting of SEQ ID NOs: 741 and 744;
(7β-2) a polypeptide comprising the amino acid sequence of XXXXXXXKDXVEDXE (SEQ ID NO: 742), preferably a polypeptide comprising the amino acid sequence of XXXXXXXKDEVEDXE (SEQ ID NO: 743), more preferably a polypeptide comprising an amino acid sequence in which any 1, 2, 3, 4, 5, 6, 7, 8 or 9 of the amino acids at positions 1-7, 10 and 14 of SEQ ID NO: 744 are substituted by alanine, further preferably a polypeptide comprising an amino acid sequence in which any 1, 2, 3, 4, 5, 6, 7 or 8 of the amino acids at positions 1-7 and 14 of SEQ ID NO: 744 are substituted by other amino acids.

### (9β) Epitope of lysine--tRNA ligase, cytoplasmic-like isoform X2 (spot 9α)

In the present invention, the polypeptide (9β) can be any polypeptide selected from the group consisting of 9β-1) to (9β-3) as defined below:
(9β-1) a polypeptide comprising at least one amino acid sequence selected from the group consisting of SEQ ID NOs: 747 and 749;
(9β-2) a polypeptide comprising the amino acid sequence of XXEXXXXKDX (SEQ ID NO: 745), preferably a polypeptide comprising the amino acid sequence of NXEXXXXKDX (SEQ ID NO: 746), more preferably a polypeptide comprising an amino acid sequence in which any 1, 2, 3, 4, 5, 6 or 7 of the amino acids at positions 1, 2, 4-7 and 10 of SEQ ID NO: 747 are substituted by alanine, further preferably a polypeptide comprising an amino acid sequence in which any 1, 2, 3, 4, 5 or 6 of the amino acids at positions 2, 4-7 and 10 of SEQ ID NO: 747 are substituted by other amino acids;
(9β-3) a polypeptide comprising the amino acid sequence of LDFLEXET (SEQ ID NO: 748), preferably a polypeptide comprising an amino acid sequence in which the amino acid at position 6 of SEQ ID NO: 749 is substituted by alanine, more preferably a polypeptide comprising an amino acid sequence in which the amino acid at position 6 of SEQ ID NO: 749 is substituted by another amino acid.

### (11β) Epitope of polyadenylate-binding protein 8 isoform X3 (spot 11α)

In the present invention, the polypeptide (11β) can be any polypeptide selected from the group consisting of (11β-1) to (11β-2) as defined below:
(11β-1) a polypeptide comprising at least one amino acid sequence selected from the group consisting of SEQ ID NOs: 753 and 754;
(11β-2) a polypeptide comprising the amino acid sequence of PLXQQXXXXX (SEQ ID NO: 751), preferably a polypeptide comprising the amino acid sequence of PLXQQGXXXX (SEQ ID NO: 752), more preferably a polypeptide comprising an amino acid sequence in which any 1, 2, 3, 4, 5 or 6 of the amino acids at positions 3 and 6-10 of SEQ ID NO: 753 are substituted by alanine, further preferably a polypeptide comprising an amino acid sequence in which any 1, 2, 3, 4 or 5 of the amino acids at positions 3 and 7-10 of SEQ ID NO: 753 are substituted by other amino acids.

### (12β) Epitope of pyrophosphate--fructose 6-phosphate 1-phosphotransferase subunit alpha-like (spot 12α)

In the present invention, the polypeptide (12β) can be a polypeptide comprising the amino acid sequence of SEQ ID NO: 755.

### (13β) Epitope of protein disulfide isomerase-like protein precursor (spot 13α)

In the present invention, the polypeptide (13β) can be any polypeptide selected from the group consisting of (13β-1) to (13β-2) as defined below:
(13β-1) a polypeptide comprising the amino acid sequence of SEQ ID NO: 758;
(13β-2) a polypeptide comprising the amino acid sequence of XXGXXXXXXXXXXXE (SEQ ID NO: 756), preferably a polypeptide comprising the amino acid sequence of XXGXLXXXXGXRTXE (SEQ ID NO: 757), more preferably a polypeptide comprising an amino acid sequence in which any 1, 2, 3, 4, 5, 6, 7, 8, 9, 10, 11, 12 or 13 of the amino acids at positions 1, 2 and 4-14 of SEQ ID NO: 758 are substituted by alanine, further preferably a polypeptide comprising an amino acid sequence in which any 1, 2, 3, 4, 5, 6, 7, 8 or 9 of the amino acids at positions 1, 2, 4, 6-9, 11 and 14 of SEQ ID NO: 758 are substituted by other amino acids.

### (14β) Epitope of V-type proton ATPase subunit B 2 (spot 14α)

In the present invention, the polypeptide (14β) can be any polypeptide selected from the group consisting of (14β-1) to (14β-3) as defined below:
(14β-1) a polypeptide comprising at least one amino acid sequence selected from the group consisting of SEQ ID NOs: 761 and 764;
(14β-2) a polypeptide comprising the amino acid sequence of XXXXXLLXXXXEEDN (SEQ ID NO: 759), preferably a polypeptide comprising the amino acid sequence of XXXXXLLEXXXEEDN (SEQ ID NO: 760), more preferably a polypeptide comprising an amino acid sequence in which any 1, 2, 3, 4, 5, 6, 7, 8 or 9 of the amino acids at positions 1-5 and 8-11 of SEQ ID NO: 761 are substituted by alanine, further preferably a polypeptide comprising an amino acid sequence in which any 1, 2, 3, 4, 5, 6, 7 or 8 of the amino acids at positions 1-5 and 9-11 of SEQ ID NO: 761 are substituted by other amino acids;
(14β-3) a polypeptide comprising the amino acid sequence of RKXXXXXXXXXYXNX (SEQ ID NO: 762), preferably a polypeptide comprising the amino acid sequence of RKDHSXXXXXLYANX (SEQ ID NO: 763), more preferably a polypeptide comprising an amino acid sequence in which any 1, 2, 3, 4, 5, 6, 7, 8, 9, 10 or 11 of the amino acids at positions 3-11, 13 and 15 of SEQ ID NO: 764 are substituted by alanine, further preferably a polypeptide comprising an amino acid sequence in which any 1, 2, 3, 4, 5 or 6 of the amino acids at positions 6-10 and 15 of SEQ ID NO: 764 are substituted by other amino acids.

### (15β) Epitope of UTP-glucose-1-phosphate uridylyltransferase (spot 15α, 16α)

In the present invention, the polypeptide (15β) can be any polypeptide selected from the group consisting of (15β-1) to (15β-2) as defined below:
(15β-1) a polypeptide comprising the amino acid sequence of SEQ ID NO: 766;
(15β-2) a polypeptide comprising the amino acid sequence of XGKXXXXXXXXXXXX (SEQ ID NO: 765), preferably a polypeptide comprising an amino acid sequence in which any 1, 2, 3, 4, 5, 6, 7, 8, 9, 10, 11, 12 or 13 of the amino acids at positions 1 and 4-15 of SEQ ID NO: 766 are substituted by alanine, more preferably a polypeptide comprising an amino acid sequence in which any 1, 2, 3, 4, 5, 6, 7, 8, 9, 10, 11, 12 or 13 of the amino acids at positions 1 and 4-15 of SEQ ID NO: 766 are substituted by other amino acids.

### (17β) Epitope of guanosine nucleotide diphosphate dissociation inhibitor 2 (spot 17α)

In the present invention, the polypeptide (17β) can be a polypeptide comprising the amino acid sequence of SEQ ID NO: 767.

### (18β) Epitope of hypothetical protein GLYMA_10G028300 (spot 18α)

In the present invention, the polypeptide (18β) can be any polypeptide selected from the group consisting of (18β-1) to (18β-3) as defined below:
(18β-1) a polypeptide comprising at least one amino acid sequence selected from the group consisting of SEQ ID NOs: 750, 768, 771, 772 and 775;
(18β-2) a polypeptide comprising the amino acid sequence of XXXXXXXNXXIFEED (SEQ ID NO: 769), preferably a polypeptide comprising the amino acid sequence of XXXXXXXNXYIFEED (SEQ ID NO: 770), more preferably a polypeptide comprising an amino acid sequence in which any 1, 2, 3, 4, 5, 6, 7, 8 or 9 of the amino acids at positions 1-7, 9 and 10 of SEQ ID NO: 771 are substituted by alanine, further preferably a polypeptide comprising an amino acid sequence in which any 1, 2, 3, 4, 5, 6, 7 or 8 of the amino acids at positions 1-7 and 9 of SEQ ID NO: 771 are substituted by other amino acids;
(18β-3) a polypeptide comprising the amino acid sequence of XELPREXRXX (SEQ ID NO: 773), preferably a polypeptide comprising the amino acid sequence of XELPREERXX (SEQ ID NO: 774), more preferably a polypeptide comprising an amino acid sequence in which any 1, 2, 3 or 4 of the amino acids at positions 1, 7, 9 and 10 of SEQ ID NO: 775 are substituted by alanine, further preferably a polypeptide comprising an amino acid sequence in which any 1, 2 or 3 of the amino acids at positions 1, 9 and 10 of SEQ ID NO: 775 are substituted by other amino acids.

### (19β) Epitope of sucrose binding protein homolog S-64 (spot 19α)

In the present invention, the polypeptide (19β) can be any polypeptide selected from the group consisting of (19β-1) to (19β-5) as defined below:
(19β-1) a polypeptide comprising at least one amino acid sequence selected from the group consisting of SEQ ID NOs: 778, 781, 784, 786 and 787;
(19β-2) a polypeptide comprising the amino acid sequence of XXXXXPLYXXXXDEX (SEQ ID NO: 776), preferably a polypeptide comprising the amino acid sequence of XXXXXPLYIXXXDEX (SEQ ID NO: 777), more preferably a polypeptide comprising an amino acid sequence in which any 1, 2, 3, 4, 5, 6, 7, 8, 9 or 10 of the amino acids at positions 1-5, 9-12 and 15 of SEQ ID NO: 778 are substituted by alanine, further preferably a polypeptide comprising an amino acid sequence in which any 1, 2, 3, 4, 5, 6, 7, 8 or 9 of the amino acids at positions 1-5, 10-12 and 15 of SEQ ID NO: 778 are substituted by other amino acids;
(19β-3) a polypeptide comprising the amino acid sequence of XXXXXXXXRXRRXXX (SEQ ID NO: 779), preferably a polypeptide comprising the amino acid sequence of XXXXXSXXRXRRXXX (SEQ ID NO: 780), more preferably a polypeptide comprising an amino acid sequence in which any 1, 2, 3, 4, 5, 6, 7, 8, 9, 10, 11 or 12 of the amino acids at positions 1-8, 10 and 13-15 of SEQ ID NO: 781 are substituted by alanine, further preferably a polypeptide comprising an amino acid sequence in which any 1, 2, 3, 4, 5, 6, 7, 8, 9, 10 or 11 of the amino acids at positions 1-5, 7, 8, 10 and 13-15 of SEQ ID NO: 782 are substituted by other amino acids;
(19β-4) a polypeptide comprising the amino acid sequence of XXXXXXXXXXEEEXX (SEQ ID NO: 782), preferably a polypeptide comprising the amino acid sequence of XKEEEHQEXXEEEQX (SEQ ID NO: 783), more preferably a polypeptide comprising an amino acid sequence in which any 1, 2, 3, 4, 5, 6, 7, 8, 9, 10, 11 or 12 of the amino acids at positions 1-10, 14 and 15 of SEQ ID NO: 784 are substituted by alanine, further preferably a polypeptide comprising an amino acid sequence in which any 1, 2, 3 or 4 of the amino acids at positions 1, 9, 10 and 15 of SEQ ID NO: 784 are substituted by other amino acids; (19β-5) a polypeptide comprising the amino acid sequence of XXGKXXXXXXXXXXX (SEQ ID NO: 785), preferably a polypeptide comprising an amino acid sequence in which any 1, 2, 3, 4, 5, 6, 7, 8, 9, 10, 11, 12 or 13 of the amino acids at positions 1, 2 and 5-15 of SEQ ID NO: 786 are substituted by alanine, more preferably a polypeptide comprising an amino acid sequence in which any 1, 2, 3, 4, 5, 6, 7, 8, 9, 10, 11, 12 or 13 of the amino acids at positions 1, 2 and 5-15 of SEQ ID NO: 786 are substituted by other amino acids.

### (20β) Epitope of Seed biotin-containing protein SBP65 (spot 20α)

In the present invention, the polypeptide (20β) can be a polypeptide comprising at least one amino acid sequence selected from the group consisting of SEQ ID NOs: 788-792.

In one mode, the polypeptide (20β) may not comprise the full-length amino acid sequence (SEQ ID NO: 12) of Seed biotin-containing protein SBP65 or a variant or a homolog having 100% or at least 90%, at least 80% or at least 70% identity to the total length of this amino acid sequence.

SEQ ID NOs: 788-792 correspond to amino acids 131-145 (SEQ ID NO: 788), amino acids 251-258 (SEQ ID NO: 789), amino acids 281-295 (SEQ ID NO: 790), amino acids 441-448 (SEQ ID NO: 791), and amino acids 531-545 (SEQ ID NO: 792), respectively, of SEQ ID NO: 12.

### (21β) Epitope of Ribulose bisphosphate carboxylase large chain (spot 21α)

In the present invention, the polypeptide (21β) can be a polypeptide comprising at least one amino acid sequence selected from the group consisting of SEQ ID NOs: 793-802.

In one mode, the polypeptide (21β) may not comprise the full-length amino acid sequence (SEQ ID NO: 4) of Ribulose bisphosphate carboxylase large chain or a variant or a homolog having 100% or at least 90%, at least 80% or at least 70% identity to the total length of this amino acid sequence.

SEQ ID NOs: 793-802 correspond to amino acids 31-34 (SEQ ID NO: 793), amino acids 101-108 (SEQ ID NO: 794), amino acids 201-209 (SEQ ID NO: 795), amino acids 221-235 (SEQ ID NO: 796), and amino acids 237-245 (SEQ ID NO: 797), and amino acids 291-295 (SEQ ID NO: 798), amino acids 351-365 (SEQ ID NO: 799), amino acids 411-417 (SEQ ID NO: 800), amino acids 431-445 (SEQ ID NO: 801), and amino acids 461-475 (SEQ ID NO: 802), respectively, of SEQ ID NO: 4.

### (22β) Epitope of Gamma-glutamyl hydrolase (spot 22α)

In the present invention, the polypeptide (22β) can be a polypeptide comprising at least one amino acid sequence selected from the group consisting of SEQ ID NOs: 803-808.

In one mode, the polypeptide (22β) may not comprise the full-length amino acid sequence (SEQ ID NO: 6) of Gamma-glutamyl hydrolase or a variant or a homolog having 100% or at least 90%, at least 80% or at least 70% identity to the total length of this amino acid sequence.

SEQ ID NOs: 803-808 correspond to amino acids 61-75 (SEQ ID NO: 803), amino acids 161-175 (SEQ ID NO: 804), amino acids 241-255 (SEQ ID NO: 805), amino acids 261-275 (SEQ ID NO: 806), amino acids 288-294 (SEQ ID NO: 807), and amino acids 301-315 (SEQ ID NO: 808), respectively, of SEQ ID NO: 6.

### (23β) Epitope of Guanine nucleotide-binding protein subunit beta-like protein (spot 23α)

In the present invention, the polypeptide (23β) can be a polypeptide comprising at least one amino acid sequence selected from the group consisting of SEQ ID NOs: 809-812.

In one mode, the polypeptide (23β) may not comprise the full-length amino acid sequence (SEQ ID NO: 8) of Guanine nucleotide-binding protein subunit beta-like protein or a variant or a homolog having 100% or at least 90%, at least 80% or at least 70% identity to the total length of this amino acid sequence.

SEQ ID NOs: 809-812 correspond to amino acids 41-55 (SEQ ID NO: 809), amino acids 111-125 (SEQ ID NO: 810), amino acids 227-235 (SEQ ID NO: 811), and amino acids 270-278 (SEQ ID NO: 812), respectively, of SEQ ID NO: 8.

### (24β) Epitope of Protein SLE3 (spot 24α)

In the present invention, the polypeptide (24β) can be a polypeptide comprising the amino acid sequence of SEQ ID NO: 813.

In one mode, the polypeptide (24β) may not comprise the full-length amino acid sequence (SEQ ID NO: 10) of Protein SLE3 or a variant or a homolog having 100% or at least 90%, at least 80% or at least 70% identity to the total length of this amino acid sequence.

SEQ ID NO: 813 corresponds to amino acids 71-85 of SEQ ID NO: 10.

### (25β) Epitope of succinyl-CoA ligase [ADP-forming] subunit beta, mitochondrial (spot 25α)

In the present invention, the polypeptide (25β) can be a polypeptide comprising at least one amino acid sequence selected from the group consisting of SEQ ID NOs: 814 and 815.

### (26β) Epitope of phosphoglycerate kinase, cytosolic (spot 26α)

In the present invention, the polypeptide (26β) can be any polypeptide selected from the group consisting of 26β-1) and (26β-2) as defined below:
(26β-1) a polypeptide comprising at least one amino acid sequence selected from the group consisting of SEQ ID NOs: 816 and 819; and
(26β-2) a polypeptide comprising the amino acid sequence of FDKXXXX (SEQ ID NO: 817), preferably a polypeptide comprising the amino acid sequence of FDKFXXX (SEQ ID NO: 818), more preferably a polypeptide comprising an amino acid sequence in which any 1, 2, 3 or 4 of the amino acids at positions 4 to 7 of SEQ ID NO: 819 are substituted by alanine, further preferably a polypeptide comprising an amino acid sequence in which any 1, 2 or 3 of the amino acids at positions 5 to 7 of SEQ ID NO: 819 are substituted by other amino acids.

### (27β) Epitope of alcohol dehydrogenase 1 (spot 27α)

In the present invention, the polypeptide (27β) can be a polypeptide comprising the amino acid sequence of SEQ ID NO: 820.

### (28β) Epitope of 35 kDa seed maturation protein isoform X1 (spot 28α)

In the present invention, the polypeptide (28β) can be any polypeptide selected from the group consisting of (28β-1) to (28β-4) as defined below:
(28β-1) a polypeptide comprising at least one amino acid sequence selected from the group consisting of SEQ ID NOs: 823, 826, 829 and 830-835;
(28β-2) a polypeptide comprising the amino acid sequence of XQXXXXENKP (SEQ ID NO: 821), preferably a polypeptide comprising the amino acid sequence of EQXQXXENKP (SEQ ID NO: 822), more preferably a polypeptide comprising an amino acid sequence in which any 1, 2, 3, 4 or 5 of the amino acids at positions 1 and 3-6 of SEQ ID NO: 823 are substituted by alanine, further preferably a polypeptide comprising an amino acid sequence in which any 1, 2 or 3 of the amino acids at positions 3, 5 and 6 of SEQ ID NO: 823 are substituted by other amino acids;
(28β-3) a polypeptide comprising the amino acid sequence of XKXXXXEKXXXXXXX (SEQ ID NO: 824), preferably a polypeptide comprising the amino acid sequence of XKDXXXEKXXXXXDX (SEQ ID NO: 825), more preferably a polypeptide comprising an amino acid sequence in which any 1, 2, 3, 4, 5, 6, 7, 8, 9, 10, 11 or 12 of the amino acids at positions 1, 3-6 and 9-15 of SEQ ID NO: 826 are substituted by alanine, further preferably a polypeptide comprising an amino acid sequence in which any 1, 2, 3, 4, 5, 6, 7, 8, 9 or 10 of the amino acids at positions 1, 4-6, 9-13 and 15 of SEQ ID NO: 826 are substituted by other amino acids;
(28β-4) a polypeptide comprising the amino acid sequence of XXXDXXREEXXXXRX (SEQ ID NO: 827), preferably a polypeptide comprising the amino acid sequence of XXXDXXREEEEXXRX (SEQ ID NO: 828), more preferably a polypeptide comprising an amino acid sequence in which any 1, 2, 3, 4, 5, 6, 7, 8, 9 or 10 of the amino acids at positions 1-3, 5, 6, 10-13 and 15 of SEQ ID NO: 829 are substituted by alanine, further preferably a polypeptide comprising an amino acid sequence in which any 1, 2, 3, 4, 5, 6, 7 or 8 of the amino acids at positions 1-3, 5, 6, 12, 13 and 15 of SEQ ID NO: 829 are substituted by other amino acids.

### (29β) Epitope of glyceraldehyde-3-phosphate dehydrogenase isoform X1 (spot 29α, 30α, 31α, 32α, 33α)

In the present invention, the polypeptide (29β) can be any polypeptide selected from the group consisting of (29β-1) to (29β-2) as defined below:
(28β-1) a polypeptide comprising at least one amino acid sequence selected from the group consisting of SEQ ID NOs: 838 and 839;
(28β-2) a polypeptide comprising the amino acid sequence of XXXXXXXDKP (SEQ ID NO: 836), preferably a polypeptide comprising the amino acid sequence of XXXXXFXDKP (SEQ ID NO: 837), more preferably a polypeptide comprising an amino acid sequence in which any 1, 2, 3, 4, 5, 6 or 7 of the amino acids at positions 1-7 of SEQ ID NO: 838 are substituted by alanine, further preferably a polypeptide comprising an amino acid sequence in which any 1, 2, 3, 4, 5 or 6 of the amino acids at positions 1-5 and 7 of SEQ ID NO: 838 are substituted by other amino acids.

### (34β) Epitope of bifunctional UDP-glucose 4-epimerase and UDP-xylose 4-epimerase 1-like (spot 34α)

In the present invention, the polypeptide (34β) can be any polypeptide selected from the group consisting of 34β-1) to (34β-2) as defined below:
(34β-1) a polypeptide comprising at least one amino acid sequence selected from the group consisting of SEQ ID NOs: 842 and 843-846;
(34β-2) a polypeptide comprising the amino acid sequence of XXXXXXXXXXXDDXE (SEQ ID NO: 840), preferably a polypeptide comprising the amino acid sequence of LXXXXXXXXXXDDXE (SEQ ID NO: 841), more preferably a polypeptide comprising an amino acid sequence in which any 1, 2, 3, 4, 5, 6, 7, 8, 9, 10, 11 or 12 of the amino acids at positions 1-11 and 14 of SEQ ID NO: 842 are substituted by alanine, further preferably a polypeptide comprising an amino acid sequence in which any 1, 2, 3, 4, 5, 6, 7, 8, 9, 10 or 11 of the amino acids at positions 2-11 and 14 of SEQ ID NO: 842 are substituted by other amino acids.

### (35β) Epitope of elongation factor 1-alpha (spot 35α)

In the present invention, the polypeptide (35β) can be any polypeptide selected from the group consisting of (35β-1) to (35β-4) as defined below:
(35β-1) a polypeptide comprising at least one amino acid sequence selected from the group consisting of SEQ ID NOs: 849, 850, 853 and 856;
(35β-2) a polypeptide comprising the amino acid sequence of XXXXDKXXXERXXXE (SEQ ID NO: 847), preferably a polypeptide comprising the amino acid sequence of XXXXDKRVXERXXXE (SEQ ID NO: 848), more preferably a polypeptide comprising an amino acid sequence in which any 1, 2, 3, 4, 5, 6, 7, 8, 9 or 10 of the amino acids at positions 1-4, 7-9 and 12-14 of SEQ ID NO: 849 are substituted by alanine, further preferably a polypeptide comprising an amino acid sequence in which any 1, 2, 3, 4, 5, 6, 7 or 8 of the amino acids at positions 1-4, 9 and 12-14 of SEQ ID NO: 849 are substituted by other amino acids;
(35β-3) a polypeptide comprising the amino acid sequence of PISXFEXXX (SEQ ID NO: 851), preferably a polypeptide comprising the amino acid sequence of PISGFEXXX (SEQ ID NO: 852), more preferably a polypeptide comprising an amino acid sequence in which any 1, 2, 3, 4 or 5 of the amino acids at positions 4 and 7-9 of SEQ ID NO: 853 are substituted by alanine, further preferably a polypeptide comprising an amino acid sequence in which any 1, 2 or 3 of the amino acids at positions 7-9 of SEQ ID NO: 853 are substituted by other amino acids;
(35β-4) a polypeptide comprising the amino acid sequence of XXEXLDXXXX (SEQ ID NO: 854), preferably a polypeptide comprising the amino acid sequence of LLEXLDXXXX (SEQ ID NO: 855), more preferably a polypeptide comprising an amino acid sequence in which any 1, 2, 3, 4, 5, 6 or 7 of the amino acids at positions 1, 2, 4 and 7-10 of SEQ ID NO: 856 are substituted by alanine, further preferably a polypeptide comprising an amino acid sequence in which any 1, 2, 3, 4 or 5 of the amino acids at positions 4 and 7-10 of SEQ ID NO: 856 are substituted by other amino acids.

### (36β) Epitope of seed maturation protein PM34 (spot 36α, 37α)

In the present invention, the polypeptide (36β) can be any polypeptide selected from the group consisting of 36β-1) to (36β-2) as defined below:
(36β-1) a polypeptide comprising at least one amino acid sequence selected from the group consisting of SEQ ID NOs: 857, 858 and 861;
(36β-2) a polypeptide comprising the amino acid sequence of KXXXXQQQ (SEQ ID NO: 859), preferably a polypeptide comprising the amino acid sequence of KXXXQQQQ (SEQ ID NO: 860), more preferably a polypeptide comprising an amino acid sequence in which any 1, 2, 3 or 4 of the amino acids at positions 2-5 of SEQ ID NO: 861 are substituted by alanine, further preferably a polypeptide comprising an amino acid sequence in which any 1, 2 or 3 of the amino acids at positions 2-4 of SEQ ID NO: 861 are substituted by other amino acids.

### (38β) Epitope of seed biotin-containing protein SBP65-like isoform X1 (spot 38α)

In the present invention, the polypeptide (38β) can be any polypeptide selected from the group consisting of (38β-1) to (38β-13) as defined below:
(38β-1) a polypeptide comprising at least one amino acid sequence selected from the group consisting of SEQ ID NOs: 864, 867, 869, 871, 874, 877, 880, 882, 884, 887, 889 and 892;
(38β-2) a polypeptide comprising the amino acid sequence of RGKXXXX (SEQ ID NO: 862), preferably a polypeptide comprising an amino acid sequence in which any 1, 2, 3 or 4 of the amino acids at positions 4-7 of SEQ ID NO: 864 are substituted by alanine, further preferably a polypeptide comprising an amino acid sequence in which any 1, 2, 3 or 4 of the amino acids at positions 4-7 of SEQ ID NO: 864 are substituted by other amino acids;
(38β-3) a polypeptide comprising the amino acid sequence of XRGKXXXXXX (SEQ ID NO: 865), preferably a polypeptide comprising the amino acid sequence of RRGKXXXXXX (SEQ ID NO: 866), more preferably a polypeptide comprising an amino acid sequence in which any 1, 2, 3, 4, 5, 6 or 7 of the amino acids at positions 1 and 5-10 of SEQ ID NO: 867 are substituted by alanine, further preferably a polypeptide comprising an amino acid sequence in which any 1, 2, 3, 4, 5 or 6 of the amino acids at positions 5-10 of SEQ ID NO: 867 are substituted by other amino acids;
(38β-4) a polypeptide comprising the amino acid sequence of RXKXXXXXXXXXXXE (SEQ ID NO: 868), preferably a polypeptide comprising an amino acid sequence in which any 1, 2, 3, 4, 5, 6, 7, 8, 9, 10, 11 or 12 of the amino acids at positions 2 and 4-14 of SEQ ID NO: 869 are substituted by alanine, further preferably a polypeptide comprising an amino acid sequence in which any 1, 2, 3, 4, 5, 6, 7, 8, 9, 10, 11 or 12 of the amino acids at positions 2 and 4-14 of SEQ ID NO: 869 are substituted by other amino acids;
(38β-5) a polypeptide comprising the amino acid sequence of XXXXXXXRRXXXXXR (SEQ ID NO: 870), preferably a polypeptide comprising an amino acid sequence in which any 1, 2, 3, 4, 5, 6, 7, 8, 9, 10, 11 or 12 of the amino acids at positions 1-7 and 10-14 of SEQ ID NO: 871 are substituted by alanine, further preferably a polypeptide comprising an amino acid sequence in which any 1, 2, 3, 4, 5, 6, 7, 8, 9, 10, 11 or 12 of the amino acids at positions 1-7 and 10-14 of SEQ ID NO: 871 are substituted by other amino acids;
(38β-6) a polypeptide comprising the amino acid sequence of XXXKGRVVXEXXXXX (SEQ ID NO: 872), preferably a polypeptide comprising the amino acid sequence of LPDKGRVVXESEKKE (SEQ ID NO: 873), more preferably a polypeptide comprising an amino acid sequence in which any 1, 2, 3, 4, 5, 6, 7, 8 or 9 of the amino acids at positions 1-3, 9 and 11-15 of SEQ ID NO: 874 are substituted by alanine, further preferably a polypeptide comprising an amino acid sequence in which any 1, 2, 3, 4, 5, 6, 7, 8 or 9 of the amino acids at positions 1-3, 9 and 11-15 of SEQ ID NO: 874 are substituted by other amino acids;
(38β-7) a polypeptide comprising the amino acid sequence of ATKAKDXXREXXXXX (SEQ ID NO: 875), preferably a polypeptide comprising the amino acid sequence of ATKAKDVTREXXXXX (SEQ ID NO: 876), more preferably a polypeptide comprising an amino acid sequence in which any 1, 2, 3, 4, 5, 6 or 7 of the amino acids at positions 7, 8 and 11-15 of SEQ ID NO: 877 are substituted by alanine, further preferably a polypeptide comprising an amino acid sequence in which any 1, 2, 3, 4 or 5 of the amino acids at positions 11-15 of SEQ ID NO: 877 are substituted by other amino acids;
(38β-8) a polypeptide comprising the amino acid sequence of XGXKGQXKKXXXXXX (SEQ ID NO: 878), preferably a polypeptide comprising the amino acid sequence of XGXKGQTKKXXGEEQ (SEQ ID NO: 879), more preferably a polypeptide comprising an amino acid sequence in which any 1, 2, 3, 4, 5, 6, 7, 8 or 9 of the amino acids at positions 1, 3, 7 and 10-15 of SEQ ID NO: 880 are substituted by alanine, further preferably a polypeptide comprising an amino acid sequence in which any 1, 2, 3 or 4 of the amino acids at positions 1, 3, 10 and 11 of SEQ ID NO: 880 are substituted by other amino acids;
(38β-9) a polypeptide comprising the amino acid sequence of RXXXETKEGX (SEQ ID NO: 881), preferably a polypeptide comprising an amino acid sequence in which any 1, 2, 3 or 4 of the amino acids at positions 2-4 and 10 of SEQ ID NO: 882 are substituted by alanine, further preferably a polypeptide comprising an amino acid sequence in which any 1, 2, 3 or 4 of the amino acids at positions 2-4 and 10 of SEQ ID NO: 882 are substituted by other amino acids;
(38β-10) a polypeptide comprising the amino acid sequence of XXXXNKQSSL (SEQ ID NO: 883), preferably a polypeptide comprising an amino acid sequence in which any 1, 2, 3 or 4 of the amino acids at positions 1-4 of SEQ ID NO: 884 are substituted by alanine, further preferably a polypeptide comprising an amino acid sequence in which any 1, 2, 3 or 4 of the amino acids at positions 1-4 of SEQ ID NO: 884 are substituted by other amino acids;
(38β-11) a polypeptide comprising the amino acid sequence of XKXVAXXXXXXXXEX (SEQ ID NO: 885), preferably a polypeptide comprising the amino acid sequence of XKXVAEXXXQXASEL (SEQ ID NO: 886), more preferably a polypeptide comprising an amino acid sequence in which any 1, 2, 3, 4, 5, 6, 7, 8, 9, 10 or 11 of the amino acids at positions 1, 3, 6-13 and 15 of SEQ ID NO: 887 are substituted by alanine, further preferably a polypeptide comprising an amino acid sequence in which any 1, 2, 3, 4, 5 or 6 of the amino acids at positions 1, 3, 7-9 and 11 of SEQ ID NO: 887 are substituted by other amino acids;
(38β-12) a polypeptide comprising the amino acid sequence of XXKVEAEXXX (SEQ ID NO: 888), preferably a polypeptide comprising an amino acid sequence in which any 1, 2, 3, 4 or 5 of the amino acids at positions 1, 2 and 8-10 of SEQ ID NO: 889 are substituted by alanine, further preferably a polypeptide comprising an amino acid sequence in which any 1, 2, 3, 4 or 5 of the amino acids at positions 1, 2 and 8-10 of SEQ ID NO: 889 are substituted by other amino acids;
(38β-13) a polypeptide comprising the amino acid sequence of XXXXIAEIAE (SEQ ID NO: 890), preferably a polypeptide comprising the amino acid sequence of XXETIAEIAE (SEQ ID NO: 891), more preferably a polypeptide comprising an amino acid sequence in which any 1, 2, 3 or 4 of the amino acids at positions 1-4 of SEQ ID NO: 892 are substituted by alanine, further preferably a polypeptide comprising an amino acid sequence in which any 1 or 2 of the amino acids at positions 1 and 2 of SEQ ID NO: 892 are substituted by other amino acids.

### (39β) Epitope of ATP synthase subunit O, mitochondrial-like (spot 39α)

In the present invention, the polypeptide (39β) can be any polypeptide selected from the group consisting of (39β-1) to (39β-2) as defined below:
(39β-1) a polypeptide comprising the amino acid sequence of SEQ ID NOs: 895;
(39β-2) a polypeptide comprising the amino acid sequence of XYXXSXKXXXXEKVX (SEQ ID NO: 893), preferably a polypeptide comprising the amino acid sequence of XYIXSXKXXXXEKVX (SEQ ID NO: 894), more preferably a polypeptide comprising an amino acid sequence in which any 1, 2, 3, 4, 5, 6, 7, 8 or 9 of the amino acids at positions 1, 3, 4, 6, 8-11 and 15 of SEQ ID NO: 895 are substituted by alanine, further preferably a polypeptide comprising an amino acid sequence in which any 1, 2, 3, 4, 5, 6, 7 or 8 of the amino acids at positions 1, 4, 6, 8-11 and 15 of SEQ ID NO: 895 are substituted by other amino acids.

### (40β) Epitope of P24 oleosin isoform A (spot 40α, 56α)

In the present invention, the polypeptide (40β) can be any polypeptide selected from the group consisting of 40β-1) to (40β-3) as defined below:
(40β-1) a polypeptide comprising at least one amino acid sequence selected from the group consisting of SEQ ID NOs: 898, 899 and 902;
(40β-2) a polypeptide comprising the amino acid sequence of YEXGV (SEQ ID NO: 896), preferably a polypeptide comprising the amino acid sequence of YEXGV (SEQ ID NO: 897), more preferably a polypeptide comprising an amino acid sequence in which any 1 or 2 of the amino acids at positions 3 and 6 of SEQ ID NO: 898 are substituted by alanine, further preferably a polypeptide comprising an amino acid sequence in which any 1 or 2 of the amino acids at positions 3 and 6 of SEQ ID NO: 898 are substituted by other amino acids; (40β-3) a polypeptide comprising the amino acid sequence of XXXXXVTAX (SEQ ID NO: 900), preferably a polypeptide comprising the amino acid sequence of VXXXXVTAX (SEQ ID NO: 901), more preferably a polypeptide comprising an amino acid sequence in which any 1, 2, 3, 4, 5 or 6 of the amino acids at positions 1-5 and 9 of SEQ ID NO: 902 are substituted by alanine, further preferably a polypeptide comprising an amino acid sequence in which any 1, 2, 3, 4 or 5 of the amino acids at positions 2-5 and 9 of SEQ ID NO: 902 are substituted by other amino acids.

### (41β) Epitope of uncharacterized protein At3g49720-like isoform X2 (spot 41α)

In the present invention, the polypeptide (41β) can be a polypeptide comprising the amino acid sequence of SEQ ID NO: 903.

### (42β) Epitope of superoxide dismutase [Mn], mitochondrial (spot 42α, 43α)

In the present invention, the polypeptide (42β) can be any polypeptide selected from the group consisting of 42β-1) to (42β-2) as defined below:
(42β-1) a polypeptide comprising the amino acid of SEQ ID NOs: 906;
(42β-2) a polypeptide comprising the amino acid sequence of XXWXXXXXXXEKEXX (SEQ ID NO: 904), preferably a polypeptide comprising the amino acid sequence of XXWXXXXEXXEKESX (SEQ ID NO: 905), more preferably a polypeptide comprising an amino acid sequence in which any 1, 2, 3, 4, 5, 6, 7, 8, 9, 10 or 11 of the amino acids at positions 1, 2, 4-10, 14 and 15 of SEQ ID NO: 906 are substituted by alanine, further preferably a polypeptide comprising an amino acid sequence in which any 1, 2, 3, 4, 5, 6, 7, 8 or 9 of the amino acids at positions 1, 2, 4-7, 9, 10 and 15 of SEQ ID NO: 906 are substituted by other amino acids.

### (44β) Epitope of Ferritin-2 (spot 44α)

In the present invention, the polypeptide (44β) can be any polypeptide selected from the group consisting of 44β-1) to (44β-2) as defined below:
(44β-1) a polypeptide comprising the amino acid of SEQ ID NOs: 909;
(44β-2) a polypeptide comprising the amino acid sequence of XXXXEEEREHAXQLI (SEQ ID NO: 907), preferably a polypeptide comprising an amino acid sequence in which any 1, 2, 3, 4 or 5 of the amino acids at positions 1-4 and 12 of SEQ ID NO: 909 are substituted by alanine, more preferably a polypeptide comprising an amino acid sequence in which any 1, 2, 3, 4 or 5 of the amino acids at positions 1-4 and 12 of SEQ ID NO: 909 are substituted by other amino acids.

In one mode, the polypeptide (44β) may not comprise the full-length amino acid sequence (SEQ ID NO: 14) of Ferritin-2 or a variant or a homolog having 100% or at least 90%, at least 80% or at least 70% identity to the total length of this amino acid sequence.

SEQ ID NO: 909 corresponds to amino acids 131-145 of SEQ ID NO: 14.

### (45β) Epitope of uncharacterized protein LOC100499771 (spot 45α)

In the present invention, the polypeptide (45β) can be a polypeptide comprising at least one amino acid sequence selected from the group consisting of SEQ ID NOs: 910-912.

### (46β) Epitope of seed maturation protein PM22 (spot 46α)

In the present invention, the polypeptide (46β) can be any polypeptide selected from the group consisting of 46β-1) to (46β-2) as defined below:
(46β-1) a polypeptide comprising the amino acid of SEQ ID NOs: 915;
(46β-2) a polypeptide comprising the amino acid sequence of XDXDXXXDX (SEQ ID NO: 913), preferably a polypeptide comprising the amino acid sequence of XDIDXXXDX (SEQ ID NO: 914), more preferably a polypeptide comprising an amino acid sequence in which any 1, 2, 3, 4, 5 or 6 of the amino acids at positions 1, 3, 5-7 and 9 of SEQ ID NO: 915 are substituted by alanine, further preferably a polypeptide comprising an amino acid sequence in which any 1, 2, 3 or 4 of the amino acids at positions 1 and 5-7 of SEQ ID NO: 915 are substituted by other amino acids.

### (47β) Epitope of eukaryotic translation initiation factor 5A-2 (spot 47α)

In the present invention, the polypeptide (47β) can be a polypeptide comprising at least one amino acid sequence selected from the group consisting of SEQ ID NOs: 916-918.

### (48β) Epitope of uncharacterized protein LOC100306570 (spot 48α)

In the present invention, the polypeptide (48β) can be any polypeptide selected from the group consisting of (48β-1) to (48β-2) as defined below:
(48β-1) a polypeptide comprising at least one amino acid sequence selected from the group consisting of SEQ ID NOs: 921 and 922;
(48β-2) a polypeptide comprising the amino acid sequence of XXXXXXFDK (SEQ ID NO: 919), preferably a polypeptide comprising the amino acid sequence of XXXXPXFDK (SEQ ID NO: 920), more preferably a polypeptide comprising an amino acid sequence in which any 1, 2, 3, 4, 5 or 6 of the amino acids at positions 1-6 of SEQ ID NO: 921 are substituted by alanine, further preferably a polypeptide comprising an amino acid sequence in which any 1, 2, 3, 4 or 5 of the amino acids at positions 1-4 and 6 of SEQ ID NO: 921 are substituted by other amino acids.

### (49β) Epitope of Bowman-Birk type proteinase inhibitor D-II (spot 49α)

In the present invention, the polypeptide (49β) can be a polypeptide comprising the amino acid sequence of SEQ ID NO: 923.

In one mode, the polypeptide (49β) may not comprise the full-length amino acid sequence (SEQ ID NO: 2) of Bowman-Birk type proteinase inhibitor D-II or a variant or a homolog having 100% or at least 90%, at least 80% or at least 70% identity to the total length of this amino acid sequence.

SEQ ID NO: 923 corresponds to amino acids 79-83 of SEQ ID NO: 2.

### (50β) Epitope of uncharacterized protein LOC100813859 (spot 50α)

In the present invention, the polypeptide (50β) can be any polypeptide selected from the group consisting of 50β-1) to (50β-2) as defined below:
(50β-1) a polypeptide comprising at least one amino acid sequence selected from the group consisting of SEQ ID NOs: 924 and 927;
(50β-2) a polypeptide comprising the amino acid sequence of XXXXKQXXX (SEQ ID NO: 925), preferably a polypeptide comprising the amino acid sequence of XXDXKQXXX (SEQ ID NO: 926), more preferably a polypeptide comprising an amino acid sequence in which any 1, 2, 3, 4, 5, 6 or 7 of the amino acids at positions 1-4 and 7-9 of SEQ ID NO: 927 are substituted by alanine, further preferably a polypeptide comprising an amino acid sequence in which any 1, 2, 3, 4, 5 or 6 of the amino acids at positions 1, 2, 4 and 7-9 of SEQ ID NO: 927 are substituted by other amino acids.

### (51β) Epitope of 14-3-3-like protein (spot 51α)

In the present invention, the polypeptide (51β) can be a polypeptide comprising at least one amino acid sequence selected from the group consisting of SEQ ID NOs: 928-930.

### (52β) Epitope of probable fructokinase-4 (spot 52α)

In the present invention, the polypeptide (52β) comprising an epitope can be any polypeptide selected from the group consisting of 52β-1) and (52β-2) as defined below:
(52β-1) a polypeptide comprising at least one amino acid sequence selected from the group consisting of SEQ ID NOs: 933 and 934-938; and
(52β-2) a polypeptide comprising the amino acid sequence of XXXXXXSXXEXLXXX (SEQ ID NO: 931), preferably a polypeptide comprising the amino acid sequence of XXGXXXSXXEXLXXX (SEQ ID NO: 932), more preferably a polypeptide comprising an amino acid sequence in which any 1, 2, 3, 4, 5, 6, 7, 8, 9, 10, 11 or 12 of the amino acids at positions 1-6, 8, 9, 11, and 13-15 of SEQ ID NO: 933 are substituted by alanine, further preferably a polypeptide comprising an amino acid sequence in which any 1, 2, 3, 4, 5, 6, 7, 8, 9, 10 or 11 of the amino acids at positions 1, 2, 4-6, 8, 9, 11, and 13-15 of SEQ ID NO: 933 are substituted by other amino acids.

### (53β) Epitope of triosephosphate isomerase isoform X1 (spot 53α)

In the present invention, the polypeptide (53β) can be a polypeptide comprising the amino acid sequence of SEQ ID NO: 939.

### (54β) Epitope of superoxide dismutase [Fe], chloroplastic isoform X1 (spot 54α)

In the present invention, the polypeptide (54β) can be any polypeptide selected from the group consisting of 54β-1) to (54β-2) as defined below:
(54β-1) a polypeptide comprising at least one amino acid sequence selected from the group consisting of SEQ ID NOs: 940 and 943;
(54β-2) a polypeptide comprising the amino acid sequence of KXXXXTX (SEQ ID NO: 941), preferably a polypeptide comprising the amino acid sequence of KXXXXTQ (SEQ ID NO: 942), more preferably a polypeptide comprising an amino acid sequence in which any 1, 2, 3, 4 or 5 of the amino acids at positions 2-5 and 7 of SEQ ID NO: 943 are substituted by alanine, further preferably a polypeptide comprising an amino acid sequence in which any 1, 2, 3 or 4 of the amino acids at positions 2-5 of SEQ ID NO: 943 are substituted by other amino acids.

### (55β) Epitope of 51 kDa seed maturation protein precursor (spot 55α)

In the present invention, the polypeptide (55β) can be any polypeptide selected from the group consisting of (55β-1) to (55β-4) as defined below:
(55β-1) a polypeptide comprising at least one amino acid sequence selected from the group consisting of SEQ ID NOs: 946, 949 and 952;
(55β-2) a polypeptide comprising the amino acid sequence of TKXXXSDXX (SEQ ID NO: 944), preferably a polypeptide comprising the amino acid sequence of TKDXXSDXX (SEQ ID NO: 945), more preferably a polypeptide comprising an amino acid sequence in which any 1, 2, 3, 4 or 5 of the amino acids at positions 3-5, 8 and 9 of SEQ ID NO: 946 are substituted by alanine, further preferably a polypeptide comprising an amino acid sequence in which any 1, 2, 3 or 4 of the amino acids at positions 4, 5, 8 and 9 of SEQ ID NO: 946 are substituted by other amino acids;
(55β-3) a polypeptide comprising the amino acid sequence of VKDXXXDA (SEQ ID NO: 947), preferably a polypeptide comprising the amino acid sequence of VKDYXXDA (SEQ ID NO: 948), more preferably a polypeptide comprising an amino acid sequence in which any 1, 2 or 3 of the amino acids at positions 4-6 of SEQ ID NO: 949 are substituted by alanine, further preferably a polypeptide comprising an amino acid sequence in which any 1 or 2 of the amino acids at positions 5 and 6 of SEQ ID NO: 949 are substituted by other amino acids;
(55β-4) a polypeptide comprising the amino acid sequence of KDXXXXXXQ (SEQ ID NO: 950), preferably a polypeptide comprising the amino acid sequence of KDXXXXXAQ (SEQ ID NO: 951), more preferably a polypeptide comprising an amino acid sequence in which any 1, 2, 3, 4, 5 or 6 of the amino acids at positions 3-8 of SEQ ID NO: 952 are substituted by alanine, further preferably a polypeptide comprising an amino acid sequence in which any 1, 2, 3, 4 or 5 of the amino acids at positions 3-7 of SEQ ID NO: 952 are substituted by other amino acids.

### (57β) Epitope of Gly m 4

In the present invention, the polypeptide (57β) can be any polypeptide selected from the group consisting of (56β-1) to (56β-2) as defined below:
(57β-1) a polypeptide comprising the amino acid of SEQ ID NOs: 955;
(57β-2) a polypeptide comprising the amino acid sequence of XXXXXXXEXYXLXHP (SEQ ID NO: 953), preferably a polypeptide comprising the amino acid sequence of XXXXXXIEAYLLAHP (SEQ ID NO: 954), more preferably a polypeptide comprising an amino acid sequence in which any 1, 2, 3, 4, 5, 6, 7, 8, 9 or 10 of the amino acids at positions 1-7, 9, 11 and 13 of SEQ ID NO: 955 are substituted by alanine, further preferably a polypeptide comprising an amino acid sequence in which any 1, 2, 3, 4, 5 or 6 of the amino acids at positions 1-6 of SEQ ID NO: 955 are substituted by other amino acids.

In one mode, the polypeptide (57β) may not comprise the full-length amino acid sequence (UniProt accession number: P26987.1) of Gly m 4 or a variant or a homolog having 100% or at least 90%, at least 80% or at least 70% identity to the total length of this amino acid sequence.

SEQ ID NO: 955 corresponds to amino acids 141-155 of UniProt accession number: P26987.1.

### (58β) Epitopes of Gly m 5α and Gly m 5α'

In the present invention, the polypeptide (58β) can be a polypeptide comprising at least one amino acid sequence selected from the group consisting of SEQ ID NOs: 956-960.

In one mode, the polypeptide (58β) comprising an epitope may not comprise the full-length amino acid sequence (NCBI accession number: NP_001236856.1) of Gly m 5α or the full-length amino acid sequence (SEQ ID NO: NCBI accession number: NP_001237316.1) of Gly m 5α', or a variant or a homolog having 100% or at least 90%, at least 80% or at least 70% identity to the total length of any of these amino acid sequences.

SEQ ID NOs: 956-960 correspond to amino acids 173-181 (SEQ ID NO: 956), amino acids 294-301 (SEQ ID NO: 957), amino acids 301-309 (SEQ ID NO: 958), amino acids 414-422 (SEQ ID NO: 959), and amino acids 294-301 (SEQ ID NO: 960), respectively, of NCBI accession number: NP_001236856.1.

### (59β) Epitopes of Gly m 6.01 and Gly m 6.02

In the present invention, the polypeptide (59β) can be a polypeptide comprising at least one amino acid sequence selected from the group consisting of SEQ ID NOs: 961 and 962.

In one mode, the polypeptide (59β) may not comprise the full-length amino acid sequence (UniProt accession number: CAA33215.1) of Gly m 6.01 or the full-length amino acid sequence (UniProt accession number: BAA00154.1) of Gly m 6.02, or a variant or a homolog having 100% or at least 90%, at least 80% or at least 70% identity to the total length of any of these amino acid sequences.

SEQ ID NOs: 961 and 962 correspond to amino acids 201-215 and amino acids 451-465, respectively, of UniProt accession number: CAA33215.1.

### (60β) Epitope of Gly m 8

In the present invention, the polypeptide (60β) can be a polypeptide comprising the amino acid sequence of SEQ ID NO: 963.

In one mode, the polypeptide (60β) may not comprise the full-length amino acid sequence (UniProt accession number: AAB71140.1) of Gly m 8 or a variant or a homolog having 100% or at least 90%, at least 80% or at least 70% identity to the total length of this amino acid sequence.

SEQ ID NO: 963 corresponds to amino acids 71-85 of UniProt accession number: AAB71140.1.

The residue of X as to the aforementioned polypeptides (1β) to (60β) is an amino acid residue at a site where binding activity against IgE antibodies from allergic patients was maintained even after substitution by alanine in alanine scanning described in Example 4. It is well known to those skilled in the art that even when such a site is substituted by any other amino acids, it is highly probable that this binding activity against IgE antibodies is maintained.

The lengths of the aforementioned polypeptides (1β) to (60β) are not particularly limited. In a preferred mode, the lengths of the aforementioned polypeptides (1β) to (60β) can be at least 500 amino acids, at least 300 amino acids, at least 200 amino acids, at least 100 amino acids, at least 50 amino acids, at least 30 amino acids, at least 20 amino acids, at least 10 amino acids, or at least 5 amino acids.

The aforementioned polypeptides (1β) to (60β) may be prepared by a technique of chemical synthesis such as solid-phase peptide synthesis. Alternatively, polypeptides comprising an epitope may be obtained by expressing them as recombinant proteins using a genetic recombination technique well known to those skilled in the art and by separating and purifying them using protein purification methods well known to those skilled in the art.

### Diagnosis kit and method (2)

The present invention provides a method for providing an indicator for diagnosing an allergy in a subject, the method comprising the steps of:
(i) contacting a sample obtained from the subject with an antigen, wherein the sample is a solution comprising an Ig antibody;
(ii) detecting binding between the IgE antibody present in the sample obtained from the subject and the antigen; and
(iii) when the binding between the IgE antibody in the subject and the antigen is detected, an indicator of the fact that the subject is allergic is provided;
   wherein the antigen is at least one of the aforementioned polypeptides (1β) to (60β), or a polypeptide in which two or more of the aforementioned polypeptides (1β) to (60β) are joined together via or without a spacer.

Hereinafter, the at least one of the aforementioned polypeptides (1β) to (60β), or the polypeptide in which two or more of the aforementioned polypeptides (1β) to (60β) are joined together via or without a spacer is referred to as the "antigen including (1β) to (60β)" in the present specification. The type of the spacer is not particularly limited, and an ordinary spacer that is used by those skilled in the art for joining together two or more peptides can be used. The spacer may be, for example, a hydrocarbon chain such as Acp(6)-OH.

The sample obtained from a subject is as described above in the subsection titled "Diagnosis kit and method (1)".

Detection of contact and binding between the sample obtained from a subject and the antigen can be carried out by using a known method described above in the subsection titled "Diagnosis kit and method (1)", such as ELISA (Enzyme-Linked Immunosorbent Assay), sandwich immunoassay, immunoblotting, immunoprecipitation, or immunochromatography.

The antigen may be provided as an isolated antigen including (1β) to (60β) in a state immobilized on a carrier. In this case, the steps (i) and (ii) mentioned above can be carried out using ELISA or sandwich immunoassay, or the like. In this respect, the step (i) mentioned above can be carried out by contacting the sample obtained from a subject with an antigen-immobilized surface.

The antigen including (1β) to (60β) specifically binds to IgE antibodies from allergic patients. Therefore, when binding between an IgE antibody from a subject and the antigen is detected, an indicator of the fact that the subject is allergic to soybean is provided.

The present invention further provides a kit for diagnosing an allergy, comprising at least one antigen including (1β) to (60β). The diagnosis kit of this invention may be used in the aforementioned method for providing an indicator for diagnosing an allergy or in a diagnosis method as described later. The diagnosis kit of this invention may comprise not only the at least one antigen including (1β) to (60β), but also an anti-IgE antibody labeled with an enzyme and a chromogenic or luminescent substrate serving as a substrate for the enzyme. Also, a fluorescent-labeled anti-IgE antibody may be used. In the diagnosis kit of this invention, the antigen may be provided in a state immobilized on a carrier. The diagnosis kit of this invention may also be provided together with instructions on the procedure for diagnosis or a package containing said instructions.

The present invention further provides a composition for diagnosing an allergy, comprising at least one antigen including (1β) to (60β). The diagnosis composition of this invention can be used in a diagnosis method as described below. The diagnosis composition of this invention may further comprise a pharmaceutically acceptable carrier and/or additives commonly used with the antigen of this invention depending on the need.

In one mode, the present invention provides a method for diagnosing an allergy in a subject, the method comprising:
(i) contacting a sample obtained from the subject with an antigen;
(ii) detecting binding between an IgE antibody present in the sample obtained from the subject and the antigen; and
(iii) when the binding between the IgE antibody in the subject and the antigen is detected, diagnosing the subject as being allergic to a soybean;
   wherein the antigen is at least one of the aforementioned polypeptides (1β) to (60β), or a polypeptide in which two or more of the aforementioned polypeptides (1β) to (60β) are joined together via or without a spacer (i.e., the antigen including (1β) to (60β)). In this method, the steps (i) and (ii) are performed as described above regarding the corresponding steps of the method for providing an indicator for diagnosing an allergy.

In another mode, the present invention provides a method for diagnosing an allergy in a subject, the method comprising administering to the subject at least one antigen including (1β) to (60β). This method may be performed in the form of a skin test characterized by applying the antigen onto the skin. The skin test includes forms of tests described above in the subsection titled "Diagnosis kit and method (1)". If a skin reaction such as swelling occurs in a skin portion to which the antigen has been applied, the subject of interest is diagnosed as having an allergy. The amount of the antigen to be applied to the skin in such tests can be, for example, not more than 100 µg per dose.

In yet another mode, the present invention provides at least one antigen including (1β) to (60β), intended for use as an active ingredient for a load test for allergy diagnosis.

In still another mode, the present invention provides at least one antigen including (1β) to (60β), intended for use in the diagnosis of an allergy.

In still another mode, the present invention provides use of at least one antigen including (1β) to (60β) for the production of a diagnostic agent for an allergy.

In this subsection, the allergy to be diagnosed or detected can be allergies to allergens in which allergen components having the aforementioned polypeptides (1β) to (60β) are present. Thus, detection of the allergy can be detection of not only an allergy to a single allergen, but also allergies including cross-reactivity.

### Pharmaceutical composition and treatment method (2)

The present invention provides a pharmaceutical composition comprising at least one antigen including (1β) to (60β). In one mode, the aforementioned pharmaceutical composition is used for the treatment of an allergy.

The present invention also provides a method for treating an allergy, the method comprising administering at least one antigen including (1β) to (60β) to a patient in need of a treatment for an allergy.

In another mode, the present invention provides at least one antigen including (1β) to (60β), intended for use in the treatment for an allergy. In yet another mode, the present invention provides use of at least one antigen including (1β) to (60β) for the production of a therapeutic agent for an allergy.

In still another mode, the at least one antigen including (1β) to (60β) can be used as an active ingredient for hyposensitization therapy for an allergy. Here, the antigen protein to be used in the hyposensitization therapy may be a polypeptide that has been expressed and purified and may be a polypeptide that has been expressed in food such as rice, such as rice-based vaccine expressing pollen allergens.

The administration route, administration dose, frequency and/or period of the pharmaceutical composition of this invention, and other ingredients to be contained in the pharmaceutical composition, and the dosage form can be as described above in the subsection titled "Pharmaceutical composition and treatment method (1)". In the case of using the antigen including (1β) to (60β), for example, the dose to an adult patient may be a dose of not more than 100 µg per dose.

In this subsection, the allergy to be treated can be allergies to allergens in which allergen components having the aforementioned polypeptides (1β) to (60β) are present. Thus, detection of the allergy can be detection of not only an allergy to a single allergen, but also allergies including cross-reactivity.

### Tester (2)

The present invention provides a tester comprising an antibody for at least one antigen including (1β) to (60β).

The antibody can be prepared by a conventional method. For example, the antibody may be prepared by immunizing a mammal such as rabbit with the aforementioned antigen including (1β) to (60β). The antibody may be an Ig antibody, a polyclonal antibody, a monoclonal antibody, or an antigen-binding fragment thereof (e.g., Fab, F(ab')₂, Fab').

Further, in the aforementioned tester, the antibody may be provided in a form bound to a carrier. The type of the carrier is not particularly limited as long as it is usable for detection of binding between an antibody and the antigen including (1β) to (60β). Any given carrier known to those skilled in the art can be used.

In one mode, the aforementioned tester can be used for the purposes described above in the subsection titled "Tester (1)".

A method for determining the presence or absence of an antigen is as described above in the subsection titled "Tester (1)".

Another mode of the present invention includes a tester for determining the presence or absence of an antigen of an allergy in an object of interest, the tester comprising a primer appropriate for an epitope. The primer is not limited and may be designed so as to comprise, for example, a portion of the nucleotide sequence of a nucleic acid encoding any of the amino acid sequences defined above in (1β) to (60β), or a complementary strand thereof. Alternatively, the primer may be designed so as to be the nucleotide sequence of a region upstream or downstream of a portion encoding an epitope that is any of the amino acid sequences defined above in (1β) to (60β), in a nucleic acid encoding a protein comprising the epitope, or a complementary strand thereof. Here, the position of the epitope in the full-length sequence of an antigen is as defined above in the subsections titled "Antigen" and "Epitope of antigen". Particularly, when mRNA is of interest, the tester has a complementary primer of a poly-A tail.

For example, DNA is amplified by PCR (Polymerase Chain Reaction) including RT-PCR using templated DNA or mRNA obtained from a sample and the aforementioned primer, and the presence or absence of a nucleic acid encoding the amino acid sequences defined above in (1β) to (60β) in the sequence of the amplified DNA is determined to determine the presence or absence of the antigen. Amplification methods by PCR can be exemplified by RACE.

### Allergen-free food and the like (2)

The present invention provides a food raw material or an edible processed product in which at least one antigen including (1β) to (60β) is eliminated or reduced.

The method for eliminating or reducing the antigen of the present invention in a food raw material or an edible processed product is not limited. The techniques described above in the subsection titled "Allergen-free food and the like (1)" may be used. Elimination or reduction of at least one antigen including (1β) to (60β) may be achieved by eliminating or reducing the whole antigen or may be achieved by cleaving or removing the polypeptides defined in (1β) to (60β) from the antigen protein. The "removal" includes deletion and modification.

The edible processed product in which the antigen of the present invention is eliminated or reduced may be a processed product of the food raw material in which the antigen of the present invention is eliminated or reduced, such as powdered milk obtained with purified amino acids as a raw material. In the case of using an ordinary food raw material, a treatment for removing or reducing the polypeptide of this invention is performed before or after preparation of an edible processed product. The techniques described in the subsection titled "Allergen-free food and the like (1)" may be used as methods for removing or reducing the antigen of the present invention in an edible processed product obtained with an ordinary food raw material.

### Method for producing allergen-free food (2)

The present invention provides a method for producing an edible processed product in which an antigen is eliminated or reduced, the method comprising the step of confirming that the antigen is eliminated or reduced, in a production process of the processed product, wherein the antigen is at least one of the aforementioned antigen including (1β) to (60β) and antigens (1α) to (55α).

In the production method, elimination or reduction of an antigen means that at least one antigen including (1β) to (60β) is eliminated or reduced, or the polypeptides defined in (1β) to (60β) are cleaved or removed from the antigen protein.

A technique of confirming that the antigen is eliminated or reduced in the production process of the edible processed product is not particularly limited, and any technique capable of detecting at least one of the aforementioned antigen including (1β) to (60β) and antigens (1α) to (55α) may be used. For example, the presence or absence of the polypeptide or the antigen in the processed product may be confirmed from the binding activity of an antibody for at least one of the aforementioned antigen including (1β) to (60β) and antigens (1α) to (55α) against a sample containing a material resulting from the production process of the processed product. Details of such a method are as described above in the subsection titled "Diagnosis kit and method (1)". Thus, in the production method, the "IgE antibody from a subject" described above in the subsection titled "Diagnosis kit and method (1)" is replaced with the "antibody for at least one of the aforementioned polypeptides (1β) to (60β) and antigens (1α) to (55α)", and the "antigen" described above in the subsection titled "Diagnosis kit and method (1)" is replaced with the "sample containing a material resulting from the production process of the edible processed product". The techniques described above in the subsection titled "Diagnosis kit and method (1)" can be used to confirm that the antigen is eliminated or reduced in the production process of the edible processed product.

### EXAMPLES

The following describes examples of the present invention. The technical scope of this invention is not limited by these examples.

### Example 1: Confirmation of a protein pattern

Proteins contained in soybean were investigated using a two-dimensional electrophoresis method described below.

### Protein extraction

Commercially available dry soybeans (several beans) were powdered. 500 µl of water was added to 50 mg of the powder and shaking extraction was conducted using a vortex mixer at 25°C for 10 minutes. After the shaking extraction, a liquid protein extract was collected.

Thereafter, the precipitation procedure was repeated twice using a 2D-CleanUP Kit (produced by GE). In the first round of precipitation, the collected liquid protein extract was precipitated by adding TCA (trichloroacetic acid) thereto and the precipitated product produced by this procedure (TCA-precipitated product) was collected. In the second round of precipitation, the TCA-precipitated product collected above was further precipitated by adding acetone thereto and the precipitated product (sample) produced by this procedure was collected.

### Preparation of a sample solution

Part of the collected sample (50 µg on a protein weight basis) was dissolved in 150 µL of a DeStreak Rehydration Solution (produced by GE), which is a swelling buffer for first-dimensional isoelectric focusing gels, thereby obtaining a sample solution for first-dimensional isoelectric focusing (sample solution for swelling). The constituents of the DeStreak Rehydration Solution are as mentioned below.
7M thiourea
2M urea
4% (w/v) of CHAPS (3-[(3-cholamidopropyl)dimethylammonio]propanesulfonate)
0.5% (v/v) IPG buffer; produced by GE
Moderate amount of BPB (bromophenol blue)

### Penetration of the sample into first-dimensional isoelectric focusing gels

First-dimensional isoelectric focusing gel strips (Immobiline Drystrip IPG gels (pH3-10NL); produced by GE) were immersed in 140 µL of the foregoing sample solution for first-dimensional isoelectric focusing (sample solution for swelling) and impregnated with the solution at room temperature overnight.

In this example, an IPGphor electrophoresis system produced by GE was used.

An electrophoresis tray was filled with silicone oil. Filter paper moisten with water was positioned at both ends of the gel strips impregnated with the sample, and the gel strips were set in the electrophoresis tray such that the gel strips were covered with silicone oil. Electrodes were placed on the gel strips with the filter paper intervening therebetween.

The maximum current of the isoelectric focusing system was set to 75 µA per gel strip, and the first-dimensional isoelectric focusing was carried out according to the following voltage program: (1) a constant voltage step was performed at a constant voltage of 300 V until the volt-hours reached 750 Vhr (the current variation width during electrophoresis for 30 minutes before the end of this step was 5 µA); (2) the voltage was increased gradually to 1000 V for 300 Vhr; (3) the voltage was further increased gradually to 5000 V for 4500 Vhr; and then (4) the voltage was held at a constant voltage of 5000 V until the total Vhr reached 12000.

### SDS equilibration of isoelectric focusing gels

After the aforementioned first-dimensional isoelectric focusing was done, the gel strips were taken out of the isoelectric focusing system, immersed in an equilibration buffer containing a reducing agent, and shaken at room temperature for 15 minutes. The constituents of the equilibration buffer containing the reducing agent are as mentioned below.
100 mM Tris-HCl (pH 8.0)
6M urea
30% (v/v) glycerol
2% (w/v) SDS
1% (w/v) DTT

Next, the equilibration buffer containing the reducing agent was removed, and then the gel strips were immersed in an equilibration buffer containing an alkylating agent and shaken at room temperature for 15 minutes to obtain SDS-equilibrated gels. The constituents of the equilibration buffer containing the alkylating agent are as mentioned below.
100 mM Tris-HCl (pH 8.0)
6M urea
30% (v/v) glycerol
2% (w/v) SDS
2.5% (w/v) iodoacetamide

### Second-dimensional SDS-PAGE

In this example, the XCell SureLock Mini-Cell electrophoresis system produced by Life Technologies was used. The second-dimensional electrophoresis gels used were NuPAGE 4-12% Bis-Tris Gels produced by Life Technologies. Also, an electrophoresis buffer composed of the following constituents was prepared and used.
50 mM MOPS
50 mM Tris base
0.1% (w/v) SDS
1 mM EDTA

Further, an agarose solution for gel adhesion was used in this example, which was prepared by dissolving 0.5% (w/v) Agarose S (produced by Nippon Gene Co., Ltd.) and a moderate amount of BPB (bromophenol blue) in the electrophoresis buffer.

SDS-PAGE wells were washed well with the electrophoresis buffer, and then the buffer used for the washing was removed. Next, the washed wells were charged with the fully dissolved agarose solution for gel adhesion. Next, the SDS-equilibrated gel strips were immersed in agarose and closely adhered to second-dimensional electrophoresis gels using tweezers. After it was confirmed that agarose was fully fixed with the gels being closely adhered to each other, electrophoresis was performed at a constant voltage of 200 V for about 45 minutes.

### Fluorescent staining of gels

The gels were fluorescently stained with SYPRO Ruby (produced by Life Technologies).

First, an airtight container to be used was washed well in advance with 98% (v/v) ethanol. The electrophoresed second-dimensional electrophoresis gel strips were taken out of the SDS-PAGE system, placed onto the washed airtight container, and treated twice by immersion in 50% (v/v) methanol and 7% (v/v) aqueous solution containing acetic acid for 30 minutes. Then, a further immersion treatment was done for 10 minutes, with the solution being replaced by water. Next, the second-dimensional electrophoresis gel strips were immersed in 40 mL of SYPRO Ruby and shaken at room temperature overnight. Thereafter, the SYPRO Ruby was removed, and then the second-dimensional electrophoresis gel strips were washed with water and shaken in 10% (v/v) methanol and 7% (v/v) aqueous solution containing acetic acid for 30 minutes. Further shaking was done for at least 30 minutes, with the solution being replaced by water.

### Analysis

The second-dimensional electrophoresis gels obtained through the foregoing series of treatments were subjected to fluorescent image scanning on Typhoon 9400 (produced by GE). The results of the two-dimensional electrophoresis are shown in Fig. 1. Molecular weight marker bands are found at the left of the panels. The positions of the bands denote particular molecular weights (in KDa).

### Example 2: Identification of antigens by immunoblotting

Identification of antigens by immunoblotting was carried out by taking all the steps up to the step of "Second-dimensional SDS-PAGE" as described above in Example 1, followed by the steps of "Transfer to membrane", "Immunoblotting" and "Analysis" as described below.

### Transfer to membrane

Transfer to membrane was done using the following transfer system and transfer buffer.
Transfer system: XCell SureLock Mini-Cell and XCell II Blot Module (produced by Life Technologies)
Transfer buffer: NuPAGE Transfer Buffer (X20) (produced by Life Technologies), used in a form diluted 20-fold with milliQ water.

To be specific, proteins in the two-dimensional electrophoresis gels were transferred to a membrane (PVDF membrane) according to the following procedure.
(1) The PVDF membrane was immersed in 100% methanol followed by milliQ water, and then moved into the transfer buffer to hydrophilize the PVDF membrane.
(2) After sponge, filter paper, the gels treated by second-dimensional SDS-PAGE, the hydrophilized PVDF membrane, filter paper, and sponge were put in place in this order, the transfer system was energized at a constant voltage of 30 V for one hour.

### Immunoblotting

Immunoblotting of the membrane was carried out using, as a primary antibody, a serum sample from a patient with a soybean allergy or a serum sample from a healthy subject.

Immunoblotting of the membrane was carried out according to the following procedure.
(1) The transferred membrane was shaken in a 5% skim milk/PBST solution (a PBS buffer containing 0.3% Tween 20 nonionic surfactant) at room temperature for one hour.
(2) The membrane was left to stand in a solution of 3% primary antibody serum in 5% skim milk/PBST at room temperature for one hour.
(3) The membrane was washed with a PBST solution (5 min. × 3 times).
(4) The membrane was left to stand in a 1:2500 dilution of the secondary antibody, anti-human IgE-HRP (horseradish peroxidase), with a 3% skim milk/PBST solution at room temperature for one hour.
(5) The membrane was washed with a PBST solution (5 min. × 3 times).
(6) The membrane was left to stand in Pierce Western Blotting Substrate Plus (produced by Thermo Fisher Scientific) for 5 minutes.

### Analysis

The membrane obtained through the foregoing series of treatments was subjected to fluorescent image scanning on Typhoon 9400 (produced by GE).

The immunoblots obtained with the serum from the soybean-allergic patient were compared with those obtained with the control serum from the healthy subject. By immunoblotting using the serum from soybean-allergic Patient 1α, Patient 2α, Patient 3α, Patient 4α, Patient 5α, Patient 6α, Patient 7α, Patient 8α, and Patient 9α, 56 spots were detected (Figs. 3A to I), which are different from the spots detected when the serum of the healthy subject was used (Fig. 2) and different from those of the known soybean allergen proteins.

The molecular weights and isoelectric points of the 56 spots are as follows.
Spot 1α: Molecular weight 80 to 110 kDa, pI 3.0 to 6.0
Spot 2α: Molecular weight 60 to 110 kDa, pI 3.0 to 6.0
Spot 3α: Molecular weight 100 to 150 kDa, pI 4.0 to 6.0
Spot 4α: Molecular weight 80 to 110 kDa, pI 4.0 to 7.0
Spot 5α: Molecular weight 80 to 160 kDa, pI 4.0 to 7.0
Spot 6α: Molecular weight 80 to 110 kDa, pI 4.0 to 7.0
Spot 7α: Molecular weight 60 to 110 kDa, pI 4.0 to 7.0
Spot 8α: Molecular weight 60 to 110 kDa, pI 4.0 to 8.0
Spot 9α: Molecular weight 60 to 110 kDa, pI 4.0 to 8.0
Spot 10α: Molecular weight 40 to 80 kDa, pI 4.0 to 7.0
Spot 11α: Molecular weight 40 to 80 kDa, pI 4.0 to 8.0
Spot 12α: Molecular weight 40 to 80 kDa, pI 4.0 to 8.0
Spot 13α: Molecular weight 40 to 80 kDa, pI 4.0 to 7.0
Spot 14α: Molecular weight 40 to 80 kDa, pI 3.0 to 6.0
Spot 15α, 16α: Molecular weight 30 to 80 kDa, pI 4.0 to 7.0
Spot 17α: Molecular weight 30 to 80 kDa, pI 4.0 to 7.0
Spot 18α: Molecular weight 40 to 80 kDa, pI 4.0 to 8.0
Spot 19α: Molecular weight 40 to 80 kDa, pI 4.0 to 8.0
Spot 20α: Molecular weight 60 to 75 kDa, pI 6.0 to 8.0
Spot 21α: Molecular weight 40 to 60 kDa, pI 5.0 to 8.0
Spot 22α: Molecular weight 25 to 37 kDa, pI 8.0 to 10.0
Spot 23α: Molecular weight 25 to 37 kDa, pI 7.0 to 9.0
Spot 24α: Molecular weight 5 to 20 kDa, pI 5.0 to 7.0
Spot 25α: Molecular weight 30 to 80 kDa, pI 4.0 to 7.0
Spot 26α: Molecular weight 30 to 80 kDa, pI 4.0 to 7.0
Spot 27α: Molecular weight 30 to 80 kDa, pI 4.0 to 7.0
Spot 28α: Molecular weight 20 to 60 kDa, pI 4.0 to 7.0
Spot 29α, 30α, 31α, 32α, 33α: Molecular weight 20 to 60 kDa, pI 4.0 to 8.0
Spot 34α: Molecular weight 20 to 60 kDa, pI 6.0 to 10.0
Spot 35α: Molecular weight 30 to 80 kDa, pI 6.0 to 10.0
Spot 36α, 37α: Molecular weight 20 to 60 kDa, pI 4.0 to 8.0
Spot 38α: Molecular weight 80 to 160 kDa, pI 6.0 to 10.0
Spot 39α: Molecular weight 10 to 50 kDa, pI 6.0 to 10.0
Spot 40α, 56α: Molecular weight 10 to 50 kDa, pI 6.0 to 10.0
Spot 41α: Molecular weight 10 to 50 kDa, pI 6.0 to 10.0
Spot 42α, 43α: Molecular weight 10 to 50 kDa, pI 6.0 to 10.0
Spot 44α: Molecular weight 10 to 50 kDa, pI 4.0 to 7.0
Spot 45α: Molecular weight 5 to 40 kDa, pI 4.0 to 7.0
Spot 46α: Molecular weight 5 to 40 kDa, pI 4.0 to 7.0
Spot 47α: Molecular weight 5 to 40 kDa, pI 4.0 to 7.0
Spot 48α: Molecular weight 5 to 40 kDa, pI 4.0 to 8.0
Spot 49α: Molecular weight 5 to 20 kDa, pI 4.0 to 7.0
Spot 50α: Molecular weight 5 to 40 kDa, pI 3.0 to 6.0
Spot 51α: Molecular weight 10 to 50 kDa, pI 3.0 to 6.0
Spot 52α: Molecular weight 20 to 60 kDa, pI 4.0 to 7.0
Spot 53α: Molecular weight 10 to 50 kDa, pI 4.0 to 8.0
Spot 54α: Molecular weight 10 to 50 kDa, pI 4.0 to 8.0
Spot 55α: Molecular weight 40 to 80 kDa, pI 6.0 to 10.0

### Example 3: Mass spectrometry and identification of antigens

The amino acid sequences of the antigens that form the 56 protein spots were identified by mass spectroscopy.

To be specific, protein extraction and mass spectroscopy were done by the following procedure.
(1) Soybeans were subjected to protein extraction, two-dimensional electrophoresis and transfer to membrane by following the procedures described in Example 1 and 2, and the resulting membrane was stained by shaking in a solution of 0.008% Direct blue in 40% ethanol and 10% acetic acid.
(2) Then, the membrane was decolorized by repeating a 5-minute treatment with 40% ethanol and 10% acetic acid three times, washed with water for 5 minutes, and then dried by air.
(3) A protein spot of interest was cut out with a clean cutter blade and put into a centrifugal tube. The cut membrane was subjected to hydrophilization with 50 µL of methanol, followed by washing with 100 µL of water twice and then centrifugal cleaning. Thereafter, 20 µL of 20 mM NH₄HCO₃ and 50% acetonitrile were added.
(4) 1 µL of 1 pmol/µL lysyl endopeptidase (produced by WAKO) was added, and the solution was left to stand at 37°C for 60 minutes and then collected in a new centrifugal tube. After 20 µL of 20 mM NH₄HCO₃ and 70% acetonitrile were added to the membrane, the membrane was immersed therein at room temperature for 10 minutes, and the resulting solution was further collected. The solution was dissolved with 0.1% formic acid and 10 µL of 4% acetonitrile and transferred to a tube.
(5) The collected solution was dried under reduced pressure, dissolved with 15 µL of solution A (a 0.1% formic acid/4% acetonitrile solution), and analyzed by mass spectroscopy (ESI-TOF5600, produced by AB Sciex). (6) Identification of proteins based on the MS data obtained with the mass spectrometer was done by searching the NCBI database.

### Results

The mass spectrometry of the spots resulted in the detection of the following amino acid sequences. Furthermore, the spots were identified as the following proteins by the analysis of the mass data obtained from the mass spectrometer for the spots at NCBI.

### Spot 1α:

The amino acid sequences set forth in SEQ ID NOs: 71 to 87 were detected by the mass spectrometry. The protein identified from this mass data was endoplasmin homolog isoform X2 (NCBI accession number XP_006596543.1, amino acid sequence: SEQ ID NO: 70, encoding nucleotide sequence: SEQ ID NO: 69).

The amino acid sequences set forth in SEQ ID NOs: 90 to 104 were detected by the mass spectrometry. The protein identified from this mass data was endoplasmin homolog isoform X2 (NCBI accession number XP_006601356.1, amino acid sequence: SEQ ID NO: 89, encoding nucleotide sequence: SEQ ID NO: 88).

### Spot 2α

The amino acid sequences set forth in SEQ ID NOs: 107 to 120 were detected by the mass spectrometry. The protein identified from this mass data was heat shock cognate protein 80 (NCBI accession number XP_003544594.1, amino acid sequence: SEQ ID NO: 106, encoding nucleotide sequence: SEQ ID NO: 105).

The amino acid sequences set forth in SEQ ID NOs: 123 to 137 were detected by the mass spectrometry. The protein identified from this mass data was heat shock protein 90-1 (NCBI accession number NP_001236612.1, amino acid sequence: SEQ ID NO: 122, encoding nucleotide sequence: SEQ ID NO: 121).

The amino acid sequences set forth in SEQ ID NOs: 140 to 153 were detected by the mass spectrometry. The protein identified from this mass data was hypothetical protein GLYMA_02G302500 (NCBI accession number KRH73936.1, amino acid sequence: SEQ ID NO: 139, encoding nucleotide sequence: SEQ ID NO: 138).

### Spot 3α:

The amino acid sequences set forth in SEQ ID NOs: 62 to 68 were detected by the mass spectrometry. The protein identified from this mass data was Cell division cycle protein 48 homolog (UniProt accession number P54774, amino acid sequence: SEQ ID NO: 16, encoding nucleotide sequence: SEQ ID NO: 15).

### Spot 4α:

The amino acid sequences set forth in SEQ ID NOs: 156, 157 were detected by the mass spectrometry. The protein identified from this mass data was embryo-specific urease isoform X1 (NCBI accession number XP_014630847.1, amino acid sequence: SEQ ID NO: 155, encoding nucleotide sequence: SEQ ID NO: 154).

### Spot 5α:

The amino acid sequences set forth in SEQ ID NOs: 160, 161 were detected by the mass spectrometry. The protein identified from this mass data was alpha-1,4 glucan phosphorylase L isozyme, chloroplastic/amyloplastic (NCBI accession number XP_006603904.1, amino acid sequence: SEQ ID NO: 159, encoding nucleotide sequence: SEQ ID NO: 158).

### Spot 6α:

The amino acid sequences set forth in SEQ ID NOs: 164 to 167 were detected by the mass spectrometry. The protein identified from this mass data was aconitate hydratase 1 (NCBI accession number XP_003537655.1, amino acid sequence: SEQ ID NO: 163, encoding nucleotide sequence: SEQ ID NO: 162).

The amino acid sequences set forth in SEQ ID NOs: 170 to 172 were detected by the mass spectrometry. The protein identified from this mass data was aconitate hydratase 1 (NCBI accession number XP_003517155.1, amino acid sequence: SEQ ID NO: 169, encoding nucleotide sequence: SEQ ID NO: 168).

### Spot 7α:

The amino acid sequences set forth in SEQ ID NOs: 175 to 187 were detected by the mass spectrometry. The protein identified from this mass data was methionine synthase (NCBI accession number NP_001235794.1, amino acid sequence: SEQ ID NO: 174, encoding nucleotide sequence: SEQ ID NO: 173).

### Spot 8α:

The amino acid sequences set forth in SEQ ID NOs: 190 to 199 were detected by the mass spectrometry. The protein identified from this mass data was transketolase, chloroplastic (NCBI accession number XP_003521870.1, amino acid sequence: SEQ ID NO: 189, encoding nucleotide sequence: SEQ ID NO: 188).

### Spot 9α:

The amino acid sequence set forth in SEQ ID NO: 202 was detected by the mass spectrometry. The protein identified from this mass data was lysine-tRNA ligase, cytoplasmic-like isoform X2 (NCBI accession number XP_014625509.1, amino acid sequence: SEQ ID NO: 201, encoding nucleotide sequence: SEQ ID NO: 200).

### Spot 10α

The amino acid sequences set forth in SEQ ID NOs: 205 to 218 were detected by the mass spectrometry. The protein identified from this mass data was chaperonin CPN60-2, mitochondrial (NCBI accession number XP_003556325.1, amino acid sequence: SEQ ID NO: 204, encoding nucleotide sequence: SEQ ID NO: 203).

The amino acid sequences set forth in SEQ ID NOs: 221 to 231 were detected by the mass spectrometry. The protein identified from this mass data was chaperonin CPN60-2, mitochondrial-like isoform X1 (NCBI accession number XP_003535967.1, amino acid sequence: SEQ ID NO: 220, encoding nucleotide sequence: SEQ ID NO: 219).

### Spot 11α:

The amino acid sequences set forth in SEQ ID NOs: 234 to 237 were detected by the mass spectrometry. The protein identified from this mass data was polyadenylate-binding protein 8 isoform X3 (NCBI accession number XP_006578034.1, amino acid sequence: SEQ ID NO: 233, encoding nucleotide sequence: SEQ ID NO: 232).

### Spot 12α:

The amino acid sequence set forth in SEQ ID NO: 240 was detected by the mass spectrometry. The protein identified from this mass data was pyrophosphate-fructose 6-phosphate 1-phosphotransferase subunit alpha-like (NCBI accession number XP_003555655.1, amino acid sequence: SEQ ID NO: 239, encoding nucleotide sequence: SEQ ID NO: 238).

### Spot 13α:

The amino acid sequences set forth in SEQ ID NOs: 243 to 252 were detected by the mass spectrometry. The protein identified from this mass data was protein disulfide isomerase-like protein precursor (NCBI accession number NP_001238009.1, amino acid sequence: SEQ ID NO: 242, encoding nucleotide sequence: SEQ ID NO: 241).

The amino acid sequences set forth in SEQ ID NOs: 255 to 278 were detected by the mass spectrometry. The protein identified from this mass data was protein disulfide-isomerase (NCBI accession number XP_006581588.1, amino acid sequence: SEQ ID NO: 254, encoding nucleotide sequence: SEQ ID NO: 253).

### Spot 14α:

The amino acid sequences set forth in SEQ ID NOs: 281 to 284 were detected by the mass spectrometry. The protein identified from this mass data was V-type proton ATPase subunit B 2 (NCBI accession number XP_003552473.1, amino acid sequence: SEQ ID NO: 280, encoding nucleotide sequence: SEQ ID NO: 279).

The amino acid sequences set forth in SEQ ID NOs: 287 to 289 were detected by the mass spectrometry. The protein identified from this mass data was V-type proton ATPase subunit B 2 (NCBI accession number XP_006605857.1, amino acid sequence: SEQ ID NO: 286, encoding nucleotide sequence: SEQ ID NO: 275).

### Spots 15α, 16α:

The amino acid sequences set forth in SEQ ID NOs: 292 to 304 were detected by the mass spectrometry. The protein identified from this mass data was UTP-glucose-1-phosphate uridylyltransferase (NCBI accession number XP_003544964.1, amino acid sequence: SEQ ID NO: 291, encoding nucleotide sequence: SEQ ID NO: 290).

The amino acid sequences set forth in SEQ ID NOs: 307 to 318 were detected by the mass spectrometry. The protein identified from this mass data was hypothetical protein GLYMA_02G241100 (NCBI accession number KRH72929.1, amino acid sequence: SEQ ID NO: 306, encoding nucleotide sequence: SEQ ID NO: 305).

### Spot 17α:

The amino acid sequences set forth in SEQ ID NOs: 321 to 325 were detected by the mass spectrometry. The protein identified from this mass data was guanosine nucleotide diphosphate dissociation inhibitor 2 (NCBI accession number XP_003531293.1, amino acid sequence: SEQ ID NO: 320, encoding nucleotide sequence: SEQ ID NO: 319).

The amino acid sequences set forth in SEQ ID NOs: 328 to 332 were detected by the mass spectrometry. The protein identified from this mass data was rab GDP dissociation inhibitor alpha-like (NCBI accession number NP_001276273.1, amino acid sequence: SEQ ID NO: 327, encoding nucleotide sequence: SEQ ID NO: 326).

### Spot 18α:

The amino acid sequences set forth in SEQ ID NOs: 335 to 339 were detected by the mass spectrometry. The protein identified from this mass data was hypothetical protein GLYMA_10G028300 (NCBI accession number KRH32039.1, amino acid sequence: SEQ ID NO: 334, encoding nucleotide sequence: SEQ ID NO: 333).

### Spot 19α:

The amino acid sequences set forth in SEQ ID NOs: 342 to 346 were detected by the mass spectrometry. The protein identified from this mass data was sucrose binding protein homolog S-64 (NCBI accession number AAF05723.1, amino acid sequence: SEQ ID NO: 341, encoding nucleotide sequence: SEQ ID NO: 340).

### Spot 20α:

The amino acid sequences set forth in SEQ ID NOs: 35 to 53 were detected by the mass spectrometry. The protein identified from this mass data was Seed biotin-containing protein SBP65 (UniProt accession number Q39846, amino acid sequence: SEQ ID NO: 12, encoding nucleotide sequence: SEQ ID NO: 11).

### Spot 21α:

The amino acid sequences set forth in SEQ ID NOs: 21 to 28 were detected by the mass spectrometry. The protein identified from this mass data was Ribulose bisphosphate carboxylase large chain (UniProt accession number P27066, amino acid sequence: SEQ ID NO: 4, encoding nucleotide sequence: SEQ ID NO: 3).

### Spot 22α:

The amino acid sequences set forth in SEQ ID NOs: 29, 30 were detected by the mass spectrometry. The protein identified from this mass data was Gamma-glutamyl hydrolase (UniProt accession number P93164, amino acid sequence: SEQ ID NO: 6, encoding nucleotide sequence: SEQ ID NO: 5).

### Spot 23α:

The amino acid sequences set forth in SEQ ID NOs: 31, 32 were detected by the mass spectrometry. The protein identified from this mass data was Guanine nucleotide-binding protein subunit beta-like protein (UniProt accession number Q39836, amino acid sequence: SEQ ID NO: 8, encoding nucleotide sequence: SEQ ID NO: 7).

### Spot 24α:

The amino acid sequences set forth in SEQ ID NOs: 33, 34 were detected by the mass spectrometry. The protein identified from this mass data was Protein SLE3 (UniProt accession number C6T0L2, amino acid sequence: SEQ ID NO: 10, encoding nucleotide sequence: SEQ ID NO: 9).

### Spot 25α:

The amino acid sequences set forth in SEQ ID NOs: 349 to 356 were detected by the mass spectrometry. The protein identified from this mass data was succinyl-CoA ligase [ADP-forming] subunit beta, mitochondrial (NCBI accession number XP_003537078.1, amino acid sequence: SEQ ID NO: 348, encoding nucleotide sequence: SEQ ID NO: 347).

The amino acid sequences set forth in SEQ ID NOs: 359 to 365 were detected by the mass spectrometry. The protein identified from this mass data was succinyl-CoA ligase [ADP-forming] subunit beta, mitochondrial (NCBI accession number XP_003542431.1, amino acid sequence: SEQ ID NO: 358, encoding nucleotide sequence: SEQ ID NO: 357).

### Spot 26α:

The amino acid sequences set forth in SEQ ID NOs: 368 to 382 were detected by the mass spectrometry. The protein identified from this mass data was phosphoglycerate kinase, cytosolic (NCBI accession number XP_003546821.1, amino acid sequence: SEQ ID NO: 367, encoding nucleotide sequence: SEQ ID NO: 366).

The amino acid sequences set forth in SEQ ID NOs: 385 to 392 were detected by the mass spectrometry. The protein identified from this mass data was phosphoglycerate kinase, cytosolic (NCBI accession number XP_003531482.1, amino acid sequence: SEQ ID NO: 384, encoding nucleotide sequence: SEQ ID NO: 383).

The amino acid sequences set forth in SEQ ID NOs: 395 to 409 were detected by the mass spectrometry. The protein identified from this mass data was phosphoglycerate kinase, cytosolic (NCBI accession number XP_003531483.1, amino acid sequence: SEQ ID NO: 394, encoding nucleotide sequence: SEQ ID NO: 393).

### Spot 27α:

The amino acid sequences set forth in SEQ ID NOs: 412 to 416 were detected by the mass spectrometry. The protein identified from this mass data was alcohol dehydrogenase 1 (NCBI accession number XP_003526673.1, amino acid sequence: SEQ ID NO: 411, encoding nucleotide sequence: SEQ ID NO: 410).

The amino acid sequences set forth in SEQ ID NOs: 419, 420 were detected by the mass spectrometry. The protein identified from this mass data was alcohol dehydrogenase 1 (NCBI accession number XP_003545664.3, amino acid sequence: SEQ ID NO: 418, encoding nucleotide sequence: SEQ ID NO: 417).

The amino acid sequences set forth in SEQ ID NOs: 423 to 427 were detected by the mass spectrometry. The protein identified from this mass data was alcohol dehydrogenase 1-like (NCBI accession number XP_003544738.1, amino acid sequence: SEQ ID NO: 422, encoding nucleotide sequence: SEQ ID NO: 421).

### Spot 28α:

The amino acid sequences set forth in SEQ ID NOs: 430 to 433 were detected by the mass spectrometry. The protein identified from this mass data was 35 kDa seed maturation protein isoform X1 (NCBI accession number XP_006576267.1, amino acid sequence: SEQ ID NO: 429, encoding nucleotide sequence: SEQ ID NO: 428).

### Spots 29α, 30α, 31α, 32α, 33α:

The amino acid sequences set forth in SEQ ID NOs: 436 to 439 were detected by the mass spectrometry. The protein identified from this mass data was glyceraldehyde-3-phosphate dehydrogenase isoform X1 (NCBI accession number XP_014631598.1, amino acid sequence: SEQ ID NO: 435, encoding nucleotide sequence: SEQ ID NO: 434).

The amino acid sequences set forth in SEQ ID NOs: 442 to 445 were detected by the mass spectrometry. The protein identified from this mass data was glyceraldehyde-3-phosphate dehydrogenase, cytosolic (NCBI accession number XP_006578692.1, amino acid sequence: SEQ ID NO: 441, encoding nucleotide sequence: SEQ ID NO: 440).

The amino acid sequences set forth in SEQ ID NOs: 448 to 451 were detected by the mass spectrometry. The protein identified from this mass data was uncharacterized protein LOC100782924 (NCBI accession number NP_001240046.1, amino acid sequence: SEQ ID NO: 447, encoding nucleotide sequence: SEQ ID NO: 446).

### Spot 34α:

The amino acid sequence set forth in SEQ ID NO: 454 was detected by the mass spectrometry. The protein identified from this mass data was bifunctional UDP-glucose 4-epimerase and UDP-xylose 4-epimerase 1-like (NCBI accession number XP_003532591.1, amino acid sequence: SEQ ID NO: 453, encoding nucleotide sequence: SEQ ID NO: 452).

The amino acid sequences set forth in SEQ ID NOs: 457, 458 were detected by the mass spectrometry. The protein identified from this mass data was uncharacterized protein LOC100803483 (NCBI accession number NP_001241075.1, amino acid sequence: SEQ ID NO: 456, encoding nucleotide sequence: SEQ ID NO: 455).

### Spot 35α:

The amino acid sequences set forth in SEQ ID NOs: 461 to 466 were detected by the mass spectrometry. The protein identified from this mass data was elongation factor 1-alpha (NCBI accession number XP_003553292.1, amino acid sequence: SEQ ID NO: 460, encoding nucleotide sequence: SEQ ID NO: 459).

The amino acid sequences set forth in SEQ ID NOs: 469 to 471 were detected by the mass spectrometry. The protein identified from this mass data was elongation factor 1-alpha (NCBI accession number XP_003547695.1, amino acid sequence: SEQ ID NO: 468, encoding nucleotide sequence: SEQ ID NO: 467).

The amino acid sequences set forth in SEQ ID NOs: 474 to 477 were detected by the mass spectrometry. The protein identified from this mass data was elongation factor-1A isoform X1 (NCBI accession number XP_006600131.1, amino acid sequence: SEQ ID NO: 473, encoding nucleotide sequence: SEQ ID NO: 472).

### Spots 36α, 37α:

The amino acid sequences set forth in SEQ ID NOs: 480 to 483 were detected by the mass spectrometry. The protein identified from this mass data was seed maturation protein PM34 (NCBI accession number NP_001238285.1, amino acid sequence: SEQ ID NO: 479, encoding nucleotide sequence: SEQ ID NO: 478).

The amino acid sequences set forth in SEQ ID NOs: 486, 487 were detected by the mass spectrometry. The protein identified from this mass data was uncharacterized protein LOC100809384 (NCBI accession number NP_001241158.1, amino acid sequence: SEQ ID NO: 485, encoding nucleotide sequence: SEQ ID NO: 484).

The amino acid sequences set forth in SEQ ID NOs: 490 to 493 were detected by the mass spectrometry. The protein identified from this mass data was hypothetical protein GLYMA_10G155300 (NCBI accession number KRH33956.1, amino acid sequence: SEQ ID NO: 489, encoding nucleotide sequence: SEQ ID NO: 488).

### Spot 38α

The amino acid sequences set forth in SEQ ID NOs: 496 to 513 were detected by the mass spectrometry. The protein identified from this mass data was seed biotin-containing protein SBP65-like isoform X1 (NCBI accession number XP_003526237.1, amino acid sequence: SEQ ID NO: 495, encoding nucleotide sequence: SEQ ID NO: 494).

### Spot 39α:

The amino acid sequence set forth in SEQ ID NO: 516 was detected by the mass spectrometry. The protein identified from this mass data was ATP synthase subunit O, mitochondrial-like (NCBI accession number XP_003546884.1, amino acid sequence: SEQ ID NO: 515, encoding nucleotide sequence: SEQ ID NO: 514).

### Spots 40α, 56α:

The amino acid sequences set forth in SEQ ID NOs: 519, 520 were detected by the mass spectrometry. The protein identified from this mass data was P24 oleosin isoform A (NCBI accession number XP_003553822.1, amino acid sequence: SEQ ID NO: 518, encoding nucleotide sequence: SEQ ID NO: 517).

### Spot 41α:

The amino acid sequence set forth in SEQ ID NO: 523 was detected by the mass spectrometry. The protein identified from this mass data was uncharacterized protein At3g49720-like isoform X2 (NCBI accession number XP_006600908.1, amino acid sequence: SEQ ID NO: 522, encoding nucleotide sequence: SEQ ID NO: 521).

### Spots 42α, 43α

The amino acid sequences set forth in SEQ ID NOs: 526 to 531 were detected by the mass spectrometry. The protein identified from this mass data was superoxide dismutase [Mn], mitochondrial (NCBI accession number XP_003526813.1, amino acid sequence: SEQ ID NO: 525, encoding nucleotide sequence: SEQ ID NO: 524).

The amino acid sequences set forth in SEQ ID NOs: 534 to 539 were detected by the mass spectrometry. The protein identified from this mass data was hypothetical protein GLYMA_04G221300 (NCBI accession number KRH64185.1, amino acid sequence: SEQ ID NO: 533, encoding nucleotide sequence: SEQ ID NO: 532).

### Spot 44α:

The amino acid sequences set forth in SEQ ID NOs: 54 to 61 were detected by the mass spectrometry. The protein identified from this mass data was Ferritin-2 (UniProt accession number Q94IC4, amino acid sequence: SEQ ID NO: 14, encoding nucleotide sequence: SEQ ID NO: 13).

### Spot 45α:

The amino acid sequences set forth in SEQ ID NOs: 542 to 545 were detected by the mass spectrometry. The protein identified from this mass data was uncharacterized protein LOC100499771 (NCBI accession number NP_001235797.1, amino acid sequence: SEQ ID NO: 541, encoding nucleotide sequence: SEQ ID NO: 540).

The amino acid sequences set forth in SEQ ID NOs: 548 to 551 were detected by the mass spectrometry. The protein identified from this mass data was hypothetical protein GLYMA_09G192800 (NCBI accession number KRH39322.1, amino acid sequence: SEQ ID NO: 547, encoding nucleotide sequence: SEQ ID NO: 546).

### Spot 46α:

The amino acid sequence set forth in SEQ ID NO: 554 was detected by the mass spectrometry. The protein identified from this mass data was seed maturation protein PM22 (NCBI accession number NP_001237935.1, amino acid sequence: SEQ ID NO: 553, encoding nucleotide sequence: SEQ ID NO: 552).

### Spot 47α:

The amino acid sequences set forth in SEQ ID NOs: 557, 558 were detected by the mass spectrometry. The protein identified from this mass data was eukaryotic translation initiation factor 5A-2 (NCBI accession number XP_003549690.1, amino acid sequence: SEQ ID NO: 556, encoding nucleotide sequence: SEQ ID NO: 555).

The amino acid sequences set forth in SEQ ID NOs: 561 to 563 were detected by the mass spectrometry. The protein identified from this mass data was uncharacterized protein LOC100305510 (NCBI accession number NP_001236395.1, amino acid sequence: SEQ ID NO: 560, encoding nucleotide sequence: SEQ ID NO: 559).

### Spot 48α:

The amino acid sequences set forth in SEQ ID NOs: 566 to 570 were detected by the mass spectrometry. The protein identified from this mass data was uncharacterized protein LOC100306570 (NCBI accession number NP_001236895.1, amino acid sequence: SEQ ID NO: 565, encoding nucleotide sequence: SEQ ID NO: 564).

### Spot 49α:

The amino acid sequences set forth in SEQ ID NOs: 17 to 20 were detected by the mass spectrometry. The protein identified from this mass data was Bowman-Birk type proteinase inhibitor D-II (UniProt accession number P01064, amino acid sequence: SEQ ID NO: 2, encoding nucleotide sequence: SEQ ID NO: 1).

### Spot 50α:

The amino acid sequences set forth in SEQ ID NOs: 573 to 576 were detected by the mass spectrometry. The protein identified from this mass data was uncharacterized protein LOC100813859 (NCBI accession number NP_001239816.1, amino acid sequence: SEQ ID NO: 572, encoding nucleotide sequence: SEQ ID NO: 571).

### Spot 51α:

The amino acid sequences set forth in SEQ ID NOs: 579 to 582 were detected by the mass spectrometry. The protein identified from this mass data was 14-3-3-like protein (NCBI accession number XP_003526571.1, amino acid sequence: SEQ ID NO: 578, encoding nucleotide sequence: SEQ ID NO: 577).

### Spot 52α:

The amino acid sequences set forth in SEQ ID NOs: 585 to 588 were detected by the mass spectrometry. The protein identified from this mass data was probable fructokinase-4 (NCBI accession number XP_003543564.1, amino acid sequence: SEQ ID NO: 584, encoding nucleotide sequence: SEQ ID NO: 583).

### Spot 53α:

The amino acid sequences set forth in SEQ ID NOs: 591 to 597 were detected by the mass spectrometry. The protein identified from this mass data was triosephosphate isomerase isoform X1 (NCBI accession number XP_006593480.2, amino acid sequence: SEQ ID NO: 590, encoding nucleotide sequence: SEQ ID NO: 589).

The amino acid sequences set forth in SEQ ID NOs: 600 to 604 were detected by the mass spectrometry. The protein identified from this mass data was triosephosphate isomerase, cytosolic (NCBI accession number XP_003547334.1, amino acid sequence: SEQ ID NO: 599, encoding nucleotide sequence: SEQ ID NO: 598).

### Spot 54α:

The amino acid sequences set forth in SEQ ID NOs: 607 to 611 were detected by the mass spectrometry. The protein identified from this mass data was superoxide dismutase [Fe], chloroplastic isoform X1 (NCBI accession number XP_014627751.1, amino acid sequence: SEQ ID NO: 606, encoding nucleotide sequence: SEQ ID NO: 605).

The amino acid sequences set forth in SEQ ID NOs: 614 to 616 were detected by the mass spectrometry. The protein identified from this mass data was iron-superoxide dismutase (NCBI accession number NP_001237901.1, amino acid sequence: SEQ ID NO: 613, encoding nucleotide sequence: SEQ ID NO: 612).

### Spot 55α:

The amino acid sequences set forth in SEQ ID NOs: 619 to 635 were detected by the mass spectrometry. The protein identified from this mass data was 51 kDa seed maturation protein precursor (NCBI accession number NP_001238138.1, amino acid sequence: SEQ ID NO: 618, encoding nucleotide sequence: SEQ ID NO: 617).

The amino acid sequences set forth in SEQ ID NOs: 638 to 649 were detected by the mass spectrometry. The protein identified from this mass data was Lea protein precursor (NCBI accession number NP_001238201.1, amino acid sequence: SEQ ID NO: 637, encoding nucleotide sequence: SEQ ID NO: 636).

### Example 4: Identification of epitopes (1)

Identification of epitopes was carried out by the following procedure as to each of the antigen proteins identified in Example 3.

### (A) Epitope mapping

Epitope mapping was carried out using a library of overlapping peptide fragments (length: 15 amino acids) corresponding to the sequences of proteins bound patient-specifically to IgE antibodies in serum, which were obtained as a result of 2D-western of soybeans.

The peptides to be synthesized were shifted by 10 amino acids. Thus, each peptide had an overlap of 5 amino acids with each of the peptides previous and subsequent thereto.

For preparation of peptide arrays, peptide synthesis of interest was carried out in incremental steps on a modified cellulose membrane by the Intavis SPOT synthesis technique, which is a technique of synthesizing peptides of interest through 9-fluorenylmethoxycarbonyl (Fmoc) chemical reaction, using an automated synthesis apparatus (Intavis MultiPep RS). The aforementioned modified cellulose membrane (hereinafter also referred to as the membrane) bound to the obtained peptides of interest was used in epitope mapping.

The presence or absence of binding, to each peptide fragment, of an IgE antibody present in the serum of a patient allergic to a soybean was measured by immunoblotting using the membrane on which the peptide fragments were synthesized by SPOT. Immunoblotting of the membrane was carried out according to the following procedure using, as a primary antibody, a serum sample from an allergic patient, and anti-human IgE-HRP as a secondary antibody.
(1) The membrane was subjected to hydrophilization with methanol for 5 minutes.
(2) The hydrophilized membrane was shaken overnight in a 5% skim milk/PBST solution (a PBS buffer containing 0.1% Tween 20 nonionic surfactant) at 4°C.
(3) The membrane was washed with a PBST solution three times.
(4) The serum sample (1:40, a 5% skim milk/PBST solution) was added thereto as a primary antibody and shaken overnight at 4°C. In this operation, a protease inhibitor (Complete, produced by Roche) was added.
(5) The membrane was washed with a PBST solution three times.
(6) Anti-human IgE-HRP (1:10,000, a 5% skim milk/PBST solution) was added thereto as a secondary antibody and shaken at room temperature for one hour.
(7) The membrane was washed with a PBST solution three times.
(8) The membrane was left to stand in ECL plus (produced by Thermo Fisher Scientific) for 5 minutes.
(9) The membrane treated as described above in (1) to (8) was subjected to fluorescent image scanning using Amersham Imager 600.

Peptide fragments bound to IgE antibodies from allergic patients in immunoblotting were used as candidate peptides of epitopes.

### (B) Overlapping

Thereafter, for the sequences of peptides bound patient-specifically to IgE antibodies in serum in the epitope mapping described above in (A), a library of overlapping peptide fragments (length: 10 amino acids) was obtained as sequences having the sequences of these peptides as well as additional sequences subsequent to the peptides in the amino acid sequences of antigen proteins on which the epitopes were detected. Synthesis of this library was carried out by the Fmoc chemical synthesis method in the same manner as in (A) mentioned above. The peptides to be synthesized were shifted by one amino acid. Thus, each peptide had an overlap of 9 amino acids with the peptide subsequent thereto.

The presence or absence of binding of an IgE antibody from an allergic patient to each peptide fragment in this library of overlapping peptide fragments was measured by immunoblotting in the same manner as in (A) mentioned above. A common sequence of peptides bound to IgE antibodies from allergic patients was identified to determine peptides having the minimum number of amino acids functioning as an epitope. The peptides determined in (B) are referred to as the "original sequences".

As one example of the results, the results of determining the original sequences by the overlapping technique as to a partial sequence (a region of amino acids 571-590 of SEQ ID NO: 495/SEQ ID NO: 964 of seed biotin-containing protein SBP65-like isoform X1 (spot 38α) are shown in Fig. 4. Strong binding of polypeptides having the amino acid sequences of SEQ ID NOs: 965-967 to the IgE antibody from the allergic patient was observed. Slightly weak binding of SEQ ID NO: 968 to the IgE antibody from the allergic patient was observed. Binding of SEQ ID NOs: 969-975 to the IgE antibody from the allergic patient was not observed. As a result, SEQ ID NO: 864, a common sequence of SEQ ID NOs: 965-968, was identified as an original sequence.

### (C) Alanine scanning

From the "original sequences" determined as described above in (B), a library of peptide fragments in which N-terminal amino acids were substituted one by one by alanine according to a technique called alanine scanning (Non Patent Literature 1) was prepared by the Fmoc chemical synthesis method in the same manner as in (A) mentioned above. The presence or absence of binding of an IgE antibody present in the serum of an allergic patient to each peptide fragment was measured by immunoblotting in the same manner as in (A) mentioned above. Amino acids at positions where the binding activity against IgE antibodies from allergic patients was lost by the substitution by alanine were confirmed as positions important for exertion of antigenicity. Also, Amino acids at positions where the binding activity against IgE antibodies from allergic patients was maintained even after the substitution by alanine were confirmed as substitutable positions that were not important for exertion of antigenicity. Common sequences (consensus sequences) important for exertion of antigenicity were found by this analysis.

As one example of the results, the results of performing alanine scanning as to the amino acid sequence of SEQ ID NO: 864 identified in (B) are shown in Fig. 5. Peptides having SEQ ID NOs: 977-979 in which the amino acids at positions 1-3, respectively, of SEQ ID NO: 864 were substituted by alanine lost their binding activity against IgE antibodies from allergic patients. Thus, the amino acids at positions 1-3 of SEQ ID NO: 864 were identified as amino acids important for the binding to IgE antibodies from allergic patients.

### (D) Results

### (1β) Epitope of endoplasmin homolog isoform X2 (spot 1α)

SEQ ID NOs: 652, 655, 657, and 658-660 were identified as epitopes of endoplasmin homolog isoform X2 by the procedure of (B) mentioned above.

Amino acids important for binding to IgE antibodies from allergic patients were identified by the procedure of (C) mentioned above as to each of SEQ ID NOs: 652, 655, 657, and 658-660.

It was revealed for SEQ ID NO: 652 that the amino acids at positions 3 and 5 are amino acids particularly important for the binding to IgE antibodies from allergic patients, and the amino acid at position 2 is an amino acid that influences the binding to IgE antibodies from allergic patients. The amino acids at positions 1, 4, and 6-8 of SEQ ID NO: 652 had no influence on the binding activity against IgE antibodies from allergic patients when substituted by alanine.

It was revealed for SEQ ID NO: 655 that the amino acids at positions 4-7 are amino acids particularly important for the binding to IgE antibodies from allergic patients, and the amino acid at position 2 is an amino acid that influences the binding to IgE antibodies from allergic patients. The amino acids at positions 1, 3, 8 and 9 of SEQ ID NO: 655 had no influence on the binding activity against IgE antibodies from allergic patients when substituted by alanine.

For SEQ ID NO: 657, the amino acids at positions 1-5 and 11 were amino acids particularly important for the binding to IgE antibodies from allergic patients. The amino acids at positions 6-10 and 12-15 of SEQ ID NO: 657 had no influence on the binding activity against IgE antibodies from allergic patients when substituted by alanine.

For SEQ ID NOs: 658-660, all the amino acids were amino acids particularly important for the binding to IgE antibodies from allergic patients.

### (2β) Epitope of heat shock cognate protein 80 (spot 2α)

SEQ ID NOs: 663, 666, 669, 670-672, 675, 676-678, 681, 684, 687, 689, and 690-692 were identified as epitopes of heat shock cognate protein 80 by the procedure of (B) mentioned above.

Amino acids important for binding to IgE antibodies from allergic patients were identified by the procedure of (C) mentioned above as to each of SEQ ID NOs: 663, 666, 669, 670-672, 675-678, 681, 684, 687 and 689-692.

It was revealed for SEQ ID NO: 663 that the amino acids at positions 1, 4 and 5 are amino acids particularly important for the binding to IgE antibodies from allergic patients, and the amino acid at position 7 is an amino acid that influences the binding to IgE antibodies from allergic patients. The amino acids at positions 2, 3, 6, 8 and 9 of SEQ ID NO: 663 had no influence on the binding activity against IgE antibodies from allergic patients when substituted by alanine.

It was revealed for SEQ ID NO: 666 that the amino acids at positions 5-8 are amino acids particularly important for the binding to IgE antibodies from allergic patients, and the amino acid at position 12 is an amino acid that influences the binding to IgE antibodies from allergic patients. The amino acids at positions 1-4, 9-11, and 13-15 of SEQ ID NO: 666 had no influence on the binding activity against IgE antibodies from allergic patients when substituted by alanine.

It was revealed for SEQ ID NO: 669 that the amino acids at positions 2-4 are amino acids particularly important for the binding to IgE antibodies from allergic patients, and the amino acid at position 1 is an amino acid that influences the binding to IgE antibodies from allergic patients. The amino acid at position 5 of SEQ ID NO: 669 had no influence on the binding activity against IgE antibodies from allergic patients when substituted by alanine.

It was revealed for SEQ ID NO: 675 that the amino acids at positions 3-5, 13 and 14 are amino acids particularly important for the binding to IgE antibodies from allergic patients, and the amino acid at position 15 is an amino acid that influences the binding to IgE antibodies from allergic patients. The amino acids at positions 1, 2 and 6-12 of SEQ ID NO: 675 had no influence on the binding activity against IgE antibodies from allergic patients when substituted by alanine.

It was revealed for SEQ ID NO: 681 that the amino acids at positions 5, 10 and 14 are amino acids particularly important for the binding to IgE antibodies from allergic patients, and the amino acids at positions 6-8 and 15 are amino acids that influence the binding to IgE antibodies from allergic patients. The amino acids at positions 1-4, 9 and 11-13 of SEQ ID NO: 681 had no influence on the binding activity against IgE antibodies from allergic patients when substituted by alanine.

It was revealed for SEQ ID NO: 684 that the amino acids at positions 2, 5, 10, and 11 are amino acids particularly important for the binding to IgE antibodies from allergic patients. The amino acids at positions 1, 3, 4, 6-9, and 12-15 of SEQ ID NO: 684 had no influence on the binding activity against IgE antibodies from allergic patients when substituted by alanine.

It was revealed for SEQ ID NO: 687 that the amino acids at positions 1, 5, 10, and 11 are amino acids particularly important for the binding to IgE antibodies from allergic patients. The amino acids at positions 2, 3, 4, 6-9 and 12-15 of SEQ ID NO: 687 had no influence on the binding activity against IgE antibodies from allergic patients when substituted by alanine.

It was revealed for SEQ ID NO: 689 that the amino acids at positions 1-3, 12 and 13 are amino acids particularly important for the binding to IgE antibodies from allergic patients. The amino acids at positions 4-11, 14 and 15 of SEQ ID NO: 689 had no influence on the binding activity against IgE antibodies from allergic patients when substituted by alanine.

For SEQ ID NOs: 670-672, 676-678, and 690-692, all the amino acids were amino acids particularly important for the binding to IgE antibodies from allergic patients.

### (3β) Epitope of Cell division cycle protein 48 homolog (spot 3α)

SEQ ID NOs: 695, 697, 699, and 700-716 were identified as epitopes of Cell division cycle protein 48 homolog by the procedure of (B) mentioned above.

Amino acids important for binding to IgE antibodies from allergic patients were identified by the procedure of (C) mentioned above as to each of SEQ ID NOs: 695, 697, 699 and 700-716.

It was revealed for SEQ ID NO: 695 that the amino acids at positions 11, 12 and 14 are amino acids particularly important for the binding to IgE antibodies from allergic patients, and the amino acids at positions 4-10, 13 and 15 are amino acids that influence the binding to IgE antibodies from allergic patients. The amino acids at positions 1-3 of SEQ ID NO: 695 had no influence on the binding activity against IgE antibodies from allergic patients when substituted by alanine.

It was revealed for SEQ ID NO: 697 that the amino acids at positions 14 and 15 are amino acids particularly important for the binding to IgE antibodies from allergic patients. The amino acids at positions 1-13 of SEQ ID NO: 697 had no influence on the binding activity against IgE antibodies from allergic patients when substituted by alanine.

It was revealed for SEQ ID NO: 699 that the amino acids at positions 5-7 are amino acids particularly important for the binding to IgE antibodies from allergic patients. The amino acids at positions 1-4 of SEQ ID NO: 699 had no influence on the binding activity against IgE antibodies from allergic patients when substituted by alanine.

For SEQ ID NOs: 700-716, all the amino acids were amino acids particularly important for the binding to IgE antibodies from allergic patients.

### (4β) Epitope of embryo-specific urease isoform X1 (spot 4α)

SEQ ID NOs: 719, 722, 725, 726-728, and 731 were identified as epitopes of embryo-specific urease isoform X1 by the procedure of (B) mentioned above.

Amino acids important for binding to IgE antibodies from allergic patients were identified by the procedure of (C) mentioned above as to each of SEQ ID NOs: 719, 722, 725, 726-728, and 731.

It was revealed for SEQ ID NO: 719 that the amino acids at positions 8-10 are amino acids particularly important for the binding to IgE antibodies from allergic patients, and the amino acid at position 6 is an amino acid that influences the binding to IgE antibodies from allergic patients. The amino acids at positions 1-5 and 7 of SEQ ID NO: 719 had no influence on the binding activity against IgE antibodies from allergic patients when substituted by alanine.

It was revealed for SEQ ID NO: 722 that the amino acids at positions 1 and 4-6 are amino acids particularly important for the binding to IgE antibodies from allergic patients, and the amino acid at position 2 is an amino acid that influences the binding to IgE antibodies from allergic patients. The amino acids at positions 3 and 7 of SEQ ID NO: 722 had no influence on the binding activity against IgE antibodies from allergic patients when substituted by alanine.

It was revealed for SEQ ID NO: 725 that the amino acids at positions 4, 6, 8 and 10 are amino acids particularly important for the binding to IgE antibodies from allergic patients, and the amino acid at position 9 is an amino acid that influences the binding to IgE antibodies from allergic patients. The amino acids at positions 1-3, 5 and 7 of SEQ ID NO: 725 had no influence on the binding activity against IgE antibodies from allergic patients when substituted by alanine.

It was revealed for SEQ ID NO: 731 that the amino acids at positions 4, 6, 10, 11, 14 and 15 are amino acids particularly important for the binding to IgE antibodies from allergic patients, and the amino acid at position 3 is an amino acid that influences the binding to IgE antibodies from allergic patients. The amino acids at positions 1, 2, 5, 7-9, 12 and 13 of SEQ ID NO: 731 had no influence on the binding activity against IgE antibodies from allergic patients when substituted by alanine.

For SEQ ID NOs: 726-728, all the amino acids were amino acids particularly important for the binding to IgE antibodies from allergic patients.

### (5β) Epitope of alpha-1,4 glucan phosphorylase L isozyme, chloroplastic/amyloplastic (spot 5α)

SEQ ID NOs: 734 and 735-738 were identified as epitopes of alpha-1,4 glucan phosphorylase L isozyme, chloroplastic/amyloplastic by the procedure of (B) mentioned above.

Amino acids important for binding to IgE antibodies from allergic patients were identified by the procedure of (C) mentioned above as to each of SEQ ID NOs: 734, 735-738.

It was revealed for SEQ ID NO: 734 that the amino acids at positions 7, 8, 10, 12 and 14 are amino acids particularly important for the binding to IgE antibodies from allergic patients, and the amino acids at positions 15 is an amino acid that influences the binding to IgE antibodies from allergic patients. The amino acids at positions 1-6, 9, 11 and 13 of SEQ ID NO: 734 had no influence on the binding activity against IgE antibodies from allergic patients when substituted by alanine.

For SEQ ID NOs: 735-738, all the amino acids were amino acids particularly important for the binding to IgE antibodies from allergic patients.

### (6β) Epitope of aconitate hydratase 1 (spot 6α)

SEQ ID NOs: 739 and 740 were identified as epitopes of aconitate hydratase 1 by the procedure of (B) mentioned above.

Amino acids important for binding to IgE antibodies from allergic patients were identified by the procedure of (C) mentioned above as to each of SEQ ID NOs: 739 and 740.

For SEQ ID NOs: 739 and 740, all the amino acids were amino acids particularly important for the binding to IgE antibodies from allergic patients.

### (7β) Epitope of methionine synthase (spot 7α)

SEQ ID NOs: 741 and 744 were identified as epitopes of methionine synthase by the procedure of (B) mentioned above.

Amino acids important for binding to IgE antibodies from allergic patients were identified by the procedure of (C) mentioned above as to each of SEQ ID NOs: 741 and 744.

It was revealed for SEQ ID NO: 744 that the amino acids at positions 8, 9, 11-13 and 15 are amino acids particularly important for the binding to IgE antibodies from allergic patients, and the amino acid at position 10 is an amino acid that influences the binding to IgE antibodies from allergic patients. The amino acids at positions 1-7 and 14 of SEQ ID NO: 744 had no influence on the binding activity against IgE antibodies from allergic patients when substituted by alanine.

For SEQ ID NO: 741, all the amino acids were amino acids particularly important for the binding to IgE antibodies from allergic patients.

### (9β) Epitope of lysine--tRNA ligase, cytoplasmic-like isoform X2 (spot 9α)

SEQ ID NOs: 747 and 749 were identified as epitopes of lysine--tRNA ligase, cytoplasmic-like isoform X2 by the procedure of (B) mentioned above.

Amino acids important for binding to IgE antibodies from allergic patients were identified by the procedure of (C) mentioned above as to each of SEQ ID NOs: 747 and 749.

It was revealed for SEQ ID NO: 747 that the amino acids at positions 3, 8 and 9 are amino acids particularly important for the binding to IgE antibodies from allergic patients, and the amino acid at position 1 is an amino acid that influences the binding to IgE antibodies from allergic patients. The amino acids at positions 2, 4-7 and 10 of SEQ ID NO: 747 had no influence on the binding activity against IgE antibodies from allergic patients when substituted by alanine.

It was revealed for SEQ ID NO: 749 that the amino acids at positions 1-5, 7, and 8 are amino acids particularly important for the binding to IgE antibodies from allergic patients. The amino acid at position 6 of SEQ ID NO: 749 had no influence on the binding activity against IgE antibodies from allergic patients when substituted by alanine.

### (11β) Epitope of polyadenylate-binding protein 8 isoform X3 (spot 11α)

SEQ ID NOs: 753 and 754 were identified as epitopes of polyadenylate-binding protein 8 isoform X3 by the procedure of (B) mentioned above.

Amino acids important for binding to IgE antibodies from allergic patients were identified by the procedure of (C) mentioned above as to each of SEQ ID NOs: 753 and 754.

It was revealed for SEQ ID NO: 753 that the amino acids at positions 1, 2, 4 and 5 are amino acids particularly important for the binding to IgE antibodies from allergic patients, and the amino acid at position 6 is an amino acid that influences the binding to IgE antibodies from allergic patients. The amino acids at positions 3 and 7-10 of SEQ ID NO: 753 had no influence on the binding activity against IgE antibodies from allergic patients when substituted by alanine.

For SEQ ID NO: 754, all the amino acids were amino acids particularly important for the binding to IgE antibodies from allergic patients.

### (12β) Epitope of pyrophosphate--fructose 6-phosphate 1-phosphotransferase subunit alpha-like (spot 12α)

SEQ ID NO: 755 was identified as an epitope of pyrophosphate--fructose 6-phosphate 1-phosphotransferase subunit alpha-like by the procedure of (B) mentioned above.

Amino acids important for binding to IgE antibodies from allergic patients were identified by the procedure of (C) mentioned above as to SEQ ID NO: 755.

For SEQ ID NO: 755, all the amino acids were amino acids particularly important for the binding to IgE antibodies from allergic patients.

### (13β) Epitope of protein disulfide isomerase-like protein precursor (spot 13α)

SEQ ID NO: 758 was identified as an epitope of protein disulfide isomerase-like protein precursor by the procedure of (B) mentioned above.

Amino acids important for binding to IgE antibodies from allergic patients were identified by the procedure of (C) mentioned above as to SEQ ID NO: 758.

It was revealed for SEQ ID NO: 758 that the amino acids at positions 3 and 15 are amino acids particularly important for the binding to IgE antibodies from allergic patients, and the amino acids at positions 5, 10, 12 and 13 are amino acids that influence the binding to IgE antibodies from allergic patients. The amino acids at positions 1, 2, 4, 6-9, 11 and 14 of SEQ ID NO: 758 had no influence on the binding activity against IgE antibodies from allergic patients when substituted by alanine.

### (14β) Epitope of V-type proton ATPase subunit B 2 (spot 14α)

SEQ ID NOs: 761 and 764 were identified as epitopes of V-type proton ATPase subunit B 2 by the procedure of (B) mentioned above.

Amino acids important for binding to IgE antibodies from allergic patients were identified by the procedure of (C) mentioned above as to each of SEQ ID NOs: 761 and 764.

It was revealed for SEQ ID NO: 761 that the amino acids at positions 6, 7 and 12-15 are amino acids particularly important for the binding to IgE antibodies from allergic patients, and the amino acid at position 8 is an amino acid that influences the binding to IgE antibodies from allergic patients. The amino acids at positions 1-5 and 9-11 of SEQ ID NO: 761 had no influence on the binding activity against IgE antibodies from allergic patients when substituted by alanine.

It was revealed for SEQ ID NO: 764 that the amino acids at positions 1, 2, 12 and 14 are amino acids particularly important for the binding to IgE antibodies from allergic patients, and the amino acids at positions 3-5, 11 and 13 are amino acids that influence the binding to IgE antibodies from allergic patients. The amino acids at positions 6-10 and 15 of SEQ ID NO: 746 had no influence on the binding activity against IgE antibodies from allergic patients when substituted by alanine.

### (15β) Epitope of UTP-glucose-1-phosphate uridylyltransferase (spots 15α and 16α)

SEQ ID NO: 766 was identified as an epitope of UTP-glucose-1-phosphate uridylyltransferase by the procedure of (B) mentioned above.

Amino acids important for binding to IgE antibodies from allergic patients were identified by the procedure of (C) mentioned above as to SEQ ID NOs: 766.

It was revealed for SEQ ID NO: 766 that the amino acids at positions 2 and 3 are amino acids particularly important for the binding to IgE antibodies from allergic patients. The amino acids at positions 1 and 4-15 of SEQ ID NO: 766 had no influence on the binding activity against IgE antibodies from allergic patients when substituted by alanine.

### (17β) Epitope of guanosine nucleotide diphosphate dissociation inhibitor 2 (spot 17α)

SEQ ID NO: 767 was identified as an epitope of guanosine nucleotide diphosphate dissociation inhibitor 2 by the procedure of (B) mentioned above.

Amino acids important for binding to IgE antibodies from allergic patients were identified by the procedure of (C) mentioned above as to SEQ ID NO: 767.

For SEQ ID NO: 767, all the amino acids were amino acids particularly important for the binding to IgE antibodies from allergic patients.

### (18β) Epitope of hypothetical protein GLYMA 10G028300 (spot 18α)

SEQ ID NOs: 750, 768, 771, 772, and 775 were identified as epitopes of hypothetical protein GLYMA_10G028300 by the procedure of (B) mentioned above.

Amino acids important for binding to IgE antibodies from allergic patients were identified by the procedure of (C) mentioned above as to each of SEQ ID NOs: 750, 768, 771, 772 and 775.

It was revealed for SEQ ID NO: 771 that the amino acids at positions 8 and 11-15 are amino acids particularly important for the binding to IgE antibodies from allergic patients, and the amino acid at position 10 is an amino acid that influences the binding to IgE antibodies from allergic patients. The amino acids at positions 1-7 and 9 of SEQ ID NO: 771 had no influence on the binding activity against IgE antibodies from allergic patients when substituted by alanine.

It was revealed for SEQ ID NO: 775 that the amino acids at positions 2-6 and 8 are amino acids particularly important for the binding to IgE antibodies from allergic patients, and the amino acid at position 7 is an amino acid that influences the binding to IgE antibodies from allergic patients. The amino acids at positions 1, 9 and 10 of SEQ ID NO: 775 had no influence on the binding activity against IgE antibodies from allergic patients when substituted by alanine.

For SEQ ID NOs: 750, 768 and 772, all the amino acids were amino acids particularly important for the binding to IgE antibodies from allergic patients.

### (19β) Epitope of sucrose binding protein homolog S-64 (spot 19α)

SEQ ID NOs: 778, 781, 784, 786, and 787 were identified as epitopes of sucrose binding protein homolog S-64 by the procedure of (B) mentioned above.

Amino acids important for binding to IgE antibodies from allergic patients were identified by the procedure of (C) mentioned above as to each of SEQ ID NOs: 778, 781, 784, 786 and 787.

It was revealed for SEQ ID NO: 778 that the amino acids at positions 6-8, 13 and 14 are amino acids particularly important for the binding to IgE antibodies from allergic patients, and the amino acid at position 9 is an amino acid that influences the binding to IgE antibodies from allergic patients. The amino acids at positions 1-5, 10-12 and 15 of SEQ ID NO: 778 had no influence on the binding activity against IgE antibodies from allergic patients when substituted by alanine.

It was revealed for SEQ ID NO: 781 that the amino acids at positions 9, 11 and 12 are amino acids particularly important for the binding to IgE antibodies from allergic patients, and the amino acid at position 6 is an amino acid that influences the binding to IgE antibodies from allergic patients. The amino acids at positions 1-5, 7, 8, 10 and 13-15 of SEQ ID NO: 781 had no influence on the binding activity against IgE antibodies from allergic patients when substituted by alanine.

It was revealed for SEQ ID NO: 784 that the amino acids at positions 11-13 are amino acids particularly important for the binding to IgE antibodies from allergic patients, and the amino acids at positions 2-8 and 14 are amino acids that influence the binding to IgE antibodies from allergic patients. The amino acids at positions 1, 9, 10 and 15 of SEQ ID NO: 784 had no influence on the binding activity against IgE antibodies from allergic patients when substituted by alanine.

It was revealed for SEQ ID NO: 786 that the amino acids at positions 2 and 3 are amino acids particularly important for the binding to IgE antibodies from allergic patients. The amino acids at positions 1-2 and 5-15 of SEQ ID NO: 786 had no influence on the binding activity against IgE antibodies from allergic patients when substituted by alanine.

For SEQ ID NO: 787, all the amino acids were amino acids particularly important for the binding to IgE antibodies from allergic patients.

### (20β) Epitope of Seed biotin-containing protein SBP65 (spot 20α)

SEQ ID NOs: 788-792 were identified as epitopes of Seed biotin-containing protein SBP65 by the procedure of (B) mentioned above.

Amino acids important for binding to IgE antibodies from allergic patients were identified by the procedure of (C) mentioned above as to each of SEQ ID NOs: 788-792.

For SEQ ID NOs: 788-792, all the amino acids were amino acids particularly important for the binding to IgE antibodies from allergic patients.

### (21β) Epitope of Ribulose bisphosphate carboxylase large chain (spot 21α)

SEQ ID NOs: 793-802 were identified as epitopes of Ribulose bisphosphate carboxylase large chain by the procedure of (B) mentioned above.

Amino acids important for binding to IgE antibodies from allergic patients were identified by the procedure of (C) mentioned above as to each of SEQ ID NOs: 793-802.

For SEQ ID NOs: 793-802, all the amino acids were amino acids particularly important for the binding to IgE antibodies from allergic patients.

### (22β) Epitope of Gamma-glutamyl hydrolase (spot 22α)

SEQ ID NOs: 803-808 were identified as epitopes of Gamma-glutamyl hydrolase by the procedure of (B) mentioned above.

Amino acids important for binding to IgE antibodies from allergic patients were identified by the procedure of (C) mentioned above as to each of SEQ ID NOs: 803-808.

For SEQ ID NOs: 803-808, all the amino acids were amino acids particularly important for the binding to IgE antibodies from allergic patients.

### (23β) Epitope of Guanine nucleotide-binding protein subunit beta-like protein (spot 23α)

SEQ ID NOs: 809-812 were identified as epitopes of Guanine nucleotide-binding protein subunit beta-like protein by the procedure of (B) mentioned above.

Amino acids important for binding to IgE antibodies from allergic patients were identified by the procedure of (C) mentioned above as to each of SEQ ID NOs: 809-812.

For SEQ ID NOs: 809-812, all the amino acids were amino acids particularly important for the binding to IgE antibodies from allergic patients.

### (24β) Epitope of Protein SLE3 (spot 24α)

SEQ ID NO: 813 was identified as an epitope of Protein SLE3 by the procedure of (B) mentioned above.

Amino acids important for binding to IgE antibodies from allergic patients were identified by the procedure of (C) mentioned above as to SEQ ID NO: 813.

For SEQ ID NO: 813, all the amino acids were amino acids particularly important for the binding to IgE antibodies from allergic patients.

### (25β) Epitope of succinyl-CoA ligase [ADP-forming] subunit beta, mitochondrial (spot 25α)

SEQ ID NOs: 814 and 815 were identified as epitopes of succinyl-CoA ligase [ADP-forming] subunit beta, mitochondrial by the procedure of (B) mentioned above.

Amino acids important for binding to IgE antibodies from allergic patients were identified by the procedure of (C) mentioned above as to each of SEQ ID NOs: 814 and 815.

For SEQ ID NOs: 814 and 815, all the amino acids were amino acids particularly important for the binding to IgE antibodies from allergic patients.

### (26β) Epitope of phosphoglycerate kinase, cytosolic (spot 26α)

SEQ ID NOs: 816 and 819 were identified as epitopes of phosphoglycerate kinase, cytosolic by the procedure of (B) mentioned above.

Amino acids important for binding to IgE antibodies from allergic patients were identified by the procedure of (C) mentioned above as to each of SEQ ID NOs: 816 and 819.

It was revealed for SEQ ID NO: 819 that the amino acids at positions 1-3 are amino acids particularly important for the binding to IgE antibodies from allergic patients, and the amino acid at position 4 is an amino acid that influences the binding to IgE antibodies from allergic patients. The amino acids at positions 5-7 of SEQ ID NO: 819 had no influence on the binding activity against IgE antibodies from allergic patients when substituted by alanine.

For SEQ ID NO: 816, all the amino acids were amino acids particularly important for the binding to IgE antibodies from allergic patients.

### (27β) Epitope of alcohol dehydrogenase 1 (spot 27α)

SEQ ID NO: 820 was identified as an epitope of alcohol dehydrogenase 1 by the procedure of (B) mentioned above.

Amino acids important for binding to IgE antibodies from allergic patients were identified by the procedure of (C) mentioned above as to SEQ ID NO: 820.

For SEQ ID NO: 820, all the amino acids were amino acids particularly important for the binding to IgE antibodies from allergic patients.

### (28β) Epitope of 35 kDa seed maturation protein isoform X1 (spot 28α)

SEQ ID NOs: 823, 826, 829, and 830-835 were identified as epitopes of 35 kDa seed maturation protein isoform X1 by the procedure of (B) mentioned above.

Amino acids important for binding to IgE antibodies from allergic patients were identified by the procedure of (C) mentioned above as to each of SEQ ID NOs: 823, 826, 829 and 830-835.

It was revealed for SEQ ID NO: 823 that the amino acids at positions 2 and 7-10 are amino acids particularly important for the binding to IgE antibodies from allergic patients, and the amino acids at positions 1, 4 are amino acids that influence the binding to IgE antibodies from allergic patients. The amino acids at positions 3, 5 and 6 of SEQ ID NO: 823 had no influence on the binding activity against IgE antibodies from allergic patients when substituted by alanine.

It was revealed for SEQ ID NO: 826 that the amino acids at positions 2, 7 and 8 are amino acids particularly important for the binding to IgE antibodies from allergic patients, and the amino acids at positions 3 and 14 are amino acids that influence the binding to IgE antibodies from allergic patients. The amino acids at positions 1, 4-6, 9-13 and 15 of SEQ ID NO: 826 had no influence on the binding activity against IgE antibodies from allergic patients when substituted by alanine.

It was revealed for SEQ ID NO: 829 that the amino acids at positions 4, 7-9 and 14 are amino acids particularly important for the binding to IgE antibodies from allergic patients, and the amino acids at positions 10 and 11 are amino acids that influence the binding to IgE antibodies from allergic patients. The amino acids at positions 1-3, 5, 6, 12, 13 and 15 of SEQ ID NO: 829 had no influence on the binding activity against IgE antibodies from allergic patients when substituted by alanine.

For SEQ ID NOs: 830-835, all the amino acids were amino acids particularly important for the binding to IgE antibodies from allergic patients.

### (29β) Epitope of glyceraldehyde-3-phosphate dehydrogenase isoform X1 (spots 29α, 30α, 31α, 32α, and 33α)

SEQ ID NOs: 838 and 839 were identified as epitopes of glyceraldehyde-3-phosphate dehydrogenase isoform X1 by the procedure of (B) mentioned above.

Amino acids important for binding to IgE antibodies from allergic patients were identified by the procedure of (C) mentioned above as to each of SEQ ID NOs: 838 and 839.

It was revealed for SEQ ID NO: 838 that the amino acids at positions 8-10 are amino acids particularly important for the binding to IgE antibodies from allergic patients, and the amino acid at position 6 is an amino acid that influences the binding to IgE antibodies from allergic patients. The amino acids at positions 1-5 and 7 of SEQ ID NO: 838 had no influence on the binding activity against IgE antibodies from allergic patients when substituted by alanine.

For SEQ ID NO: 839, all the amino acids were amino acids particularly important for the binding to IgE antibodies from allergic patients.

### (34β) Epitope of bifunctional UDP-glucose 4-epimerase and UDP-xylose 4-epimerase 1-like (spot 34α)

SEQ ID NOs: 842 and 843-846 were identified as epitopes of bifunctional UDP-glucose 4-epimerase and UDP-xylose 4-epimerase 1-like by the procedure of (B) mentioned above.

Amino acids important for binding to IgE antibodies from allergic patients were identified by the procedure of (C) mentioned above as to each of SEQ ID NOs: 842 and 843-846.

It was revealed for SEQ ID NO: 842 that the amino acids at positions 12, 13 and 15 are amino acids particularly important for the binding to IgE antibodies from allergic patients, and the amino acid at position 1 is an amino acid that influences the binding to IgE antibodies from allergic patients. The amino acids at positions 2-11 and 14 of SEQ ID NO: 842 had no influence on the binding activity against IgE antibodies from allergic patients when substituted by alanine.

For SEQ ID NOs: 843-846, all the amino acids were amino acids particularly important for the binding to IgE antibodies from allergic patients.

### (35β) Epitope of elongation factor 1-alpha (spot 35α)

SEQ ID NOs: 849, 850, 853, and 856 were identified as epitopes of elongation factor 1-alpha by the procedure of (B) mentioned above.

Amino acids important for binding to IgE antibodies from allergic patients were identified by the procedure of (C) mentioned above as to each of SEQ ID NOs: 849, 850, 853 and 856.

It was revealed for SEQ ID NO: 849 that the amino acids at positions 5, 6, 10, 11 and 15 are amino acids particularly important for the binding to IgE antibodies from allergic patients, and the amino acids at positions 7 and 8 are amino acids that influence the binding to IgE antibodies from allergic patients. The amino acids at positions 1-4, 9 and 12-14 of SEQ ID NO: 849 had no influence on the binding activity against IgE antibodies from allergic patients when substituted by alanine.

It was revealed for SEQ ID NO: 853 that the amino acids at positions 1-3, 5 and 6 are amino acids particularly important for the binding to IgE antibodies from allergic patients, and the amino acid at position 4 is an amino acid that influences the binding to IgE antibodies from allergic patients. The amino acids at positions 7-9 of SEQ ID NO: 853 had no influence on the binding activity against IgE antibodies from allergic patients when substituted by alanine.

It was revealed for SEQ ID NO: 856 that the amino acids at positions 3, 5 and 6 are amino acids particularly important for the binding to IgE antibodies from allergic patients, and the amino acids at positions 1 and 2 are amino acids that influence the binding to IgE antibodies from allergic patients. The amino acids at positions 4 and 7-10 of SEQ ID NO: 856 had no influence on the binding activity against IgE antibodies from allergic patients when substituted by alanine.

For SEQ ID NO: 850, all the amino acids were amino acids particularly important for the binding to IgE antibodies from allergic patients.

### (36β) Epitope of seed maturation protein PM34 (spots 36α and 37α)

SEQ ID NOs: 857, 858, and 861 were identified as epitopes of seed maturation protein PM34 by the procedure of (B) mentioned above.

Amino acids important for binding to IgE antibodies from allergic patients were identified by the procedure of (C) mentioned above as to each of SEQ ID NOs: 857, 858, and 861.

It was revealed for SEQ ID NO: 861 that the amino acids at positions 1 and 6-8 are amino acids particularly important for the binding to IgE antibodies from allergic patients, and the amino acid at position 5 is an amino acid that influences the binding to IgE antibodies from allergic patients. The amino acids at positions 2-4 of SEQ ID NO: 861 had no influence on the binding activity against IgE antibodies from allergic patients when substituted by alanine.

For SEQ ID NOs: 857 and 858, all the amino acids were amino acids particularly important for the binding to IgE antibodies from allergic patients.

### (38β) Epitope of seed biotin-containing protein SBP65-like isoform X1 (spot 38α)

SEQ ID NOs: 864, 867, 869, 871, 874, 877, 880, 882, 884, 887, 889, and 892 were identified as epitopes of seed biotin-containing protein SBP65-like isoform X1 by the procedure of (B) mentioned above.

Amino acids important for binding to IgE antibodies from allergic patients were identified by the procedure of (C) mentioned above as to each of SEQ ID NOs: 864, 867, 869, 871, 874, 877, 880, 882, 884, 887, 889, and 892.

It was revealed for SEQ ID NO: 864 that the amino acids at positions 1-3 are amino acids particularly important for the binding to IgE antibodies from allergic patients. The amino acids at positions 4-7 of SEQ ID NO: 864 had no influence on the binding activity against IgE antibodies from allergic patients when substituted by alanine.

It was revealed for SEQ ID NO: 867 that the amino acids at positions 2-4 are amino acids particularly important for the binding to IgE antibodies from allergic patients, and the amino acid at position 1 is an amino acid that influences the binding to IgE antibodies from allergic patients. The amino acids at positions 5-10 of SEQ ID NO: 867 had no influence on the binding activity against IgE antibodies from allergic patients when substituted by alanine.

It was revealed for SEQ ID NO: 869 that the amino acids at positions 1, 3 and 15 are amino acids particularly important for the binding to IgE antibodies from allergic patients.

The amino acids at positions 2 and 4-14 of SEQ ID NO: 869 had no influence on the binding activity against IgE antibodies from allergic patients when substituted by alanine.

It was revealed for SEQ ID NO: 871 that the amino acids at positions 8, 9 and 15 are amino acids particularly important for the binding to IgE antibodies from allergic patients. The amino acids at positions 1-7 and 10-14 of SEQ ID NO: 871 had no influence on the binding activity against IgE antibodies from allergic patients when substituted by alanine.

It was revealed for SEQ ID NO: 874 that the amino acids at positions 4-8 and 10 are amino acids particularly important for the binding to IgE antibodies from allergic patients, and the amino acids at positions 1-3 are amino acids that influence the binding to IgE antibodies from allergic patients. The amino acid at position 9 of SEQ ID NO: 874 had no influence on the binding activity against IgE antibodies from allergic patients when substituted by alanine.

It was revealed for SEQ ID NO: 877 that the amino acids at positions 1-6, 9 and 10 are amino acids particularly important for the binding to IgE antibodies from allergic patients, and the amino acids at positions 7 and 8 are amino acids that influence the binding to IgE antibodies from allergic patients. The amino acids at positions 11-15 of SEQ ID NO: 877 had no influence on the binding activity against IgE antibodies from allergic patients when substituted by alanine.

It was revealed for SEQ ID NO: 880 that the amino acids at positions 2, 4-6, 8 and 9 are amino acids particularly important for the binding to IgE antibodies from allergic patients, and the amino acids at positions 7 and 12-15 are amino acids that influence the binding to IgE antibodies from allergic patients. The amino acids at positions 1, 3, 10 and 11 of SEQ ID NO: 880 had no influence on the binding activity against IgE antibodies from allergic patients when substituted by alanine.

It was revealed for SEQ ID NO: 882 that the amino acids at positions 1 and 5-9 are amino acids particularly important for the binding to IgE antibodies from allergic patients. The amino acids at positions 2-4 and 10 of SEQ ID NO: 882 had no influence on the binding activity against IgE antibodies from allergic patients when substituted by alanine.

It was revealed for SEQ ID NO: 884 that the amino acids at positions 5-10 are amino acids particularly important for the binding to IgE antibodies from allergic patients. The amino acids at positions 1-4 of SEQ ID NO: 884 had no influence on the binding activity against IgE antibodies from allergic patients when substituted by alanine.

It was revealed for SEQ ID NO: 887 that the amino acids at positions 2, 4, 5 and 14 are amino acids particularly important for the binding to IgE antibodies from allergic patients, and the amino acids at positions 6, 10, 12, 13 and 15 are amino acids that influence the binding to IgE antibodies from allergic patients. The amino acids at positions 1, 3, 7-9 and 11 of SEQ ID NO: 887 had no influence on the binding activity against IgE antibodies from allergic patients when substituted by alanine.

It was revealed for SEQ ID NO: 889 that the amino acids at positions 3-7 are amino acids particularly important for the binding to IgE antibodies from allergic patients. The amino acids at positions 1, 2 and 8-10 of SEQ ID NO: 889 had no influence on the binding activity against IgE antibodies from allergic patients when substituted by alanine.

It was revealed for SEQ ID NO: 892 that the amino acids at positions 5-10 are amino acids particularly important for the binding to IgE antibodies from allergic patients, and the amino acids at positions 3 and 4 are amino acids that influence the binding to IgE antibodies from allergic patients. The amino acids at positions 1 and 2 of SEQ ID NO: 892 had no influence on the binding activity against IgE antibodies from allergic patients when substituted by alanine.

### (39β) Epitope of ATP synthase subunit O, mitochondrial-like (spot 39α)

SEQ ID NO: 895 was identified as an epitope of ATP synthase subunit O, mitochondrial-like by the procedure of (B) mentioned above.

Amino acids important for binding to IgE antibodies from allergic patients were identified by the procedure of (C) mentioned above as to SEQ ID NO: 895.

It was revealed for SEQ ID NO: 895 that the amino acids at positions 2, 5, 7, and 12-14 are amino acids particularly important for the binding to IgE antibodies from allergic patients, and the amino acid at position 3 is an amino acid that influences the binding to IgE antibodies from allergic patients. The amino acids at positions 1, 4, 6, 8-11, and 15 of SEQ ID NO: 895 had no influence on the binding activity against IgE antibodies from allergic patients when substituted by alanine.

### (40β) Epitope of P24 oleosin isoform A (spots 40α and 56α)

SEQ ID NOs: 898, 899, and 902 were identified as epitopes of P24 oleosin isoform A by the procedure of (B) mentioned above.

Amino acids important for binding to IgE antibodies from allergic patients were identified by the procedure of (C) mentioned above as to each of SEQ ID NOs: 898, 899 and 902.

It was revealed for SEQ ID NO: 898 that the amino acids at positions 1, 2, 4 and 5 are amino acids particularly important for the binding to IgE antibodies from allergic patients, and the amino acid at position 6 is an amino acid that influences the binding to IgE antibodies from allergic patients. The amino acid at position 3 of SEQ ID NO: 898 had no influence on the binding activity against IgE antibodies from allergic patients when substituted by alanine.

It was revealed for SEQ ID NO: 902 that the amino acids at positions 6-8 are amino acids particularly important for the binding to IgE antibodies from allergic patients, and the amino acid at position 1 is an amino acid that influences the binding to IgE antibodies from allergic patients. The amino acids at positions 2-5 and 9 of SEQ ID NO: 902 had no influence on the binding activity against IgE antibodies from allergic patients when substituted by alanine.

For SEQ ID NO: 899, all the amino acids were amino acids particularly important for the binding to IgE antibodies from allergic patients.

### (41β) Epitope of uncharacterized protein At3g49720-like isoform X2 (spot 41α)

SEQ ID NO: 903 was identified as an epitope of uncharacterized protein At3g49720-like isoform X2 by the procedure of (B) mentioned above.

Amino acids important for binding to IgE antibodies from allergic patients were identified by the procedure of (C) mentioned above as to SEQ ID NO: 903.

For SEQ ID NO: 903, all the amino acids were amino acids particularly important for the binding to IgE antibodies from allergic patients.

### (42β) Epitope of superoxide dismutase [Mn], mitochondrial (spots 42α and 43α)

SEQ ID NO: 906 was identified as an epitope of superoxide dismutase [Mn], mitochondrial by the procedure of (B) mentioned above.

Amino acids important for binding to IgE antibodies from allergic patients were identified by the procedure of (C) mentioned above as to SEQ ID NO: 906.

It was revealed for SEQ ID NO: 906 that the amino acids at positions 3 and 11-13 are amino acids particularly important for the binding to IgE antibodies from allergic patients, and the amino acids at positions 8 and 14 are amino acids that influence the binding to IgE antibodies from allergic patients. The amino acids at positions 1, 2, 4-7, 9, 10, and 15 of SEQ ID NO: 906 had no influence on the binding activity against IgE antibodies from allergic patients when substituted by alanine.

### (44β) Epitope of Ferritin-2 (spot 44α)

SEQ ID NO: 909 was identified as an epitope of Ferritin-2 by the procedure of (B) mentioned above.

Amino acids important for binding to IgE antibodies from allergic patients were identified by the procedure of (C) mentioned above as to SEQ ID NO: 909.

It was revealed for SEQ ID NO: 909 that the amino acids at positions 5-11 and 13-15 are amino acids particularly important for the binding to IgE antibodies from allergic patients. The amino acids at positions 1-4 and 12 of SEQ ID NO: 909 had no influence on the binding activity against IgE antibodies from allergic patients when substituted by alanine.

### (45β) Epitope of uncharacterized protein LOC100499771 (spot 45α)

SEQ ID NOs: 910-912 were identified as epitopes of uncharacterized protein LOC100499771 by the procedure of (B) mentioned above.

Amino acids important for binding to IgE antibodies from allergic patients were identified by the procedure of (C) mentioned above as to each of SEQ ID NOs: 910-912.

For SEQ ID NOs: 910-912, all the amino acids were amino acids particularly important for the binding to IgE antibodies from allergic patients.

### (46β) Epitope of seed maturation protein PM22 (spot 46α)

SEQ ID NO: 915 was identified as an epitope of seed maturation protein PM22 by the procedure of (B) mentioned above.

Amino acids important for binding to IgE antibodies from allergic patients were identified by the procedure of (C) mentioned above as to SEQ ID NO: 915.

It was revealed for SEQ ID NO: 915 that the amino acids at positions 2, 4, and 8 are amino acids particularly important for the binding to IgE antibodies from allergic patients, and the amino acid at position 3 is an amino acid that influences the binding to IgE antibodies from allergic patients. The amino acids at positions 1, 5-7, and 9 of SEQ ID NO: 915 had no influence on the binding activity against IgE antibodies from allergic patients when substituted by alanine.

### (47β) Epitope of eukaryotic translation initiation factor 5A-2 (spot 47α)

SEQ ID NOs: 916-918 were identified as epitopes of eukaryotic translation initiation factor 5A-2 by the procedure of (B) mentioned above.

Amino acids important for binding to IgE antibodies from allergic patients were identified by the procedure of (C) mentioned above as to each of SEQ ID NOs: 916-918.

For SEQ ID NOs: 916-918, all the amino acids were amino acids particularly important for the binding to IgE antibodies from allergic patients.

### (48β) Epitope of uncharacterized protein LOC100306570 (spot 48α)

SEQ ID NOs: 921 and 922 were identified as epitopes of uncharacterized protein LOC100306570 by the procedure of (B) mentioned above.

Amino acids important for binding to IgE antibodies from allergic patients were identified by the procedure of (C) mentioned above as to each of SEQ ID NOs: 921 and 922.

It was revealed for SEQ ID NO: 921 that the amino acids at positions 7-9 are amino acids particularly important for the binding to IgE antibodies from allergic patients, and the amino acid at position 5 is an amino acid that influences the binding to IgE antibodies from allergic patients. The amino acids at positions 1-4 and 6 of SEQ ID NO: 921 had no influence on the binding activity against IgE antibodies from allergic patients when substituted by alanine.

For SEQ ID NO: 922, all the amino acids were amino acids particularly important for the binding to IgE antibodies from allergic patients.

### (49β) Epitope of Bowman-Birk type proteinase inhibitor D-II (spot 49α)

SEQ ID NO: 923 was identified as an epitope of Bowman-Birk type proteinase inhibitor D-II by the procedure of (B) mentioned above.

Amino acids important for binding to IgE antibodies from allergic patients were identified by the procedure of (C) mentioned above as to SEQ ID NO: 923.

For SEQ ID NO: 923, all the amino acids were amino acids particularly important for the binding to IgE antibodies from allergic patients.

### (50β) Epitope of uncharacterized protein LQC100813859 (spot 50α)

SEQ ID NOs: 924 and 927 were identified as epitopes of uncharacterized protein LOC100813859 by the procedure of (B) mentioned above.

Amino acids important for binding to IgE antibodies from allergic patients were identified by the procedure of (C) mentioned above as to each of SEQ ID NOs: 924 and 927.

It was revealed for SEQ ID NO: 927 that the amino acids at positions 5 and 6 are amino acids particularly important for the binding to IgE antibodies from allergic patients, and the amino acid at position 3 is an amino acid that influences the binding to IgE antibodies from allergic patients. The amino acids at positions 1, 2, 4, and 7-9 of SEQ ID NO: 927 had no influence on the binding activity against IgE antibodies from allergic patients when substituted by alanine.

For SEQ ID NO: 924, all the amino acids were amino acids particularly important for the binding to IgE antibodies from allergic patients.

### (51β) Epitope of 14-3-3-like protein (spot 51α)

SEQ ID NOs: 928-930 were identified as epitopes of 14-3-3-like protein by the procedure of (B) mentioned above.

Amino acids important for binding to IgE antibodies from allergic patients were identified by the procedure of (C) mentioned above as to each of SEQ ID NOs: 928-930.

For SEQ ID NOs: 928-930, all the amino acids were amino acids particularly important for the binding to IgE antibodies from allergic patients.

### (52β) Epitope of probable fructokinase-4 (spot 52α)

SEQ ID NOs: 933 and 934-938 were identified as epitopes of probable fructokinase-4 by the procedure of (B) mentioned above.

Amino acids important for binding to IgE antibodies from allergic patients were identified by the procedure of (C) mentioned above as to each of SEQ ID NOs: 933 and 934-938.

It was revealed for SEQ ID NO: 933 that the amino acids at positions 7, 10, and 12 are amino acids particularly important for the binding to IgE antibodies from allergic patients, and the amino acid at position 3 is an amino acid that influences the binding to IgE antibodies from allergic patients. The amino acids at positions 1, 2, 4-6, 8, 9, 11, and 13-15 of SEQ ID NO: 933 had no influence on the binding activity against IgE antibodies from allergic patients when substituted by alanine.

For SEQ ID NOs: 934-938, all the amino acids were amino acids particularly important for the binding to IgE antibodies from allergic patients.

### (53β) Epitope of triosephosphate isomerase isoform X1 (spot 53α)

SEQ ID NO: 939 was identified as an epitope of triosephosphate isomerase isoform X1 by the procedure of (B) mentioned above.

Amino acids important for binding to IgE antibodies from allergic patients were identified by the procedure of (C) mentioned above as to SEQ ID NO: 939.

For SEQ ID NO: 939, all the amino acids were amino acids particularly important for the binding to IgE antibodies from allergic patients.

### (54β) Epitope of superoxide dismutase [Fe], chloroplastic isoform X1 (spot 54α)

SEQ ID NOs: 940 and 943 were identified as epitopes of superoxide dismutase [Fe], chloroplastic isoform X1 by the procedure of (B) mentioned above.

Amino acids important for binding to IgE antibodies from allergic patients were identified by the procedure of (C) mentioned above as to each of SEQ ID NOs: 940 and 943.

It was revealed for SEQ ID NO: 943 that the amino acids at positions 1 and 6 are amino acids particularly important for the binding to IgE antibodies from allergic patients, and the amino acid at position 7 is an amino acid that influences the binding to IgE antibodies from allergic patients. The amino acids at positions 2-5 of SEQ ID NO: 943 had no influence on the binding activity against IgE antibodies from allergic patients when substituted by alanine.

For SEQ ID NO: 940, all the amino acids were amino acids particularly important for the binding to IgE antibodies from allergic patients.

### (55β) Epitope of 51 kDa seed maturation protein precursor (spot 55α)

SEQ ID NOs: 946, 949, and 952 were identified as epitopes of 51 kDa seed maturation protein precursor by the procedure of (B) mentioned above.

Amino acids important for binding to IgE antibodies from allergic patients were identified by the procedure of (C) mentioned above as to each of SEQ ID NOs: 946, 949, and 952.

It was revealed for SEQ ID NO: 946 that the amino acids at positions 1, 2, 6, and 7 are amino acids particularly important for the binding to IgE antibodies from allergic patients, and the amino acid at position 3 is an amino acid that influences the binding to IgE antibodies from allergic patients. The amino acids at positions 4, 5, 8, and 9 of SEQ ID NO: 946 had no influence on the binding activity against IgE antibodies from allergic patients when substituted by alanine.

It was revealed for SEQ ID NO: 949 that the amino acids at positions 1-3, 7, and 8 are amino acids particularly important for the binding to IgE antibodies from allergic patients, and the amino acid at position 4 is an amino acid that influences the binding to IgE antibodies from allergic patients. The amino acids at positions 5 and 6 of SEQ ID NO: 949 had no influence on the binding activity against IgE antibodies from allergic patients when substituted by alanine.

It was revealed for SEQ ID NO: 952 that the amino acids at positions 1, 2, and 9 are amino acids particularly important for the binding to IgE antibodies from allergic patients, and the amino acid at position 8 is an amino acid that influences the binding to IgE antibodies from allergic patients. The amino acids at positions 3-7 of SEQ ID NO: 952 had no influence on the binding activity against IgE antibodies from allergic patients when substituted by alanine.

### Example 5: Identification of epitopes (2)

Identification of epitopes was carried out by the same procedure as in Example 4 as to each of the following antigen proteins.

### (57β) Epitope of Gly m 4 (UniProt accession number: P26987.1)

SEQ ID NO: 955 was identified as an epitope of Gly m 4 by the procedure of (B) of Example 4.

Amino acids important for binding to IgE antibodies from allergic patients were identified by the procedure of (C) of Example 4 as to SEQ ID NO: 955.

It was revealed for SEQ ID NO: 955 that the amino acids at positions 8, 10, 12, 14, and 15 are amino acids particularly important for the binding to IgE antibodies from allergic patients, and the amino acids at positions 7, 9, 11, and 13 are amino acids that influence the binding to IgE antibodies from allergic patients. The amino acids at positions 1-6 of SEQ ID NO: 955 had no influence on the binding activity against IgE antibodies from allergic patients when substituted by alanine.

### (58β) Epitopes of Gly m 5α (NCBI accession number: NP 001236856.1) and Gly m 5α' (NCBI accession number: NP_001237316.1)

SEQ ID NOs: 956-960 were identified as epitopes of Gly m 5α or Gly m 5α' by the procedure of (B) of Example 4.

Amino acids important for binding to IgE antibodies from allergic patients were identified by the procedure of (C) of Example 4 as to each of SEQ ID NOs: 956-960.

For SEQ ID NOs: 956-960, all the amino acids were amino acids particularly important for the binding to IgE antibodies from allergic patients.

### (59β) Epitopes of Gly m 6.01 (UniProt accession number: CAA33215.1) and Gly m 6.02 (UniProt accession number: BAA00154.1)

SEQ ID NOs: 961 and 962 were identified as epitopes of Gly m 6.01 or Gly m 6.02 by the procedure of (B) of Example 4.

Amino acids important for binding to IgE antibodies from allergic patients were identified by the procedure of (C) of Example 4 as to each of SEQ ID NOs: 961 and 962.

For SEQ ID NOs: 961 and 962, all the amino acids were amino acids particularly important for the binding to IgE antibodies from allergic patients.

### (60β) Epitope of Gly m 8 (UniProt accession number: AAB71140.1)

SEQ ID NO: 963 was identified as an epitope of Gly m 8 by the procedure of (B) of Example 4.

Amino acids important for binding to IgE antibodies from allergic patients were identified by the procedure of (C) of Example 4 as to SEQ ID NO: 963.

For SEQ ID NO: 963, all the amino acids were amino acids particularly important for the binding to IgE antibodies from allergic patients.

### Example 6: Study on cross-reactivity

Amino acids important for binding activity against IgE antibodies from allergic patients were identified as to SEQ ID NO: 871, one of the polypeptides in spot 38β, to obtain SEQ ID NO: 870 (hereinafter also referred to as a key sequence). A protein having this key sequence was compared against NCBI protein data and analyzed by PHI-BLAST. As a result, peach-derived kinesin-like protein KIN-7C comprising SEQ ID NO: 984 was identified as an amino acid sequence corresponding to the key sequence, and apple-derived uncharacterized protein LOC103433727 isoform X2 comprising SEQ ID NO: 985 was identified as an amino acid sequence corresponding to the key sequence.

The binding activity of peptides comprising the amino acid sequences of SEQ ID NOs: 871, 984 and 985 against an IgE antibody from one patient having allergies to a soybean, a peach and an apple was confirmed by ELISA. The peptides were synthesized such that the peptides were N-terminally biotinylated by the Fmoc method.

To be specific, ELISA was carried out according to the following procedure.
(1) The concentrations of the biotinylated peptides were adjusted to 1 µg/mL with PBST.
(2) Each peptide solution was added at 40 µL to each well of a 384-well plate coated with streptavidin, and shaken at room temperature for one hour. After collection of the solution, the wells were washed with PBS five times.
(3) A 1:5 dilution of Blocking one (diluted with MQ) was added at 40 µL thereto and shaken at room temperature for 15 minutes. After removal of the solution, the wells were washed with PBS five times.
(4) A diluted serum solution (1:10, diluting solution: Blocking one produced by Nacalai Tesque, Inc.) was added at 40 µL thereto and shaken at room temperature for one hour. After removal of the solution, the wells were washed with PBS five times.
(5) A diluted secondary antibody solution (1:5000, diluting solution: Blocking one produced by Nacalai Tesque, Inc.) was added at 40 µL thereto and shaken at room temperature for one hour. After removal of the solution, the wells were washed with PBS five times.
(6) 1-Step Ultra TMB-ELISA (Thermo Fisher Scientific) was added at 40 µL thereto and shaken at room temperature for 15 minutes.
(7) 2 M H₂SO₄ was added at 40 µL thereto. Absorbance at 450 nm was measured.

The results are shown in Fig. 6. As shown in Fig. 6, the peptides comprising the amino acid sequences of SEQ ID NOs: 871, 984 and 985 exhibited binding activity against the IgE antibody from the patient having allergies to a soybean, a peach and an apple. Also, these peptides bound more strongly to the IgE antibody from the allergic patient than to an IgE antibody from a healthy subject who was not an allergic patient. This indicates that this key sequence can be used for detecting the cross-reactivity of an allergen.

This result also indicates that the polypeptides (1β) to (60β) of the present invention can also be used for the detection of the cross-reactivity of an allergen.

## Claims

1. A polypeptide specifically binding to an IgE antibody from an allergic patient, the polypeptide being any one of the following:
(1β) a polypeptide comprising at least one amino acid sequence selected from the group consisting of SEQ ID NOs: 650-660;
(2β) a polypeptide comprising at least one amino acid sequence selected from the group consisting of SEQ ID NOs: 661-692;
(4β) a polypeptide comprising at least one amino acid sequence selected from the group consisting of SEQ ID NOs: 717-731;
(5β) a polypeptide comprising at least one amino acid sequence selected from the group consisting of SEQ ID NOs: 732-738;
(6β) a polypeptide comprising at least one amino acid sequence selected from the group consisting of SEQ ID NOs: 739 and 740;
(7β) a polypeptide comprising at least one amino acid sequence selected from the group consisting of SEQ ID NOs: 741-744;
(9β) a polypeptide comprising at least one amino acid sequence selected from the group consisting of SEQ ID NOs: 745-749;
(11β) a polypeptide comprising at least one amino acid sequence selected from the group consisting of SEQ ID NOs: 751-754;
(12β) a polypeptide comprising the amino acid sequence of SEQ ID NO: 755;
(13β) a polypeptide comprising at least one amino acid sequence selected from the group consisting of SEQ ID NOs: 756-758;
(14β) a polypeptide comprising at least one amino acid sequence selected from the group consisting of SEQ ID NOs: 759-764;
(15β) a polypeptide comprising at least one amino acid sequence selected from the group consisting of SEQ ID NOs: 765 and 766;
(17β) a polypeptide comprising the amino acid sequence of SEQ ID NO: 767;
(18β) a polypeptide comprising at least one amino acid sequence selected from the group consisting of SEQ ID NOs: 750 and 768-775;
(19β) a polypeptide comprising at least one amino acid sequence selected from the group consisting of SEQ ID NOs: 776-787;
(25β) a polypeptide comprising at least one amino acid sequence selected from the group consisting of SEQ ID NOs: 814 and 815;
(26β) a polypeptide comprising at least one amino acid sequence selected from the group consisting of SEQ ID NOs: 816-819;
(27β) a polypeptide comprising the amino acid sequence of SEQ ID NO: 820;
(28β) a polypeptide comprising at least one amino acid sequence selected from the group consisting of SEQ ID NOs: 821-835;
(29β) a polypeptide comprising at least one amino acid sequence selected from the group consisting of SEQ ID NOs: 836-839;
(34β) a polypeptide comprising at least one amino acid sequence selected from the group consisting of SEQ ID NOs: 840-846;
(35β) a polypeptide comprising at least one amino acid sequence selected from the group consisting of SEQ ID NOs: 847-856;
(36β) a polypeptide comprising at least one amino acid sequence selected from the group consisting of SEQ ID NOs: 857-861;
(38β) a polypeptide comprising at least one amino acid sequence selected from the group consisting of SEQ ID NOs: 862-892;
(39β) a polypeptide comprising at least one amino acid sequence selected from the group consisting of SEQ ID NOs: 893-895;
(40β) a polypeptide comprising at least one amino acid sequence selected from the group consisting of SEQ ID NOs: 896-902;
(41β) a polypeptide comprising the amino acid sequence of SEQ ID NO: 903;
(42β) a polypeptide comprising at least one amino acid sequence selected from the group consisting of SEQ ID NOs: 904-906;
(45β) a polypeptide comprising at least one amino acid sequence selected from the group consisting of SEQ ID NOs: 910-912;
(46β) a polypeptide comprising at least one amino acid sequence selected from the group consisting of SEQ ID NOs: 913-915;
(47β) a polypeptide comprising at least one amino acid sequence selected from the group consisting of SEQ ID NOs: 916-918;
(48β) a polypeptide comprising at least one amino acid sequence selected from the group consisting of SEQ ID NOs: 919-922;
(50β) a polypeptide comprising at least one amino acid sequence selected from the group consisting of SEQ ID NOs: 924-927;
(51β) a polypeptide comprising at least one amino acid sequence selected from the group consisting of SEQ ID NOs: 928-930;
(52β) a polypeptide comprising at least one amino acid sequence selected from the group consisting of SEQ ID NOs: 931-938;
(53β) a polypeptide comprising the amino acid sequence of SEQ ID NO: 939;
(54β) a polypeptide comprising at least one amino acid sequence selected from the group consisting of SEQ ID NOs: 940-943; and
(55β) a polypeptide comprising at least one amino acid sequence selected from the group consisting of SEQ ID NOs: 944-952.

2. A polypeptide specifically binding to an IgE antibody from an allergic patient, the polypeptide being any one of the following:
(3β) a polypeptide comprising at least one amino acid sequence selected from the group consisting of SEQ ID NOs: 693-716;
(20β) a polypeptide comprising at least one amino acid sequence selected from the group consisting of SEQ ID NOs: 788-792;
(21β) a polypeptide comprising at least one amino acid sequence selected from the group consisting of SEQ ID NOs: 793-802;
(22β) a polypeptide comprising at least one amino acid sequence selected from the group consisting of SEQ ID NOs: 803-808;
(23β) a polypeptide comprising at least one amino acid sequence selected from the group consisting of SEQ ID NOs: 809-812;
(24β) a polypeptide comprising the amino acid sequence of SEQ ID NO: 813;
(44β) a polypeptide comprising at least one amino acid sequence selected from the group consisting of SEQ ID NOs: 907-909;
(49β) a polypeptide comprising the amino acid sequence of SEQ ID NO: 923;
(57β) a polypeptide comprising at least one amino acid sequence selected from the group consisting of SEQ ID NOs: 953-955;
(58β) a polypeptide comprising at least one amino acid sequence selected from the group consisting of SEQ ID NOs: 956-960;
(59β) a polypeptide comprising at least one amino acid sequence selected from the group consisting of SEQ ID NOs: 961 and 962; and
(60β) a polypeptide comprising the amino acid sequence of SEQ ID NO: 963.

3. The polypeptide according to claim 1 or 2, wherein the number of amino acid residues is 500 or less.

4. A kit for diagnosing an allergy, comprising at least one of polypeptides according to any one of claims 1 to 3.

5. A composition for diagnosing an allergy, the composition comprising at least one of polypeptides according to any one of claims 1 to 3 as an antigen.

6. A method for providing an indicator for diagnosing an allergy in a subject, the method comprising the steps of:
(i) contacting a sample obtained from the subject with an antigen, wherein the sample is a solution comprising an Ig antibody;
(ii) detecting binding between the IgE antibody present in the sample obtained from the subject and the antigen; and
(iii) when the binding between the IgE antibody in the subject and the antigen is detected, an indicator of the fact that the subject is allergic is provided;
wherein the antigen is at least one of polypeptides according to any one of claims 1 to 3.

7. A pharmaceutical composition comprising at least one of polypeptides according to any one of claims 1 to 3.

8. The pharmaceutical composition according to claim 7, wherein the pharmaceutical composition is intended for the treatment of an allergy.

9. A tester for determining the presence or absence of an antigen in an object of interest, the tester comprising an antibody that binds to at least one of polypeptides according to any one of claims 1 to 3.

10. A food raw material or an edible processed product in which an antigen is eliminated or reduced, or a polypeptide is cleaved or removed, wherein the antigen is at least one of polypeptides according to any one of claims 1 to 3.

11. A method for producing an edible processed product in which an antigen is eliminated or reduced, or a polypeptide is cleaved or removed, the method comprising the step of confirming that the antigen is eliminated or reduced, or the polypeptide is cleaved or removed, in a production process of the processed product, wherein the antigen is at least one of polypeptides according to any one of claims 1 to 3.


SEQUENCE LISTING

<110>  FUJITA ACADEMY
       HOYU CO., LTD.

<120>  Antigen of Allergy and Epitope thereof

<130>  FA1621-17185

<150>  PCT/JP2017/008593
<151>  2017-03-03

<160>  985

<170>  PatentIn version 3.5

<210>  1
<211>  249
<212>  DNA
<213>  Glycine max

<400>  1
atgtgtattc tgagcttcct caaaagtgat cagtcatcaa gttatgatga tgatgagtat      60

tcaaaaccat gctgtgatct ctgcatgtgc acacgctcaa tgcctcctca atgcagctgt     120

gaagatatta ggctgaattc atgccactca gattgtaaga gctgtatgtg cacacgctca     180

cagccaggac agtgtcgttg tcttgacacc aacgacttct gctacaaacc ttgcaagtcc     240

agagatgac                                                             249


<210>  2
<211>  83
<212>  PRT
<213>  Glycine max

<400>  2

Met Cys Ile Leu Ser Phe Leu Lys Ser Asp Gln Ser Ser Ser Tyr Asp
1               5                   10                  15


Asp Asp Glu Tyr Ser Lys Pro Cys Cys Asp Leu Cys Met Cys Thr Arg
            20                  25                  30


Ser Met Pro Pro Gln Cys Ser Cys Glu Asp Ile Arg Leu Asn Ser Cys
        35                  40                  45


His Ser Asp Cys Lys Ser Cys Met Cys Thr Arg Ser Gln Pro Gly Gln
    50                  55                  60


Cys Arg Cys Leu Asp Thr Asn Asp Phe Cys Tyr Lys Pro Cys Lys Ser
65                  70                  75                  80


Arg Asp Asp

<210>  3
<211>  1425
<212>  DNA
<213>  Glycine max

<400>  3
atgtcaccac aaacagagac taaagcaagt gttgggttca aagctggtgt taaagattat      60

aaattgactt attatactcc tgactatgaa accaaagata ctgatatctt ggcagcattc     120

cgagtaactc ctcaaccagg agttccgcct gaagaagcag gtgccgcggt agccgccgaa     180

tcttctactg gtacatggac aactgtgtgg accgatgggc ttaccagtct tgatcgttac     240

aaagggcgat gctacggcct tgaacctgtt gctggggaag aaaatcaata tattgcttat     300

gtagcttatc ccttagacct ttttgaagaa ggttctgtta ctaacatgtt tacttccatt     360

gtcggtaatg tatttgggtt caaggccctg cgtgctctac gtctggagga tttgcgaatc     420

cctactgctt atattaaaac tttccaaggt ccgcctcatg gcatccaagt tgagagagat     480

aaattgaaca agtatggtcg tcccctatta ggatgtacta ttaaacctaa attggggtta     540

tccgctaaga attatggtag agctgtttat gaatgtcttc gtgggggact tgattttacc     600

aaagatgatg aaaatgtgaa ttcccaacca tttatgcgtt ggagagaccg tttcttattt     660

tgtgccgaag ccatttttaa atcacaggct gaaacaggtg aaatcaaagg gcattacttg     720

aatgcaactg cgggtacatg cgaagaaatg atgaaaagag ctgtatttgc cagagaatta     780

ggcgttccta tcgtaatgca tgattattta acggggggat tcactgcaaa tactagcttg     840

gctcattatt gccgagataa tggtctactt cttcatatac accgtgcaat gcatgcagtt     900

atcgacagac aaaagaatca tggtatgcac tttcgtgtac tagctaaagc attacgtttg     960

tctggtggag atcatgttca cgccggtacc gtagtaggta aacttgaagg ggaaagagaa    1020

atcactttag gttttgttga tttactacgt gatgattttg ttgaaaaaga tcgaagtcgc    1080

ggtatttatt tcactcagga ttgggtttct ctaccaggtg ttttgcctgt agcttcggga    1140

ggtattcacg tttggcatat gcctgctctg accgagatct ttggggatga ttctgtactc    1200

caatttggcg gaggaacttt aggacaccct tggggaaatg caccaggtgc tgtagctaat    1260

cgagtagctc ttgaagcatg tgtgcaggct cgaaatgaag gacgcgatct tgctcgtgaa    1320

ggtaatgaaa ttatccgtga ggctagcaaa tggagtcctg aattagctgc tgcttgtgaa    1380

gtatggaagg agatcaaatt tgaattcgaa gcaatggata ctttg                    1425

<210>  4
<211>  475
<212>  PRT
<213>  Glycine max

<400>  4

Met Ser Pro Gln Thr Glu Thr Lys Ala Ser Val Gly Phe Lys Ala Gly
1               5                   10                  15

Val Lys Asp Tyr Lys Leu Thr Tyr Tyr Thr Pro Asp Tyr Glu Thr Lys
            20                  25                  30

Asp Thr Asp Ile Leu Ala Ala Phe Arg Val Thr Pro Gln Pro Gly Val
        35                  40                  45

Pro Pro Glu Glu Ala Gly Ala Ala Val Ala Ala Glu Ser Ser Thr Gly
    50                  55                  60

Thr Trp Thr Thr Val Trp Thr Asp Gly Leu Thr Ser Leu Asp Arg Tyr
65                  70                  75                  80

Lys Gly Arg Cys Tyr Gly Leu Glu Pro Val Ala Gly Glu Glu Asn Gln
                85                  90                  95

Tyr Ile Ala Tyr Val Ala Tyr Pro Leu Asp Leu Phe Glu Glu Gly Ser
            100                 105                 110

Val Thr Asn Met Phe Thr Ser Ile Val Gly Asn Val Phe Gly Phe Lys
        115                 120                 125

Ala Leu Arg Ala Leu Arg Leu Glu Asp Leu Arg Ile Pro Thr Ala Tyr
    130                 135                 140

Ile Lys Thr Phe Gln Gly Pro Pro His Gly Ile Gln Val Glu Arg Asp
145                 150                 155                 160

Lys Leu Asn Lys Tyr Gly Arg Pro Leu Leu Gly Cys Thr Ile Lys Pro
                165                 170                 175

Lys Leu Gly Leu Ser Ala Lys Asn Tyr Gly Arg Ala Val Tyr Glu Cys
            180                 185                 190

Leu Arg Gly Gly Leu Asp Phe Thr Lys Asp Asp Glu Asn Val Asn Ser
        195                 200                 205

Gln Pro Phe Met Arg Trp Arg Asp Arg Phe Leu Phe Cys Ala Glu Ala
    210                 215                 220

Ile Phe Lys Ser Gln Ala Glu Thr Gly Glu Ile Lys Gly His Tyr Leu
225                 230                 235                 240

Asn Ala Thr Ala Gly Thr Cys Glu Glu Met Met Lys Arg Ala Val Phe
                245                 250                 255

Ala Arg Glu Leu Gly Val Pro Ile Val Met His Asp Tyr Leu Thr Gly
            260                 265                 270

Gly Phe Thr Ala Asn Thr Ser Leu Ala His Tyr Cys Arg Asp Asn Gly
        275                 280                 285

Leu Leu Leu His Ile His Arg Ala Met His Ala Val Ile Asp Arg Gln
    290                 295                 300

Lys Asn His Gly Met His Phe Arg Val Leu Ala Lys Ala Leu Arg Leu
305                 310                 315                 320

Ser Gly Gly Asp His Val His Ala Gly Thr Val Val Gly Lys Leu Glu
                325                 330                 335

Gly Glu Arg Glu Ile Thr Leu Gly Phe Val Asp Leu Leu Arg Asp Asp
            340                 345                 350

Phe Val Glu Lys Asp Arg Ser Arg Gly Ile Tyr Phe Thr Gln Asp Trp
        355                 360                 365

Val Ser Leu Pro Gly Val Leu Pro Val Ala Ser Gly Gly Ile His Val
    370                 375                 380

Trp His Met Pro Ala Leu Thr Glu Ile Phe Gly Asp Asp Ser Val Leu
385                 390                 395                 400

Gln Phe Gly Gly Gly Thr Leu Gly His Pro Trp Gly Asn Ala Pro Gly
                405                 410                 415

Ala Val Ala Asn Arg Val Ala Leu Glu Ala Cys Val Gln Ala Arg Asn
            420                 425                 430

Glu Gly Arg Asp Leu Ala Arg Glu Gly Asn Glu Ile Ile Arg Glu Ala
        435                 440                 445

Ser Lys Trp Ser Pro Glu Leu Ala Ala Ala Cys Glu Val Trp Lys Glu
    450                 455                 460

Ile Lys Phe Glu Phe Glu Ala Met Asp Thr Leu
465                 470                 475

<210>  5
<211>  1026
<212>  DNA
<213>  Glycine max

<400>  5

atgccaaacg acagcgttct gagtcttttc ttcttcgtta ccctcttcac atgccttctc 60

tctgctactt cccacgatga ccatattttc ctaccgagtc aactccatga cgatgactca 120

gtgagttgca ctgcaaccga cccaagcctt aactacaagc cggtgatcgg aatcctgacc 180

caccccggcg acggtgcctc tggccggctc agcaacgcca ccggggtttc ttacatcgcc 240

gcctcctacg ttaaattcgt ggagtccggt ggtgctagag ttattcccct tatctacaat 300

gagtcacctg agaaccttaa caagaagctc gatttggtca atggagtgct cttcactggt 360

gggtgggctg tttctggtcc atatttggat actcttggaa acatttttaa gaaagcttta 420

gagagaaatg atgctggaga ccatttccca gtaattgcct tcaacttagg tggaaacctc 480

gtaataagga tcgtaagcga gcaaactgat attcttgaac catttactgc gtcgagtttg 540

ccaagttctc ttgtattgtg gaacgaagca aatgctaaag gaagtctatt tcaaagattt 600

ccttcagatt tgttgacaca gctgaagaca gattgtctcg tcctgcacaa tcatcgttat 660

gccatatccc cacgcaagct tcaatataat accaagttat ctgacttttt tgagattctg 720

gcaacatctg gggatagaga tggaaagact ttcgtttcca cagcacgtgg taggaaatat 780

cccgtgactg tcaacctatg gcaaccagag aaaaatgcgt ttgagtgggc tacatctttg 840

aaagcccctc acacagagga tgcaattcgg gttactcagt ccacagcaaa ctttttcata 900

agtgaagcta ggaaatccac aaacacacct gatgctcaaa aagtgcgaga tagtctcatc 960

tacaattaca aacctacttt tggtggaacg gctgggaaag gatatgatca agtgtatctc 1020

tttgaa 1026

<210> 6
<211> 342
<212> PRT
<213> Glycine max

<400> 6

Met Pro Asn Asp Ser Val Leu Ser Leu Phe Phe Phe Val Thr Leu Phe
1               5                   10                  15

Thr Cys Leu Leu Ser Ala Thr Ser His Asp Asp His Ile Phe Leu Pro
            20                  25                  30

Ser Gln Leu His Asp Asp Asp Ser Val Ser Cys Thr Ala Thr Asp Pro
        35                  40                  45

Ser Leu Asn Tyr Lys Pro Val Ile Gly Ile Leu Thr His Pro Gly Asp
    50                  55                  60

Gly Ala Ser Gly Arg Leu Ser Asn Ala Thr Gly Val Ser Tyr Ile Ala
65                  70                  75                  80

Ala Ser Tyr Val Lys Phe Val Glu Ser Gly Gly Ala Arg Val Ile Pro
                85                  90                  95

Leu Ile Tyr Asn Glu Ser Pro Glu Asn Leu Asn Lys Lys Leu Asp Leu
            100                 105                 110

Val Asn Gly Val Leu Phe Thr Gly Gly Trp Ala Val Ser Gly Pro Tyr
        115                 120                 125

Leu Asp Thr Leu Gly Asn Ile Phe Lys Lys Ala Leu Glu Arg Asn Asp
    130                 135                 140

Ala Gly Asp His Phe Pro Val Ile Ala Phe Asn Leu Gly Gly Asn Leu
145                 150                 155                 160

Val Ile Arg Ile Val Ser Glu Gln Thr Asp Ile Leu Glu Pro Phe Thr
                165                 170                 175

Ala Ser Ser Leu Pro Ser Ser Leu Val Leu Trp Asn Glu Ala Asn Ala
            180                 185                 190

Lys Gly Ser Leu Phe Gln Arg Phe Pro Ser Asp Leu Leu Thr Gln Leu
        195                 200                 205

Lys Thr Asp Cys Leu Val Leu His Asn His Arg Tyr Ala Ile Ser Pro
    210                 215                 220

Arg Lys Leu Gln Tyr Asn Thr Lys Leu Ser Asp Phe Phe Glu Ile Leu
225                 230                 235                 240

Ala Thr Ser Gly Asp Arg Asp Gly Lys Thr Phe Val Ser Thr Ala Arg
                245                 250                 255

Gly Arg Lys Tyr Pro Val Thr Val Asn Leu Trp Gln Pro Glu Lys Asn
            260                 265                 270

Ala Phe Glu Trp Ala Thr Ser Leu Lys Ala Pro His Thr Glu Asp Ala
        275                 280                 285

Ile Arg Val Thr Gln Ser Thr Ala Asn Phe Phe Ile Ser Glu Ala Arg
    290                 295                 300

Lys Ser Thr Asn Thr Pro Asp Ala Gln Lys Val Arg Asp Ser Leu Ile
305                 310                 315                 320

Tyr Asn Tyr Lys Pro Thr Phe Gly Gly Thr Ala Gly Lys Gly Tyr Asp
                325                 330                 335

Gln Val Tyr Leu Phe Glu
            340

<210>  7
<211>  975
<212>  DNA
<213>  Glycine max

<400>  7
atggcggaag gactcgtcct gaagggaacc atgcgcgccc acaccgacgt cgtgacggcg      60

attgccactc ccatcgacaa ctccgacatg atcgtgacgg cgtcgcgcga cagatccatc     120

attctctggc acctcaccaa ggaggacaag acctacggtg tcccccgccg ccgcctcacc     180

ggacattctc acttcgtcca ggacgtcgtc ctctcatccg acggtcagtt cgccctctcc     240

ggctcctggg acggcgagct ccgcctctgg gacctcgcgg ctggcacctc tgcccgccgc     300

ttcgttggcc acaccaagga cgtgctctcc gtggcgttct ccatcgacaa ccgtcagatc     360

gtgtcggcct ctcgtgaccg cacgatcaag ctgtggaaca ccctgggtga gtgcaagtac     420

acaatccaag atggcgatgc gcattcggat tgggtaagtt gcgtccgttt cagccctagc     480

actcttcagc caaccattgt ttctgcttca tgggacagga ccgttaaggt ttggaacctg     540

accaactgca agctgaggaa cacccttgct ggacacaatg ggtatgtgaa tactgttgct     600

gtttcccctg atggctctct ctgtgccagt ggtggcaaag atggggttat tcttctgtgg     660

gatttggctg agggtaagcg tctttactct ctcgatgctg gctcaatcat ccatgcactc     720

tgcttcagcc ccagcaggta ctggctctgc gccgccaccg agcagagcat caagatctgg     780

gatttggaga gcaagagtat cgttgaggat ttgaaggttg acctcaagac tgaggctgat     840

gccacctccg gtggtggtaa cgccaacaag aagaaggtta tttattgcac aagtttgaac     900

tggagtgcgg atggaagcac tttgtttagt ggctataccg atggtgtggc cagagtttgg     960

gctattggac gttat                                                      975

<210>  8
<211>  325
<212>  PRT
<213>  Glycine max

<400>  8

Met Ala Glu Gly Leu Val Leu Lys Gly Thr Met Arg Ala His Thr Asp
1               5                   10                  15

Val Val Thr Ala Ile Ala Thr Pro Ile Asp Asn Ser Asp Met Ile Val
            20                  25                  30

Thr Ala Ser Arg Asp Arg Ser Ile Ile Leu Trp His Leu Thr Lys Glu

        35                  40                  45

Asp Lys Thr Tyr Gly Val Pro Arg Arg Arg Leu Thr Gly His Ser His
    50                  55                  60

Phe Val Gln Asp Val Val Leu Ser Ser Asp Gly Gln Phe Ala Leu Ser
65                  70                  75                  80

Gly Ser Trp Asp Gly Glu Leu Arg Leu Trp Asp Leu Ala Ala Gly Thr
                85                  90                  95

Ser Ala Arg Arg Phe Val Gly His Thr Lys Asp Val Leu Ser Val Ala
            100                 105                 110

Phe Ser Ile Asp Asn Arg Gln Ile Val Ser Ala Ser Arg Asp Arg Thr
        115                 120                 125

Ile Lys Leu Trp Asn Thr Leu Gly Glu Cys Lys Tyr Thr Ile Gln Asp
    130                 135                 140

Gly Asp Ala His Ser Asp Trp Val Ser Cys Val Arg Phe Ser Pro Ser
145                 150                 155                 160

Thr Leu Gln Pro Thr Ile Val Ser Ala Ser Trp Asp Arg Thr Val Lys
                165                 170                 175

Val Trp Asn Leu Thr Asn Cys Lys Leu Arg Asn Thr Leu Ala Gly His
            180                 185                 190

Asn Gly Tyr Val Asn Thr Val Ala Val Ser Pro Asp Gly Ser Leu Cys
        195                 200                 205

Ala Ser Gly Gly Lys Asp Gly Val Ile Leu Leu Trp Asp Leu Ala Glu
    210                 215                 220

Gly Lys Arg Leu Tyr Ser Leu Asp Ala Gly Ser Ile Ile His Ala Leu
225                 230                 235                 240

Cys Phe Ser Pro Ser Arg Tyr Trp Leu Cys Ala Ala Thr Glu Gln Ser
                245                 250                 255

Ile Lys Ile Trp Asp Leu Glu Ser Lys Ser Ile Val Glu Asp Leu Lys
            260                 265                 270

Val Asp Leu Lys Thr Glu Ala Asp Ala Thr Ser Gly Gly Gly Asn Ala
        275                 280                 285

Asn Lys Lys Lys Val Ile Tyr Cys Thr Ser Leu Asn Trp Ser Ala Asp
    290                 295                 300

Gly Ser Thr Leu Phe Ser Gly Tyr Thr Asp Gly Val Ala Arg Val Trp
305                 310                 315                 320

Ala Ile Gly Arg Tyr
                325

<210>  9
<211>  303
<212>  DNA
<213>  Glycine max

<400>  9
atggcatctc atcaacaaaa caagcaagag cttgatgaaa gggcaaggca aggagagact      60

gttgttccgg gtggcactgg tggcaagagc cttgaggctc agcaacatct tgctgaagga     120

aggagcaggg gagggaaaac aaggaaggag cagctgggga cagaaggata ccatgaaatg     180

ggacgcaaag gtgggttgag cactatggac aagtcaggag aagaacgtgc tcaagaggaa     240

gccattgaca tcgatgagtc caagttcagg actggtaata accaagacaa gaaccagaac     300

aag                                                                   303

<210>  10
<211>  101
<212>  PRT
<213>  Glycine max

<400>  10

Met Ala Ser His Gln Gln Asn Lys Gln Glu Leu Asp Glu Arg Ala Arg
1               5                   10                  15

Gln Gly Glu Thr Val Val Pro Gly Gly Thr Gly Gly Lys Ser Leu Glu
            20                  25                  30

Ala Gln Gln His Leu Ala Glu Gly Arg Ser Arg Gly Gly Lys Thr Arg
        35                  40                  45

Lys Glu Gln Leu Gly Thr Glu Gly Tyr His Glu Met Gly Arg Lys Gly
    50                  55                  60

Gly Leu Ser Thr Met Asp Lys Ser Gly Glu Glu Arg Ala Gln Glu Glu
65                  70                  75                  80

Ala Ile Asp Ile Asp Glu Ser Lys Phe Arg Thr Gly Asn Asn Gln Asp
                85                  90                  95

Lys Asn Gln Asn Lys

            100

<210> 11
<211> 1929
<212> DNA
<213> Glycine max

<400> 11
atggcgtctg aacaattggc tcgcagagag aacaccacca ccgagaagga gattcacgtt 60

gagaaacaca gggttcccaa gatggccacc cacttcgagc accttgcgga gcaagctaaa 120

gaatctgaca tcaccgctgg gaaggatact ccgcagggta gcatagaagc cttgcaagct 180

ggagagaggg tcaaagatca tgcaggaaaa gccatgggag atattggtgg acgtgggaaa 240

gccagagaga ctcacgagct gggtgcacac tttgaatccc tggctgacaa agtaactgac 300

catgcagcag ccaatgtagt tggaaacaaa gagagtcaaa gagaagctcg tggtggtgtt 360

cgtgatgttg ggaagttcga aatgagaact gaagggggag aaaaaggaaa caaggataga 420

ccagaactga aaacacgaac cagagaggta ataggaagaa cggagaaaga aagaggaaga 480

gaaagtggag gacaagtagt ggcagagaaa ggaagagaaa cagaaactgc aaggggaagg 540

gttggtgctg aaaatgaagg tgcaagaaca accgctgtta taacttgcac gttggaaaaa 600

ggtggtggta ctcagaaacc aataagagaa gaagagagag agagtgaaag tgaaagatca 660

gcgtgggaac agatttccaa ctacagtgat caagccacac aaggtgtgaa ggaaaagtac 720

gagagagcaa agcaagcagc aagcgagaca ctgaacacca caacacaaac cgcacaagag 780

aaaagcgcac aggcaaagaa tcttgcagca caagcaaagg acgcaacgtt agagaagggt 840

cagcaaggct atgctgtgac aaaggacacc atttcaagtg cagcgaaaac cgcttcggag 900

aaaactgcgc cagtggcaga gaaggccaaa gactacactc ttcaggctgc ggagaaggcc 960

aagagtgcgg gtggcacaac ggcgagttac gtcggagaga aagcggtgca ggccaaggat 1020

gtcgccgtgg agagtgggaa gagtgcggct gggtatgctg ccaaggtggc tgctgatttg 1080

agggacaagg ccaccgcggt ggggtgggcc gcggcccatt tttcggcgga aaagacggtg 1140

gaagggacca aggccgccgc ccatgttgtc gagggagcgg ccgggtatgc cggacataag 1200

gccgcggagc ttgcttcgat gtcggccggt gctgttaaag ggctggctgc ttctgctggt 1260

gagactgcta aggaatacac cgcaaagaag aaagaggagg cacagcggga attggaggcc 1320

aaaaaaccat ctcaacctca ggaagctgaa gagaggccat cagaaggaat tggcgaaact 1380

gtgaggcagt atgcacaaaa accaaagcca agtgaaggaa accctcagaa ggagggaacc 1440

ggaagcatcg tgtttacagc aataggcgaa acagtaagca gtgccggtga gaaggtaaag 1500

aaaccgttta aaaacactat gggaggagaa agtgaaggag gaggagggaa agaagaggga 1560

aagagtgtca ttggtaaaag tttgaccagc attggtgaga agctgggtga tgcaaaacag 1620

agagaggagc tgttagacaa cgtcactggc aacatcaccg agggaggagg cgaggtgttg 1680

ggagcagtag gtgaaaccgt tgctgagatt ggacagaaca tgatgaagcc agcggagata 1740

gttcaggaac gcgctcacgt tcgacaagcg ggtggggtct tggatgccat tggtgaaacc 1800

attgctgaga ttgctgaaac gactcgagtc atggtttctg gagaggacga gagagtgctg 1860

aggcagagcg tggtgttgga gactcgcgta actggtcgtg ccaagcatga agaagggtcg 1920

cacggtgct 1929

<210> 12
<211> 643
<212> PRT
<213> Glycine max

<400> 12

Met Ala Ser Glu Gln Leu Ala Arg Arg Glu Asn Thr Thr Thr Glu Lys
1               5                   10                  15

Glu Ile His Val Glu Lys His Arg Val Pro Lys Met Ala Thr His Phe
            20                  25                  30

Glu His Leu Ala Glu Gln Ala Lys Glu Ser Asp Ile Thr Ala Gly Lys
        35                  40                  45

Asp Thr Pro Gln Gly Ser Ile Glu Ala Leu Gln Ala Gly Glu Arg Val
    50                  55                  60

Lys Asp His Ala Gly Lys Ala Met Gly Asp Ile Gly Gly Arg Gly Lys
65                  70                  75                  80

Ala Arg Glu Thr His Glu Leu Gly Ala His Phe Glu Ser Leu Ala Asp
                85                  90                  95

Lys Val Thr Asp His Ala Ala Ala Asn Val Val Gly Asn Lys Glu Ser
            100                 105                 110

Gln Arg Glu Ala Arg Gly Gly Val Arg Asp Val Gly Lys Phe Glu Met
        115                 120                 125

Arg Thr Glu Gly Gly Glu Lys Gly Asn Lys Asp Arg Pro Glu Leu Lys
    130                 135                 140

Thr Arg Thr Arg Glu Val Ile Gly Arg Thr Glu Lys Glu Arg Gly Arg
145                 150                 155                 160

Glu Ser Gly Gly Gln Val Val Ala Glu Lys Gly Arg Glu Thr Glu Thr
                165                 170                 175

Ala Arg Gly Arg Val Gly Ala Glu Asn Glu Gly Ala Arg Thr Thr Ala
            180                 185                 190
Val Ile Thr Cys Thr Leu Glu Lys Gly Gly Gly Thr Gln Lys Pro Ile
        195                 200                 205
Arg Glu Glu Glu Arg Glu Ser Glu Ser Glu Arg Ser Ala Trp Glu Gln
    210                 215                 220
Ile Ser Asn Tyr Ser Asp Gln Ala Thr Gln Gly Val Lys Glu Lys Tyr
225                 230                 235                 240
Glu Arg Ala Lys Gln Ala Ala Ser Glu Thr Leu Asn Thr Thr Thr Gln
                245                 250                 255
Thr Ala Gln Glu Lys Ser Ala Gln Ala Lys Asn Leu Ala Ala Gln Ala
            260                 265                 270
Lys Asp Ala Thr Leu Glu Lys Gly Gln Gln Gly Tyr Ala Val Thr Lys
        275                 280                 285
Asp Thr Ile Ser Ser Ala Ala Lys Thr Ala Ser Glu Lys Thr Ala Pro
    290                 295                 300
Val Ala Glu Lys Ala Lys Asp Tyr Thr Leu Gln Ala Ala Glu Lys Ala
305                 310                 315                 320
Lys Ser Ala Gly Gly Thr Thr Ala Ser Tyr Val Gly Glu Lys Ala Val
                325                 330                 335
Gln Ala Lys Asp Val Ala Val Glu Ser Gly Lys Ser Ala Ala Gly Tyr
            340                 345                 350
Ala Ala Lys Val Ala Ala Asp Leu Arg Asp Lys Ala Thr Ala Val Gly
        355                 360                 365
Trp Ala Ala Ala His Phe Ser Ala Glu Lys Thr Val Glu Gly Thr Lys
    370                 375                 380
Ala Ala Ala His Val Val Glu Gly Ala Ala Gly Tyr Ala Gly His Lys
385                 390                 395                 400
Ala Ala Glu Leu Ala Ser Met Ser Ala Gly Ala Val Lys Gly Leu Ala
                405                 410                 415
Ala Ser Ala Gly Glu Thr Ala Lys Glu Tyr Thr Ala Lys Lys Lys Glu
            420                 425                 430

Glu Ala Gln Arg Glu Leu Glu Ala Lys Lys Pro Ser Gln Pro Gln Glu
      435                 440                 445

Ala Glu Glu Arg Pro Ser Glu Gly Ile Gly Glu Thr Val Arg Gln Tyr
    450                 455                 460

Ala Gln Lys Pro Lys Pro Ser Glu Gly Asn Pro Gln Lys Glu Gly Thr
465                 470                 475                 480

Gly Ser Ile Val Phe Thr Ala Ile Gly Glu Thr Val Ser Ser Ala Gly
                485                 490                 495

Glu Lys Val Lys Lys Pro Phe Lys Asn Thr Met Gly Gly Glu Ser Glu
            500                 505                 510

Gly Gly Gly Gly Lys Glu Glu Gly Lys Ser Val Ile Gly Lys Ser Leu
      515                 520                 525

Thr Ser Ile Gly Glu Lys Leu Gly Asp Ala Lys Gln Arg Glu Glu Leu
    530                 535                 540

Leu Asp Asn Val Thr Gly Asn Ile Thr Glu Gly Gly Gly Glu Val Leu
545                 550                 555                 560

Gly Ala Val Gly Glu Thr Val Ala Glu Ile Gly Gln Asn Met Met Lys
                565                 570                 575

Pro Ala Glu Ile Val Gln Glu Arg Ala His Val Arg Gln Ala Gly Gly
            580                 585                 590

Val Leu Asp Ala Ile Gly Glu Thr Ile Ala Glu Ile Ala Glu Thr Thr
      595                 600                 605

Arg Val Met Val Ser Gly Glu Asp Glu Arg Val Leu Arg Gln Ser Val
    610                 615                 620

Val Leu Glu Thr Arg Val Thr Gly Arg Ala Lys His Glu Glu Gly Ser
625                 630                 635                 640

His Gly Ala

<210>  13
<211>  771
<212>  DNA
<213>  Glycine max

<220>
<221>  misc_feature
<222>  (242)..(242)
<223>  n is a, c, g, or t

<400>  13
atggcccttt cttgctccaa agttttaagc ttttatctct cgcccgttgt gggtggtggt      60

gatgttccga aaaagctcac tttttcttct tttttgggtc tttctaaggg tgttggtgga     120

agcaggagct cgagggtgtg tgctgcttca aatgcacccg caccactcgc tggggtcata     180

tttgaaccat ttcaagagct caagaaggat tatcttgctg ttccgattgc acacaatgtt     240

tncttggctc gtcagaacta tgcagatgac tctgaatctg caatcaacga gcagatcaat     300

gtggaataca acgtttccta tgtgtaccac gccttgtttg cgtacttcga cagagacaac     360

atagctctca aaggacttgc aaagttcttc aaggaatcaa gtgaagaaga aagagagcat     420

gccgagcagc ttataaagta tcagaacatt cgtggtggac gagtggtgct gcacccaatc     480

acaagtcctc cctcggaatt tgagcattcg gaaaaagggg atgccttgta tgccatggaa     540

ctagctttgt ctttggagaa gttgacgaat gagaaacttc tgcacgttca cagtgtggca     600

gaacgtaaca atgatcctca aytdgcagac ttcattgaga gcgagttctt gtatgagcag     660

gttaaatcga ttaaaaagat tgcagagtat gtggctcaac tgagattagt tggaaagggt     720

catggtgtgt ggcactttga tcaaaagctt cttcacgatg aagatcatgt a              771


<210>  14
<211>  257
<212>  PRT
<213>  Glycine max


<220>
<221>  misc_feature
<222>  (81)..(81)
<223>  Xaa can be any amino acid

<220>
<221>  misc_feature
<222>  (208)..(208)
<223>  Xaa can be any amino acid

<400>  14

Met Ala Leu Ser Cys Ser Lys Val Leu Ser Phe Tyr Leu Ser Pro Val
1               5                   10                  15


Val Gly Gly Gly Asp Val Pro Lys Lys Leu Thr Phe Ser Ser Phe Leu
            20                  25                  30


Gly Leu Ser Lys Gly Val Gly Gly Ser Arg Ser Ser Arg Val Cys Ala
        35                  40                  45

Ala Ser Asn Ala Pro Ala Pro Leu Ala Gly Val Ile Phe Glu Pro Phe
    50                  55                  60

Gln Glu Leu Lys Lys Asp Tyr Leu Ala Val Pro Ile Ala His Asn Val
65                  70                  75                  80

Xaa Leu Ala Arg Gln Asn Tyr Ala Asp Asp Ser Glu Ser Ala Ile Asn
                85                  90                  95

Glu Gln Ile Asn Val Glu Tyr Asn Val Ser Tyr Val Tyr His Ala Leu
            100                 105                 110

Phe Ala Tyr Phe Asp Arg Asp Asn Ile Ala Leu Lys Gly Leu Ala Lys
        115                 120                 125

Phe Phe Lys Glu Ser Ser Glu Glu Glu Arg Glu His Ala Glu Gln Leu
    130                 135                 140

Ile Lys Tyr Gln Asn Ile Arg Gly Gly Arg Val Val Leu His Pro Ile
145                 150                 155                 160

Thr Ser Pro Pro Ser Glu Phe Glu His Ser Glu Lys Gly Asp Ala Leu
                165                 170                 175

Tyr Ala Met Glu Leu Ala Leu Ser Leu Glu Lys Leu Thr Asn Glu Lys
            180                 185                 190

Leu Leu His Val His Ser Val Ala Glu Arg Asn Asn Asp Pro Gln Xaa
        195                 200                 205

Ala Asp Phe Ile Glu Ser Glu Phe Leu Tyr Glu Gln Val Lys Ser Ile
    210                 215                 220

Lys Lys Ile Ala Glu Tyr Val Ala Gln Leu Arg Leu Val Gly Lys Gly
225                 230                 235                 240

His Gly Val Trp His Phe Asp Gln Lys Leu Leu His Asp Glu Asp His
                245                 250                 255

Val

<210>  15
<211>  2421
<212>  DNA
<213>  Glycine max

<400>  15

atgtctcagc aaggcgaatc atcggacccg aaatcgggga agaaggactt ctcaaccgcg 60

attctggaac gcaaaaagtc gccgaaccgg cttgtcgtgg atgaggctgt caacgacgac 120

aactccgtgg tcaccatgca tccccaaacc atggaaaagc ttcagctttt ccgaggggac 180

actatcctca tcaagggtaa gaaacggaag gataccattt gcatagctct tgctgatgag 240

aactgtgagg agccgaagat caggatgaat aaagttgtga ggtcaaattt gcgggttcgg 300

cttggagatg ttgtgtctgt gcaccagtgc ccggatgtta aatatgggaa acgtgtgcac 360

attctgccta ttgatgatac cattgaaggt gtcactggca atctgtttga tgctttcttg 420

aaaccctatt tcttggaagc ttaccgtcct gtcagaaaag gagatctatt tcttgtccgg 480

ggagggatga gaagtgtaga gttcaaggtg gttgaaaccg atcctggaga atattgtgtt 540

gttgctccag acactgaaat tttctgtgag ggggagcctt tgaaaagaga agatgaagag 600

agactggatg aagtcgggta tgatgatgtg ggtggtgtca ggaaacaaat ggctcagatt 660

cgtgagttgg ttgagcttcc attgaggcat ccacagcttt ttaaatcaat tggtgtgaaa 720

ccacccaaag gaattttact ttatggaccc ccaggttctg ggaagacatt aatagcaaga 780

gctgtagcta atgaaactgg agcttttttc ttttgtatta acggaccaga gattatgtcc 840

aaacttgctg gagagagtga aagcaatctc aggaaagcgt ttgaagaagc agaaaagaat 900

gcaccatcca ttatcttcat tgatgaaatt gattccattg cccccaagcg ggagaagaca 960

catggtgaag ttgaaaggag gattgtttca caacttttga ctcttatgga tggattaaaa 1020

tctcgtgctc atgttattgt tattggagct actaatcgtc caaatagcat tgacccagca 1080

ttgagaaggt ttggtagatt tgatagggaa attgatattg gtgttcccga tgaagttggg 1140

agacttgaag tccttcgcat acatactaaa aacatgaagc tatctgatga tgttgattta 1200

gagagaattg caaaagatac tcatgggtat gttggtgctg accttgctgc cctttgcact 1260

gaagctgctt tgcaatgcat tagggagaag atggatgtaa ttgacttgga ggatgagact 1320

attgatgcag aagtactgaa ttccatggca gttacaaatg agcatttcca gactgccctt 1380

ggaacaagca acccatcagc tttacgtgaa actgttgttg aagtgcctaa tgtcagctgg 1440

gaagacattg gaggccttga aaatgtcaag cgtgaactgc aagagactgt acaatatcca 1500

gttgagcacc cagaaaagtt tgagaagttt ggtatgtctc catctaaagg agtacttttc 1560

tatggtcctc caggatgtgg gaaaactttg ttagccaaag caattgctaa tgaatgtcaa 1620

gctaacttca tcagtgtgaa aggtccagaa ttgcttacaa tgtggtttgg tgaaagtgaa 1680

gctaatgtta gagaaatttt tgacaaggcc agacaatcgg ctccatgtgt cctcttcttt 1740

gatgagcttg actctattgc cactcagaga ggaagcagtg ttggggatgc tggtggtgcg 1800

gctgatagag ttctaaatca acttttgact gaaatggatg gcatgtctgc caaaaagact 1860

gtgttcataa ttggggcaac taacagacct gacatcattg acccagcact tcttcgacca 1920

ggccgtctgg atcaattgat ctatattcct ctccctgatg aggattcccg gcatcaaatc 1980

tttaaagcct gcttgaggaa gtcacctatc gcaaaaaatg ttgatttgag agcattggct 2040

aggcacactc aagggttcag tggtgctgat attacagaaa tatgccagcg agcatgcaaa 2100

tatgccataa gagaaaacat agagaaggac attgagcgag agaggaagag tagggaaaac 2160

cctgaggcca tggatgaaga taccgtcgac gacgaagttg cggagatcaa ggctgctcat 2220

tttgaggagt caatgaagtt tgcacggagg agtgttagtg atgctgatat tcgcaagtat 2280

caggcatttg ctcagacctt acagcaatca agagggtttg gaagtgaatt taggtttcca 2340

gagtctggag ataggactac tactggatct gacccctttg cagcttctgc tggtggggct 2400

gacgaagatg atctatatag t 2421

<210> 16
<211> 807
<212> PRT
<213> Glycine max

<400> 16

Met Ser Gln Gln Gly Glu Ser Ser Asp Pro Lys Ser Gly Lys Lys Asp
1               5                   10                  15

Phe Ser Thr Ala Ile Leu Glu Arg Lys Lys Ser Pro Asn Arg Leu Val
            20                  25                  30

Val Asp Glu Ala Val Asn Asp Asp Asn Ser Val Val Thr Met His Pro
        35                  40                  45

Gln Thr Met Glu Lys Leu Gln Leu Phe Arg Gly Asp Thr Ile Leu Ile
    50                  55                  60

Lys Gly Lys Lys Arg Lys Asp Thr Ile Cys Ile Ala Leu Ala Asp Glu
65                  70                  75                  80

Asn Cys Glu Glu Pro Lys Ile Arg Met Asn Lys Val Val Arg Ser Asn
                85                  90                  95

Leu Arg Val Arg Leu Gly Asp Val Val Ser Val His Gln Cys Pro Asp
            100                 105                 110

Val Lys Tyr Gly Lys Arg Val His Ile Leu Pro Ile Asp Asp Thr Ile
        115                 120                 125

Glu Gly Val Thr Gly Asn Leu Phe Asp Ala Phe Leu Lys Pro Tyr Phe
    130                 135                 140

Leu Glu Ala Tyr Arg Pro Val Arg Lys Gly Asp Leu Phe Leu Val Arg
145                 150                 155                 160

Gly Gly Met Arg Ser Val Glu Phe Lys Val Val Glu Thr Asp Pro Gly
                165                 170                 175

Glu Tyr Cys Val Val Ala Pro Asp Thr Glu Ile Phe Cys Glu Gly Glu
            180                 185                 190

Pro Leu Lys Arg Glu Asp Glu Glu Arg Leu Asp Glu Val Gly Tyr Asp
        195                 200                 205

Asp Val Gly Gly Val Arg Lys Gln Met Ala Gln Ile Arg Glu Leu Val
    210                 215                 220

Glu Leu Pro Leu Arg His Pro Gln Leu Phe Lys Ser Ile Gly Val Lys
225                 230                 235                 240

Pro Pro Lys Gly Ile Leu Leu Tyr Gly Pro Pro Gly Ser Gly Lys Thr
                245                 250                 255

Leu Ile Ala Arg Ala Val Ala Asn Glu Thr Gly Ala Phe Phe Phe Cys
            260                 265                 270

Ile Asn Gly Pro Glu Ile Met Ser Lys Leu Ala Gly Glu Ser Glu Ser
        275                 280                 285

Asn Leu Arg Lys Ala Phe Glu Glu Ala Glu Lys Asn Ala Pro Ser Ile
    290                 295                 300

Ile Phe Ile Asp Glu Ile Asp Ser Ile Ala Pro Lys Arg Glu Lys Thr
305                 310                 315                 320

His Gly Glu Val Glu Arg Arg Ile Val Ser Gln Leu Leu Thr Leu Met
                325                 330                 335

Asp Gly Leu Lys Ser Arg Ala His Val Ile Val Ile Gly Ala Thr Asn
            340                 345                 350

Arg Pro Asn Ser Ile Asp Pro Ala Leu Arg Arg Phe Gly Arg Phe Asp
        355                 360                 365

Arg Glu Ile Asp Ile Gly Val Pro Asp Glu Val Gly Arg Leu Glu Val
    370                 375                 380

Leu Arg Ile His Thr Lys Asn Met Lys Leu Ser Asp Asp Val Asp Leu
385                 390                 395                 400

Glu Arg Ile Ala Lys Asp Thr His Gly Tyr Val Gly Ala Asp Leu Ala
                405                 410                 415

Ala Leu Cys Thr Glu Ala Ala Leu Gln Cys Ile Arg Glu Lys Met Asp
            420                 425                 430

Val Ile Asp Leu Glu Asp Glu Thr Ile Asp Ala Glu Val Leu Asn Ser
        435                 440                 445

Met Ala Val Thr Asn Glu His Phe Gln Thr Ala Leu Gly Thr Ser Asn
    450                 455                 460

Pro Ser Ala Leu Arg Glu Thr Val Val Glu Val Pro Asn Val Ser Trp
465                 470                 475                 480

Glu Asp Ile Gly Gly Leu Glu Asn Val Lys Arg Glu Leu Gln Glu Thr
                485                 490                 495

Val Gln Tyr Pro Val Glu His Pro Glu Lys Phe Glu Lys Phe Gly Met
            500                 505                 510

Ser Pro Ser Lys Gly Val Leu Phe Tyr Gly Pro Pro Gly Cys Gly Lys
        515                 520                 525

Thr Leu Leu Ala Lys Ala Ile Ala Asn Glu Cys Gln Ala Asn Phe Ile
    530                 535                 540

Ser Val Lys Gly Pro Glu Leu Leu Thr Met Trp Phe Gly Glu Ser Glu
545                 550                 555                 560

Ala Asn Val Arg Glu Ile Phe Asp Lys Ala Arg Gln Ser Ala Pro Cys
                565                 570                 575

Val Leu Phe Phe Asp Glu Leu Asp Ser Ile Ala Thr Gln Arg Gly Ser
            580                 585                 590

Ser Val Gly Asp Ala Gly Gly Ala Ala Asp Arg Val Leu Asn Gln Leu
        595                 600                 605

Leu Thr Glu Met Asp Gly Met Ser Ala Lys Lys Thr Val Phe Ile Ile
    610                 615                 620

Gly Ala Thr Asn Arg Pro Asp Ile Ile Asp Pro Ala Leu Leu Arg Pro
625                 630                 635                 640

Gly Arg Leu Asp Gln Leu Ile Tyr Ile Pro Leu Pro Asp Glu Asp Ser
                645                 650                 655

Arg His Gln Ile Phe Lys Ala Cys Leu Arg Lys Ser Pro Ile Ala Lys
            660                 665                 670

Asn Val Asp Leu Arg Ala Leu Ala Arg His Thr Gln Gly Phe Ser Gly
        675                 680                 685

Ala Asp Ile Thr Glu Ile Cys Gln Arg Ala Cys Lys Tyr Ala Ile Arg
    690                 695                 700

Glu Asn Ile Glu Lys Asp Ile Glu Arg Glu Arg Lys Ser Arg Glu Asn
705                 710                 715                 720

Pro Glu Ala Met Asp Glu Asp Thr Val Asp Asp Glu Val Ala Glu Ile
                725                 730                 735

Lys Ala Ala His Phe Glu Glu Ser Met Lys Phe Ala Arg Arg Ser Val
            740                 745                 750

Ser Asp Ala Asp Ile Arg Lys Tyr Gln Ala Phe Ala Gln Thr Leu Gln
        755                 760                 765

Gln Ser Arg Gly Phe Gly Ser Glu Phe Arg Phe Pro Glu Ser Gly Asp
    770                 775                 780

Arg Thr Thr Thr Gly Ser Asp Pro Phe Ala Ala Ser Ala Gly Gly Ala
785                 790                 795                 800

Asp Glu Asp Asp Leu Tyr Ser
                805

<210> 17
<211> 13
<212> PRT
<213> Glycine max

<400> 17

Asp Gln Ser Ser Ser Tyr Asp Asp Asp Glu Tyr Ser Lys
1               5                   10

<210> 18
<211> 15
<212> PRT
<213> Glycine max

<400> 18

Lys Ser Asp Gln Ser Ser Ser Tyr Asp Asp Asp Glu Tyr Ser Lys
1               5                   10                  15

<210>  19
<211>  14
<212>  PRT
<213>  Glycine max

<400>  19

Ser Asp Gln Ser Ser Ser Tyr Asp Asp Asp Glu Tyr Ser Lys
1               5                   10

<210>  20
<211>  9
<212>  PRT
<213>  Glycine max

<400>  20

Ser Tyr Asp Asp Asp Glu Tyr Ser Lys
1               5

<210>  21
<211>  18
<212>  PRT
<213>  Glycine max

<400>  21

Ala Leu Arg Ala Leu Arg Leu Glu Asp Leu Arg Ile Pro Thr Ala Tyr
1               5                   10                  15

Ile Lys

<210>  22
<211>  18
<212>  PRT
<213>  Glycine max

<400>  22

Ala Leu Arg Leu Ser Gly Gly Asp His Val His Ala Gly Thr Val Val
1               5                   10                  15

Gly Lys

<210>  23
<211>  22
<212>  PRT
<213>  Glycine max

<400>  23

Leu Glu Gly Glu Arg Glu Ile Thr Leu Gly Phe Val Asp Leu Leu Arg
1               5                   10                  15

Asp Asp Phe Val Glu Lys
            20

<210>  24
<211>  14
<212>  PRT
<213>  Glycine max

<400>  24

Leu Arg Leu Glu Asp Leu Arg Ile Pro Thr Ala Tyr Ile Lys
1               5                   10

<210>  25
<211>  11
<212>  PRT
<213>  Glycine max

<400>  25

Leu Thr Tyr Tyr Thr Pro Asp Tyr Glu Thr Lys
1               5                   10

<210>  26
<211>  11
<212>  PRT
<213>  Glycine max

<400>  26

Thr Asp Gly Leu Thr Ser Leu Asp Arg Tyr Lys
1               5                   10

<210>  27
<211>  14
<212>  PRT
<213>  Glycine max

<400>  27

Thr Phe Gln Gly Pro Pro His Gly Ile Gln Val Glu Arg Asp
1               5                   10

<210>  28
<211>  15
<212>  PRT
<213>  Glycine max

<400>  28

Thr Phe Gln Gly Pro Pro His Gly Ile Gln Val Glu Arg Asp Lys
1               5                   10                  15

<210>  29
<211>  24
<212>  PRT

<213>  Glycine max

<400>  29

Ala Pro His Thr Glu Asp Ala Ile Arg Val Thr Gln Ser Thr Ala Asn
1               5                   10                  15

Phe Phe Ile Ser Glu Ala Arg Lys
            20

<210>  30
<211>  23
<212>  PRT
<213>  Glycine max

<400>  30

Phe Val Glu Ser Gly Gly Ala Arg Val Ile Pro Leu Ile Tyr Asn Glu
1               5                   10                  15

Ser Pro Glu Asn Leu Asn Lys
            20

<210>  31
<211>  7
<212>  PRT
<213>  Glycine max

<400>  31

Ile Trp Asp Leu Glu Ser Lys
1               5

<210>  32
<211>  7
<212>  PRT
<213>  Glycine max

<400>  32

Ser Ile Val Glu Asp Leu Lys
1               5

<210>  33
<211>  21
<212>  PRT
<213>  Glycine max

<400>  33

Gln Glu Leu Asp Glu Arg Ala Arg Gln Gly Glu Thr Val Val Pro Gly
1               5                   10                  15

Gly Thr Gly Gly Lys
            20

<210>  34
<211>  17
<212>  PRT
<213>  Glycine max

<400>  34

Ser Gly Glu Glu Arg Ala Gln Glu Glu Ala Ile Asp Ile Asp Glu Ser
1               5                   10                  15

Lys

<210>  35
<211>  16
<212>  PRT
<213>  Glycine max

<400>  35

Ala Ala Ala His Val Val Glu Gly Ala Ala Gly Tyr Ala Gly His Lys
1               5                   10                  15

<210>  36
<211>  11
<212>  PRT
<213>  Glycine max

<400>  36

Ala Lys Asp Tyr Thr Leu Gln Ala Ala Glu Lys
1               5                   10

<210>  37
<211>  17
<212>  PRT
<213>  Glycine max

<400>  37

Ala Arg Glu Thr His Glu Leu Gly Ala His Phe Glu Ser Leu Ala Asp
1               5                   10                  15

Lys

<210>  38
<211>  15
<212>  PRT
<213>  Glycine max

<400>  38

Ala Thr Ala Val Gly Trp Ala Ala Ala His Phe Ser Ala Glu Lys
1               5                   10                  15

<210>  39
<211>  17
<212>  PRT
<213>  Glycine max

<400>  39

Asp Thr Pro Gln Gly Ser Ile Glu Ala Leu Gln Ala Gly Glu Arg Val
1               5                   10                  15

Lys

<210>  40
<211>  9
<212>  PRT
<213>  Glycine max

<400>  40

Asp Tyr Thr Leu Gln Ala Ala Glu Lys
1               5

<210>  41
<211>  10
<212>  PRT
<213>  Glycine max

<400>  41

Glu Glu Ala Gln Arg Glu Leu Glu Ala Lys
1               5                   10

<210>  42
<211>  21
<212>  PRT
<213>  Glycine max

<400>  42

Glu Gly Thr Gly Ser Ile Val Phe Thr Ala Ile Gly Glu Thr Val Ser
1               5                   10                  15

Ser Ala Gly Glu Lys
            20

<210>  43
<211>  14
<212>  PRT
<213>  Glycine max

<400>  43

Glu Arg Gly Arg Glu Ser Gly Gly Gln Val Val Ala Glu Lys
1               5                   10

<210>  44
<211>  11
<212>  PRT
<213>  Glycine max

<400>  44

Gly Leu Ala Ala Ser Ala Gly Glu Thr Ala Lys
1               5                   10


<210>  45
<211>  9
<212>  PRT
<213>  Glycine max

<400>  45

Gly Gln Gln Gly Tyr Ala Val Thr Lys
1               5


<210>  46
<211>  26
<212>  PRT
<213>  Glycine max

<400>  46

Lys Pro Ser Gln Pro Gln Glu Ala Glu Glu Arg Pro Ser Glu Gly Ile
1               5                   10                  15


Gly Glu Thr Val Arg Gln Tyr Ala Gln Lys
            20                  25


<210>  47
<211>  8
<212>  PRT
<213>  Glycine max

<400>  47

Pro Ala Glu Ile Val Gln Glu Arg
1               5


<210>  48
<211>  15
<212>  PRT
<213>  Glycine max

<400>  48

Pro Gln Gly Ser Ile Glu Ala Leu Gln Ala Gly Glu Arg Val Lys
1               5                   10                  15


<210>  49
<211>  17
<212>  PRT

<213>  Glycine max

<400>  49

Gln Ala Ala Ser Glu Thr Leu Asn Thr Thr Thr Gln Thr Ala Gln Glu
1               5                   10                  15

Lys

<210>  50
<211>  13
<212>  PRT
<213>  Glycine max

<400>  50

Ser Ala Gly Gly Thr Thr Ala Ser Tyr Val Gly Glu Lys
1               5                   10

<210>  51
<211>  8
<212>  PRT
<213>  Glycine max

<400>  51

Ser Leu Thr Ser Ile Gly Glu Lys
1               5

<210>  52
<211>  16
<212>  PRT
<213>  Glycine max

<400>  52

Thr Pro Gln Gly Ser Ile Glu Ala Leu Gln Ala Gly Glu Arg Val Lys
1               5                   10                  15

<210>  53
<211>  13
<212>  PRT
<213>  Glycine max

<400>  53

Val Thr Asp His Ala Ala Ala Asn Val Val Gly Asn Lys
1               5                   10

<210>  54
<211>  22
<212>  PRT
<213>  Glycine max

<400>  54

Ala Ala Ser Asn Ala Pro Ala Pro Leu Ala Gly Val Ile Phe Glu Pro
1               5                   10                  15

Phe Gln Glu Leu Lys Lys
            20

<210>  55
<211>  21
<212>  PRT
<213>  Glycine max

<400>  55

Ala Ser Asn Ala Pro Ala Pro Leu Ala Gly Val Ile Phe Glu Pro Phe
1               5                   10                  15

Gln Glu Leu Lys Lys
            20

<210>  56
<211>  18
<212>  PRT
<213>  Glycine max

<400>  56

Phe Phe Lys Glu Ser Ser Glu Glu Glu Arg Glu His Ala Glu Gln Leu
1               5                   10                  15

Ile Lys

<210>  57
<211>  10
<212>  PRT
<213>  Glycine max

<400>  57

Gly His Gly Val Trp His Phe Asp Gln Lys
1               5                   10

<210>  58
<211>  13
<212>  PRT
<213>  Glycine max

<400>  58

Ile Ala Glu Tyr Val Ala Gln Leu Arg Leu Val Gly Lys
1               5                   10

<210>  59
<211>  14
<212>  PRT

<213>  Glycine max

<400>  59

Lys Ile Ala Glu Tyr Val Ala Gln Leu Arg Leu Val Gly Lys
1               5                   10


<210>  60
<211>  8
<212>  PRT
<213>  Glycine max

<400>  60

Leu Leu His Asp Glu Asp His Val
1               5


<210>  61
<211>  20
<212>  PRT
<213>  Glycine max

<400>  61

Ser Asn Ala Pro Ala Pro Leu Ala Gly Val Ile Phe Glu Pro Phe Gln
1               5                   10                  15


Glu Leu Lys Lys
            20


<210>  62
<211>  10
<212>  PRT
<213>  Glycine max

<400>  62

Ala Gly Glu Ser Glu Ser Asn Leu Arg Lys
1               5                   10


<210>  63
<211>  13
<212>  PRT
<213>  Glycine max

<400>  63

Gly Glu Ser Glu Ala Asn Val Arg Glu Ile Phe Asp Lys
1               5                   10


<210>  64
<211>  12
<212>  PRT
<213>  Glycine max

<400>  64

Gly Ile Leu Leu Tyr Gly Pro Pro Gly Ser Gly Lys
1               5                   10

<210>  65
<211>  11
<212>  PRT
<213>  Glycine max

<400>  65

Leu Ala Gly Glu Ser Glu Ser Asn Leu Arg Lys
1               5                   10

<210>  66
<211>  12
<212>  PRT
<213>  Glycine max

<400>  66

Leu Ser Asp Asp Val Asp Leu Glu Arg Ile Ala Lys
1               5                   10

<210>  67
<211>  16
<212>  PRT
<213>  Glycine max

<400>  67

Arg Glu Leu Gln Glu Thr Val Gln Tyr Pro Val Glu His Pro Glu Lys
1               5                   10                  15

<210>  68
<211>  15
<212>  PRT
<213>  Glycine max

<400>  68

Thr Val Phe Ile Ile Gly Ala Thr Asn Arg Pro Asp Ile Ile Asp
1               5                   10                  15

<210>  69
<211>  2448
<212>  DNA
<213>  Glycine max

<400>  69
atgaggaagt ggacggttgc ttccgctctc cttcttctct ctctcctctt tctctttgca      60

gatcaaggtc gaaagtttca ggcgaatgcc gagggagatt ccgacgagct tgtagatcca     120

cccaaggtgg aggacaagat cggtgctgtg cctcatggcc tttcaaccga ttcagatgtc     180

gtcaagaggg aggcggagtc gatctcgaag agatctctcc ggagcaacgc ggagaagttt     240

gagttccaag cggaagtgtc gcggcttatg gatatcatca tcaattctct ctacagtaac     300

aaggatatct tcctcagaga gttgatttcc aatgcttctg atgctttgga caagattagg 360

ttcctctccc tcacggataa ggaggtgttg ggagaaggtg ataacaccaa gcttgatatt 420

cagattaagt tggacaagga aaagaaaatt ctctcaattc gagacagagg tatcggtatg 480

acgaaggagg atttgataaa gaatttgggg acaatagcga aatctggaac ttctgcattt 540

gttgagaaga tgcaaacaag tggagatctc aatctgattg ggcagtttgg agtcggcttc 600

tactctgttt atcttgtggc cgactatgtc gaagttatta gcaagaataa cgatgataag 660

cagtatgtat gggaatcaaa agctgatgga gcattcgcta tttcagaaga tacatggaat 720

gaaccacttg gacgaggaac tgagattaga ttacacctca aggaagaggc tggggaatac 780

ttggaggagt ctaaactgaa agaattggtg aagagatact ctgaatttat taacttccct 840

atctatattt gggcaagcaa agaagttgat gtggaggttc ccgctgatga agatgattcc 900

agtgatgaag aggattcatc tgaaagcagc tccaaggagg aaagcgaaga tgaagatgct 960

gataaaagtg aagatgaaga aaaaaagcct aagacaaaga cagtgaagga aacaacttat 1020

gagtgggaac ttttaaatga tgtgaaagct atatggctga ggaatccaaa agaggttaca 1080

gaagaagaat acaccaaatt ctaccactct cttgccaagg atttcagtga tgagaaacct 1140

ttagcatgga gtcatttcac tgctgagggt gatgttgagt tcaaggcagt cctgtttgta 1200

ccacctaagg cacctcaaga tttatatgag agctattata acgcaaacaa atcgaacctg 1260

aagttgtatg ttagacgtgt gttcatttca gatgaattta atgaactgtt gcccaagtac 1320

ctaaactttt tgctgggtct tgttgattct gacactttgc cacttaatgt atcacgagaa 1380

atgcttcaac aacatagtag tctgaagaca atcaagaaga aacttattag gaaagccctt 1440

gatatgatcc gcaggattgc tgatgaggat cctgacgagt caactgacaa agaaaagaaa 1500

gatgcatcct ctgacaatga tgagaagaaa ggtcaatatt ctaagttctg gaatgagttt 1560

ggtaaatcca ttaaacttgg tatcatcgag gatgccacca acagaaatcg tttggcaaaa 1620

ctgctcagat ttgagagcac caagtctgag ggtaaattga catctctgga tcagtacata 1680

tctagaatga aaactgggca gaaggatatc ttttacataa ctggaaccag caaagaacaa 1740

ctggaaaact ctcccttcct tgagcggctt aagaagaaaa attttgaggt tattttcttc 1800

acggatccag ttgatgagta cttgatgcaa tacctgatgg attatgaaga caagaagttc 1860

caaaatgtgt ccaaggaggg tttgaaactt gggaaagatt caaaagacaa ggaactgaag 1920

gaatctttta aggatcttac caagtggtgg aaaactgctc tttccaaaga caatgttgat 1980

gatgtgaaga tttccaaccg attagataac actccttgtg tagtggtgac atcaaaattt 2040

ggttggagtg ctaacatgga gagaatcatg cagtctcaaa ctttgtcaga tgctagcaag 2100

caagcataca tgcgcggcaa gagggttctt gagatcaatc ctaggcaccc tattatcaag 2160

gagctccgag agagagtagt aaagaaccct gaggatgagg gtgtgaagca tacagcacag     2220

ctgatgtacc agactgcact ctttgaaagc ggcttcctgc ttgatgatcc caaggatttt     2280

gcttcccgga tttatgattc agtgaagact agcctggaca tcagtcctga agcaacagtt     2340

gaggaggaag atgacactga agtagaggct gaaagtgacg cgaaagaaga ttcaaagcct     2400

gaaactgatg ctgttaacga tgataatgat gtcaaggacg agttgtag                  2448

<210>  70
<211>  815
<212>  PRT
<213>  Glycine max

<400>  70

Met Arg Lys Trp Thr Val Ala Ser Ala Leu Leu Leu Leu Ser Leu Leu
1               5                   10                  15

Phe Leu Phe Ala Asp Gln Gly Arg Lys Phe Gln Ala Asn Ala Glu Gly
            20                  25                  30

Asp Ser Asp Glu Leu Val Asp Pro Pro Lys Val Glu Asp Lys Ile Gly
        35                  40                  45

Ala Val Pro His Gly Leu Ser Thr Asp Ser Asp Val Val Lys Arg Glu
    50                  55                  60

Ala Glu Ser Ile Ser Lys Arg Ser Leu Arg Ser Asn Ala Glu Lys Phe
65                  70                  75                  80

Glu Phe Gln Ala Glu Val Ser Arg Leu Met Asp Ile Ile Ile Asn Ser
                85                  90                  95

Leu Tyr Ser Asn Lys Asp Ile Phe Leu Arg Glu Leu Ile Ser Asn Ala
            100                 105                 110

Ser Asp Ala Leu Asp Lys Ile Arg Phe Leu Ser Leu Thr Asp Lys Glu
        115                 120                 125

Val Leu Gly Glu Gly Asp Asn Thr Lys Leu Asp Ile Gln Ile Lys Leu
    130                 135                 140

Asp Lys Glu Lys Lys Ile Leu Ser Ile Arg Asp Arg Gly Ile Gly Met
145                 150                 155                 160

Thr Lys Glu Asp Leu Ile Lys Asn Leu Gly Thr Ile Ala Lys Ser Gly
                165                 170                 175

Thr Ser Ala Phe Val Glu Lys Met Gln Thr Ser Gly Asp Leu Asn Leu

180                 185                 190

Ile Gly Gln Phe Gly Val Gly Phe Tyr Ser Val Tyr Leu Val Ala Asp
        195                 200                 205

Tyr Val Glu Val Ile Ser Lys Asn Asn Asp Asp Lys Gln Tyr Val Trp
    210                 215                 220

Glu Ser Lys Ala Asp Gly Ala Phe Ala Ile Ser Glu Asp Thr Trp Asn
225                 230                 235                 240

Glu Pro Leu Gly Arg Gly Thr Glu Ile Arg Leu His Leu Lys Glu Glu
                245                 250                 255

Ala Gly Glu Tyr Leu Glu Glu Ser Lys Leu Lys Glu Leu Val Lys Arg
            260                 265                 270

Tyr Ser Glu Phe Ile Asn Phe Pro Ile Tyr Ile Trp Ala Ser Lys Glu
        275                 280                 285

Val Asp Val Glu Val Pro Ala Asp Glu Asp Asp Ser Ser Asp Glu Glu
    290                 295                 300

Asp Ser Ser Glu Ser Ser Ser Lys Glu Glu Ser Glu Asp Glu Asp Ala
305                 310                 315                 320

Asp Lys Ser Glu Asp Glu Glu Lys Lys Pro Lys Thr Lys Thr Val Lys
                325                 330                 335

Glu Thr Thr Tyr Glu Trp Glu Leu Leu Asn Asp Val Lys Ala Ile Trp
            340                 345                 350

Leu Arg Asn Pro Lys Glu Val Thr Glu Glu Glu Tyr Thr Lys Phe Tyr
        355                 360                 365

His Ser Leu Ala Lys Asp Phe Ser Asp Glu Lys Pro Leu Ala Trp Ser
    370                 375                 380

His Phe Thr Ala Glu Gly Asp Val Glu Phe Lys Ala Val Leu Phe Val
385                 390                 395                 400

Pro Pro Lys Ala Pro Gln Asp Leu Tyr Glu Ser Tyr Tyr Asn Ala Asn
                405                 410                 415

Lys Ser Asn Leu Lys Leu Tyr Val Arg Arg Val Phe Ile Ser Asp Glu
            420                 425                 430

Phe Asn Glu Leu Leu Pro Lys Tyr Leu Asn Phe Leu Leu Gly Leu Val
        435                 440                 445

Asp Ser Asp Thr Leu Pro Leu Asn Val Ser Arg Glu Met Leu Gln Gln
    450                 455                 460

His Ser Ser Leu Lys Thr Ile Lys Lys Lys Leu Ile Arg Lys Ala Leu
465                 470                 475                 480

Asp Met Ile Arg Arg Ile Ala Asp Glu Asp Pro Asp Glu Ser Thr Asp
                485                 490                 495

Lys Glu Lys Lys Asp Ala Ser Ser Asp Asn Asp Glu Lys Lys Gly Gln
            500                 505                 510

Tyr Ser Lys Phe Trp Asn Glu Phe Gly Lys Ser Ile Lys Leu Gly Ile
        515                 520                 525

Ile Glu Asp Ala Thr Asn Arg Asn Arg Leu Ala Lys Leu Leu Arg Phe
    530                 535                 540

Glu Ser Thr Lys Ser Glu Gly Lys Leu Thr Ser Leu Asp Gln Tyr Ile
545                 550                 555                 560

Ser Arg Met Lys Thr Gly Gln Lys Asp Ile Phe Tyr Ile Thr Gly Thr
                565                 570                 575

Ser Lys Glu Gln Leu Glu Asn Ser Pro Phe Leu Glu Arg Leu Lys Lys
            580                 585                 590

Lys Asn Phe Glu Val Ile Phe Phe Thr Asp Pro Val Asp Glu Tyr Leu
        595                 600                 605

Met Gln Tyr Leu Met Asp Tyr Glu Asp Lys Lys Phe Gln Asn Val Ser
    610                 615                 620

Lys Glu Gly Leu Lys Leu Gly Lys Asp Ser Lys Asp Lys Glu Leu Lys
625                 630                 635                 640

Glu Ser Phe Lys Asp Leu Thr Lys Trp Trp Lys Thr Ala Leu Ser Lys
                645                 650                 655

Asp Asn Val Asp Asp Val Lys Ile Ser Asn Arg Leu Asp Asn Thr Pro
            660                 665                 670

Cys Val Val Val Thr Ser Lys Phe Gly Trp Ser Ala Asn Met Glu Arg
        675                 680                 685

Ile Met Gln Ser Gln Thr Leu Ser Asp Ala Ser Lys Gln Ala Tyr Met
    690                 695                 700

Arg Gly Lys Arg Val Leu Glu Ile Asn Pro Arg His Pro Ile Ile Lys
705                 710                 715                 720

Glu Leu Arg Glu Arg Val Val Lys Asn Pro Glu Asp Glu Gly Val Lys
                725                 730                 735

His Thr Ala Gln Leu Met Tyr Gln Thr Ala Leu Phe Glu Ser Gly Phe
            740                 745                 750

Leu Leu Asp Asp Pro Lys Asp Phe Ala Ser Arg Ile Tyr Asp Ser Val
        755                 760                 765

Lys Thr Ser Leu Asp Ile Ser Pro Glu Ala Thr Val Glu Glu Glu Asp
    770                 775                 780

Asp Thr Glu Val Glu Ala Glu Ser Asp Ala Lys Glu Asp Ser Lys Pro
785                 790                 795                 800

Glu Thr Asp Ala Val Asn Asp Asp Asn Asp Val Lys Asp Glu Leu
                805                 810                 815

<210>  71
<211>  14
<212>  PRT
<213>  Glycine max

<400>  71

Ala Pro Gln Asp Leu Tyr Glu Ser Tyr Tyr Asn Ala Asn Lys
1               5                   10

<210>  72
<211>  8
<212>  PRT
<213>  Glycine max

<400>  72

Ala Val Leu Phe Val Pro Pro Lys
1               5

<210>  73
<211>  11
<212>  PRT
<213>  Glycine max

<400>  73

Asp Phe Ala Ser Arg Ile Tyr Asp Ser Val Lys

1               5                   10


<210>  74
<211>  17
<212>  PRT
<213>  Glycine max

<400>  74

Asp Ile Phe Leu Arg Glu Leu Ile Ser Asn Ala Ser Asp Ala Leu Asp
1               5                   10                  15


Lys




<210>  75
<211>  10
<212>  PRT
<213>  Glycine max

<400>  75

Asp Ile Phe Tyr Ile Thr Gly Thr Ser Lys
1               5                   10


<210>  76
<211>  7
<212>  PRT
<213>  Glycine max

<400>  76

Asp Asn Val Asp Asp Val Lys
1               5


<210>  77
<211>  17
<212>  PRT
<213>  Glycine max

<400>  77

Glu Asp Ser Lys Pro Glu Thr Asp Ala Val Asn Asp Asp Asn Asp Val
1               5                   10                  15


Lys




<210>  78
<211>  11
<212>  PRT
<213>  Glycine max

<400>  78

Glu Glu Ala Gly Glu Tyr Leu Glu Glu Ser Lys

1               5                   10


<210>  79
<211>  13
<212>  PRT
<213>  Glycine max

<400>  79

Glu Gln Leu Glu Asn Ser Pro Phe Leu Glu Arg Leu Lys
1               5                   10


<210>  80
<211>  10
<212>  PRT
<213>  Glycine max

<400>  80

Glu Val Leu Gly Glu Gly Asp Asn Thr Lys
1               5                   10


<210>  81
<211>  9
<212>  PRT
<213>  Glycine max

<400>  81

Glu Val Thr Glu Glu Glu Tyr Thr Lys
1               5


<210>  82
<211>  7
<212>  PRT
<213>  Glycine max

<400>  82

Phe Tyr His Ser Leu Ala Lys
1               5


<210>  83
<211>  16
<212>  PRT
<213>  Glycine max

<400>  83

Ile Gly Ala Val Pro His Gly Leu Ser Thr Asp Ser Asp Val Val Lys
1               5                   10                  15


<210>  84
<211>  15
<212>  PRT
<213>  Glycine max

<400>  84

Leu Gly Ile Ile Glu Asp Ala Thr Asn Arg Asn Arg Leu Ala Lys
1               5                   10                  15


<210>  85
<211>  8
<212>  PRT
<213>  Glycine max

<400>  85

Asn Pro Glu Asp Glu Gly Val Lys
1               5


<210>  86
<211>  9
<212>  PRT
<213>  Glycine max

<400>  86

Ser Gly Thr Ser Ala Phe Val Glu Lys
1               5


<210>  87
<211>  26
<212>  PRT
<213>  Glycine max

<400>  87

Thr Ser Leu Asp Ile Ser Pro Glu Ala Thr Val Glu Glu Glu Asp Asp
1               5                   10                  15


Thr Glu Val Glu Ala Glu Ser Asp Ala Lys
            20                  25


<210>  88
<211>  2442
<212>  DNA
<213>  Glycine max

<400>  88
atgaggaagt ggacggttgc ttccgctctc cttcttctct ctctcctctt tctctttgca      60

gatcaaggtc gaaagtttca ggcgaatgcc gagggagatt ccgacgagct tgtagatcct     120

ccgaaggtgg aggacaagat cggtgctgtg cctcatggcc tttccaccga ttcagatgtc     180

gtcaagaggg agtcggagtc gatctcgaag agatctctcc ggagcaatgc ggagaagttt     240

gagttccaag cggaagtgtc acggcttatg gatattatca tcaattctct ctacagtaac     300

aaggatatct tcctcagaga gttgatttcc aatgcttctg atgctttgga caagattagg     360

ttcctttccc tcacggataa ggacgttttg ggagaaggtg ataacaccaa gcttgatatt     420

cagattaagt tggacaagga aaagaaaatt ctctcaattc gagacagagg tattggtatg 480

acgaaggagg atttgataaa gaatttgggg acaatagcaa aatctggaac ttctgcattt 540

gttgagaaga tgcaaacaag tggagatctc aatctgattg ggcagtttgg agtcggcttc 600

tactctgttt atctcgtggc tgactatgtc gaagtcatta gcaagaataa tgatgataaa 660

cagtatgtat gggaatcaaa agctgatgga gcatttgcaa tttcagaaga tacatggaat 720

gaacctctag ggcgtggaac tgagattaga ttacacctca aggaagaggc tggggagtac 780

ttgcaggagt ctaaactgaa agaattggtg aagagatact ctgaatttat taacttccct 840

atctatattt gggcaagcaa agaggttgat gtggaggttc ccgctgatga agatgattcc 900

agtgatgaag aggattcatc tgaaagcagc tccaaggagg aaagcgaaga tgaagatgct 960

gataaaagtg aagacgaaga aaaaaagccc aagacaaaga cggtgaagga aacaacttat 1020

gaatgggaac ttttaaatga tgtgaaagct atatggctga ggaatccaaa ggaggttaca 1080

gaagaagaat acaccaaatt ctaccactct cttgccaagg atttcagtga tgagaaacct 1140

ttagcatgga gtcatttcac tgctgagggc gatgtcgagt tcaaggcagt cctgtttgta 1200

ccacctaagg cacctcaaga tttatacgag agctattata acgcaaacaa atccaacctg 1260

aagttgtatg ttagacgtgt gttcatttca gatgaattta atgaactgtt gcctaagtac 1320

ctaaactttt tgctgggtct tgttgattct gacacattgc cacttaatgt atcgcgagaa 1380

atgcttcagc aacatagtag tctaaagaca atcaagaaga aacttattag gaaagccctt 1440

gatatgatcc gtaggattgc tgatgaggat cctgatgagt ccactgacaa agaaaagaaa 1500

gatacgtcat ctgacaataa tgagaagaaa ggtcaatatt ccaagttctg gaatgagttt 1560

ggtaaatcca ttaaacttgg tatcatcgag gatgccacca acagaaatcg tttggcaaaa 1620

ctgctcagat ttgagagcac caagtctgag ggtaaattga catctctgga tcagtacata 1680

tctagaatga aagctgggca gaaggatatc ttttacataa ctggaaccag caaagaacaa 1740

ctggaaaact ctccattcct tgagcggctt aagaagaaaa attttgaggt tattttcttc 1800

acggatccag ttgatgaata cttgatgcaa tacctgatgg attatgaaga caagaagttc 1860

caaaatgtgt ccaaggaggg tttgaaactc gggaaagatt caaaagacaa ggaactgaag 1920

gaatccttca aggaccttac caagtggtgg aaaactgctc tttccaaaga caatgttgat 1980

gatgtgaaga tttccaaccg attggacaac actccttgtg tagtggtaac atcaaaattt 2040

ggttggagtg ctaacatgga gagaatcatg cagtctcaaa ctttgtctga tgctagcaag 2100

caagcataca tgcgcggcaa gagggttctt gaggtcaatc ctaggcaccc tattatcaag 2160

gagctccgag agagagtagt aaagaaccct gaggatgagg gtgtgaaaca tacagcacag 2220

ttgatgtacc agaccgcact cttcgaaagt ggcttcctgc ttgatgatcc caaggatttt 2280

gcttcccgta tttatgattc agttaagacc agcttggaca tcagtcctga agcaacagtt 2340

gaggaggaag atgacaccga agtagaggct gaaagtgatt caaagcctga agctgatgcg     2400

gttcatgatg gtgatgttga tgatgccaag gacgagttgt ag                         2442

<210>  89
<211>  813
<212>  PRT
<213>  Glycine max

<400>  89

Met Arg Lys Trp Thr Val Ala Ser Ala Leu Leu Leu Leu Ser Leu Leu
1               5                   10                  15

Phe Leu Phe Ala Asp Gln Gly Arg Lys Phe Gln Ala Asn Ala Glu Gly
            20                  25                  30

Asp Ser Asp Glu Leu Val Asp Pro Pro Lys Val Glu Asp Lys Ile Gly
        35                  40                  45

Ala Val Pro His Gly Leu Ser Thr Asp Ser Asp Val Val Lys Arg Glu
    50                  55                  60

Ser Glu Ser Ile Ser Lys Arg Ser Leu Arg Ser Asn Ala Glu Lys Phe
65                  70                  75                  80

Glu Phe Gln Ala Glu Val Ser Arg Leu Met Asp Ile Ile Ile Asn Ser
                85                  90                  95

Leu Tyr Ser Asn Lys Asp Ile Phe Leu Arg Glu Leu Ile Ser Asn Ala
            100                 105                 110

Ser Asp Ala Leu Asp Lys Ile Arg Phe Leu Ser Leu Thr Asp Lys Asp
        115                 120                 125

Val Leu Gly Glu Gly Asp Asn Thr Lys Leu Asp Ile Gln Ile Lys Leu
    130                 135                 140

Asp Lys Glu Lys Lys Ile Leu Ser Ile Arg Asp Arg Gly Ile Gly Met
145                 150                 155                 160

Thr Lys Glu Asp Leu Ile Lys Asn Leu Gly Thr Ile Ala Lys Ser Gly
                165                 170                 175

Thr Ser Ala Phe Val Glu Lys Met Gln Thr Ser Gly Asp Leu Asn Leu
            180                 185                 190

Ile Gly Gln Phe Gly Val Gly Phe Tyr Ser Val Tyr Leu Val Ala Asp
        195                 200                 205

Tyr Val Glu Val Ile Ser Lys Asn Asn Asp Asp Lys Gln Tyr Val Trp
    210                 215                 220

Glu Ser Lys Ala Asp Gly Ala Phe Ala Ile Ser Glu Asp Thr Trp Asn
225                 230                 235                 240

Glu Pro Leu Gly Arg Gly Thr Glu Ile Arg Leu His Leu Lys Glu Glu
                245                 250                 255

Ala Gly Glu Tyr Leu Gln Glu Ser Lys Leu Lys Glu Leu Val Lys Arg
            260                 265                 270

Tyr Ser Glu Phe Ile Asn Phe Pro Ile Tyr Ile Trp Ala Ser Lys Glu
        275                 280                 285

Val Asp Val Glu Val Pro Ala Asp Glu Asp Asp Ser Ser Asp Glu Glu
    290                 295                 300

Asp Ser Ser Glu Ser Ser Ser Lys Glu Glu Ser Glu Asp Glu Asp Ala
305                 310                 315                 320

Asp Lys Ser Glu Asp Glu Glu Lys Lys Pro Lys Thr Lys Thr Val Lys
                325                 330                 335

Glu Thr Thr Tyr Glu Trp Glu Leu Leu Asn Asp Val Lys Ala Ile Trp
            340                 345                 350

Leu Arg Asn Pro Lys Glu Val Thr Glu Glu Glu Tyr Thr Lys Phe Tyr
        355                 360                 365

His Ser Leu Ala Lys Asp Phe Ser Asp Glu Lys Pro Leu Ala Trp Ser
    370                 375                 380

His Phe Thr Ala Glu Gly Asp Val Glu Phe Lys Ala Val Leu Phe Val
385                 390                 395                 400

Pro Pro Lys Ala Pro Gln Asp Leu Tyr Glu Ser Tyr Tyr Asn Ala Asn
                405                 410                 415

Lys Ser Asn Leu Lys Leu Tyr Val Arg Arg Val Phe Ile Ser Asp Glu
            420                 425                 430

Phe Asn Glu Leu Leu Pro Lys Tyr Leu Asn Phe Leu Leu Gly Leu Val
        435                 440                 445

Asp Ser Asp Thr Leu Pro Leu Asn Val Ser Arg Glu Met Leu Gln Gln

450                 455                 460

His Ser Ser Leu Lys Thr Ile Lys Lys Lys Leu Ile Arg Lys Ala Leu
465                 470                 475                 480

Asp Met Ile Arg Arg Ile Ala Asp Glu Asp Pro Asp Glu Ser Thr Asp
                485                 490                 495

Lys Glu Lys Lys Asp Thr Ser Ser Asp Asn Asn Glu Lys Lys Gly Gln
            500                 505                 510

Tyr Ser Lys Phe Trp Asn Glu Phe Gly Lys Ser Ile Lys Leu Gly Ile
        515                 520                 525

Ile Glu Asp Ala Thr Asn Arg Asn Arg Leu Ala Lys Leu Leu Arg Phe
    530                 535                 540

Glu Ser Thr Lys Ser Glu Gly Lys Leu Thr Ser Leu Asp Gln Tyr Ile
545                 550                 555                 560

Ser Arg Met Lys Ala Gly Gln Lys Asp Ile Phe Tyr Ile Thr Gly Thr
                565                 570                 575

Ser Lys Glu Gln Leu Glu Asn Ser Pro Phe Leu Glu Arg Leu Lys Lys
            580                 585                 590

Lys Asn Phe Glu Val Ile Phe Phe Thr Asp Pro Val Asp Glu Tyr Leu
        595                 600                 605

Met Gln Tyr Leu Met Asp Tyr Glu Asp Lys Lys Phe Gln Asn Val Ser
    610                 615                 620

Lys Glu Gly Leu Lys Leu Gly Lys Asp Ser Lys Asp Lys Glu Leu Lys
625                 630                 635                 640

Glu Ser Phe Lys Asp Leu Thr Lys Trp Trp Lys Thr Ala Leu Ser Lys
                645                 650                 655

Asp Asn Val Asp Asp Val Lys Ile Ser Asn Arg Leu Asp Asn Thr Pro
            660                 665                 670

Cys Val Val Val Thr Ser Lys Phe Gly Trp Ser Ala Asn Met Glu Arg
        675                 680                 685

Ile Met Gln Ser Gln Thr Leu Ser Asp Ala Ser Lys Gln Ala Tyr Met
    690                 695                 700

Arg Gly Lys Arg Val Leu Glu Val Asn Pro Arg His Pro Ile Ile Lys
705                 710                 715                 720

Glu Leu Arg Glu Arg Val Val Lys Asn Pro Glu Asp Glu Gly Val Lys
                725                 730                 735

His Thr Ala Gln Leu Met Tyr Gln Thr Ala Leu Phe Glu Ser Gly Phe
            740                 745                 750

Leu Leu Asp Asp Pro Lys Asp Phe Ala Ser Arg Ile Tyr Asp Ser Val
        755                 760                 765

Lys Thr Ser Leu Asp Ile Ser Pro Glu Ala Thr Val Glu Glu Glu Asp
    770                 775                 780

Asp Thr Glu Val Glu Ala Glu Ser Asp Ser Lys Pro Glu Ala Asp Ala
785                 790                 795                 800

Val His Asp Gly Asp Val Asp Asp Ala Lys Asp Glu Leu
                805                 810

<210>  90
<211>  14
<212>  PRT
<213>  Glycine max

<400>  90

Ala Pro Gln Asp Leu Tyr Glu Ser Tyr Tyr Asn Ala Asn Lys
1               5                   10

<210>  91
<211>  8
<212>  PRT
<213>  Glycine max

<400>  91

Ala Val Leu Phe Val Pro Pro Lys
1               5

<210>  92
<211>  11
<212>  PRT
<213>  Glycine max

<400>  92

Asp Phe Ala Ser Arg Ile Tyr Asp Ser Val Lys
1               5                   10

<210>  93
<211>  17

<212>  PRT
<213>  Glycine max

<400>  93

Asp Ile Phe Leu Arg Glu Leu Ile Ser Asn Ala Ser Asp Ala Leu Asp
1               5                   10                  15

Lys

<210>  94
<211>  10
<212>  PRT
<213>  Glycine max

<400>  94

Asp Ile Phe Tyr Ile Thr Gly Thr Ser Lys
1               5                   10

<210>  95
<211>  7
<212>  PRT
<213>  Glycine max

<400>  95

Asp Asn Val Asp Asp Val Lys
1               5

<210>  96
<211>  10
<212>  PRT
<213>  Glycine max

<400>  96

Asp Val Leu Gly Glu Gly Asp Asn Thr Lys
1               5                   10

<210>  97
<211>  11
<212>  PRT
<213>  Glycine max

<400>  97

Glu Glu Ala Gly Glu Tyr Leu Gln Glu Ser Lys
1               5                   10

<210>  98
<211>  13
<212>  PRT
<213>  Glycine max

<400>  98

Glu Gln Leu Glu Asn Ser Pro Phe Leu Glu Arg Leu Lys
1               5                   10

<210>  99
<211>  9
<212>  PRT
<213>  Glycine max

<400>  99

Glu Val Thr Glu Glu Glu Tyr Thr Lys
1               5

<210>  100
<211>  7
<212>  PRT
<213>  Glycine max

<400>  100

Phe Tyr His Ser Leu Ala Lys
1               5

<210>  101
<211>  16
<212>  PRT
<213>  Glycine max

<400>  101

Ile Gly Ala Val Pro His Gly Leu Ser Thr Asp Ser Asp Val Val Lys
1               5                   10                  15

<210>  102
<211>  15
<212>  PRT
<213>  Glycine max

<400>  102

Leu Gly Ile Ile Glu Asp Ala Thr Asn Arg Asn Arg Leu Ala Lys
1               5                   10                  15

<210>  103
<211>  8
<212>  PRT
<213>  Glycine max

<400>  103

Asn Pro Glu Asp Glu Gly Val Lys
1               5

<210>  104
<211>  9
<212>  PRT

<213>  Glycine max

<400>  104

Ser Gly Thr Ser Ala Phe Val Glu Lys
1               5


<210>  105
<211>  2103
<212>  DNA
<213>  Glycine max

<400>  105
atggcttcgg agactgagac gttcgctttt caggccgaga tcaaccagct cctcagtcta      60

ataatcaaca ccttctacag caacaaggag atctttcttc gtgagctcat cagcaatgcc     120

tctgacgctt tggacaagat tcgatttgag agtcttacag acaagagcaa gcttgatgct     180

cagccagagt tgttcattca cattattcct gacaagacca acaacacatt gtctatcatt     240

gacagtggta ttggcatgac taaggctgat ttggtgaata acctcggtac tattgcaagg     300

tctgggacca aggaattcat ggaagccctg gctgctggtg ctgacgtgag catgattggg     360

cagttcggtg ttggtttcta ctcggcttat cttgtagctg ataaggttat tgttaccaca     420

aagcacaatg atgatgaaca gtatgtctgg gagtcccatg ccggtggctc atttactgtg     480

acaagagata catctggtga gaaccttggt aggggaacaa agataacttt gtttcttaag     540

gaagatcaac ttgagtatct tgaggaacgc cgtctgaagg atttgataaa gaagcattct     600

gaattcatta gctatccaat ttctctctgg attgagaaga ctactgaaaa ggagatttct     660

gatgatgagg atgaggaaga gaagaaggat gaggagggta aagttgaaga tgtggatgaa     720

gataaggaga aggaggagaa gaaaaagaag accattaagg aggtatctca cgagtggtca     780

ttggtgaaca agcagaagcc tatttggatg aggaaacctg aagagattac gaaagaagaa     840

tatgctgctt tctacaagag tcttaccaat gattgggaag agcatttggc tgttaagcac     900

ttttctgttg agggtcagct ggagtttaag gctgtcctct ttatccccaa gagggcacct     960

tttgatctct ttgacaccag gaagaagcct aacaacatca aactgtatgt ccgccgtgtc    1020

ttcatcatgg acaactgtga ggagttgatg cctgaatatc ttagctttgt taagggtatt    1080

gtggattctg aggatcttcc tctcaacatt tctagagaaa tgctgcagca gaacaagatc    1140

ctgaaagtca tcaggaagaa cttggtcaag aagtgcattg agatgttctt tgaaattgct    1200

gaaaacaagg aagactataa caagttttat gaagccttct ctaagaacct gaaacttggt    1260

attcatgagg attctcagaa caagacaaag ctagctgaat tgctcaggta tcactccact    1320

aagagtggtg atgagatgac cagcctcaag gactatgtta ccaggatgaa ggaaggacag    1380

aatgacatct actacattac tggtgaaagc aagaaagctg tcgagaattc cccctttctt    1440

gaaaagctta agaagaaggg atacgaggtt ctctacatgg ttgatgctat tgatgagtat    1500

gctgttggcc agcttaagga atttgaggga aagaagttgg tctctgctac caaggaaggc 1560

ctaaaacttg atgagagcga agacgagaag aaaaagaagg aagaactaaa ggataaattt 1620

gagggtcttt gccatgtgat caaggatgtg ttgggtgaca aggtggagaa agttgtggtg 1680

tctgatcgtg ttgtggattc tccttgctgt ctggtgacag gtgaatatgg gtggacagcc 1740

aacatggaaa ggattatgaa agcacaggca ttgagggaca gcagcatggc tggatacatg 1800

tcaagcaaga agacgatgga gattaaccct gagaacccca tcatggagga gctgaggaag 1860

cgagcagatg ctgataagaa tgacaagtct gtgaaggatc tcgtgctctt gctctttgag 1920

actgctcttc taacttctgg gttcagcctt gatgatccca acactttcgg taacaggatt 1980

cacaggatgc tgaagcttgg actgagcatt gatgaagatg ctggtgaagc agatgctgac 2040

atgcctcctc ttgaggacgc tgatgcagat gctgagggta gcaagatgga agaagttgat 2100

taa 2103

<210> 106
<211> 700
<212> PRT
<213> Glycine max

<400> 106

Met Ala Ser Glu Thr Glu Thr Phe Ala Phe Gln Ala Glu Ile Asn Gln
1               5                   10                  15

Leu Leu Ser Leu Ile Ile Asn Thr Phe Tyr Ser Asn Lys Glu Ile Phe
            20                  25                  30

Leu Arg Glu Leu Ile Ser Asn Ala Ser Asp Ala Leu Asp Lys Ile Arg
        35                  40                  45

Phe Glu Ser Leu Thr Asp Lys Ser Lys Leu Asp Ala Gln Pro Glu Leu
    50                  55                  60

Phe Ile His Ile Ile Pro Asp Lys Thr Asn Asn Thr Leu Ser Ile Ile
65                  70                  75                  80

Asp Ser Gly Ile Gly Met Thr Lys Ala Asp Leu Val Asn Asn Leu Gly
                85                  90                  95

Thr Ile Ala Arg Ser Gly Thr Lys Glu Phe Met Glu Ala Leu Ala Ala
            100                 105                 110

Gly Ala Asp Val Ser Met Ile Gly Gln Phe Gly Val Gly Phe Tyr Ser
        115                 120                 125

Ala Tyr Leu Val Ala Asp Lys Val Ile Val Thr Thr Lys His Asn Asp
    130                 135                 140

Asp Glu Gln Tyr Val Trp Glu Ser His Ala Gly Gly Ser Phe Thr Val
145                 150                 155                 160

Thr Arg Asp Thr Ser Gly Glu Asn Leu Gly Arg Gly Thr Lys Ile Thr
                165                 170                 175

Leu Phe Leu Lys Glu Asp Gln Leu Glu Tyr Leu Glu Glu Arg Arg Leu
            180                 185                 190

Lys Asp Leu Ile Lys Lys His Ser Glu Phe Ile Ser Tyr Pro Ile Ser
        195                 200                 205

Leu Trp Ile Glu Lys Thr Thr Glu Lys Glu Ile Ser Asp Asp Glu Asp
    210                 215                 220

Glu Glu Glu Lys Lys Asp Glu Glu Gly Lys Val Glu Asp Val Asp Glu
225                 230                 235                 240

Asp Lys Glu Lys Glu Glu Lys Lys Lys Lys Thr Ile Lys Glu Val Ser
                245                 250                 255

His Glu Trp Ser Leu Val Asn Lys Gln Lys Pro Ile Trp Met Arg Lys
            260                 265                 270

Pro Glu Glu Ile Thr Lys Glu Glu Tyr Ala Ala Phe Tyr Lys Ser Leu
        275                 280                 285

Thr Asn Asp Trp Glu Glu His Leu Ala Val Lys His Phe Ser Val Glu
    290                 295                 300

Gly Gln Leu Glu Phe Lys Ala Val Leu Phe Ile Pro Lys Arg Ala Pro
305                 310                 315                 320

Phe Asp Leu Phe Asp Thr Arg Lys Lys Pro Asn Asn Ile Lys Leu Tyr
                325                 330                 335

Val Arg Arg Val Phe Ile Met Asp Asn Cys Glu Glu Leu Met Pro Glu
            340                 345                 350

Tyr Leu Ser Phe Val Lys Gly Ile Val Asp Ser Glu Asp Leu Pro Leu
        355                 360                 365

Asn Ile Ser Arg Glu Met Leu Gln Gln Asn Lys Ile Leu Lys Val Ile
    370                 375                 380

Arg Lys Asn Leu Val Lys Lys Cys Ile Glu Met Phe Phe Glu Ile Ala
385                 390                 395                 400

Glu Asn Lys Glu Asp Tyr Asn Lys Phe Tyr Glu Ala Phe Ser Lys Asn
                405                 410                 415

Leu Lys Leu Gly Ile His Glu Asp Ser Gln Asn Lys Thr Lys Leu Ala
            420                 425                 430

Glu Leu Leu Arg Tyr His Ser Thr Lys Ser Gly Asp Glu Met Thr Ser
        435                 440                 445

Leu Lys Asp Tyr Val Thr Arg Met Lys Glu Gly Gln Asn Asp Ile Tyr
    450                 455                 460

Tyr Ile Thr Gly Glu Ser Lys Lys Ala Val Glu Asn Ser Pro Phe Leu
465                 470                 475                 480

Glu Lys Leu Lys Lys Lys Gly Tyr Glu Val Leu Tyr Met Val Asp Ala
                485                 490                 495

Ile Asp Glu Tyr Ala Val Gly Gln Leu Lys Glu Phe Glu Gly Lys Lys
            500                 505                 510

Leu Val Ser Ala Thr Lys Glu Gly Leu Lys Leu Asp Glu Ser Glu Asp
        515                 520                 525

Glu Lys Lys Lys Lys Glu Glu Leu Lys Asp Lys Phe Glu Gly Leu Cys
    530                 535                 540

His Val Ile Lys Asp Val Leu Gly Asp Lys Val Glu Lys Val Val Val
545                 550                 555                 560

Ser Asp Arg Val Val Asp Ser Pro Cys Cys Leu Val Thr Gly Glu Tyr
                565                 570                 575

Gly Trp Thr Ala Asn Met Glu Arg Ile Met Lys Ala Gln Ala Leu Arg
            580                 585                 590

Asp Ser Ser Met Ala Gly Tyr Met Ser Ser Lys Lys Thr Met Glu Ile
        595                 600                 605

Asn Pro Glu Asn Pro Ile Met Glu Glu Leu Arg Lys Arg Ala Asp Ala
    610                 615                 620

Asp Lys Asn Asp Lys Ser Val Lys Asp Leu Val Leu Leu Leu Phe Glu
625                 630                 635                 640

Thr Ala Leu Leu Thr Ser Gly Phe Ser Leu Asp Asp Pro Asn Thr Phe
                645                 650                 655

Gly Asn Arg Ile His Arg Met Leu Lys Leu Gly Leu Ser Ile Asp Glu
            660                 665                 670

Asp Ala Gly Glu Ala Asp Ala Asp Met Pro Pro Leu Glu Asp Ala Asp
        675                 680                 685

Ala Asp Ala Glu Gly Ser Lys Met Glu Glu Val Asp
    690                 695                 700

<210>  107
<211>  16
<212>  PRT
<213>  Glycine max

<400>  107

Ala Asp Leu Val Asn Asn Leu Gly Thr Ile Ala Arg Ser Gly Thr Lys
1               5                   10                  15

<210>  108
<211>  10
<212>  PRT
<213>  Glycine max

<400>  108

Ala Val Glu Asn Ser Pro Phe Leu Glu Lys
1               5                   10

<210>  109
<211>  13
<212>  PRT
<213>  Glycine max

<400>  109

Glu Asp Gln Leu Glu Tyr Leu Glu Glu Arg Arg Leu Lys
1               5                   10

<210>  110
<211>  8
<212>  PRT
<213>  Glycine max

<400>  110

Glu Glu Tyr Ala Ala Phe Tyr Lys
1               5

<210>  111

<211>  14
<212>  PRT
<213>  Glycine max

<400>  111

Glu Gly Gln Asn Asp Ile Tyr Tyr Ile Thr Gly Glu Ser Lys
1               5                   10


<210>  112
<211>  17
<212>  PRT
<213>  Glycine max

<400>  112

Glu Ile Phe Leu Arg Glu Leu Ile Ser Asn Ala Ser Asp Ala Leu Asp
1               5                   10                  15


Lys



<210>  113
<211>  7
<212>  PRT
<213>  Glycine max

<400>  113

Phe Tyr Glu Ala Phe Ser Lys
1               5


<210>  114
<211>  11
<212>  PRT
<213>  Glycine max

<400>  114

His Phe Ser Val Glu Gly Gln Leu Glu Phe Lys
1               5                   10


<210>  115
<211>  9
<212>  PRT
<213>  Glycine max

<400>  115

Ile Arg Phe Glu Ser Leu Thr Asp Lys
1               5


<210>  116
<211>  11
<212>  PRT
<213>  Glycine max

<400>  116

Leu Ala Glu Leu Leu Arg Tyr His Ser Thr Lys
1               5                   10


<210>  117
<211>  8
<212>  PRT
<213>  Glycine max

<400>  117

Leu Asp Glu Ser Glu Asp Glu Lys
1               5


<210>  118
<211>  10
<212>  PRT
<213>  Glycine max

<400>  118

Leu Gly Ile His Glu Asp Ser Gln Asn Lys
1               5                   10


<210>  119
<211>  11
<212>  PRT
<213>  Glycine max

<400>  119

Arg Ala Pro Phe Asp Leu Phe Asp Thr Arg Lys
1               5                   10


<210>  120
<211>  8
<212>  PRT
<213>  Glycine max

<400>  120

Val Glu Asp Val Asp Glu Asp Lys
1               5


<210>  121
<211>  2109
<212>  DNA
<213>  Glycine max

<400>  121
atggcggaga cagagacgtt cgcattccaa gcagagatca accagctcct cagtctcatc      60

atcaacacct tctactccaa caaggagatt ttccttcgtg aactcatcag caacgcctcc     120

gatgctttgg acaagattcg atttgagagt ttgacagata agagcaagct cgatgctcag     180

ccagagttgt tcattcatat tattccagac aagactaaca atacactgac cattgttgac     240

agtggtattg gcatgaccaa agctgatttg gtgaacaact tgggtacaat tgctagatct 300

ggtaccaagg aattcatgga agccttggct gctggtgctg atgttagtat gattggacag 360

tttggtgtgg ggttctactc tgcatatctt gttgctgaga aggtcattgt tacaaccaaa 420

cacaatgatg atgaacagta tgtttgggag tctcaagctg gaggttcttt cactgtcacc 480

agggataaca ctggtgaggt tcttggcagg ggtactaaga tcactctcta cctcaaggaa 540

gatcagcttg agtaccttga ggagcgtcgc ttgaaggatc tgatcaagaa gcattctgag 600

ttcattagct acccaatttc tctttggatt gagaagacta ctgagaagga gatctctgac 660

gatgaggatg aggaagagaa gaaggatgag gagggtaagg tggaggatgt tgacgaagag 720

aaggagaagg aagaaaaaaa gaaaaagaag atcaaggaag tgtcacatga atggtccttg 780

gtgaacaagc agaagcccat ttggatgagg aagcctgagg agatcacaaa agaggagtat 840

tctgctttct acaagagtct taccaatgac tgggaagagc atttggctgt caagcacttc 900

tctgttgagg gtcagttgga gtttaaggct atactctttg ttcccaagag ggcacctttt 960

gacctctttg acacaaggaa gaagcctaac aacattaagc tctatgtccg ccgtgtcttt 1020

attatggaca actgtgagga gttgattcct gagtatttgg gctttgtcaa gggtattgtt 1080

gattctgaag atctcccact caacatttca agagaaatgt tgcaacagaa caagatcttg 1140

aaggtcatcc gtaagaactt ggtcaagaag tgccttgagc tcttctttga aattgcagag 1200

aacaaggagg attacaacaa gttctatgag gctttctcta agaacttaaa gcttggtatc 1260

catgaggatt cccagaacaa gggaaagatt gctgaattgc tcaggtacca ctccaccaag 1320

agtggtgatg agctgacaag cctcaaggat tatgtgacca ggatgaagga aggtcagagt 1380

gacatctact acatcaccgg tgaaagcaag aaggctgttg agaactcccc attcttggag 1440

aagttgaaga agaaaggtta tgaggttctt ttcatggttg atgccattga tgaatatgct 1500

gttggccagc tcaaggaatt tgagggcaag aagcttgtct ctgccaccaa ggagggtctc 1560

aaacttgacg agagtgaaga tgagaagaag aagcaagagg agttgaagga gaagtttgac 1620

aacctatgca aggtgatcaa ggatgttttg ggtgacaagg ttgagaaggt tgtagtctct 1680

gaccgtgttg ttgattcacc atgctgtctg gtgactggag aatacggttg gactgcaaac 1740

atggaaagga tcatgaaagc tcaagcccta agggacaaca gcatggcagg atacatgtca 1800

agcaagaaga caatggagat caaccctgag aacccaatca tggaggagct caggaagcgt 1860

gcggatgctg acaagaacga caaatccgtg aaggaccttg ttctgttgct gttcgagact 1920

gctcttctca cttctggttt cagccttgac gaacccaaca cttttggaaa caggatccac 1980

agaatgttga agcttggact gagcatcgac gaggatgcag ctgaagctga tgctgatgct 2040

gatatgcccc cactggagga ggaggctgaa gctgatgctg agggcagcaa aatggaggag 2100

gttgattaa                                                              2109

<210>  122
<211>  702
<212>  PRT
<213>  Glycine max

<400>  122

Met Ala Glu Thr Glu Thr Phe Ala Phe Gln Ala Glu Ile Asn Gln Leu
1               5                   10                  15

Leu Ser Leu Ile Ile Asn Thr Phe Tyr Ser Asn Lys Glu Ile Phe Leu
            20                  25                  30

Arg Glu Leu Ile Ser Asn Ala Ser Asp Ala Leu Asp Lys Ile Arg Phe
        35                  40                  45

Glu Ser Leu Thr Asp Lys Ser Lys Leu Asp Ala Gln Pro Glu Leu Phe
    50                  55                  60

Ile His Ile Ile Pro Asp Lys Thr Asn Asn Thr Leu Thr Ile Val Asp
65                  70                  75                  80

Ser Gly Ile Gly Met Thr Lys Ala Asp Leu Val Asn Asn Leu Gly Thr
                85                  90                  95

Ile Ala Arg Ser Gly Thr Lys Glu Phe Met Glu Ala Leu Ala Ala Gly
            100                 105                 110

Ala Asp Val Ser Met Ile Gly Gln Phe Gly Val Gly Phe Tyr Ser Ala
        115                 120                 125

Tyr Leu Val Ala Glu Lys Val Ile Val Thr Thr Lys His Asn Asp Asp
    130                 135                 140

Glu Gln Tyr Val Trp Glu Ser Gln Ala Gly Gly Ser Phe Thr Val Thr
145                 150                 155                 160

Arg Asp Asn Thr Gly Glu Val Leu Gly Arg Gly Thr Lys Ile Thr Leu
                165                 170                 175

Tyr Leu Lys Glu Asp Gln Leu Glu Tyr Leu Glu Glu Arg Arg Leu Lys
            180                 185                 190

Asp Leu Ile Lys Lys His Ser Glu Phe Ile Ser Tyr Pro Ile Ser Leu
        195                 200                 205

Trp Ile Glu Lys Thr Thr Glu Lys Glu Ile Ser Asp Asp Glu Asp Glu

210                 215                 220

Glu Glu Lys Lys Asp Glu Glu Gly Lys Val Glu Asp Val Asp Glu Glu
225                 230                 235                 240

Lys Glu Lys Glu Glu Lys Lys Lys Lys Lys Ile Lys Glu Val Ser His
                245                 250                 255

Glu Trp Ser Leu Val Asn Lys Gln Lys Pro Ile Trp Met Arg Lys Pro
            260                 265                 270

Glu Glu Ile Thr Lys Glu Glu Tyr Ser Ala Phe Tyr Lys Ser Leu Thr
        275                 280                 285

Asn Asp Trp Glu Glu His Leu Ala Val Lys His Phe Ser Val Glu Gly
    290                 295                 300

Gln Leu Glu Phe Lys Ala Ile Leu Phe Val Pro Lys Arg Ala Pro Phe
305                 310                 315                 320

Asp Leu Phe Asp Thr Arg Lys Lys Pro Asn Asn Ile Lys Leu Tyr Val
                325                 330                 335

Arg Arg Val Phe Ile Met Asp Asn Cys Glu Glu Leu Ile Pro Glu Tyr
            340                 345                 350

Leu Gly Phe Val Lys Gly Ile Val Asp Ser Glu Asp Leu Pro Leu Asn
        355                 360                 365

Ile Ser Arg Glu Met Leu Gln Gln Asn Lys Ile Leu Lys Val Ile Arg
    370                 375                 380

Lys Asn Leu Val Lys Lys Cys Leu Glu Leu Phe Phe Glu Ile Ala Glu
385                 390                 395                 400

Asn Lys Glu Asp Tyr Asn Lys Phe Tyr Glu Ala Phe Ser Lys Asn Leu
                405                 410                 415

Lys Leu Gly Ile His Glu Asp Ser Gln Asn Lys Gly Lys Ile Ala Glu
            420                 425                 430

Leu Leu Arg Tyr His Ser Thr Lys Ser Gly Asp Glu Leu Thr Ser Leu
        435                 440                 445

Lys Asp Tyr Val Thr Arg Met Lys Glu Gly Gln Ser Asp Ile Tyr Tyr
    450                 455                 460

Ile Thr Gly Glu Ser Lys Lys Ala Val Glu Asn Ser Pro Phe Leu Glu
465                 470                 475                 480

Lys Leu Lys Lys Lys Gly Tyr Glu Val Leu Phe Met Val Asp Ala Ile
                485                 490                 495

Asp Glu Tyr Ala Val Gly Gln Leu Lys Glu Phe Glu Gly Lys Lys Leu
            500                 505                 510

Val Ser Ala Thr Lys Glu Gly Leu Lys Leu Asp Glu Ser Glu Asp Glu
        515                 520                 525

Lys Lys Lys Gln Glu Glu Leu Lys Glu Lys Phe Asp Asn Leu Cys Lys
    530                 535                 540

Val Ile Lys Asp Val Leu Gly Asp Lys Val Glu Lys Val Val Val Ser
545                 550                 555                 560

Asp Arg Val Val Asp Ser Pro Cys Cys Leu Val Thr Gly Glu Tyr Gly
                565                 570                 575

Trp Thr Ala Asn Met Glu Arg Ile Met Lys Ala Gln Ala Leu Arg Asp
            580                 585                 590

Asn Ser Met Ala Gly Tyr Met Ser Ser Lys Lys Thr Met Glu Ile Asn
        595                 600                 605

Pro Glu Asn Pro Ile Met Glu Glu Leu Arg Lys Arg Ala Asp Ala Asp
    610                 615                 620

Lys Asn Asp Lys Ser Val Lys Asp Leu Val Leu Leu Leu Phe Glu Thr
625                 630                 635                 640

Ala Leu Leu Thr Ser Gly Phe Ser Leu Asp Glu Pro Asn Thr Phe Gly
                645                 650                 655

Asn Arg Ile His Arg Met Leu Lys Leu Gly Leu Ser Ile Asp Glu Asp
            660                 665                 670

Ala Ala Glu Ala Asp Ala Asp Ala Asp Met Pro Pro Leu Glu Glu Glu
        675                 680                 685

Ala Glu Ala Asp Ala Glu Gly Ser Lys Met Glu Glu Val Asp
    690                 695                 700

<210>  123
<211>  16
<212>  PRT

<213>  Glycine max

<400>  123

Ala Asp Leu Val Asn Asn Leu Gly Thr Ile Ala Arg Ser Gly Thr Lys
1               5                   10                  15


<210>  124
<211>  10
<212>  PRT
<213>  Glycine max

<400>  124

Ala Val Glu Asn Ser Pro Phe Leu Glu Lys
1               5                   10


<210>  125
<211>  13
<212>  PRT
<213>  Glycine max

<400>  125

Glu Asp Gln Leu Glu Tyr Leu Glu Glu Arg Arg Leu Lys
1               5                   10


<210>  126
<211>  8
<212>  PRT
<213>  Glycine max

<400>  126

Glu Glu Tyr Ser Ala Phe Tyr Lys
1               5


<210>  127
<211>  14
<212>  PRT
<213>  Glycine max

<400>  127

Glu Gly Gln Ser Asp Ile Tyr Tyr Ile Thr Gly Glu Ser Lys
1               5                   10


<210>  128
<211>  17
<212>  PRT
<213>  Glycine max

<400>  128

Glu Ile Phe Leu Arg Glu Leu Ile Ser Asn Ala Ser Asp Ala Leu Asp
1               5                   10                  15

Lys

<210> 129
<211> 7
<212> PRT
<213> Glycine max

<400> 129

Phe Tyr Glu Ala Phe Ser Lys
1               5

<210> 130
<211> 11
<212> PRT
<213> Glycine max

<400> 130

His Phe Ser Val Glu Gly Gln Leu Glu Phe Lys
1               5                   10

<210> 131
<211> 11
<212> PRT
<213> Glycine max

<400> 131

Ile Ala Glu Leu Leu Arg Tyr His Ser Thr Lys
1               5                   10

<210> 132
<211> 9
<212> PRT
<213> Glycine max

<400> 132

Ile Arg Phe Glu Ser Leu Thr Asp Lys
1               5

<210> 133
<211> 8
<212> PRT
<213> Glycine max

<400> 133

Leu Asp Glu Ser Glu Asp Glu Lys
1               5

<210> 134
<211> 10
<212> PRT
<213> Glycine max

<400>  134

Leu Gly Ile His Glu Asp Ser Gln Asn Lys
1               5                   10

<210>  135
<211>  11
<212>  PRT
<213>  Glycine max

<400>  135

Arg Ala Pro Phe Asp Leu Phe Asp Thr Arg Lys
1               5                   10

<210>  136
<211>  9
<212>  PRT
<213>  Glycine max

<400>  136

Ser Gly Asp Glu Leu Thr Ser Leu Lys
1               5

<210>  137
<211>  8
<212>  PRT
<213>  Glycine max

<400>  137

Val Glu Asp Val Asp Glu Glu Lys
1               5

<210>  138
<211>  2109
<212>  DNA
<213>  Glycine max

<400>  138
atggcttcgg agactgagac gtttgctttt caggccgaga tcaaccagct cctcagtcta      60

atcatcaaca ccttctacag caacaaggag atctttcttc gtgagctcat cagcaatgcc     120

tctgatgctt tggacaagat tcgatttgag agtcttacag acaagagcaa gcttgatgct     180

cagccagagt tgttcattca cattattcct gacaagacca acaacacatt gtctatcatt     240

gacagtggta ttggcatgac taaggccgat ttggtgaata acctcggtac tattgcaagg     300

tctgggacca aggaattcat ggaagccctg gctgctggtg ctgatgtgag catgattgga     360

cagtttggtg ttggtttcta ctcggcttat cttgtagctg acaaggttat tgttaccaca     420

aagcacaatg atgatgagca gtatgtctgg gagtcccatg ctggtggctc atttactgtg     480

acaagggata catctggtga gaaccttggt aggggaacaa agataacttt gtttctcaag     540

gaagatcaac ttgagtatct tgaggaacgc cgtctgaagg atttgataaa gaagcattct 600

gaattcatta gctatccaat ttctctttgg attgagaaga ccactgaaaa ggagatttct 660

gatgatgagg acgaggaaga gaagaaggat gaggagggta aagttgaaga tgtggatgaa 720

gataaggaga aggaggagaa gaaaaagaag accattaagg aggtatctca tgagtggtca 780

ttggtgaaca agcagaagcc tatttggatg aggaaacctg aagagatcac aaaagaagaa 840

tatgctgctt tctacaagag tcttaccaat gattgggaag agcatttggc tgttaagcac 900

ttttctgttg agggtcagct ggagtttaag gctgtcctct ttatccccaa gagggcacct 960

tttgatcttt ttgacactaa gaagaagcct aacaacatca aactgtatgt ccgccgtgtt 1020

ttcatcatgg acaactgtga ggagttgatg ccagaatatc ttagctttgt taagggtatt 1080

gtggattctg aggatcttcc tctcaacatt tctagagaaa tgctgcagca gaacaagatc 1140

ctgaaagtca tcaggaagaa cttggtcaag aagtgcattg agatgttctt tgaaattgct 1200

gagaacaagg aagactataa caagttttat gaagccttct ctaagaatct gaaacttggt 1260

attcatgagg attctcagaa caagacaaaa cttgctgaat tgctcaggta tcactccacc 1320

aagagtggtg atgagatgac cagcctcaag gactatgtta ccaggatgaa ggaagggcag 1380

aatgacatct actacattac tggtgaaagc aagaaagctg ttgagaattc ccctttcctt 1440

gaaaagctta agaagaaggg gttcgaggtt ctctacatgg ttgatgctat tgacgagtat 1500

gctgttggtc agcttaagga atttgaggga aagaagttgg tctctgctac taaggaaggc 1560

ctgaaacttg acgagagcga agacgaaaag aaaaagaagg aagaattgaa ggagaaattt 1620

gagggtctct gccatgtgat caaggatgtg ttgggtgaca aggtggagaa agttgtggtg 1680

tctgatcgtg ttgtggattc tccttgctgt ctcgtgactg gtgaatatgg gtggacagcc 1740

aacatggaaa ggattatgaa agcacaggca ttgagggaca gcagcatggc tggatacatg 1800

tcgagcaaga agaccatgga gattaaccct gagaacccca tcatggatga actgaggaag 1860

cgagctgatg ctgacaagaa tgacaaatct gtgaaggatc tcgttctcct actctttgag 1920

actgctcttc taacttctgg gttcagcctt gatgatccca acactttcgg taacaggatt 1980

cacaggatgc tgaagcttgg actgagcatt gatgaagatg ctggtgaagc agatgctgac 2040

atgcctcctc tcgaggacgc cgatgctgat gccgatgctg agggtagcaa gatggaagaa 2100

gttgattaa 2109

<210> 139
<211> 702
<212> PRT
<213> Glycine max

<400> 139

Met Ala Ser Glu Thr Glu Thr Phe Ala Phe Gln Ala Glu Ile Asn Gln
1               5                   10                  15

Leu Leu Ser Leu Ile Ile Asn Thr Phe Tyr Ser Asn Lys Glu Ile Phe
            20                  25                  30

Leu Arg Glu Leu Ile Ser Asn Ala Ser Asp Ala Leu Asp Lys Ile Arg
        35                  40                  45

Phe Glu Ser Leu Thr Asp Lys Ser Lys Leu Asp Ala Gln Pro Glu Leu
    50                  55                  60

Phe Ile His Ile Ile Pro Asp Lys Thr Asn Asn Thr Leu Ser Ile Ile
65                  70                  75                  80

Asp Ser Gly Ile Gly Met Thr Lys Ala Asp Leu Val Asn Asn Leu Gly
                85                  90                  95

Thr Ile Ala Arg Ser Gly Thr Lys Glu Phe Met Glu Ala Leu Ala Ala
            100                 105                 110

Gly Ala Asp Val Ser Met Ile Gly Gln Phe Gly Val Gly Phe Tyr Ser
        115                 120                 125

Ala Tyr Leu Val Ala Asp Lys Val Ile Val Thr Thr Lys His Asn Asp
    130                 135                 140

Asp Glu Gln Tyr Val Trp Glu Ser His Ala Gly Gly Ser Phe Thr Val
145                 150                 155                 160

Thr Arg Asp Thr Ser Gly Glu Asn Leu Gly Arg Gly Thr Lys Ile Thr
                165                 170                 175

Leu Phe Leu Lys Glu Asp Gln Leu Glu Tyr Leu Glu Glu Arg Arg Leu
            180                 185                 190

Lys Asp Leu Ile Lys Lys His Ser Glu Phe Ile Ser Tyr Pro Ile Ser
        195                 200                 205

Leu Trp Ile Glu Lys Thr Thr Glu Lys Glu Ile Ser Asp Asp Glu Asp
    210                 215                 220

Glu Glu Glu Lys Lys Asp Glu Glu Gly Lys Val Glu Asp Val Asp Glu
225                 230                 235                 240

Asp Lys Glu Lys Glu Glu Lys Lys Lys Lys Thr Ile Lys Glu Val Ser
                245                 250                 255

His Glu Trp Ser Leu Val Asn Lys Gln Lys Pro Ile Trp Met Arg Lys
            260                 265                 270

Pro Glu Glu Ile Thr Lys Glu Glu Tyr Ala Ala Phe Tyr Lys Ser Leu
        275                 280                 285

Thr Asn Asp Trp Glu Glu His Leu Ala Val Lys His Phe Ser Val Glu
    290                 295                 300

Gly Gln Leu Glu Phe Lys Ala Val Leu Phe Ile Pro Lys Arg Ala Pro
305                 310                 315                 320

Phe Asp Leu Phe Asp Thr Lys Lys Lys Pro Asn Asn Ile Lys Leu Tyr
                325                 330                 335

Val Arg Arg Val Phe Ile Met Asp Asn Cys Glu Glu Leu Met Pro Glu
            340                 345                 350

Tyr Leu Ser Phe Val Lys Gly Ile Val Asp Ser Glu Asp Leu Pro Leu
        355                 360                 365

Asn Ile Ser Arg Glu Met Leu Gln Gln Asn Lys Ile Leu Lys Val Ile
    370                 375                 380

Arg Lys Asn Leu Val Lys Lys Cys Ile Glu Met Phe Phe Glu Ile Ala
385                 390                 395                 400

Glu Asn Lys Glu Asp Tyr Asn Lys Phe Tyr Glu Ala Phe Ser Lys Asn
                405                 410                 415

Leu Lys Leu Gly Ile His Glu Asp Ser Gln Asn Lys Thr Lys Leu Ala
            420                 425                 430

Glu Leu Leu Arg Tyr His Ser Thr Lys Ser Gly Asp Glu Met Thr Ser
        435                 440                 445

Leu Lys Asp Tyr Val Thr Arg Met Lys Glu Gly Gln Asn Asp Ile Tyr
    450                 455                 460

Tyr Ile Thr Gly Glu Ser Lys Lys Ala Val Glu Asn Ser Pro Phe Leu
465                 470                 475                 480

Glu Lys Leu Lys Lys Lys Gly Phe Glu Val Leu Tyr Met Val Asp Ala
                485                 490                 495

Ile Asp Glu Tyr Ala Val Gly Gln Leu Lys Glu Phe Glu Gly Lys Lys
            500                 505                 510

Leu Val Ser Ala Thr Lys Glu Gly Leu Lys Leu Asp Glu Ser Glu Asp
        515                 520                 525

Glu Lys Lys Lys Lys Glu Glu Leu Lys Glu Lys Phe Glu Gly Leu Cys
    530                 535                 540

His Val Ile Lys Asp Val Leu Gly Asp Lys Val Glu Lys Val Val Val
545                 550                 555                 560

Ser Asp Arg Val Val Asp Ser Pro Cys Cys Leu Val Thr Gly Glu Tyr
                565                 570                 575

Gly Trp Thr Ala Asn Met Glu Arg Ile Met Lys Ala Gln Ala Leu Arg
            580                 585                 590

Asp Ser Ser Met Ala Gly Tyr Met Ser Ser Lys Lys Thr Met Glu Ile
        595                 600                 605

Asn Pro Glu Asn Pro Ile Met Asp Glu Leu Arg Lys Arg Ala Asp Ala
    610                 615                 620

Asp Lys Asn Asp Lys Ser Val Lys Asp Leu Val Leu Leu Leu Phe Glu
625                 630                 635                 640

Thr Ala Leu Leu Thr Ser Gly Phe Ser Leu Asp Asp Pro Asn Thr Phe
                645                 650                 655

Gly Asn Arg Ile His Arg Met Leu Lys Leu Gly Leu Ser Ile Asp Glu
            660                 665                 670

Asp Ala Gly Glu Ala Asp Ala Asp Met Pro Pro Leu Glu Asp Ala Asp
        675                 680                 685

Ala Asp Ala Asp Ala Glu Gly Ser Lys Met Glu Glu Val Asp
    690                 695                 700

<210>  140
<211>  16
<212>  PRT
<213>  Glycine max

<400>  140

Ala Asp Leu Val Asn Asn Leu Gly Thr Ile Ala Arg Ser Gly Thr Lys
1               5                   10                  15

<210>  141
<211>  10

<212>  PRT
<213>  Glycine max

<400>  141

Ala Val Glu Asn Ser Pro Phe Leu Glu Lys
1               5                   10


<210>  142
<211>  13
<212>  PRT
<213>  Glycine max

<400>  142

Glu Asp Gln Leu Glu Tyr Leu Glu Glu Arg Arg Leu Lys
1               5                   10


<210>  143
<211>  8
<212>  PRT
<213>  Glycine max

<400>  143

Glu Glu Tyr Ala Ala Phe Tyr Lys
1               5


<210>  144
<211>  14
<212>  PRT
<213>  Glycine max

<400>  144

Glu Gly Gln Asn Asp Ile Tyr Tyr Ile Thr Gly Glu Ser Lys
1               5                   10


<210>  145
<211>  17
<212>  PRT
<213>  Glycine max

<400>  145

Glu Ile Phe Leu Arg Glu Leu Ile Ser Asn Ala Ser Asp Ala Leu Asp
1               5                   10                  15


Lys


<210>  146
<211>  7
<212>  PRT
<213>  Glycine max

<400>  146

Phe Tyr Glu Ala Phe Ser Lys
1               5

<210>  147
<211>  11
<212>  PRT
<213>  Glycine max

<400>  147

His Phe Ser Val Glu Gly Gln Leu Glu Phe Lys
1               5                   10

<210>  148
<211>  9
<212>  PRT
<213>  Glycine max

<400>  148

Ile Arg Phe Glu Ser Leu Thr Asp Lys
1               5

<210>  149
<211>  11
<212>  PRT
<213>  Glycine max

<400>  149

Leu Ala Glu Leu Leu Arg Tyr His Ser Thr Lys
1               5                   10

<210>  150
<211>  8
<212>  PRT
<213>  Glycine max

<400>  150

Leu Asp Glu Ser Glu Asp Glu Lys
1               5

<210>  151
<211>  10
<212>  PRT
<213>  Glycine max

<400>  151

Leu Gly Ile His Glu Asp Ser Gln Asn Lys
1               5                   10

<210>  152
<211>  10
<212>  PRT

<213>  Glycine max

<400>  152

Arg Ala Pro Phe Asp Leu Phe Asp Thr Lys
1               5                   10


<210>  153
<211>  8
<212>  PRT
<213>  Glycine max

<400>  153

Val Glu Asp Val Asp Glu Asp Lys
1               5


<210>  154
<211>  2109
<212>  DNA
<213>  Glycine max

<400>  154
atggcttcgg agactgagac gtttgctttt caggccgaga tcaaccagct cctcagtcta      60

atcatcaaca ccttctacag caacaaggag atctttcttc gtgagctcat cagcaatgcc     120

tctgatgctt tggacaagat tcgatttgag agtcttacag acaagagcaa gcttgatgct     180

cagccagagt tgttcattca cattattcct gacaagacca acaacacatt gtctatcatt     240

gacagtggta ttggcatgac taaggccgat ttggtgaata acctcggtac tattgcaagg     300

tctgggacca aggaattcat ggaagccctg gctgctggtg ctgatgtgag catgattgga     360

cagtttggtg ttggtttcta ctcggcttat cttgtagctg acaaggttat tgttaccaca     420

aagcacaatg atgatgagca gtatgtctgg gagtcccatg ctggtggctc atttactgtg     480

acaagggata catctggtga gaaccttggt aggggaacaa agataacttt gtttctcaag     540

gaagatcaac ttgagtatct tgaggaacgc cgtctgaagg atttgataaa gaagcattct     600

gaattcatta gctatccaat ttctctttgg attgagaaga ccactgaaaa ggagatttct     660

gatgatgagg acgaggaaga gaagaaggat gaggagggta aagttgaaga tgtggatgaa     720

gataaggaga aggaggagaa gaaaaagaag accattaagg aggtatctca tgagtggtca     780

ttggtgaaca agcagaagcc tatttggatg aggaaacctg aagagatcac aaaagaagaa     840

tatgctgctt tctacaagag tcttaccaat gattgggaag agcatttggc tgttaagcac     900

ttttctgttg agggtcagct ggagtttaag gctgtcctct ttatccccaa gagggcacct     960

tttgatcttt ttgacactaa gaagaagcct aacaacatca aactgtatgt ccgccgtgtt    1020

ttcatcatgg acaactgtga ggagttgatg ccagaatatc ttagctttgt taagggtatt    1080

gtggattctg aggatcttcc tctcaacatt tctagagaaa tgctgcagca gaacaagatc    1140

ctgaaagtca tcaggaagaa cttggtcaag aagtgcattg agatgttctt tgaaattgct 1200

gagaacaagg aagactataa caagttttat gaagccttct ctaagaatct gaaacttggt 1260

attcatgagg attctcagaa caagacaaaa cttgctgaat tgctcaggta tcactccacc 1320

aagagtggtg atgagatgac cagcctcaag gactatgtta ccaggatgaa ggaagggcag 1380

aatgacatct actacattac tggtgaaagc aagaaagctg ttgagaattc ccctttcctt 1440

gaaaagctta agaagaaggg gttcgaggtt ctctacatgg ttgatgctat tgacgagtat 1500

gctgttggtc agcttaagga atttgaggga aagaagttgg tctctgctac taaggaaggc 1560

ctgaaacttg acgagagcga agacgaaaag aaaaagaagg aagaattgaa ggagaaattt 1620

gagggtctct gccatgtgat caaggatgtg ttgggtgaca aggtggagaa agttgtggtg 1680

tctgatcgtg ttgtggattc tccttgctgt ctcgtgactg gtgaatatgg gtggacagcc 1740

aacatggaaa ggattatgaa agcacaggca ttgagggaca gcagcatggc tggatacatg 1800

tcgagcaaga agaccatgga gattaaccct gagaacccca tcatggatga actgaggaag 1860

cgagctgatg ctgacaagaa tgacaaatct gtgaaggatc tcgttctcct actctttgag 1920

actgctcttc taacttctgg gttcagcctt gatgatccca acactttcgg taacaggatt 1980

cacaggatgc tgaagcttgg actgagcatt gatgaagatg ctggtgaagc agatgctgac 2040

atgcctcctc tcgaggacgc cgatgctgat gccgatgctg agggtagcaa gatggaagaa 2100

gttgattaa 2109

<210> 155
<211> 839
<212> PRT
<213> Glycine max

<400> 155

Met Lys Leu Ser Pro Arg Glu Val Glu Lys Leu Gly Leu His Asn Ala
1               5                   10                  15

Gly Tyr Leu Ala Gln Lys Arg Leu Ala Arg Gly Leu Arg Leu Asn Tyr
            20                  25                  30

Thr Glu Ala Val Ala Leu Ile Ala Thr Gln Ile Met Glu Phe Ala Arg
        35                  40                  45

Asp Gly Glu Lys Thr Val Ala Gln Leu Met Cys Ile Gly Lys His Leu
    50                  55                  60

Leu Gly Arg Arg Gln Val Leu Pro Glu Val Gln His Leu Leu Asn Ala
65                  70                  75                  80

Val Gln Val Glu Ala Thr Phe Pro Asp Gly Thr Lys Leu Val Thr Val

85                  90                  95

His Asp Pro Ile Ser Cys Glu His Gly Asp Leu Gly Gln Ala Leu Phe
            100                 105                 110

Gly Ser Phe Leu Pro Val Pro Ser Leu Asp Lys Phe Ala Glu Asn Lys
        115                 120                 125

Glu Asp Asn Arg Ile Pro Gly Glu Ile Ile Tyr Gly Asp Gly Ser Leu
    130                 135                 140

Val Leu Asn Pro Gly Lys Asn Ala Val Ile Leu Lys Val Val Ser Asn
145                 150                 155                 160

Gly Asp Arg Pro Ile Gln Val Gly Ser His Tyr His Phe Ile Glu Val
                165                 170                 175

Asn Pro Tyr Leu Thr Phe Asp Arg Arg Lys Ala Tyr Gly Met Arg Leu
            180                 185                 190

Asn Ile Ala Ala Gly Thr Ala Val Arg Phe Glu Pro Gly Asp Ser Lys
        195                 200                 205

Ser Val Lys Leu Val Arg Ile Gly Gly Asn Lys Val Ile Arg Gly Gly
    210                 215                 220

Asn Gly Ile Ala Asp Gly Gln Val Asn Glu Thr Asn Leu Arg Glu Ala
225                 230                 235                 240

Met Glu Ala Val Cys Lys Arg Gly Phe Gly His Lys Glu Glu Glu Asp
                245                 250                 255

Ala Ser Glu Gly Ile Thr Gly Asp Pro Asp Ser Pro Phe Thr Thr Ile
            260                 265                 270

Ile Pro Arg Glu Glu Tyr Ala Asn Lys Tyr Gly Pro Thr Thr Gly Asp
        275                 280                 285

Lys Ile Arg Leu Gly Asp Thr Asp Leu Phe Ala Lys Ile Glu Lys Asp
    290                 295                 300

Phe Ala Leu Tyr Gly Asp Glu Cys Val Phe Gly Gly Gly Lys Val Leu
305                 310                 315                 320

Arg Asp Gly Met Gly Gln Ser Cys Gly Asp Pro Pro Ala Ile Ser Leu
                325                 330                 335

Asp Thr Val Ile Thr Asn Ala Val Ile Ile Asp Tyr Ser Gly Ile Ile
            340                 345                 350

Lys Ala Asp Ile Gly Ile Lys Asp Gly Leu Ile Val Ser Ile Gly Lys
        355                 360                 365

Ala Gly Asn Pro Asp Ile Met Asp Asp Val Phe Phe Asn Met Ile Ile
    370                 375                 380

Gly Ala Asn Thr Glu Val Ile Ala Gly Glu Gly Leu Ile Val Thr Ala
385                 390                 395                 400

Gly Ala Ile Asp Cys His Val His Tyr Ile Cys Pro Gln Leu Val Asp
                405                 410                 415

Glu Ala Ile Ser Ser Gly Ile Thr Thr Leu Val Gly Gly Gly Thr Gly
            420                 425                 430

Pro Thr Ala Gly Thr Arg Ala Thr Thr Cys Thr Pro Ala Pro Ser Gln
        435                 440                 445

Met Lys Leu Met Leu Gln Ser Thr Asp Asp Leu Pro Leu Asn Phe Gly
    450                 455                 460

Phe Thr Gly Lys Gly Ser Ser Ser Lys Pro Asp Glu Leu His Asp Ile
465                 470                 475                 480

Ile Lys Ala Gly Ala Met Gly Leu Lys Leu His Glu Asp Trp Gly Ser
                485                 490                 495

Thr Pro Ala Ala Ile Asp Ser Cys Leu Thr Val Ala Asp Gln Tyr Asp
            500                 505                 510

Ile Gln Ile Asn Ile His Thr Asp Thr Leu Asn Glu Ala Gly Phe Val
        515                 520                 525

Glu His Ser Ile Ala Ala Phe Lys Gly Arg Thr Ile His Thr Tyr His
    530                 535                 540

Ser Glu Gly Ala Gly Gly Gly His Ala Pro Asp Ile Ile Lys Val Cys
545                 550                 555                 560

Gly Met Lys Asn Val Leu Pro Ser Ser Thr Asn Pro Thr Arg Pro Leu
                565                 570                 575

Thr Leu Asn Thr Ile Asp Glu His Leu Asp Met Leu Met Val Cys His
            580                 585                 590

His Leu Asn Arg Glu Ile Pro Glu Asp Leu Ala Phe Ala Cys Ser Arg
        595                 600                 605

Ile Arg Glu Gly Thr Ile Ala Ala Glu Asp Ile Leu His Asp Ile Gly
    610                 615                 620

Ala Ile Ser Ile Ile Ser Ser Asp Ser Gln Ala Met Gly Arg Val Gly
625                 630                 635                 640

Glu Val Ile Ser Arg Thr Trp Gln Thr Ala Asn Lys Met Lys Val Gln
                645                 650                 655

Arg Gly Pro Leu Gln Pro Gly Glu Ser Asp Asn Asp Asn Phe Arg Ile
            660                 665                 670

Lys Arg Tyr Ile Ala Lys Tyr Thr Ile Asn Pro Ala Ile Ala Asn Gly
        675                 680                 685

Phe Ser Gln Tyr Val Gly Ser Val Glu Val Gly Lys Leu Ala Asp Leu
    690                 695                 700

Val Met Trp Lys Pro Ser Phe Phe Gly Ala Lys Pro Glu Met Val Ile
705                 710                 715                 720

Lys Gly Gly Val Val Ala Trp Ala Asp Met Gly Asp Pro Asn Ala Ser
                725                 730                 735

Ile Pro Thr Pro Glu Pro Val Lys Met Arg Pro Met Phe Gly Thr Leu
            740                 745                 750

Gly Lys Ala Gly Gly Ala Leu Ser Ile Ala Phe Val Ser Lys Ala Ala
        755                 760                 765

Val Asp Gln Arg Val His Ala Leu Tyr Gly Leu Asn Lys Arg Val Glu
    770                 775                 780

Ala Val Gly Asn Val Arg Lys Leu Thr Lys Leu Asp Met Lys Leu Asn
785                 790                 795                 800

Asp Ser Leu Pro Gln Ile Thr Val Asp Pro Asp Asn Tyr Thr Val Thr
                805                 810                 815

Ala Asp Gly Glu Val Leu Thr Ser Phe Ala Thr Thr Phe Val Pro Leu
            820                 825                 830

Ser Arg Asn Tyr Phe Leu Phe
        835

<210>  156
<211>  12
<212>  PRT
<213>  Glycine max

<400>  156

Ala Gly Gly Ala Leu Ser Ile Ala Phe Val Ser Lys
1               5                   10


<210>  157
<211>  9
<212>  PRT
<213>  Glycine max

<400>  157

Asp Gly Leu Ile Val Ser Ile Gly Lys
1               5


<210>  158
<211>  2946
<212>  DNA
<213>  Glycine max

<400>  158
atggcttctt cttccacgac aatgcggttc tccgcggcct ccaccggcgc cgcggcggcg      60

cttcctcgcc ggagctccgt cgccggattc atcggcgtcg cggccagatc ctccgccaaa     120

tcgaggctcc gcttcatcgg aaggaacgcg aacttgagcc tccggatgag gaggatgagc     180

tctttctccg tcgtgaagtg cgtctccgga agcgaggcca aggtgcagga taccgtggcc     240

aaacaacaag aagctacaac ctctctcagc tccttcacgc cagatgcttc atctattgcc     300

tcaagtatta aataccatgc agagttcact ccattgtttt ctccagagaa ttttgatcta     360

ccacaggctt ttcttgcaac tgcacaaagt gttcgagatt ctctcattat aaactggaat     420

gctacctatg attactatga gaaactgaat gttaagcagg catattacct ttcgatggaa     480

tttttgcagg gtagagcatt attaaatgca ataggcaatc tggagctaac tggtccctat     540

gctgaggctt tgagcaagct cgggcataaa ttagaaaatg tggcatacca ggagccagat     600

gctgcacttg gaaatggtgg tcttgggcgt cttgcttcat gtttcttgga ctctttggca     660

accttgaact atccagcttg gggttatgga ttgagatata agtatggttt atttaaacag     720

cgaataacca aagatgggca ggaggaagtt gctgaagatt ggcttgagat gggcaatccc     780

tgggagatta ttagaaatga tgtttcatat cctgtaaagt tctatggcaa ggttgtttca     840

gggtcagatg gtaaaaaaca ttggattgga ggagaagata taaaagctgt tgcacatgat     900

gttcccatac caggatataa aactaagact acaatcaact tgagactttg gtctacaaaa     960

gctgcatcag aagaatttga tttatctgct tttaatgctg gaaggcacac tgaagcatct    1020

gaggctctag caaatgctga aaagatatgc tatatactct accctgggga tgaaccaata 1080

gagggcaaga tccttcgcct caagcaacaa tatactttat gttctgcttc tcttcaagac 1140

atcattgctc gttttgagag aagatcagga gcaaatgtta attgggaaga gtttcccgag 1200

aaggttgcag tgcagatgaa tgatactcac ccaacgttgt gcatcccaga gctcatgaga 1260

attttaatag atgtaaaggg cttaaattgg aaggatgctt ggaatattac acaaaggact 1320

gtagcatata caaaccacac tgttcttcca gaggcattag agaaatggag cttggatctt 1380

atgcagaaac tgcttccacg ccatattgag ataatagaaa tgattgacga ggagctggtt 1440

cggaccataa ttgcagaata tggcacagaa aattcagact tgctggagaa aaaattgaag 1500

gaaatgagaa tactagaaaa tgtcgaattg actgcagaat ttgcagatat actggttaag 1560

tccaaagagg ccattgatat tcctagtgag gagctgcaaa gttctgaaca agcagaagca 1620

gaagacgaaa aagatgatga tgaagtggaa gcagttgcta aaaagaatgg aacagatgaa 1680

agttccattg aggatgagaa agaagagttg cctgagccag taccagaacc accaaaattg 1740

gttcgtatgg caaatttgtg tgttgtgggt ggtcatgcag tgaatggggt tgcagagata 1800

catagtgaaa ttgtaaaaga tgatgttttc aatgcatttt ataagttgtg gcccgagaag 1860

ttccaaaaca aaacaaatgg tgtgacacca aggagatgga tcaggttctg caatccagat 1920

ctgagtaaaa taataaccga gtggataggt actgaggatt gggtcctaaa tactggaaaa 1980

ttggccgagc tacggaagtt tgtagacaat gaggatctac aagtgcagtg gagggaagca 2040

aagaggagta acaaagttaa agttgcagca ttcatcagag aaaaaacagg gtattctgtc 2100

agtcctgatg caatgtttga catccaggtg aagagaatcc atgaatataa gcgacaatta 2160

atgaacatat ttggaattgt ttaccgatac aagaagatga aagaaatgag tgcggcagaa 2220

agggaagcaa attttgttcc tagagtttgt atatttgggg gaaaagcatt tgctacatat 2280

gtgcaagcca agagaattgt gaaatttatt acagatgttg gggctacagt aaatcatgac 2340

cctgaaatag gagatctact taaggttgtt tttgtccctg attataatgt cagtgttgcc 2400

gagatgctta ttccagcaag tgaattgtca caacatatca gtactgctgg aatggaggca 2460

agtggaacta gcaatatgaa atttgcaatg aatggctgca tattgattgg aactttagat 2520

ggggccaatg ttgaaataag ggaagaggtt ggtgcagaca acttcttcct ctttggtgcc 2580

aaggctcatg aaattgctgg gctgaggaaa gaaagagcag agggaaagtt tgtgcctgat 2640

ccacgatttg aagaagttaa agaatttgtc agaagtggca tttttggatc ttacaactat 2700

gacgaattga tgggatcctt agaaggaaat gaaggttttg gtcgagcaga ttatttcctt 2760

gtagggaaag actttcccag ctacatagaa tgccaagaga aggttgatga agcataccgt 2820

gaccaaacta aatggacaag aatgtcaata ttgaacacag caggttcata caagtttagc 2880

agtgaccgca cgattcatga atatgcccga gagatatgga acattgaacc agtccaattg     2940

ccatag                                                                2946

<210>  159
<211>  981
<212>  PRT
<213>  Glycine max

<400>  159

Met Ala Ser Ser Ser Thr Thr Met Arg Phe Ser Ala Ala Ser Thr Gly
1               5                   10                  15

Ala Ala Ala Ala Leu Pro Arg Arg Ser Ser Val Ala Gly Phe Ile Gly
            20                  25                  30

Val Ala Ala Arg Ser Ser Ala Lys Ser Arg Leu Arg Phe Ile Gly Arg
        35                  40                  45

Asn Ala Asn Leu Ser Leu Arg Met Arg Arg Met Ser Ser Phe Ser Val
    50                  55                  60

Val Lys Cys Val Ser Gly Ser Glu Ala Lys Val Gln Asp Thr Val Ala
65                  70                  75                  80

Lys Gln Gln Glu Ala Thr Thr Ser Leu Ser Ser Phe Thr Pro Asp Ala
                85                  90                  95

Ser Ser Ile Ala Ser Ser Ile Lys Tyr His Ala Glu Phe Thr Pro Leu
            100                 105                 110

Phe Ser Pro Glu Asn Phe Asp Leu Pro Gln Ala Phe Leu Ala Thr Ala
        115                 120                 125

Gln Ser Val Arg Asp Ser Leu Ile Ile Asn Trp Asn Ala Thr Tyr Asp
    130                 135                 140

Tyr Tyr Glu Lys Leu Asn Val Lys Gln Ala Tyr Tyr Leu Ser Met Glu
145                 150                 155                 160

Phe Leu Gln Gly Arg Ala Leu Leu Asn Ala Ile Gly Asn Leu Glu Leu
                165                 170                 175

Thr Gly Pro Tyr Ala Glu Ala Leu Ser Lys Leu Gly His Lys Leu Glu
            180                 185                 190

Asn Val Ala Tyr Gln Glu Pro Asp Ala Ala Leu Gly Asn Gly Gly Leu
        195                 200                 205

Gly Arg Leu Ala Ser Cys Phe Leu Asp Ser Leu Ala Thr Leu Asn Tyr
    210                 215                 220

Pro Ala Trp Gly Tyr Gly Leu Arg Tyr Lys Tyr Gly Leu Phe Lys Gln
225                 230                 235                 240

Arg Ile Thr Lys Asp Gly Gln Glu Glu Val Ala Glu Asp Trp Leu Glu
                245                 250                 255

Met Gly Asn Pro Trp Glu Ile Ile Arg Asn Asp Val Ser Tyr Pro Val
            260                 265                 270

Lys Phe Tyr Gly Lys Val Val Ser Gly Ser Asp Gly Lys Lys His Trp
        275                 280                 285

Ile Gly Gly Glu Asp Ile Lys Ala Val Ala His Asp Val Pro Ile Pro
    290                 295                 300

Gly Tyr Lys Thr Lys Thr Thr Ile Asn Leu Arg Leu Trp Ser Thr Lys
305                 310                 315                 320

Ala Ala Ser Glu Glu Phe Asp Leu Ser Ala Phe Asn Ala Gly Arg His
                325                 330                 335

Thr Glu Ala Ser Glu Ala Leu Ala Asn Ala Glu Lys Ile Cys Tyr Ile
            340                 345                 350

Leu Tyr Pro Gly Asp Glu Pro Ile Glu Gly Lys Ile Leu Arg Leu Lys
        355                 360                 365

Gln Gln Tyr Thr Leu Cys Ser Ala Ser Leu Gln Asp Ile Ile Ala Arg
    370                 375                 380

Phe Glu Arg Arg Ser Gly Ala Asn Val Asn Trp Glu Glu Phe Pro Glu
385                 390                 395                 400

Lys Val Ala Val Gln Met Asn Asp Thr His Pro Thr Leu Cys Ile Pro
                405                 410                 415

Glu Leu Met Arg Ile Leu Ile Asp Val Lys Gly Leu Asn Trp Lys Asp
            420                 425                 430

Ala Trp Asn Ile Thr Gln Arg Thr Val Ala Tyr Thr Asn His Thr Val
        435                 440                 445

Leu Pro Glu Ala Leu Glu Lys Trp Ser Leu Asp Leu Met Gln Lys Leu
    450                 455                 460

Leu Pro Arg His Ile Glu Ile Ile Glu Met Ile Asp Glu Glu Leu Val
465                 470                 475                 480

Arg Thr Ile Ile Ala Glu Tyr Gly Thr Glu Asn Ser Asp Leu Leu Glu
                485                 490                 495

Lys Lys Leu Lys Glu Met Arg Ile Leu Glu Asn Val Glu Leu Thr Ala
            500                 505                 510

Glu Phe Ala Asp Ile Leu Val Lys Ser Lys Glu Ala Ile Asp Ile Pro
        515                 520                 525

Ser Glu Glu Leu Gln Ser Ser Glu Gln Ala Glu Ala Glu Asp Glu Lys
    530                 535                 540

Asp Asp Asp Glu Val Glu Ala Val Ala Lys Lys Asn Gly Thr Asp Glu
545                 550                 555                 560

Ser Ser Ile Glu Asp Glu Lys Glu Glu Leu Pro Glu Pro Val Pro Glu
                565                 570                 575

Pro Pro Lys Leu Val Arg Met Ala Asn Leu Cys Val Val Gly Gly His
            580                 585                 590

Ala Val Asn Gly Val Ala Glu Ile His Ser Glu Ile Val Lys Asp Asp
        595                 600                 605

Val Phe Asn Ala Phe Tyr Lys Leu Trp Pro Glu Lys Phe Gln Asn Lys
    610                 615                 620

Thr Asn Gly Val Thr Pro Arg Arg Trp Ile Arg Phe Cys Asn Pro Asp
625                 630                 635                 640

Leu Ser Lys Ile Ile Thr Glu Trp Ile Gly Thr Glu Asp Trp Val Leu
                645                 650                 655

Asn Thr Gly Lys Leu Ala Glu Leu Arg Lys Phe Val Asp Asn Glu Asp
            660                 665                 670

Leu Gln Val Gln Trp Arg Glu Ala Lys Arg Ser Asn Lys Val Lys Val
        675                 680                 685

Ala Ala Phe Ile Arg Glu Lys Thr Gly Tyr Ser Val Ser Pro Asp Ala
    690                 695                 700

Met Phe Asp Ile Gln Val Lys Arg Ile His Glu Tyr Lys Arg Gln Leu

705                 710                 715                 720

Met Asn Ile Phe Gly Ile Val Tyr Arg Tyr Lys Lys Met Lys Glu Met
                725                 730                 735

Ser Ala Ala Glu Arg Glu Ala Asn Phe Val Pro Arg Val Cys Ile Phe
            740                 745                 750

Gly Gly Lys Ala Phe Ala Thr Tyr Val Gln Ala Lys Arg Ile Val Lys
        755                 760                 765

Phe Ile Thr Asp Val Gly Ala Thr Val Asn His Asp Pro Glu Ile Gly
    770                 775                 780

Asp Leu Leu Lys Val Val Phe Val Pro Asp Tyr Asn Val Ser Val Ala
785                 790                 795                 800

Glu Met Leu Ile Pro Ala Ser Glu Leu Ser Gln His Ile Ser Thr Ala
                805                 810                 815

Gly Met Glu Ala Ser Gly Thr Ser Asn Met Lys Phe Ala Met Asn Gly
            820                 825                 830

Cys Ile Leu Ile Gly Thr Leu Asp Gly Ala Asn Val Glu Ile Arg Glu
        835                 840                 845

Glu Val Gly Ala Asp Asn Phe Phe Leu Phe Gly Ala Lys Ala His Glu
    850                 855                 860

Ile Ala Gly Leu Arg Lys Glu Arg Ala Glu Gly Lys Phe Val Pro Asp
865                 870                 875                 880

Pro Arg Phe Glu Glu Val Lys Glu Phe Val Arg Ser Gly Ile Phe Gly
                885                 890                 895

Ser Tyr Asn Tyr Asp Glu Leu Met Gly Ser Leu Glu Gly Asn Glu Gly
            900                 905                 910

Phe Gly Arg Ala Asp Tyr Phe Leu Val Gly Lys Asp Phe Pro Ser Tyr
        915                 920                 925

Ile Glu Cys Gln Glu Lys Val Asp Glu Ala Tyr Arg Asp Gln Thr Lys
    930                 935                 940

Trp Thr Arg Met Ser Ile Leu Asn Thr Ala Gly Ser Tyr Lys Phe Ser
945                 950                 955                 960

Ser Asp Arg Thr Ile His Glu Tyr Ala Arg Glu Ile Trp Asn Ile Glu
                965                 970                 975

Pro Val Gln Leu Pro
            980

<210>  160
<211>  9
<212>  PRT
<213>  Glycine max

<400>  160

Ala Phe Ala Thr Tyr Val Gln Ala Lys
1               5

<210>  161
<211>  23
<212>  PRT
<213>  Glycine max

<400>  161

Gln Gln Glu Ala Thr Thr Ser Leu Ser Ser Phe Thr Pro Asp Ala Ser
1               5                   10                  15

Ser Ile Ala Ser Ser Ile Lys
            20

<210>  162
<211>  2706
<212>  DNA
<213>  Glycine max

<400>  162
atggcgactg agaatccttt caatagcatt ttgacgacgc tcgaaaagcc cggtggtgct      60

ggcgagtttg gaaagtactt tagcttgcct gctctcaacg atcgtagaat cgatagactt     120

ccttactctg tgaggatact tttggaatct gcaatccgga actgtgatga gtttcaagtt     180

aagagtaacg acgttgaaaa aattattgat tgggagaata cctctccgaa gctggtggag     240

attccattca agccagctag agtgcttctc caggatttta ctggcgtgcc tgctgttgtc     300

gatcttgctt gcatgcgaga tgccatgaac aaacttggtg gtgattctaa taaaattaat     360

cccttggtac cagttgatct tgtcattgat cactctgttc aagttgatgt ggcaagatca     420

gaaaatgctg tccaggcaaa tatggaactt gaattccaaa gaaacaagga aagatttggt     480

ttcctcaaat ggggttctaa tgcattcaat aatatgcttg tcgttcctcc tggatcaggg     540

atagttcacc aggttaattt agaatacctt ggtagagttg tgttcaacac aaatggtgtg     600

ctttatcctg acagcgttgt tggaactgat tcacacacaa ctatgattga tggcctgggt     660

gttgctggat ggggagttgg tggaatagaa gcagaagctg caatgcttgg ccaacccatg     720

agcatggtcc taccgggtgt cgttgggttc aaattattag gcaaactacg agatggtgtc 780

acagctactg acttggtgtt gactgttaca caaatgctaa gaaagcatgg ggttgttggc 840

aagtttgtag agttttatgg ggaaggcatg agtgaactgt ctttggcaga tcgtgccacc 900

atagcaaaca tgtctcctga gtatggtgca accatgggct tctttcctgt ggatcatgtc 960

actttgcaat atttgagact gactggcaga agtgatgaga ctgtttctat gatagaatcg 1020

tacttacgag caaataagat gtttgtggat tacagcgagc cccaagtgga gagagtgtac 1080

tcatcgtatt tggaactcaa tcttgaggat gtagaaccct gtgtctcagg tccaaaaagg 1140

cctcatgacc gtgtcccttt gagagaaatg aaggtagact ggcatgcctg cctcaacaat 1200

aaagttggat tcaagggttt tgctgtacca aaagaatctc agaataaggt tgcagagttc 1260

acattccaag ggacaccagc acatcttagg cacggtgatg ttgttatagc tgctatcacc 1320

agttgtacaa atacttcaaa tcctagcgta atgcttggag ctgcattggt tgcaaagaaa 1380

gcatgtgaat tgggattgca ggtgaagcca tggattaaaa caagccttgc cccaggttct 1440

ggtgttgtga ctaagtattt gcagaggagt ggcttgcaga agtatttgaa tgagctaggg 1500

tttaatatag ttgggtatgg atgcaccaca tgcattggaa attcagggga tattaatgaa 1560

gctgttgcat ctgcaattac tgaaaatgat atagtagctg cagctgtact gtctggaaat 1620

aggaactttg agggccgagt tcacccatta acaagagcta attatcttgc ttctcctcct 1680

cttgttgttg cctatgccct tgctggcaca gtggacattg actttgacac tgaacccatt 1740

gggataggga aggatggcac aaagatcttc ttcaggdata tttggccatc cagtgaagaa 1800

atagcaaatg ttgtacaatc aagtgtgctg cctgctatgt ttagggacac atataatgcg 1860

atcacccaag ggaatcccat gtggaataat ttatctgttc caactggcac tctttatgct 1920

tgggacccta catcaactta tattcatgag ccaccttatt ttagagatat gagcatgtct 1980

cccccaggct ctcatggagt gaaagatgct tactgtttgc tcaactttgg agacagtatc 2040

acaactgacc acatctctcc agctggcagc atacataagg acagtcctgc agccagatac 2100

cttatagaac gtggggttga tagacgggac ttcaactcct atggaagtcg ccgtggtaat 2160

gatgaggtga tggcccgagg aacttttgcc aatattcgca ttgtcaacaa atttttgaat 2220

ggagaagttg gacctaaaac tattcatatt ccttctgggg agaagttatc agtctttgat 2280

gctgcagaga aatacaagag tgaggggcat gatatgatta tcttggctgg tgctgagtat 2340

gggagtggaa gctctcgtga ttgggctgca aaggggccaa tgctactggg cgtgaaagct 2400

gtcatagcaa aaagttttga aaggattcac cgaagtaatt tggtgggaat gggtatcatt 2460

ccattgtgtt ttaagcccgg ggatgatgct gattctcttg gattaactgg ccatgaacgt 2520

tacaccattg atctaccaag caatgtgaat gaaatacgac ccggccaaga tgtcacagtg 2580

gtgacagatg ccgggaaatc atttgtatct acactgagat ttgatacaga ggttgaacta 2640

gcttacttca accatggagg catcttacaa tatgtcatca gaaacatggt caatgctaaa 2700

cattga 2706

<210> 163
<211> 901
<212> PRT
<213> Glycine max

<400> 163

Met Ala Thr Glu Asn Pro Phe Asn Ser Ile Leu Thr Thr Leu Glu Lys
1               5                   10                  15

Pro Gly Gly Ala Gly Glu Phe Gly Lys Tyr Phe Ser Leu Pro Ala Leu
            20                  25                  30

Asn Asp Arg Arg Ile Asp Arg Leu Pro Tyr Ser Val Arg Ile Leu Leu
        35                  40                  45

Glu Ser Ala Ile Arg Asn Cys Asp Glu Phe Gln Val Lys Ser Asn Asp
    50                  55                  60

Val Glu Lys Ile Ile Asp Trp Glu Asn Thr Ser Pro Lys Leu Val Glu
65                  70                  75                  80

Ile Pro Phe Lys Pro Ala Arg Val Leu Leu Gln Asp Phe Thr Gly Val
                85                  90                  95

Pro Ala Val Val Asp Leu Ala Cys Met Arg Asp Ala Met Asn Lys Leu
            100                 105                 110

Gly Gly Asp Ser Asn Lys Ile Asn Pro Leu Val Pro Val Asp Leu Val
        115                 120                 125

Ile Asp His Ser Val Gln Val Asp Val Ala Arg Ser Glu Asn Ala Val
    130                 135                 140

Gln Ala Asn Met Glu Leu Glu Phe Gln Arg Asn Lys Glu Arg Phe Gly
145                 150                 155                 160

Phe Leu Lys Trp Gly Ser Asn Ala Phe Asn Asn Met Leu Val Val Pro
                165                 170                 175

Pro Gly Ser Gly Ile Val His Gln Val Asn Leu Glu Tyr Leu Gly Arg
            180                 185                 190

Val Val Phe Asn Thr Asn Gly Val Leu Tyr Pro Asp Ser Val Val Gly

195                 200                 205

Thr Asp Ser His Thr Thr Met Ile Asp Gly Leu Gly Val Ala Gly Trp
    210                 215                 220

Gly Val Gly Gly Ile Glu Ala Glu Ala Ala Met Leu Gly Gln Pro Met
225                 230                 235                 240

Ser Met Val Leu Pro Gly Val Val Gly Phe Lys Leu Leu Gly Lys Leu
                245                 250                 255

Arg Asp Gly Val Thr Ala Thr Asp Leu Val Leu Thr Val Thr Gln Met
            260                 265                 270

Leu Arg Lys His Gly Val Val Gly Lys Phe Val Glu Phe Tyr Gly Glu
        275                 280                 285

Gly Met Ser Glu Leu Ser Leu Ala Asp Arg Ala Thr Ile Ala Asn Met
    290                 295                 300

Ser Pro Glu Tyr Gly Ala Thr Met Gly Phe Phe Pro Val Asp His Val
305                 310                 315                 320

Thr Leu Gln Tyr Leu Arg Leu Thr Gly Arg Ser Asp Glu Thr Val Ser
                325                 330                 335

Met Ile Glu Ser Tyr Leu Arg Ala Asn Lys Met Phe Val Asp Tyr Ser
            340                 345                 350

Glu Pro Gln Val Glu Arg Val Tyr Ser Ser Tyr Leu Glu Leu Asn Leu
        355                 360                 365

Glu Asp Val Glu Pro Cys Val Ser Gly Pro Lys Arg Pro His Asp Arg
    370                 375                 380

Val Pro Leu Arg Glu Met Lys Val Asp Trp His Ala Cys Leu Asn Asn
385                 390                 395                 400

Lys Val Gly Phe Lys Gly Phe Ala Val Pro Lys Glu Ser Gln Asn Lys
                405                 410                 415

Val Ala Glu Phe Thr Phe Gln Gly Thr Pro Ala His Leu Arg His Gly
            420                 425                 430

Asp Val Val Ile Ala Ala Ile Thr Ser Cys Thr Asn Thr Ser Asn Pro
        435                 440                 445

Ser Val Met Leu Gly Ala Ala Leu Val Ala Lys Lys Ala Cys Glu Leu
    450                 455                 460

Gly Leu Gln Val Lys Pro Trp Ile Lys Thr Ser Leu Ala Pro Gly Ser
465                 470                 475                 480

Gly Val Val Thr Lys Tyr Leu Gln Arg Ser Gly Leu Gln Lys Tyr Leu
                485                 490                 495

Asn Glu Leu Gly Phe Asn Ile Val Gly Tyr Gly Cys Thr Thr Cys Ile
            500                 505                 510

Gly Asn Ser Gly Asp Ile Asn Glu Ala Val Ala Ser Ala Ile Thr Glu
        515                 520                 525

Asn Asp Ile Val Ala Ala Ala Val Leu Ser Gly Asn Arg Asn Phe Glu
    530                 535                 540

Gly Arg Val His Pro Leu Thr Arg Ala Asn Tyr Leu Ala Ser Pro Pro
545                 550                 555                 560

Leu Val Val Ala Tyr Ala Leu Ala Gly Thr Val Asp Ile Asp Phe Asp
                565                 570                 575

Thr Glu Pro Ile Gly Ile Gly Lys Asp Gly Thr Lys Ile Phe Phe Arg
            580                 585                 590

Asp Ile Trp Pro Ser Ser Glu Glu Ile Ala Asn Val Val Gln Ser Ser
        595                 600                 605

Val Leu Pro Ala Met Phe Arg Asp Thr Tyr Asn Ala Ile Thr Gln Gly
    610                 615                 620

Asn Pro Met Trp Asn Asn Leu Ser Val Pro Thr Gly Thr Leu Tyr Ala
625                 630                 635                 640

Trp Asp Pro Thr Ser Thr Tyr Ile His Glu Pro Pro Tyr Phe Arg Asp
                645                 650                 655

Met Ser Met Ser Pro Pro Gly Ser His Gly Val Lys Asp Ala Tyr Cys
            660                 665                 670

Leu Leu Asn Phe Gly Asp Ser Ile Thr Thr Asp His Ile Ser Pro Ala
        675                 680                 685

Gly Ser Ile His Lys Asp Ser Pro Ala Ala Arg Tyr Leu Ile Glu Arg
    690                 695                 700

Gly Val Asp Arg Arg Asp Phe Asn Ser Tyr Gly Ser Arg Arg Gly Asn
705                 710                 715                 720

Asp Glu Val Met Ala Arg Gly Thr Phe Ala Asn Ile Arg Ile Val Asn
                725                 730                 735

Lys Phe Leu Asn Gly Glu Val Gly Pro Lys Thr Ile His Ile Pro Ser
            740                 745                 750

Gly Glu Lys Leu Ser Val Phe Asp Ala Ala Glu Lys Tyr Lys Ser Glu
        755                 760                 765

Gly His Asp Met Ile Ile Leu Ala Gly Ala Glu Tyr Gly Ser Gly Ser
    770                 775                 780

Ser Arg Asp Trp Ala Ala Lys Gly Pro Met Leu Leu Gly Val Lys Ala
785                 790                 795                 800

Val Ile Ala Lys Ser Phe Glu Arg Ile His Arg Ser Asn Leu Val Gly
                805                 810                 815

Met Gly Ile Ile Pro Leu Cys Phe Lys Pro Gly Asp Asp Ala Asp Ser
            820                 825                 830

Leu Gly Leu Thr Gly His Glu Arg Tyr Thr Ile Asp Leu Pro Ser Asn
        835                 840                 845

Val Asn Glu Ile Arg Pro Gly Gln Asp Val Thr Val Val Thr Asp Ala
    850                 855                 860

Gly Lys Ser Phe Val Ser Thr Leu Arg Phe Asp Thr Glu Val Glu Leu
865                 870                 875                 880

Ala Tyr Phe Asn His Gly Gly Ile Leu Gln Tyr Val Ile Arg Asn Met
                885                 890                 895

Val Asn Ala Lys His
            900

<210>  164
<211>  9
<212>  PRT
<213>  Glycine max

<400>  164

Phe Leu Asn Gly Glu Val Gly Pro Lys
1               5

<210>  165
<211>  9
<212>  PRT
<213>  Glycine max

<400>  165

Leu Ser Val Phe Asp Ala Ala Glu Lys
1               5


<210>  166
<211>  9
<212>  PRT
<213>  Glycine max

<400>  166

Leu Ser Val Phe Asp Val Ala Glu Lys
1               5


<210>  167
<211>  12
<212>  PRT
<213>  Glycine max

<400>  167

Thr Ser Leu Ala Pro Gly Ser Gly Val Val Thr Lys
1               5                   10


<210>  168
<211>  2706
<212>  DNA
<213>  Glycine max

<400>  168
atggcgactg agaacccttt caatagcatt ttgaggacgc tcgagaagcc cggcggcgct      60

ggcgagtttg gaaagtactt tagcttgccc gctctcaacg atcctagaat cgatagactt     120

ccttactctg tgaggatact tttggaatct gcaatccgta actgtgatga gtttcaagtt     180

aagagtaacg acattgaaaa aattattgat tgggagaata cctctcccaa gctggtggag     240

attccattca agccagctag agtgcttctg caggacttta ctggggtgcc tgctgtggtt     300

gatcttgctt gcatgcgaga tgcaatgaac aaacttggtg gtgattctaa taaaattaac     360

cccttggtac cagttgatct tgtcattgat cactctgttc aagttgatgt ggcaagatca     420

gaaaatgctg tccaggcaaa tatggaactt gaattccaaa gaaacaagga aagatttggt     480

ttcctcaaat ggggttctaa tgcattcaat aatatgcttg tcgttcctcc tggatcaggg     540

atagttcatc aggttaattt agaatacctt ggtagagttg tgttcaacac aaatggtgtg     600

ctttatccag acagcgttgt cggaactgat tcacacacta ctatgataga tggcctgggt     660

gttgctggat ggggagttgg tggaatagaa gcagaagctg caatgcttgg ccagcccatg     720

agcatggtcc taccgggtgt tgttgggttc aaattattag gcaaactacg agatggtgtc 780
acagctactg acttggtgtt gactgttact caaatgctaa gaaagcatgg ggttgttggc 840
aagtttgtag agttttatgg ggaaggcatg agtgaactgt ctttggcaga tcgtgccact 900
atagcaaaca tgtctcctga gtatggtgca accatgggct tctttcctgt ggatcatgtc 960
actttgcaat atttgagact gactggcaga agtgatgaga ctgtttctat gatagaatcc 1020
tatttacgag caaataagat gtttgtggat tatagcgagc cccaagtgga gagagtgtac 1080
tcatcctatt tggaactcaa tcttgaggat gtagagccct gtgtctcagg tccaaaaagg 1140
cctcacgacc gtgtcccttt gagagaaatg aaggtagact ggcatgcctg cctcaacaat 1200
aaagttggat ttaagggttt tgctgtatca aaagaatctc agaataaggt tgcagagttc 1260
acattccaag ggacaccagc acatcttagg catggtgatg ttgttatagc tgctatcacc 1320
agttgtacaa atacttcaaa tcctagcgta atgcttggag ctgcattggt tgcaaagaaa 1380
gcatgtgaat tgggattgca ggtgaagcca tggattaaaa caagtcttgc cccaggttct 1440
ggtgttgtaa ctaagtattt gcagaggagt ggcttgcaga agtatttgaa tgagctaggg 1500
tttaatatag ttgggtatgg atgcaccaca tgcattggaa attcagggga tattaatgaa 1560
gctgttgcat ctgcgattac cgaaaatgat atagtagctg cagctgtatt gtctggaaat 1620
aggaactttg agggccgagt tcacccatta acaagagcta attatcttgc ttctcctcct 1680
cttgttgttg cctatgccct tgctggcaca gtggacattg actttgacac tgaacccatt 1740
gggataggga aggatggaac agagatcttc ttcaaggata tttggccatc cagtgaagaa 1800
atagcaaatg ttgtacaatc aagtgtgctg cctgatatgt ttagggacac atataatgca 1860
atcacccaag ggaatcccat gtggaataat ttatctgttc caacgggcac tctttatgct 1920
tgggacccta catcgactta tattcatgag ccaccttatt ttagagatat gagcatgtct 1980
cccccaggct ctcatggagt gaaggatgct tactgtttgc tcaactttgg agacagtatc 2040
acaactgacc acatctctcc agctggcagc atacataagg acagtcctgc ggccagatac 2100
cttatagaac gtggggttga tagacgggac ttcaactcct atggaagtcg ccgtggtaat 2160
gatgaggtga tggcccgagg aacgtttgcc aatattcgca ttgtcaacaa atttttgaat 2220
ggagaagttg gacctaaaac tattcatatt ccttctgggg agaagttgtc agtctttgac 2280
gttgcagaga aatacaagag tgaagggcat gatatgatta tcttggctgg tgctgagtat 2340
gggagtggaa gctctcgtga ttgggctgca aaggggccaa tgctactggg cgtgaaagct 2400
gtcatagcaa aaagttttga aaggattcac cgaagtaatt tggtgggaat gggtatcatt 2460
cctttgtgtt ttaagcccgg ggaggatgct gattctcttg gattaactgg tcaagaacgt 2520
tacaccattg atctaccaag caatgtgaat gaaatacgac ccggtcaaga tgtcacagtg 2580

gtgacagaca ccgggaaatc atttgtatct acattgagat ttgatactga ggttgaacta 2640

gcttacttca accatggagg tatcttacaa tatgtcatca gaaacttgat caatgctaaa 2700

cattga 2706

<210> 169
<211> 901
<212> PRT
<213> Glycine max

<400> 169

Met Ala Thr Glu Asn Pro Phe Asn Ser Ile Leu Arg Thr Leu Glu Lys
1               5                   10                  15

Pro Gly Gly Ala Gly Glu Phe Gly Lys Tyr Phe Ser Leu Pro Ala Leu
            20                  25                  30

Asn Asp Pro Arg Ile Asp Arg Leu Pro Tyr Ser Val Arg Ile Leu Leu
        35                  40                  45

Glu Ser Ala Ile Arg Asn Cys Asp Glu Phe Gln Val Lys Ser Asn Asp
    50                  55                  60

Ile Glu Lys Ile Ile Asp Trp Glu Asn Thr Ser Pro Lys Leu Val Glu
65                  70                  75                  80

Ile Pro Phe Lys Pro Ala Arg Val Leu Leu Gln Asp Phe Thr Gly Val
                85                  90                  95

Pro Ala Val Val Asp Leu Ala Cys Met Arg Asp Ala Met Asn Lys Leu
            100                 105                 110

Gly Gly Asp Ser Asn Lys Ile Asn Pro Leu Val Pro Val Asp Leu Val
        115                 120                 125

Ile Asp His Ser Val Gln Val Asp Val Ala Arg Ser Glu Asn Ala Val
    130                 135                 140

Gln Ala Asn Met Glu Leu Glu Phe Gln Arg Asn Lys Glu Arg Phe Gly
145                 150                 155                 160

Phe Leu Lys Trp Gly Ser Asn Ala Phe Asn Asn Met Leu Val Val Pro
                165                 170                 175

Pro Gly Ser Gly Ile Val His Gln Val Asn Leu Glu Tyr Leu Gly Arg
            180                 185                 190

Val Val Phe Asn Thr Asn Gly Val Leu Tyr Pro Asp Ser Val Val Gly

195                 200                 205

Thr Asp Ser His Thr Thr Met Ile Asp Gly Leu Gly Val Ala Gly Trp
    210                 215                 220

Gly Val Gly Gly Ile Glu Ala Glu Ala Ala Met Leu Gly Gln Pro Met
225                 230                 235                 240

Ser Met Val Leu Pro Gly Val Val Gly Phe Lys Leu Leu Gly Lys Leu
                245                 250                 255

Arg Asp Gly Val Thr Ala Thr Asp Leu Val Leu Thr Val Thr Gln Met
            260                 265                 270

Leu Arg Lys His Gly Val Val Gly Lys Phe Val Glu Phe Tyr Gly Glu
        275                 280                 285

Gly Met Ser Glu Leu Ser Leu Ala Asp Arg Ala Thr Ile Ala Asn Met
    290                 295                 300

Ser Pro Glu Tyr Gly Ala Thr Met Gly Phe Phe Pro Val Asp His Val
305                 310                 315                 320

Thr Leu Gln Tyr Leu Arg Leu Thr Gly Arg Ser Asp Glu Thr Val Ser
                325                 330                 335

Met Ile Glu Ser Tyr Leu Arg Ala Asn Lys Met Phe Val Asp Tyr Ser
            340                 345                 350

Glu Pro Gln Val Glu Arg Val Tyr Ser Ser Tyr Leu Glu Leu Asn Leu
        355                 360                 365

Glu Asp Val Glu Pro Cys Val Ser Gly Pro Lys Arg Pro His Asp Arg
    370                 375                 380

Val Pro Leu Arg Glu Met Lys Val Asp Trp His Ala Cys Leu Asn Asn
385                 390                 395                 400

Lys Val Gly Phe Lys Gly Phe Ala Val Ser Lys Glu Ser Gln Asn Lys
                405                 410                 415

Val Ala Glu Phe Thr Phe Gln Gly Thr Pro Ala His Leu Arg His Gly
            420                 425                 430

Asp Val Val Ile Ala Ala Ile Thr Ser Cys Thr Asn Thr Ser Asn Pro
        435                 440                 445

Ser Val Met Leu Gly Ala Ala Leu Val Ala Lys Lys Ala Cys Glu Leu
    450                 455                 460

Gly Leu Gln Val Lys Pro Trp Ile Lys Thr Ser Leu Ala Pro Gly Ser
465                 470                 475                 480

Gly Val Val Thr Lys Tyr Leu Gln Arg Ser Gly Leu Gln Lys Tyr Leu
                485                 490                 495

Asn Glu Leu Gly Phe Asn Ile Val Gly Tyr Gly Cys Thr Thr Cys Ile
            500                 505                 510

Gly Asn Ser Gly Asp Ile Asn Glu Ala Val Ala Ser Ala Ile Thr Glu
        515                 520                 525

Asn Asp Ile Val Ala Ala Ala Val Leu Ser Gly Asn Arg Asn Phe Glu
    530                 535                 540

Gly Arg Val His Pro Leu Thr Arg Ala Asn Tyr Leu Ala Ser Pro Pro
545                 550                 555                 560

Leu Val Val Ala Tyr Ala Leu Ala Gly Thr Val Asp Ile Asp Phe Asp
                565                 570                 575

Thr Glu Pro Ile Gly Ile Gly Lys Asp Gly Thr Glu Ile Phe Phe Lys
            580                 585                 590

Asp Ile Trp Pro Ser Ser Glu Glu Ile Ala Asn Val Val Gln Ser Ser
        595                 600                 605

Val Leu Pro Asp Met Phe Arg Asp Thr Tyr Asn Ala Ile Thr Gln Gly
    610                 615                 620

Asn Pro Met Trp Asn Asn Leu Ser Val Pro Thr Gly Thr Leu Tyr Ala
625                 630                 635                 640

Trp Asp Pro Thr Ser Thr Tyr Ile His Glu Pro Pro Tyr Phe Arg Asp
                645                 650                 655

Met Ser Met Ser Pro Pro Gly Ser His Gly Val Lys Asp Ala Tyr Cys
            660                 665                 670

Leu Leu Asn Phe Gly Asp Ser Ile Thr Thr Asp His Ile Ser Pro Ala
        675                 680                 685

Gly Ser Ile His Lys Asp Ser Pro Ala Ala Arg Tyr Leu Ile Glu Arg
    690                 695                 700

Gly Val Asp Arg Arg Asp Phe Asn Ser Tyr Gly Ser Arg Arg Gly Asn
705                 710                 715                 720

Asp Glu Val Met Ala Arg Gly Thr Phe Ala Asn Ile Arg Ile Val Asn
                725                 730                 735

Lys Phe Leu Asn Gly Glu Val Gly Pro Lys Thr Ile His Ile Pro Ser
            740                 745                 750

Gly Glu Lys Leu Ser Val Phe Asp Val Ala Glu Lys Tyr Lys Ser Glu
        755                 760                 765

Gly His Asp Met Ile Ile Leu Ala Gly Ala Glu Tyr Gly Ser Gly Ser
    770                 775                 780

Ser Arg Asp Trp Ala Ala Lys Gly Pro Met Leu Leu Gly Val Lys Ala
785                 790                 795                 800

Val Ile Ala Lys Ser Phe Glu Arg Ile His Arg Ser Asn Leu Val Gly
                805                 810                 815

Met Gly Ile Ile Pro Leu Cys Phe Lys Pro Gly Glu Asp Ala Asp Ser
            820                 825                 830

Leu Gly Leu Thr Gly Gln Glu Arg Tyr Thr Ile Asp Leu Pro Ser Asn
        835                 840                 845

Val Asn Glu Ile Arg Pro Gly Gln Asp Val Thr Val Val Thr Asp Thr
    850                 855                 860

Gly Lys Ser Phe Val Ser Thr Leu Arg Phe Asp Thr Glu Val Glu Leu
865                 870                 875                 880

Ala Tyr Phe Asn His Gly Gly Ile Leu Gln Tyr Val Ile Arg Asn Leu
                885                 890                 895

Ile Asn Ala Lys His
            900

<210>  170
<211>  9
<212>  PRT
<213>  Glycine max

<400>  170

Phe Leu Asn Gly Glu Val Gly Pro Lys
1               5

<210>  171
<211>  9
<212>  PRT
<213>  Glycine max

<400>  171

Leu Ser Val Phe Asp Val Ala Glu Lys
1               5


<210>  172
<211>  12
<212>  PRT
<213>  Glycine max

<400>  172

Thr Ser Leu Ala Pro Gly Ser Gly Val Val Thr Lys
1               5                   10


<210>  173
<211>  2292
<212>  DNA
<213>  Glycine max

<400>  173
atggcatctc acatcgttgg atacccccgt atgggtccca agagagagct caagttcgct      60

ctcgagtctt tctgggatgg caagagcagc gccgaggatt tgcagaaggt gtcttctgat     120

ctcagggcat ccatctggaa gcagatggct gatgctggga tcaagtacat ccccagcaac     180

actttctctc actatgacca ggttctcgac gccaccgcca ccctcggtgc cgttccacca     240

aggtacggct ggaccggcgg cgagattggg tttgatacct acttctccat ggccagaggt     300

aatgctaccg tgccagctat ggagatgacc aagtggttcg acaccaacta ccactttatt     360

gtccctgaat tgggccctga tgtgaacttc acctatgctt ctcacaaggc tgttgatgaa     420

tacaaggagg ccaaggcgct tggagtggat accgttccgg tcctcgttgg ccctgttaca     480

tacctgttgc tctccaagcc tgccaaggga gttgagaaat ccttttctct cctctctctc     540

cttcccaagg ttcttgctgt ctacaaggaa gttattgctg accttaaggc agctggtgct     600

tcatggattc agtttgatga gcctaccctt gtcttggacc ttgagtctca caagttgcaa     660

gcattcactg acgcatatgc agaacttgcg cctgctttgt ctggtttgaa tgttcttgtt     720

gagacctact ttgctgacat ccctgctgag gcatacaaga ccctcacatc tctgaatggc     780

gtcactgcat atggatttga tttggtccgt ggaaccaaca ctcttgattt gatcaagggt     840

ggatttccca gcggaaaata cctctttgct ggagtggttg atggaaggaa catctgggcc     900

aatgaccttg ctgcttctct cactaccttg cagggtcttg agggcattgt gggcaaagat     960

aagcttgttg tgtccacctc ctcctccctt cttcacactg ctgttgatct agttaacgag    1020

accaagttgg atgatgagat caagtcatgg ctagcttttg ctgcccaaaa aattgttgaa 1080

gttaacgcat tggctaaagc attgtctggc cacaaggatg aggccttctt ctctggtaat 1140

gctgctgctc tggcttcaag gaagtcttct ccaagagtga ccaacgaggc tgttcagaag 1200

gctgctgctg cattgaaggg ttcagatcat cgccgtgcaa caaatgtcag tgccagactg 1260

gattctcaac aaaagaagct caaccttcca atcctgccaa ccaccactat tggatccttc 1320

cctcagactg tagaactgag gagggtacgc cgtgaattca aggctaacaa gatctccgag 1380

gaagagtatg taaagtcaat taaggaggaa attcgcaaag ttgttgagct tcaagaagag 1440

cttgatattg atgttcttgt tcatggagaa ccagagagaa atgatatggt tgagtacttc 1500

ggtgaacaat tgtcaggctt tgccttcacc gttaatgggt gggtgcaatc ctatggttcc 1560

cgttgcgtga agccaccgat catctatggt gatgtgagcc gcccaaagcc aatgaccgtc 1620

ttctggtcat ctctggctca gagctttacc aagcgcccaa tgaagggaat gcttaccggt 1680

cctgttacca ttctcaactg gtcctttgtt agaaatgacc aacctagatc tgagaccacc 1740

taccagattg ctttgtctat caaggacgaa gtggaagacc ttgaaaaggc tggcatcact 1800

gttatccaaa ttgatgaagc tgctttgaga gagggtcttc cactgaggaa atcagagcaa 1860

gctcactact tggactgggc tgtccatgcc ttcagaatca ccaatgttgg tgtccaggat 1920

accacccaga tccacactca catgtgctac tcgaatttca acgacatcat ccactccatc 1980

atcgacatgg acgccgatgt tatcaccatt gagaactctc gctccgacga gaagcttctg 2040

tcagtcttcc gcgaaggtgt gaagtatggt gctggaattg gccctggtgt ctatgacatc 2100

cactccccaa gaataccacc aactgaagaa attgctgaca gaatcaacaa gatgctggca 2160

gtgctcgaga agaacatctt gtgggtgaac cctgactgtg ggctcaagac ccgtaagtac 2220

actgaggtga agccagccct cacaaacatg gttgccgcag caaaactcat ccgcaacgaa 2280

cttgccaagt ga 2292

<210> 174
<211> 763
<212> PRT
<213> Glycine max

<400> 174

Met Ala Ser His Ile Val Gly Tyr Pro Arg Met Gly Pro Lys Arg Glu
1               5                   10                  15

Leu Lys Phe Ala Leu Glu Ser Phe Trp Asp Gly Lys Ser Ser Ala Glu
            20                  25                  30

Asp Leu Gln Lys Val Ser Ser Asp Leu Arg Ala Ser Ile Trp Lys Gln
        35                  40                  45

Met Ala Asp Ala Gly Ile Lys Tyr Ile Pro Ser Asn Thr Phe Ser His
    50                  55                  60

Tyr Asp Gln Val Leu Asp Ala Thr Ala Thr Leu Gly Ala Val Pro Pro
65                  70                  75                  80

Arg Tyr Gly Trp Thr Gly Gly Glu Ile Gly Phe Asp Thr Tyr Phe Ser
                85                  90                  95

Met Ala Arg Gly Asn Ala Thr Val Pro Ala Met Glu Met Thr Lys Trp
            100                 105                 110

Phe Asp Thr Asn Tyr His Phe Ile Val Pro Glu Leu Gly Pro Asp Val
        115                 120                 125

Asn Phe Thr Tyr Ala Ser His Lys Ala Val Asp Glu Tyr Lys Glu Ala
    130                 135                 140

Lys Ala Leu Gly Val Asp Thr Val Pro Val Leu Val Gly Pro Val Thr
145                 150                 155                 160

Tyr Leu Leu Leu Ser Lys Pro Ala Lys Gly Val Glu Lys Ser Phe Ser
                165                 170                 175

Leu Leu Ser Leu Leu Pro Lys Val Leu Ala Val Tyr Lys Glu Val Ile
            180                 185                 190

Ala Asp Leu Lys Ala Ala Gly Ala Ser Trp Ile Gln Phe Asp Glu Pro
        195                 200                 205

Thr Leu Val Leu Asp Leu Glu Ser His Lys Leu Gln Ala Phe Thr Asp
    210                 215                 220

Ala Tyr Ala Glu Leu Ala Pro Ala Leu Ser Gly Leu Asn Val Leu Val
225                 230                 235                 240

Glu Thr Tyr Phe Ala Asp Ile Pro Ala Glu Ala Tyr Lys Thr Leu Thr
                245                 250                 255

Ser Leu Asn Gly Val Thr Ala Tyr Gly Phe Asp Leu Val Arg Gly Thr
            260                 265                 270

Asn Thr Leu Asp Leu Ile Lys Gly Gly Phe Pro Ser Gly Lys Tyr Leu
        275                 280                 285

Phe Ala Gly Val Val Asp Gly Arg Asn Ile Trp Ala Asn Asp Leu Ala
    290                 295                 300

Ala Ser Leu Thr Thr Leu Gln Gly Leu Glu Gly Ile Val Gly Lys Asp
305                 310                 315                 320

Lys Leu Val Val Ser Thr Ser Ser Ser Leu Leu His Thr Ala Val Asp
                325                 330                 335

Leu Val Asn Glu Thr Lys Leu Asp Asp Glu Ile Lys Ser Trp Leu Ala
            340                 345                 350

Phe Ala Ala Gln Lys Ile Val Glu Val Asn Ala Leu Ala Lys Ala Leu
        355                 360                 365

Ser Gly His Lys Asp Glu Ala Phe Phe Ser Gly Asn Ala Ala Ala Leu
    370                 375                 380

Ala Ser Arg Lys Ser Ser Pro Arg Val Thr Asn Glu Ala Val Gln Lys
385                 390                 395                 400

Ala Ala Ala Ala Leu Lys Gly Ser Asp His Arg Arg Ala Thr Asn Val
                405                 410                 415

Ser Ala Arg Leu Asp Ser Gln Gln Lys Lys Leu Asn Leu Pro Ile Leu
            420                 425                 430

Pro Thr Thr Thr Ile Gly Ser Phe Pro Gln Thr Val Glu Leu Arg Arg
        435                 440                 445

Val Arg Arg Glu Phe Lys Ala Asn Lys Ile Ser Glu Glu Glu Tyr Val
    450                 455                 460

Lys Ser Ile Lys Glu Glu Ile Arg Lys Val Val Glu Leu Gln Glu Glu
465                 470                 475                 480

Leu Asp Ile Asp Val Leu Val His Gly Glu Pro Glu Arg Asn Asp Met
                485                 490                 495

Val Glu Tyr Phe Gly Glu Gln Leu Ser Gly Phe Ala Phe Thr Val Asn
            500                 505                 510

Gly Trp Val Gln Ser Tyr Gly Ser Arg Cys Val Lys Pro Pro Ile Ile
        515                 520                 525

Tyr Gly Asp Val Ser Arg Pro Lys Pro Met Thr Val Phe Trp Ser Ser
    530                 535                 540

Leu Ala Gln Ser Phe Thr Lys Arg Pro Met Lys Gly Met Leu Thr Gly

545                 550                 555                 560

Pro Val Thr Ile Leu Asn Trp Ser Phe Val Arg Asn Asp Gln Pro Arg
                565                 570                 575

Ser Glu Thr Thr Tyr Gln Ile Ala Leu Ser Ile Lys Asp Glu Val Glu
            580                 585                 590

Asp Leu Glu Lys Ala Gly Ile Thr Val Ile Gln Ile Asp Glu Ala Ala
        595                 600                 605

Leu Arg Glu Gly Leu Pro Leu Arg Lys Ser Glu Gln Ala His Tyr Leu
    610                 615                 620

Asp Trp Ala Val His Ala Phe Arg Ile Thr Asn Val Gly Val Gln Asp
625                 630                 635                 640

Thr Thr Gln Ile His Thr His Met Cys Tyr Ser Asn Phe Asn Asp Ile
                645                 650                 655

Ile His Ser Ile Ile Asp Met Asp Ala Asp Val Ile Thr Ile Glu Asn
            660                 665                 670

Ser Arg Ser Asp Glu Lys Leu Leu Ser Val Phe Arg Glu Gly Val Lys
        675                 680                 685

Tyr Gly Ala Gly Ile Gly Pro Gly Val Tyr Asp Ile His Ser Pro Arg
    690                 695                 700

Ile Pro Pro Thr Glu Glu Ile Ala Asp Arg Ile Asn Lys Met Leu Ala
705                 710                 715                 720

Val Leu Glu Lys Asn Ile Leu Trp Val Asn Pro Asp Cys Gly Leu Lys
                725                 730                 735

Thr Arg Lys Tyr Thr Glu Val Lys Pro Ala Leu Thr Asn Met Val Ala
            740                 745                 750

Ala Ala Lys Leu Ile Arg Asn Glu Leu Ala Lys
        755                 760

<210>  175
<211>  21
<212>  PRT
<213>  Glycine max

<400>  175

Ala Gly Ile Thr Val Ile Gln Ile Asp Glu Ala Ala Leu Arg Glu Gly

1               5                   10                  15

Leu Pro Leu Arg Lys
            20

<210>  176
<211>  24
<212>  PRT
<213>  Glycine max

<400>  176

Ala Leu Gly Val Asp Thr Val Pro Val Leu Val Gly Pro Val Thr Tyr
1               5                   10                  15

Leu Leu Leu Ser Lys Pro Ala Lys
            20

<210>  177
<211>  16
<212>  PRT
<213>  Glycine max

<400>  177

Asp Glu Ala Phe Phe Ser Gly Asn Ala Ala Ala Leu Ala Ser Arg Lys
1               5                   10                  15

<210>  178
<211>  8
<212>  PRT
<213>  Glycine max

<400>  178

Asp Glu Val Glu Asp Leu Glu Lys
1               5

<210>  179
<211>  8
<212>  PRT
<213>  Glycine max

<400>  179

Ile Ser Glu Glu Glu Tyr Val Lys
1               5

<210>  180
<211>  9
<212>  PRT
<213>  Glycine max

<400>  180

Ile Val Glu Val Asn Ala Leu Ala Lys

1               5


<210>  181
<211>  8
<212>  PRT
<213>  Glycine max

<400>  181

Leu Ile Arg Asn Glu Leu Ala Lys
1               5


<210>  182
<211>  10
<212>  PRT
<213>  Glycine max

<400>  182

Leu Leu Ser Val Phe Arg Glu Gly Val Lys
1               5                   10


<210>  183
<211>  21
<212>  PRT
<213>  Glycine max

<400>  183

Leu Val Val Ser Thr Ser Ser Ser Leu Leu His Thr Ala Val Asp Leu
1               5                   10                  15


Val Asn Glu Thr Lys
            20


<210>  184
<211>  10
<212>  PRT
<213>  Glycine max

<400>  184

Ser Phe Ser Leu Leu Ser Leu Leu Pro Lys
1               5                   10


<210>  185
<211>  12
<212>  PRT
<213>  Glycine max

<400>  185

Ser Ser Pro Arg Val Thr Asn Glu Ala Val Gln Lys
1               5                   10


<210>  186

<211>  26
<212>  PRT
<213>  Glycine max

<400>  186

Thr Leu Thr Ser Leu Asn Gly Val Thr Ala Tyr Gly Phe Asp Leu Val
1               5                   10                  15

Arg Gly Thr Asn Thr Leu Asp Leu Ile Lys
            20                  25

<210>  187
<211>  29
<212>  PRT
<213>  Glycine max

<400>  187

Tyr Gly Ala Gly Ile Gly Pro Gly Val Tyr Asp Ile His Ser Pro Arg
1               5                   10                  15

Ile Pro Pro Thr Glu Glu Ile Ala Asp Arg Ile Asn Lys
            20                  25

<210>  188
<211>  2196
<212>  DNA
<213>  Glycine max

<400>  188
atggcttctt ccacttcctc tatctctctc tcccaagccc tcctccgccc caccaccacc      60

tctctcccct cctcctcctc ctccctccgc ctcaccccca ctctccgccc taccccaccc     120

gccctctccc tccgccgccg cctcaccacc tccatccgcg ccgccgcctc cgtcaaaacc     180

gtcgagaagg caaccgcgga cgccgcgctg gtcgaaaaat ccgtcaacac gatccgcttc     240

ctcgccatcg acgccgtcga gaaggcgaat tcgggtcacc cggggctccc catgggatgc     300

gctccgatgg gtcacgtgct atatgatgaa accatgaagt ataaccctaa gaaccctttc     360

tggttcaacc gcgatcgctt cgttctctcc gctgggcacg ggtgcatgct tcagtacgcg     420

cttctgcacc tcgctgggtt cgatagcgtc aaggaagagg atttgaggga atttcgccaa     480

tgggggagca gaactccggg acaccctgag aattttgaga ctccgggaat tgaagttaca     540

acaggtcctc ttggtcaggg gattgccaat gctgttggtt tggcccttgc agagaagcac     600

ttggctgcaa gatacaacaa gcctgacaat gagattgttg accattacac gtatgctatt     660

ctgggtgatg gttgtcaaat ggagggaatt gcaaatgaag catgctcact tgctggccac     720

tggggactgg ggaagctgat agcattttat gatgacaatc atatttctat tgatggtaac     780

acagagattg cgttcaccga gagtgttgat agtcgttttg agggacttgg gtggcatgtt     840

atttgggtaa agaatggaaa taatggctat gatgatattc gtgctgccat taaggaagca 900

aaagctgtca aagacaaacc cacattaatc aaggtcacaa caaccattgg ttatggttct 960

cctaacaagg ctaactccta cagtgtgcat ggaagtgcac tgggtgccaa agaagttgat 1020

gccaccaggc agaaccttgg atggtcacat gagccattcc acgtgcctga ggatgtcaaa 1080

aagcattgga gtcgccacac ccctgagggt gctgcacttg aagctgagtg gaatgctaag 1140

tttgctgagt atgaaaagaa atacaaggag gaggctgcag aattgaaatc tattatcaat 1200

ggtgaattcc ctgctggttg ggagaaagca cttccgacat acactccaga gagcccagcg 1260

gatgccacca gaaacctgtc tcaaacaaac cttaatgccc ttgcaaaggt tcttcccggt 1320

ctgcttggtg gcagtgcaga tcttgcttct tccaacatga ccttgctcaa aatgttcggg 1380

gacttccaaa aggatactcc agcagagcgt aatgttagat tcggtgttag agaacacgga 1440

atgggagcta tctgcaacgg cattgctctt cacagccctg gactgattcc atattgtgca 1500

accttctttg tattcactga ctacatgaga ggtgccataa ggctttctgc gctgtctgag 1560

gctggggtta tttatgtcat gacccatgat tcaataggac ttggagaaga tgggccaacc 1620

caccagccaa ttgagcacct agcaagcttc cgggcaatgc caaacatttt gatgcttcgt 1680

cccgccgacg gtaacgaaac agccggagca tacaaagtgg ccgtgctcaa caggaagaga 1740

ccctccattc ttgccctatc caggcaaaaa ctgccccagc ttcccggaac ttccattgaa 1800

ggagttgaaa agggtggtta caccatttcg gacaactcca ctggcaacaa gcctgatgtc 1860

attttgatcg gaactggttc ggaattggaa atcgctgcca aagctgctga tgacctaagg 1920

aaggaaggga aggctgttag agttgtttcc cttgtttctt gggaactttt tgatgagcaa 1980

tcagaagcct acaaggagag tgttttccct gctgctgttt cagccagagt tagcattgag 2040

gcaggatcaa catttgggtg ggagaaaatt gttggagcaa agggaaaagc aataggcatt 2100

gatcgttttg gagctagtgc tccagctgga agaatataca aagaatttgg tatcactaag 2160

gaagctgttg ttgctgcagc taaagagctt atctag 2196

<210> 189
<211> 731
<212> PRT
<213> Glycine max

<400> 189

Met Ala Ser Ser Thr Ser Ser Ile Ser Leu Ser Gln Ala Leu Leu Arg
1               5                   10                  15

Pro Thr Thr Thr Ser Leu Pro Ser Ser Ser Ser Leu Arg Leu Thr
            20                  25                  30

Pro Thr Leu Arg Pro Thr Pro Pro Ala Leu Ser Leu Arg Arg Arg Leu

35                  40                  45

Thr Thr Ser Ile Arg Ala Ala Ala Ser Val Lys Thr Val Glu Lys Ala
    50                  55                  60

Thr Ala Asp Ala Ala Leu Val Glu Lys Ser Val Asn Thr Ile Arg Phe
65                  70                  75                  80

Leu Ala Ile Asp Ala Val Glu Lys Ala Asn Ser Gly His Pro Gly Leu
                85                  90                  95

Pro Met Gly Cys Ala Pro Met Gly His Val Leu Tyr Asp Glu Thr Met
            100                 105                 110

Lys Tyr Asn Pro Lys Asn Pro Phe Trp Phe Asn Arg Asp Arg Phe Val
        115                 120                 125

Leu Ser Ala Gly His Gly Cys Met Leu Gln Tyr Ala Leu Leu His Leu
    130                 135                 140

Ala Gly Phe Asp Ser Val Lys Glu Glu Asp Leu Arg Glu Phe Arg Gln
145                 150                 155                 160

Trp Gly Ser Arg Thr Pro Gly His Pro Glu Asn Phe Glu Thr Pro Gly
                165                 170                 175

Ile Glu Val Thr Thr Gly Pro Leu Gly Gln Gly Ile Ala Asn Ala Val
            180                 185                 190

Gly Leu Ala Leu Ala Glu Lys His Leu Ala Ala Arg Tyr Asn Lys Pro
        195                 200                 205

Asp Asn Glu Ile Val Asp His Tyr Thr Tyr Ala Ile Leu Gly Asp Gly
    210                 215                 220

Cys Gln Met Glu Gly Ile Ala Asn Glu Ala Cys Ser Leu Ala Gly His
225                 230                 235                 240

Trp Gly Leu Gly Lys Leu Ile Ala Phe Tyr Asp Asp Asn His Ile Ser
                245                 250                 255

Ile Asp Gly Asn Thr Glu Ile Ala Phe Thr Glu Ser Val Asp Ser Arg
            260                 265                 270

Phe Glu Gly Leu Gly Trp His Val Ile Trp Val Lys Asn Gly Asn Asn
        275                 280                 285

Gly Tyr Asp Asp Ile Arg Ala Ala Ile Lys Glu Ala Lys Ala Val Lys
    290                 295                 300

Asp Lys Pro Thr Leu Ile Lys Val Thr Thr Thr Ile Gly Tyr Gly Ser
305                 310                 315                 320

Pro Asn Lys Ala Asn Ser Tyr Ser Val His Gly Ser Ala Leu Gly Ala
                325                 330                 335

Lys Glu Val Asp Ala Thr Arg Gln Asn Leu Gly Trp Ser His Glu Pro
            340                 345                 350

Phe His Val Pro Glu Asp Val Lys Lys His Trp Ser Arg His Thr Pro
        355                 360                 365

Glu Gly Ala Ala Leu Glu Ala Glu Trp Asn Ala Lys Phe Ala Glu Tyr
    370                 375                 380

Glu Lys Lys Tyr Lys Glu Glu Ala Ala Glu Leu Lys Ser Ile Ile Asn
385                 390                 395                 400

Gly Glu Phe Pro Ala Gly Trp Glu Lys Ala Leu Pro Thr Tyr Thr Pro
                405                 410                 415

Glu Ser Pro Ala Asp Ala Thr Arg Asn Leu Ser Gln Thr Asn Leu Asn
            420                 425                 430

Ala Leu Ala Lys Val Leu Pro Gly Leu Leu Gly Gly Ser Ala Asp Leu
        435                 440                 445

Ala Ser Ser Asn Met Thr Leu Leu Lys Met Phe Gly Asp Phe Gln Lys
    450                 455                 460

Asp Thr Pro Ala Glu Arg Asn Val Arg Phe Gly Val Arg Glu His Gly
465                 470                 475                 480

Met Gly Ala Ile Cys Asn Gly Ile Ala Leu His Ser Pro Gly Leu Ile
                485                 490                 495

Pro Tyr Cys Ala Thr Phe Phe Val Phe Thr Asp Tyr Met Arg Gly Ala
            500                 505                 510

Ile Arg Leu Ser Ala Leu Ser Glu Ala Gly Val Ile Tyr Val Met Thr
        515                 520                 525

His Asp Ser Ile Gly Leu Gly Glu Asp Gly Pro Thr His Gln Pro Ile
    530                 535                 540

Glu His Leu Ala Ser Phe Arg Ala Met Pro Asn Ile Leu Met Leu Arg
545                 550                 555                 560

Pro Ala Asp Gly Asn Glu Thr Ala Gly Ala Tyr Lys Val Ala Val Leu
                565                 570                 575

Asn Arg Lys Arg Pro Ser Ile Leu Ala Leu Ser Arg Gln Lys Leu Pro
            580                 585                 590

Gln Leu Pro Gly Thr Ser Ile Glu Gly Val Glu Lys Gly Gly Tyr Thr
        595                 600                 605

Ile Ser Asp Asn Ser Thr Gly Asn Lys Pro Asp Val Ile Leu Ile Gly
    610                 615                 620

Thr Gly Ser Glu Leu Glu Ile Ala Ala Lys Ala Ala Asp Asp Leu Arg
625                 630                 635                 640

Lys Glu Gly Lys Ala Val Arg Val Val Ser Leu Val Ser Trp Glu Leu
                645                 650                 655

Phe Asp Glu Gln Ser Glu Ala Tyr Lys Glu Ser Val Phe Pro Ala Ala
            660                 665                 670

Val Ser Ala Arg Val Ser Ile Glu Ala Gly Ser Thr Phe Gly Trp Glu
        675                 680                 685

Lys Ile Val Gly Ala Lys Gly Lys Ala Ile Gly Ile Asp Arg Phe Gly
    690                 695                 700

Ala Ser Ala Pro Ala Gly Arg Ile Tyr Lys Glu Phe Gly Ile Thr Lys
705                 710                 715                 720

Glu Ala Val Val Ala Ala Ala Lys Glu Leu Ile
                725                 730

<210>  190
<211>  18
<212>  PRT
<213>  Glycine max

<400>  190

Ala Ile Gly Ile Asp Arg Phe Gly Ala Ser Ala Pro Ala Gly Arg Ile
1               5                   10                  15

Tyr Lys

<210>  191
<211>  27
<212>  PRT
<213>  Glycine max

<400>  191

Ala Leu Pro Thr Tyr Thr Pro Glu Ser Pro Ala Asp Ala Thr Arg Asn
1               5                   10                  15

Leu Ser Gln Thr Asn Leu Asn Ala Leu Ala Lys
            20                  25

<210>  192
<211>  14
<212>  PRT
<213>  Glycine max

<400>  192

Ala Asn Ser Tyr Ser Val His Gly Ser Ala Leu Gly Ala Lys
1               5                   10

<210>  193
<211>  10
<212>  PRT
<213>  Glycine max

<400>  193

Ala Thr Ala Asp Ala Ala Leu Val Glu Lys
1               5                   10

<210>  194
<211>  7
<212>  PRT
<213>  Glycine max

<400>  194

Asp Lys Pro Thr Leu Ile Lys
1               5

<210>  195
<211>  30
<212>  PRT
<213>  Glycine max

<400>  195

Gly Gly Tyr Thr Ile Ser Asp Asn Ser Thr Gly Asn Lys Pro Asp Val
1               5                   10                  15

Ile Leu Ile Gly Thr Gly Ser Glu Leu Glu Ile Ala Ala Lys
            20                  25                  30

<210>  196
<211>  14
<212>  PRT
<213>  Glycine max

<400>  196

Leu Pro Gln Leu Pro Gly Thr Ser Ile Glu Gly Val Glu Lys
1               5                   10


<210>  197
<211>  14
<212>  PRT
<213>  Glycine max

<400>  197

Asn Gly Asn Asn Gly Tyr Asp Asp Ile Arg Ala Ala Ile Lys
1               5                   10


<210>  198
<211>  15
<212>  PRT
<213>  Glycine max

<400>  198

Ser Val Asn Thr Ile Arg Phe Leu Ala Ile Asp Ala Val Glu Lys
1               5                   10                  15


<210>  199
<211>  12
<212>  PRT
<213>  Glycine max

<400>  199

Val Thr Thr Thr Ile Gly Tyr Gly Ser Pro Asn Lys
1               5                   10


<210>  200
<211>  1797
<212>  DNA
<213>  Glycine max

<400>  200
atggaagttc cttcggaggt accatctact gacactggca ccagcaccgg caccggcgaa      60

accatgagca agaatgcgtt gaagcgggag ctgaagaaca aacagagaga agaagaaagg     120

aagcgcaagg atgaggagaa ggccaaaaag gcagctgaag cacaacaggc aaaggataac     180

aaatctgcac cagctgatga tgaggatatg gatccaactc aataccttga gaacaggcta     240

aagtatcttg cagttcaaaa gacagaaggg aagaacccgt atcctcataa attctttgtc     300

actatgtcta ttgatcaata catcaaggaa tatggaggtt taagcaacgg gcaacacatt     360

gaggatgtct ctgtgtcttt ggctggccgg atcatgcaca aacgcacctc tggttctaag 420

cttgtctttt atgacctgca cggtggtggt tgcaaggtcc aggttatggc tgatgctagt 480

aaatcagact tggatgaggc tggattttcc aaattccatt ctaatgtgaa gcgcggtgac 540

atagttggtg tcactgggtt cccaggcaaa agtaagaagg gcgaacttag tatttttccc 600

aagaattttg tgtcgctgtc tcattgtttg cacatgatgc caaggcaaaa gtctgctgct 660

gctgctattg ataatagaaa tccatggata ccgggaagta ctaggaatcc tgaaacatat 720

attttgaagg atcaggaaac tagatatcgg ttacgccatt tggatttgat gcttaatcca 780

gaggttcgag acatattcaa gaccaggtct aaaatcattt cttacattag gaggttcctt 840

gatgaccttg atttcttgga ggttgaaaca cctatgatga atatgattgc tggtggagct 900

gcagcccgtc catttgtaac acatcacaat gatcttaaca tgaggttatt catgaggatt 960

gctccagaac tgtatcttaa ggagttggtt gttggtggac tggatcgtgt ttatgaaatt 1020

ggtaaacaat ttaggaatga gggcatagat ttgactcata atcctgagtt tactacctgt 1080

gagttctata tggcttataa ggactacaat gacttaatgg atctaacaga gcaaatgttg 1140

agtggtatgg ttaaggagct tacgaaaggc agctataaaa tcaagtatca tgcagatggg 1200

attgataagg aacctattga aattgacttt actacacctt ttagaaggat tgacatgatt 1260

gaagagttag agaagatggc aggtctaagt attcccaaag acttgtcaag tgaggaagcc 1320

aatcaatatt tgaaggatgc atgcgtgaag tatgagatca aatgtcctcc ccctgagaca 1380

actgctcgtt tgttggataa acttgtgggt cactttttgg aagaaacgtg tgtaaatcct 1440

acattcatca aaaaccatcc tgagatcatg agtcctctag caaagtggca cagatcaaaa 1500

ccaggcctga ctgaacgttt tgaattgttt gttaataaac gtgaactttg caatgcatat 1560

actgaattga atgaccctgt agtacaacga cagagatttg ctgaacaact caaggatcgg 1620

caatctggcg atgatgaagc aatggcctta gatgaaacat tttgtacggc tctagaatat 1680

ggtttaccac ctactggtgg ttggggtttg ggcattgatc ggttgaccat gttactgact 1740

gattcacaga atattaagga ggttcttctc ttccctgcca tgaaacctca agactga 1797

<210> 201
<211> 598
<212> PRT
<213> Glycine max

<400> 201
   Met Glu Val Pro Ser Glu Val Pro Ser Thr Asp Thr Gly Thr Ser Thr
   1 5 10 15
   Gly Thr Gly Glu Thr Met Ser Lys Asn Ala Leu Lys Arg Glu Leu Lys
   20 25 30
   Asn Lys Gln Arg Glu Glu Glu Arg Lys Arg Lys Asp Glu Glu Lys Ala
   35 40 45
   Lys Lys Ala Ala Glu Ala Gln Gln Ala Lys Asp Asn Lys Ser Ala Pro
   50 55 60
   Ala Asp Asp Glu Asp Met Asp Pro Thr Gln Tyr Leu Glu Asn Arg Leu
   65 70 75 80
   Lys Tyr Leu Ala Val Gln Lys Thr Glu Gly Lys Asn Pro Tyr Pro His
   85 90 95
   Lys Phe Phe Val Thr Met Ser Ile Asp Gln Tyr Ile Lys Glu Tyr Gly
   100 105 110
   Gly Leu Ser Asn Gly Gln His Ile Glu Asp Val Ser Val Ser Leu Ala
   115 120 125
   Gly Arg Ile Met His Lys Arg Thr Ser Gly Ser Lys Leu Val Phe Tyr
   130 135 140
   Asp Leu His Gly Gly Gly Cys Lys Val Gln Val Met Ala Asp Ala Ser
   145 150 155 160
   Lys Ser Asp Leu Asp Glu Ala Gly Phe Ser Lys Phe His Ser Asn Val
   165 170 175
   Lys Arg Gly Asp Ile Val Gly Val Thr Gly Phe Pro Gly Lys Ser Lys
   180 185 190
   Lys Gly Glu Leu Ser Ile Phe Pro Lys Asn Phe Val Ser Leu Ser His
   195 200 205
   Cys Leu His Met Met Pro Arg Gln Lys Ser Ala Ala Ala Ala Ile Asp
   210 215 220
   Asn Arg Asn Pro Trp Ile Pro Gly Ser Thr Arg Asn Pro Glu Thr Tyr
   225 230 235 240
   Ile Leu Lys Asp Gln Glu Thr Arg Tyr Arg Leu Arg His Leu Asp Leu
   245 250 255
   Met Leu Asn Pro Glu Val Arg Asp Ile Phe Lys Thr Arg Ser Lys Ile
   260 265 270
   Ile Ser Tyr Ile Arg Arg Phe Leu Asp Asp Leu Asp Phe Leu Glu Val
   275 280 285
   Glu Thr Pro Met Met Asn Met Ile Ala Gly Gly Ala Ala Ala Arg Pro
       290                 295                 300
   Phe Val Thr His His Asn Asp Leu Asn Met Arg Leu Phe Met Arg Ile
   305                 310                 315                 320
   Ala Pro Glu Leu Tyr Leu Lys Glu Leu Val Val Gly Gly Leu Asp Arg
                   325                 330                 335
   Val Tyr Glu Ile Gly Lys Gln Phe Arg Asn Glu Gly Ile Asp Leu Thr
               340                 345                 350
   His Asn Pro Glu Phe Thr Thr Cys Glu Phe Tyr Met Ala Tyr Lys Asp
           355                 360                 365
   Tyr Asn Asp Leu Met Asp Leu Thr Glu Gln Met Leu Ser Gly Met Val
       370                 375                 380
   Lys Glu Leu Thr Lys Gly Ser Tyr Lys Ile Lys Tyr His Ala Asp Gly
   385                 390                 395                 400
   Ile Asp Lys Glu Pro Ile Glu Ile Asp Phe Thr Thr Pro Phe Arg Arg
                   405                 410                 415
   Ile Asp Met Ile Glu Glu Leu Glu Lys Met Ala Gly Leu Ser Ile Pro
               420                 425                 430
   Lys Asp Leu Ser Ser Glu Glu Ala Asn Gln Tyr Leu Lys Asp Ala Cys
           435                 440                 445
   Val Lys Tyr Glu Ile Lys Cys Pro Pro Pro Glu Thr Thr Ala Arg Leu
       450                 455                 460
   Leu Asp Lys Leu Val Gly His Phe Leu Glu Glu Thr Cys Val Asn Pro
   465                 470                 475                 480
   Thr Phe Ile Lys Asn His Pro Glu Ile Met Ser Pro Leu Ala Lys Trp
                   485                 490                 495
   His Arg Ser Lys Pro Gly Leu Thr Glu Arg Phe Glu Leu Phe Val Asn
               500                 505                 510
   Lys Arg Glu Leu Cys Asn Ala Tyr Thr Glu Leu Asn Asp Pro Val Val
           515                 520                 525
   Gln Arg Gln Arg Phe Ala Glu Gln Leu Lys Asp Arg Gln Ser Gly Asp
   530                 535                 540
   Asp Glu Ala Met Ala Leu Asp Glu Thr Phe Cys Thr Ala Leu Glu Tyr
   545                 550                 555                 560
   Gly Leu Pro Pro Thr Gly Gly Trp Gly Leu Gly Ile Asp Arg Leu Thr
                   565                 570                 575
   Met Leu Leu Thr Asp Ser Gln Asn Ile Lys Glu Val Leu Leu Phe Pro
               580                 585                 590
   Ala Met Lys Pro Gln Asp
           595

<210>  202
<211>  12
<212>  PRT
<213>  Glycine max

<400>  202

Asp Leu Ser Ser Glu Glu Ala Asn Gln Tyr Leu Lys
1               5                   10

<210>  203
<211>  1728
<212>  DNA
<213>  Glycine max

<400>  203
atgtaccgct ttgcaacctc cctcgcctcc aaagctagaa ttgccaggag cagcactcaa      60

cagattggaa gtagggtcac ttggaacagg aattatgcgg ccaaagacat taagtttggt     120

gtggaggctc gggctctgat gcttaagggt gtcgaagagc tcgctgatgc cgttaaagta     180

acaatgggcc ctaaggggcg taatgtggtt attgagcaaa gttttggagc acctaaagtg     240

acgaaagatg gagtaaccgt tgctaagagc attgaattca aggacaaagt caagaatatc     300

ggtgccagtc ttgtaaagca ggttgctaat gctactaatg acgtggctgg tgatggaacc     360

acttgtgcta caattcttac taaagcaata tttacggaag gatgtaaatc agttgcagct     420

ggaatgaatg cgatggacct gagacgaggt ataaatatgg ctgttgatgc tgtggtgaca     480

aatctgaaaa gcagagcacg tatgattagc acttctgaag aaattgccca ggttggaaca     540

atatctgcca atggggagag ggaaattggt gagttaattg caaaagctat ggagaaagtt     600

ggcaaagagg gtgtcatcac aatctcagat ggcaagacat tgtacaatga gttggaagtc     660

gttgaaggaa tgaagctcga caggggatac atttctccat atttcataac aaaccagaag     720

aaccagaaat gtgaacttga ggatcctctc attataatcc atgaaaagaa aatctcaagc     780

ataaatgcta tcgttaaagt attagagtta gctttgaaga gacaaagatc tttattgatt     840

gtcgccgagg atgtggaaag tgatgccctt gcaactctta ttctaaataa acttcgtgct 900

ggaatcaagg tatgtgccat caaagctcct ggttttggtg aaaaccgtaa atctggtctc 960

caggatcttg ctgttcttac aggaggtcaa cttatcaccg aagagcttgg tttgaatctt 1020

gaaaaagtag acctggactt atttggctct tgcaagaaga taacaatttc taaagatgac 1080

actgtcatcc ttgatggagc tggtgataag aaagcaattg aggaaagatg tgagcagatt 1140

agatcagcaa ttgaaaatag cacttcagat tatgacaagg aaaagttaca ggaaagatta 1200

gccaagcttt ctgggggtgt tgcagtactg aagattggag gagccagtga ggctgaagtt 1260

ggtgagaaga aggatagagt gacagatgcc ttaaatgcaa ccaaggcagc tgtagaggag 1320

ggcatagtac ctggtggtgg tgttgctctt ctttatgcat cgagtgagtt ggataagctt 1380

caaactgcca actttgatca aaagataggt gtccagatca tccaaaatgc tttgaagact 1440

cctgtgcaca caattgcttc aaatgcagga gttgagggtg ctgttgttgt tggcaaacta 1500

ttggaacaga atgatcctga tcttggatat gatgcagcca aaggtgaata tgttgatatg 1560

gttaaaactg gaattattga tccattgaag gttattagga ccgccttggt tgatgcagcc 1620

agtgtgtcat ctttgatgac aacaactgaa gctgttgtgt ctgaactccc gaaggacgat 1680

aaagatgttc ctgccatggg tggtggcatg ggtggcatgg attattaa 1728

<210> 204
<211> 575
<212> PRT
<213> Glycine max

<400> 204

Met Tyr Arg Phe Ala Thr Ser Leu Ala Ser Lys Ala Arg Ile Ala Arg
1               5                   10                  15

Ser Ser Thr Gln Gln Ile Gly Ser Arg Val Thr Trp Asn Arg Asn Tyr
            20                  25                  30

Ala Ala Lys Asp Ile Lys Phe Gly Val Glu Ala Arg Ala Leu Met Leu
        35                  40                  45

Lys Gly Val Glu Glu Leu Ala Asp Ala Val Lys Val Thr Met Gly Pro
    50                  55                  60

Lys Gly Arg Asn Val Val Ile Glu Gln Ser Phe Gly Ala Pro Lys Val
65                  70                  75                  80

Thr Lys Asp Gly Val Thr Val Ala Lys Ser Ile Glu Phe Lys Asp Lys
                85                  90                  95

Val Lys Asn Ile Gly Ala Ser Leu Val Lys Gln Val Ala Asn Ala Thr
        100                 105                 110

Asn Asp Val Ala Gly Asp Gly Thr Thr Cys Ala Thr Ile Leu Thr Lys
    115                 120                 125

Ala Ile Phe Thr Glu Gly Cys Lys Ser Val Ala Ala Gly Met Asn Ala
    130                 135                 140

Met Asp Leu Arg Arg Gly Ile Asn Met Ala Val Asp Ala Val Val Thr
145                 150                 155                 160

Asn Leu Lys Ser Arg Ala Arg Met Ile Ser Thr Ser Glu Glu Ile Ala
                165                 170                 175

Gln Val Gly Thr Ile Ser Ala Asn Gly Glu Arg Glu Ile Gly Glu Leu
            180                 185                 190

Ile Ala Lys Ala Met Glu Lys Val Gly Lys Glu Gly Val Ile Thr Ile
        195                 200                 205

Ser Asp Gly Lys Thr Leu Tyr Asn Glu Leu Glu Val Val Glu Gly Met
    210                 215                 220

Lys Leu Asp Arg Gly Tyr Ile Ser Pro Tyr Phe Ile Thr Asn Gln Lys
225                 230                 235                 240

Asn Gln Lys Cys Glu Leu Glu Asp Pro Leu Ile Ile Ile His Glu Lys
                245                 250                 255

Lys Ile Ser Ser Ile Asn Ala Ile Val Lys Val Leu Glu Leu Ala Leu
            260                 265                 270

Lys Arg Gln Arg Ser Leu Leu Ile Val Ala Glu Asp Val Glu Ser Asp
        275                 280                 285

Ala Leu Ala Thr Leu Ile Leu Asn Lys Leu Arg Ala Gly Ile Lys Val
    290                 295                 300

Cys Ala Ile Lys Ala Pro Gly Phe Gly Glu Asn Arg Lys Ser Gly Leu
305                 310                 315                 320

Gln Asp Leu Ala Val Leu Thr Gly Gly Gln Leu Ile Thr Glu Glu Leu
                325                 330                 335

Gly Leu Asn Leu Glu Lys Val Asp Leu Asp Leu Phe Gly Ser Cys Lys
            340                 345                 350

Lys Ile Thr Ile Ser Lys Asp Asp Thr Val Ile Leu Asp Gly Ala Gly
        355                 360                 365

Asp Lys Lys Ala Ile Glu Glu Arg Cys Glu Gln Ile Arg Ser Ala Ile
    370                 375                 380

Glu Asn Ser Thr Ser Asp Tyr Asp Lys Glu Lys Leu Gln Glu Arg Leu
385                 390                 395                 400

Ala Lys Leu Ser Gly Gly Val Ala Val Leu Lys Ile Gly Gly Ala Ser
                405                 410                 415

Glu Ala Glu Val Gly Glu Lys Lys Asp Arg Val Thr Asp Ala Leu Asn
            420                 425                 430

Ala Thr Lys Ala Ala Val Glu Glu Gly Ile Val Pro Gly Gly Gly Val
        435                 440                 445

Ala Leu Leu Tyr Ala Ser Ser Glu Leu Asp Lys Leu Gln Thr Ala Asn
    450                 455                 460

Phe Asp Gln Lys Ile Gly Val Gln Ile Ile Gln Asn Ala Leu Lys Thr
465                 470                 475                 480

Pro Val His Thr Ile Ala Ser Asn Ala Gly Val Glu Gly Ala Val Val
                485                 490                 495

Val Gly Lys Leu Leu Glu Gln Asn Asp Pro Asp Leu Gly Tyr Asp Ala
            500                 505                 510

Ala Lys Gly Glu Tyr Val Asp Met Val Lys Thr Gly Ile Ile Asp Pro
        515                 520                 525

Leu Lys Val Ile Arg Thr Ala Leu Val Asp Ala Ala Ser Val Ser Ser
    530                 535                 540

Leu Met Thr Thr Thr Glu Ala Val Val Ser Glu Leu Pro Lys Asp Asp
545                 550                 555                 560

Lys Asp Val Pro Ala Met Gly Gly Gly Met Gly Gly Met Asp Tyr
                565                 570                 575

<210>  205
<211>  24
<212>  PRT
<213>  Glycine max

<400>  205

Ala Ala Val Glu Glu Gly Ile Val Pro Gly Gly Gly Val Ala Leu Leu
1               5                   10                  15

Tyr Ala Ser Ser Glu Leu Asp Lys
            20

<210>  206
<211>  11
<212>  PRT
<213>  Glycine max

<400>  206

Asp Arg Val Thr Asp Ala Leu Asn Ala Thr Lys
1               5                   10

<210>  207
<211>  10
<212>  PRT
<213>  Glycine max

<400>  207

Glu Gly Val Ile Thr Ile Ser Asp Gly Lys
1               5                   10

<210>  208
<211>  14
<212>  PRT
<213>  Glycine max

<400>  208

Gly Arg Asn Val Val Ile Glu Gln Ser Phe Gly Ala Pro Lys
1               5                   10

<210>  209
<211>  10
<212>  PRT
<213>  Glycine max

<400>  209

Gly Val Glu Glu Leu Ala Asp Ala Val Lys
1               5                   10

<210>  210
<211>  12
<212>  PRT
<213>  Glycine max

<400>  210

Ile Gly Gly Ala Ser Glu Ala Glu Val Gly Glu Lys
1               5                   10

<210>  211
<211>  11
<212>  PRT
<213>  Glycine max

<400>  211

Ile Gly Val Gln Ile Ile Gln Asn Ala Leu Lys
1               5                   10


<210>  212
<211>  9
<212>  PRT
<213>  Glycine max

<400>  212

Ile Ser Ser Ile Asn Ala Ile Val Lys
1               5


<210>  213
<211>  15
<212>  PRT
<213>  Glycine max

<400>  213

Leu Leu Glu Gln Asn Asp Pro Asp Leu Gly Tyr Asp Ala Ala Lys
1               5                   10                  15


<210>  214
<211>  9
<212>  PRT
<213>  Glycine max

<400>  214

Leu Gln Thr Ala Asn Phe Asp Gln Lys
1               5


<210>  215
<211>  9
<212>  PRT
<213>  Glycine max

<400>  215

Leu Ser Gly Gly Val Ala Val Leu Lys
1               5


<210>  216
<211>  8
<212>  PRT
<213>  Glycine max

<400>  216

Asn Ile Gly Ala Ser Leu Val Lys
1               5

<210>  217
<211>  8
<212>  PRT
<213>  Glycine max

<400>  217

Thr Gly Ile Ile Asp Pro Leu Lys
1               5

<210>  218
<211>  20
<212>  PRT
<213>  Glycine max

<400>  218

Thr Pro Val His Thr Ile Ala Ser Asn Ala Gly Val Glu Gly Ala Val
1               5                   10                  15

Val Val Gly Lys
            20

<210>  219
<211>  1725
<212>  DNA
<213>  Glycine max

<400>  219
atgtatcgct ttgcttctaa cctcgcttcc aaagcaagga ttgctaggag tagcagccac      60

cagattggaa gtaggctgag ttcgagcaga aactatgcgg ctaaagacat tagatttggt     120

gtggaggctc gggcactgat gcttaagggt gttgaagagc tagctgatgc tgtcaaagta     180

accatgggtc ccaaggggcg taatgttgtg attgagcaaa gttttggtgc ccctaaagta     240

accaaagatg gagtaactgt tgcaaagagc attgaattca aggataaagt taagaatgtt     300

ggtgccagtc ttgtaaagca ggttgcaaat gctactaatg atgtggctgg tgatggaacc     360

acgtgcgcta cagtccttac acgagcaata tttactgaag gctgcaaatc agttgcggct     420

ggaatgaatg caatggacct gaggcgtggt ataagtatgg ctgtcgatgc tgtggtgaca     480

aacttgaaaa gcagagcaag gatgattagc acatctgaag aaatagcaca ggtagggaca     540

atatctgcta acggagagag agaaattggt gagttgattg caaaagctat ggagaaagtt     600

ggcaaagagg gagtaatcac aatttcagat ggcaaaacct tatataatga gttggaagtt     660

gttgaaggaa tgaagctcga caggggctac atatctcctt atttcattac aaatgataag     720

aaccagaaat gtgaacttga agatcccctc attctaattc atgagaagaa aatctcgagt     780

ataaatgcta tagttaaagt cttagagttg gctttgaaga gacaaagatc tttattgatt     840

attgccgagg atgtggaaag tgatgcactt gcaactctta ttctaaataa gcttcgggct 900

ggaattaagg tatgtgctat taaagcccct ggttttggcg aaaataggaa agccaatctt 960

caagatcttg cagttctcac aggaggagca cttattactg aagagcttgg cttgaagctt 1020

gaaaaagtgg atttggatat gcttggcacc tgtaaaaaaa taacagtttc taaggatgac 1080

actgtcattc ttgacggagc aggtgacaag aaagcacttg aggaaagatg cgagcagatt 1140

aggtctgcaa ttgaaaacag cacctcagat tatgacaagg aaaagttaca ggaaagatta 1200

gccaagcttt ctggtggtgt tgccgtgctt aagattggag gagccagtga agctgaagtt 1260

ggtgagaaga aggatagagt gacagatgcc ttaaatgcaa ctaaggctgc tgtagaggaa 1320

ggcattgtat caggtggtgg tgttgctctt ctatatgcgt caaaagagtt ggataaactt 1380

caaactgcta actttgatca gaagattggt gtccagatta tccaaaatgc tttaaagaca 1440

cctgtgctca caattgcatc aaatgcgggt gttgagggtg cagttgttgt tggcaaacta 1500

ttggaacagg agaatcatga tctaggatat gatgcagcca aaggtgaata tgttgatatg 1560

gttaaagctg gaatcataga ccctttgaag gtgattagga cagccttggt tgatgcagcc 1620

agtgtatcat ctttgatgac aacaactgaa gctgttgtgt ctgaacttcc aaacgatgat 1680

aaagatactc cagctatggc tggtggcatg ggtggcatgg gttat 1725

<210> 220
<211> 575
<212> PRT
<213> Glycine max

<400> 220

Met Tyr Arg Phe Ala Ser Asn Leu Ala Ser Lys Ala Arg Ile Ala Arg
1               5                   10                  15

Ser Ser Ser His Gln Ile Gly Ser Arg Leu Ser Ser Ser Arg Asn Tyr
            20                  25                  30

Ala Ala Lys Asp Ile Arg Phe Gly Val Glu Ala Arg Ala Leu Met Leu
        35                  40                  45

Lys Gly Val Glu Glu Leu Ala Asp Ala Val Lys Val Thr Met Gly Pro
    50                  55                  60

Lys Gly Arg Asn Val Val Ile Glu Gln Ser Phe Gly Ala Pro Lys Val
65                  70                  75                  80

Thr Lys Asp Gly Val Thr Val Ala Lys Ser Ile Glu Phe Lys Asp Lys
                85                  90                  95

Val Lys Asn Val Gly Ala Ser Leu Val Lys Gln Val Ala Asn Ala Thr
        100                 105                 110

Asn Asp Val Ala Gly Asp Gly Thr Thr Cys Ala Thr Val Leu Thr Arg
    115                 120                 125

Ala Ile Phe Thr Glu Gly Cys Lys Ser Val Ala Ala Gly Met Asn Ala
    130                 135                 140

Met Asp Leu Arg Arg Gly Ile Ser Met Ala Val Asp Ala Val Val Thr
145                 150                 155                 160

Asn Leu Lys Ser Arg Ala Arg Met Ile Ser Thr Ser Glu Glu Ile Ala
                165                 170                 175

Gln Val Gly Thr Ile Ser Ala Asn Gly Glu Arg Glu Ile Gly Glu Leu
            180                 185                 190

Ile Ala Lys Ala Met Glu Lys Val Gly Lys Glu Gly Val Ile Thr Ile
        195                 200                 205

Ser Asp Gly Lys Thr Leu Tyr Asn Glu Leu Glu Val Val Glu Gly Met
    210                 215                 220

Lys Leu Asp Arg Gly Tyr Ile Ser Pro Tyr Phe Ile Thr Asn Asp Lys
225                 230                 235                 240

Asn Gln Lys Cys Glu Leu Glu Asp Pro Leu Ile Leu Ile His Glu Lys
                245                 250                 255

Lys Ile Ser Ser Ile Asn Ala Ile Val Lys Val Leu Glu Leu Ala Leu
            260                 265                 270

Lys Arg Gln Arg Ser Leu Leu Ile Ile Ala Glu Asp Val Glu Ser Asp
        275                 280                 285

Ala Leu Ala Thr Leu Ile Leu Asn Lys Leu Arg Ala Gly Ile Lys Val
    290                 295                 300

Cys Ala Ile Lys Ala Pro Gly Phe Gly Glu Asn Arg Lys Ala Asn Leu
305                 310                 315                 320

Gln Asp Leu Ala Val Leu Thr Gly Gly Ala Leu Ile Thr Glu Glu Leu
                325                 330                 335

Gly Leu Lys Leu Glu Lys Val Asp Leu Asp Met Leu Gly Thr Cys Lys
            340                 345                 350

Lys Ile Thr Val Ser Lys Asp Asp Thr Val Ile Leu Asp Gly Ala Gly
        355                 360                 365

Asp Lys Lys Ala Leu Glu Glu Arg Cys Glu Gln Ile Arg Ser Ala Ile
    370                 375                 380

Glu Asn Ser Thr Ser Asp Tyr Asp Lys Glu Lys Leu Gln Glu Arg Leu
385                 390                 395                 400

Ala Lys Leu Ser Gly Gly Val Ala Val Leu Lys Ile Gly Gly Ala Ser
                405                 410                 415

Glu Ala Glu Val Gly Glu Lys Lys Asp Arg Val Thr Asp Ala Leu Asn
            420                 425                 430

Ala Thr Lys Ala Ala Val Glu Glu Gly Ile Val Ser Gly Gly Gly Val
        435                 440                 445

Ala Leu Leu Tyr Ala Ser Lys Glu Leu Asp Lys Leu Gln Thr Ala Asn
    450                 455                 460

Phe Asp Gln Lys Ile Gly Val Gln Ile Ile Gln Asn Ala Leu Lys Thr
465                 470                 475                 480

Pro Val Leu Thr Ile Ala Ser Asn Ala Gly Val Glu Gly Ala Val Val
                485                 490                 495

Val Gly Lys Leu Leu Glu Gln Glu Asn His Asp Leu Gly Tyr Asp Ala
            500                 505                 510

Ala Lys Gly Glu Tyr Val Asp Met Val Lys Ala Gly Ile Ile Asp Pro
        515                 520                 525

Leu Lys Val Ile Arg Thr Ala Leu Val Asp Ala Ala Ser Val Ser Ser
    530                 535                 540

Leu Met Thr Thr Thr Glu Ala Val Val Ser Glu Leu Pro Asn Asp Asp
545                 550                 555                 560

Lys Asp Thr Pro Ala Met Ala Gly Gly Met Gly Gly Met Gly Tyr
                565                 570                 575

<210>  221
<211>  8
<212>  PRT
<213>  Glycine max

<400>  221

Ala Gly Ile Ile Asp Pro Leu Lys
1               5

<210>  222
<211>  11
<212>  PRT
<213>  Glycine max

<400>  222

Asp Arg Val Thr Asp Ala Leu Asn Ala Thr Lys
1               5                   10

<210>  223
<211>  10
<212>  PRT
<213>  Glycine max

<400>  223

Glu Gly Val Ile Thr Ile Ser Asp Gly Lys
1               5                   10

<210>  224
<211>  14
<212>  PRT
<213>  Glycine max

<400>  224

Gly Arg Asn Val Val Ile Glu Gln Ser Phe Gly Ala Pro Lys
1               5                   10

<210>  225
<211>  10
<212>  PRT
<213>  Glycine max

<400>  225

Gly Val Glu Glu Leu Ala Asp Ala Val Lys
1               5                   10

<210>  226
<211>  12
<212>  PRT
<213>  Glycine max

<400>  226

Ile Gly Gly Ala Ser Glu Ala Glu Val Gly Glu Lys
1               5                   10

<210>  227
<211>  11
<212>  PRT

<213>  Glycine max

<400>  227

Ile Gly Val Gln Ile Ile Gln Asn Ala Leu Lys
1               5                   10


<210>  228
<211>  9
<212>  PRT
<213>  Glycine max

<400>  228

Ile Ser Ser Ile Asn Ala Ile Val Lys
1               5


<210>  229
<211>  15
<212>  PRT
<213>  Glycine max

<400>  229

Leu Leu Glu Gln Glu Asn His Asp Leu Gly Tyr Asp Ala Ala Lys
1               5                   10                  15


<210>  230
<211>  9
<212>  PRT
<213>  Glycine max

<400>  230

Leu Gln Thr Ala Asn Phe Asp Gln Lys
1               5


<210>  231
<211>  9
<212>  PRT
<213>  Glycine max

<400>  231

Leu Ser Gly Gly Val Ala Val Leu Lys
1               5


<210>  232
<211>  1869
<212>  DNA
<213>  Glycine max

<400>  232
atggctcaag ttcttgagaa ccccaccatt gacgcggcgg cttccggcgc gaacccctcg      60

ctgacgacca tctcgttgta cgtcggggat cttcaccacg acgtcaacga tccgcagctg     120

tatgatctct tcaaccaggt tgcccaagtc gtttccgttc gaatttgcag ggacgtggcc     180

acccaacaat cccttggtta cggctacgtc aatttcagca acgctcacga tgccgcaaaa 240

gcaattgatg tgctgaattt cactccactg aacggcaaaa tcattaggat aatgtactct 300

attcgggacc ctagtgctcg gaagagtggt gcagctaatg ttttcataaa gaatttggac 360

aaggcgattg atcacaaggc tttgtacgat acattttcgg cttttgggaa tattctatct 420

tgcaaggttg cgacagatgc ttctggccag tctaaaggcc atggttttgt gcagtttgag 480

agtgaagaat ctgcacaaaa tgcaattgac aagttaaacg gcatgctgat caatgataag 540

caggtctttg tgggtccttt cctgcggaag caggatagag agagtgctct cagtggcaca 600

aagtttaata acgttttcgt gaaaaacctg ttggattcaa tgactgaggc tgacttggag 660

agaatttttg gagaatatgg agctattact agcgctgtag tgatgagaga tgtagatggt 720

aaatcaaaag gttttggttt tgtcaatttt gcaaatgtag acgatgctgc taaagctgtg 780

gaggctctta atggaaagaa ttttgatggt aaggagtggt atgttggaaa agcccagaaa 840

aagtctgaaa gggagcttga actgaaaggg caacatgaac agattacaaa ggagactgtt 900

gataaatatc atggtacaaa cttgtatatc aagaacttgg atgatagtgt tggtgatgaa 960

gaactcatgg aactgttctc tgagtttggt acgataacct catgcaaggt tatgcgagat 1020

ccaaatggaa ttagtagagg atctggattt gtttcatttt caattgctga gggagcaact 1080

cgggctcttg gtgagatgaa tggtaaaatg gttgctggca aacctctcta tgttgccctt 1140

gcacagagaa aagaagatag aagagcaagg ttacaggcac aattttcaca atcgaggcct 1200

gctgcaataa cccctaatgt ttcacctcga atgcctctat accctctagg ggctcctgcg 1260

ataggacaac aatttttgta tgggcaagca gctccagcca cgatacctca acattttgtt 1320

cctggaatga ggccaggtgg tgctccaaac ttctatgttc cactggttca gcagggtcaa 1380

cagggacagc gcccaggagg acaccgagga tcaggtccta tgcaacagct ccagcagcca 1440

ttgccattga tgcaccaaca gatgcttcca aggggacatg tctatcgata tccttctggc 1500

tccaatatgc aaaatattca actggcagga gtacctggag gaatggtcac ttatggcatt 1560

ggtcaacaaa tgcccacgca ggccctggcc tcggcccttg ccaatgctac tcctgaacag 1620

cagaggacta tgcttggtga agctttatac ccgttagtag ataagctgga gcatgaagca 1680

gcagcaaagg ttactggtat gctcttagag atggatcagc ctgaggtatt acatctgatt 1740

gagtcaccgg atgctctcaa ggctaaagtt gttgaggcca tggatgtgtt gaagaaagtt 1800

acacagcagc aatcaaacag cccagccgat caactggctt cactctctct gaatgataat 1860

gtcgagtct 1869

<210> 233
<211> 623
<212> PRT

<213>  Glycine max

<400>  233

Met Ala Gln Val Leu Glu Asn Pro Thr Ile Asp Ala Ala Ala Ser Gly
1               5                   10                  15

Ala Asn Pro Ser Leu Thr Thr Ile Ser Leu Tyr Val Gly Asp Leu His
            20                  25                  30

His Asp Val Asn Asp Pro Gln Leu Tyr Asp Leu Phe Asn Gln Val Ala
        35                  40                  45

Gln Val Val Ser Val Arg Ile Cys Arg Asp Val Ala Thr Gln Gln Ser
    50                  55                  60

Leu Gly Tyr Gly Tyr Val Asn Phe Ser Asn Ala His Asp Ala Ala Lys
65                  70                  75                  80

Ala Ile Asp Val Leu Asn Phe Thr Pro Leu Asn Gly Lys Ile Ile Arg
                85                  90                  95

Ile Met Tyr Ser Ile Arg Asp Pro Ser Ala Arg Lys Ser Gly Ala Ala
            100                 105                 110

Asn Val Phe Ile Lys Asn Leu Asp Lys Ala Ile Asp His Lys Ala Leu
        115                 120                 125

Tyr Asp Thr Phe Ser Ala Phe Gly Asn Ile Leu Ser Cys Lys Val Ala
    130                 135                 140

Thr Asp Ala Ser Gly Gln Ser Lys Gly His Gly Phe Val Gln Phe Glu
145                 150                 155                 160

Ser Glu Glu Ser Ala Gln Asn Ala Ile Asp Lys Leu Asn Gly Met Leu
                165                 170                 175

Ile Asn Asp Lys Gln Val Phe Val Gly Pro Phe Leu Arg Lys Gln Asp
            180                 185                 190

Arg Glu Ser Ala Leu Ser Gly Thr Lys Phe Asn Asn Val Phe Val Lys
        195                 200                 205

Asn Leu Leu Asp Ser Met Thr Glu Ala Asp Leu Glu Arg Ile Phe Gly
    210                 215                 220

Glu Tyr Gly Ala Ile Thr Ser Ala Val Val Met Arg Asp Val Asp Gly
225                 230                 235                 240

Lys Ser Lys Gly Phe Gly Phe Val Asn Phe Ala Asn Val Asp Asp Ala
                245                 250                 255

Ala Lys Ala Val Glu Ala Leu Asn Gly Lys Asn Phe Asp Gly Lys Glu
            260                 265                 270

Trp Tyr Val Gly Lys Ala Gln Lys Lys Ser Glu Arg Glu Leu Glu Leu
        275                 280                 285

Lys Gly Gln His Glu Gln Ile Thr Lys Glu Thr Val Asp Lys Tyr His
    290                 295                 300

Gly Thr Asn Leu Tyr Ile Lys Asn Leu Asp Asp Ser Val Gly Asp Glu
305                 310                 315                 320

Glu Leu Met Glu Leu Phe Ser Glu Phe Gly Thr Ile Thr Ser Cys Lys
                325                 330                 335

Val Met Arg Asp Pro Asn Gly Ile Ser Arg Gly Ser Gly Phe Val Ser
            340                 345                 350

Phe Ser Ile Ala Glu Gly Ala Thr Arg Ala Leu Gly Glu Met Asn Gly
        355                 360                 365

Lys Met Val Ala Gly Lys Pro Leu Tyr Val Ala Leu Ala Gln Arg Lys
    370                 375                 380

Glu Asp Arg Arg Ala Arg Leu Gln Ala Gln Phe Ser Gln Ser Arg Pro
385                 390                 395                 400

Ala Ala Ile Thr Pro Asn Val Ser Pro Arg Met Pro Leu Tyr Pro Leu
                405                 410                 415

Gly Ala Pro Ala Ile Gly Gln Gln Phe Leu Tyr Gly Gln Ala Ala Pro
            420                 425                 430

Ala Thr Ile Pro Gln His Phe Val Pro Gly Met Arg Pro Gly Gly Ala
        435                 440                 445

Pro Asn Phe Tyr Val Pro Leu Val Gln Gln Gly Gln Gln Gly Gln Arg
    450                 455                 460

Pro Gly Gly His Arg Gly Ser Gly Pro Met Gln Gln Leu Gln Gln Pro
465                 470                 475                 480

Leu Pro Leu Met His Gln Gln Met Leu Pro Arg Gly His Val Tyr Arg
                485                 490                 495

Tyr Pro Ser Gly Ser Asn Met Gln Asn Ile Gln Leu Ala Gly Val Pro
                500                 505                 510

Gly Gly Met Val Thr Tyr Gly Ile Gly Gln Gln Met Pro Thr Gln Ala
        515                 520                 525

Leu Ala Ser Ala Leu Ala Asn Ala Thr Pro Glu Gln Gln Arg Thr Met
    530                 535                 540

Leu Gly Glu Ala Leu Tyr Pro Leu Val Asp Lys Leu Glu His Glu Ala
545                 550                 555                 560

Ala Ala Lys Val Thr Gly Met Leu Leu Glu Met Asp Gln Pro Glu Val
                565                 570                 575

Leu His Leu Ile Glu Ser Pro Asp Ala Leu Lys Ala Lys Val Val Glu
                580                 585                 590

Ala Met Asp Val Leu Lys Lys Val Thr Gln Gln Gln Ser Asn Ser Pro
        595                 600                 605

Ala Asp Gln Leu Ala Ser Leu Ser Leu Asn Asp Asn Val Glu Ser
    610                 615                 620

<210>  234
<211>  13
<212>  PRT
<213>  Glycine max

<400>  234

Ala Ile Asp Val Leu Asn Phe Thr Pro Leu Asn Gly Lys
1               5                   10

<210>  235
<211>  7
<212>  PRT
<213>  Glycine max

<400>  235

Phe Asn Asn Val Phe Val Lys
1               5

<210>  236
<211>  15
<212>  PRT
<213>  Glycine max

<400>  236

Gly Phe Gly Phe Val Asn Phe Ala Asn Val Asp Asp Ala Ala Lys
1               5                   10                  15

<210>  237
<211>  9
<212>  PRT
<213>  Glycine max

<400>  237

Ser Gly Ala Ala Asn Val Phe Ile Lys
1               5

<210>  238
<211>  1851
<212>  DNA
<213>  Glycine max

<400>  238
atggattctg attatggtat tcccagggaa ctctcagatc ttcaaaagat tcggtctttg      60

taccagccag agctccctcc ttgtctccag ggaaccactg tgagggttga atttggtgac     120

gcaaccacca ctgctgaccc cactgatgca ctcaccgtct gcagggcttt tcctaacact     180

tatggacacc ctttggctca ctttctcagg gccactgcca aagtccctga tgctcagatc     240

ataactgagt tccctcctat tagggtgggg attgtctttt gtgggagaca atctcctgga     300

gggcataatg tcatttgggg tatccacaat gctctcaaaa ttcacaatcc caacagtgtt     360

ttgcttggat ttcttggtgg ttcagaaggt ctatttgccc agaaaactct ggagataact     420

gacgatattc tttcgacgta caaaaatcaa ggtggttatg atttgcttgg gcggacgaag     480

gatcaaatta ggaccacaga acaagttaat gctgcacttg ctgcatgcaa caatttgaaa     540

cttgatggcc ttgtcataat cggaggggtg acatcaaaca cagatgctgc acagcttgct     600

gaaacatttg ttgtagcaaa atgccccaca aaggtggttg gtgttcctgt cactctgaat     660

ggagatctta aaaaccagtt tgtagaaact aatgttggtt ttgatacaat ttgtaaggtt     720

aattctcagc tcatcagcaa tgtctgcact gatgctctct ctgcagagaa gtattattat     780

ttcatccgtc ttatgggacg caaggcatca cacgttgctt tggaatgcac cctccagtcc     840

catccaaata tggtaattct tggtgaggag gttgctgcat caaagcttac cctttctgaa     900

attgcaaaac agattactga tgcaattcag gctagagcag agcaagacaa ataccatgga     960

gtcattctcc ttccagaagg gctgatagag agtattcctg aagtgtatgc cctcttgaag    1020

gaaattcatg gtttactcag acaaggtgtt gaagttgaca agatatcttc tcagctaaca    1080

ccatgggcat ctgctttatt tgaatttctg ccacccttta ttaggagaca gttgctcctt    1140

taccctgaat ccgacgactc agcacagtta tcacagattg aaactgagaa attactagca    1200

tatcttgtgg aagtcgagat taacaagcgt ctgaaagaag gcacatacaa ggggaagaaa    1260

ttcaatgcca tttgccactt ttttggttat caagctcgtg gatctctgcc atcaaaattt 1320

gactgtgatt acgcatatgt ccttgggcac atctgctacc atatactggc cgctggtcta 1380

aatggataca tggcaacttt aactaacctg aagaatccag taaacaagtg gaaatgtggg 1440

gctgctccaa ttgcatctat gatgactgta aagcgctggt ctccaaatcc aggagctaca 1500

tcaattggaa aacctgctat tcatccagct actgtggact tgagaggcaa agcatatgag 1560

ctgttgagac aaaaggcaca aagctttctt atggatgaca tctacagaaa tccaggtcca 1620

cttcagtttg atggtcccgg cgcagatgcc aaggttataa ctctttctgt tgaagaccag 1680

gactacatgg gccgcattaa gaaacttcag gagtaccttg aacaggttcg aaccattgtc 1740

aagccaggat gccctcagga ggttttgaaa gctgctctaa gcgtcatggc gtcggttacc 1800

gaagttctag ctgcaatgtc aacatcttat accaacaccc tggcatccct g 1851

<210> 239
<211> 617
<212> PRT
<213> Glycine max

<400> 239

Met Asp Ser Asp Tyr Gly Ile Pro Arg Glu Leu Ser Asp Leu Gln Lys
1               5                   10                  15

Ile Arg Ser Leu Tyr Gln Pro Glu Leu Pro Pro Cys Leu Gln Gly Thr
            20                  25                  30

Thr Val Arg Val Glu Phe Gly Asp Ala Thr Thr Thr Ala Asp Pro Thr
        35                  40                  45

Asp Ala Leu Thr Val Cys Arg Ala Phe Pro Asn Thr Tyr Gly His Pro
    50                  55                  60

Leu Ala His Phe Leu Arg Ala Thr Ala Lys Val Pro Asp Ala Gln Ile
65                  70                  75                  80

Ile Thr Glu Phe Pro Pro Ile Arg Val Gly Ile Val Phe Cys Gly Arg
                85                  90                  95

Gln Ser Pro Gly Gly His Asn Val Ile Trp Gly Ile His Asn Ala Leu
            100                 105                 110

Lys Ile His Asn Pro Asn Ser Val Leu Leu Gly Phe Leu Gly Gly Ser
        115                 120                 125

Glu Gly Leu Phe Ala Gln Lys Thr Leu Glu Ile Thr Asp Asp Ile Leu
    130                 135                 140

Ser Thr Tyr Lys Asn Gln Gly Gly Tyr Asp Leu Leu Gly Arg Thr Lys
145                 150                 155                 160

Asp Gln Ile Arg Thr Thr Glu Gln Val Asn Ala Ala Leu Ala Ala Cys
                165                 170                 175

Asn Asn Leu Lys Leu Asp Gly Leu Val Ile Ile Gly Gly Val Thr Ser
            180                 185                 190

Asn Thr Asp Ala Ala Gln Leu Ala Glu Thr Phe Val Val Ala Lys Cys
        195                 200                 205

Pro Thr Lys Val Val Gly Val Pro Val Thr Leu Asn Gly Asp Leu Lys
    210                 215                 220

Asn Gln Phe Val Glu Thr Asn Val Gly Phe Asp Thr Ile Cys Lys Val
225                 230                 235                 240

Asn Ser Gln Leu Ile Ser Asn Val Cys Thr Asp Ala Leu Ser Ala Glu
                245                 250                 255

Lys Tyr Tyr Tyr Phe Ile Arg Leu Met Gly Arg Lys Ala Ser His Val
            260                 265                 270

Ala Leu Glu Cys Thr Leu Gln Ser His Pro Asn Met Val Ile Leu Gly
        275                 280                 285

Glu Glu Val Ala Ala Ser Lys Leu Thr Leu Ser Glu Ile Ala Lys Gln
    290                 295                 300

Ile Thr Asp Ala Ile Gln Ala Arg Ala Glu Gln Asp Lys Tyr His Gly
305                 310                 315                 320

Val Ile Leu Leu Pro Glu Gly Leu Ile Glu Ser Ile Pro Glu Val Tyr
                325                 330                 335

Ala Leu Leu Lys Glu Ile His Gly Leu Leu Arg Gln Gly Val Glu Val
            340                 345                 350

Asp Lys Ile Ser Ser Gln Leu Thr Pro Trp Ala Ser Ala Leu Phe Glu
        355                 360                 365

Phe Leu Pro Pro Phe Ile Arg Arg Gln Leu Leu Leu Tyr Pro Glu Ser
    370                 375                 380

Asp Asp Ser Ala Gln Leu Ser Gln Ile Glu Thr Glu Lys Leu Leu Ala
385                 390                 395                 400

Tyr Leu Val Glu Val Glu Ile Asn Lys Arg Leu Lys Glu Gly Thr Tyr
                405                 410                 415

Lys Gly Lys Lys Phe Asn Ala Ile Cys His Phe Phe Gly Tyr Gln Ala
            420                 425                 430

Arg Gly Ser Leu Pro Ser Lys Phe Asp Cys Asp Tyr Ala Tyr Val Leu
        435                 440                 445

Gly His Ile Cys Tyr His Ile Leu Ala Ala Gly Leu Asn Gly Tyr Met
    450                 455                 460

Ala Thr Leu Thr Asn Leu Lys Asn Pro Val Asn Lys Trp Lys Cys Gly
465                 470                 475                 480

Ala Ala Pro Ile Ala Ser Met Met Thr Val Lys Arg Trp Ser Pro Asn
                485                 490                 495

Pro Gly Ala Thr Ser Ile Gly Lys Pro Ala Ile His Pro Ala Thr Val
            500                 505                 510

Asp Leu Arg Gly Lys Ala Tyr Glu Leu Leu Arg Gln Lys Ala Gln Ser
        515                 520                 525

Phe Leu Met Asp Asp Ile Tyr Arg Asn Pro Gly Pro Leu Gln Phe Asp
    530                 535                 540

Gly Pro Gly Ala Asp Ala Lys Val Ile Thr Leu Ser Val Glu Asp Gln
545                 550                 555                 560

Asp Tyr Met Gly Arg Ile Lys Lys Leu Gln Glu Tyr Leu Glu Gln Val
                565                 570                 575

Arg Thr Ile Val Lys Pro Gly Cys Pro Gln Glu Val Leu Lys Ala Ala
            580                 585                 590

Leu Ser Val Met Ala Ser Val Thr Glu Val Leu Ala Ala Met Ser Thr
        595                 600                 605

Ser Tyr Thr Asn Thr Leu Ala Ser Leu
    610                 615

<210>  240
<211>  13
<212>  PRT
<213>  Glycine max

<400>  240

Val Val Gly Val Pro Val Thr Leu Asn Gly Asp Leu Lys
1               5                   10


<210>  241
<211>  1575
<212>  DNA
<213>  Glycine max

<400>  241
atgcccaaat tttttcatag cattttcaga ggaacaatgg cgggtagggt ttcgacgtgc      60

ttcttcttcg tgtttgctct ttctctcctt cttccttttc agatctccgc tgaggaatca     120

tcggagaaag agttcgtact cacattggat cactctaact ttcacgacac tgtcagcaag     180

cacgatttca tcgtcgtcga gttctacgct ccatggtgtg gccactgtaa gaagcttgct     240

cccgagtatg agaaagctgc ttctatcttg agtagtcatg atcctccggt tgttttggct     300

aaaattgatg ccaatgagga gaagaacaaa gaccttgcat ctcaatatga tgttagggga     360

tacccaacaa ttaagattct gagaaatggg ggaaagaatg ttcaggaata caaaggtccc     420

cgtgaagcag atggcatcgt tgactatttg aagaaacaaa gtggtcctgc ttcaactgaa     480

attaaatctg ctgatgaagc tactgctttc attggtgaaa ataaagttgc tattgttgga     540

gttttcccga aattttctgg agaggagttt gacaactttt ctgccttagc agagaagttg     600

cgttctgact atgacttcgg tcacactctg aatgccaaac accttccaag aggagagtca     660

tcagtttctg gtcccgtagt taggttgttc aagccattcg atgaactttt tgttgacttc     720

caggatttca atgtggaagc tctagagaaa tttgttgagg aatctagtac ccctgttgtt     780

actgtcttta acaatgatcc gagcaatcac cctttcgttg ccaaattctt caacagtccc     840

aatgcaaagg caatgttgtt catcaacttc actgccgaag gtgctgaatc tttcaaatca     900

aaataccgtg aagctgctga gcaacataaa caacagggtg taagcttttt ggtgggagat     960

gttgagtcta gtcaaggtgc tttccagtat tttggactga aagaagaaca agtacctcta    1020

attatcattc aacacaatga cgggaaaaag ttttttaaac ccaatttgga agctgatcac    1080

attccaactt ggttgaaggc gtacaaggat ggtaatgttg caccatttgt gaagtctgaa    1140

cccattcctg aagctaacga tgaacctgtt aaagtggtag ttgggaacag tcttgaggac    1200

attgttttca aatctgggaa gaatgttctg ctggagtttt atgctccctg gtgtggtcat    1260

tgcaaacagt tggctccaat attggacgaa gttgctatct catatcaaag tgatgctgat    1320

gttgttattg caaaactgga tgcaactgcc aacgatatcc caagtgaaac ctttgatgtc    1380

caaggttatc caaccgtgta cttcaggtca gcaagtggaa agttatcaca atacgaaggg    1440

ggtaggacca aggaagacat catagaattc atcgaaaaga accgggacaa acctgctcag    1500

caagaacaag gacaagataa acctgctcag caagaacaag gacaagatga gcaagaaaaa    1560

ggaaaagatg agctt                                                    1575

<210>  242
<211>  525
<212>  PRT
<213>  Glycine max

<400>  242

Met Pro Lys Phe Phe His Ser Ile Phe Arg Gly Thr Met Ala Gly Arg
1               5                   10                  15

Val Ser Thr Cys Phe Phe Phe Val Phe Ala Leu Ser Leu Leu Leu Pro
            20                  25                  30

Phe Gln Ile Ser Ala Glu Glu Ser Ser Glu Lys Glu Phe Val Leu Thr
        35                  40                  45

Leu Asp His Ser Asn Phe His Asp Thr Val Ser Lys His Asp Phe Ile
    50                  55                  60

Val Val Glu Phe Tyr Ala Pro Trp Cys Gly His Cys Lys Lys Leu Ala
65                  70                  75                  80

Pro Glu Tyr Glu Lys Ala Ala Ser Ile Leu Ser Ser His Asp Pro Pro
                85                  90                  95

Val Val Leu Ala Lys Ile Asp Ala Asn Glu Glu Lys Asn Lys Asp Leu
            100                 105                 110

Ala Ser Gln Tyr Asp Val Arg Gly Tyr Pro Thr Ile Lys Ile Leu Arg
        115                 120                 125

Asn Gly Gly Lys Asn Val Gln Glu Tyr Lys Gly Pro Arg Glu Ala Asp
    130                 135                 140

Gly Ile Val Asp Tyr Leu Lys Lys Gln Ser Gly Pro Ala Ser Thr Glu
145                 150                 155                 160

Ile Lys Ser Ala Asp Glu Ala Thr Ala Phe Ile Gly Glu Asn Lys Val
                165                 170                 175

Ala Ile Val Gly Val Phe Pro Lys Phe Ser Gly Glu Glu Phe Asp Asn
            180                 185                 190

Phe Ser Ala Leu Ala Glu Lys Leu Arg Ser Asp Tyr Asp Phe Gly His
        195                 200                 205

Thr Leu Asn Ala Lys His Leu Pro Arg Gly Glu Ser Ser Val Ser Gly
    210                 215                 220

Pro Val Val Arg Leu Phe Lys Pro Phe Asp Glu Leu Phe Val Asp Phe
225                 230                 235                 240

Gln Asp Phe Asn Val Glu Ala Leu Glu Lys Phe Val Glu Glu Ser Ser
                245                 250                 255

Thr Pro Val Val Thr Val Phe Asn Asn Asp Pro Ser Asn His Pro Phe
            260                 265                 270

Val Ala Lys Phe Phe Asn Ser Pro Asn Ala Lys Ala Met Leu Phe Ile
        275                 280                 285

Asn Phe Thr Ala Glu Gly Ala Glu Ser Phe Lys Ser Lys Tyr Arg Glu
    290                 295                 300

Ala Ala Glu Gln His Lys Gln Gln Gly Val Ser Phe Leu Val Gly Asp
305                 310                 315                 320

Val Glu Ser Ser Gln Gly Ala Phe Gln Tyr Phe Gly Leu Lys Glu Glu
                325                 330                 335

Gln Val Pro Leu Ile Ile Ile Gln His Asn Asp Gly Lys Lys Phe Phe
            340                 345                 350

Lys Pro Asn Leu Glu Ala Asp His Ile Pro Thr Trp Leu Lys Ala Tyr
        355                 360                 365

Lys Asp Gly Asn Val Ala Pro Phe Val Lys Ser Glu Pro Ile Pro Glu
    370                 375                 380

Ala Asn Asp Glu Pro Val Lys Val Val Val Gly Asn Ser Leu Glu Asp
385                 390                 395                 400

Ile Val Phe Lys Ser Gly Lys Asn Val Leu Leu Glu Phe Tyr Ala Pro
                405                 410                 415

Trp Cys Gly His Cys Lys Gln Leu Ala Pro Ile Leu Asp Glu Val Ala
            420                 425                 430

Ile Ser Tyr Gln Ser Asp Ala Asp Val Val Ile Ala Lys Leu Asp Ala
        435                 440                 445

Thr Ala Asn Asp Ile Pro Ser Glu Thr Phe Asp Val Gln Gly Tyr Pro
    450                 455                 460

Thr Val Tyr Phe Arg Ser Ala Ser Gly Lys Leu Ser Gln Tyr Glu Gly
465                 470                 475                 480

Gly Arg Thr Lys Glu Asp Ile Ile Glu Phe Ile Glu Lys Asn Arg Asp
                485                 490                 495

Lys Pro Ala Gln Gln Glu Gln Gly Gln Asp Lys Pro Ala Gln Gln Glu
            500                 505                 510

Gln Gly Gln Asp Glu Gln Glu Lys Gly Lys Asp Glu Leu
        515                 520                 525

<210>  243
<211>  16
<212>  PRT
<213>  Glycine max

<400>  243

Ala Ala Ser Ile Leu Ser Ser His Asp Pro Pro Val Val Leu Ala Lys
1               5                   10                  15

<210>  244
<211>  9
<212>  PRT
<213>  Glycine max

<400>  244

Asp Gly Asn Val Ala Pro Phe Val Lys
1               5

<210>  245
<211>  15
<212>  PRT
<213>  Glycine max

<400>  245

Asp Leu Ala Ser Gln Tyr Asp Val Arg Gly Tyr Pro Thr Ile Lys
1               5                   10                  15

<210>  246
<211>  9
<212>  PRT
<213>  Glycine max

<400>  246

Glu Asp Ile Ile Glu Phe Ile Glu Lys
1               5

<210>  247
<211>  15

<212>  PRT
<213>  Glycine max

<400>  247

Glu Glu Gln Val Pro Leu Ile Ile Ile Gln His Asn Asp Gly Lys
1               5                   10                  15


<210>  248
<211>  17
<212>  PRT
<213>  Glycine max

<400>  248

Glu Phe Val Leu Thr Leu Asp His Ser Asn Phe His Asp Thr Val Ser
1               5                   10                  15


Lys


<210>  249
<211>  16
<212>  PRT
<213>  Glycine max

<400>  249

Phe Phe Lys Pro Asn Leu Glu Ala Asp His Ile Pro Thr Trp Leu Lys
1               5                   10                  15


<210>  250
<211>  8
<212>  PRT
<213>  Glycine max

<400>  250

Phe Phe Asn Ser Pro Asn Ala Lys
1               5


<210>  251
<211>  15
<212>  PRT
<213>  Glycine max

<400>  251

Phe Ser Gly Glu Glu Phe Asp Asn Phe Ser Ala Leu Ala Glu Lys
1               5                   10                  15


<210>  252
<211>  25
<212>  PRT
<213>  Glycine max

<400>  252

Phe Val Glu Glu Ser Ser Thr Pro Val Val Thr Val Phe Asn Asn Asp
1               5                   10                  15

Pro Ser Asn His Pro Phe Val Ala Lys
            20                  25

<210>  253
<211>  1509
<212>  DNA
<213>  Glycine max

<400>  253
atggcgggta cggtttcgac ttgtttcttc ttcgtgtttc ttctttctct ccttcttcct      60

tttcagatct ccgctgagga atcctcggag aaggagttcg tactcacatt ggatcactct     120

aactttcacg acactgtcag caagcacgat ttcatcgtcg tcgagttcta cgccccatgg     180

tgtggtcact gtaagaagct tgctccggag tatgagaaag ctgcttctat cttgagtagt     240

catgatcctc ctattgtttt ggctaaagta gatgccaatg aggagaagaa caaagacctt     300

gcatcacaat atgatgttaa gggattccca acaattaata ttctgagaaa tgggggaaag     360

aatgttcagg aatacaaagg tccccgtgaa gcagatggca tcgttgacta tttgaagaaa     420

caaagtggtc ctgcttcaac tgaaattaaa tctgctgatg aagctactgc ttttattggt     480

gaaaataaag ttgctattgt tggagttttc ccgaaatttt ctggagagga gtttgacaac     540

ttttctgcct tagcagagaa gttgcgttct gattatgact ttggtcacac tctgaatgcc     600

aaactccttc caagaggaga gtcatcagtt tctgggcccg tagttaggct gttcaagcca     660

ttcgatgaac tttttgttga cttccaggat ttcaatgtgg aagctctaga gaagtttgtt     720

gaggaatcta gtacccctgt tgttactgtc tttaacaatg aaccgagcaa tcaccctttc     780

gttgtcaaat tcttcaacag tcccaacgca aaggcaatgt tgttcatcaa cttcactgcc     840

gaaggtgctg aagctatcaa atcaaaatac cgtgaagctg ctgagcaata taaacaacag     900

ggtgtcagct ttttggtggg agatgttgag tctagtcaag gtgctttcca gtattttgga     960

ctgaaagaag agcaagtacc tctaattatc attcaacata atgatgggaa aaagtttttt    1020

aaacccaatt tggaagctga tcacattcca acctggttga aggcatacaa ggatggtcat    1080

gttgcaccat ttgttaagtc tgaacccatt cccgagacta acgatgaacc tgttaaagtg    1140

gtagttgggg ccagtcttga ggacattgtt ttcaaatctg ggaagaatgt tctgctggag    1200

ttttatgctc cctggtgtgg tcattgcaaa cagttggctc caatattgga tgaagttgct    1260

atctcatatc aaaatgaagc tgatgttgtt attgcaaaac tggatgcaac tgccaacgat    1320

atcccaagtg aaacctttga tgtccaaggt tatcctaccg tgtacttcag gtcagcaagt    1380

ggaaagttat cacaatacga cggcggtagg accaaggaag acatcataga attcatcgaa    1440

aagaaccggg ataaacctgc tcagcaagaa caaggaaaag atgagcaaga acaaggaaaa 1500

gatgagctt 1509

<210> 254
<211> 503
<212> PRT
<213> Glycine max

<400> 254

Met Ala Gly Thr Val Ser Thr Cys Phe Phe Phe Val Phe Leu Leu Ser
1               5                   10                  15

Leu Leu Leu Pro Phe Gln Ile Ser Ala Glu Glu Ser Ser Glu Lys Glu
            20                  25                  30

Phe Val Leu Thr Leu Asp His Ser Asn Phe His Asp Thr Val Ser Lys
        35                  40                  45

His Asp Phe Ile Val Val Glu Phe Tyr Ala Pro Trp Cys Gly His Cys
    50                  55                  60

Lys Lys Leu Ala Pro Glu Tyr Glu Lys Ala Ala Ser Ile Leu Ser Ser
65                  70                  75                  80

His Asp Pro Pro Ile Val Leu Ala Lys Val Asp Ala Asn Glu Glu Lys
                85                  90                  95

Asn Lys Asp Leu Ala Ser Gln Tyr Asp Val Lys Gly Phe Pro Thr Ile
            100                 105                 110

Asn Ile Leu Arg Asn Gly Gly Lys Asn Val Gln Glu Tyr Lys Gly Pro
        115                 120                 125

Arg Glu Ala Asp Gly Ile Val Asp Tyr Leu Lys Lys Gln Ser Gly Pro
    130                 135                 140

Ala Ser Thr Glu Ile Lys Ser Ala Asp Glu Ala Thr Ala Phe Ile Gly
145                 150                 155                 160

Glu Asn Lys Val Ala Ile Val Gly Val Phe Pro Lys Phe Ser Gly Glu
                165                 170                 175

Glu Phe Asp Asn Phe Ser Ala Leu Ala Glu Lys Leu Arg Ser Asp Tyr
            180                 185                 190

Asp Phe Gly His Thr Leu Asn Ala Lys Leu Leu Pro Arg Gly Glu Ser
        195                 200                 205

Ser Val Ser Gly Pro Val Val Arg Leu Phe Lys Pro Phe Asp Glu Leu
    210                 215                 220

Phe Val Asp Phe Gln Asp Phe Asn Val Glu Ala Leu Glu Lys Phe Val
225                 230                 235                 240

Glu Glu Ser Ser Thr Pro Val Val Thr Val Phe Asn Asn Glu Pro Ser
                245                 250                 255

Asn His Pro Phe Val Val Lys Phe Phe Asn Ser Pro Asn Ala Lys Ala
            260                 265                 270

Met Leu Phe Ile Asn Phe Thr Ala Glu Gly Ala Glu Ala Ile Lys Ser
        275                 280                 285

Lys Tyr Arg Glu Ala Ala Glu Gln Tyr Lys Gln Gln Gly Val Ser Phe
    290                 295                 300

Leu Val Gly Asp Val Glu Ser Ser Gln Gly Ala Phe Gln Tyr Phe Gly
305                 310                 315                 320

Leu Lys Glu Glu Gln Val Pro Leu Ile Ile Ile Gln His Asn Asp Gly
                325                 330                 335

Lys Lys Phe Phe Lys Pro Asn Leu Glu Ala Asp His Ile Pro Thr Trp
            340                 345                 350

Leu Lys Ala Tyr Lys Asp Gly His Val Ala Pro Phe Val Lys Ser Glu
        355                 360                 365

Pro Ile Pro Glu Thr Asn Asp Glu Pro Val Lys Val Val Val Gly Ala
    370                 375                 380

Ser Leu Glu Asp Ile Val Phe Lys Ser Gly Lys Asn Val Leu Leu Glu
385                 390                 395                 400

Phe Tyr Ala Pro Trp Cys Gly His Cys Lys Gln Leu Ala Pro Ile Leu
                405                 410                 415

Asp Glu Val Ala Ile Ser Tyr Gln Asn Glu Ala Asp Val Val Ile Ala
            420                 425                 430

Lys Leu Asp Ala Thr Ala Asn Asp Ile Pro Ser Glu Thr Phe Asp Val
        435                 440                 445

Gln Gly Tyr Pro Thr Val Tyr Phe Arg Ser Ala Ser Gly Lys Leu Ser
    450                 455                 460

Gln Tyr Asp Gly Gly Arg Thr Lys Glu Asp Ile Ile Glu Phe Ile Glu
465                 470                 475                 480

Lys Asn Arg Asp Lys Pro Ala Gln Gln Glu Gln Gly Lys Asp Glu Gln
                485                 490                 495

Glu Gln Gly Lys Asp Glu Leu
            500

<210>  255
<211>  16
<212>  PRT
<213>  Glycine max

<400>  255

Ala Ala Ser Ile Leu Ser Ser His Asp Pro Pro Ile Val Leu Ala Lys
1               5                   10                  15

<210>  256
<211>  9
<212>  PRT
<213>  Glycine max

<400>  256

Asp Gly His Val Ala Pro Phe Val Lys
1               5

<210>  257
<211>  9
<212>  PRT
<213>  Glycine max

<400>  257

Asp Leu Ala Ser Gln Tyr Asp Val Lys
1               5

<210>  258
<211>  9
<212>  PRT
<213>  Glycine max

<400>  258

Glu Asp Ile Ile Glu Phe Ile Glu Lys
1               5

<210>  259
<211>  15
<212>  PRT
<213>  Glycine max

<400>  259

Glu Glu Gln Val Pro Leu Ile Ile Ile Gln His Asn Asp Gly Lys
1               5                   10                  15


<210>  260
<211>  17
<212>  PRT
<213>  Glycine max

<400>  260

Glu Phe Val Leu Thr Leu Asp His Ser Asn Phe His Asp Thr Val Ser
1               5                   10                  15


Lys



<210>  261
<211>  16
<212>  PRT
<213>  Glycine max

<400>  261

Phe Phe Lys Pro Asn Leu Glu Ala Asp His Ile Pro Thr Trp Leu Lys
1               5                   10                  15


<210>  262
<211>  8
<212>  PRT
<213>  Glycine max

<400>  262

Phe Phe Asn Ser Pro Asn Ala Lys
1               5


<210>  263
<211>  15
<212>  PRT
<213>  Glycine max

<400>  263

Phe Ser Gly Glu Glu Phe Asp Asn Phe Ser Ala Leu Ala Glu Lys
1               5                   10                  15


<210>  264
<211>  25
<212>  PRT
<213>  Glycine max

<400>  264

Phe Val Glu Glu Ser Ser Thr Pro Val Val Thr Val Phe Asn Asn Glu
1               5                   10                  15

Pro Ser Asn His Pro Phe Val Val Lys
            20                  25

<210>  265
<211>  13
<212>  PRT
<213>  Glycine max

<400>  265

Gly Phe Pro Thr Ile Asn Ile Leu Arg Asn Gly Gly Lys
1               5                   10

<210>  266
<211>  13
<212>  PRT
<213>  Glycine max

<400>  266

Gly Pro Arg Glu Ala Asp Gly Ile Val Asp Tyr Leu Lys
1               5                   10

<210>  267
<211>  7
<212>  PRT
<213>  Glycine max

<400>  267

Leu Ala Pro Glu Tyr Glu Lys
1               5

<210>  268
<211>  29
<212>  PRT
<213>  Glycine max

<400>  268

Leu Asp Ala Thr Ala Asn Asp Ile Pro Ser Glu Thr Phe Asp Val Gln
1               5                   10                  15

Gly Tyr Pro Thr Val Tyr Phe Arg Ser Ala Ser Gly Lys
            20                  25

<210>  269
<211>  14
<212>  PRT
<213>  Glycine max

<400>  269

Leu Arg Ser Asp Tyr Asp Phe Gly His Thr Leu Asn Ala Lys
1               5                   10

<210>  270
<211>  10
<212>  PRT
<213>  Glycine max

<400>  270

Leu Ser Gln Tyr Asp Gly Gly Arg Thr Lys
1               5                   10


<210>  271
<211>  23
<212>  PRT
<213>  Glycine max

<400>  271

Gln Leu Ala Pro Ile Leu Asp Glu Val Ala Ile Ser Tyr Gln Asn Glu
1               5                   10                  15


Ala Asp Val Val Ile Ala Lys
            20


<210>  272
<211>  24
<212>  PRT
<213>  Glycine max

<400>  272

Gln Gln Gly Val Ser Phe Leu Val Gly Asp Val Glu Ser Ser Gln Gly
1               5                   10                  15


Ala Phe Gln Tyr Phe Gly Leu Lys
            20


<210>  273
<211>  10
<212>  PRT
<213>  Glycine max

<400>  273

Gln Ser Gly Pro Ala Ser Thr Glu Ile Lys
1               5                   10


<210>  274
<211>  13
<212>  PRT
<213>  Glycine max

<400>  274

Ser Ala Asp Glu Ala Thr Ala Phe Ile Gly Glu Asn Lys
1               5                   10

<210>  275
<211>  13
<212>  PRT
<213>  Glycine max

<400>  275

Ser Glu Pro Ile Pro Glu Thr Asn Asp Glu Pro Val Lys
1               5                   10


<210>  276
<211>  9
<212>  PRT
<213>  Glycine max

<400>  276

Val Ala Ile Val Gly Val Phe Pro Lys
1               5


<210>  277
<211>  13
<212>  PRT
<213>  Glycine max

<400>  277

Val Val Val Gly Ala Ser Leu Glu Asp Ile Val Phe Lys
1               5                   10


<210>  278
<211>  9
<212>  PRT
<213>  Glycine max

<400>  278

Tyr Arg Glu Ala Ala Glu Gln Tyr Lys
1               5


<210>  279
<211>  1467
<212>  DNA
<213>  Glycine max

<400>  279
atgggtgtgg ctcaaaacat tcccgatgcg gaggagggaa ccttggagat tggaatggaa      60

tacagaactg tttctggagt tgctgggcca ttggtcattc ttgataaagt taagggaccc     120

aagtttcaag agattgttaa cattcgctta ggagatggta ctactcggcg tggacaagtg     180

ctagaagttg atggtgaaaa ggctgttgtt caggtctttg agggtacatc tgggattgac     240

aataaattta caactgtgca atttactgga gaggtgttaa aaactccagt atcattggac     300

atgcttggcc gcatattcaa tggttctgga aaaccaattg ataatggtcc acccatttta     360

cctgaggctt acttggacat atcaggaagt tccatcaacc ctagtgagag aacctatcca 420

gaggagatga tacagacagg aatatctacc attgatgtca tgaattctat tgctagaggt 480

caaaagatcc ctctattctc agctgctggt ctcccccata atgaaattgc tgcacagata 540

tgccgtcagg ctggcttagt caagcgacta gaaaagtctg ataatcttct cgagggtgga 600

ggagaagagg acaattttgc cattgtgttt gcagctatgg gagttaacat ggagactgca 660

cagtttttca aacgtgactt tgaggaaaat ggctcgatgg agagagtgac ccttttcctt 720

aatctggcaa atgatcctac gattgagcgt atcatcactc ctcgtattgc tcttactact 780

gctgaatatt tggcatatga atgtggcaag catgttcttg tgatactaac tgatatgagt 840

tcttatgctg atgctcttcg tgaggtatct gctgcccgag aagaagtacc tggtagacgt 900

gggtatcccg gctacatgta tacagatctt gcaacaatat acgaacgtgc tggaagaatt 960

gaggggcgaa aaggctctat tacacaaatt ccaattctaa ccatgcctaa tgatgatatt 1020

acgcatccaa ctcctgatct tacaggatat attactgagg gacagatata tatcgacaga 1080

cagttgtata acaggcagat atatcctcca atcaatgtcc ttccatcatt gtctcgtttg 1140

atgaagagtg ccattggtga gggaatgaca cgaaaggatc attctgatgt gtccaaccag 1200

ctctatgcaa attatgccat tggaaaggat gtccaggcaa tgaaagccgt ggtcggagaa 1260

gaagcacttt catctgagga cctgctgtat ttggaatttt tggagaagtt tgagaggaag 1320

tttgttgccc aaggagcata cgatacacgc aatatcttcc agtcactaga tcttgcatgg 1380

actttgcttc ggattttccc tcgcgagctt ctccaccgta ttcctgcaaa gactcttgat 1440

cagttctaca gccgagatgc cagtaac 1467

<210> 280
<211> 489
<212> PRT
<213> Glycine max

<400> 280

Met Gly Val Ala Gln Asn Ile Pro Asp Ala Glu Glu Gly Thr Leu Glu
1               5                   10                  15

Ile Gly Met Glu Tyr Arg Thr Val Ser Gly Val Ala Gly Pro Leu Val
            20                  25                  30

Ile Leu Asp Lys Val Lys Gly Pro Lys Phe Gln Glu Ile Val Asn Ile
        35                  40                  45

Arg Leu Gly Asp Gly Thr Thr Arg Arg Gly Gln Val Leu Glu Val Asp
    50                  55                  60

Gly Glu Lys Ala Val Val Gln Val Phe Glu Gly Thr Ser Gly Ile Asp
65                  70                  75                  80

Asn Lys Phe Thr Thr Val Gln Phe Thr Gly Glu Val Leu Lys Thr Pro
                85                  90                  95

Val Ser Leu Asp Met Leu Gly Arg Ile Phe Asn Gly Ser Gly Lys Pro
            100                 105                 110

Ile Asp Asn Gly Pro Pro Ile Leu Pro Glu Ala Tyr Leu Asp Ile Ser
        115                 120                 125

Gly Ser Ser Ile Asn Pro Ser Glu Arg Thr Tyr Pro Glu Glu Met Ile
    130                 135                 140

Gln Thr Gly Ile Ser Thr Ile Asp Val Met Asn Ser Ile Ala Arg Gly
145                 150                 155                 160

Gln Lys Ile Pro Leu Phe Ser Ala Ala Gly Leu Pro His Asn Glu Ile
                165                 170                 175

Ala Ala Gln Ile Cys Arg Gln Ala Gly Leu Val Lys Arg Leu Glu Lys
            180                 185                 190

Ser Asp Asn Leu Leu Glu Gly Gly Gly Glu Glu Asp Asn Phe Ala Ile
        195                 200                 205

Val Phe Ala Ala Met Gly Val Asn Met Glu Thr Ala Gln Phe Phe Lys
    210                 215                 220

Arg Asp Phe Glu Glu Asn Gly Ser Met Glu Arg Val Thr Leu Phe Leu
225                 230                 235                 240

Asn Leu Ala Asn Asp Pro Thr Ile Glu Arg Ile Ile Thr Pro Arg Ile
                245                 250                 255

Ala Leu Thr Thr Ala Glu Tyr Leu Ala Tyr Glu Cys Gly Lys His Val
            260                 265                 270

Leu Val Ile Leu Thr Asp Met Ser Ser Tyr Ala Asp Ala Leu Arg Glu
        275                 280                 285

Val Ser Ala Ala Arg Glu Glu Val Pro Gly Arg Arg Gly Tyr Pro Gly
    290                 295                 300

Tyr Met Tyr Thr Asp Leu Ala Thr Ile Tyr Glu Arg Ala Gly Arg Ile
305                 310                 315                 320

Glu Gly Arg Lys Gly Ser Ile Thr Gln Ile Pro Ile Leu Thr Met Pro
                325                 330                 335

Asn Asp Asp Ile Thr His Pro Thr Pro Asp Leu Thr Gly Tyr Ile Thr
            340                 345                 350

Glu Gly Gln Ile Tyr Ile Asp Arg Gln Leu Tyr Asn Arg Gln Ile Tyr
        355                 360                 365

Pro Pro Ile Asn Val Leu Pro Ser Leu Ser Arg Leu Met Lys Ser Ala
    370                 375                 380

Ile Gly Glu Gly Met Thr Arg Lys Asp His Ser Asp Val Ser Asn Gln
385                 390                 395                 400

Leu Tyr Ala Asn Tyr Ala Ile Gly Lys Asp Val Gln Ala Met Lys Ala
                405                 410                 415

Val Val Gly Glu Glu Ala Leu Ser Ser Glu Asp Leu Leu Tyr Leu Glu
            420                 425                 430

Phe Leu Glu Lys Phe Glu Arg Lys Phe Val Ala Gln Gly Ala Tyr Asp
        435                 440                 445

Thr Arg Asn Ile Phe Gln Ser Leu Asp Leu Ala Trp Thr Leu Leu Arg
    450                 455                 460

Ile Phe Pro Arg Glu Leu Leu His Arg Ile Pro Ala Lys Thr Leu Asp
465                 470                 475                 480

Gln Phe Tyr Ser Arg Asp Ala Ser Asn
                485

<210>  281
<211>  17
<212>  PRT
<213>  Glycine max

<400>  281

Asp His Ser Asp Val Ser Asn Gln Leu Tyr Ala Asn Tyr Ala Ile Gly
1               5                   10                  15

Lys

<210>  282
<211>  26
<212>  PRT

<213>  Glycine max

<400>  282

Phe Gln Glu Ile Val Asn Ile Arg Leu Gly Asp Gly Thr Thr Arg Arg
1               5                   10                  15

Gly Gln Val Leu Glu Val Asp Gly Glu Lys
            20                  25

<210>  283
<211>  12
<212>  PRT
<213>  Glycine max

<400>  283

Phe Thr Thr Val Gln Phe Thr Gly Glu Val Leu Lys
1               5                   10

<210>  284
<211>  12
<212>  PRT
<213>  Glycine max

<400>  284

Thr Leu Asp Gln Phe Tyr Ser Arg Asp Ala Ser Asn
1               5                   10

<210>  285
<211>  1464
<212>  DNA
<213>  Glycine max

<400>  285
atgggtgtgg ctgagaacat tcccgatatg gaggagggta ccttggagat tggaatggaa      60

tacagaactg tatctggagt tgctggacca ttggtcattc ttgacaaagt taagggaccc     120

aaatttcaag agattgttaa tattcgttta ggagatggaa ctactcggcg tggacaagtt     180

ctagaggttg atggcgaaaa ggctgttgtt caggtatttg aaggcacatc tgggatcgac     240

aataagttta caactgtgca atttactgga gaggtgttaa aaactcctgt ctcactggac     300

atgcttggac gcatatttaa tggttctgga aaaccaattg ataatggtcc accaatttta     360

cctgaggctt acctggatat ttctggaagt tccatcaatc ctagtgagag aacctatcct     420

gaggagatga tacagacagg aatatctaca attgatgtca tgaattctat tgctagaggt     480

caaaagatcc ctctattctc agcagctggt ctcccccata atgaaattgc tgcacagatc     540

tgtcgtcagg ctggcttagt caagcgacta gagaaatctg ataatcttct tgagggtgga     600

gaagaggaca attttgctat tgtgtttgca gctatgggag ttaacatgga gactgcacag     660

tttttcaagc gtgactttga ggagaatgga tcaatggaga gagtgactct tttccttaat     720

ttggcaaatg atcctacaat tgagcgtatt atcactcctc gtattgctct tactactgct 780

gaatatttgg cttatgaatg tggcaagcac gttcttgtga tactcacaga catgagttct 840

tatgctgatg ctcttcgtga ggtatctgct gcccgagaag aagtgccggg tagacgtggt 900

tatcctggat atatgtatac agatcttgca acaatttatg aacgagctgg gagaattgag 960

gggcgtaaag gctctattac acaaattcca attctaacca tgcccaatga tgatattacg 1020

cacccaactc ctgatctaac aggatatatc actgaggggc agatatacat cgacaggcag 1080

ctgtataata gacagatata cccaccaatc aacgtcctcc catcactatc tcggttgatg 1140

aagagtgcta ttggtgaggg aatgactaga agggatcatt ctgatgtgtc caaccagctc 1200

tatgcaaatt atgctattgg aaaggatgtc caggcaatga aagcagtggt tggagaagaa 1260

gcactttcat ctgaggacct gctgtatctg gaatttttgg agaagtttga gaggaagttt 1320

gttgcccagg gagcctatga cacccgcaat atattccagt cacttgatct tgcatggact 1380

ttactccgga ttttccctcg tgaacttctt catcgtatac cagccaagac tcttgaccag 1440

ttttacagca gagatgctgg taat 1464

<210> 286
<211> 488
<212> PRT
<213> Glycine max

<400> 286

Met Gly Val Ala Glu Asn Ile Pro Asp Met Glu Glu Gly Thr Leu Glu
1               5                   10                  15

Ile Gly Met Glu Tyr Arg Thr Val Ser Gly Val Ala Gly Pro Leu Val
            20                  25                  30

Ile Leu Asp Lys Val Lys Gly Pro Lys Phe Gln Glu Ile Val Asn Ile
        35                  40                  45

Arg Leu Gly Asp Gly Thr Thr Arg Arg Gly Gln Val Leu Glu Val Asp
    50                  55                  60

Gly Glu Lys Ala Val Val Gln Val Phe Glu Gly Thr Ser Gly Ile Asp
65                  70                  75                  80

Asn Lys Phe Thr Thr Val Gln Phe Thr Gly Glu Val Leu Lys Thr Pro
                85                  90                  95

Val Ser Leu Asp Met Leu Gly Arg Ile Phe Asn Gly Ser Gly Lys Pro
            100                 105                 110

Ile Asp Asn Gly Pro Pro Ile Leu Pro Glu Ala Tyr Leu Asp Ile Ser
        115                 120                 125

Gly Ser Ser Ile Asn Pro Ser Glu Arg Thr Tyr Pro Glu Glu Met Ile
    130                 135                 140

Gln Thr Gly Ile Ser Thr Ile Asp Val Met Asn Ser Ile Ala Arg Gly
145                 150                 155                 160

Gln Lys Ile Pro Leu Phe Ser Ala Ala Gly Leu Pro His Asn Glu Ile
                165                 170                 175

Ala Ala Gln Ile Cys Arg Gln Ala Gly Leu Val Lys Arg Leu Glu Lys
            180                 185                 190

Ser Asp Asn Leu Leu Glu Gly Gly Glu Glu Asp Asn Phe Ala Ile Val
        195                 200                 205

Phe Ala Ala Met Gly Val Asn Met Glu Thr Ala Gln Phe Phe Lys Arg
    210                 215                 220

Asp Phe Glu Glu Asn Gly Ser Met Glu Arg Val Thr Leu Phe Leu Asn
225                 230                 235                 240

Leu Ala Asn Asp Pro Thr Ile Glu Arg Ile Ile Thr Pro Arg Ile Ala
                245                 250                 255

Leu Thr Thr Ala Glu Tyr Leu Ala Tyr Glu Cys Gly Lys His Val Leu
            260                 265                 270

Val Ile Leu Thr Asp Met Ser Ser Tyr Ala Asp Ala Leu Arg Glu Val
        275                 280                 285

Ser Ala Ala Arg Glu Glu Val Pro Gly Arg Arg Gly Tyr Pro Gly Tyr
    290                 295                 300

Met Tyr Thr Asp Leu Ala Thr Ile Tyr Glu Arg Ala Gly Arg Ile Glu
305                 310                 315                 320

Gly Arg Lys Gly Ser Ile Thr Gln Ile Pro Ile Leu Thr Met Pro Asn
                325                 330                 335

Asp Asp Ile Thr His Pro Thr Pro Asp Leu Thr Gly Tyr Ile Thr Glu
            340                 345                 350

Gly Gln Ile Tyr Ile Asp Arg Gln Leu Tyr Asn Arg Gln Ile Tyr Pro
        355                 360                 365

Pro Ile Asn Val Leu Pro Ser Leu Ser Arg Leu Met Lys Ser Ala Ile
    370                 375                 380

Gly Glu Gly Met Thr Arg Arg Asp His Ser Asp Val Ser Asn Gln Leu
385                 390                 395                 400

Tyr Ala Asn Tyr Ala Ile Gly Lys Asp Val Gln Ala Met Lys Ala Val
                405                 410                 415

Val Gly Glu Glu Ala Leu Ser Ser Glu Asp Leu Leu Tyr Leu Glu Phe
            420                 425                 430

Leu Glu Lys Phe Glu Arg Lys Phe Val Ala Gln Gly Ala Tyr Asp Thr
        435                 440                 445

Arg Asn Ile Phe Gln Ser Leu Asp Leu Ala Trp Thr Leu Leu Arg Ile
    450                 455                 460

Phe Pro Arg Glu Leu Leu His Arg Ile Pro Ala Lys Thr Leu Asp Gln
465                 470                 475                 480

Phe Tyr Ser Arg Asp Ala Gly Asn
                485

<210>  287
<211>  26
<212>  PRT
<213>  Glycine max

<400>  287

Phe Gln Glu Ile Val Asn Ile Arg Leu Gly Asp Gly Thr Thr Arg Arg
1               5                   10                  15

Gly Gln Val Leu Glu Val Asp Gly Glu Lys
            20                  25

<210>  288
<211>  12
<212>  PRT
<213>  Glycine max

<400>  288

Phe Thr Thr Val Gln Phe Thr Gly Glu Val Leu Lys
1               5                   10

<210>  289
<211>  12
<212>  PRT
<213>  Glycine max

<400>  289

Thr Leu Asp Gln Phe Tyr Ser Arg Asp Ala Gly Asn
1               5                   10


<210>  290
<211>  1407
<212>  DNA
<213>  Glycine max

<400>  290
atggccaccg ctgccgagaa actctccgct ctcaaatccg ccgtcgccgg attgaacgaa      60

atcagtgaga atgagaagaa cggattcatc agcctcgtcg gccgctatct cagtggcgaa     120

gcgcagcatg tggaatggag caagatccag acgcctacgg acgaagtggt tgtgccttac     180

gacactttgg cgccaactcc tgaaggttct tcggaggtga agaatctatt ggacaagctt     240

gtggtgttga agctaaatgg aggcttggga acaactatgg gttgcactgg tcctaaatct     300

gtaattgaag ttcgtgatgg gttgacattt ctagatttaa ttgtcatcca aattgagaat     360

ctcaattcca aatatggaag caatgttcct ttgcttttga tgaattcatt caacactcat     420

gatgacactc aaaagattgt tgaaaaatac caaaactcaa atattgagat tcatactttt     480

aaccagagcc agtatcctcg attggttgtt gaggactttt tgccattgcc ttccaaaggg     540

catactgaca aggatggatg gtaccctcct ggccatggtg atgtcttccc atcattattg     600

aacagtggca aacttgatgc actattgtca cagggtaaag agtatgtgtt tgttgccaat     660

tcggataact tgggagctat agttgacttg aaaatcttga atcatttgat ccagaacaag     720

aatgaatact gtatggaggt gactcccaaa acattggctg atgtaaaggg tggcactttg     780

atttcttacg aaggaagggt tcagctcctg gaaattgcac aagtccccga tgaacatgtc     840

aatgagttca agtcaataga gaagttcaaa attttcaaca caaataattt gtgggtgaac     900

ttaaacgcag ttaaaaggct tgttgaagct gatgctctta agatggaaat tattcccaat     960

ccaaaggaag ttgacggaat aaaagttctt cagctggaaa ctgcagctgg tgctgcaata    1020

aggttctttg acaaggctat tgggattaat gttcctcgat cacgattcct tcctgtgaag    1080

gcaacttcag atttgcttct tgtccagtct gacctctaca ctttggaaga cggatttgtc    1140

attcggaaca aagctaggga aaatcctgaa aacccttcta ttgaactggg accagaattt    1200

aagaaggtta gcaacttctt gggccgcttc aagtcaattc ctagtatcgt tgagcttgac    1260

agtctaaaag tggctggtga tgtatggttt ggagctggtg ttatcctcaa ggggaaagtc    1320

agcattgtat caaagcccgg tgtaaagctg gaagttcccg acggtgttgc cattgtagac    1380

aaggaaatta atggcccaga ggacctg                                        1407


<210>  291

<211>  469
<212>  PRT
<213>  Glycine max

<400>  291

Met Ala Thr Ala Ala Glu Lys Leu Ser Ala Leu Lys Ser Ala Val Ala
1               5                   10                  15

Gly Leu Asn Glu Ile Ser Glu Asn Glu Lys Asn Gly Phe Ile Ser Leu
            20                  25                  30

Val Gly Arg Tyr Leu Ser Gly Glu Ala Gln His Val Glu Trp Ser Lys
        35                  40                  45

Ile Gln Thr Pro Thr Asp Glu Val Val Val Pro Tyr Asp Thr Leu Ala
    50                  55                  60

Pro Thr Pro Glu Gly Ser Ser Glu Val Lys Asn Leu Leu Asp Lys Leu
65                  70                  75                  80

Val Val Leu Lys Leu Asn Gly Gly Leu Gly Thr Thr Met Gly Cys Thr
                85                  90                  95

Gly Pro Lys Ser Val Ile Glu Val Arg Asp Gly Leu Thr Phe Leu Asp
            100                 105                 110

Leu Ile Val Ile Gln Ile Glu Asn Leu Asn Ser Lys Tyr Gly Ser Asn
        115                 120                 125

Val Pro Leu Leu Leu Met Asn Ser Phe Asn Thr His Asp Asp Thr Gln
    130                 135                 140

Lys Ile Val Glu Lys Tyr Gln Asn Ser Asn Ile Glu Ile His Thr Phe
145                 150                 155                 160

Asn Gln Ser Gln Tyr Pro Arg Leu Val Val Glu Asp Phe Leu Pro Leu
                165                 170                 175

Pro Ser Lys Gly His Thr Asp Lys Asp Gly Trp Tyr Pro Pro Gly His
            180                 185                 190

Gly Asp Val Phe Pro Ser Leu Leu Asn Ser Gly Lys Leu Asp Ala Leu
        195                 200                 205

Leu Ser Gln Gly Lys Glu Tyr Val Phe Val Ala Asn Ser Asp Asn Leu
    210                 215                 220

Gly Ala Ile Val Asp Leu Lys Ile Leu Asn His Leu Ile Gln Asn Lys

225                 230                 235                 240

Asn Glu Tyr Cys Met Glu Val Thr Pro Lys Thr Leu Ala Asp Val Lys
                245                 250                 255

Gly Gly Thr Leu Ile Ser Tyr Glu Gly Arg Val Gln Leu Leu Glu Ile
            260                 265                 270

Ala Gln Val Pro Asp Glu His Val Asn Glu Phe Lys Ser Ile Glu Lys
        275                 280                 285

Phe Lys Ile Phe Asn Thr Asn Asn Leu Trp Val Asn Leu Asn Ala Val
    290                 295                 300

Lys Arg Leu Val Glu Ala Asp Ala Leu Lys Met Glu Ile Ile Pro Asn
305                 310                 315                 320

Pro Lys Glu Val Asp Gly Ile Lys Val Leu Gln Leu Glu Thr Ala Ala
                325                 330                 335

Gly Ala Ala Ile Arg Phe Phe Asp Lys Ala Ile Gly Ile Asn Val Pro
            340                 345                 350

Arg Ser Arg Phe Leu Pro Val Lys Ala Thr Ser Asp Leu Leu Leu Val
        355                 360                 365

Gln Ser Asp Leu Tyr Thr Leu Glu Asp Gly Phe Val Ile Arg Asn Lys
    370                 375                 380

Ala Arg Glu Asn Pro Glu Asn Pro Ser Ile Glu Leu Gly Pro Glu Phe
385                 390                 395                 400

Lys Lys Val Ser Asn Phe Leu Gly Arg Phe Lys Ser Ile Pro Ser Ile
                405                 410                 415

Val Glu Leu Asp Ser Leu Lys Val Ala Gly Asp Val Trp Phe Gly Ala
            420                 425                 430

Gly Val Ile Leu Lys Gly Lys Val Ser Ile Val Ser Lys Pro Gly Val
        435                 440                 445

Lys Leu Glu Val Pro Asp Gly Val Ala Ile Val Asp Lys Glu Ile Asn
    450                 455                 460

Gly Pro Glu Asp Leu
465

<210>  292
<211>  15
<212>  PRT
<213>  Glycine max

<400>  292

Ala Ile Gly Ile Asn Val Pro Arg Ser Arg Phe Leu Pro Val Lys
1               5                   10                  15


<210>  293
<211>  17
<212>  PRT
<213>  Glycine max

<400>  293

Ala Arg Glu Asn Pro Glu Asn Pro Ser Ile Glu Leu Gly Pro Glu Phe
1               5                   10                  15


Lys


<210>  294
<211>  24
<212>  PRT
<213>  Glycine max

<400>  294

Ala Thr Ser Asp Leu Leu Leu Val Gln Ser Asp Leu Tyr Thr Leu Glu
1               5                   10                  15


Asp Gly Phe Val Ile Arg Asn Lys
            20


<210>  295
<211>  8
<212>  PRT
<213>  Glycine max

<400>  295

Glu Ile Asn Gly Pro Glu Asp Leu
1               5


<210>  296
<211>  18
<212>  PRT
<213>  Glycine max

<400>  296

Glu Tyr Val Phe Val Ala Asn Ser Asp Asn Leu Gly Ala Ile Val Asp
1               5                   10                  15

Leu Lys

<210> 297
<211> 9
<212> PRT
<213> Glycine max

<400> 297

Ile Leu Asn His Leu Ile Gln Asn Lys
1               5

<210> 298
<211> 26
<212> PRT
<213> Glycine max

<400> 298

Ile Gln Thr Pro Thr Asp Glu Val Val Val Pro Tyr Asp Thr Leu Ala
1               5                   10                  15

Pro Thr Pro Glu Gly Ser Ser Glu Val Lys
            20                  25

<210> 299
<211> 9
<212> PRT
<213> Glycine max

<400> 299

Leu Asp Ala Leu Leu Ser Gln Gly Lys
1               5

<210> 300
<211> 9
<212> PRT
<213> Glycine max

<400> 300

Arg Leu Val Glu Ala Asp Ala Leu Lys
1               5

<210> 301
<211> 14
<212> PRT
<213> Glycine max

<400> 301

Ser Ala Val Ala Gly Leu Asn Glu Ile Ser Glu Asn Glu Lys
1               5                   10

<210>  302
<211>  12
<212>  PRT
<213>  Glycine max

<400>  302

Ser Ile Pro Ser Ile Val Glu Leu Asp Ser Leu Lys
1               5                   10


<210>  303
<211>  17
<212>  PRT
<213>  Glycine max

<400>  303

Val Leu Gln Leu Glu Thr Ala Ala Gly Ala Ala Ile Arg Phe Phe Asp
1               5                   10                  15


Lys


<210>  304
<211>  9
<212>  PRT
<213>  Glycine max

<400>  304

Val Ser Asn Phe Leu Gly Arg Phe Lys
1               5


<210>  305
<211>  1353
<212>  DNA
<213>  Glycine max

<400>  305
atgaccaccg ccaccgagaa gctctccgct ctcaaatccg ccgtcgccgg attgaacgaa      60

atcagtgaga gtgagaagaa cggattcatc agcctcgtca gccgctatct cagtggcgaa     120

gcgcagcatg tggaatggag caagatccag acgcctacgg acgaagtggt tgtgccttac     180

gacactttgg cgccaactcc tgatggttct tcggacgtga agaatctatt ggacaagctt     240

gtggtgttga agctaaatgg aggcttgggc acaactatgg gttgcactgg tcctaaatct     300

gtaattgaag ttcgtgatgg gttgacattt ctagatttaa ttgtgatcca gattgagaat     360

ctcaattcca aatatggaag caatgttcct ttgcttttga tgaattcatt caacactcat     420

gatgacactc aaaagattgt tgaaaaatac caaaactcca atattgagat tcatactttt     480

aaccagggca tactgacaag gatggatggt accctcctgg cccatggaga tgtctttcca     540

tcattattga acagtggcaa acttgatgca ctattgtcac agggtaaaga gtatgtattt     600

gttgccaatt cagataactt gggagctata gttgacttga aaatcttaaa tcatttgatc 660

cagaacaaga atgaatactg tatggaggtg actcccaaaa cattggctga tgtaaagggt 720

ggcactttga tttcttacga aggaaggctt ttggaaattg cacaagtccc agatgaacat 780

gtcaatgagt tcaagtcaat agagaagttc aaaattttca acacaaataa tttgtgggtg 840

aacttaaatg cagttaaaag gcttgttgaa gctgatgctc ttaagatgga aattattccc 900

aatccaaagg aagttgatgg aataaaagtt cttcagctgg aaactgcagc tggtgctgca 960

ataaggttct ttgacaaggc tattgggatt aatgttcctc gatcacgatt ccttcctgtg 1020

aaggcaactt cagatttgct tcttgtccag tctgacctct acactttgga agatggattt 1080

gtcattcgga acaaagctag ggcaaatcct gaaaaccctt ctattgaact tgggccagaa 1140

tttaagaagg ttagcaactt cttgggccgc ttcaagtcaa ttcccagtat tgttgagctt 1200

gacagtctaa aagtggctgg caatgtatgg tttggagctg gtgttatcct caagggaaaa 1260

atcagtatcg tggccaatcc tggtgttaag ctggaagttc ccgatggtgc tgtcatttcg 1320

gataaggaaa ttaatggccc agaggacctc ctg 1353

<210> 306
<211> 451
<212> PRT
<213> Glycine max

<400> 306

Met Thr Thr Ala Thr Glu Lys Leu Ser Ala Leu Lys Ser Ala Val Ala
1               5                   10                  15

Gly Leu Asn Glu Ile Ser Glu Ser Glu Lys Asn Gly Phe Ile Ser Leu
            20                  25                  30

Val Ser Arg Tyr Leu Ser Gly Glu Ala Gln His Val Glu Trp Ser Lys
        35                  40                  45

Ile Gln Thr Pro Thr Asp Glu Val Val Val Pro Tyr Asp Thr Leu Ala
    50                  55                  60

Pro Thr Pro Asp Gly Ser Ser Asp Val Lys Asn Leu Leu Asp Lys Leu
65                  70                  75                  80

Val Val Leu Lys Leu Asn Gly Gly Leu Gly Thr Thr Met Gly Cys Thr
                85                  90                  95

Gly Pro Lys Ser Val Ile Glu Val Arg Asp Gly Leu Thr Phe Leu Asp
            100                 105                 110

Leu Ile Val Ile Gln Ile Glu Asn Leu Asn Ser Lys Tyr Gly Ser Asn

115                 120                 125

Val Pro Leu Leu Leu Met Asn Ser Phe Asn Thr His Asp Asp Thr Gln
    130                 135                 140

Lys Ile Val Glu Lys Tyr Gln Asn Ser Asn Ile Glu Ile His Thr Phe
145                 150                 155                 160

Asn Gln Gly Ile Leu Thr Arg Met Asp Gly Thr Leu Leu Ala His Gly
                165                 170                 175

Asp Val Phe Pro Ser Leu Leu Asn Ser Gly Lys Leu Asp Ala Leu Leu
            180                 185                 190

Ser Gln Gly Lys Glu Tyr Val Phe Val Ala Asn Ser Asp Asn Leu Gly
        195                 200                 205

Ala Ile Val Asp Leu Lys Ile Leu Asn His Leu Ile Gln Asn Lys Asn
    210                 215                 220

Glu Tyr Cys Met Glu Val Thr Pro Lys Thr Leu Ala Asp Val Lys Gly
225                 230                 235                 240

Gly Thr Leu Ile Ser Tyr Glu Gly Arg Leu Leu Glu Ile Ala Gln Val
                245                 250                 255

Pro Asp Glu His Val Asn Glu Phe Lys Ser Ile Glu Lys Phe Lys Ile
            260                 265                 270

Phe Asn Thr Asn Asn Leu Trp Val Asn Leu Asn Ala Val Lys Arg Leu
        275                 280                 285

Val Glu Ala Asp Ala Leu Lys Met Glu Ile Ile Pro Asn Pro Lys Glu
    290                 295                 300

Val Asp Gly Ile Lys Val Leu Gln Leu Glu Thr Ala Ala Gly Ala Ala
305                 310                 315                 320

Ile Arg Phe Phe Asp Lys Ala Ile Gly Ile Asn Val Pro Arg Ser Arg
                325                 330                 335

Phe Leu Pro Val Lys Ala Thr Ser Asp Leu Leu Leu Val Gln Ser Asp
            340                 345                 350

Leu Tyr Thr Leu Glu Asp Gly Phe Val Ile Arg Asn Lys Ala Arg Ala
        355                 360                 365

Asn Pro Glu Asn Pro Ser Ile Glu Leu Gly Pro Glu Phe Lys Lys Val
    370                 375                 380

Ser Asn Phe Leu Gly Arg Phe Lys Ser Ile Pro Ser Ile Val Glu Leu
385                 390                 395                 400

Asp Ser Leu Lys Val Ala Gly Asn Val Trp Phe Gly Ala Gly Val Ile
                405                 410                 415

Leu Lys Gly Lys Ile Ser Ile Val Ala Asn Pro Gly Val Lys Leu Glu
            420                 425                 430

Val Pro Asp Gly Ala Val Ile Ser Asp Lys Glu Ile Asn Gly Pro Glu
        435                 440                 445

Asp Leu Leu
    450

<210>  307
<211>  15
<212>  PRT
<213>  Glycine max

<400>  307

Ala Ile Gly Ile Asn Val Pro Arg Ser Arg Phe Leu Pro Val Lys
1               5                   10                  15

<210>  308
<211>  24
<212>  PRT
<213>  Glycine max

<400>  308

Ala Thr Ser Asp Leu Leu Leu Val Gln Ser Asp Leu Tyr Thr Leu Glu
1               5                   10                  15

Asp Gly Phe Val Ile Arg Asn Lys
            20

<210>  309
<211>  18
<212>  PRT
<213>  Glycine max

<400>  309

Glu Tyr Val Phe Val Ala Asn Ser Asp Asn Leu Gly Ala Ile Val Asp
1               5                   10                  15

Leu Lys

<210>  310
<211>  9
<212>  PRT
<213>  Glycine max

<400>  310

Ile Leu Asn His Leu Ile Gln Asn Lys
1               5


<210>  311
<211>  26
<212>  PRT
<213>  Glycine max

<400>  311

Ile Gln Thr Pro Thr Asp Glu Val Val Val Pro Tyr Asp Thr Leu Ala
1               5                   10                  15


Pro Thr Pro Asp Gly Ser Ser Asp Val Lys
            20                  25


<210>  312
<211>  9
<212>  PRT
<213>  Glycine max

<400>  312

Leu Asp Ala Leu Leu Ser Gln Gly Lys
1               5


<210>  313
<211>  12
<212>  PRT
<213>  Glycine max

<400>  313

Leu Glu Val Pro Asp Gly Ala Val Ile Ser Asp Lys
1               5                   10


<210>  314
<211>  9
<212>  PRT
<213>  Glycine max

<400>  314

Arg Leu Val Glu Ala Asp Ala Leu Lys
1               5


<210>  315
<211>  14

<212>  PRT
<213>  Glycine max

<400>  315

Ser Ala Val Ala Gly Leu Asn Glu Ile Ser Glu Ser Glu Lys
1               5                   10

<210>  316
<211>  12
<212>  PRT
<213>  Glycine max

<400>  316

Ser Ile Pro Ser Ile Val Glu Leu Asp Ser Leu Lys
1               5                   10

<210>  317
<211>  17
<212>  PRT
<213>  Glycine max

<400>  317

Val Leu Gln Leu Glu Thr Ala Ala Gly Ala Ala Ile Arg Phe Phe Asp
1               5                   10                  15

Lys

<210>  318
<211>  9
<212>  PRT
<213>  Glycine max

<400>  318

Val Ser Asn Phe Leu Gly Arg Phe Lys
1               5

<210>  319
<211>  1332
<212>  DNA
<213>  Glycine max

<400>  319
   atggatgagg agtacgatgt gatcgtgttg ggcacgggtc tcaaggagtg cattctcagc      60
   ggtcttctct ccgtcgatgg cctcaaggtt ctgcacatgg acaggaatga ctattacgga     120
   ggagaatcca cttcgctcaa tcttattcag ctttggaaga gattcagggg agatgacaag     180
   cctcctccac atttgggagc tagcagggac tataacatcg atatgaaccc taagtttatt     240
   atggctaatg gcaatttggt gcgggttctc atacatactg atgttactaa gtatctctct     300
   ttcaaagctg tggatgggag ctttgtgttt aataaaggaa aggttcataa ggtgcccgca     360
   aatgacatgg aggccttgaa gtctccactt atggggctat ttgaaaaacg ccgagcacgc 420
   aagtttttca tttatgtgca aaattacaat gagagtgatc ccaagaccca tgaagggatg 480
   gacttgacaa gagtgactac taaagagttg atagcaaaat acggtcttga tgataacact 540
   atagacttca ttggtcatgc tttggcgctt catagtgatg atcgctactt ggctgagcca 600
   gcactggaca ctgtgaagag aatgaagtta tatgcagagt ctcttgcacg ctttcaagga 660
   gggtcacctt acatatatcc tttgtatggt ttaggagagc ttccccaggc atttgcacgg 720
   cttagtgctg tttatggtgg gacatatatg ttgaataagc ctgagtgcaa ggtagaattt 780
   gatgaggaag gaaaggttgt tggtgtcaca tctgaagggg aaactgcaaa gtgcaaaaaa 840
   gttgtttgtg atccatcata tttgcccggc aaggtgagga aggttggtag ggttgcaaga 900
   gcaatagcca taatgagtca cccaatcccc agcactgatg attcccattc ggtgcaaatt 960
   attttaccac agaaacagtt gggtcggaag tcagacatgt acctattttg ttgttcatat 1020
   actcacaatg ttgctccaaa gggaaaattt attgcatttg tatcaaccga ggcagagaca 1080
   gatcagccag caattgaact aaaacctgga attgaccttc taggacctgt tgatgagata 1140
   ttttttgata tctatgacag atatgaaccg gtcaatgaac ccactttgga caactgcttt 1200
   gtatcaacaa gttatgatgc tactactcac tttgagtcga ccgtggatga tgttctcaac 1260
   atgtatacat tgataactgg aaaggttatt gacctgagtg tggatcttag tgctgctagt 1320
   gctgcagaag ag 1332

<210> 320
<211> 444
<212> PRT
<213> Glycine max

<400> 320

Met Asp Glu Glu Tyr Asp Val Ile Val Leu Gly Thr Gly Leu Lys Glu
1               5                   10                  15

Cys Ile Leu Ser Gly Leu Leu Ser Val Asp Gly Leu Lys Val Leu His
            20                  25                  30

Met Asp Arg Asn Asp Tyr Tyr Gly Gly Glu Ser Thr Ser Leu Asn Leu
        35                  40                  45

Ile Gln Leu Trp Lys Arg Phe Arg Gly Asp Asp Lys Pro Pro Pro His
    50                  55                  60

Leu Gly Ala Ser Arg Asp Tyr Asn Ile Asp Met Asn Pro Lys Phe Ile
65                  70                  75                  80

Met Ala Asn Gly Asn Leu Val Arg Val Leu Ile His Thr Asp Val Thr
                85                  90                  95

Lys Tyr Leu Ser Phe Lys Ala Val Asp Gly Ser Phe Val Phe Asn Lys
            100                 105                 110

Gly Lys Val His Lys Val Pro Ala Asn Asp Met Glu Ala Leu Lys Ser
        115                 120                 125

Pro Leu Met Gly Leu Phe Glu Lys Arg Arg Ala Arg Lys Phe Phe Ile
    130                 135                 140

Tyr Val Gln Asn Tyr Asn Glu Ser Asp Pro Lys Thr His Glu Gly Met
145                 150                 155                 160

Asp Leu Thr Arg Val Thr Thr Lys Glu Leu Ile Ala Lys Tyr Gly Leu
                165                 170                 175

Asp Asp Asn Thr Ile Asp Phe Ile Gly His Ala Leu Ala Leu His Ser
            180                 185                 190

Asp Asp Arg Tyr Leu Ala Glu Pro Ala Leu Asp Thr Val Lys Arg Met
        195                 200                 205

Lys Leu Tyr Ala Glu Ser Leu Ala Arg Phe Gln Gly Gly Ser Pro Tyr
    210                 215                 220

Ile Tyr Pro Leu Tyr Gly Leu Gly Glu Leu Pro Gln Ala Phe Ala Arg
225                 230                 235                 240

Leu Ser Ala Val Tyr Gly Gly Thr Tyr Met Leu Asn Lys Pro Glu Cys
                245                 250                 255

Lys Val Glu Phe Asp Glu Glu Gly Lys Val Val Gly Val Thr Ser Glu
            260                 265                 270

Gly Glu Thr Ala Lys Cys Lys Lys Val Val Cys Asp Pro Ser Tyr Leu
        275                 280                 285

Pro Gly Lys Val Arg Lys Val Gly Arg Val Ala Arg Ala Ile Ala Ile
    290                 295                 300

Met Ser His Pro Ile Pro Ser Thr Asp Asp Ser His Ser Val Gln Ile
305                 310                 315                 320

Ile Leu Pro Gln Lys Gln Leu Gly Arg Lys Ser Asp Met Tyr Leu Phe
                325                 330                 335

Cys Cys Ser Tyr Thr His Asn Val Ala Pro Lys Gly Lys Phe Ile Ala
            340                 345                 350

Phe Val Ser Thr Glu Ala Glu Thr Asp Gln Pro Ala Ile Glu Leu Lys
        355                 360                 365

Pro Gly Ile Asp Leu Leu Gly Pro Val Asp Glu Ile Phe Phe Asp Ile
    370                 375                 380

Tyr Asp Arg Tyr Glu Pro Val Asn Glu Pro Thr Leu Asp Asn Cys Phe
385                 390                 395                 400

Val Ser Thr Ser Tyr Asp Ala Thr Thr His Phe Glu Ser Thr Val Asp
                405                 410                 415

Asp Val Leu Asn Met Tyr Thr Leu Ile Thr Gly Lys Val Ile Asp Leu
            420                 425                 430

Ser Val Asp Leu Ser Ala Ala Ser Ala Ala Glu Glu
        435                 440


<210>  321
<211>  10
<212>  PRT
<213>  Glycine max

<400>  321

Ala Val Asp Gly Ser Phe Val Phe Asn Lys
1               5                   10


<210>  322
<211>  14
<212>  PRT
<213>  Glycine max

<400>  322

Phe Phe Ile Tyr Val Gln Asn Tyr Asn Glu Ser Asp Pro Lys
1               5                   10


<210>  323
<211>  8
<212>  PRT
<213>  Glycine max

<400>  323

Val Glu Phe Asp Glu Glu Gly Lys
1               5


<210>  324

<211>  16
<212>  PRT
<213>  Glycine max

<400>  324

Val Ile Asp Leu Ser Val Asp Leu Ser Ala Ala Ser Ala Ala Glu Glu
1               5                   10                  15


<210>  325
<211>  12
<212>  PRT
<213>  Glycine max

<400>  325

Val Val Gly Val Thr Ser Glu Gly Glu Thr Ala Lys
1               5                   10


<210>  326
<211>  1332
<212>  DNA
<213>  Glycine max

<400>  326
atggatgagg agtacgatgt gatcgtgttg ggcacgggtc tcaaggagtg cattctcagc      60

ggtcttctct ccgtcgatgg cctcaaggtt ctgcatatgg ataggaacga ctattacgga     120

ggagaatcca cttcgctcaa tcttattcag ctttggaaga gattcagggg agatgacaag     180

cctcctccac atttgggcgc tagcagggac tacaacatcg atatgaaccc caagtttatt     240

atggctaatg gcaatttggt gcgggttctc atacataccg atgttacaaa gtatctctat     300

ttcaaagctg tggatgggag ctatgtgttt aataaaggaa aggttcataa ggtgcctgca     360

aacgacatgg aggccttgaa gtccccactt atggggctat ttgaaaaacg ccgggcacgc     420

aagtttttca tttatgtgca aaactacaac gagagcgatc ccaagaccca cgaggggatg     480

gacttgacaa gagtgactac taaagagttg atagcaaaat acggccttga tgacaacact     540

atagacttca ttggtcatgc tttggcgctt catagcgatg atcgctactt ggctgagcca     600

gcactggaca ccgtgaagag aatgaagtta tatgcagagt ctcttgcacg ctttcaagga     660

gggtcacctt acatatatcc tttgtatggt ttaggagagc ttccccaggc atttgcacgg     720

cttagtgctg tttatggtgg aacatatatg ttgaataagc ctgagtgcaa ggtagaattt     780

gatgaggaag gaaaggttgt tggtgttaca tctgaagggg aaactgcaaa gtgtaaaaaa     840

gttgtttgtg atccatcata tttgcctggc aaggtgagga aggttggtag ggttgcaagg     900

gcgatagcca tcatgagcca cccaatcccc agcactgatg attcccattc agtgcaaatt     960

attttaccac agaaacagtt gggtcggaag tcagacatgt acctattttg ttgttcgtat    1020

actcacaatg ttgctccaaa gggaaaattt attgcatttg tatcaactga ggcagagaca    1080

gatcagccgg caattggact gaaacctgga attgaccttc taggacctgt tgatgagata 1140

ttttttgata tctatgacag atatgaacca gtcaacgaac ccactttgga caactgcttt 1200

atatcaacaa gttatgatgc tactactcac tttgagtcga ctgtggatga cgttctcaac 1260

atgtatacat tgataactgg aaaggttatt gacctcagtg tggatcttag tgctgctagt 1320

gctgcagaag ag 1332

<210> 327
<211> 444
<212> PRT
<213> Glycine max

<400> 327

Met Asp Glu Glu Tyr Asp Val Ile Val Leu Gly Thr Gly Leu Lys Glu
1               5                   10                  15

Cys Ile Leu Ser Gly Leu Leu Ser Val Asp Gly Leu Lys Val Leu His
            20                  25                  30

Met Asp Arg Asn Asp Tyr Tyr Gly Gly Glu Ser Thr Ser Leu Asn Leu
        35                  40                  45

Ile Gln Leu Trp Lys Arg Phe Arg Gly Asp Asp Lys Pro Pro Pro His
    50                  55                  60

Leu Gly Ala Ser Arg Asp Tyr Asn Ile Asp Met Asn Pro Lys Phe Ile
65                  70                  75                  80

Met Ala Asn Gly Asn Leu Val Arg Val Leu Ile His Thr Asp Val Thr
                85                  90                  95

Lys Tyr Leu Tyr Phe Lys Ala Val Asp Gly Ser Tyr Val Phe Asn Lys
            100                 105                 110

Gly Lys Val His Lys Val Pro Ala Asn Asp Met Glu Ala Leu Lys Ser
        115                 120                 125

Pro Leu Met Gly Leu Phe Glu Lys Arg Arg Ala Arg Lys Phe Phe Ile
    130                 135                 140

Tyr Val Gln Asn Tyr Asn Glu Ser Asp Pro Lys Thr His Glu Gly Met
145                 150                 155                 160

Asp Leu Thr Arg Val Thr Thr Lys Glu Leu Ile Ala Lys Tyr Gly Leu
                165                 170                 175

Asp Asp Asn Thr Ile Asp Phe Ile Gly His Ala Leu Ala Leu His Ser

            180                 185                 190

Asp Asp Arg Tyr Leu Ala Glu Pro Ala Leu Asp Thr Val Lys Arg Met
        195                 200                 205

Lys Leu Tyr Ala Glu Ser Leu Ala Arg Phe Gln Gly Gly Ser Pro Tyr
    210                 215                 220

Ile Tyr Pro Leu Tyr Gly Leu Gly Glu Leu Pro Gln Ala Phe Ala Arg
225                 230                 235                 240

Leu Ser Ala Val Tyr Gly Gly Thr Tyr Met Leu Asn Lys Pro Glu Cys
                245                 250                 255

Lys Val Glu Phe Asp Glu Glu Gly Lys Val Val Gly Val Thr Ser Glu
            260                 265                 270

Gly Glu Thr Ala Lys Cys Lys Lys Val Val Cys Asp Pro Ser Tyr Leu
        275                 280                 285

Pro Gly Lys Val Arg Lys Val Gly Arg Val Ala Arg Ala Ile Ala Ile
    290                 295                 300

Met Ser His Pro Ile Pro Ser Thr Asp Asp Ser His Ser Val Gln Ile
305                 310                 315                 320

Ile Leu Pro Gln Lys Gln Leu Gly Arg Lys Ser Asp Met Tyr Leu Phe
                325                 330                 335

Cys Cys Ser Tyr Thr His Asn Val Ala Pro Lys Gly Lys Phe Ile Ala
            340                 345                 350

Phe Val Ser Thr Glu Ala Glu Thr Asp Gln Pro Ala Ile Gly Leu Lys
        355                 360                 365

Pro Gly Ile Asp Leu Leu Gly Pro Val Asp Glu Ile Phe Phe Asp Ile
    370                 375                 380

Tyr Asp Arg Tyr Glu Pro Val Asn Glu Pro Thr Leu Asp Asn Cys Phe
385                 390                 395                 400

Ile Ser Thr Ser Tyr Asp Ala Thr Thr His Phe Glu Ser Thr Val Asp
                405                 410                 415

Asp Val Leu Asn Met Tyr Thr Leu Ile Thr Gly Lys Val Ile Asp Leu
            420                 425                 430

Ser Val Asp Leu Ser Ala Ala Ser Ala Ala Glu Glu
        435                 440

<210>  328
<211>  10
<212>  PRT
<213>  Glycine max

<400>  328

Ala Val Asp Gly Ser Tyr Val Phe Asn Lys
1               5                   10

<210>  329
<211>  14
<212>  PRT
<213>  Glycine max

<400>  329

Phe Phe Ile Tyr Val Gln Asn Tyr Asn Glu Ser Asp Pro Lys
1               5                   10

<210>  330
<211>  8
<212>  PRT
<213>  Glycine max

<400>  330

Val Glu Phe Asp Glu Glu Gly Lys
1               5

<210>  331
<211>  16
<212>  PRT
<213>  Glycine max

<400>  331

Val Ile Asp Leu Ser Val Asp Leu Ser Ala Ala Ser Ala Ala Glu Glu
1               5                   10                  15

<210>  332
<211>  12
<212>  PRT
<213>  Glycine max

<400>  332

Val Val Gly Val Thr Ser Glu Gly Glu Thr Ala Lys
1               5                   10

<210>  333
<211>  1512
<212>  DNA
<213>  Glycine max

<400>  333
atggcgatga gaaccaaact ttctttagct atctttttct tttttctttt agccttattt      60

tcaaacctag cctttggcaa atgtaaagaa accgaggtag aagaagaaga ccctgagctc     120

gtaacatgca aacaccaatg ccaacagcca cagcaataca ctgagggtga caagcgggta     180

tgcttgcaaa gatgtgacag gtatcatcgt atgaagcaag agcgagagaa acaaatccaa     240

gaggaaactc gtgagaagaa agaggaggaa agccgtgaaa gggaagaaga acaacaagag     300

cagcatgagg aacaagatga aaatccctac atttttgagg aagataagga ttttgagacc     360

agagtcgaaa ctgaaggtgg cagaattcgg gttctcaaga agttcactga aaaatccaag     420

cttcttcagg gcattgagaa cttccgtttg gccattttgg aagctagagc acacacgttc     480

gtgtctcctc gccactttga ttccgaggtt gtttttttca acatcaaggg gcgagccgta     540

cttgggttgg tgagtgaaag tgaaacagaa aaaatcaccc ttgaacctgg ggacatgata     600

cacataccag ctggcacccc actgtacatc gttaacagag atgagaatga taagctcttc     660

cttgccatgc tccatatacc tgtctctgtc tctactcctg gaaaatttga ggaatttttc     720

gggcctggag gacgagaccc agaatcggtc ctctcagcat tcagctggaa tgtgctgcaa     780

gctgcgctcc aaaccccaaa aggaaagtta gaaaagcttt ttgatcaaca gaacgaggga     840

agtattttcg caataagcag agaacaggtg cgtgcgttgg cccctaccaa gaaaagctct     900

tggtggccat tcggtggcga atccaagcct caattcaata ttttcagcaa gcgtcccact     960

atctccaacg gatatggtcg tttaactgaa gttggtcctg acgatgatga aaagagttgg    1020

cttcaaagac tcaatctcat gcttaccttt accaacatca cccagagatc tatgagtact    1080

attcactaca actcacatgc aacgaagata gcactggtga tcgatggcag agggcatctt    1140

caaatatcat gtccacacat gtcatcaagg tcgtcacact cgaagcatga taagagtagc    1200

ccttcatacc atagaatcag ttccgatttg aagcctggaa tggtgtttgt tgtccctccc    1260

ggtcatccct tcgtcaccat agcttccaat aaagagaatc tcctcatgat ttgcttcgag    1320

gttaacgctc gagacaacaa gaagtttaca tttgcaggga aggacaacat tgtgagttct    1380

ctggacaacg ttgctaagga gcttgccttt aactatcctt ctgagatggt gaacggagta    1440

ttcgaccgaa aggagagttt ctttttccct tttgagttgc cgagagagga gcgtggtcgt    1500

cgcgctgatg cg                                                        1512

<210>  334
<211>  504
<212>  PRT
<213>  Glycine max

<400>  334

Met Ala Met Arg Thr Lys Leu Ser Leu Ala Ile Phe Phe Phe Phe Leu

1               5                   10                  15

Leu Ala Leu Phe Ser Asn Leu Ala Phe Gly Lys Cys Lys Glu Thr Glu
            20                  25                  30

Val Glu Glu Glu Asp Pro Glu Leu Val Thr Cys Lys His Gln Cys Gln
        35                  40                  45

Gln Pro Gln Gln Tyr Thr Glu Gly Asp Lys Arg Val Cys Leu Gln Arg
    50                  55                  60

Cys Asp Arg Tyr His Arg Met Lys Gln Glu Arg Glu Lys Gln Ile Gln
65                  70                  75                  80

Glu Glu Thr Arg Glu Lys Lys Glu Glu Glu Ser Arg Glu Arg Glu Glu
                85                  90                  95

Glu Gln Gln Glu Gln His Glu Glu Gln Asp Glu Asn Pro Tyr Ile Phe
            100                 105                 110

Glu Glu Asp Lys Asp Phe Glu Thr Arg Val Glu Thr Glu Gly Gly Arg
        115                 120                 125

Ile Arg Val Leu Lys Lys Phe Thr Glu Lys Ser Lys Leu Leu Gln Gly
    130                 135                 140

Ile Glu Asn Phe Arg Leu Ala Ile Leu Glu Ala Arg Ala His Thr Phe
145                 150                 155                 160

Val Ser Pro Arg His Phe Asp Ser Glu Val Val Phe Phe Asn Ile Lys
                165                 170                 175

Gly Arg Ala Val Leu Gly Leu Val Ser Glu Ser Glu Thr Glu Lys Ile
            180                 185                 190

Thr Leu Glu Pro Gly Asp Met Ile His Ile Pro Ala Gly Thr Pro Leu
        195                 200                 205

Tyr Ile Val Asn Arg Asp Glu Asn Asp Lys Leu Phe Leu Ala Met Leu
    210                 215                 220

His Ile Pro Val Ser Val Ser Thr Pro Gly Lys Phe Glu Glu Phe Phe
225                 230                 235                 240

Gly Pro Gly Gly Arg Asp Pro Glu Ser Val Leu Ser Ala Phe Ser Trp
                245                 250                 255

Asn Val Leu Gln Ala Ala Leu Gln Thr Pro Lys Gly Lys Leu Glu Lys
            260                 265                 270

Leu Phe Asp Gln Gln Asn Glu Gly Ser Ile Phe Ala Ile Ser Arg Glu
        275                 280                 285

Gln Val Arg Ala Leu Ala Pro Thr Lys Lys Ser Ser Trp Trp Pro Phe
    290                 295                 300

Gly Gly Glu Ser Lys Pro Gln Phe Asn Ile Phe Ser Lys Arg Pro Thr
305                 310                 315                 320

Ile Ser Asn Gly Tyr Gly Arg Leu Thr Glu Val Gly Pro Asp Asp Asp
                325                 330                 335

Glu Lys Ser Trp Leu Gln Arg Leu Asn Leu Met Leu Thr Phe Thr Asn
            340                 345                 350

Ile Thr Gln Arg Ser Met Ser Thr Ile His Tyr Asn Ser His Ala Thr
        355                 360                 365

Lys Ile Ala Leu Val Ile Asp Gly Arg Gly His Leu Gln Ile Ser Cys
    370                 375                 380

Pro His Met Ser Ser Arg Ser Ser His Ser Lys His Asp Lys Ser Ser
385                 390                 395                 400

Pro Ser Tyr His Arg Ile Ser Ser Asp Leu Lys Pro Gly Met Val Phe
                405                 410                 415

Val Val Pro Pro Gly His Pro Phe Val Thr Ile Ala Ser Asn Lys Glu
            420                 425                 430

Asn Leu Leu Met Ile Cys Phe Glu Val Asn Ala Arg Asp Asn Lys Lys
        435                 440                 445

Phe Thr Phe Ala Gly Lys Asp Asn Ile Val Ser Ser Leu Asp Asn Val
    450                 455                 460

Ala Lys Glu Leu Ala Phe Asn Tyr Pro Ser Glu Met Val Asn Gly Val
465                 470                 475                 480

Phe Asp Arg Lys Glu Ser Phe Phe Phe Pro Phe Glu Leu Pro Arg Glu
                485                 490                 495

Glu Arg Gly Arg Arg Ala Asp Ala
            500

<210>  335
<211>  17
<212>  PRT
<213>  Glycine max

<400>  335

Asp Phe Glu Thr Arg Val Glu Thr Glu Gly Gly Arg Ile Arg Val Leu
1               5                   10                  15

Lys

<210>  336
<211>  12
<212>  PRT
<213>  Glycine max

<400>  336

Asp Asn Ile Val Ser Ser Leu Asp Asn Val Ala Lys
1               5                   10

<210>  337
<211>  15
<212>  PRT
<213>  Glycine max

<400>  337

Gly Arg Ala Val Leu Gly Leu Val Ser Glu Ser Glu Thr Glu Lys
1               5                   10                  15

<210>  338
<211>  25
<212>  PRT
<213>  Glycine max

<400>  338

Leu Phe Asp Gln Gln Asn Glu Gly Ser Ile Phe Ala Ile Ser Arg Glu
1               5                   10                  15

Gln Val Arg Ala Leu Ala Pro Thr Lys
            20                  25

<210>  339
<211>  21
<212>  PRT
<213>  Glycine max

<400>  339

Arg Pro Thr Ile Ser Asn Gly Tyr Gly Arg Leu Thr Glu Val Gly Pro
1               5                   10                  15

Asp Asp Asp Glu Lys
            20

<210>  340
<211>  1467
<212>  DNA
<213>  Glycine max

<400>  340
atggcgacca gagccaagct ttctttagct atcttccttt tctttctttt agccttgatt      60

tcaaacctag ccttgggcaa acttaaagaa accgaggtcg aagaagatcc cgagctcgta     120

acatgcaaac accagtgcca acagcaacgg caatacactg agagtgacaa gcgaacatgc     180

ttgcaacaat gtgacagtat gaagcaagag cgagagaaac aagtcgaaga ggaaactcgc     240

gagaaggaag aagaacatca agagcagcat gaggaggagc aagaccaaaa tccctacgtt     300

tttgaagaag ataaggattt ttcgaccaga gtcgaaacag aaggtggcag cattcgggtt     360

ctcaagaagt tcactgagaa atccaagctt cttcaaggca atgagaattt ccgtttggcc     420

atcttagaag ctagagcaca cacgttcgtg tccccacgcc actttgattc cgaggttgtc     480

ttgttcaaca ttaaggggag agccgtactt gggttggtga gggaaagtga aacagaaaaa     540

atcaccctag aacctggaga catgatacac ataccagcag gcacaccact gtacatcgtt     600

aacagagatg agaatgagaa gctcctcctt gccatgctcc atatacctgt ctctactcct     660

ggaaaatttg aggaattttt cgggcctgga ggacgagacc cagaatcggt cctctcagca     720

ttcagctgga atgtgctgca agctgcgctc caaaccccaa aaggaaagtt agaaaggctt     780

tttaatcaac agaacgaggg aagtattttc aaaataagca gagaacgggt gcgtgcgttg     840

gcccccacca agaaaagctc ttggtggcca ttcggcggcg aatccaaggc tcaattcaat     900

attttcagca agcgtcccac tttctccaac ggatatggcc gtttaactga agttggtcct     960

gatgatgaaa agagttggct tcaaagactc aacctcatgc ttacctttac caacatcacc    1020

cagagatcta tgagtactat tcactacaac tcacatgcaa cgaagatagc actggtgatg    1080

gatggtagag ggcatcttca aatatcatgt ccacacatgt catcaaggtc agactcaaag    1140

catgataaga gtagcccctc ataccataga atcagtgcgg acttgaagcc tggaatggtg    1200

tttgttgtcc ctcctggtca tcccttcgtc actatagctt ccaataaaga gaatctcctc    1260

ataatttgct tcgaggttaa cgttcgagac aacaagaagt ttacgtttgc agggaaggac    1320

aacattgtga gctctctgga caacgtagct aaggagctgg cctttaacta tccttctgag    1380

atggtgaacg gagtcttcga aagaaaggag agtctctttt tccccttcga gttgccgagc    1440

gaggagcgtg gtcgtcgcgc tgttgcg                                        1467

<210>  341

<211>  489
<212>  PRT
<213>  Glycine max

<400>  341

Met Ala Thr Arg Ala Lys Leu Ser Leu Ala Ile Phe Leu Phe Phe Leu
1               5                   10                  15

Leu Ala Leu Ile Ser Asn Leu Ala Leu Gly Lys Leu Lys Glu Thr Glu
            20                  25                  30

Val Glu Glu Asp Pro Glu Leu Val Thr Cys Lys His Gln Cys Gln Gln
        35                  40                  45

Gln Arg Gln Tyr Thr Glu Ser Asp Lys Arg Thr Cys Leu Gln Gln Cys
    50                  55                  60

Asp Ser Met Lys Gln Glu Arg Glu Lys Gln Val Glu Glu Glu Thr Arg
65                  70                  75                  80

Glu Lys Glu Glu Glu His Gln Glu Gln His Glu Glu Glu Gln Asp Gln
                85                  90                  95

Asn Pro Tyr Val Phe Glu Glu Asp Lys Asp Phe Ser Thr Arg Val Glu
            100                 105                 110

Thr Glu Gly Gly Ser Ile Arg Val Leu Lys Lys Phe Thr Glu Lys Ser
        115                 120                 125

Lys Leu Leu Gln Gly Asn Glu Asn Phe Arg Leu Ala Ile Leu Glu Ala
    130                 135                 140

Arg Ala His Thr Phe Val Ser Pro Arg His Phe Asp Ser Glu Val Val
145                 150                 155                 160

Leu Phe Asn Ile Lys Gly Arg Ala Val Leu Gly Leu Val Arg Glu Ser
                165                 170                 175

Glu Thr Glu Lys Ile Thr Leu Glu Pro Gly Asp Met Ile His Ile Pro
            180                 185                 190

Ala Gly Thr Pro Leu Tyr Ile Val Asn Arg Asp Glu Asn Glu Lys Leu
        195                 200                 205

Leu Leu Ala Met Leu His Ile Pro Val Ser Thr Pro Gly Lys Phe Glu
    210                 215                 220

Glu Phe Phe Gly Pro Gly Gly Arg Asp Pro Glu Ser Val Leu Ser Ala

225                 230                 235                 240

Phe Ser Trp Asn Val Leu Gln Ala Ala Leu Gln Thr Pro Lys Gly Lys
                245                 250                 255

Leu Glu Arg Leu Phe Asn Gln Gln Asn Glu Gly Ser Ile Phe Lys Ile
            260                 265                 270

Ser Arg Glu Arg Val Arg Ala Leu Ala Pro Thr Lys Lys Ser Ser Trp
        275                 280                 285

Trp Pro Phe Gly Gly Glu Ser Lys Ala Gln Phe Asn Ile Phe Ser Lys
    290                 295                 300

Arg Pro Thr Phe Ser Asn Gly Tyr Gly Arg Leu Thr Glu Val Gly Pro
305                 310                 315                 320

Asp Asp Glu Lys Ser Trp Leu Gln Arg Leu Asn Leu Met Leu Thr Phe
                325                 330                 335

Thr Asn Ile Thr Gln Arg Ser Met Ser Thr Ile His Tyr Asn Ser His
            340                 345                 350

Ala Thr Lys Ile Ala Leu Val Met Asp Gly Arg Gly His Leu Gln Ile
        355                 360                 365

Ser Cys Pro His Met Ser Ser Arg Ser Asp Ser Lys His Asp Lys Ser
    370                 375                 380

Ser Pro Ser Tyr His Arg Ile Ser Ala Asp Leu Lys Pro Gly Met Val
385                 390                 395                 400

Phe Val Val Pro Pro Gly His Pro Phe Val Thr Ile Ala Ser Asn Lys
                405                 410                 415

Glu Asn Leu Leu Ile Ile Cys Phe Glu Val Asn Val Arg Asp Asn Lys
            420                 425                 430

Lys Phe Thr Phe Ala Gly Lys Asp Asn Ile Val Ser Ser Leu Asp Asn
        435                 440                 445

Val Ala Lys Glu Leu Ala Phe Asn Tyr Pro Ser Glu Met Val Asn Gly
    450                 455                 460

Val Phe Glu Arg Lys Glu Ser Leu Phe Phe Pro Phe Glu Leu Pro Ser
465                 470                 475                 480

Glu Glu Arg Gly Arg Arg Ala Val Ala
                485

<210>  342
<211>  8
<212>  PRT
<213>  Glycine max

<400>  342

Ala Gln Phe Asn Ile Phe Ser Lys
1               5

<210>  343
<211>  17
<212>  PRT
<213>  Glycine max

<400>  343

Asp Phe Ser Thr Arg Val Glu Thr Glu Gly Gly Ser Ile Arg Val Leu
1               5                   10                  15

Lys

<210>  344
<211>  12
<212>  PRT
<213>  Glycine max

<400>  344

Asp Asn Ile Val Ser Ser Leu Asp Asn Val Ala Lys
1               5                   10

<210>  345
<211>  15
<212>  PRT
<213>  Glycine max

<400>  345

Gly Arg Ala Val Leu Gly Leu Val Arg Glu Ser Glu Thr Glu Lys
1               5                   10                  15

<210>  346
<211>  20
<212>  PRT
<213>  Glycine max

<400>  346

Arg Pro Thr Phe Ser Asn Gly Tyr Gly Arg Leu Thr Glu Val Gly Pro
1               5                   10                  15

Asp Asp Glu Lys
            20

<210>  347
<211>  1266
<212>  DNA
<213>  Glycine max

<400>  347
atggtgagag gattgctcaa caagctcgtc tctcgctccc tctcggtcgc cggaaaatgg      60

cagcacaatc aactccgccg cctcaacatc cacgagtacc agggtgctga attgatgagc     120

aaacacggag tcaacgttcc gagaggcgtg gctgtttcct ctgttgaaga ggccagaaag     180

gtcatcaagg accttttccc taatgaaaat gagttggtgg ttaagagtca aattttagcc     240

ggcggacgag gtttgggaac tttcaaaagc ggccttaagg gaggagtgca cattgttaag     300

actgaccagg ttgaagacat agctgggaag atgcttggac agatactagt taccaaacag     360

actggtcctc agggaaaaat agttagcaag gtttacttgt gtgaaaagct gtcacttgta     420

aatgagatgt actttgctat aacactggat cgtacgagtg ctggtccaat tataattgct     480

tgtagtaagg gaggaaccag cattgaagac cttgcagaga aatttccgga catgattata     540

aaggtacctg ttgatgtttt tgaaggtatt actgatgaag gtgctgcaaa ggttgttgat     600

ggtttggctc tcaaagtggc tgatagaaat aagtcgattg aacaagtgaa gaatttgtat     660

aaactttttg ttgattgtga ctgcactctt ttggaaatca atcccatagc ggagacagct     720

gataaccaat tggtggctgc tgatgctaag ttgaattttg atgataatgc tgcatatcgc     780

cagaaagaga tattttctct ccgtgataca acacaagagg accctcgaga ggtgactgct     840

gcaaaggcag atttaaatta tattgggtta gatggagaaa ttggctgcat ggtgaatgga     900

gcagggttag caatggccac aatggatata attaaattgc atgggggtac tcctgccaat     960

tttctggatg taggtggcaa tgcctctgaa aaccaggttg ttgaggcatt taaaatattg    1020

actgctgatg acaaagtgaa ggcaattttg gttaacattt ttggtggtat aatgaagtgt    1080

gatgttatag caagtggaat agtgaatgct gcaaaagagg ttgcactgaa agtaccagtt    1140

gtagttcgtc tggaaggcac caatgttgat caaggaaaaa gaatattgaa ggaaagtggt    1200

atggcattaa taacggctga agatttagat gatgcagctc agaaagcagt gaaagctgct    1260

tacaaa                                                                1266

<210>  348
<211>  422
<212>  PRT
<213>  Glycine max

<400>  348

Met Val Arg Gly Leu Leu Asn Lys Leu Val Ser Arg Ser Leu Ser Val

1               5                   10                  15

Ala Gly Lys Trp Gln His Asn Gln Leu Arg Arg Leu Asn Ile His Glu
            20                  25                  30

Tyr Gln Gly Ala Glu Leu Met Ser Lys His Gly Val Asn Val Pro Arg
        35                  40                  45

Gly Val Ala Val Ser Ser Val Glu Glu Ala Arg Lys Val Ile Lys Asp
    50                  55                  60

Leu Phe Pro Asn Glu Asn Glu Leu Val Val Lys Ser Gln Ile Leu Ala
65                  70                  75                  80

Gly Gly Arg Gly Leu Gly Thr Phe Lys Ser Gly Leu Lys Gly Gly Val
                85                  90                  95

His Ile Val Lys Thr Asp Gln Val Glu Asp Ile Ala Gly Lys Met Leu
            100                 105                 110

Gly Gln Ile Leu Val Thr Lys Gln Thr Gly Pro Gln Gly Lys Ile Val
        115                 120                 125

Ser Lys Val Tyr Leu Cys Glu Lys Leu Ser Leu Val Asn Glu Met Tyr
    130                 135                 140

Phe Ala Ile Thr Leu Asp Arg Thr Ser Ala Gly Pro Ile Ile Ile Ala
145                 150                 155                 160

Cys Ser Lys Gly Gly Thr Ser Ile Glu Asp Leu Ala Glu Lys Phe Pro
                165                 170                 175

Asp Met Ile Ile Lys Val Pro Val Asp Val Phe Glu Gly Ile Thr Asp
            180                 185                 190

Glu Gly Ala Ala Lys Val Val Asp Gly Leu Ala Leu Lys Val Ala Asp
        195                 200                 205

Arg Asn Lys Ser Ile Glu Gln Val Lys Asn Leu Tyr Lys Leu Phe Val
    210                 215                 220

Asp Cys Asp Cys Thr Leu Leu Glu Ile Asn Pro Ile Ala Glu Thr Ala
225                 230                 235                 240

Asp Asn Gln Leu Val Ala Ala Asp Ala Lys Leu Asn Phe Asp Asp Asn
                245                 250                 255

Ala Ala Tyr Arg Gln Lys Glu Ile Phe Ser Leu Arg Asp Thr Thr Gln
            260                 265                 270

Glu Asp Pro Arg Glu Val Thr Ala Ala Lys Ala Asp Leu Asn Tyr Ile
        275                 280                 285

Gly Leu Asp Gly Glu Ile Gly Cys Met Val Asn Gly Ala Gly Leu Ala
    290                 295                 300

Met Ala Thr Met Asp Ile Ile Lys Leu His Gly Gly Thr Pro Ala Asn
305                 310                 315                 320

Phe Leu Asp Val Gly Gly Asn Ala Ser Glu Asn Gln Val Val Glu Ala
                325                 330                 335

Phe Lys Ile Leu Thr Ala Asp Asp Lys Val Lys Ala Ile Leu Val Asn
            340                 345                 350

Ile Phe Gly Gly Ile Met Lys Cys Asp Val Ile Ala Ser Gly Ile Val
        355                 360                 365

Asn Ala Ala Lys Glu Val Ala Leu Lys Val Pro Val Val Val Arg Leu
    370                 375                 380

Glu Gly Thr Asn Val Asp Gln Gly Lys Arg Ile Leu Lys Glu Ser Gly
385                 390                 395                 400

Met Ala Leu Ile Thr Ala Glu Asp Leu Asp Asp Ala Ala Gln Lys Ala
                405                 410                 415

Val Lys Ala Ala Tyr Lys
            420

<210>  349
<211>  12
<212>  PRT
<213>  Glycine max

<400>  349

Asp Leu Phe Pro Asn Glu Asn Glu Leu Val Val Lys
1               5                   10

<210>  350
<211>  20
<212>  PRT
<213>  Glycine max

<400>  350

Glu Ile Phe Ser Leu Arg Asp Thr Thr Gln Glu Asp Pro Arg Glu Val

1               5                   10                  15

Thr Ala Ala Lys
            20

<210>  351
<211>  26
<212>  PRT
<213>  Glycine max

<400>  351

Leu His Gly Gly Thr Pro Ala Asn Phe Leu Asp Val Gly Gly Asn Ala
1               5                   10                  15

Ser Glu Asn Gln Val Val Glu Ala Phe Lys
            20                  25

<210>  352
<211>  12
<212>  PRT
<213>  Glycine max

<400>  352

Leu Asn Phe Asp Asp Asn Ala Ala Tyr Arg Gln Lys
1               5                   10

<210>  353
<211>  14
<212>  PRT
<213>  Glycine max

<400>  353

Ser Gln Ile Leu Ala Gly Gly Arg Gly Leu Gly Thr Phe Lys
1               5                   10

<210>  354
<211>  10
<212>  PRT
<213>  Glycine max

<400>  354

Thr Asp Gln Val Glu Asp Ile Ala Gly Lys
1               5                   10

<210>  355
<211>  16
<212>  PRT
<213>  Glycine max

<400>  355

Val Pro Val Val Val Arg Leu Glu Gly Thr Asn Val Asp Gln Gly Lys

1               5                   10                  15

<210>  356
<211>  8
<212>  PRT
<213>  Glycine max

<400>  356

Val Val Asp Gly Leu Ala Leu Lys
1               5

<210>  357
<211>  1266
<212>  DNA
<213>  Glycine max

<400>  357
atggtgaggg gattgctcaa caagctcgtc tctcgctccc tctccgtcgc cggaaaatgg      60

cagcacaacc aactccgccg cctcaacatc cacgagtacc agggcgctga attgatgagc     120

aaacacggag ttaatgttcc gagaggcgtg gctgtttcct ctgttgaaga ggtcagaaag     180

gccatcaagg accttttccc taatgaaaat gagttggtgg ttaagagtca aattttagcc     240

ggtggaagag gtttgggaac tttcaaaagc ggccttaagg gaggagtgca cattgttaag     300

actgaccagg ttgaagacat agctgggaag atgcttgggc agatactagt taccaaacag     360

actggtcctc agggaaaaat agttagcaag gtttacttgt gtgaaaagct gtcactagta     420

aatgagatgt actttgctat aacactggat cgtacgagtg ctggtccaat tataattgct     480

tgtagtaagg gaggaaccag cattgaagac ctagcagaga aatttccgga catgattata     540

aaggtacctg ttgatgtttt tgaaggaatt actgatgaag atgctgcaaa ggttgttgat     600

ggtttggctc tcaaagtggc tgatagaaat aagtcgattg aacaagtgaa gaatttgtat     660

aaactttttg ttgattgtga ctgcaccctt ttggaaatca atcccattgc ggagacagct     720

gataaccaat tggtggctgc tgatgctaag ttgaattttg acgataatgc tgcatatcgc     780

cagaaagaga tatttgctct ccgtgataca acacaagagg accctcgaga ggtgactgct     840

gcaaaggcag acttaaatta tattgggtta gatggagaaa taggctgcat ggtgaatgga     900

gcagggttag caatggccac aatggatata attaaattgc atgggggtac tcctgccaat     960

tttttggatg taggtggcaa tgcctctgaa ggccaggtgg ttgaggcatt taaaatattg    1020

actgctgatg acaaagtgaa ggcaattttg gttaacattt ttgggggtat aatgaagtgt    1080

gatgttatag caagtggaat agtgaatgct gcaaaagagg ttgcactgaa agtacccgta    1140

gtagttcgtc tggaaggcac caatgttgat catggaaaaa gaattttgaa ggaaagtggt    1200

atggcgttga taacggctga agatttagat gatgctgctc agaaagcagt gaaagctgct    1260

tacaaa                                                               1266

<210>  358
<211>  422
<212>  PRT
<213>  Glycine max

<400>  358

Met Val Arg Gly Leu Leu Asn Lys Leu Val Ser Arg Ser Leu Ser Val
1               5                   10                  15

Ala Gly Lys Trp Gln His Asn Gln Leu Arg Arg Leu Asn Ile His Glu
            20                  25                  30

Tyr Gln Gly Ala Glu Leu Met Ser Lys His Gly Val Asn Val Pro Arg
        35                  40                  45

Gly Val Ala Val Ser Ser Val Glu Glu Val Arg Lys Ala Ile Lys Asp
    50                  55                  60

Leu Phe Pro Asn Glu Asn Glu Leu Val Val Lys Ser Gln Ile Leu Ala
65                  70                  75                  80

Gly Gly Arg Gly Leu Gly Thr Phe Lys Ser Gly Leu Lys Gly Gly Val
                85                  90                  95

His Ile Val Lys Thr Asp Gln Val Glu Asp Ile Ala Gly Lys Met Leu
            100                 105                 110

Gly Gln Ile Leu Val Thr Lys Gln Thr Gly Pro Gln Gly Lys Ile Val
        115                 120                 125

Ser Lys Val Tyr Leu Cys Glu Lys Leu Ser Leu Val Asn Glu Met Tyr
    130                 135                 140

Phe Ala Ile Thr Leu Asp Arg Thr Ser Ala Gly Pro Ile Ile Ile Ala
145                 150                 155                 160

Cys Ser Lys Gly Gly Thr Ser Ile Glu Asp Leu Ala Glu Lys Phe Pro
                165                 170                 175

Asp Met Ile Ile Lys Val Pro Val Asp Val Phe Glu Gly Ile Thr Asp
            180                 185                 190

Glu Asp Ala Ala Lys Val Val Asp Gly Leu Ala Leu Lys Val Ala Asp
        195                 200                 205

Arg Asn Lys Ser Ile Glu Gln Val Lys Asn Leu Tyr Lys Leu Phe Val
    210                 215                 220

Asp Cys Asp Cys Thr Leu Leu Glu Ile Asn Pro Ile Ala Glu Thr Ala
225                 230                 235                 240

Asp Asn Gln Leu Val Ala Ala Asp Ala Lys Leu Asn Phe Asp Asp Asn
                245                 250                 255

Ala Ala Tyr Arg Gln Lys Glu Ile Phe Ala Leu Arg Asp Thr Thr Gln
            260                 265                 270

Glu Asp Pro Arg Glu Val Thr Ala Ala Lys Ala Asp Leu Asn Tyr Ile
        275                 280                 285

Gly Leu Asp Gly Glu Ile Gly Cys Met Val Asn Gly Ala Gly Leu Ala
    290                 295                 300

Met Ala Thr Met Asp Ile Ile Lys Leu His Gly Gly Thr Pro Ala Asn
305                 310                 315                 320

Phe Leu Asp Val Gly Gly Asn Ala Ser Glu Gly Gln Val Val Glu Ala
                325                 330                 335

Phe Lys Ile Leu Thr Ala Asp Asp Lys Val Lys Ala Ile Leu Val Asn
            340                 345                 350

Ile Phe Gly Gly Ile Met Lys Cys Asp Val Ile Ala Ser Gly Ile Val
        355                 360                 365

Asn Ala Ala Lys Glu Val Ala Leu Lys Val Pro Val Val Val Arg Leu
    370                 375                 380

Glu Gly Thr Asn Val Asp His Gly Lys Arg Ile Leu Lys Glu Ser Gly
385                 390                 395                 400

Met Ala Leu Ile Thr Ala Glu Asp Leu Asp Asp Ala Ala Gln Lys Ala
                405                 410                 415

Val Lys Ala Ala Tyr Lys
            420

<210>  359
<211>  12
<212>  PRT
<213>  Glycine max

<400>  359

Asp Leu Phe Pro Asn Glu Asn Glu Leu Val Val Lys
1               5                   10

<210>  360
<211>  20
<212>  PRT
<213>  Glycine max

<400>  360

Glu Ile Phe Ala Leu Arg Asp Thr Thr Gln Glu Asp Pro Arg Glu Val
1               5                   10                  15

Thr Ala Ala Lys
            20

<210>  361
<211>  26
<212>  PRT
<213>  Glycine max

<400>  361

Leu His Gly Gly Thr Pro Ala Asn Phe Leu Asp Val Gly Gly Asn Ala
1               5                   10                  15

Ser Glu Gly Gln Val Val Glu Ala Phe Lys
            20                  25

<210>  362
<211>  12
<212>  PRT
<213>  Glycine max

<400>  362

Leu Asn Phe Asp Asp Asn Ala Ala Tyr Arg Gln Lys
1               5                   10

<210>  363
<211>  14
<212>  PRT
<213>  Glycine max

<400>  363

Ser Gln Ile Leu Ala Gly Gly Arg Gly Leu Gly Thr Phe Lys
1               5                   10

<210>  364
<211>  10
<212>  PRT
<213>  Glycine max

<400>  364

Thr Asp Gln Val Glu Asp Ile Ala Gly Lys
1               5                   10

<210>  365
<211>  8
<212>  PRT
<213>  Glycine max

<400>  365

Val Val Asp Gly Leu Ala Leu Lys
1               5


<210>  366
<211>  1203
<212>  DNA
<213>  Glycine max

<400>  366
atggcgacta agaggagcgt gggaacgttg aaggagggtg atttgaaggg gaagagggta      60

ttcgtgaggg tcgatctgaa cgtgcctttg gatgacaacc ttaacatcac cgatgacact     120

agagtccgtg ctgctgttcc caccatcaag tacttgactg gttatggtgc taaagtgatc     180

ctttctagcc atttgggacg tccaaaaggt gtaacaccta aatacagttt gaagcctctt     240

gtgccaaggc tgtctcaact tcttggaatt gaggttacga tggccaatga ctctattggg     300

gaggaagttg aaaagttggt tacacaactt ccagaaggtg gtgttttgct tctagagaat     360

gtgaggttct acaaggagga agagaagaat gatcctgaat ttgcaaagaa gttggcttct     420

ctagctgatc tctatgtgaa tgatgcattt ggcactgccc acagagctca tgcttctaca     480

gaaggagttg ccaaatactt gaagccctct gttgcaggat tcctaatgca gaaggagctt     540

gattatctag ttggggctgt gtcaaacccc aagagaccat ttgctgctat tgtcggtgga     600

tcgaaggtgt cttccaagat tggagttatt gaatccttgt tggagaaagt taatgttctc     660

ttgcttggtg gaggaatgat ctttaccttt tacaaggctc agggttattc agttgggtca     720

tctcttgtgg aggaagacaa gcttagcctt gcaactacac ttcttgaaaa ggccaaggct     780

aaaggggttt ctttgttgct tccaactgat gttgtcatag cagacaagtt tgctgctgat     840

gctaacagca agactgtgcc ggcatcaagc atcccagatg ggtggatggg gttggatatt     900

ggtcctgatt ccatcaagac atttggtgaa gcattggata cgactcagac cattatttgg     960

aatggaccaa tgggtgtttt tgagtttgat aagtttgcca caggaacaga ggctatagcc    1020

aagaaactgg cagagctgag tgggaaggga gtgacaacca tcattggagg aggtgactcc    1080

gttgccgctg tggagaaggt tggacttgca gacaagatga gccacatctc cactggtggt    1140

ggtgccagct tagagcttct tgagggaaag caactccctg gtgtccttgc tcttgatgat    1200

gct                                                                  1203


<210>  367

<211>  401
<212>  PRT
<213>  Glycine max

<400>  367

Met Ala Thr Lys Arg Ser Val Gly Thr Leu Lys Glu Gly Asp Leu Lys
1               5                   10                  15

Gly Lys Arg Val Phe Val Arg Val Asp Leu Asn Val Pro Leu Asp Asp
            20                  25                  30

Asn Leu Asn Ile Thr Asp Asp Thr Arg Val Arg Ala Ala Val Pro Thr
        35                  40                  45

Ile Lys Tyr Leu Thr Gly Tyr Gly Ala Lys Val Ile Leu Ser Ser His
    50                  55                  60

Leu Gly Arg Pro Lys Gly Val Thr Pro Lys Tyr Ser Leu Lys Pro Leu
65                  70                  75                  80

Val Pro Arg Leu Ser Gln Leu Leu Gly Ile Glu Val Thr Met Ala Asn
                85                  90                  95

Asp Ser Ile Gly Glu Glu Val Glu Lys Leu Val Thr Gln Leu Pro Glu
            100                 105                 110

Gly Gly Val Leu Leu Leu Glu Asn Val Arg Phe Tyr Lys Glu Glu Glu
        115                 120                 125

Lys Asn Asp Pro Glu Phe Ala Lys Lys Leu Ala Ser Leu Ala Asp Leu
    130                 135                 140

Tyr Val Asn Asp Ala Phe Gly Thr Ala His Arg Ala His Ala Ser Thr
145                 150                 155                 160

Glu Gly Val Ala Lys Tyr Leu Lys Pro Ser Val Ala Gly Phe Leu Met
                165                 170                 175

Gln Lys Glu Leu Asp Tyr Leu Val Gly Ala Val Ser Asn Pro Lys Arg
            180                 185                 190

Pro Phe Ala Ala Ile Val Gly Gly Ser Lys Val Ser Ser Lys Ile Gly
        195                 200                 205

Val Ile Glu Ser Leu Leu Glu Lys Val Asn Val Leu Leu Leu Gly Gly
    210                 215                 220

Gly Met Ile Phe Thr Phe Tyr Lys Ala Gln Gly Tyr Ser Val Gly Ser

225                 230                 235                 240

Ser Leu Val Glu Glu Asp Lys Leu Ser Leu Ala Thr Thr Leu Leu Glu
                245                 250                 255

Lys Ala Lys Ala Lys Gly Val Ser Leu Leu Leu Pro Thr Asp Val Val
            260                 265                 270

Ile Ala Asp Lys Phe Ala Ala Asp Ala Asn Ser Lys Thr Val Pro Ala
        275                 280                 285

Ser Ser Ile Pro Asp Gly Trp Met Gly Leu Asp Ile Gly Pro Asp Ser
    290                 295                 300

Ile Lys Thr Phe Gly Glu Ala Leu Asp Thr Thr Gln Thr Ile Ile Trp
305                 310                 315                 320

Asn Gly Pro Met Gly Val Phe Glu Phe Asp Lys Phe Ala Thr Gly Thr
                325                 330                 335

Glu Ala Ile Ala Lys Lys Leu Ala Glu Leu Ser Gly Lys Gly Val Thr
            340                 345                 350

Thr Ile Ile Gly Gly Gly Asp Ser Val Ala Ala Val Glu Lys Val Gly
        355                 360                 365

Leu Ala Asp Lys Met Ser His Ile Ser Thr Gly Gly Gly Ala Ser Leu
    370                 375                 380

Glu Leu Leu Glu Gly Lys Gln Leu Pro Gly Val Leu Ala Leu Asp Asp
385                 390                 395                 400

Ala

<210>  368
<211>  15
<212>  PRT
<213>  Glycine max

<400>  368

Ala Gln Gly Tyr Ser Val Gly Ser Ser Leu Val Glu Glu Asp Lys
1               5                   10                  15

<210>  369
<211>  13
<212>  PRT
<213>  Glycine max

<400>  369

Glu Leu Asp Tyr Leu Val Gly Ala Val Ser Asn Pro Lys
1               5                   10


<210>  370
<211>  8
<212>  PRT
<213>  Glycine max

<400>  370

Phe Ala Ala Asp Ala Asn Ser Lys
1               5


<210>  371
<211>  10
<212>  PRT
<213>  Glycine max

<400>  371

Phe Ala Thr Gly Thr Glu Ala Ile Ala Lys
1               5                   10


<210>  372
<211>  15
<212>  PRT
<213>  Glycine max

<400>  372

Gly Val Ser Leu Leu Leu Pro Thr Asp Val Val Ile Ala Asp Lys
1               5                   10                  15


<210>  373
<211>  17
<212>  PRT
<213>  Glycine max

<400>  373

Gly Val Thr Thr Ile Ile Gly Gly Gly Asp Ser Val Ala Ala Val Glu
1               5                   10                  15


Lys


<210>  374
<211>  10
<212>  PRT
<213>  Glycine max

<400>  374

Ile Gly Val Ile Glu Ser Leu Leu Glu Lys
1               5                   10

<210>  375
<211>  28
<212>  PRT
<213>  Glycine max

<400>  375

Leu Ala Ser Leu Ala Asp Leu Tyr Val Asn Asp Ala Phe Gly Thr Ala
1               5                   10                  15

His Arg Ala His Ala Ser Thr Glu Gly Val Ala Lys
            20                  25

<210>  376
<211>  10
<212>  PRT
<213>  Glycine max

<400>  376

Leu Ser Leu Ala Thr Thr Leu Leu Glu Lys
1               5                   10

<210>  377
<211>  20
<212>  PRT
<213>  Glycine max

<400>  377

Leu Val Thr Gln Leu Pro Glu Gly Gly Val Leu Leu Leu Glu Asn Val
1               5                   10                  15

Arg Phe Tyr Lys
            20

<210>  378
<211>  7
<212>  PRT
<213>  Glycine max

<400>  378

Asn Asp Pro Glu Phe Ala Lys
1               5

<210>  379
<211>  11
<212>  PRT
<213>  Glycine max

<400>  379

Gln Leu Pro Gly Val Leu Ala Leu Asp Asp Ala
1               5                   10

<210>  380
<211>  11
<212>  PRT
<213>  Glycine max

<400>  380

Arg Pro Phe Ala Ala Ile Val Gly Gly Ser Lys
1               5                   10


<210>  381
<211>  11
<212>  PRT
<213>  Glycine max

<400>  381

Val Ile Leu Ser Ser His Leu Gly Arg Pro Lys
1               5                   10


<210>  382
<211>  8
<212>  PRT
<213>  Glycine max

<400>  382

Tyr Leu Thr Gly Tyr Gly Ala Lys
1               5


<210>  383
<211>  1455
<212>  DNA
<213>  Glycine max

<400>  383
atggcctcag ccgcatcccc caccactttc tctctcctcc actccgccac cccttcctcc      60

tcctccaccg ccacctccgc ctccccctcc ccctcgcgcg tgcagctcac ctcctccctc     120

cgcgcccgcc cgctgcgccg cctgggcttc gccgcggcgg acccgctgct cgcggcgagt     180

gtagcggcgc gcgtggcggc ggtgagagga ggaggaaagg gcgcgagggg ggtggtgtcg     240

atggcgaaga agagcgtggg ggacctgagc gcggcggacc tgaaggggaa gaaggtcttc     300

gtcagggccg acctcaacgt cccgctcgac gacaaccaga acatcaccga cgacaccaga     360

atccgcgccg ccattcccac catcaaacat ctcatccaaa acggcgccaa agtcattctc     420

tccagccact tggggcggcc gaagggtgtc actcccaaat acagcttggc gcctcttgta     480

ccccggcttt ctgagctcat tggtatccag attgtcaagg ccgaggacag tattggtcca     540

gaagtagaaa agttggtggc ttctcttcca gatggaggtg ttctccttct agaaaatgtg     600

aggttttaca aggaggaaga aaagaatgac cccgagcatg caaaaaagct tgcctctctg     660

gcagatctgt atgtgaatga tgcatttggt actgctcaca gggcacatgc atcaacagag 720

ggtgttacta aatacctgaa gccatctgtt gctggttttc ttttgcaaaa ggaacttgat 780

taccttgttg gggcagtatc aaaccccaaa aggccatttg ctgccattgt tggtggttcc 840

aaagtctcat ctaaaattgg agtgattgag tcacttttag aaaaagttga catccttctt 900

cttggtggag gaatgatctt cacattttac aaggcacaag gtctttcagt gggttcatcc 960

cttgtagaag aagataagtt ggatcttgct acatcactac ttgcaaaagc caaggaaaag 1020

ggggtgtctc tcttgttacc aagtgatgtg gtgattgcag acaaatttgc tcctgatgct 1080

aatagcaagg tcgtgccagc atctgccatc cctgatggct ggatgggatt ggatattggt 1140

ccagattctg ttaaatcatt cagtgaagca ttggatacta cccaaaccat catatggaat 1200

ggaccaatgg gagtgtttga gtttgacaag tttgctgttg gtacagaggc tattgcaaag 1260

aagctggctg acctcagtgg aaagggggtg accacaatta ttggaggagg agattctgtt 1320

gcagctgtgg agaaagtagg agttgctagt gtcatgagcc acatatctac tggtggtggt 1380

gccagtttgg agttattgga aggcaaagag cttccaggag tccttgctct tgatgaagcc 1440

gtcccagtcg ctgtg 1455

<210> 384
<211> 485
<212> PRT
<213> Glycine max

<400> 384

Met Ala Ser Ala Ala Ser Pro Thr Thr Phe Ser Leu Leu His Ser Ala
1               5                   10                  15

Thr Pro Ser Ser Ser Ser Thr Ala Thr Ser Ala Ser Pro Ser Pro Ser
            20                  25                  30

Arg Val Gln Leu Thr Ser Ser Leu Arg Ala Arg Pro Leu Arg Arg Leu
        35                  40                  45

Gly Phe Ala Ala Ala Asp Pro Leu Leu Ala Ala Ser Val Ala Ala Arg
    50                  55                  60

Val Ala Ala Val Arg Gly Gly Gly Lys Gly Ala Arg Gly Val Val Ser
65                  70                  75                  80

Met Ala Lys Lys Ser Val Gly Asp Leu Ser Ala Ala Asp Leu Lys Gly
                85                  90                  95

Lys Lys Val Phe Val Arg Ala Asp Leu Asn Val Pro Leu Asp Asp Asn
            100                 105                 110

Gln Asn Ile Thr Asp Asp Thr Arg Ile Arg Ala Ala Ile Pro Thr Ile
        115                 120                 125

Lys His Leu Ile Gln Asn Gly Ala Lys Val Ile Leu Ser Ser His Leu
    130                 135                 140

Gly Arg Pro Lys Gly Val Thr Pro Lys Tyr Ser Leu Ala Pro Leu Val
145                 150                 155                 160

Pro Arg Leu Ser Glu Leu Ile Gly Ile Gln Ile Val Lys Ala Glu Asp
                165                 170                 175

Ser Ile Gly Pro Glu Val Glu Lys Leu Val Ala Ser Leu Pro Asp Gly
            180                 185                 190

Gly Val Leu Leu Leu Glu Asn Val Arg Phe Tyr Lys Glu Glu Glu Lys
        195                 200                 205

Asn Asp Pro Glu His Ala Lys Lys Leu Ala Ser Leu Ala Asp Leu Tyr
    210                 215                 220

Val Asn Asp Ala Phe Gly Thr Ala His Arg Ala His Ala Ser Thr Glu
225                 230                 235                 240

Gly Val Thr Lys Tyr Leu Lys Pro Ser Val Ala Gly Phe Leu Leu Gln
                245                 250                 255

Lys Glu Leu Asp Tyr Leu Val Gly Ala Val Ser Asn Pro Lys Arg Pro
            260                 265                 270

Phe Ala Ala Ile Val Gly Gly Ser Lys Val Ser Ser Lys Ile Gly Val
        275                 280                 285

Ile Glu Ser Leu Leu Glu Lys Val Asp Ile Leu Leu Leu Gly Gly Gly
    290                 295                 300

Met Ile Phe Thr Phe Tyr Lys Ala Gln Gly Leu Ser Val Gly Ser Ser
305                 310                 315                 320

Leu Val Glu Glu Asp Lys Leu Asp Leu Ala Thr Ser Leu Leu Ala Lys
                325                 330                 335

Ala Lys Glu Lys Gly Val Ser Leu Leu Leu Pro Ser Asp Val Val Ile
            340                 345                 350

Ala Asp Lys Phe Ala Pro Asp Ala Asn Ser Lys Val Val Pro Ala Ser
        355                 360                 365

Ala Ile Pro Asp Gly Trp Met Gly Leu Asp Ile Gly Pro Asp Ser Val
    370                 375                 380

Lys Ser Phe Ser Glu Ala Leu Asp Thr Thr Gln Thr Ile Ile Trp Asn
385                 390                 395                 400

Gly Pro Met Gly Val Phe Glu Phe Asp Lys Phe Ala Val Gly Thr Glu
                405                 410                 415

Ala Ile Ala Lys Lys Leu Ala Asp Leu Ser Gly Lys Gly Val Thr Thr
            420                 425                 430

Ile Ile Gly Gly Gly Asp Ser Val Ala Ala Val Glu Lys Val Gly Val
        435                 440                 445

Ala Ser Val Met Ser His Ile Ser Thr Gly Gly Gly Ala Ser Leu Glu
    450                 455                 460

Leu Leu Glu Gly Lys Glu Leu Pro Gly Val Leu Ala Leu Asp Glu Ala
465                 470                 475                 480

Val Pro Val Ala Val
                485

<210>  385
<211>  13
<212>  PRT
<213>  Glycine max

<400>  385

Glu Leu Asp Tyr Leu Val Gly Ala Val Ser Asn Pro Lys
1               5                   10

<210>  386
<211>  16
<212>  PRT
<213>  Glycine max

<400>  386

Glu Leu Pro Gly Val Leu Ala Leu Asp Glu Ala Val Pro Val Ala Val
1               5                   10                  15

<210>  387
<211>  10
<212>  PRT
<213>  Glycine max

<400>  387

Phe Ala Val Gly Thr Glu Ala Ile Ala Lys
1               5                   10

<210>  388
<211>  15
<212>  PRT
<213>  Glycine max

<400>  388

Gly Val Ser Leu Leu Leu Pro Ser Asp Val Val Ile Ala Asp Lys
1               5                   10                  15

<210>  389
<211>  17
<212>  PRT
<213>  Glycine max

<400>  389

Gly Val Thr Thr Ile Ile Gly Gly Gly Asp Ser Val Ala Ala Val Glu
1               5                   10                  15

Lys

<210>  390
<211>  10
<212>  PRT
<213>  Glycine max

<400>  390

Ile Gly Val Ile Glu Ser Leu Leu Glu Lys
1               5                   10

<210>  391
<211>  11
<212>  PRT
<213>  Glycine max

<400>  391

Arg Pro Phe Ala Ala Ile Val Gly Gly Ser Lys
1               5                   10

<210>  392
<211>  11
<212>  PRT
<213>  Glycine max

<400>  392

Val Ile Leu Ser Ser His Leu Gly Arg Pro Lys
1               5                   10

<210> 393
<211> 1203
<212> DNA
<213> Glycine max

<400> 393
atggcgacta agaggagcgt gggaacgttg aaggagggtg atttgaaagg gaagagggtg 60

ttcgtgaggg tcgatctgaa cgtgcctttg gatgacaacc ttaacatcac cgatgacact 120

agagtccgtg ctgctgttcc caccatcaag tacttaactg gtcatggagc caaagtgatc 180

ctttctagcc acttgggacg tcctaaaggt gtaacaccta aatacagttt gaagcctctt 240

gtgccaaggc tgtctcaact tctcggaatt gaggttacga tggcaaatga ctctattggg 300

gaggaagttg agaagttggt tgcacaactt ccagaaggtg gtgttttgct tctagagaat 360

gtgaggttct ataaggagga agagaagaat gaccctgagt ttgcaaagaa gttggcttct 420

cttgctgatc tctacgtgaa tgatgctttc ggcactgccc acagagctca tgcttctaca 480

gaaggagtgg ccaaatactt gaagccctct gttgcaggat tccttatgca gaaggaactt 540

gactatctag ttggagctgt gtcaaacccc aagagaccat ttgctgctat cgttggtggg 600

tcaaaggtgt cttccaagat tggagttatt gagtccttgt tggagaaagt taacgttctc 660

ttgcttggtg gaggaatgat ctttaccttt tacaaggctc agggttattc agttggttca 720

tctcttgtgg aggaagacaa gcttagcctt gcaactacac ttctcgaaaa ggccaaggct 780

aaaggggttt ctttgttgct tccaactgat gtggtcatag cagacaagtt tgctgctgat 840

gctaacagca agactgtgcc agcatcaagc atcccagacg ggtggatggg attagatatt 900

ggtcccgatt ccatcaagac atttggtgaa gcattggata agacccagac cgtcatttgg 960

aatggaccaa tgggtgtttt tgagtttgat aagtttgcca caggaacaga ggccatagcc 1020

aagaaactgg cagagctgag tgggaaggga gtaacgacca tcattggagg aggtgactct 1080

gttgccgctg tggagaaggt tggtcttgca gacaagatga gccacatctc cactggtggt 1140

ggtgccagct tagagcttct tgaggggaag caactccctg gtgtccttgc ccttgatgat 1200

gct 1203

<210> 394
<211> 401
<212> PRT
<213> Glycine max

<400> 394

Met Ala Thr Lys Arg Ser Val Gly Thr Leu Lys Glu Gly Asp Leu Lys
1               5                   10                  15

Gly Lys Arg Val Phe Val Arg Val Asp Leu Asn Val Pro Leu Asp Asp
            20                  25                  30

Asn Leu Asn Ile Thr Asp Asp Thr Arg Val Arg Ala Ala Val Pro Thr
        35                  40                  45

Ile Lys Tyr Leu Thr Gly His Gly Ala Lys Val Ile Leu Ser Ser His
    50                  55                  60

Leu Gly Arg Pro Lys Gly Val Thr Pro Lys Tyr Ser Leu Lys Pro Leu
65                  70                  75                  80

Val Pro Arg Leu Ser Gln Leu Leu Gly Ile Glu Val Thr Met Ala Asn
                85                  90                  95

Asp Ser Ile Gly Glu Glu Val Glu Lys Leu Val Ala Gln Leu Pro Glu
            100                 105                 110

Gly Gly Val Leu Leu Leu Glu Asn Val Arg Phe Tyr Lys Glu Glu Glu
        115                 120                 125

Lys Asn Asp Pro Glu Phe Ala Lys Lys Leu Ala Ser Leu Ala Asp Leu
    130                 135                 140

Tyr Val Asn Asp Ala Phe Gly Thr Ala His Arg Ala His Ala Ser Thr
145                 150                 155                 160

Glu Gly Val Ala Lys Tyr Leu Lys Pro Ser Val Ala Gly Phe Leu Met
                165                 170                 175

Gln Lys Glu Leu Asp Tyr Leu Val Gly Ala Val Ser Asn Pro Lys Arg
            180                 185                 190

Pro Phe Ala Ala Ile Val Gly Gly Ser Lys Val Ser Ser Lys Ile Gly
        195                 200                 205

Val Ile Glu Ser Leu Leu Glu Lys Val Asn Val Leu Leu Leu Gly Gly
    210                 215                 220

Gly Met Ile Phe Thr Phe Tyr Lys Ala Gln Gly Tyr Ser Val Gly Ser
225                 230                 235                 240

Ser Leu Val Glu Glu Asp Lys Leu Ser Leu Ala Thr Thr Leu Leu Glu
                245                 250                 255

Lys Ala Lys Ala Lys Gly Val Ser Leu Leu Leu Pro Thr Asp Val Val
            260                 265                 270

Ile Ala Asp Lys Phe Ala Ala Asp Ala Asn Ser Lys Thr Val Pro Ala
        275                 280                 285

Ser Ser Ile Pro Asp Gly Trp Met Gly Leu Asp Ile Gly Pro Asp Ser
    290                 295                 300

Ile Lys Thr Phe Gly Glu Ala Leu Asp Lys Thr Gln Thr Val Ile Trp
305                 310                 315                 320

Asn Gly Pro Met Gly Val Phe Glu Phe Asp Lys Phe Ala Thr Gly Thr
                325                 330                 335

Glu Ala Ile Ala Lys Lys Leu Ala Glu Leu Ser Gly Lys Gly Val Thr
            340                 345                 350

Thr Ile Ile Gly Gly Gly Asp Ser Val Ala Ala Val Glu Lys Val Gly
        355                 360                 365

Leu Ala Asp Lys Met Ser His Ile Ser Thr Gly Gly Gly Ala Ser Leu
    370                 375                 380

Glu Leu Leu Glu Gly Lys Gln Leu Pro Gly Val Leu Ala Leu Asp Asp
385                 390                 395                 400

Ala

<210>  395
<211>  15
<212>  PRT
<213>  Glycine max

<400>  395

Ala Gln Gly Tyr Ser Val Gly Ser Ser Leu Val Glu Glu Asp Lys
1               5                   10                  15

<210>  396
<211>  13
<212>  PRT
<213>  Glycine max

<400>  396

Glu Leu Asp Tyr Leu Val Gly Ala Val Ser Asn Pro Lys
1               5                   10

<210>  397
<211>  8
<212>  PRT
<213>  Glycine max

<400>  397

Phe Ala Ala Asp Ala Asn Ser Lys
1               5

<210>  398
<211>  10
<212>  PRT
<213>  Glycine max

<400>  398

Phe Ala Thr Gly Thr Glu Ala Ile Ala Lys
1               5                   10

<210>  399
<211>  15
<212>  PRT
<213>  Glycine max

<400>  399

Gly Val Ser Leu Leu Leu Pro Thr Asp Val Val Ile Ala Asp Lys
1               5                   10                  15

<210>  400
<211>  17
<212>  PRT
<213>  Glycine max

<400>  400

Gly Val Thr Thr Ile Ile Gly Gly Gly Asp Ser Val Ala Ala Val Glu
1               5                   10                  15

Lys

<210>  401
<211>  10
<212>  PRT
<213>  Glycine max

<400>  401

Ile Gly Val Ile Glu Ser Leu Leu Glu Lys
1               5                   10

<210>  402
<211>  28
<212>  PRT
<213>  Glycine max

<400>  402

Leu Ala Ser Leu Ala Asp Leu Tyr Val Asn Asp Ala Phe Gly Thr Ala
1               5                   10                  15

His Arg Ala His Ala Ser Thr Glu Gly Val Ala Lys
            20                  25

<210>  403
<211>  10
<212>  PRT
<213>  Glycine max

<400>  403

Leu Ser Leu Ala Thr Thr Leu Leu Glu Lys
1               5                   10

<210>  404
<211>  20
<212>  PRT
<213>  Glycine max

<400>  404

Leu Val Ala Gln Leu Pro Glu Gly Gly Val Leu Leu Leu Glu Asn Val
1               5                   10                  15

Arg Phe Tyr Lys
            20

<210>  405
<211>  7
<212>  PRT
<213>  Glycine max

<400>  405

Asn Asp Pro Glu Phe Ala Lys
1               5

<210>  406
<211>  11
<212>  PRT
<213>  Glycine max

<400>  406

Gln Leu Pro Gly Val Leu Ala Leu Asp Asp Ala
1               5                   10

<210>  407
<211>  11
<212>  PRT
<213>  Glycine max

<400>  407

Arg Pro Phe Ala Ala Ile Val Gly Gly Ser Lys
1               5                   10

<210>  408
<211>  8
<212>  PRT
<213>  Glycine max

<400>  408

Thr Phe Gly Glu Ala Leu Asp Lys
1               5


<210>  409
<211>  11
<212>  PRT
<213>  Glycine max

<400>  409

Val Ile Leu Ser Ser His Leu Gly Arg Pro Lys
1               5                   10


<210>  410
<211>  1143
<212>  DNA
<213>  Glycine max

<400>  410
atgtcgtcga gcacagttgg tcaagtcatc aaatgcaaag ctgcggtttc atgggaggca      60

gggaagccac ttgtgattga agaagtagag gtggcaccac cacaagctgg tgaagtgcgt     120

ttgaagatcc tctacacctc tctttgccac actgatgttt acttctggga agccaagggc     180

caaactccat tgtttcctcg tatatttggt catgaagctg gagggattgt ggagagcgta     240

ggtgagggtg tgactcatct caaaccaggg gaccatgccc tcccagtgtt taccggggag     300

tgtggagaat gccctcattg caagtcagag gagagcaaca tgtgtgatct tctcaggatc     360

aacactgata ggggtgtcat gatccatgac agccaaacaa gattttctat taagggacaa     420

cctatttacc attttgttgg tacctccaca ttcagtgaat acactgttgt tcatgccgga     480

tgtgttgcca aagtcaaccc tgctgcccca cttgacaaaa tttgtgttct cagttgtgga     540

atttgcacag gtcttggggc tactatcaat gttgcaaaac caaaacctgg ttcttctgtt     600

gccatttttg gacttggagc tgttggcctt gctgctgctg aaggggcaag gatttctggt     660

gcatcaagaa ttattggggt tgatttagtt tctagcagat ttgaagaagc taagaagttt     720

ggggtcaatg aatttgtgaa cccaaaagac catgacaagc ctgtacaaga ggtgattgct     780

gcaatgacga atgggggtgt ggatcgtgct gttgaatgta ctggcagcat ccaagccatg     840

atctcggcat tcgaatgcgt ccacgatggt tggggtgttg ctgtacttgt tggagtgcca     900

aacaaagatg atgcattcaa aactcatcca gtgaatttct tgaacgagag aactctaaag     960

ggtaccttct acggaaacta caaaccccgc actgatcttc cttctgttgt ggagaagtac    1020

atgaatgggg aattggaact ggagaaattc atcactcaca cagttccatt ctcagagatt    1080

aacaaggctt ttgattacat gctgaaaggg gagtccatca gatgcatcat tcgaatgggg     1140

gag                                                                   1143

<210>  411
<211>  381
<212>  PRT
<213>  Glycine max

<400>  411

Met Ser Ser Ser Thr Val Gly Gln Val Ile Lys Cys Lys Ala Ala Val
1               5                   10                  15

Ser Trp Glu Ala Gly Lys Pro Leu Val Ile Glu Glu Val Glu Val Ala
            20                  25                  30

Pro Pro Gln Ala Gly Glu Val Arg Leu Lys Ile Leu Tyr Thr Ser Leu
        35                  40                  45

Cys His Thr Asp Val Tyr Phe Trp Glu Ala Lys Gly Gln Thr Pro Leu
    50                  55                  60

Phe Pro Arg Ile Phe Gly His Glu Ala Gly Gly Ile Val Glu Ser Val
65                  70                  75                  80

Gly Glu Gly Val Thr His Leu Lys Pro Gly Asp His Ala Leu Pro Val
                85                  90                  95

Phe Thr Gly Glu Cys Gly Glu Cys Pro His Cys Lys Ser Glu Glu Ser
            100                 105                 110

Asn Met Cys Asp Leu Leu Arg Ile Asn Thr Asp Arg Gly Val Met Ile
        115                 120                 125

His Asp Ser Gln Thr Arg Phe Ser Ile Lys Gly Gln Pro Ile Tyr His
    130                 135                 140

Phe Val Gly Thr Ser Thr Phe Ser Glu Tyr Thr Val Val His Ala Gly
145                 150                 155                 160

Cys Val Ala Lys Val Asn Pro Ala Ala Pro Leu Asp Lys Ile Cys Val
                165                 170                 175

Leu Ser Cys Gly Ile Cys Thr Gly Leu Gly Ala Thr Ile Asn Val Ala
            180                 185                 190

Lys Pro Lys Pro Gly Ser Ser Val Ala Ile Phe Gly Leu Gly Ala Val
        195                 200                 205

Gly Leu Ala Ala Ala Glu Gly Ala Arg Ile Ser Gly Ala Ser Arg Ile
    210                 215                 220

Ile Gly Val Asp Leu Val Ser Ser Arg Phe Glu Glu Ala Lys Lys Phe
225                 230                 235                 240

Gly Val Asn Glu Phe Val Asn Pro Lys Asp His Asp Lys Pro Val Gln
                245                 250                 255

Glu Val Ile Ala Ala Met Thr Asn Gly Gly Val Asp Arg Ala Val Glu
            260                 265                 270

Cys Thr Gly Ser Ile Gln Ala Met Ile Ser Ala Phe Glu Cys Val His
        275                 280                 285

Asp Gly Trp Gly Val Ala Val Leu Val Gly Val Pro Asn Lys Asp Asp
    290                 295                 300

Ala Phe Lys Thr His Pro Val Asn Phe Leu Asn Glu Arg Thr Leu Lys
305                 310                 315                 320

Gly Thr Phe Tyr Gly Asn Tyr Lys Pro Arg Thr Asp Leu Pro Ser Val
                325                 330                 335

Val Glu Lys Tyr Met Asn Gly Glu Leu Glu Leu Glu Lys Phe Ile Thr
            340                 345                 350

His Thr Val Pro Phe Ser Glu Ile Asn Lys Ala Phe Asp Tyr Met Leu
        355                 360                 365

Lys Gly Glu Ser Ile Arg Cys Ile Ile Arg Met Gly Glu
    370                 375                 380


<210>  412
<211>  10
<212>  PRT
<213>  Glycine max

<400>  412

Phe Gly Val Asn Glu Phe Val Asn Pro Lys
1               5                   10


<210>  413
<211>  13
<212>  PRT
<213>  Glycine max

<400>  413

Phe Ile Thr His Thr Val Pro Phe Ser Glu Ile Asn Lys
1               5                   10

<210>  414
<211>  19
<212>  PRT
<213>  Glycine max

<400>  414

Gly Thr Phe Tyr Gly Asn Tyr Lys Pro Arg Thr Asp Leu Pro Ser Val
1               5                   10                  15

Val Glu Lys

<210>  415
<211>  13
<212>  PRT
<213>  Glycine max

<400>  415

Thr His Pro Val Asn Phe Leu Asn Glu Arg Thr Leu Lys
1               5                   10

<210>  416
<211>  9
<212>  PRT
<213>  Glycine max

<400>  416

Val Asn Pro Ala Ala Pro Leu Asp Lys
1               5

<210>  417
<211>  1113
<212>  DNA
<213>  Glycine max

<400>  417
atgcgtgcag ctgcggtggc atgggaagca gggaagccac tgtcaataga aaccatagaa      60

gtggcaccac cccagaaagg tgaagtgcgt ttgaggatcc tcttcaactc cctttgccgt     120

agtgatgttt attggtggga tgctaaggac cagactcctc tgtttcctcg tatcttaggc     180

cacgaagctt cagggattgt ggagagcgtg ggcgagggtg tgacacatct gaaaccaggt     240

gaccatgcac tcccaatatt cactggggag tgtggtgaat gcacttattg taagtctgaa     300

gagagcaacc tgtgtgagct actcaggatc aacactgaca ggggtgttat gctgagtgat     360

ggaaaaacaa ggttctccaa aaatggccaa cccatttacc acttcgttgg aacctccact     420

ttcagtgaat acactgttct ccatgaagga tgtgtagcca agattaaccc taatgcccca     480

cttgacaaag ttgctattgt cagctgtgga ttctgcacag gttttggggc tactgtgaat 540

gttgcaaaac caaagcctaa taatactgtt gctgtctttg gcttaggagc tgttggtctt 600

gctgcctgtg aaggggcaag ggtttctggt gcatctagaa tcatcggggt tgatttactt 660

cccaatcgat ttgaacaagc caaaaaattt ggggtcactg attttgtaaa cccgaaagat 720

cacaacaaac cagtccaaga ggtcattgct gaaatgacta atggaggagt agatcgtgct 780

attgaatgca ctggaagcat ccaagcttca atctcagcat ttgaatgcac tcatgatggt 840

tggggtactg ctgtccttgt tggtgtgcca aagaaggatg ttgaattcaa aactaatcct 900

atgaagttca tggaagggag aactcttaag ggtaccttct atggtcacta cagaccccgc 960

actgacattc ctggtgttgt ggagaagtac ctcaacaagg agttggaact agacaaattt 1020

atcactcact ctgtgccatt ctcaaagatc aacactgcat ttgatcttat gctgaaaggg 1080

gagggcataa ggtgcctcat ctgcatggaa gag 1113

<210> 418
<211> 371
<212> PRT
<213> Glycine max

<400> 418

Met Arg Ala Ala Ala Val Ala Trp Glu Ala Gly Lys Pro Leu Ser Ile
1               5                   10                  15

Glu Thr Ile Glu Val Ala Pro Pro Gln Lys Gly Glu Val Arg Leu Arg
            20                  25                  30

Ile Leu Phe Asn Ser Leu Cys Arg Ser Asp Val Tyr Trp Trp Asp Ala
        35                  40                  45

Lys Asp Gln Thr Pro Leu Phe Pro Arg Ile Leu Gly His Glu Ala Ser
    50                  55                  60

Gly Ile Val Glu Ser Val Gly Glu Gly Val Thr His Leu Lys Pro Gly
65                  70                  75                  80

Asp His Ala Leu Pro Ile Phe Thr Gly Glu Cys Gly Glu Cys Thr Tyr
                85                  90                  95

Cys Lys Ser Glu Glu Ser Asn Leu Cys Glu Leu Leu Arg Ile Asn Thr
            100                 105                 110

Asp Arg Gly Val Met Leu Ser Asp Gly Lys Thr Arg Phe Ser Lys Asn
        115                 120                 125

Gly Gln Pro Ile Tyr His Phe Val Gly Thr Ser Thr Phe Ser Glu Tyr
    130                 135                 140

Thr Val Leu His Glu Gly Cys Val Ala Lys Ile Asn Pro Asn Ala Pro
145                 150                 155                 160

Leu Asp Lys Val Ala Ile Val Ser Cys Gly Phe Cys Thr Gly Phe Gly
                165                 170                 175

Ala Thr Val Asn Val Ala Lys Pro Lys Pro Asn Asn Thr Val Ala Val
            180                 185                 190

Phe Gly Leu Gly Ala Val Gly Leu Ala Ala Cys Glu Gly Ala Arg Val
        195                 200                 205

Ser Gly Ala Ser Arg Ile Ile Gly Val Asp Leu Leu Pro Asn Arg Phe
    210                 215                 220

Glu Gln Ala Lys Lys Phe Gly Val Thr Asp Phe Val Asn Pro Lys Asp
225                 230                 235                 240

His Asn Lys Pro Val Gln Glu Val Ile Ala Glu Met Thr Asn Gly Gly
                245                 250                 255

Val Asp Arg Ala Ile Glu Cys Thr Gly Ser Ile Gln Ala Ser Ile Ser
            260                 265                 270

Ala Phe Glu Cys Thr His Asp Gly Trp Gly Thr Ala Val Leu Val Gly
        275                 280                 285

Val Pro Lys Lys Asp Val Glu Phe Lys Thr Asn Pro Met Lys Phe Met
    290                 295                 300

Glu Gly Arg Thr Leu Lys Gly Thr Phe Tyr Gly His Tyr Arg Pro Arg
305                 310                 315                 320

Thr Asp Ile Pro Gly Val Val Glu Lys Tyr Leu Asn Lys Glu Leu Glu
                325                 330                 335

Leu Asp Lys Phe Ile Thr His Ser Val Pro Phe Ser Lys Ile Asn Thr
            340                 345                 350

Ala Phe Asp Leu Met Leu Lys Gly Glu Gly Ile Arg Cys Leu Ile Cys
        355                 360                 365

Met Glu Glu
    370

<210>  419
<211>  19
<212>  PRT
<213>  Glycine max

<400>  419

Gly Thr Phe Tyr Gly His Tyr Arg Pro Arg Thr Asp Ile Pro Gly Val
1               5                   10                  15

Val Glu Lys

<210>  420
<211>  9
<212>  PRT
<213>  Glycine max

<400>  420

Ile Asn Pro Asn Ala Pro Leu Asp Lys
1               5

<210>  421
<211>  1140
<212>  DNA
<213>  Glycine max

<400>  421
   atgtctagca ccgttggcca gaccatcaag tgcaaagctg cgattgcatg ggaggctggg      60
   aagccgctgg tgattgaaga agtggaggtt gcgccaccgc aagccggtga agtccgtttg     120
   aagatcctct acacctctct ttgccacact gatgtttact tctgggatgc gaagggccag     180
   actccattgt ttcctcgcat ttttggccat gaagcttcgg ggattgtgga gagcgtaggc     240
   gagggtgtga ctcatctgaa accaggtgac catgccctcc ctgtgttcac aggagagtgt     300
   ggagattgcg cccattgcaa gtcagaggag agcaacatgt gtgagctact caggatcaac     360
   accgataggg gtgtcatgat ccatgatggc caatcaaggt tctctaagaa tggacaaccc     420
   atacaccatt tcttgggaac ctctacattc agtgaataca ctgttgtcca tgctggatgt     480
   gttgcaaaga tcaaccctgc tgctccactt gacaaagttt gtgttctcag ttgtggaatt     540
   tgcacaggtt ttggtgctac tgtaaatgta gcaaaaccaa aacctggttc ctctgttgcc     600
   atatttggac ttggagctgt tggccttgcg gctgctgaag gtgcaagagt ttcaggtgct     660
   tcaagaatca ttggagttga tttagtttct gcccgatttg aagaagctaa gaagtttggg     720
   gttaatgagt ttgtgaaccc aaaggatcat gataaacctg tgcaacaggt aattgctgaa     780
   atgaccaatg gaggtgtgga tcgggctgtt gaatgtactg gcagcatcca agccatggtc     840
   tcagcattcg aatgcgtcca cgatggttgg ggtcttgctg tacttgttgg tgtgcctagt     900
   aaagatgatg cattcaaaac tgctcctatt aatttcctga acgagaggac tcttaagggc 960
   accttttatg gcaactacaa accacgcacc gatcttccat ctgttgtcga gaagtacatg 1020
   agtggggagc tagaagtgga caaattcatc actcacacag ttccattctc agagatcaac 1080
   aaagcttttg atttaatgct gaagggacag tccattaggt gtatcatccg catgcaagag 1140

<210> 422
<211> 380
<212> PRT
<213> Glycine max

<400> 422

Met Ser Ser Thr Val Gly Gln Thr Ile Lys Cys Lys Ala Ala Ile Ala
1               5                   10                  15

Trp Glu Ala Gly Lys Pro Leu Val Ile Glu Glu Val Glu Val Ala Pro
            20                  25                  30

Pro Gln Ala Gly Glu Val Arg Leu Lys Ile Leu Tyr Thr Ser Leu Cys
        35                  40                  45

His Thr Asp Val Tyr Phe Trp Asp Ala Lys Gly Gln Thr Pro Leu Phe
    50                  55                  60

Pro Arg Ile Phe Gly His Glu Ala Ser Gly Ile Val Glu Ser Val Gly
65                  70                  75                  80

Glu Gly Val Thr His Leu Lys Pro Gly Asp His Ala Leu Pro Val Phe
                85                  90                  95

Thr Gly Glu Cys Gly Asp Cys Ala His Cys Lys Ser Glu Glu Ser Asn
            100                 105                 110

Met Cys Glu Leu Leu Arg Ile Asn Thr Asp Arg Gly Val Met Ile His
        115                 120                 125

Asp Gly Gln Ser Arg Phe Ser Lys Asn Gly Gln Pro Ile His His Phe
    130                 135                 140

Leu Gly Thr Ser Thr Phe Ser Glu Tyr Thr Val Val His Ala Gly Cys
145                 150                 155                 160

Val Ala Lys Ile Asn Pro Ala Ala Pro Leu Asp Lys Val Cys Val Leu
                165                 170                 175

Ser Cys Gly Ile Cys Thr Gly Phe Gly Ala Thr Val Asn Val Ala Lys
            180                 185                 190

Pro Lys Pro Gly Ser Ser Val Ala Ile Phe Gly Leu Gly Ala Val Gly
        195                 200                 205

Leu Ala Ala Ala Glu Gly Ala Arg Val Ser Gly Ala Ser Arg Ile Ile
    210                 215                 220

Gly Val Asp Leu Val Ser Ala Arg Phe Glu Glu Ala Lys Lys Phe Gly
225                 230                 235                 240

Val Asn Glu Phe Val Asn Pro Lys Asp His Asp Lys Pro Val Gln Gln
                245                 250                 255

Val Ile Ala Glu Met Thr Asn Gly Gly Val Asp Arg Ala Val Glu Cys
            260                 265                 270

Thr Gly Ser Ile Gln Ala Met Val Ser Ala Phe Glu Cys Val His Asp
        275                 280                 285

Gly Trp Gly Leu Ala Val Leu Val Gly Val Pro Ser Lys Asp Asp Ala
    290                 295                 300

Phe Lys Thr Ala Pro Ile Asn Phe Leu Asn Glu Arg Thr Leu Lys Gly
305                 310                 315                 320

Thr Phe Tyr Gly Asn Tyr Lys Pro Arg Thr Asp Leu Pro Ser Val Val
                325                 330                 335

Glu Lys Tyr Met Ser Gly Glu Leu Glu Val Asp Lys Phe Ile Thr His
            340                 345                 350

Thr Val Pro Phe Ser Glu Ile Asn Lys Ala Phe Asp Leu Met Leu Lys
        355                 360                 365

Gly Gln Ser Ile Arg Cys Ile Ile Arg Met Gln Glu
    370                 375                 380

<210>  423
<211>  10
<212>  PRT
<213>  Glycine max

<400>  423

Phe Gly Val Asn Glu Phe Val Asn Pro Lys
1               5                   10

<210>  424
<211>  13
<212>  PRT

<213>  Glycine max

<400>  424

Phe Ile Thr His Thr Val Pro Phe Ser Glu Ile Asn Lys
1               5                   10

<210>  425
<211>  19
<212>  PRT
<213>  Glycine max

<400>  425

Gly Thr Phe Tyr Gly Asn Tyr Lys Pro Arg Thr Asp Leu Pro Ser Val
1               5                   10                  15

Val Glu Lys

<210>  426
<211>  9
<212>  PRT
<213>  Glycine max

<400>  426

Ile Asn Pro Ala Ala Pro Leu Asp Lys
1               5

<210>  427
<211>  13
<212>  PRT
<213>  Glycine max

<400>  427

Thr Ala Pro Ile Asn Phe Leu Asn Glu Arg Thr Leu Lys
1               5                   10

<210>  428
<211>  927
<212>  DNA
<213>  Glycine max

<400>  428
atggcgtcaa gacagcaatt caaggaagac agagccgagg cagctgcaaa actcgcagcg      60

aaagacatcg gcgacatcaa cagaacaaac gagcgcgacg aggggtacga cttgcacgac     120

gaagccaact tcaagcaagc acgagctgaa gcagctgcaa agctggcggc caaggatctg     180

gaggacgcta acagattaag agacaacact ttcaacacat ggaaagagca acatcagcat     240

gatgagaaca aacccggtgt gatagggtcc atgtttagag ccgccaagga agccgtggtt     300

gggaagcctc accacgagga agtttatgca aaggaaacaa agatgggtga atattcagat     360

tatgcgactc agaaggcgag ggaaacgaag cagaaggctg gggagtatac agactatgcg     420

gctcagaaag caaaagaaac caaagactat gctgctgaga aggctaagaa tgctaaagat     480

accactgtgc agaaagctgg agagtacaag gattataccg ctgagaaggc taagaatgct     540

aaagatacca ctgtgcagaa agctggagaa tacaaggatt ataccgctga gaaggcaatt     600

gaagggaagg acagtgcagt ggggaagctt ggggagctaa aggaatctgc tgctgatgct     660

gctaaaaggg ctatgggttt cttcaccggt ggcaacaaag accaaactag agaggaggag     720

gaggaaacga ggcggagtat gcaggaggtg agggtgcaag ataaggagta tggaacggga     780

cgtggtactg gagagaagtt ggtgataaaa atggaagagt cgcggccagg agcggtggcg     840

gatgcgctca aagcagcgaa tagggccatg gacggcgata tggaggagga aggcgtgctt     900

catgtggagc gtcgtaggga gaaaatg                                         927

<210>  429
<211>  309
<212>  PRT
<213>  Glycine max

<400>  429

Met Ala Ser Arg Gln Gln Phe Lys Glu Asp Arg Ala Glu Ala Ala Ala
1               5                   10                  15

Lys Leu Ala Ala Lys Asp Ile Gly Asp Ile Asn Arg Thr Asn Glu Arg
            20                  25                  30

Asp Glu Gly Tyr Asp Leu His Asp Glu Ala Asn Phe Lys Gln Ala Arg
        35                  40                  45

Ala Glu Ala Ala Ala Lys Leu Ala Ala Lys Asp Leu Glu Asp Ala Asn
    50                  55                  60

Arg Leu Arg Asp Asn Thr Phe Asn Thr Trp Lys Glu Gln His Gln His
65                  70                  75                  80

Asp Glu Asn Lys Pro Gly Val Ile Gly Ser Met Phe Arg Ala Ala Lys
                85                  90                  95

Glu Ala Val Val Gly Lys Pro His His Glu Glu Val Tyr Ala Lys Glu
            100                 105                 110

Thr Lys Met Gly Glu Tyr Ser Asp Tyr Ala Thr Gln Lys Ala Arg Glu
        115                 120                 125

Thr Lys Gln Lys Ala Gly Glu Tyr Thr Asp Tyr Ala Ala Gln Lys Ala
    130                 135                 140

Lys Glu Thr Lys Asp Tyr Ala Ala Glu Lys Ala Lys Asn Ala Lys Asp
145                 150                 155                 160

Thr Thr Val Gln Lys Ala Gly Glu Tyr Lys Asp Tyr Thr Ala Glu Lys
                165                 170                 175

Ala Lys Asn Ala Lys Asp Thr Thr Val Gln Lys Ala Gly Glu Tyr Lys
            180                 185                 190

Asp Tyr Thr Ala Glu Lys Ala Ile Glu Gly Lys Asp Ser Ala Val Gly
        195                 200                 205

Lys Leu Gly Glu Leu Lys Glu Ser Ala Ala Asp Ala Ala Lys Arg Ala
    210                 215                 220

Met Gly Phe Phe Thr Gly Gly Asn Lys Asp Gln Thr Arg Glu Glu Glu
225                 230                 235                 240

Glu Glu Thr Arg Arg Ser Met Gln Glu Val Arg Val Gln Asp Lys Glu
                245                 250                 255

Tyr Gly Thr Gly Arg Gly Thr Gly Glu Lys Leu Val Ile Lys Met Glu
            260                 265                 270

Glu Ser Arg Pro Gly Ala Val Ala Asp Ala Leu Lys Ala Ala Asn Arg
        275                 280                 285

Ala Met Asp Gly Asp Met Glu Glu Glu Gly Val Leu His Val Glu Arg
    290                 295                 300

Arg Arg Glu Lys Met
305

<210>  430
<211>  11
<212>  PRT
<213>  Glycine max

<400>  430

Ala Gly Glu Tyr Thr Asp Tyr Ala Ala Gln Lys
1               5                   10

<210>  431
<211>  24
<212>  PRT
<213>  Glycine max

<400>  431

Asp Ile Gly Asp Ile Asn Arg Thr Asn Glu Arg Asp Glu Gly Tyr Asp
1               5                   10                  15

Leu His Asp Glu Ala Asn Phe Lys
            20

<210>  432
<211>  17
<212>  PRT
<213>  Glycine max

<400>  432

Asp Leu Glu Asp Ala Asn Arg Leu Arg Asp Asn Thr Phe Asn Thr Trp
1               5                   10                  15

Lys

<210>  433
<211>  15
<212>  PRT
<213>  Glycine max

<400>  433

Glu Ala Val Val Gly Lys Pro His His Glu Glu Val Tyr Ala Lys
1               5                   10                  15

<210>  434
<211>  1104
<212>  DNA
<213>  Glycine max

<400>  434
atgggcaagg tcaagatcgg aatcaacgga tttggaagaa ttggccgttt ggtagccaga      60

gtggctctgc agagagacga tgttgaactc gttgccgtta acgacccttt catcaccacc     120

gattacatga catacatgtt taaatacgac agtgttcatg gacactggaa gcatcacgat     180

gtcaccgtta aggacgagaa gacccttctc ttcggtgaca agccagtcac tatttttgga     240

cacaggtttt tatttttcac tttaattttt cttactgttt atgattgttg tgtcctgcat     300

gtaaacgttg agtgttgttt tctctgtgaa ctcagaaacc ctgaagagat cccatggggg     360

tcaactggag ctgacatcat tgttgagtcc accggagttt tcaccgataa ggacaaggcc     420

gccgcacatt tgaagggtgg tgcaaagaag gttattattt ctgcccccag taaggatgcc     480

cccatgtttg ttgttggtgt caacgagcac gagtacaagc cagagcttga tattatttcc     540

aatgctagct gcacaaccaa ctgccttgcc ccacttgcca aggttatcaa tgacaggttt     600

ggcattgttg agggtttgat gaccactgtt cattccatca ccgctaccca gaagactgtt     660

gatggaccat cagccaagga ctggagaggt ggaagagctg cttcatttaa catcattcct     720

agcagcactg gagctgccaa ggctgttggg aaagtcctcc ctgctttgaa tggaaaattg 780

actggtatgg cattccgtgt tcccaccgtg gatgtctctg ttgttgacct cacagtgagg 840

ctggagaaag aagcttccta cgatgaaatt aaaaatgcta tcaaggagga atcagagggc 900

aagttgaagg gaattcttgg ttacactgaa gatgatgtgg tctccactga ctttatcggc 960

gatagcagat caagtatttt tgatgcaaag gctggaattg cattgaataa gaactttgtg 1020

aagcttgttt cttggtacga caacgagtgg ggatacagct cacgtgtcat tgatcttctt 1080

gtattcgttg ccaagaagtc tctt 1104

<210> 435
<211> 368
<212> PRT
<213> Glycine max

<400> 435

Met Gly Lys Val Lys Ile Gly Ile Asn Gly Phe Gly Arg Ile Gly Arg
1               5                   10                  15

Leu Val Ala Arg Val Ala Leu Gln Arg Asp Asp Val Glu Leu Val Ala
            20                  25                  30

Val Asn Asp Pro Phe Ile Thr Thr Asp Tyr Met Thr Tyr Met Phe Lys
        35                  40                  45

Tyr Asp Ser Val His Gly His Trp Lys His His Asp Val Thr Val Lys
    50                  55                  60

Asp Glu Lys Thr Leu Leu Phe Gly Asp Lys Pro Val Thr Ile Phe Gly
65                  70                  75                  80

His Arg Phe Leu Phe Phe Thr Leu Ile Phe Leu Thr Val Tyr Asp Cys
                85                  90                  95

Cys Val Leu His Val Asn Val Glu Cys Cys Phe Leu Cys Glu Leu Arg
            100                 105                 110

Asn Pro Glu Glu Ile Pro Trp Gly Ser Thr Gly Ala Asp Ile Ile Val
        115                 120                 125

Glu Ser Thr Gly Val Phe Thr Asp Lys Asp Lys Ala Ala Ala His Leu
    130                 135                 140

Lys Gly Gly Ala Lys Lys Val Ile Ile Ser Ala Pro Ser Lys Asp Ala
145                 150                 155                 160

Pro Met Phe Val Val Gly Val Asn Glu His Glu Tyr Lys Pro Glu Leu
                165                 170                 175

Asp Ile Ile Ser Asn Ala Ser Cys Thr Thr Asn Cys Leu Ala Pro Leu
            180                 185                 190

Ala Lys Val Ile Asn Asp Arg Phe Gly Ile Val Glu Gly Leu Met Thr
        195                 200                 205

Thr Val His Ser Ile Thr Ala Thr Gln Lys Thr Val Asp Gly Pro Ser
    210                 215                 220

Ala Lys Asp Trp Arg Gly Gly Arg Ala Ala Ser Phe Asn Ile Ile Pro
225                 230                 235                 240

Ser Ser Thr Gly Ala Ala Lys Ala Val Gly Lys Val Leu Pro Ala Leu
                245                 250                 255

Asn Gly Lys Leu Thr Gly Met Ala Phe Arg Val Pro Thr Val Asp Val
            260                 265                 270

Ser Val Val Asp Leu Thr Val Arg Leu Glu Lys Glu Ala Ser Tyr Asp
        275                 280                 285

Glu Ile Lys Asn Ala Ile Lys Glu Glu Ser Glu Gly Lys Leu Lys Gly
    290                 295                 300

Ile Leu Gly Tyr Thr Glu Asp Asp Val Val Ser Thr Asp Phe Ile Gly
305                 310                 315                 320

Asp Ser Arg Ser Ser Ile Phe Asp Ala Lys Ala Gly Ile Ala Leu Asn
                325                 330                 335

Lys Asn Phe Val Lys Leu Val Ser Trp Tyr Asp Asn Glu Trp Gly Tyr
            340                 345                 350

Ser Ser Arg Val Ile Asp Leu Leu Val Phe Val Ala Lys Lys Ser Leu
        355                 360                 365

<210>  436
<211>  8
<212>  PRT
<213>  Glycine max

<400>  436

Glu Ala Ser Tyr Asp Glu Ile Lys
1               5

<210>  437
<211>  27
<212>  PRT
<213>  Glycine max

<400>  437

Gly Ile Leu Gly Tyr Thr Glu Asp Asp Val Val Ser Thr Asp Phe Ile
1               5                   10                  15

Gly Asp Ser Arg Ser Ser Ile Phe Asp Ala Lys
            20                  25

<210>  438
<211>  8
<212>  PRT
<213>  Glycine max

<400>  438

Val Ile Ile Ser Ala Pro Ser Lys
1               5

<210>  439
<211>  8
<212>  PRT
<213>  Glycine max

<400>  439

Val Leu Pro Ala Leu Asn Gly Lys
1               5

<210>  440
<211>  1014
<212>  DNA
<213>  Glycine max

<400>  440
atgggcaagg tcaagatcgg aatcaacgga tttggaagaa ttggccgttt ggttgccaga      60

gtcgctctgc aaagagacga tgtggagctc gttgccgtta acgacccttt cattaccacc     120

gattacatga catacatgtt taaatacgac agtgttcacg gacactggaa gcatcacgat     180

gtcaccgtta aggacgagaa gacccttctc ttcggtgaca agccagtcac tgtttttgga     240

cacaggaacc ctgaagagat cccatggggg tcaactggag ctgatatcat tgttgagtcc     300

accggagttt tcaccgataa ggacaaggcc gccgcacatt tgaagggtgg tgcgaagaag     360

gttattattt ctgcccccag taaggatgcc cccatgtttg ttgttggtgt taacgagcac     420

gagtacaagc cagagcttga tattatttcc aatgctagct gcacaaccaa ctgccttgct     480

ccacttgcta aggttattaa tgacaggttt ggcattgttg agggtttgat gaccactgtt     540

cattccatca ctgccaccca gaagactgtt gatgggccat cagccaaaga ctggagaggt     600

ggaagagctg cttcatttaa catcattcct agcagcactg gagctgccaa ggctgttgga 660

aaagtccttc ctgctttgaa cggaaaattg actggtatgg cattccgtgt tcccaccgtg 720

gatgtctctg ttgttgacct cacagtgagg ctggagaagg aagcctccta cgatgaaatt 780

aaaaatgcta tcaaggagga atcagagggc aagttgaagg gaattcttgg ttacactgaa 840

gatgatgtgg tctccaccga ctttgtgggc gatagcagat caagtatttt tgatgcaaag 900

gctggaattg cattgaataa gaactttgtg aagcttgtct cttggtatga caacgagtgg 960

ggatacagct cacgtgtcat tgacctgctt gtattcgttg ccaagaagtc tttt 1014

<210> 441
<211> 338
<212> PRT
<213> Glycine max

<400> 441

Met Gly Lys Val Lys Ile Gly Ile Asn Gly Phe Gly Arg Ile Gly Arg
1               5                   10                  15

Leu Val Ala Arg Val Ala Leu Gln Arg Asp Asp Val Glu Leu Val Ala
            20                  25                  30

Val Asn Asp Pro Phe Ile Thr Thr Asp Tyr Met Thr Tyr Met Phe Lys
        35                  40                  45

Tyr Asp Ser Val His Gly His Trp Lys His His Asp Val Thr Val Lys
    50                  55                  60

Asp Glu Lys Thr Leu Leu Phe Gly Asp Lys Pro Val Thr Val Phe Gly
65                  70                  75                  80

His Arg Asn Pro Glu Glu Ile Pro Trp Gly Ser Thr Gly Ala Asp Ile
                85                  90                  95

Ile Val Glu Ser Thr Gly Val Phe Thr Asp Lys Asp Lys Ala Ala Ala
            100                 105                 110

His Leu Lys Gly Gly Ala Lys Lys Val Ile Ile Ser Ala Pro Ser Lys
        115                 120                 125

Asp Ala Pro Met Phe Val Val Gly Val Asn Glu His Glu Tyr Lys Pro
    130                 135                 140

Glu Leu Asp Ile Ile Ser Asn Ala Ser Cys Thr Thr Asn Cys Leu Ala
145                 150                 155                 160

Pro Leu Ala Lys Val Ile Asn Asp Arg Phe Gly Ile Val Glu Gly Leu

                165                 170                 175

Met Thr Thr Val His Ser Ile Thr Ala Thr Gln Lys Thr Val Asp Gly
            180                 185                 190

Pro Ser Ala Lys Asp Trp Arg Gly Gly Arg Ala Ala Ser Phe Asn Ile
        195                 200                 205

Ile Pro Ser Ser Thr Gly Ala Ala Lys Ala Val Gly Lys Val Leu Pro
    210                 215                 220

Ala Leu Asn Gly Lys Leu Thr Gly Met Ala Phe Arg Val Pro Thr Val
225                 230                 235                 240

Asp Val Ser Val Val Asp Leu Thr Val Arg Leu Glu Lys Glu Ala Ser
                245                 250                 255

Tyr Asp Glu Ile Lys Asn Ala Ile Lys Glu Glu Ser Glu Gly Lys Leu
            260                 265                 270

Lys Gly Ile Leu Gly Tyr Thr Glu Asp Asp Val Val Ser Thr Asp Phe
        275                 280                 285

Val Gly Asp Ser Arg Ser Ser Ile Phe Asp Ala Lys Ala Gly Ile Ala
    290                 295                 300

Leu Asn Lys Asn Phe Val Lys Leu Val Ser Trp Tyr Asp Asn Glu Trp
305                 310                 315                 320

Gly Tyr Ser Ser Arg Val Ile Asp Leu Leu Val Phe Val Ala Lys Lys
                325                 330                 335

Ser Phe

<210> 442
<211> 8
<212> PRT
<213> Glycine max

<400> 442

Glu Ala Ser Tyr Asp Glu Ile Lys
1               5

<210> 443
<211> 27
<212> PRT
<213> Glycine max

<400>  443

Gly Ile Leu Gly Tyr Thr Glu Asp Asp Val Val Ser Thr Asp Phe Val
1               5                   10                  15

Gly Asp Ser Arg Ser Ser Ile Phe Asp Ala Lys
            20                  25

<210>  444
<211>  8
<212>  PRT
<213>  Glycine max

<400>  444

Val Ile Ile Ser Ala Pro Ser Lys
1               5

<210>  445
<211>  8
<212>  PRT
<213>  Glycine max

<400>  445

Val Leu Pro Ala Leu Asn Gly Lys
1               5

<210>  446
<211>  1020
<212>  DNA
<213>  Glycine max

<400>  446
atggcatcag ataagaagat taggatcggc atcaacggat ttggaaggat tggccgtttg      60

gtggccaggg ttgcccttca aaggaatgat gttgaacttg ttgctgtcaa cgatcctttc     120

atcaccactg attacatgac atacatgttc aagtacgaca ccgttcatgg ccagtggaag     180

cattttgatg tgaaggtgaa ggactctaag acccttctct ttggtgagaa gcccgtcacc     240

gtttttggaa tcaggaaccc tgaggaaatt ccatggggtg aggttggtgc tgattatgtt     300

gttgagtcaa ctggtgtatt cactgacaag gacaaggctg ctgctcactt gaagggtggt     360

gccaagaagg ttgtcatctc tgccccaagc aaagatgcac ccatgtttgt tgttggtgtt     420

aacgagaagg agtacaagcc agagcttgac attgtttcca atgctagctg cactaccaat     480

tgtcttgctc ccctcgccaa ggttatcaac gaccgatttg gaattgtgga gggtcttatg     540

accactgttc atgccatcac agctactcag aagactgttg atggtccatc aaacaaggac     600

tggaggggtg gaagagctgc ttccttcaat ataattccca gcagcactgg agctgctaag     660

gctgtaggaa aagttcttcc agcactgaat ggtaaattga ctggcatgtc cttccgagtc     720

cccactgtag atgtttcagt tgttgacctc actgtcaggc tagagaagcc agcaacctat     780

gagcaaatca aatctgctat caaggaggaa tcagagggca aattgaaggg tattttgggt 840

tacaccgaag atgatgttgt gtctactgat tttgttggtg ataacaggtc tagcatattt 900

gacgccaagg ccggaatttc tttgaacgac aactttgtaa aacttgtttc ctggtatgac 960

aacgaatggg gttacagttc ccgtgtgatt gatttgattg tccacattgc atccgtggct 1020

<210> 447
<211> 340
<212> PRT
<213> Glycine max

<400> 447

Met Ala Ser Asp Lys Lys Ile Arg Ile Gly Ile Asn Gly Phe Gly Arg
1               5                   10                  15

Ile Gly Arg Leu Val Ala Arg Val Ala Leu Gln Arg Asn Asp Val Glu
            20                  25                  30

Leu Val Ala Val Asn Asp Pro Phe Ile Thr Thr Asp Tyr Met Thr Tyr
        35                  40                  45

Met Phe Lys Tyr Asp Thr Val His Gly Gln Trp Lys His Phe Asp Val
    50                  55                  60

Lys Val Lys Asp Ser Lys Thr Leu Leu Phe Gly Glu Lys Pro Val Thr
65                  70                  75                  80

Val Phe Gly Ile Arg Asn Pro Glu Glu Ile Pro Trp Gly Glu Val Gly
                85                  90                  95

Ala Asp Tyr Val Val Glu Ser Thr Gly Val Phe Thr Asp Lys Asp Lys
            100                 105                 110

Ala Ala Ala His Leu Lys Gly Gly Ala Lys Lys Val Val Ile Ser Ala
        115                 120                 125

Pro Ser Lys Asp Ala Pro Met Phe Val Val Gly Val Asn Glu Lys Glu
    130                 135                 140

Tyr Lys Pro Glu Leu Asp Ile Val Ser Asn Ala Ser Cys Thr Thr Asn
145                 150                 155                 160

Cys Leu Ala Pro Leu Ala Lys Val Ile Asn Asp Arg Phe Gly Ile Val
                165                 170                 175

Glu Gly Leu Met Thr Thr Val His Ala Ile Thr Ala Thr Gln Lys Thr
            180                 185                 190

Val Asp Gly Pro Ser Asn Lys Asp Trp Arg Gly Gly Arg Ala Ala Ser
        195                 200                 205

Phe Asn Ile Ile Pro Ser Ser Thr Gly Ala Ala Lys Ala Val Gly Lys
    210                 215                 220

Val Leu Pro Ala Leu Asn Gly Lys Leu Thr Gly Met Ser Phe Arg Val
225                 230                 235                 240

Pro Thr Val Asp Val Ser Val Val Asp Leu Thr Val Arg Leu Glu Lys
                245                 250                 255

Pro Ala Thr Tyr Glu Gln Ile Lys Ser Ala Ile Lys Glu Glu Ser Glu
            260                 265                 270

Gly Lys Leu Lys Gly Ile Leu Gly Tyr Thr Glu Asp Asp Val Val Ser
        275                 280                 285

Thr Asp Phe Val Gly Asp Asn Arg Ser Ser Ile Phe Asp Ala Lys Ala
    290                 295                 300

Gly Ile Ser Leu Asn Asp Asn Phe Val Lys Leu Val Ser Trp Tyr Asp
305                 310                 315                 320

Asn Glu Trp Gly Tyr Ser Ser Arg Val Ile Asp Leu Ile Val His Ile
                325                 330                 335

Ala Ser Val Ala
            340

<210>  448
<211>  11
<212>  PRT
<213>  Glycine max

<400>  448

Ala Gly Ile Ser Leu Asn Asp Asn Phe Val Lys
1               5                   10

<210>  449
<211>  27
<212>  PRT
<213>  Glycine max

<400>  449

Gly Ile Leu Gly Tyr Thr Glu Asp Asp Val Val Ser Thr Asp Phe Val
1               5                   10                  15

Gly Asp Asn Arg Ser Ser Ile Phe Asp Ala Lys
            20                  25

<210>  450
<211>  8
<212>  PRT
<213>  Glycine max

<400>  450

Val Leu Pro Ala Leu Asn Gly Lys
1               5

<210>  451
<211>  8
<212>  PRT
<213>  Glycine max

<400>  451

Val Val Ile Ser Ala Pro Ser Lys
1               5

<210>  452
<211>  1050
<212>  DNA
<213>  Glycine max

<400>  452
atggtgacct cgtcgcaaaa gattctggtt accggcggtg ccggtttcat aggcacccac      60

accgtggtcc agcttctcaa aggaggcttc agtgtttcaa taatcgacaa tttcgataac     120

tccgtcgtgg aagcagtgga ccgcgtccgc caagtggtgg gccctcagct ttctcagaac     180

ctggaattca cccagggcga tctcaggaat agggatgact tggagaaact cttctccaga     240

acaacttttg atgccgtgat ccactttgct ggcctgaaag cggttgctga aagcgtttcc     300

aagccccgtc gctattttga ttttaatttg gtcggcacca tcaacctcta ccaagttatg     360

gcaaagtata attgcagaaa gatggttttc tcatcatctg caaccgttta tggccaacct     420

gaaaagatac cgtgtgagga ggatttcagg ttacaagcta tgaatcccta tggacggacc     480

aagcttttcc tggaagaaat tgcccgagat attcagaaag ctgaaccaga atggaagatc     540

atattactga ggtacttcaa tccggttggg gctcatgaaa gtggtaaact cggtgaagat     600

cccaagggca tcccaaataa cctcatgcct tacattcagc aagtagccgt tggaagattg     660

ccggaactca atgtatatgg gcatgattat cccacaaggg atggctctgc gatccgggac     720

tatatccatg tgatggactt agcagatggt catattgctg ccttgcgaaa gctcttcaca     780

acagagaaca taggttgtac tgcttacaac ctgggaactg gtcgtggaac atccgtgctt     840

gaaatggtta cagcatttga aaaggcgtct ggcaagaaaa ttccagtaaa attatgtcca     900

agaagaccgg gagatgctac tgaggtttat gcatctacag agagagctga gaaagaactt 960

ggatggaaag caaaatatgg tgtggatgag atgtgcaggg accaatggaa ttgggcaaag 1020

aacaatcccc agggttacac ggggaagcct 1050

<210> 453
<211> 350
<212> PRT
<213> Glycine max

<400> 453

Met Val Thr Ser Ser Gln Lys Ile Leu Val Thr Gly Gly Ala Gly Phe
1               5                   10                  15

Ile Gly Thr His Thr Val Val Gln Leu Leu Lys Gly Gly Phe Ser Val
            20                  25                  30

Ser Ile Ile Asp Asn Phe Asp Asn Ser Val Val Glu Ala Val Asp Arg
        35                  40                  45

Val Arg Gln Val Val Gly Pro Gln Leu Ser Gln Asn Leu Glu Phe Thr
    50                  55                  60

Gln Gly Asp Leu Arg Asn Arg Asp Asp Leu Glu Lys Leu Phe Ser Arg
65                  70                  75                  80

Thr Thr Phe Asp Ala Val Ile His Phe Ala Gly Leu Lys Ala Val Ala
                85                  90                  95

Glu Ser Val Ser Lys Pro Arg Arg Tyr Phe Asp Phe Asn Leu Val Gly
            100                 105                 110

Thr Ile Asn Leu Tyr Gln Val Met Ala Lys Tyr Asn Cys Arg Lys Met
        115                 120                 125

Val Phe Ser Ser Ser Ala Thr Val Tyr Gly Gln Pro Glu Lys Ile Pro
    130                 135                 140

Cys Glu Glu Asp Phe Arg Leu Gln Ala Met Asn Pro Tyr Gly Arg Thr
145                 150                 155                 160

Lys Leu Phe Leu Glu Glu Ile Ala Arg Asp Ile Gln Lys Ala Glu Pro
                165                 170                 175

Glu Trp Lys Ile Ile Leu Leu Arg Tyr Phe Asn Pro Val Gly Ala His
            180                 185                 190

Glu Ser Gly Lys Leu Gly Glu Asp Pro Lys Gly Ile Pro Asn Asn Leu

195                 200                 205

Met Pro Tyr Ile Gln Gln Val Ala Val Gly Arg Leu Pro Glu Leu Asn
    210                 215                 220

Val Tyr Gly His Asp Tyr Pro Thr Arg Asp Gly Ser Ala Ile Arg Asp
225                 230                 235                 240

Tyr Ile His Val Met Asp Leu Ala Asp Gly His Ile Ala Ala Leu Arg
                245                 250                 255

Lys Leu Phe Thr Thr Glu Asn Ile Gly Cys Thr Ala Tyr Asn Leu Gly
            260                 265                 270

Thr Gly Arg Gly Thr Ser Val Leu Glu Met Val Thr Ala Phe Glu Lys
        275                 280                 285

Ala Ser Gly Lys Lys Ile Pro Val Lys Leu Cys Pro Arg Arg Pro Gly
    290                 295                 300

Asp Ala Thr Glu Val Tyr Ala Ser Thr Glu Arg Ala Glu Lys Glu Leu
305                 310                 315                 320

Gly Trp Lys Ala Lys Tyr Gly Val Asp Glu Met Cys Arg Asp Gln Trp
                325                 330                 335

Asn Trp Ala Lys Asn Asn Pro Gln Gly Tyr Thr Gly Lys Pro
            340                 345                 350

<210>  454
<211>  12
<212>  PRT
<213>  Glycine max

<400>  454

Leu Phe Leu Glu Glu Ile Ala Arg Asp Ile Gln Lys
1               5                   10

<210>  455
<211>  1050
<212>  DNA
<213>  Glycine max

<400>  455
atggtgtctt cctcccaaca cattctggtc accggtggtg ccggtttcat tggcacccac        60

accgtcgttc agcttctcaa agctggcttc agcgtttcaa taatcgacaa tttcgataac       120

tccgtcatgg aagcagtgga ccgcgtccgc caagtggttg gccctctgct ttctcagaac       180

ctccaattca cccagggcga tctccggaat agggatgact tggagaaact cttctccaaa       240

acaacatttg atgccgtgat ccactttgct ggcttgaaag cggttgctga aagcgttgcg 300

aagccccgtc gctattttga ttttaatttg gttggcacca tcaacctcta cgaatttatg 360

gcaaagtata attgcaaaaa gatggttttc tcatcatctg caaccgttta tggccaacct 420

gaaaagatac cgtgtgagga ggatttcaag ttacaagcta tgaatcccta tggacggacc 480

aagcttttcc tggaagaaat tgcccgagat attcagaaag ctgaaccaga atggaagatc 540

atattactga gatacttcaa tccagttggg gctcatgaaa gtggcaaact cggtgaagat 600

cccaagggca tcccaaataa cctcatgcct tacattcagc aagtagctgt tggaagattg 660

actgaactca atgtatacgg tcatgattat ccaacgaggg atggctctgc gatccgggac 720

tatatccatg tgatggactt ggcagatggc catattgctg ccctgcgaaa gctcttcaca 780

acggagaaca taggttgtac tgcttacaac ctgggaactg gtcgtggaac atctgtgctt 840

gaaatggttg cagcatttga aaaggcttct ggcaagaaaa ttccagtaaa attatgtcca 900

agaagaccgg gagatgcgac tgaggtttat gcatctacag agagagctga gaaagaactt 960

ggttggaagg caaactatgg tgtggaggag atgtgcaggg accaatggaa ttgggcaaag 1020

aacaatccct ggggttacgc ggggaagcct 1050

<210> 456
<211> 350
<212> PRT
<213> Glycine max

<400> 456

Met Val Thr Ser Ser Gln Lys Ile Leu Val Thr Gly Gly Ala Gly Phe
1               5                   10                  15

Ile Gly Thr His Thr Val Val Gln Leu Leu Lys Gly Gly Phe Ser Val
            20                  25                  30

Ser Ile Ile Asp Asn Phe Asp Asn Ser Val Val Glu Ala Val Asp Arg
        35                  40                  45

Val Arg Gln Val Val Gly Pro Gln Leu Ser Gln Asn Leu Glu Phe Thr
    50                  55                  60

Gln Gly Asp Leu Arg Asn Arg Asp Asp Leu Glu Lys Leu Phe Ser Arg
65                  70                  75                  80

Thr Thr Phe Asp Ala Val Ile His Phe Ala Gly Leu Lys Ala Val Ala
                85                  90                  95

Glu Ser Val Ser Lys Pro Arg Arg Tyr Phe Asp Phe Asn Leu Val Gly
            100                 105                 110

Thr Ile Asn Leu Tyr Gln Val Met Ala Lys Tyr Asn Cys Arg Lys Met
        115                 120                 125

Val Phe Ser Ser Ser Ala Thr Val Tyr Gly Gln Pro Glu Lys Ile Pro
    130                 135                 140

Cys Glu Glu Asp Phe Arg Leu Gln Ala Met Asn Pro Tyr Gly Arg Thr
145                 150                 155                 160

Lys Leu Phe Leu Glu Glu Ile Ala Arg Asp Ile Gln Lys Ala Glu Pro
                165                 170                 175

Glu Trp Lys Ile Ile Leu Leu Arg Tyr Phe Asn Pro Val Gly Ala His
            180                 185                 190

Glu Ser Gly Lys Leu Gly Glu Asp Pro Lys Gly Ile Pro Asn Asn Leu
        195                 200                 205

Met Pro Tyr Ile Gln Gln Val Ala Val Gly Arg Leu Pro Glu Leu Asn
    210                 215                 220

Val Tyr Gly His Asp Tyr Pro Thr Arg Asp Gly Ser Ala Ile Arg Asp
225                 230                 235                 240

Tyr Ile His Val Met Asp Leu Ala Asp Gly His Ile Ala Ala Leu Arg
                245                 250                 255

Lys Leu Phe Thr Thr Glu Asn Ile Gly Cys Thr Ala Tyr Asn Leu Gly
            260                 265                 270

Thr Gly Arg Gly Thr Ser Val Leu Glu Met Val Thr Ala Phe Glu Lys
        275                 280                 285

Ala Ser Gly Lys Lys Ile Pro Val Lys Leu Cys Pro Arg Arg Pro Gly
    290                 295                 300

Asp Ala Thr Glu Val Tyr Ala Ser Thr Glu Arg Ala Glu Lys Glu Leu
305                 310                 315                 320

Gly Trp Lys Ala Lys Tyr Gly Val Asp Glu Met Cys Arg Asp Gln Trp
                325                 330                 335

Asn Trp Ala Lys Asn Asn Pro Gln Gly Tyr Thr Gly Lys Pro
            340                 345                 350

<210>  457

<211>  12
<212>  PRT
<213>  Glycine max

<400>  457

Leu Phe Leu Glu Glu Ile Ala Arg Asp Ile Gln Lys
1               5                   10


<210>  458
<211>  13
<212>  PRT
<213>  Glycine max

<400>  458

Thr Thr Phe Asp Ala Val Ile His Phe Ala Gly Leu Lys
1               5                   10


<210>  459
<211>  1341
<212>  DNA
<213>  Glycine max

<400>  459
atgggtaagg aaaagactca catcaacatt gtcgtcattg gacatgtcga ctctgggaag      60

tcaactacca ctggtcattt gatctacaag cttggaggta ttgacaagcg tgtgattgag     120

aggttcgaga aggaggctgc tgagatgaac aagaggtcat tcaagtatgc ctgggtgctt     180

gacaagctca aggctgagcg cgaaagagga attaccattg atattgcttt gtggaagttt     240

gaaaccacca agtactactg cacagtcatc gatgcccccg gacatcgtga ctttatcaag     300

aacatgatta ctggtacctc ccaggctgat tgtgctgtcc ttatcattga ctccaccact     360

ggtggttttg aagctggtat ttctaaggat ggacagactc gtgagcatgc tcttcttgct     420

ttcacccttg gtgtgaagca gatgatctgc tgctgtaaca agatggatgc cactaccccg     480

aagtactcta aggctaggta tgatgaaatt gtgaaggaag tctcttccta cttgaagaag     540

gttggttaca acccagacaa gattccattt gtgcccatct ctggtttcga gggtgacaac     600

atgattgaga ggtccaccaa ccttgactgg tacaagggac caactctcct tgaggctctt     660

gaccaaatca atgagcccaa gaggccctca gacaagccac tcaggcttcc attgcaggat     720

gtctacaaaa ttggtggtat tggtaccgtg ccagtgggac gtgttgagac tggtgtcgtg     780

aagcccggta tggtggtgac ttttggtccc actgggctga caactgaggt taagtctgtt     840

gagatgcacc atgaggctct cacagaggct cttccaggtg acaatgttgg ctttaatgtg     900

aagaatgttg cagtcaagga tctcaagcgt ggttttgttg catccaactc caaggatgac     960

cctgccaagg aagctgccaa cttcacatct caagtcatta tcatgaacca ccctggccag    1020

attggaaatg gatacgcacc ggtgcttgat tgccacactt ctcacattgc tgtcaagttt    1080

tctgaaatct tgaccaagat tgacaggcga tctggtaagg agcttgagaa ggagcccaaa 1140

tttttgaaga atggtgatgc aggtatggtt aagatgattc caaccaagcc catggtggtt 1200

gaaactttct ctgagtatcc tccccttggt cgttttgctg tgagggacat gcgtcaaact 1260

gtggctgtcg gagtcatcaa gagtgttgag aagaaagacc ccaccggagc caaggtcacc 1320

aaggccgctg ccaagaagaa g 1341

<210> 460
<211> 447
<212> PRT
<213> Glycine max

<400> 460

Met Gly Lys Glu Lys Thr His Ile Asn Ile Val Val Ile Gly His Val
1               5                   10                  15

Asp Ser Gly Lys Ser Thr Thr Thr Gly His Leu Ile Tyr Lys Leu Gly
            20                  25                  30

Gly Ile Asp Lys Arg Val Ile Glu Arg Phe Glu Lys Glu Ala Ala Glu
        35                  40                  45

Met Asn Lys Arg Ser Phe Lys Tyr Ala Trp Val Leu Asp Lys Leu Lys
    50                  55                  60

Ala Glu Arg Glu Arg Gly Ile Thr Ile Asp Ile Ala Leu Trp Lys Phe
65                  70                  75                  80

Glu Thr Thr Lys Tyr Tyr Cys Thr Val Ile Asp Ala Pro Gly His Arg
                85                  90                  95

Asp Phe Ile Lys Asn Met Ile Thr Gly Thr Ser Gln Ala Asp Cys Ala
            100                 105                 110

Val Leu Ile Ile Asp Ser Thr Thr Gly Gly Phe Glu Ala Gly Ile Ser
        115                 120                 125

Lys Asp Gly Gln Thr Arg Glu His Ala Leu Leu Ala Phe Thr Leu Gly
    130                 135                 140

Val Lys Gln Met Ile Cys Cys Cys Asn Lys Met Asp Ala Thr Thr Pro
145                 150                 155                 160

Lys Tyr Ser Lys Ala Arg Tyr Asp Glu Ile Val Lys Glu Val Ser Ser
                165                 170                 175

Tyr Leu Lys Lys Val Gly Tyr Asn Pro Asp Lys Ile Pro Phe Val Pro

            180                 185                 190

Ile Ser Gly Phe Glu Gly Asp Asn Met Ile Glu Arg Ser Thr Asn Leu
        195                 200                 205

Asp Trp Tyr Lys Gly Pro Thr Leu Leu Glu Ala Leu Asp Gln Ile Asn
    210                 215                 220

Glu Pro Lys Arg Pro Ser Asp Lys Pro Leu Arg Leu Pro Leu Gln Asp
225                 230                 235                 240

Val Tyr Lys Ile Gly Gly Ile Gly Thr Val Pro Val Gly Arg Val Glu
                245                 250                 255

Thr Gly Val Val Lys Pro Gly Met Val Val Thr Phe Gly Pro Thr Gly
            260                 265                 270

Leu Thr Thr Glu Val Lys Ser Val Glu Met His His Glu Ala Leu Thr
        275                 280                 285

Glu Ala Leu Pro Gly Asp Asn Val Gly Phe Asn Val Lys Asn Val Ala
    290                 295                 300

Val Lys Asp Leu Lys Arg Gly Phe Val Ala Ser Asn Ser Lys Asp Asp
305                 310                 315                 320

Pro Ala Lys Glu Ala Ala Asn Phe Thr Ser Gln Val Ile Ile Met Asn
                325                 330                 335

His Pro Gly Gln Ile Gly Asn Gly Tyr Ala Pro Val Leu Asp Cys His
            340                 345                 350

Thr Ser His Ile Ala Val Lys Phe Ser Glu Ile Leu Thr Lys Ile Asp
        355                 360                 365

Arg Arg Ser Gly Lys Glu Leu Glu Lys Glu Pro Lys Phe Leu Lys Asn
    370                 375                 380

Gly Asp Ala Gly Met Val Lys Met Ile Pro Thr Lys Pro Met Val Val
385                 390                 395                 400

Glu Thr Phe Ser Glu Tyr Pro Pro Leu Gly Arg Phe Ala Val Arg Asp
                405                 410                 415

Met Arg Gln Thr Val Ala Val Gly Val Ile Lys Ser Val Glu Lys Lys
            420                 425                 430

Asp Pro Thr Gly Ala Lys Val Thr Lys Ala Ala Ala Lys Lys Lys
        435                 440                 445

<210>  461
<211>  8
<212>  PRT
<213>  Glycine max

<400>  461

Ala Arg Tyr Asp Glu Ile Val Lys
1               5

<210>  462
<211>  17
<212>  PRT
<213>  Glycine max

<400>  462

Asp Gly Gln Thr Arg Glu His Ala Leu Leu Ala Phe Thr Leu Gly Val
1               5                   10                  15

Lys

<210>  463
<211>  7
<212>  PRT
<213>  Glycine max

<400>  463

Glu Val Ser Ser Tyr Leu Lys
1               5

<210>  464
<211>  7
<212>  PRT
<213>  Glycine max

<400>  464

Phe Ser Glu Ile Leu Thr Lys
1               5

<210>  465
<211>  10
<212>  PRT
<213>  Glycine max

<400>  465

Ser Thr Thr Thr Gly His Leu Ile Tyr Lys
1               5                   10

<210>  466
<211>  15
<212>  PRT
<213>  Glycine max

<400>  466

Thr His Ile Asn Ile Val Val Ile Gly His Val Asp Ser Gly Lys
1               5                   10                  15


<210>  467
<211>  1341
<212>  DNA
<213>  Glycine max

<400>  467
atgggtaaag agaaggttca cattaacatt gtggtcattg gccatgttga ctctgggaag      60

tcaaccacca ctggccactt gatttacaag cttggaggta ttgacaagcg tgttattgag     120

aggtttgaga aagaagctgc tgagatgaac aagaggtcat tcaaatacgc ctgggtgctt     180

gacaagctta aggctgagcg tgagagagga attaccattg atattgcatt atggaagttt     240

gagaccacca aatactactg cactgttatt gatgctccag gacatcgtga tttcatcaag     300

aacatgatta ctggaacttc ccaggctgat tgtgctgtcc ttatcattga ttccacaact     360

ggtggttttg aagctggtat ttccaaagat ggacagactc gcgagcatgc tctacttgcc     420

ttcacccttg gtgtcaagca aatgatctgt tgctgtaaca agatggatgc cactacccca     480

aagtactcta aggccaggta tgaagaaatt gtgaaggaag tttcttccta cttgaagaag     540

gtaggttaca acccagacaa gattccattt gttcccatct caggttttga gggtgacaac     600

atgattgaga ggtccaccaa tcttgattgg tacaagggac caacccttct cgaggctctt     660

gaccagatca atgagcccaa gagaccctca gacaagcctt tgaggcttcc cttgcaggat     720

gtctacaaga ttggtggtat tggaacagtg ccagtgggac gagttgaaac tggtgtcttg     780

aagcccggta tggtggtgac ttttgctccc actggcctga caactgaagt taagtctgtg     840

gagatgcacc atgaagctct ccaggaggcc cttccaggtg acaacgtagg atttaatgtg     900

aagaatgttg cagtcaagga tctcaagcgt ggttttgttg cctcaaactc taaggatgat     960

cctgccaagg aggctgccaa cttcacatcc caagtcatta taatgaacca tcctggccaa    1020

attggaaatg gttatgctcc tgtgcttgac tgccacactt ctcacattgc cgtcaagttt    1080

gccgaacttg tgaccaagat tgacaggcga tctggtaagg agattgagaa ggagcccaaa    1140

ttcttgaaaa atggagatgc aggttatgtt aagatgattc ccaccaagcc tatggtggtc    1200

gaaactttct ccgagtatcc tcctcttggt cgttttgccg tgagggacat gcgacaaact    1260

gtggctgttg gagtcatcaa gagtgtggag aagaaggacc caactggagc caaggtcacc    1320

aaggctgcac agaagaagaa g                                              1341

<210>  468
<211>  447
<212>  PRT
<213>  Glycine max

<400>  468

Met Gly Lys Glu Lys Val His Ile Asn Ile Val Val Ile Gly His Val
1               5                   10                  15

Asp Ser Gly Lys Ser Thr Thr Thr Gly His Leu Ile Tyr Lys Leu Gly
            20                  25                  30

Gly Ile Asp Lys Arg Val Ile Glu Arg Phe Glu Lys Glu Ala Ala Glu
        35                  40                  45

Met Asn Lys Arg Ser Phe Lys Tyr Ala Trp Val Leu Asp Lys Leu Lys
    50                  55                  60

Ala Glu Arg Glu Arg Gly Ile Thr Ile Asp Ile Ala Leu Trp Lys Phe
65                  70                  75                  80

Glu Thr Thr Lys Tyr Tyr Cys Thr Val Ile Asp Ala Pro Gly His Arg
                85                  90                  95

Asp Phe Ile Lys Asn Met Ile Thr Gly Thr Ser Gln Ala Asp Cys Ala
            100                 105                 110

Val Leu Ile Ile Asp Ser Thr Thr Gly Gly Phe Glu Ala Gly Ile Ser
        115                 120                 125

Lys Asp Gly Gln Thr Arg Glu His Ala Leu Leu Ala Phe Thr Leu Gly
    130                 135                 140

Val Lys Gln Met Ile Cys Cys Cys Asn Lys Met Asp Ala Thr Thr Pro
145                 150                 155                 160

Lys Tyr Ser Lys Ala Arg Tyr Glu Glu Ile Val Lys Glu Val Ser Ser
                165                 170                 175

Tyr Leu Lys Lys Val Gly Tyr Asn Pro Asp Lys Ile Pro Phe Val Pro
            180                 185                 190

Ile Ser Gly Phe Glu Gly Asp Asn Met Ile Glu Arg Ser Thr Asn Leu
        195                 200                 205

Asp Trp Tyr Lys Gly Pro Thr Leu Leu Glu Ala Leu Asp Gln Ile Asn
    210                 215                 220

Glu Pro Lys Arg Pro Ser Asp Lys Pro Leu Arg Leu Pro Leu Gln Asp
225                 230                 235                 240

Val Tyr Lys Ile Gly Gly Ile Gly Thr Val Pro Val Gly Arg Val Glu
                245                 250                 255

Thr Gly Val Leu Lys Pro Gly Met Val Val Thr Phe Ala Pro Thr Gly
            260                 265                 270

Leu Thr Thr Glu Val Lys Ser Val Glu Met His His Glu Ala Leu Gln
        275                 280                 285

Glu Ala Leu Pro Gly Asp Asn Val Gly Phe Asn Val Lys Asn Val Ala
    290                 295                 300

Val Lys Asp Leu Lys Arg Gly Phe Val Ala Ser Asn Ser Lys Asp Asp
305                 310                 315                 320

Pro Ala Lys Glu Ala Ala Asn Phe Thr Ser Gln Val Ile Ile Met Asn
                325                 330                 335

His Pro Gly Gln Ile Gly Asn Gly Tyr Ala Pro Val Leu Asp Cys His
            340                 345                 350

Thr Ser His Ile Ala Val Lys Phe Ala Glu Leu Val Thr Lys Ile Asp
        355                 360                 365

Arg Arg Ser Gly Lys Glu Ile Glu Lys Glu Pro Lys Phe Leu Lys Asn
    370                 375                 380

Gly Asp Ala Gly Tyr Val Lys Met Ile Pro Thr Lys Pro Met Val Val
385                 390                 395                 400

Glu Thr Phe Ser Glu Tyr Pro Pro Leu Gly Arg Phe Ala Val Arg Asp
                405                 410                 415

Met Arg Gln Thr Val Ala Val Gly Val Ile Lys Ser Val Glu Lys Lys
            420                 425                 430

Asp Pro Thr Gly Ala Lys Val Thr Lys Ala Ala Gln Lys Lys Lys
        435                 440                 445

<210>  469
<211>  17
<212>  PRT
<213>  Glycine max

<400>  469

Asp Gly Gln Thr Arg Glu His Ala Leu Leu Ala Phe Thr Leu Gly Val
1               5                   10                  15

Lys

<210>  470
<211>  7
<212>  PRT
<213>  Glycine max

<400>  470

Glu Val Ser Ser Tyr Leu Lys
1               5

<210>  471
<211>  10
<212>  PRT
<213>  Glycine max

<400>  471

Ser Thr Thr Thr Gly His Leu Ile Tyr Lys
1               5                   10

<210>  472
<211>  1344
<212>  DNA
<213>  Glycine max

<400>  472
atgggtaagg aaaaggttca catcagtatt gtggtcattg gccatgtcga ctctgggaaa      60

tccactacca ctggtcacct gatttacaag cttggaggca ttgacaagcg tgttattgag     120

aggtttgaga aggaagctgc tgagatgaac aagaggtctt tcaagtatgc ctgggtgctg     180

gacaaactta aggctgagcg tgaaagagga atcaccattg atattgcttt gtggaagttt     240

gaaacaacaa agtattattg cacagttatt gatgcgcctg gacataggga tttcattaag     300

aatatgatta ctgggacatc ccaagctgac tgtgctgttc ttatcattga ttcgaccact     360

ggtggttttg aagctggtat ttccaaggat ggacagactc gtgaacatgc tctgctttca     420

ttcacccttg gtgtgaaaca gatgatttgc tgctgtaaca aaatggatgc tactacaccc     480

aagtattcca aggccaggta tgatgaaatt gtgaaggaag tctcttccta cttgaagaaa     540

gtaggataca accctgacaa gattcctttt gttcctatct ctggttttga gggagacaac     600

atgattgaga ggtccacaaa ccttgactgg tacaagggtc caactctgct tgatgcactt     660

gaccagatta gtgagcccaa gaggccctct gacaagcccc tcaggcttcc ccttcaggat     720

gtgtacaaga ttggaggtat tggaactgtg ccagtgggac gtgttgagac cggtgtcttg     780

aagcctggaa tggtggtgac ttttgcacca actggactga caactgaagt caagtctgtg 840

gagatgcacc atgaatctct tacagaggca catcctggtg acaatgtggg attcaatgtt 900

aagaatgttg ctgttaagga tttgaagcgt ggttatgttg cctcaaactc aaaggatgac 960

cctgcaaagg aggctgctaa cttcacagcc caagtcatca tcatgaacca ccctggtcag 1020

attggaaatg gctatgcccc tgtcctcgac tgccacactt ctcacattgc tgtcaagttt 1080

gctgaactca tgaccaagat tgacaggcga tccggcaaag agcttgagaa ggagcccaag 1140

tttttgaaga acggtgatgc tggttttgtt aagatgattc caaccaaacc catggttgtt 1200

gaaactttct ccgagtatcc tccacttggt aggtttgctg ttagggacat gcgtcaaact 1260

gttgctgtgg gagtcatcaa gaacgttgag aagaaggatc ctaccggagc caaggtcacc 1320

aaggctgccc agaagaagaa gtga 1344

<210> 473
<211> 447
<212> PRT
<213> Glycine max

<400> 473

Met Gly Lys Glu Lys Val His Ile Ser Ile Val Val Ile Gly His Val
1               5                   10                  15

Asp Ser Gly Lys Ser Thr Thr Thr Gly His Leu Ile Tyr Lys Leu Gly
            20                  25                  30

Gly Ile Asp Lys Arg Val Ile Glu Arg Phe Glu Lys Glu Ala Ala Glu
        35                  40                  45

Met Asn Lys Arg Ser Phe Lys Tyr Ala Trp Val Leu Asp Lys Leu Lys
    50                  55                  60

Ala Glu Arg Glu Arg Gly Ile Thr Ile Asp Ile Ala Leu Trp Lys Phe
65                  70                  75                  80

Glu Thr Thr Lys Tyr Tyr Cys Thr Val Ile Asp Ala Pro Gly His Arg
                85                  90                  95

Asp Phe Ile Lys Asn Met Ile Thr Gly Thr Ser Gln Ala Asp Cys Ala
            100                 105                 110

Val Leu Ile Ile Asp Ser Thr Thr Gly Gly Phe Glu Ala Gly Ile Ser
        115                 120                 125

Lys Asp Gly Gln Thr Arg Glu His Ala Leu Leu Ser Phe Thr Leu Gly
    130                 135                 140

Val Lys Gln Met Ile Cys Cys Cys Asn Lys Met Asp Ala Thr Thr Pro
145                 150                 155                 160

Lys Tyr Ser Lys Ala Arg Tyr Asp Glu Ile Val Lys Glu Val Ser Ser
                165                 170                 175

Tyr Leu Lys Lys Val Gly Tyr Asn Pro Asp Lys Ile Pro Phe Val Pro
            180                 185                 190

Ile Ser Gly Phe Glu Gly Asp Asn Met Ile Glu Arg Ser Thr Asn Leu
        195                 200                 205

Asp Trp Tyr Lys Gly Pro Thr Leu Leu Asp Ala Leu Asp Gln Ile Ser
    210                 215                 220

Glu Pro Lys Arg Pro Ser Asp Lys Pro Leu Arg Leu Pro Leu Gln Asp
225                 230                 235                 240

Val Tyr Lys Ile Gly Gly Ile Gly Thr Val Pro Val Gly Arg Val Glu
                245                 250                 255

Thr Gly Val Leu Lys Pro Gly Met Val Val Thr Phe Ala Pro Thr Gly
            260                 265                 270

Leu Thr Thr Glu Val Lys Ser Val Glu Met His His Glu Ser Leu Thr
        275                 280                 285

Glu Ala His Pro Gly Asp Asn Val Gly Phe Asn Val Lys Asn Val Ala
    290                 295                 300

Val Lys Asp Leu Lys Arg Gly Tyr Val Ala Ser Asn Ser Lys Asp Asp
305                 310                 315                 320

Pro Ala Lys Glu Ala Ala Asn Phe Thr Ala Gln Val Ile Ile Met Asn
                325                 330                 335

His Pro Gly Gln Ile Gly Asn Gly Tyr Ala Pro Val Leu Asp Cys His
            340                 345                 350

Thr Ser His Ile Ala Val Lys Phe Ala Glu Leu Met Thr Lys Ile Asp
        355                 360                 365

Arg Arg Ser Gly Lys Glu Leu Glu Lys Glu Pro Lys Phe Leu Lys Asn
    370                 375                 380

Gly Asp Ala Gly Phe Val Lys Met Ile Pro Thr Lys Pro Met Val Val
385                 390                 395                 400

Glu Thr Phe Ser Glu Tyr Pro Pro Leu Gly Arg Phe Ala Val Arg Asp
                405                 410                 415

Met Arg Gln Thr Val Ala Val Gly Val Ile Lys Asn Val Glu Lys Lys
            420                 425                 430

Asp Pro Thr Gly Ala Lys Val Thr Lys Ala Ala Gln Lys Lys Lys
        435                 440                 445

<210>  474
<211>  8
<212>  PRT
<213>  Glycine max

<400>  474

Ala Arg Tyr Asp Glu Ile Val Lys
1               5

<210>  475
<211>  17
<212>  PRT
<213>  Glycine max

<400>  475

Asp Gly Gln Thr Arg Glu His Ala Leu Leu Ser Phe Thr Leu Gly Val
1               5                   10                  15

Lys

<210>  476
<211>  7
<212>  PRT
<213>  Glycine max

<400>  476

Glu Val Ser Ser Tyr Leu Lys
1               5

<210>  477
<211>  10
<212>  PRT
<213>  Glycine max

<400>  477

Ser Thr Thr Thr Gly His Leu Ile Tyr Lys
1               5                   10

<210>  478

<211>  882
<212>  DNA
<213>  Glycine max

<400>  478
atggcttccg gtgaacagaa attccctcct caacaacaac aaacacagcc tgggaaggag      60

catgctatga ctccagtacc ccaattcact agccctgact acaagccttc aaataaactt      120

caagggaaga ttgcattagt cactgggggt gattctggga ttggacgagc ggtgtgtaac      180

ttgtttgcct tagaaggtgc taccgtggcc ttcacgtatg tgaaggggca tgaggacaag      240

gacgcgaggg acacattgga aatgatcaag agagcaaaga cttcggatgc caaggatcca      300

atggcaatag catctgattt gggttacgat gagaactgca agagggtggt tgatgaggtc      360

gtgagtgctt atggttgtat tgacattctg gtcaacaatg cagctgagca gtacgagtgt      420

ggaaccgtgg aggacataga cgagcctagg cttgagaggg tctttcgtac aaatatcttc      480

tcctatttct tcatggcgag gcatgccttg aagcacatga aggaaggaag cagcattatc      540

aacacgacat cagtgaatgc atacaaggga catgcgaaac tattggacta cacgtccacc      600

aagggggcaa ttgtggccta tacaaggggt cttgcccttc agctggtgag taagggaatt      660

cgggttaatg gggtggctcc agggcccatt tggacccctt tgataccagc ctctttcaag      720

gaggaagaaa cggcccaatt tggagcgcag gtcccaatga agagagctgg tcaacctatt      780

gaggttgctc cttcctatgt ttttcttgct tccaaccaat gctcctctta cataactgga      840

caagtccttc accccaatgg tggaaccgtt gtgaatggtt aa                        882

<210>  479
<211>  293
<212>  PRT
<213>  Glycine max

<400>  479

Met Ala Ser Gly Glu Gln Lys Phe Pro Pro Gln Gln Gln Gln Thr Gln
1               5                   10                  15

Pro Gly Lys Glu His Ala Met Thr Pro Val Pro Gln Phe Thr Ser Pro
            20                  25                  30

Asp Tyr Lys Pro Ser Asn Lys Leu Gln Gly Lys Ile Ala Leu Val Thr
        35                  40                  45

Gly Gly Asp Ser Gly Ile Gly Arg Ala Val Cys Asn Leu Phe Ala Leu
    50                  55                  60

Glu Gly Ala Thr Val Ala Phe Thr Tyr Val Lys Gly His Glu Asp Lys
65                  70                  75                  80

Asp Ala Arg Asp Thr Leu Glu Met Ile Lys Arg Ala Lys Thr Ser Asp
                85                  90                  95

Ala Lys Asp Pro Met Ala Ile Ala Ser Asp Leu Gly Tyr Asp Glu Asn
            100                 105                 110

Cys Lys Arg Val Val Asp Glu Val Val Ser Ala Tyr Gly Cys Ile Asp
        115                 120                 125

Ile Leu Val Asn Asn Ala Ala Glu Gln Tyr Glu Cys Gly Thr Val Glu
    130                 135                 140

Asp Ile Asp Glu Pro Arg Leu Glu Arg Val Phe Arg Thr Asn Ile Phe
145                 150                 155                 160

Ser Tyr Phe Phe Met Ala Arg His Ala Leu Lys His Met Lys Glu Gly
                165                 170                 175

Ser Ser Ile Ile Asn Thr Thr Ser Val Asn Ala Tyr Lys Gly His Ala
            180                 185                 190

Lys Leu Leu Asp Tyr Thr Ser Thr Lys Gly Ala Ile Val Ala Tyr Thr
        195                 200                 205

Arg Gly Leu Ala Leu Gln Leu Val Ser Lys Gly Ile Arg Val Asn Gly
    210                 215                 220

Val Ala Pro Gly Pro Ile Trp Thr Pro Leu Ile Pro Ala Ser Phe Lys
225                 230                 235                 240

Glu Glu Glu Thr Ala Gln Phe Gly Ala Gln Val Pro Met Lys Arg Ala
                245                 250                 255

Gly Gln Pro Ile Glu Val Ala Pro Ser Tyr Val Phe Leu Ala Ser Asn
            260                 265                 270

Gln Cys Ser Ser Tyr Ile Thr Gly Gln Val Leu His Pro Asn Gly Gly
        275                 280                 285

Thr Val Val Asn Gly
    290

<210>  480
<211>  15
<212>  PRT
<213>  Glycine max

<400>  480

Glu Gly Ser Ser Ile Ile Asn Thr Thr Ser Val Asn Ala Tyr Lys
1               5                   10                  15

<210>  481
<211>  12
<212>  PRT
<213>  Glycine max

<400>  481

Phe Pro Pro Gln Gln Gln Gln Thr Gln Pro Gly Lys
1               5                   10

<210>  482
<211>  17
<212>  PRT
<213>  Glycine max

<400>  482

Gly Ala Ile Val Ala Tyr Thr Arg Gly Leu Ala Leu Gln Leu Val Ser
1               5                   10                  15

Lys

<210>  483
<211>  8
<212>  PRT
<213>  Glycine max

<400>  483

Leu Leu Asp Tyr Thr Ser Thr Lys
1               5

<210>  484
<211>  882
<212>  DNA
<213>  Glycine max

<400>  484
atggcaagca ataaggagtc taagttccca gcacagagcc agaaaactca gccaggaaaa      60

gaacatgtaa tgaatccact cccacaagcc acaaatcctg atcacaaggc cgccaataaa     120

ctccagggaa aggtggcgtt ggtgacagga ggtgactcag gaattggcag agcggtttgc     180

ctgtgtttcg caaaagaggg tgcaaccgtg gcctttacat acgtaaaggg ccatgaggac     240

agggataaag atgatactct gaagatgctg cttgaagcta agacaagtgg tgcagacaat     300

ccattggcaa tagcagcgga tattggcttt gatgagaact gcaaacaggt cattgacctt     360

gttgtcaaag aatatggccg ccttgatgtt ctggtcaaca atgcagctga gcagcatttg     420

acaaactctg ttgaggaaat cacacaacag cagcttgaga gagtcttcgg aaccaacatc     480

ttttctcagt tctttttggt caagcatgct ctgaagcaca tgaaagaagg gagctgcatc 540

ataaactcta cttcagttaa tgcatacaat gggaatccag aagcgttgga ctacactgct 600

accaagggag caattgtggc cttcaccaga ggtctttctc agcagctagc gagtagggga 660

attagggtga atggtgtggc acctggccca gtttggacgc caatacaacc agcttcaaag 720

cctgctgaga tgattcagaa cttggggtgt gaggtgccaa tgaaccgcgt ggctcagcct 780

tgtgagattg caccatgtta tttgttcttg gcaacttgtc aggactcttc ctactttact 840

ggccaagtcc tccatccaaa tggtgggatg gtcgtcaacg ct 882

<210> 485
<211> 294
<212> PRT
<213> Glycine max

<400> 485

Met Ala Ser Asn Lys Glu Ser Lys Phe Pro Ala Gln Ser Gln Lys Thr
1               5                   10                  15

Gln Pro Gly Lys Glu His Val Met Asn Pro Leu Pro Gln Ala Thr Asn
            20                  25                  30

Pro Asp His Lys Ala Ala Asn Lys Leu Gln Gly Lys Val Ala Leu Val
        35                  40                  45

Thr Gly Gly Asp Ser Gly Ile Gly Arg Ala Val Cys Leu Cys Phe Ala
    50                  55                  60

Lys Glu Gly Ala Thr Val Ala Phe Thr Tyr Val Lys Gly His Glu Asp
65                  70                  75                  80

Arg Asp Lys Asp Asp Thr Leu Lys Met Leu Leu Glu Ala Lys Thr Ser
                85                  90                  95

Gly Ala Asp Asn Pro Leu Ala Ile Ala Ala Asp Ile Gly Phe Asp Glu
            100                 105                 110

Asn Cys Lys Gln Val Ile Asp Leu Val Val Lys Glu Tyr Gly Arg Leu
        115                 120                 125

Asp Val Leu Val Asn Asn Ala Ala Glu Gln His Leu Thr Asn Ser Val
    130                 135                 140

Glu Glu Ile Thr Gln Gln Gln Leu Glu Arg Val Phe Gly Thr Asn Ile
145                 150                 155                 160

Phe Ser Gln Phe Phe Leu Val Lys His Ala Leu Lys His Met Lys Glu

165                 170                 175

Gly Ser Cys Ile Ile Asn Ser Thr Ser Val Asn Ala Tyr Asn Gly Asn
            180                 185                 190

Pro Glu Ala Leu Asp Tyr Thr Ala Thr Lys Gly Ala Ile Val Ala Phe
        195                 200                 205

Thr Arg Gly Leu Ser Gln Gln Leu Ala Ser Arg Gly Ile Arg Val Asn
    210                 215                 220

Gly Val Ala Pro Gly Pro Val Trp Thr Pro Ile Gln Pro Ala Ser Lys
225                 230                 235                 240

Pro Ala Glu Met Ile Gln Asn Leu Gly Cys Glu Val Pro Met Asn Arg
                245                 250                 255

Val Ala Gln Pro Cys Glu Ile Ala Pro Cys Tyr Leu Phe Leu Ala Thr
            260                 265                 270

Cys Gln Asp Ser Ser Tyr Phe Thr Gly Gln Val Leu His Pro Asn Gly
        275                 280                 285

Gly Met Val Val Asn Ala
    290

<210>  486
<211>  11
<212>  PRT
<213>  Glycine max

<400>  486

Glu Gly Ala Thr Val Ala Phe Thr Tyr Val Lys
1               5                   10

<210>  487
<211>  8
<212>  PRT
<213>  Glycine max

<400>  487

Gln Val Ile Asp Leu Val Val Lys
1               5

<210>  488
<211>  879
<212>  DNA
<213>  Glycine max

<400>  488

atggcttcgg gtgaaaagaa attcccacct caacaacaac aaacacagcc tgggaaggag 60

catgctatga atccagtacc ccaattcact agccctgact acaagccttc aaataaactt 120

caaggaaaga tagcattagt gactgggggt gactctggga ttggacgagc ggtgtgtaac 180

ttgtttgcct tagaaggtgc taccgtgggc ttcacatatg tgaaggggca tgaggacaag 240

gacgcgaggg acacgttgga aatgatcaag agggcaaaga cttcggatgc taaagatcca 300

atggcggtac cagctgattt gggttacgac gagaattgca agagagtggt tgatgaggtc 360

gtgaatgctt atggttgtat tgacattctg gtcaacaatg cagctgagca atacgagtgt 420

ggaacagtgg aggacattga tgagcctagg cttgagaggg tctttcgtac aaatatcttc 480

tcctatttct tcatgaccag gcatgccttg aagcacatga aggaaggaag cagcattatc 540

aacacgacat cggtgaatgc atacaaggga aatgcgaaac tattggacta cacgtccacg 600

aagggagcaa ttgtggccta tacaagggga cttgctcttc agttggtgag taagggaatt 660

cgggttaatg gggtggctcc tgggcctatt tggaccccat tgataccctc ctctttcaag 720

gaggaagaaa cggctcaatt tggtgcccag gtgccaatga agagagctgg ccagcctatt 780

gaggttgctc cgtcttatgt tttcttgctt tgcaaccaat gctcctctta cataactgga 840

caagtccttc accccaatgg tggaaccgtt gtcaatggt 879

<210> 489
<211> 293
<212> PRT
<213> Glycine max

<400> 489

Met Ala Ser Gly Glu Lys Lys Phe Pro Pro Gln Gln Gln Gln Thr Gln
1               5                   10                  15

Pro Gly Lys Glu His Ala Met Asn Pro Val Pro Gln Phe Thr Ser Pro
            20                  25                  30

Asp Tyr Lys Pro Ser Asn Lys Leu Gln Gly Lys Ile Ala Leu Val Thr
        35                  40                  45

Gly Gly Asp Ser Gly Ile Gly Arg Ala Val Cys Asn Leu Phe Ala Leu
    50                  55                  60

Glu Gly Ala Thr Val Gly Phe Thr Tyr Val Lys Gly His Glu Asp Lys
65                  70                  75                  80

Asp Ala Arg Asp Thr Leu Glu Met Ile Lys Arg Ala Lys Thr Ser Asp
                85                  90                  95

Ala Lys Asp Pro Met Ala Val Pro Ala Asp Leu Gly Tyr Asp Glu Asn

            100                 105                 110

Cys Lys Arg Val Val Asp Glu Val Val Asn Ala Tyr Gly Cys Ile Asp
        115                 120                 125

Ile Leu Val Asn Asn Ala Ala Glu Gln Tyr Glu Cys Gly Thr Val Glu
    130                 135                 140

Asp Ile Asp Glu Pro Arg Leu Glu Arg Val Phe Arg Thr Asn Ile Phe
145                 150                 155                 160

Ser Tyr Phe Phe Met Thr Arg His Ala Leu Lys His Met Lys Glu Gly
                165                 170                 175

Ser Ser Ile Ile Asn Thr Thr Ser Val Asn Ala Tyr Lys Gly Asn Ala
            180                 185                 190

Lys Leu Leu Asp Tyr Thr Ser Thr Lys Gly Ala Ile Val Ala Tyr Thr
        195                 200                 205

Arg Gly Leu Ala Leu Gln Leu Val Ser Lys Gly Ile Arg Val Asn Gly
    210                 215                 220

Val Ala Pro Gly Pro Ile Trp Thr Pro Leu Ile Pro Ser Ser Phe Lys
225                 230                 235                 240

Glu Glu Glu Thr Ala Gln Phe Gly Ala Gln Val Pro Met Lys Arg Ala
                245                 250                 255

Gly Gln Pro Ile Glu Val Ala Pro Ser Tyr Val Phe Leu Ala Cys Asn
            260                 265                 270

Gln Cys Ser Ser Tyr Ile Thr Gly Gln Val Leu His Pro Asn Gly Gly
        275                 280                 285

Thr Val Val Asn Gly
    290

<210> 490
<211> 15
<212> PRT
<213> Glycine max

<400> 490

Glu Gly Ser Ser Ile Ile Asn Thr Thr Ser Val Asn Ala Tyr Lys
1               5                   10                  15

<210> 491

<211>  12
<212>  PRT
<213>  Glycine max

<400>  491

Phe Pro Pro Gln Gln Gln Gln Thr Gln Pro Gly Lys
1               5                   10


<210>  492
<211>  17
<212>  PRT
<213>  Glycine max

<400>  492

Gly Ala Ile Val Ala Tyr Thr Arg Gly Leu Ala Leu Gln Leu Val Ser
1               5                   10                  15


Lys


<210>  493
<211>  8
<212>  PRT
<213>  Glycine max

<400>  493

Leu Leu Asp Tyr Thr Ser Thr Lys
1               5


<210>  494
<211>  3207
<212>  DNA
<213>  Glycine max

<400>  494
atggcttctg agcaaatgcc tcgcagagag aacaacagca atagggtgat agagagagag      60

gttcacgttg agacacacag aaaagagtcc ctccctgata agggaagggt tgtgatagag     120

agtgagaaga aggagaaaga gagagagttg gcttctgata gagccaggtc tggaaatata     180

gttaaggagg acaaagaggt tgaagaagga agaagaggaa aagaaagtgg tggtggtggt     240

ggtgttcagc atgagaggaa gtttgaaacg aaagttgaag agaagaaggg ttttggggaa     300

aaggctgaac tcgagggaat aacaagagtg gtggtgggaa gagagtgcag caaagaggag     360

aaacaaggaa gaacaagaga gaaagtagag gcagagaaag caagatcaat ggagaaaact     420

agagatgaag ctaaaggaag agaacaagct gaaaccaaag gtcaaaccaa gaagggtggt     480

ggtgaggaac agggaagaag aagagaagaa ataggagaag aaaataaaca atccagcctc     540

gaagagattt ctaaatgcag agctgaagct gaagagaaag ttaaggagag aaaggagaga     600

tcaaaacaag cagcaagtaa tgaagctgcc aaagagagaa acaagcaggc aaataaggag     660

gcaacaaagg caaaagatgt cacaagagag aagggtcaac aaggcaatct taaaacagga 720

aatgagacac aagacattgg ccaaaagggt tatggtggag caaaggatac cattggtaga 780

gtggacaaca ttactacacc acttgctaaa aaggctcaag ttgctggtag caccactgtt 840

cagtatgctg gagagaaatc agcacaagcc aaagatgcta cggtaggatc aatggagaaa 900

actacaaagg atgctaaagg aagagaaaaa gcaggaacca aaggtcaaac aaagaagggt 960

ggtggtgagg aacaaggaaa aagaagagaa gaaacaggag aagaaaaaca atccagcctg 1020

gaggagattt ctaagcacag agctgaagct caacaaaaag caatgagttc aattgaaggt 1080

gctaaggaga gatacgagag agcaaaacaa gaagcaagta aagcttccaa agagagaaac 1140

attcaagcaa ataagagggc tgaagaagaa agaagaaaag cagaaagtgg taggggtgtt 1200

catgtggata agtttgaaga gaagaaggat tcagtgctga aagcagaact cgagagaaga 1260

acaagagagg tagagggaag agagtggaac aaagaggaga aacaaagaag ttgtccaaga 1320

gggaaagtag aggcagagaa agcaagacca atggaagaaa caaaactgga agctaaagga 1380

agagaaaaag ctgaaaccaa agagggtggt ggtgaggaaa aaggaatgag aagagaagaa 1440

ataggagaag aaaataaaca atccagcctg gaagagattt ttaagtacag agctgaagct 1500

caacagaaag ttaaggagag aaacgagaga gcaaaacaag cagcagaagc tgccaaagag 1560

agaaacaagc aggcaaataa agggaatgaa gaaggcagaa gagaaagaga aagtggtggg 1620

ggtgttcatc atgaggctaa gtttgaaact aaaggtgaaa aaaagaagga tttagtggga 1680

agagagcgga acaaagagga gaaacaaaga agcagtccaa gaggaaaagt agaggcagag 1740

aaagcaagac caatggaaga aacaaaggtg gaagctaaag gaagagaaaa agatgtaacc 1800

aaagagggtg gtgttgagga acaaggaaga ggaagaaaag aaaatgaaca atctagcttg 1860

aaagacattt ccaagtacag agctgaagct caacaaaatt cattcaatgc tactgaagct 1920

gctaaggaaa gatacgagag ggcaaaccaa gcgacaagtg aagctgccaa agagagaaac 1980

aagcaggcaa ataaggaggc aacaaaggca aaagatgtca caagagaaat ggatcaacaa 2040

ggcaatctta aaacagaaaa tgagacacag gacaatgacc agaaggttta tggtggagca 2100

aagtacacca ttggtagtgt ggaccacatt actacaccat ttattgtaga ggccaatctt 2160

aaatcagaaa atcagataca agacaatggc caaaagggtt atggtggagc aaaggacaca 2220

attggtagtg tagaccacat tactacacca cttgctgaaa aggccaaagc tgctggtagc 2280

accactgctc agtatgttgg ggagaaagca gcacaagcca aagatgctac tgtgcaaact 2340

ggcaagagtg ctgcaaaagt tgcctcagat ttgaggacaa aggctttggc cacaggtgg 2400

agtgctgcac attattctac tgagataact gtggagggaa ccaaggctgc aacaaatgtt 2460

gttaagggag ttgctgagta tgcaggacag aaggcttctg agcttgcagc tatgtcagtg 2520

gatactgcga aaggtttagc tgctgctgca gctggggaga atgccaagga gtacactgca 2580

aggaagaagg tggaagctga gatggatttg gaggccaaaa gggaagctca aaatcaggaa 2640

tcgaaggagt ggccatcagc aaaatccacc actgagagca ttcaaagagg acaaggacaa 2700

caagaacagc atgagcaagg agaaaaatgg gaacatggtg cacaaaggac taataagcca 2760

catgaaagca acattgtgga aacctttgag gaaaacctag gtgggattgg ggaaaaaagg 2820

gaacaaaaac cacttggaag catcattgga gagagctttc aaggagttca agaaaaaggg 2880

agtgaagtga tggcaagcat tggtgaaaaa ctgggaagtg ctgcacatac attagtgaag 2940

aagccattgg ataaggccac tgagggaggg agagaggtgt tgggagctgt aggtgaaact 3000

gtggttgaga ttggtgaaaa catggtgaaa aaaccagcac agaaagtgca gcagcatcaa 3060

ggtgaaggtg gaggaggggt gctgaatgcc attggtgaaa ccatagcaga gatagcagag 3120

acaactaagg tgattgttgc tggagaggga gaaaaggaga taatagtaaa aacacatgaa 3180

tttgaatatg gagacttgaa acttgat 3207

<210> 495
<211> 1069
<212> PRT
<213> Glycine max

<400> 495

Met Ala Ser Glu Gln Met Pro Arg Arg Glu Asn Asn Ser Asn Arg Val
1               5                   10                  15

Ile Glu Arg Glu Val His Val Glu Thr His Arg Lys Glu Ser Leu Pro
            20                  25                  30

Asp Lys Gly Arg Val Val Ile Glu Ser Glu Lys Lys Glu Lys Glu Arg
        35                  40                  45

Glu Leu Ala Ser Asp Arg Ala Arg Ser Gly Asn Ile Val Lys Glu Asp
    50                  55                  60

Lys Glu Val Glu Glu Gly Arg Arg Gly Lys Glu Ser Gly Gly Gly Gly
65                  70                  75                  80

Gly Val Gln His Glu Arg Lys Phe Glu Thr Lys Val Glu Glu Lys Lys
                85                  90                  95

Gly Phe Gly Glu Lys Ala Glu Leu Glu Gly Ile Thr Arg Val Val Val
            100                 105                 110

Gly Arg Glu Cys Ser Lys Glu Glu Lys Gln Gly Arg Thr Arg Glu Lys
        115                 120                 125

Val Glu Ala Glu Lys Ala Arg Ser Met Glu Lys Thr Arg Asp Glu Ala
    130                 135                 140

Lys Gly Arg Glu Gln Ala Glu Thr Lys Gly Gln Thr Lys Lys Gly Gly
145                 150                 155                 160

Gly Glu Glu Gln Gly Arg Arg Arg Glu Glu Ile Gly Glu Glu Asn Lys
                165                 170                 175

Gln Ser Ser Leu Glu Glu Ile Ser Lys Cys Arg Ala Glu Ala Glu Glu
            180                 185                 190

Lys Val Lys Glu Arg Lys Glu Arg Ser Lys Gln Ala Ala Ser Asn Glu
        195                 200                 205

Ala Ala Lys Glu Arg Asn Lys Gln Ala Asn Lys Glu Ala Thr Lys Ala
    210                 215                 220

Lys Asp Val Thr Arg Glu Lys Gly Gln Gln Gly Asn Leu Lys Thr Gly
225                 230                 235                 240

Asn Glu Thr Gln Asp Ile Gly Gln Lys Gly Tyr Gly Gly Ala Lys Asp
                245                 250                 255

Thr Ile Gly Arg Val Asp Asn Ile Thr Thr Pro Leu Ala Lys Lys Ala
            260                 265                 270

Gln Val Ala Gly Ser Thr Thr Val Gln Tyr Ala Gly Glu Lys Ser Ala
        275                 280                 285

Gln Ala Lys Asp Ala Thr Val Gly Ser Met Glu Lys Thr Thr Lys Asp
    290                 295                 300

Ala Lys Gly Arg Glu Lys Ala Gly Thr Lys Gly Gln Thr Lys Lys Gly
305                 310                 315                 320

Gly Gly Glu Glu Gln Gly Lys Arg Arg Glu Glu Thr Gly Glu Glu Lys
                325                 330                 335

Gln Ser Ser Leu Glu Glu Ile Ser Lys His Arg Ala Glu Ala Gln Gln
            340                 345                 350

Lys Ala Met Ser Ser Ile Glu Gly Ala Lys Glu Arg Tyr Glu Arg Ala
        355                 360                 365

Lys Gln Glu Ala Ser Lys Ala Ser Lys Glu Arg Asn Ile Gln Ala Asn
    370                 375                 380

Lys Arg Ala Glu Glu Glu Arg Arg Lys Ala Glu Ser Gly Arg Gly Val
385                 390                 395                 400

His Val Asp Lys Phe Glu Glu Lys Lys Asp Ser Val Leu Lys Ala Glu
                405                 410                 415

Leu Glu Arg Arg Thr Arg Glu Val Glu Gly Arg Glu Trp Asn Lys Glu
            420                 425                 430

Glu Lys Gln Arg Ser Cys Pro Arg Gly Lys Val Glu Ala Glu Lys Ala
        435                 440                 445

Arg Pro Met Glu Glu Thr Lys Leu Glu Ala Lys Gly Arg Glu Lys Ala
    450                 455                 460

Glu Thr Lys Glu Gly Gly Gly Glu Glu Lys Gly Met Arg Arg Glu Glu
465                 470                 475                 480

Ile Gly Glu Glu Asn Lys Gln Ser Ser Leu Glu Glu Ile Phe Lys Tyr
                485                 490                 495

Arg Ala Glu Ala Gln Gln Lys Val Lys Glu Arg Asn Glu Arg Ala Lys
            500                 505                 510

Gln Ala Ala Glu Ala Ala Lys Glu Arg Asn Lys Gln Ala Asn Lys Gly
        515                 520                 525

Asn Glu Glu Gly Arg Arg Glu Arg Glu Ser Gly Gly Gly Val His His
    530                 535                 540

Glu Ala Lys Phe Glu Thr Lys Gly Glu Lys Lys Lys Asp Leu Val Gly
545                 550                 555                 560

Arg Glu Arg Asn Lys Glu Glu Lys Gln Arg Ser Ser Pro Arg Gly Lys
                565                 570                 575

Val Glu Ala Glu Lys Ala Arg Pro Met Glu Glu Thr Lys Val Glu Ala
            580                 585                 590

Lys Gly Arg Glu Lys Asp Val Thr Lys Glu Gly Gly Val Glu Glu Gln
        595                 600                 605

Gly Arg Gly Arg Lys Glu Asn Glu Gln Ser Ser Leu Lys Asp Ile Ser
    610                 615                 620

Lys Tyr Arg Ala Glu Ala Gln Gln Asn Ser Phe Asn Ala Thr Glu Ala

625                 630                 635                 640

Ala Lys Glu Arg Tyr Glu Arg Ala Asn Gln Ala Thr Ser Glu Ala Ala
                645                 650                 655

Lys Glu Arg Asn Lys Gln Ala Asn Lys Glu Ala Thr Lys Ala Lys Asp
            660                 665                 670

Val Thr Arg Glu Met Asp Gln Gln Gly Asn Leu Lys Thr Glu Asn Glu
        675                 680                 685

Thr Gln Asp Asn Asp Gln Lys Gly Tyr Gly Gly Ala Lys Tyr Thr Ile
    690                 695                 700

Gly Ser Val Asp His Ile Thr Thr Pro Phe Ile Val Glu Ala Asn Leu
705                 710                 715                 720

Lys Ser Glu Asn Gln Ile Gln Asp Asn Gly Gln Lys Gly Tyr Gly Gly
                725                 730                 735

Ala Lys Asp Thr Ile Gly Ser Val Asp His Ile Thr Thr Pro Leu Ala
            740                 745                 750

Glu Lys Ala Lys Ala Ala Gly Ser Thr Thr Ala Gln Tyr Val Gly Glu
        755                 760                 765

Lys Ala Ala Gln Ala Lys Asp Ala Thr Val Gln Thr Gly Lys Ser Ala
    770                 775                 780

Ala Lys Val Ala Ser Asp Leu Arg Asp Lys Ala Leu Ala Thr Gly Trp
785                 790                 795                 800

Ser Ala Ala His Tyr Ser Thr Glu Ile Thr Val Glu Gly Thr Lys Ala
                805                 810                 815

Ala Thr Asn Val Val Lys Gly Val Ala Glu Tyr Ala Gly Gln Lys Ala
            820                 825                 830

Ser Glu Leu Ala Ala Met Ser Val Asp Thr Ala Lys Gly Leu Ala Ala
        835                 840                 845

Ala Ala Ala Gly Glu Asn Ala Lys Glu Tyr Thr Ala Arg Lys Lys Val
    850                 855                 860

Glu Ala Glu Met Asp Leu Glu Ala Lys Arg Glu Ala Gln Asn Gln Glu
865                 870                 875                 880

Ser Lys Glu Trp Pro Ser Ala Lys Ser Thr Thr Glu Ser Ile Gln Arg
                885                 890                 895

Gly Gln Gly Gln Gln Glu Gln His Glu Gln Gly Glu Lys Trp Glu His
            900                 905                 910

Gly Ala Gln Arg Thr Asn Lys Pro His Glu Ser Asn Ile Val Glu Thr
        915                 920                 925

Phe Glu Glu Asn Leu Gly Gly Ile Gly Glu Lys Arg Glu Gln Lys Pro
    930                 935                 940

Leu Gly Ser Ile Ile Gly Glu Ser Phe Gln Gly Val Gln Glu Lys Gly
945                 950                 955                 960

Ser Glu Val Met Ala Ser Ile Gly Glu Lys Leu Gly Ser Ala Ala His
                965                 970                 975

Thr Leu Val Lys Lys Pro Leu Asp Lys Ala Thr Glu Gly Gly Arg Glu
            980                 985                 990

Val Leu Gly Ala Val Gly Glu Thr Val Val Glu Ile Gly Glu Asn Met
        995                 1000                1005

Val Lys Lys Pro Ala Gln Lys Val Gln Gln His Gln Gly Glu Gly
    1010                1015                1020

Gly Gly Gly Val Leu Asn Ala Ile Gly Glu Thr Ile Ala Glu Ile
    1025                1030                1035

Ala Glu Thr Thr Lys Val Ile Val Ala Gly Glu Gly Glu Lys Glu
    1040                1045                1050

Ile Ile Val Lys Thr His Glu Phe Glu Tyr Gly Asp Leu Lys Leu
    1055                1060                1065

Asp

<210> 496
<211> 13
<212> PRT
<213> Glycine max

<400> 496

Ala Ala Gly Ser Thr Thr Ala Gln Tyr Val Gly Glu Lys
1               5                   10

<210>  497
<211>  21
<212>  PRT
<213>  Glycine max

<400>  497

Ala Leu Ala Thr Gly Trp Ser Ala Ala His Tyr Ser Thr Glu Ile Thr
1               5                   10                  15

Val Glu Gly Thr Lys
            20

<210>  498
<211>  15
<212>  PRT
<213>  Glycine max

<400>  498

Ala Gln Val Ala Gly Ser Thr Thr Val Gln Tyr Ala Gly Glu Lys
1               5                   10                  15

<210>  499
<211>  15
<212>  PRT
<213>  Glycine max

<400>  499

Asp Thr Ile Gly Arg Val Asp Asn Ile Thr Thr Pro Leu Ala Lys
1               5                   10                  15

<210>  500
<211>  16
<212>  PRT
<213>  Glycine max

<400>  500

Asp Thr Ile Gly Ser Val Asp His Ile Thr Thr Pro Leu Ala Glu Lys
1               5                   10                  15

<210>  501
<211>  16
<212>  PRT
<213>  Glycine max

<400>  501

Glu Arg Glu Leu Ala Ser Asp Arg Ala Arg Ser Gly Asn Ile Val Lys
1               5                   10                  15

<210>  502
<211>  15
<212>  PRT
<213>  Glycine max

<400>  502

Glu Arg Tyr Glu Arg Ala Asn Gln Ala Thr Ser Glu Ala Ala Lys
1               5                   10                  15


<210>  503
<211>  18
<212>  PRT
<213>  Glycine max

<400>  503

Gly Gly Gly Glu Glu Gln Gly Arg Arg Arg Glu Glu Ile Gly Glu Glu
1               5                   10                  15


Asn Lys




<210>  504
<211>  12
<212>  PRT
<213>  Glycine max

<400>  504

Gly Leu Ala Ala Ala Ala Ala Gly Glu Asn Ala Lys
1               5                   10


<210>  505
<211>  9
<212>  PRT
<213>  Glycine max

<400>  505

Gly Arg Val Val Ile Glu Ser Glu Lys
1               5


<210>  506
<211>  9
<212>  PRT
<213>  Glycine max

<400>  506

Gly Val Ala Glu Tyr Ala Gly Gln Lys
1               5


<210>  507
<211>  10
<212>  PRT
<213>  Glycine max

<400>  507

Leu Gly Ser Ala Ala His Thr Leu Val Lys

1               5                   10


<210>  508
<211>  9
<212>  PRT
<213>  Glycine max

<400>  508

Gln Ser Ser Leu Glu Glu Ile Phe Lys
1               5


<210>  509
<211>  9
<212>  PRT
<213>  Glycine max

<400>  509

Gln Ser Ser Leu Glu Glu Ile Ser Lys
1               5


<210>  510
<211>  20
<212>  PRT
<213>  Glycine max

<400>  510

Arg Glu Gln Lys Pro Leu Gly Ser Ile Ile Gly Glu Ser Phe Gln Gly
1               5                   10                  15


Val Gln Glu Lys
            20


<210>  511
<211>  11
<212>  PRT
<213>  Glycine max

<400>  511

Thr Gly Asn Glu Thr Gln Asp Ile Gly Gln Lys
1               5                   10


<210>  512
<211>  9
<212>  PRT
<213>  Glycine max

<400>  512

Val Ile Val Ala Gly Glu Gly Glu Lys
1               5


<210>  513

<211>  28
<212>  PRT
<213>  Glycine max

<400>  513

Val Gln Gln His Gln Gly Glu Gly Gly Gly Gly Val Leu Asn Ala Ile
1               5                   10                  15

Gly Glu Thr Ile Ala Glu Ile Ala Glu Thr Thr Lys
            20                  25

<210>  514
<211>  735
<212>  DNA
<213>  Glycine max

<400>  514
atggcactgt gcaaccgcgt cagatcggga atctctctct tcaccaaatc cacgttcctc      60

acttcccagc gatccactct ccaacgatcc ctcgttgccc ctttcatttc cctcgcctcc     120

agaaattacg ccaacgtgcc ggggcaaaag gaaaccaaag ttaaggtccc tctggctttg     180

tttggaggtt caggaaacta tgcctctgct ttgtatattg catctgtgaa agccaatgca     240

gtggaaaagg ttgagactga gcttctgcaa tttgttgagg cagtaaagaa tagtcccata     300

ttttcacagt ttataaggga cttgtctgtg gctaaggatg ttagagttaa agttatccag     360

gatatttgtg gggaagccaa gttttcggat gttacaaaga acttccttgt tattgttgct     420

gagaatggaa ggcttaaaaa catagacacc attgcaaaga ggtttgcaca gttgtcaatg     480

gcatataagg gagaagtgca agcgactgtg accactgttg ttcctcttcc ccctgaagag     540

gaaaaggcat tgaaggagac tcttcaggaa ataataggtt ctggagcgaa ggttcatctt     600

gaacagaaga ttgatccaag catacttggt gggcttgtgc ttgaatttag ccagaaggtc     660

tttgacatgt caattaagac cagggcacaa cagatggaga ggatcctccg agagcccgtg     720

tccattgcca acatc                                                      735

<210>  515
<211>  245
<212>  PRT
<213>  Glycine max

<400>  515

Met Ala Leu Cys Asn Arg Val Arg Ser Gly Ile Ser Leu Phe Thr Lys
1               5                   10                  15

Ser Thr Phe Leu Thr Ser Gln Arg Ser Thr Leu Gln Arg Ser Leu Val
            20                  25                  30

Ala Pro Phe Ile Ser Leu Ala Ser Arg Asn Tyr Ala Asn Val Pro Gly

35                  40                  45

Gln Lys Glu Thr Lys Val Lys Val Pro Leu Ala Leu Phe Gly Gly Ser
    50                  55                  60

Gly Asn Tyr Ala Ser Ala Leu Tyr Ile Ala Ser Val Lys Ala Asn Ala
65                  70                  75                  80

Val Glu Lys Val Glu Thr Glu Leu Leu Gln Phe Val Glu Ala Val Lys
                85                  90                  95

Asn Ser Pro Ile Phe Ser Gln Phe Ile Arg Asp Leu Ser Val Ala Lys
            100                 105                 110

Asp Val Arg Val Lys Val Ile Gln Asp Ile Cys Gly Glu Ala Lys Phe
        115                 120                 125

Ser Asp Val Thr Lys Asn Phe Leu Val Ile Val Ala Glu Asn Gly Arg
    130                 135                 140

Leu Lys Asn Ile Asp Thr Ile Ala Lys Arg Phe Ala Gln Leu Ser Met
145                 150                 155                 160

Ala Tyr Lys Gly Glu Val Gln Ala Thr Val Thr Thr Val Val Pro Leu
                165                 170                 175

Pro Pro Glu Glu Glu Lys Ala Leu Lys Glu Thr Leu Gln Glu Ile Ile
            180                 185                 190

Gly Ser Gly Ala Lys Val His Leu Glu Gln Lys Ile Asp Pro Ser Ile
        195                 200                 205

Leu Gly Gly Leu Val Leu Glu Phe Ser Gln Lys Val Phe Asp Met Ser
    210                 215                 220

Ile Lys Thr Arg Ala Gln Gln Met Glu Arg Ile Leu Arg Glu Pro Val
225                 230                 235                 240

Ser Ile Ala Asn Ile
                245

<210>  516
<211>  12
<212>  PRT
<213>  Glycine max

<400>  516

Glu Thr Leu Gln Glu Ile Ile Gly Ser Gly Ala Lys

1               5                   10

<210>  517
<211>  678
<212>  DNA
<213>  Glycine max

<400>  517
atgaccacac aagtaccacc acacagtgtc caagtccaca caacaacaca ccgctacgaa      60

gctggcgtcg tcccccccgg agcccgtttc gaaccgccgc gttatgaagc cggcgtcaaa     120

gcgccctcca tttaccattc cgagagaggt ccaacgactt cccaggtcct cgccgtcctc     180

gccggcctcc cggtcggtgg catcctgctc ctcctggcgg gattgaccct ggcaggaacc     240

ctcaccggac tggcagttgc cacgccgctc ttcgtcctct tcagcccggt gctggttcca     300

gccacggtcg ccatcggcct ggccgtggcc gggttcctga cgtcgggggc cttcgggctc     360

acggcgctgt cttccttctc ctggatcctg aactacatcc gggagaccca gccagcctcg     420

gagaacctcg ccgccgccgc gaagcatcac ctcgccgagg cggcggagta cgtggggcag     480

aagacgaagg aagtggggca gaagaccaag gaggttgggc aggatattca gagcaaggca     540

caggatacaa gggaggcagc tgcaagggat gcaaggaggc cagctgcaag ggatgcaagg     600

gaagctgctg caagggatgc aaaggtggag gcaagggatg taaagagaac aacagtgaca     660

gcaacaaccg caaccgca                                                   678

<210>  518
<211>  226
<212>  PRT
<213>  Glycine max

<400>  518

Met Thr Thr Gln Val Pro Pro His Ser Val Gln Val His Thr Thr Thr
1               5                   10                  15

His Arg Tyr Glu Ala Gly Val Val Pro Pro Gly Ala Arg Phe Glu Pro
            20                  25                  30

Pro Arg Tyr Glu Ala Gly Val Lys Ala Pro Ser Ile Tyr His Ser Glu
        35                  40                  45

Arg Gly Pro Thr Thr Ser Gln Val Leu Ala Val Leu Ala Gly Leu Pro
    50                  55                  60

Val Gly Gly Ile Leu Leu Leu Leu Ala Gly Leu Thr Leu Ala Gly Thr
65                  70                  75                  80

Leu Thr Gly Leu Ala Val Ala Thr Pro Leu Phe Val Leu Phe Ser Pro
                85                  90                  95

Val Leu Val Pro Ala Thr Val Ala Ile Gly Leu Ala Val Ala Gly Phe
                100                 105                 110

Leu Thr Ser Gly Ala Phe Gly Leu Thr Ala Leu Ser Ser Phe Ser Trp
        115                 120                 125

Ile Leu Asn Tyr Ile Arg Glu Thr Gln Pro Ala Ser Glu Asn Leu Ala
    130                 135                 140

Ala Ala Ala Lys His His Leu Ala Glu Ala Ala Glu Tyr Val Gly Gln
145                 150                 155                 160

Lys Thr Lys Glu Val Gly Gln Lys Thr Lys Glu Val Gly Gln Asp Ile
                165                 170                 175

Gln Ser Lys Ala Gln Asp Thr Arg Glu Ala Ala Ala Arg Asp Ala Arg
            180                 185                 190

Glu Ala Ala Ala Arg Asp Ala Arg Glu Ala Ala Ala Arg Asp Ala Lys
        195                 200                 205

Val Glu Ala Arg Asp Val Lys Arg Thr Thr Val Thr Ala Thr Thr Ala
    210                 215                 220

Thr Ala
225

<210>  519
<211>  9
<212>  PRT
<213>  Glycine max

<400>  519

Glu Val Gly Gln Asp Ile Gln Ser Lys
1               5

<210>  520
<211>  11
<212>  PRT
<213>  Glycine max

<400>  520

Arg Thr Thr Val Thr Ala Thr Thr Ala Thr Ala
1               5                   10

<210>  521
<211>  627
<212>  DNA

<213>  Glycine max

<400>  521
atgacaagga aacaagcaag ttccacgaga cgtggcggtc tttcaggagt cttgcattcc      60

aaatccaaat cttctcctct actctccata tcccttgtcc tatttgtagc catccttctc     120

atcctttatg cttgtatcgg gtcaggtata ttgggaggaa gaaaggatgt gattagcatg     180

gctgaagatg atttttcatg cacttttgaa gttccaagtg caattcctgt tttgaagaat     240

gcatatggtg gcagcatgaa aaatgttttg cacgtaggcc ctgagagttg ttcggtggtc     300

tccaaatttt taagagaagg ggaaactgaa gcatggggtg tggagccata tgacttagac     360

gatgctgata ggaactgcaa ggctcttgtg cagaaaggca ttatacgtgt tgcagatata     420

aaattccctt taccatacag ggtgaagtct ttttctcatg ttatagtgtc agatgctttg     480

gattacctgt ctccaaaata tataaacaaa actcttcctg agctagcaag ggtatcttct     540

gatggtatta ttattttcac aggttaccca ggtcaaccga gagctaaaat agcaccatta     600

tccaaatttg gccgtccaca taggtgc                                         627

<210>  522
<211>  209
<212>  PRT
<213>  Glycine max

<400>  522

Met Thr Arg Lys Gln Ala Ser Ser Thr Arg Arg Gly Gly Leu Ser Gly
1               5                   10                  15

Val Leu His Ser Lys Ser Lys Ser Ser Pro Leu Leu Ser Ile Ser Leu
            20                  25                  30

Val Leu Phe Val Ala Ile Leu Leu Ile Leu Tyr Ala Cys Ile Gly Ser
        35                  40                  45

Gly Ile Leu Gly Gly Arg Lys Asp Val Ile Ser Met Ala Glu Asp Asp
    50                  55                  60

Phe Ser Cys Thr Phe Glu Val Pro Ser Ala Ile Pro Val Leu Lys Asn
65                  70                  75                  80

Ala Tyr Gly Gly Ser Met Lys Asn Val Leu His Val Gly Pro Glu Ser
                85                  90                  95

Cys Ser Val Val Ser Lys Phe Leu Arg Glu Gly Glu Thr Glu Ala Trp
            100                 105                 110

Gly Val Glu Pro Tyr Asp Leu Asp Asp Ala Asp Arg Asn Cys Lys Ala
        115                 120                 125

Leu Val Gln Lys Gly Ile Ile Arg Val Ala Asp Ile Lys Phe Pro Leu
    130                 135                 140

Pro Tyr Arg Val Lys Ser Phe Ser His Val Ile Val Ser Asp Ala Leu
145                 150                 155                 160

Asp Tyr Leu Ser Pro Lys Tyr Ile Asn Lys Thr Leu Pro Glu Leu Ala
                165                 170                 175

Arg Val Ser Ser Asp Gly Ile Ile Ile Phe Thr Gly Tyr Pro Gly Gln
            180                 185                 190

Pro Arg Ala Lys Ile Ala Pro Leu Ser Lys Phe Gly Arg Pro His Arg
        195                 200                 205

Cys

<210>  523
<211>  8
<212>  PRT
<213>  Glycine max

<400>  523

Asn Ala Tyr Gly Gly Ser Met Lys
1               5

<210>  524
<211>  723
<212>  DNA
<213>  Glycine max

<400>  524
atggccgcgc gagctctgtt gaccagaaaa accctagcca ccgtgctccg caacgacgcg      60

aagcccataa tcggagttgg cataacagca gcggctactc attcacgcgg gttgcacgtg     120

tacacgctac ccgatctgga ttacgactat ggcgcactgg agccagccat cagcggcgac     180

atcatgcagc tgcaccacca gaagcaccac cagacttaca tcaccaacta caacaaggcc     240

ctcgagcagc tccaagacgc catcgccaag aaagattcct ccgccgtcgt taagctccag     300

ggcgccatca agttcaacgg cggaggtcat gtcaaccatt ctattttctg gaaaaatcta     360

gctcctgttc gtgaaggagg tggtgaacca cccaagggtt cactgggatg ggctattgac     420

acacattttg gttcttttga agcattaata caaaaagtta acgcagaagg tgctgcacta     480

caggggtctg gatgggtgtg gcttggtctg gacaaagagt tgaagaggct tgtagttgaa     540

accactgcca accaggaccc actggttact aagggaccaa atttggttcc attgattggt     600

attgatgttt gggagcatgc gtactactta cagtacaaga atgttagacc agactatctg 660

aagaacattt ggaaagttat taattggaaa tatgccagtg aagtgtatga gaaagagagc 720

tct 723

<210> 525
<211> 241
<212> PRT
<213> Glycine max

<400> 525

Met Ala Ala Arg Ala Leu Leu Thr Arg Lys Thr Leu Ala Thr Val Leu
1               5                   10                  15

Arg Asn Asp Ala Lys Pro Ile Ile Gly Val Gly Ile Thr Ala Ala Ala
            20                  25                  30

Thr His Ser Arg Gly Leu His Val Tyr Thr Leu Pro Asp Leu Asp Tyr
        35                  40                  45

Asp Tyr Gly Ala Leu Glu Pro Ala Ile Ser Gly Asp Ile Met Gln Leu
    50                  55                  60

His His Gln Lys His His Gln Thr Tyr Ile Thr Asn Tyr Asn Lys Ala
65                  70                  75                  80

Leu Glu Gln Leu Gln Asp Ala Ile Ala Lys Lys Asp Ser Ser Ala Val
                85                  90                  95

Val Lys Leu Gln Gly Ala Ile Lys Phe Asn Gly Gly Gly His Val Asn
            100                 105                 110

His Ser Ile Phe Trp Lys Asn Leu Ala Pro Val Arg Glu Gly Gly Gly
        115                 120                 125

Glu Pro Pro Lys Gly Ser Leu Gly Trp Ala Ile Asp Thr His Phe Gly
    130                 135                 140

Ser Phe Glu Ala Leu Ile Gln Lys Val Asn Ala Glu Gly Ala Ala Leu
145                 150                 155                 160

Gln Gly Ser Gly Trp Val Trp Leu Gly Leu Asp Lys Glu Leu Lys Arg
                165                 170                 175

Leu Val Val Glu Thr Thr Ala Asn Gln Asp Pro Leu Val Thr Lys Gly
            180                 185                 190

Pro Asn Leu Val Pro Leu Ile Gly Ile Asp Val Trp Glu His Ala Tyr

195                 200                 205

Tyr Leu Gln Tyr Lys Asn Val Arg Pro Asp Tyr Leu Lys Asn Ile Trp
    210                 215                 220

Lys Val Ile Asn Trp Lys Tyr Ala Ser Glu Val Tyr Glu Lys Glu Ser
225                 230                 235                 240

Ser

<210>  526
<211>  11
<212>  PRT
<213>  Glycine max

<400>  526

Ala Leu Glu Gln Leu Gln Asp Ala Ile Ala Lys
1               5                   10

<210>  527
<211>  11
<212>  PRT
<213>  Glycine max

<400>  527

His His Gln Thr Tyr Ile Thr Asn Tyr Asn Lys
1               5                   10

<210>  528
<211>  14
<212>  PRT
<213>  Glycine max

<400>  528

Asn Leu Ala Pro Val Arg Glu Gly Gly Gly Glu Pro Pro Lys
1               5                   10

<210>  529
<211>  8
<212>  PRT
<213>  Glycine max

<400>  529

Asn Val Arg Pro Asp Tyr Leu Lys
1               5

<210>  530
<211>  16
<212>  PRT
<213>  Glycine max

<400>  530

Arg Leu Val Val Glu Thr Thr Ala Asn Gln Asp Pro Leu Val Thr Lys
1               5                   10                  15


<210>  531
<211>  8
<212>  PRT
<213>  Glycine max

<400>  531

Tyr Ala Ser Glu Val Tyr Glu Lys
1               5


<210>  532
<211>  720
<212>  DNA
<213>  Glycine max

<400>  532
atggccgcgc gagccctgtt gaccagaaaa accctagcca ccgttctccg caacgacgcg      60

aagcccataa tcggagctgg catagctgca gctactcatt ctcgtgggtt gcacgtgtac     120

acgctccccg atctggatta cgactatggc gctctggagc cagccatcag cggcgaaatc     180

atgcagctgc accaccagaa gcaccaccag acttacatca ccaacttcaa caaggccctc     240

gagcagctcc aagacgccgt cgccaagaaa gattcctccg ccgtcgttaa gctccagggc     300

gccatcaagt tcaacggcgg aggtcacgtc aaccattcta ttttctggaa aaatctagct     360

cctgttcgtg aaggaggtgg tgaaccaccc aagggttcac tgggatgggc cattgacaca     420

cattttggct cttttgaagc attagtacaa aaagttaatg cagaaggtgc tgcactacag     480

gggtctggat gggtgtggct tggtctggac aaagagttga agaggcttgt agttgaaacc     540

actgccaacc aggacccact ggttactaag ggaccaaatt tggttccatt gcttggtatt     600

gatgtttggg agcatgcgta ctacttacag tacaagaatg ttagaccaga ctatctgaag     660

aacatttgga aagttattaa ttggaaatat gccagtgaag tgtatgagaa agagagctct     720


<210>  533
<211>  240
<212>  PRT
<213>  Glycine max

<400>  533

Met Ala Ala Arg Ala Leu Leu Thr Arg Lys Thr Leu Ala Thr Val Leu
1               5                   10                  15


Arg Asn Asp Ala Lys Pro Ile Ile Gly Ala Gly Ile Ala Ala Ala Thr
            20                  25                  30

His Ser Arg Gly Leu His Val Tyr Thr Leu Pro Asp Leu Asp Tyr Asp
        35                  40                  45

Tyr Gly Ala Leu Glu Pro Ala Ile Ser Gly Glu Ile Met Gln Leu His
    50                  55                  60

His Gln Lys His His Gln Thr Tyr Ile Thr Asn Phe Asn Lys Ala Leu
65                  70                  75                  80

Glu Gln Leu Gln Asp Ala Val Ala Lys Lys Asp Ser Ser Ala Val Val
                85                  90                  95

Lys Leu Gln Gly Ala Ile Lys Phe Asn Gly Gly Gly His Val Asn His
            100                 105                 110

Ser Ile Phe Trp Lys Asn Leu Ala Pro Val Arg Glu Gly Gly Gly Glu
        115                 120                 125

Pro Pro Lys Gly Ser Leu Gly Trp Ala Ile Asp Thr His Phe Gly Ser
    130                 135                 140

Phe Glu Ala Leu Val Gln Lys Val Asn Ala Glu Gly Ala Ala Leu Gln
145                 150                 155                 160

Gly Ser Gly Trp Val Trp Leu Gly Leu Asp Lys Glu Leu Lys Arg Leu
                165                 170                 175

Val Val Glu Thr Thr Ala Asn Gln Asp Pro Leu Val Thr Lys Gly Pro
            180                 185                 190

Asn Leu Val Pro Leu Leu Gly Ile Asp Val Trp Glu His Ala Tyr Tyr
        195                 200                 205

Leu Gln Tyr Lys Asn Val Arg Pro Asp Tyr Leu Lys Asn Ile Trp Lys
    210                 215                 220

Val Ile Asn Trp Lys Tyr Ala Ser Glu Val Tyr Glu Lys Glu Ser Ser
225                 230                 235                 240

<210> 534
<211> 11
<212> PRT
<213> Glycine max

<400> 534

Ala Leu Glu Gln Leu Gln Asp Ala Val Ala Lys
1               5                   10

<210>  535
<211>  11
<212>  PRT
<213>  Glycine max

<400>  535

His His Gln Thr Tyr Ile Thr Asn Phe Asn Lys
1               5                   10


<210>  536
<211>  14
<212>  PRT
<213>  Glycine max

<400>  536

Asn Leu Ala Pro Val Arg Glu Gly Gly Gly Glu Pro Pro Lys
1               5                   10


<210>  537
<211>  8
<212>  PRT
<213>  Glycine max

<400>  537

Asn Val Arg Pro Asp Tyr Leu Lys
1               5


<210>  538
<211>  16
<212>  PRT
<213>  Glycine max

<400>  538

Arg Leu Val Val Glu Thr Thr Ala Asn Gln Asp Pro Leu Val Thr Lys
1               5                   10                  15


<210>  539
<211>  8
<212>  PRT
<213>  Glycine max

<400>  539

Tyr Ala Ser Glu Val Tyr Glu Lys
1               5


<210>  540
<211>  486
<212>  DNA
<213>  Glycine max

<400>  540
atggctccaa ttgcagtggg agatgtcatt ccagacggca ttttggctta cttggatgaa        60

gaaaacaaac cccaaactgt ctctattcac tctctcgccg ccggcaagaa agtcatcatc 120

tttggggttc ccggtgcctt cactcctaca tgcagcttga agcacgtgcc tggcttcatt 180

gagagagcag aagagctgaa aggaaagggt gtggacgaaa taatctgtat cagcgtgaat 240

gatccctttg tgatgaactc atgggccaaa acgttcccag agaacaagca tgttaagttc 300

cttgctgatg gtgcagccaa atacaccaat gccctcggtc ttgagcttga cctcaccgac 360

aagggtctcg gcgtccgctc aaagaggttt gctttgctgg tggaagacct caaggtgaag 420

gttgcaaatg ttgaaagtgg aggagagttc accatctcca gtgctgaaga gatcatcaag 480

gccctc 486

<210> 541
<211> 162
<212> PRT
<213> Glycine max

<400> 541

Met Ala Pro Ile Ala Val Gly Asp Val Ile Pro Asp Gly Ile Leu Ala
1               5                   10                  15

Tyr Leu Asp Glu Glu Asn Lys Pro Gln Thr Val Ser Ile His Ser Leu
            20                  25                  30

Ala Ala Gly Lys Lys Val Ile Ile Phe Gly Val Pro Gly Ala Phe Thr
        35                  40                  45

Pro Thr Cys Ser Leu Lys His Val Pro Gly Phe Ile Glu Arg Ala Glu
    50                  55                  60

Glu Leu Lys Gly Lys Gly Val Asp Glu Ile Ile Cys Ile Ser Val Asn
65                  70                  75                  80

Asp Pro Phe Val Met Asn Ser Trp Ala Lys Thr Phe Pro Glu Asn Lys
                85                  90                  95

His Val Lys Phe Leu Ala Asp Gly Ala Ala Lys Tyr Thr Asn Ala Leu
            100                 105                 110

Gly Leu Glu Leu Asp Leu Thr Asp Lys Gly Leu Gly Val Arg Ser Lys
        115                 120                 125

Arg Phe Ala Leu Leu Val Glu Asp Leu Lys Val Lys Val Ala Asn Val
    130                 135                 140

Glu Ser Gly Gly Glu Phe Thr Ile Ser Ser Ala Glu Glu Ile Ile Lys
145                 150                 155                 160

Ala Leu

<210> 542
<211> 13
<212> PRT
<213> Glycine max

<400> 542

His Val Pro Gly Phe Ile Glu Arg Ala Glu Glu Leu Lys
1               5                   10

<210> 543
<211> 10
<212> PRT
<213> Glycine max

<400> 543

Arg Phe Ala Leu Leu Val Glu Asp Leu Lys
1               5                   10

<210> 544
<211> 20
<212> PRT
<213> Glycine max

<400> 544

Val Ala Asn Val Glu Ser Gly Gly Glu Phe Thr Ile Ser Ser Ala Glu
1               5                   10                  15

Glu Ile Ile Lys
            20

<210> 545
<211> 14
<212> PRT
<213> Glycine max

<400> 545

Tyr Thr Asn Ala Leu Gly Leu Glu Leu Asp Leu Thr Asp Lys
1               5                   10

<210> 546
<211> 486
<212> DNA
<213> Glycine max

<400> 546
atggctccaa ttgcagtggg aaacgtgatt ccagacggca ttttggctta cttggatgaa 60

gaaaacaaac cccaaactgt ctccattcac tctctcgcca ccggcaagaa agtcatcatc 120

tttggggttc ccggtgcctt cactcctact tgcagcttga agcacgttcc tggcttcatt 180

gagagagcag aagagctgaa agggaagggt gtggacgaaa taatctgtat cagtgtgaat 240

gatccatttg tgatgaactc atgggccaaa acattcccag agaacaagca tgttaagttc 300

cttgctgatg gtgcagccaa atacaccaat gcccttggtc ttgagcttga cctcactgac 360

aagggtctcg gcgtccgctc aaagaggttt gctctgctgg tggaagacct caaggtgaag 420

gttgcgaatg ttgaaagtgg aggagagttc accatctcca gtgctgaaga gatcatcaag 480

gccctt 486

<210> 547
<211> 162
<212> PRT
<213> Glycine max

<400> 547

Met Ala Pro Ile Ala Val Gly Asn Val Ile Pro Asp Gly Ile Leu Ala
1               5                   10                  15

Tyr Leu Asp Glu Glu Asn Lys Pro Gln Thr Val Ser Ile His Ser Leu
            20                  25                  30

Ala Thr Gly Lys Lys Val Ile Ile Phe Gly Val Pro Gly Ala Phe Thr
        35                  40                  45

Pro Thr Cys Ser Leu Lys His Val Pro Gly Phe Ile Glu Arg Ala Glu
    50                  55                  60

Glu Leu Lys Gly Lys Gly Val Asp Glu Ile Ile Cys Ile Ser Val Asn
65                  70                  75                  80

Asp Pro Phe Val Met Asn Ser Trp Ala Lys Thr Phe Pro Glu Asn Lys
                85                  90                  95

His Val Lys Phe Leu Ala Asp Gly Ala Ala Lys Tyr Thr Asn Ala Leu
            100                 105                 110

Gly Leu Glu Leu Asp Leu Thr Asp Lys Gly Leu Gly Val Arg Ser Lys
        115                 120                 125

Arg Phe Ala Leu Leu Val Glu Asp Leu Lys Val Lys Val Ala Asn Val
    130                 135                 140

Glu Ser Gly Gly Glu Phe Thr Ile Ser Ser Ala Glu Glu Ile Ile Lys
145                 150                 155                 160

Ala Leu

<210> 548
<211> 13
<212> PRT
<213> Glycine max

<400> 548

His Val Pro Gly Phe Ile Glu Arg Ala Glu Glu Leu Lys
1               5                   10

<210> 549
<211> 10
<212> PRT
<213> Glycine max

<400> 549

Arg Phe Ala Leu Leu Val Glu Asp Leu Lys
1               5                   10

<210> 550
<211> 20
<212> PRT
<213> Glycine max

<400> 550

Val Ala Asn Val Glu Ser Gly Gly Glu Phe Thr Ile Ser Ser Ala Glu
1               5                   10                  15

Glu Ile Ile Lys
            20

<210> 551
<211> 14
<212> PRT
<213> Glycine max

<400> 551

Tyr Thr Asn Ala Leu Gly Leu Glu Leu Asp Leu Thr Asp Lys
1               5                   10

<210> 552
<211> 456
<212> DNA
<213> Glycine max

<400> 552
atgtcgcagt tgctggataa agccaagaac ttcgtgtcgg agaaggtaaa tgatatggcc 60

aagccggagg cgagtgtcac tgacgtggac ttcaagcgcg tgagcaaaga taatgtcgag 120

tacttggcca aggtctctgt tcgtaaccct tattcaactt ccatacccat ttgtgagatc 180

aactactctt tcaaaagcgc atccagggag atagcatcag ggaaaattcc agacccagga 240

tcgttgaagg caaaggacac aacaatggtg gatgtgccag ttaaggttcc atacagcata 300

ttgatgagct tggcaaagga cattggtgct gactgggaca tagactatca attggatctt 360

ggtctggtta ttgatgttcc tgtcattggc atcttcacca ttcctctctc tcagaaagga 420

gagatcaagc taccaaccct ctctaccatg ttcgcc 456

<210> 553
<211> 152
<212> PRT
<213> Glycine max

<400> 553

Met Ser Gln Leu Leu Asp Lys Ala Lys Asn Phe Val Ser Glu Lys Val
1               5                   10                  15

Asn Asp Met Ala Lys Pro Glu Ala Ser Val Thr Asp Val Asp Phe Lys
            20                  25                  30

Arg Val Ser Lys Asp Asn Val Glu Tyr Leu Ala Lys Val Ser Val Arg
        35                  40                  45

Asn Pro Tyr Ser Thr Ser Ile Pro Ile Cys Glu Ile Asn Tyr Ser Phe
    50                  55                  60

Lys Ser Ala Ser Arg Glu Ile Ala Ser Gly Lys Ile Pro Asp Pro Gly
65                  70                  75                  80

Ser Leu Lys Ala Lys Asp Thr Thr Met Val Asp Val Pro Val Lys Val
                85                  90                  95

Pro Tyr Ser Ile Leu Met Ser Leu Ala Lys Asp Ile Gly Ala Asp Trp
            100                 105                 110

Asp Ile Asp Tyr Gln Leu Asp Leu Gly Leu Val Ile Asp Val Pro Val
        115                 120                 125

Ile Gly Ile Phe Thr Ile Pro Leu Ser Gln Lys Gly Glu Ile Lys Leu
    130                 135                 140

Pro Thr Leu Ser Thr Met Phe Ala
145                 150

<210> 554
<211> 8
<212> PRT
<213> Glycine max

<400>  554

Asp Asn Val Glu Tyr Leu Ala Lys
1               5


<210>  555
<211>  480
<212>  DNA
<213>  Glycine max

<400>  555
atgtcggacg aagagcacca tttcgagtcc aaggcagacg ccggtgcctc caaaacctac      60

cctcagcaag ccggtaccat tcgcaagaac ggctacatcg tcatcaaagg acgcccctgc      120

aaggttgtgg aagtttcgac ttccaaaact ggaaagcacg gtcacgccaa gtgccacttt      180

gttggaattg atattttcac tgccaagaaa cttgaggata ttgtgccctc ttctcacaac      240

tgtgatgttc ctcatgtgaa tcgtactgat taccagctca ttgatattgc tgaggatgga      300

tttttgagtc tgcttactga aaatgggaac accaaggatg acctcaagct tcccactgat      360

gagagtctgc tcactcagat aaaggatgga tttgctgagg gcaaggatct tgttgtgtct      420

gtcatgtctg ctatgggtga ggaacagatt tgcgccctga aggatattgg gccaaagaac      480


<210>  556
<211>  160
<212>  PRT
<213>  Glycine max

<400>  556

Met Ser Asp Glu Glu His His Phe Glu Ser Lys Ala Asp Ala Gly Ala
1               5                   10                  15


Ser Lys Thr Tyr Pro Gln Gln Ala Gly Thr Ile Arg Lys Asn Gly Tyr
            20                  25                  30


Ile Val Ile Lys Gly Arg Pro Cys Lys Val Val Glu Val Ser Thr Ser
        35                  40                  45


Lys Thr Gly Lys His Gly His Ala Lys Cys His Phe Val Gly Ile Asp
    50                  55                  60


Ile Phe Thr Ala Lys Lys Leu Glu Asp Ile Val Pro Ser Ser His Asn
65                  70                  75                  80


Cys Asp Val Pro His Val Asn Arg Thr Asp Tyr Gln Leu Ile Asp Ile
                85                  90                  95


Ala Glu Asp Gly Phe Leu Ser Leu Leu Thr Glu Asn Gly Asn Thr Lys
            100                 105                 110

Asp Asp Leu Lys Leu Pro Thr Asp Glu Ser Leu Leu Thr Gln Ile Lys
        115                 120                 125

Asp Gly Phe Ala Glu Gly Lys Asp Leu Val Val Ser Val Met Ser Ala
    130                 135                 140

Met Gly Glu Glu Gln Ile Cys Ala Leu Lys Asp Ile Gly Pro Lys Asn
145                 150                 155                 160

<210>  557
<211>  12
<212>  PRT
<213>  Glycine max

<400>  557

Leu Pro Thr Asp Glu Ser Leu Leu Thr Gln Ile Lys
1               5                   10

<210>  558
<211>  7
<212>  PRT
<213>  Glycine max

<400>  558

Asn Gly Tyr Ile Val Ile Lys
1               5

<210>  559
<211>  480
<212>  DNA
<213>  Glycine max

<400>  559
   atgtcggacg aagagcatca cttcgattct caggcggatg cgggagcgtc caagacgtat      60
   cctcagcagg ctggtaccat tcgcaagaat ggctacatcg tcatcaaatc caggccttgc     120
   aaggttgtag aggtctctac gtcaaagaca ggcaaacatg gacatgcaaa gtgtcacttt     180
   gctgcaattg atatttttaa tgggaaaaag cttgaagata ttgttccatc atcccacaac     240
   tgtgatgttc cacatgtgaa tcgcactgac tatcagctta ttgatatctc tgaagacggc     300
   tttgtgagtc tgttgactga taatggaaat accaaggatg atctgaagct tcctactgat     360
   gaggcattgc ttgctcagat taaagatgga ttttctgaag gaaaagacct tgtggtttct     420
   gttatgtctg caatgggaga agaacagatc tgttcactga aggacatcgg tcccaaaaat     480

<210>  560
<211>  160
<212>  PRT
<213>  Glycine max

<400>  560

Met Ser Asp Glu Glu His His Phe Asp Ser Gln Ala Asp Ala Gly Ala
1               5                   10                  15

Ser Lys Thr Tyr Pro Gln Gln Ala Gly Thr Ile Arg Lys Asn Gly Tyr
            20                  25                  30

Ile Val Ile Lys Ser Arg Pro Cys Lys Val Val Glu Val Ser Thr Ser
        35                  40                  45

Lys Thr Gly Lys His Gly His Ala Lys Cys His Phe Ala Ala Ile Asp
    50                  55                  60

Ile Phe Asn Gly Lys Lys Leu Glu Asp Ile Val Pro Ser Ser His Asn
65                  70                  75                  80

Cys Asp Val Pro His Val Asn Arg Thr Asp Tyr Gln Leu Ile Asp Ile
                85                  90                  95

Ser Glu Asp Gly Phe Val Ser Leu Leu Thr Asp Asn Gly Asn Thr Lys
            100                 105                 110

Asp Asp Leu Lys Leu Pro Thr Asp Glu Ala Leu Leu Ala Gln Ile Lys
        115                 120                 125

Asp Gly Phe Ser Glu Gly Lys Asp Leu Val Val Ser Val Met Ser Ala
    130                 135                 140

Met Gly Glu Glu Gln Ile Cys Ser Leu Lys Asp Ile Gly Pro Lys Asn
145                 150                 155                 160


<210>  561
<211>  7
<212>  PRT
<213>  Glycine max

<400>  561

Asn Gly Tyr Ile Val Ile Lys
1               5


<210>  562
<211>  11
<212>  PRT
<213>  Glycine max

<400>  562

Thr Tyr Pro Gln Gln Ala Gly Thr Ile Arg Lys
1               5                   10

<210>  563
<211>  8
<212>  PRT
<213>  Glycine max

<400>  563

Val Val Glu Val Ser Thr Ser Lys
1               5


<210>  564
<211>  501
<212>  DNA
<213>  Glycine max

<400>  564
atggctagcc aatcaaacac taaatcagtt cacgatttca cagttaaaga tgccagggga      60

aataatgtta atctcgccga ttacaaagga aaggtccttc tcattgtcaa tgttgcctca     120

caatgcggct tgactaattc aaactacaca gagcttaacc aattgtatga gaaatacaaa     180

gggaaaggac ttgaaattct ggcattccca tgcaatcagt ttggggctca ggagccagga     240

actaatgaag agattgtgga gtttgcttgt actcgcttca aagctgagtt tcccattttc     300

gacaaggtgg atgtgaatgg tgacaatgct gctccactgt acaagtttct gaaatcaagc     360

aaaggtggac tctttgggga tagtatcaaa tggaacttct ccaaattcct tgttgataag     420

gatgggaatg tcgttgaccg ttatgcaccc acaacttctc ctctcagcat tgagaaagac     480

ataaagaagc tgctagatgc a                                               501


<210>  565
<211>  167
<212>  PRT
<213>  Glycine max

<400>  565

Met Ala Ser Gln Ser Asn Thr Lys Ser Val His Asp Phe Thr Val Lys
1               5                   10                  15


Asp Ala Arg Gly Asn Asn Val Asn Leu Ala Asp Tyr Lys Gly Lys Val
            20                  25                  30


Leu Leu Ile Val Asn Val Ala Ser Gln Cys Gly Leu Thr Asn Ser Asn
        35                  40                  45


Tyr Thr Glu Leu Asn Gln Leu Tyr Glu Lys Tyr Lys Gly Lys Gly Leu
    50                  55                  60


Glu Ile Leu Ala Phe Pro Cys Asn Gln Phe Gly Ala Gln Glu Pro Gly
65                  70                  75                  80

Thr Asn Glu Glu Ile Val Glu Phe Ala Cys Thr Arg Phe Lys Ala Glu
                85                  90                  95

Phe Pro Ile Phe Asp Lys Val Asp Val Asn Gly Asp Asn Ala Ala Pro
            100                 105                 110

Leu Tyr Lys Phe Leu Lys Ser Ser Lys Gly Gly Leu Phe Gly Asp Ser
        115                 120                 125

Ile Lys Trp Asn Phe Ser Lys Phe Leu Val Asp Lys Asp Gly Asn Val
    130                 135                 140

Val Asp Arg Tyr Ala Pro Thr Thr Ser Pro Leu Ser Ile Glu Lys Asp
145                 150                 155                 160

Ile Lys Lys Leu Leu Asp Ala
                165

<210>  566
<211>  8
<212>  PRT
<213>  Glycine max

<400>  566

Ala Glu Phe Pro Ile Phe Asp Lys
1               5

<210>  567
<211>  13
<212>  PRT
<213>  Glycine max

<400>  567

Asp Ala Arg Gly Asn Asn Val Asn Leu Ala Asp Tyr Lys
1               5                   10

<210>  568
<211>  19
<212>  PRT
<213>  Glycine max

<400>  568

Asp Gly Asn Val Val Asp Arg Tyr Ala Pro Thr Thr Ser Pro Leu Ser
1               5                   10                  15

Ile Glu Lys

<210>  569
<211>  9
<212>  PRT
<213>  Glycine max

<400>  569

Gly Gly Leu Phe Gly Asp Ser Ile Lys
1               5


<210>  570
<211>  13
<212>  PRT
<213>  Glycine max

<400>  570

Val Asp Val Asn Gly Asp Asn Ala Ala Pro Leu Tyr Lys
1               5                   10


<210>  571
<211>  357
<212>  DNA
<213>  Glycine max

<400>  571
atggcttccg aagaatccca gaacaagccc cccagcgagc agtccactac cgaactcatc        60

acgagcgcga agctggtggc ggaggcggcg cagtcggctc tgaagagcga cagcgacaaa       120

gtggacaagg ccaaggtggc tgacgctgcc ggcgatcttc tcgacgcggc ggggaagtat       180

ggaaaacttg atgacaaaca aggcataggg caatatgttg acaaggctgc tgattatctg       240

cataattacc agggtgataa taccactgct ccttcacagc ctgcagagtc taaaggtgaa       300

gaaagtggtg gtgggttagg tggtcttgca aacttggctg gaggcttctt caaaaag          357


<210>  572
<211>  119
<212>  PRT
<213>  Glycine max

<400>  572

Met Ala Ser Glu Glu Ser Gln Asn Lys Pro Pro Ser Glu Gln Ser Thr
1               5                   10                  15


Thr Glu Leu Ile Thr Ser Ala Lys Leu Val Ala Glu Ala Ala Gln Ser
            20                  25                  30


Ala Leu Lys Ser Asp Ser Asp Lys Val Asp Lys Ala Lys Val Ala Asp
        35                  40                  45


Ala Ala Gly Asp Leu Leu Asp Ala Ala Gly Lys Tyr Gly Lys Leu Asp
    50                  55                  60

Asp Lys Gln Gly Ile Gly Gln Tyr Val Asp Lys Ala Ala Asp Tyr Leu
65                  70                  75                  80

His Asn Tyr Gln Gly Asp Asn Thr Thr Ala Pro Ser Gln Pro Ala Glu
                85                  90                  95

Ser Lys Gly Glu Glu Ser Gly Gly Gly Leu Gly Gly Leu Ala Asn Leu
            100                 105                 110

Ala Gly Gly Phe Phe Lys Lys
        115

<210>  573
<211>  23
<212>  PRT
<213>  Glycine max

<400>  573

Ala Ala Asp Tyr Leu His Asn Tyr Gln Gly Asp Asn Thr Thr Ala Pro
1               5                   10                  15

Ser Gln Pro Ala Glu Ser Lys
            20

<210>  574
<211>  11
<212>  PRT
<213>  Glycine max

<400>  574

Leu Val Ala Glu Ala Ala Gln Ser Ala Leu Lys
1               5                   10

<210>  575
<211>  9
<212>  PRT
<213>  Glycine max

<400>  575

Gln Gly Ile Gly Gln Tyr Val Asp Lys
1               5

<210>  576
<211>  14
<212>  PRT
<213>  Glycine max

<400>  576

Val Ala Asp Ala Ala Gly Asp Leu Leu Asp Ala Ala Gly Lys
1               5                   10

<210>  577
<211>  777
<212>  DNA
<213>  Glycine max

<400>  577
atggcggccg ctccctcgcc tcgcgaagag aacgtctaca tggcgaagct cgccgagcag      60

gccgagcgct acgaagagat ggtggagttc atggagaagg tctccgccgc cgccgacaac     120

gaggaactta ccgtggagga gaggaacctc ctctccgtcg cctacaagaa cgtgatcgga     180

gcgcgccgcg cctcgtggcg catcatctcc tcgatcgagc agaaggagga gagccgcggc     240

aacgaggatc acgtctccgt catccgcgac taccgatcca agatcgagtc cgagctctcc     300

aacatctgcg acggtatcct caagctcctc gactctcgcc tcattccatc cgcttcctcc     360

ggcgattcca aggtcttcta cctcaagatg aagggagatt accacagata ccttgccgag     420

ttcaagaccg gcgccgagcg taaggaggct gccgagagca ctctctctgc atacaaagcc     480

gcacaggaca ttgcgaatgc cgaactgcct cctactcatc caattaggct tggccttgct     540

cttaactttt ctgtatttta ctacgaaatc cttaattctc cggatcgtgc ctgcaacctt     600

gcaaaacagg cttttgatga agctattgct gaattggata cactgggaga ggagtcatac     660

aaggatagca ctttgatcat gcaactcctt cgtgataacc tcacactttg gacctctgac     720

atgcaggacg atggagcaga tgaaattaaa gaagcagcac cgaaaccgga tgaccag        777

<210>  578
<211>  259
<212>  PRT
<213>  Glycine max

<400>  578

Met Ala Ala Ala Pro Ser Pro Arg Glu Glu Asn Val Tyr Met Ala Lys
1               5                   10                  15

Leu Ala Glu Gln Ala Glu Arg Tyr Glu Glu Met Val Glu Phe Met Glu
            20                  25                  30

Lys Val Ser Ala Ala Ala Asp Asn Glu Glu Leu Thr Val Glu Glu Arg
        35                  40                  45

Asn Leu Leu Ser Val Ala Tyr Lys Asn Val Ile Gly Ala Arg Arg Ala
    50                  55                  60

Ser Trp Arg Ile Ile Ser Ser Ile Glu Gln Lys Glu Glu Ser Arg Gly
65                  70                  75                  80

Asn Glu Asp His Val Ser Val Ile Arg Asp Tyr Arg Ser Lys Ile Glu

85                  90                  95

Ser Glu Leu Ser Asn Ile Cys Asp Gly Ile Leu Lys Leu Leu Asp Ser
            100                 105                 110

Arg Leu Ile Pro Ser Ala Ser Ser Gly Asp Ser Lys Val Phe Tyr Leu
        115                 120                 125

Lys Met Lys Gly Asp Tyr His Arg Tyr Leu Ala Glu Phe Lys Thr Gly
    130                 135                 140

Ala Glu Arg Lys Glu Ala Ala Glu Ser Thr Leu Ser Ala Tyr Lys Ala
145                 150                 155                 160

Ala Gln Asp Ile Ala Asn Ala Glu Leu Pro Pro Thr His Pro Ile Arg
                165                 170                 175

Leu Gly Leu Ala Leu Asn Phe Ser Val Phe Tyr Tyr Glu Ile Leu Asn
            180                 185                 190

Ser Pro Asp Arg Ala Cys Asn Leu Ala Lys Gln Ala Phe Asp Glu Ala
        195                 200                 205

Ile Ala Glu Leu Asp Thr Leu Gly Glu Glu Ser Tyr Lys Asp Ser Thr
    210                 215                 220

Leu Ile Met Gln Leu Leu Arg Asp Asn Leu Thr Leu Trp Thr Ser Asp
225                 230                 235                 240

Met Gln Asp Asp Gly Ala Asp Glu Ile Lys Glu Ala Ala Pro Lys Pro
                245                 250                 255

Asp Asp Gln

<210>  579
<211>  11
<212>  PRT
<213>  Glycine max

<400>  579

Glu Ala Ala Glu Ser Thr Leu Ser Ala Tyr Lys
1               5                   10

<210>  580
<211>  16
<212>  PRT
<213>  Glycine max

<400>  580

Leu Leu Asp Ser Arg Leu Ile Pro Ser Ala Ser Ser Gly Asp Ser Lys
1               5                   10                  15


<210>  581
<211>  19
<212>  PRT
<213>  Glycine max

<400>  581

Gln Ala Phe Asp Glu Ala Ile Ala Glu Leu Asp Thr Leu Gly Glu Glu
1               5                   10                  15


Ser Tyr Lys


<210>  582
<211>  23
<212>  PRT
<213>  Glycine max

<400>  582

Val Ser Ala Ala Ala Asp Asn Glu Glu Leu Thr Val Glu Glu Arg Asn
1               5                   10                  15


Leu Leu Ser Val Ala Tyr Lys
            20


<210>  583
<211>  993
<212>  DNA
<213>  Glycine max

<400>  583
atggcgttga acaatggcgt ccccgccacc ggcaccggcc tcatcgtcag cttcggtgag      60

atgctcatcg acttcgtccc caccgtctct ggcgtgtccc tggccgaggc ccctggcttc     120

ctcaaggccc ccggcggcgc ccccgctaac gtcgccatcg ccgtgtcgcg cctcggcggc     180

aaagccgcct tcgtcggcaa gctcggcgac gacgagttcg gccacatgct cgccggaatc     240

ctcaaggaaa acggcgttcg cgccgacggc atcaactttg accagggcgc acgcaccgcc     300

ctggccttcg tgaccctacg cgccgacggg gagcgtgagt tcatgttcta cagaaacccc     360

agcgccgaca tgctcctcaa gcccgaagaa ctcaatctcg aactcatcag atctgcaaaa     420

gttttccatt acggatcaat cagtttgatc gtggagccat gcagatcagc acacttgaag     480

gcaatggaag tagccaagga atctgggtgc ttgctctcct atgaccccaa ccttcgtcta     540

cctttgtggc cttcggctga ggaagctcgt aagcaaatac tgagcatttg ggagaaggct     600

gatttgatca aggtcagtga tgcggagctt gagttcctca caggaagtga caagattgat     660

gatgaatctg ctttgtcatt gtggcacccc aatttgaagt tgctccttgt cactcttggg 720

gaacatggtt ccagatacta caccaagagt ttcaaaggat cggtagatgc tttccatgtc 780

aatacagttg atacaactgg tgccggtgat tcctttgttg gtgctttatt ggccaagatt 840

gtcgatgatc agtccatact tgaagatgaa ccaaggttaa gagaagtact aaagtttgca 900

aatgcatgtg gagctattac aactacccaa aagggagcaa ttccggccct tcccaaagag 960

gaggctgcac tgaaactgat caaagggggg tca 993

<210> 584
<211> 331
<212> PRT
<213> Glycine max

<400> 584

Met Ala Leu Asn Asn Gly Val Pro Ala Thr Gly Thr Gly Leu Ile Val
1               5                   10                  15

Ser Phe Gly Glu Met Leu Ile Asp Phe Val Pro Thr Val Ser Gly Val
            20                  25                  30

Ser Leu Ala Glu Ala Pro Gly Phe Leu Lys Ala Pro Gly Gly Ala Pro
        35                  40                  45

Ala Asn Val Ala Ile Ala Val Ser Arg Leu Gly Gly Lys Ala Ala Phe
    50                  55                  60

Val Gly Lys Leu Gly Asp Asp Glu Phe Gly His Met Leu Ala Gly Ile
65                  70                  75                  80

Leu Lys Glu Asn Gly Val Arg Ala Asp Gly Ile Asn Phe Asp Gln Gly
                85                  90                  95

Ala Arg Thr Ala Leu Ala Phe Val Thr Leu Arg Ala Asp Gly Glu Arg
            100                 105                 110

Glu Phe Met Phe Tyr Arg Asn Pro Ser Ala Asp Met Leu Leu Lys Pro
        115                 120                 125

Glu Glu Leu Asn Leu Glu Leu Ile Arg Ser Ala Lys Val Phe His Tyr
    130                 135                 140

Gly Ser Ile Ser Leu Ile Val Glu Pro Cys Arg Ser Ala His Leu Lys
145                 150                 155                 160

Ala Met Glu Val Ala Lys Glu Ser Gly Cys Leu Leu Ser Tyr Asp Pro
                165                 170                 175

Asn Leu Arg Leu Pro Leu Trp Pro Ser Ala Glu Glu Ala Arg Lys Gln
            180                 185                 190

Ile Leu Ser Ile Trp Glu Lys Ala Asp Leu Ile Lys Val Ser Asp Ala
        195                 200                 205

Glu Leu Glu Phe Leu Thr Gly Ser Asp Lys Ile Asp Asp Glu Ser Ala
    210                 215                 220

Leu Ser Leu Trp His Pro Asn Leu Lys Leu Leu Leu Val Thr Leu Gly
225                 230                 235                 240

Glu His Gly Ser Arg Tyr Tyr Thr Lys Ser Phe Lys Gly Ser Val Asp
                245                 250                 255

Ala Phe His Val Asn Thr Val Asp Thr Thr Gly Ala Gly Asp Ser Phe
            260                 265                 270

Val Gly Ala Leu Leu Ala Lys Ile Val Asp Asp Gln Ser Ile Leu Glu
        275                 280                 285

Asp Glu Pro Arg Leu Arg Glu Val Leu Lys Phe Ala Asn Ala Cys Gly
    290                 295                 300

Ala Ile Thr Thr Thr Gln Lys Gly Ala Ile Pro Ala Leu Pro Lys Glu
305                 310                 315                 320

Glu Ala Ala Leu Lys Leu Ile Lys Gly Gly Ser
                325                 330

<210> 585
<211> 19
<212> PRT
<213> Glycine max

<400> 585

Ala Pro Gly Gly Ala Pro Ala Asn Val Ala Ile Ala Val Ser Arg Leu
1               5                   10                  15

Gly Gly Lys

<210> 586
<211> 27
<212> PRT
<213> Glycine max

<400> 586

Gly Ser Val Asp Ala Phe His Val Asn Thr Val Asp Thr Thr Gly Ala
1               5                   10                  15

Gly Asp Ser Phe Val Gly Ala Leu Leu Ala Lys
            20                  25

<210>  587
<211>  19
<212>  PRT
<213>  Glycine max

<400>  587

Ile Val Asp Asp Gln Ser Ile Leu Glu Asp Glu Pro Arg Leu Arg Glu
1               5                   10                  15

Val Leu Lys

<210>  588
<211>  14
<212>  PRT
<213>  Glycine max

<400>  588

Val Ser Asp Ala Glu Leu Glu Phe Leu Thr Gly Ser Asp Lys
1               5                   10

<210>  589
<211>  759
<212>  DNA
<213>  Glycine max

<400>  589
atgggcagaa agttcttcgt tggtggcaac tggaaatgca atgggaccac tgaggaggtg      60

aagaagattg ttactacttt aaatgaagct aaagtccctg gagaagatgt tgtagaagtt     120

gttgtgagcc ctccttttgt gttccttcct tttgtaaaaa gtttgctgcg ccctgatttc     180

catgtctcgg cccaaaattg ttgggttcgc aaaggtggtg cttatactgg agaggttagt     240

gctgaaatgc ttgttaattt gggaattcct tgggttatta ttggtcactc tgaacggagg     300

cagcttttga atgaatcaaa tgagtttgtg ggagataaag ttgcctatgc acttcaacaa     360

ggtctgaaag ttatagcatg cattggggaa actcttgaac agcgtgaagc tggtacaaca     420

acggctgttg ttgctgagca aacaaaagca attgcagcta aaatatcaaa ttgggacaat     480

gtcgttttgg cctatgagcc agtttgggcc attggaacag gaaaggttgc aactcctgct     540

caggctcaag aggttcatgc tgatttaagg aaatgggttc atgacaatgt gagtgctgaa     600

gttgctgcat ctgtaagaat tatctatgga ggctctgtaa atggaggaaa ctgcaaagaa     660

ttggcagcac agcccgatgt tgatggattt ttggttggtg gtgcatccct caaggcggaa 720

tttgtggaca tcataaacgc tgccactgtg aagaagaat 759

<210> 590
<211> 253
<212> PRT
<213> Glycine max

<400> 590

Met Gly Arg Lys Phe Phe Val Gly Gly Asn Trp Lys Cys Asn Gly Thr
1               5                   10                  15

Thr Glu Glu Val Lys Lys Ile Val Thr Thr Leu Asn Glu Ala Lys Val
            20                  25                  30

Pro Gly Glu Asp Val Val Glu Val Val Val Ser Pro Pro Phe Val Phe
        35                  40                  45

Leu Pro Phe Val Lys Ser Leu Leu Arg Pro Asp Phe His Val Ser Ala
    50                  55                  60

Gln Asn Cys Trp Val Arg Lys Gly Gly Ala Tyr Thr Gly Glu Val Ser
65                  70                  75                  80

Ala Glu Met Leu Val Asn Leu Gly Ile Pro Trp Val Ile Ile Gly His
                85                  90                  95

Ser Glu Arg Arg Gln Leu Leu Asn Glu Ser Asn Glu Phe Val Gly Asp
            100                 105                 110

Lys Val Ala Tyr Ala Leu Gln Gln Gly Leu Lys Val Ile Ala Cys Ile
        115                 120                 125

Gly Glu Thr Leu Glu Gln Arg Glu Ala Gly Thr Thr Thr Ala Val Val
    130                 135                 140

Ala Glu Gln Thr Lys Ala Ile Ala Ala Lys Ile Ser Asn Trp Asp Asn
145                 150                 155                 160

Val Val Leu Ala Tyr Glu Pro Val Trp Ala Ile Gly Thr Gly Lys Val
                165                 170                 175

Ala Thr Pro Ala Gln Ala Gln Glu Val His Ala Asp Leu Arg Lys Trp
            180                 185                 190

Val His Asp Asn Val Ser Ala Glu Val Ala Ala Ser Val Arg Ile Ile
        195                 200                 205

Tyr Gly Gly Ser Val Asn Gly Gly Asn Cys Lys Glu Leu Ala Ala Gln
    210                 215                 220

Pro Asp Val Asp Gly Phe Leu Val Gly Gly Ala Ser Leu Lys Ala Glu
225                 230                 235                 240

Phe Val Asp Ile Ile Asn Ala Ala Thr Val Lys Lys Asn
                245                 250

<210>  591
<211>  13
<212>  PRT
<213>  Glycine max

<400>  591

Ala Glu Phe Val Asp Ile Ile Asn Ala Ala Thr Val Lys
1               5                   10

<210>  592
<211>  19
<212>  PRT
<213>  Glycine max

<400>  592

Glu Leu Ala Ala Gln Pro Asp Val Asp Gly Phe Leu Val Gly Gly Ala
1               5                   10                  15

Ser Leu Lys

<210>  593
<211>  8
<212>  PRT
<213>  Glycine max

<400>  593

Phe Phe Val Gly Gly Asn Trp Lys
1               5

<210>  594
<211>  9
<212>  PRT
<213>  Glycine max

<400>  594

Ile Val Thr Thr Leu Asn Glu Ala Lys
1               5

<210>  595
<211>  16

<212>  PRT
<213>  Glycine max

<400>  595

Val Ala Thr Pro Ala Gln Ala Gln Glu Val His Ala Asp Leu Arg Lys
1               5                   10                  15


<210>  596
<211>  10
<212>  PRT
<213>  Glycine max

<400>  596

Val Ala Tyr Ala Leu Gln Gln Gly Leu Lys
1               5                   10


<210>  597
<211>  22
<212>  PRT
<213>  Glycine max

<400>  597

Val Pro Gly Glu Asp Val Val Glu Val Val Val Ser Pro Pro Phe Val
1               5                   10                  15


Phe Leu Pro Phe Val Lys
            20


<210>  598
<211>  759
<212>  DNA
<213>  Glycine max

<400>  598
atgggcagaa aattcttcgt tggtggcaac tggaaatgca atgggaccac tgaggaggta      60

aagaagattg ttactacttt gaatgaggct aaagtccctg gagaagatgt cgtagaagtt     120

gttgtgagcc ctccttttgt gttccttcct gttgtaaaaa gtttgctgcg ccctgatttc     180

catgtttcgg cacaaaactg ttgggttcgc aaaggtggtg cttataccgg tgaggttagt     240

gctgaaatgc ttgttaattt gggaattcct tgggttatta ttggtcactc tgaacggagg     300

cagcttttaa atgaatcaaa tgagtttgtg ggagataaag ttgcctatgc acttcaacaa     360

ggtctaaaag ttattgcatg cattggggag actctcgaac agcgtgaagc tggtacaaca     420

acggctgttg tttctgagca aacaaaagca attgcagcta aaatatcaaa ttgggacaat     480

gtcgttttgg cctacgagcc agtttgggcc attggaacag gaaaggttgc tactcctgct     540

caggctcaag aggtccatgc tgatttgagg aaatgggttc atgacaatgt gagtgctgaa     600

gttgctgcat ctgtaagaat tatctatgga ggttctgtaa atggaggaaa ctgcaaagaa     660

ttggccgcac agcccgatgt tgatggattt ttggttggtg gtgcctccct gaagccggag 720

ttcgtggaca tcataaatgc tgccactgtg aagaagaat 759

<210> 599
<211> 253
<212> PRT
<213> Glycine max

<400> 599

Met Gly Arg Lys Phe Phe Val Gly Gly Asn Trp Lys Cys Asn Gly Thr
1               5                   10                  15

Thr Glu Glu Val Lys Lys Ile Val Thr Thr Leu Asn Glu Ala Lys Val
            20                  25                  30

Pro Gly Glu Asp Val Val Glu Val Val Val Ser Pro Pro Phe Val Phe
        35                  40                  45

Leu Pro Val Val Lys Ser Leu Leu Arg Pro Asp Phe His Val Ser Ala
    50                  55                  60

Gln Asn Cys Trp Val Arg Lys Gly Gly Ala Tyr Thr Gly Glu Val Ser
65                  70                  75                  80

Ala Glu Met Leu Val Asn Leu Gly Ile Pro Trp Val Ile Ile Gly His
                85                  90                  95

Ser Glu Arg Arg Gln Leu Leu Asn Glu Ser Asn Glu Phe Val Gly Asp
            100                 105                 110

Lys Val Ala Tyr Ala Leu Gln Gln Gly Leu Lys Val Ile Ala Cys Ile
        115                 120                 125

Gly Glu Thr Leu Glu Gln Arg Glu Ala Gly Thr Thr Thr Ala Val Val
    130                 135                 140

Ser Glu Gln Thr Lys Ala Ile Ala Ala Lys Ile Ser Asn Trp Asp Asn
145                 150                 155                 160

Val Val Leu Ala Tyr Glu Pro Val Trp Ala Ile Gly Thr Gly Lys Val
                165                 170                 175

Ala Thr Pro Ala Gln Ala Gln Glu Val His Ala Asp Leu Arg Lys Trp
            180                 185                 190

Val His Asp Asn Val Ser Ala Glu Val Ala Ala Ser Val Arg Ile Ile
        195                 200                 205

Tyr Gly Gly Ser Val Asn Gly Gly Asn Cys Lys Glu Leu Ala Ala Gln
    210                 215                 220

Pro Asp Val Asp Gly Phe Leu Val Gly Gly Ala Ser Leu Lys Pro Glu
225                 230                 235                 240

Phe Val Asp Ile Ile Asn Ala Ala Thr Val Lys Lys Asn
                245                 250

<210>  600
<211>  32
<212>  PRT
<213>  Glycine max

<400>  600

Glu Leu Ala Ala Gln Pro Asp Val Asp Gly Phe Leu Val Gly Gly Ala
1               5                   10                  15

Ser Leu Lys Pro Glu Phe Val Asp Ile Ile Asn Ala Ala Thr Val Lys
            20                  25                  30

<210>  601
<211>  8
<212>  PRT
<213>  Glycine max

<400>  601

Phe Phe Val Gly Gly Asn Trp Lys
1               5

<210>  602
<211>  9
<212>  PRT
<213>  Glycine max

<400>  602

Ile Val Thr Thr Leu Asn Glu Ala Lys
1               5

<210>  603
<211>  16
<212>  PRT
<213>  Glycine max

<400>  603

Val Ala Thr Pro Ala Gln Ala Gln Glu Val His Ala Asp Leu Arg Lys
1               5                   10                  15

<210>  604
<211>  10

<212>  PRT
<213>  Glycine max

<400>  604

Val Ala Tyr Ala Leu Gln Gln Gly Leu Lys
1               5                   10


<210>  605
<211>  765
<212>  DNA
<213>  Glycine max

<400>  605
atggcctcat tgggtgggtt acaaaatgtg agcggcatca attttcttat caaagagaaa      60

ggatgttgta tgctgcaggg tccaaaagtc aatgcaaagt tcgagctgaa gccgccacca     120

tatccactga atggtttgga gccggtgatg agccagcaga cacttgagtt tcactggggg     180

aagcaccaca agacttatgt ggaaaatctg aaaaaacaag ttgttgggac agagcttgat     240

gggaagtcac tagaagagat tattgtcaca tcatacaata agggtgacat tcttccagct     300

ttcaacaatg cagcacaggt atggaaccat gacttcttct gggagtgcat gaaaccaggt     360

ggaggtggaa agccatccgg ggagcttcta gaactgattg aaagagactt tggttcattt     420

gtaaaattcc ttgatgagtt caaggctgct gctgcaacac aatttggttc agggtgggct     480

tggctagcat atagagcaag aaaatttgat ggggaaaatg tagcaaatcc tccttcaccc     540

gatgaggaca acaagctagt ggtgctcaag agtcccaatg ctgtgaaccc ccttgtttgg     600

ggaggttact acccacttct taccattgat gtttgggagc atgcttacta ccttgatttt     660

cagaaccggc gtcctgatta tatatcagtg ttcatggata agcttgtttc ctgggatgca     720

gtgagctcta gacttgaaca agctaaggct ttaattacca gtgca                     765


<210>  606
<211>  255
<212>  PRT
<213>  Glycine max

<400>  606

Met Ala Ser Leu Gly Gly Leu Gln Asn Val Ser Gly Ile Asn Phe Leu
1               5                   10                  15


Ile Lys Glu Lys Gly Cys Cys Met Leu Gln Gly Pro Lys Val Asn Ala
            20                  25                  30


Lys Phe Glu Leu Lys Pro Pro Pro Tyr Pro Leu Asn Gly Leu Glu Pro
        35                  40                  45


Val Met Ser Gln Gln Thr Leu Glu Phe His Trp Gly Lys His His Lys
    50                  55                  60

Thr Tyr Val Glu Asn Leu Lys Lys Gln Val Val Gly Thr Glu Leu Asp
65                  70                  75                  80

Gly Lys Ser Leu Glu Glu Ile Ile Val Thr Ser Tyr Asn Lys Gly Asp
                85                  90                  95

Ile Leu Pro Ala Phe Asn Asn Ala Ala Gln Val Trp Asn His Asp Phe
            100                 105                 110

Phe Trp Glu Cys Met Lys Pro Gly Gly Gly Gly Lys Pro Ser Gly Glu
        115                 120                 125

Leu Leu Glu Leu Ile Glu Arg Asp Phe Gly Ser Phe Val Lys Phe Leu
    130                 135                 140

Asp Glu Phe Lys Ala Ala Ala Ala Thr Gln Phe Gly Ser Gly Trp Ala
145                 150                 155                 160

Trp Leu Ala Tyr Arg Ala Arg Lys Phe Asp Gly Glu Asn Val Ala Asn
                165                 170                 175

Pro Pro Ser Pro Asp Glu Asp Asn Lys Leu Val Val Leu Lys Ser Pro
            180                 185                 190

Asn Ala Val Asn Pro Leu Val Trp Gly Gly Tyr Tyr Pro Leu Leu Thr
        195                 200                 205

Ile Asp Val Trp Glu His Ala Tyr Tyr Leu Asp Phe Gln Asn Arg Arg
    210                 215                 220

Pro Asp Tyr Ile Ser Val Phe Met Asp Lys Leu Val Ser Trp Asp Ala
225                 230                 235                 240

Val Ser Ser Arg Leu Glu Gln Ala Lys Ala Leu Ile Thr Ser Ala
                245                 250                 255

<210>  607
<211>  17
<212>  PRT
<213>  Glycine max

<400>  607

Phe Asp Gly Glu Asn Val Ala Asn Pro Pro Ser Pro Asp Glu Asp Asn
1               5                   10                  15

Lys

<210>  608
<211>  15
<212>  PRT
<213>  Glycine max

<400>  608

Leu Val Ser Trp Asp Ala Val Ser Ser Arg Leu Glu Gln Ala Lys
1               5                   10                  15


<210>  609
<211>  10
<212>  PRT
<213>  Glycine max

<400>  609

Gln Val Val Gly Thr Glu Leu Asp Gly Lys
1               5                   10


<210>  610
<211>  12
<212>  PRT
<213>  Glycine max

<400>  610

Ser Leu Glu Glu Ile Ile Val Thr Ser Tyr Asn Lys
1               5                   10


<210>  611
<211>  7
<212>  PRT
<213>  Glycine max

<400>  611

Thr Tyr Val Glu Asn Leu Lys
1               5


<210>  612
<211>  732
<212>  DNA
<213>  Glycine max

<400>  612
atggcctcat tgggtgggtt acaaaatcta agcggcatca attttcttat caaaaaagag      60

ggtccaaaag tcaatgcgaa gttcgagctg aagccgccac catatccact gaatggtttg     120

gagccggtga tgagccagca gacacttgag tttcattggg ggaaacacca caagacatat     180

gtggaaaatc tgaaaaaaca aattgttggg acagagcttg atgggaaatc actagaagag     240

attatagtga catcatataa taagggtgac attcttccag ctttcaacaa tgcagcacag     300

gtatggaacc atgacttctt ctgggagtgc atgaaaccag gtggaggtgg aaagccatcc     360

ggggagcttc tagaactgat tgaaagagac tttggctcat ttgaaaaatt ccttgatgag 420

tttaaggctg ctgctgcaac acaatttggt tcaggatggg cttggcttgc atatagagca 480

agcaaatttg atggggaaaa tgcagcaaat cctccttcac cagatgagga caacaagcta 540

gtggtgctca agagtcccaa tgctgtgaac ccccttgttt ggggaggcta ttatccactt 600

cttaccattg atgtttggga gcatgcttac aaccttgatt ttcagaaccg gcgccctgat 660

tatatatcag tgttcatgga taagcttgtt tcctgggatg cagtgagttc tagacttgaa 720

caagcaaggc tt 732

<210> 613
<211> 244
<212> PRT
<213> Glycine max

<400> 613

Met Ala Ser Leu Gly Gly Leu Gln Asn Leu Ser Gly Ile Asn Phe Leu
1               5                   10                  15

Ile Lys Lys Glu Gly Pro Lys Val Asn Ala Lys Phe Glu Leu Lys Pro
            20                  25                  30

Pro Pro Tyr Pro Leu Asn Gly Leu Glu Pro Val Met Ser Gln Gln Thr
        35                  40                  45

Leu Glu Phe His Trp Gly Lys His His Lys Thr Tyr Val Glu Asn Leu
    50                  55                  60

Lys Lys Gln Ile Val Gly Thr Glu Leu Asp Gly Lys Ser Leu Glu Glu
65                  70                  75                  80

Ile Ile Val Thr Ser Tyr Asn Lys Gly Asp Ile Leu Pro Ala Phe Asn
                85                  90                  95

Asn Ala Ala Gln Val Trp Asn His Asp Phe Phe Trp Glu Cys Met Lys
            100                 105                 110

Pro Gly Gly Gly Gly Lys Pro Ser Gly Glu Leu Leu Glu Leu Ile Glu
        115                 120                 125

Arg Asp Phe Gly Ser Phe Glu Lys Phe Leu Asp Glu Phe Lys Ala Ala
    130                 135                 140

Ala Ala Thr Gln Phe Gly Ser Gly Trp Ala Trp Leu Ala Tyr Arg Ala
145                 150                 155                 160

Ser Lys Phe Asp Gly Glu Asn Ala Ala Asn Pro Pro Ser Pro Asp Glu
                165                 170                 175

Asp Asn Lys Leu Val Val Leu Lys Ser Pro Asn Ala Val Asn Pro Leu
            180                 185                 190

Val Trp Gly Gly Tyr Tyr Pro Leu Leu Thr Ile Asp Val Trp Glu His
        195                 200                 205

Ala Tyr Asn Leu Asp Phe Gln Asn Arg Arg Pro Asp Tyr Ile Ser Val
    210                 215                 220

Phe Met Asp Lys Leu Val Ser Trp Asp Ala Val Ser Ser Arg Leu Glu
225                 230                 235                 240

Gln Ala Arg Leu

<210>  614
<211>  10
<212>  PRT
<213>  Glycine max

<400>  614

Gln Ile Val Gly Thr Glu Leu Asp Gly Lys
1               5                   10

<210>  615
<211>  12
<212>  PRT
<213>  Glycine max

<400>  615

Ser Leu Glu Glu Ile Ile Val Thr Ser Tyr Asn Lys
1               5                   10

<210>  616
<211>  7
<212>  PRT
<213>  Glycine max

<400>  616

Thr Tyr Val Glu Asn Leu Lys
1               5

<210>  617
<211>  1419
<212>  DNA
<213>  Glycine max

<400>  617

atggcttcta aggtggtttc ggttttggtt atagcgatga tgttgtttgc catgaactgt 60

aactgcacta gtgttggcca catgccttca accaaagagg aaggtcacga ttttcaggag 120

tctaaagcca agaccacaca aactgcgaac aaggccatgg aaactggtaa agagggacaa 180

gaagccgcag agtcatggac agaatgggct aaagagaaac tcagtgaagg tcttggcttc 240

aaacatgatc aggaatccaa agaatccacc actaacaaag tctctgatta tgccactgac 300

actgcacaga aatccaagga ttatgcaact gacactgctc agaaatcaaa ggactatgca 360

ggtgatgctg ctcagaaatc aaaggactac gcaggtgatg ctgctcagaa aaccaaagac 420

tatgccagtg acactgcaca gacatcaaag gactatgctg gtgatgctgc tcagaaatca 480

aagggctatg ttggtgatgc tgcacaaaag accaaagaat atgttggtga tgcagcacag 540

aagacaaagg attatgccac tgacgcagca cagaagacca aagattatgc tactcagaag 600

accaaggact atgcaagtga tgcaactgat gctgcaaaga agactaaaga ttatgctgct 660

cagaagacta aggactatgc aagtgaagct agtgatgttg cacagaacac aaaagattat 720

gctgctcaga agaccaagga ctatgccagc ggtggtgcgc agaagaccaa ggattatgcc 780

agcggtggtg cgcagaagac caaggactat gcaagtgatg ctgcgcagaa gaccaaggac 840

tatgcaagtg atggtgctca aaagagcaaa gaatatgcgg gtgatgtagc actgaatgcc 900

aaagattatg ctcaaaaaag caaagattac gcaggtgatg ctgcacagaa tgtcaaagat 960

tatgcaagtg atgcagtgca gaagagaaaa gagtattctg gtgatgcttc tcacaagagc 1020

aaagaggctt ctgattatgc cagtgagacg gctaagaaga ccaaagatta tgtcggtgat 1080

gctgctcaga gaagcaaagg ggctgctgaa tatgctagtg atgcggctca gaggaccaaa 1140

gagtatgctg gtgatgctac caagagaagc aaagaggctt ctaatgatca tgccaatgac 1200

atggctcaga agaccaaaga ctacgccagt gacactgctc agagaaccaa ggaaaaactc 1260

caagacattg catctgaagc tggtcaatac agtgcagaga aagctagaga aatgaaggat 1320

gcagcagcag agaaggcaag tgacatagca aaggctgcaa agcaaaaatc acaagaggtg 1380

aaagagaaat taggtggaca acaccgggat gccgagctt 1419

<210> 618
<211> 473
<212> PRT
<213> Glycine max

<400> 618

Met Ala Ser Lys Val Val Ser Val Leu Val Ile Ala Met Met Leu Phe
1               5                   10                  15

Ala Met Asn Cys Asn Cys Thr Ser Val Gly His Met Pro Ser Thr Lys
            20                  25                  30

Glu Glu Gly His Asp Phe Gln Glu Ser Lys Ala Lys Thr Thr Gln Thr
        35                  40                  45

Ala Asn Lys Ala Met Glu Thr Gly Lys Glu Gly Gln Glu Ala Ala Glu
    50                  55                  60

Ser Trp Thr Glu Trp Ala Lys Glu Lys Leu Ser Glu Gly Leu Gly Phe
65                  70                  75                  80

Lys His Asp Gln Glu Ser Lys Glu Ser Thr Thr Asn Lys Val Ser Asp
                85                  90                  95

Tyr Ala Thr Asp Thr Ala Gln Lys Ser Lys Asp Tyr Ala Thr Asp Thr
            100                 105                 110

Ala Gln Lys Ser Lys Asp Tyr Ala Gly Asp Ala Ala Gln Lys Ser Lys
        115                 120                 125

Asp Tyr Ala Gly Asp Ala Ala Gln Lys Thr Lys Asp Tyr Ala Ser Asp
    130                 135                 140

Thr Ala Gln Thr Ser Lys Asp Tyr Ala Gly Asp Ala Ala Gln Lys Ser
145                 150                 155                 160

Lys Gly Tyr Val Gly Asp Ala Ala Gln Lys Thr Lys Glu Tyr Val Gly
                165                 170                 175

Asp Ala Ala Gln Lys Thr Lys Asp Tyr Ala Thr Asp Ala Ala Gln Lys
            180                 185                 190

Thr Lys Asp Tyr Ala Thr Gln Lys Thr Lys Asp Tyr Ala Ser Asp Ala
        195                 200                 205

Thr Asp Ala Ala Lys Lys Thr Lys Asp Tyr Ala Ala Gln Lys Thr Lys
    210                 215                 220

Asp Tyr Ala Ser Glu Ala Ser Asp Val Ala Gln Asn Thr Lys Asp Tyr
225                 230                 235                 240

Ala Ala Gln Lys Thr Lys Asp Tyr Ala Ser Gly Gly Ala Gln Lys Thr
                245                 250                 255

Lys Asp Tyr Ala Ser Gly Gly Ala Gln Lys Thr Lys Asp Tyr Ala Ser
            260                 265                 270

Asp Ala Ala Gln Lys Thr Lys Asp Tyr Ala Ser Asp Gly Ala Gln Lys
        275                 280                 285

Ser Lys Glu Tyr Ala Gly Asp Val Ala Leu Asn Ala Lys Asp Tyr Ala
    290                 295                 300

Gln Lys Ser Lys Asp Tyr Ala Gly Asp Ala Ala Gln Asn Val Lys Asp
305                 310                 315                 320

Tyr Ala Ser Asp Ala Val Gln Lys Arg Lys Glu Tyr Ser Gly Asp Ala
                325                 330                 335

Ser His Lys Ser Lys Glu Ala Ser Asp Tyr Ala Ser Glu Thr Ala Lys
            340                 345                 350

Lys Thr Lys Asp Tyr Val Gly Asp Ala Ala Gln Arg Ser Lys Gly Ala
        355                 360                 365

Ala Glu Tyr Ala Ser Asp Ala Ala Gln Arg Thr Lys Glu Tyr Ala Gly
    370                 375                 380

Asp Ala Thr Lys Arg Ser Lys Glu Ala Ser Asn Asp His Ala Asn Asp
385                 390                 395                 400

Met Ala Gln Lys Thr Lys Asp Tyr Ala Ser Asp Thr Ala Gln Arg Thr
                405                 410                 415

Lys Glu Lys Leu Gln Asp Ile Ala Ser Glu Ala Gly Gln Tyr Ser Ala
            420                 425                 430

Glu Lys Ala Arg Glu Met Lys Asp Ala Ala Ala Glu Lys Ala Ser Asp
        435                 440                 445

Ile Ala Lys Ala Ala Lys Gln Lys Ser Gln Glu Val Lys Glu Lys Leu
    450                 455                 460

Gly Gly Gln His Arg Asp Ala Glu Leu
465                 470

<210>  619
<211>  11
<212>  PRT
<213>  Glycine max

<400>  619

Asp Tyr Ala Gly Asp Ala Ala Gln Asn Val Lys
1               5                   10

<210>  620
<211>  9

<212>  PRT
<213>  Glycine max

<400>  620

Asp Tyr Ala Ser Asp Ala Ala Gln Lys
1               5


<210>  621
<211>  11
<212>  PRT
<213>  Glycine max

<400>  621

Asp Tyr Ala Ser Asp Ala Thr Asp Ala Ala Lys
1               5                   10


<210>  622
<211>  9
<212>  PRT
<213>  Glycine max

<400>  622

Asp Tyr Ala Ser Asp Ala Val Gln Lys
1               5


<210>  623
<211>  11
<212>  PRT
<213>  Glycine max

<400>  623

Asp Tyr Ala Ser Asp Thr Ala Gln Arg Thr Lys
1               5                   10


<210>  624
<211>  11
<212>  PRT
<213>  Glycine max

<400>  624

Asp Tyr Ala Ser Asp Thr Ala Gln Thr Ser Lys
1               5                   10


<210>  625
<211>  14
<212>  PRT
<213>  Glycine max

<400>  625

Asp Tyr Ala Ser Glu Ala Ser Asp Val Ala Gln Asn Thr Lys
1               5                   10

<210>  626
<211>  9
<212>  PRT
<213>  Glycine max

<400>  626

Asp Tyr Ala Thr Asp Ala Ala Gln Lys
1               5


<210>  627
<211>  11
<212>  PRT
<213>  Glycine max

<400>  627

Asp Tyr Val Gly Asp Ala Ala Gln Arg Ser Lys
1               5                   10


<210>  628
<211>  11
<212>  PRT
<213>  Glycine max

<400>  628

Glu Ala Ser Asp Tyr Ala Ser Glu Thr Ala Lys
1               5                   10


<210>  629
<211>  11
<212>  PRT
<213>  Glycine max

<400>  629

Glu Tyr Ala Gly Asp Val Ala Leu Asn Ala Lys
1               5                   10


<210>  630
<211>  9
<212>  PRT
<213>  Glycine max

<400>  630

Glu Tyr Val Gly Asp Ala Ala Gln Lys
1               5


<210>  631
<211>  14
<212>  PRT
<213>  Glycine max

<400>  631

Gly Ala Ala Glu Tyr Ala Ser Asp Ala Ala Gln Arg Thr Lys
1               5                   10

<210>  632
<211>  9
<212>  PRT
<213>  Glycine max

<400>  632

Gly Tyr Val Gly Asp Ala Ala Gln Lys
1               5

<210>  633
<211>  15
<212>  PRT
<213>  Glycine max

<400>  633

Leu Gln Asp Ile Ala Ser Glu Ala Gly Gln Tyr Ser Ala Glu Lys
1               5                   10                  15

<210>  634
<211>  8
<212>  PRT
<213>  Glycine max

<400>  634

Leu Ser Glu Gly Leu Gly Phe Lys
1               5

<210>  635
<211>  11
<212>  PRT
<213>  Glycine max

<400>  635

Val Ser Asp Tyr Ala Thr Asp Thr Ala Gln Lys
1               5                   10

<210>  636
<211>  1374
<212>  DNA
<213>  Glycine max

<400>  636
atggcttcga gggtggtttc ggttttggtg atagcgatga tgttgtttgc catgaactgt      60

aactgcacta gtgttggcca catgccttca accaaagagg aaggtcacga ttttcaggag     120

gctaaagcca agacaacaca aactgcgaac aaggccatgg aaactggtaa agatgggaaa     180

gaagccgcag agtcatggac agaatgggct aaagagaaac tcactgaagg tcttggcttc     240

aaacatgatc aagaatccac cactaagaaa gtctctgact atgccggtga tgctgctcag     300

aaatcaaagg actatgccgg tgacactgct cagaaaacca aggactatgc cggtgacact 360

gctcagaaaa ccaaggacta tgccggtgac actgcacaga catcaaagga ctatgccggt 420

gatgctgctc agaaatcaaa gggctatgtc ggtgatgctg cacaaaagac caaagaatat 480

gttggtgatg ctgctcagaa gaccatggat tatgccactg atgcagcaca gaagaccaaa 540

gattatgcta ctcagaagac taaggactat gcaagtgatg caactgatgc tgcaaaaaag 600

actaaagatt atgctgctca gaagaccaag gattatgcaa gtgatgcaac tgatgctgca 660

aaaaagacta aggattatgc tgcgcagaag accaaggact atgccagtgg tggtgcgcag 720

aagaccaagg actatgccag tggtgctgcg cagaagacca aggactatgc aagtgatgct 780

gcgcagagga ccaaggacca tgcaagtgat ggtgctcaaa agagcaaaga atatgcgggt 840

gatgtagcac agaatgccaa agattatgct caaaagagca aagattatgc gggtgatgct 900

gtacagaata tcaaagatta tgcaaatgat gcagcgcaca agagcaaaga ttatgctggt 960

gatgcttctc acaagagcaa agaagcttct gattatgcca gtgagacggc tcagaagacc 1020

aaagactatg ttggtgatgc tgctcagaaa agcaaagagg cttctgaata tgctagtgat 1080

gcggctcaga ggaccaaaga gtatgctggt gatgccacca agagaagcaa agaggcttct 1140

gatcatgcca atgacatggc tcggaagacc aaagactacg ccagtgacac tgctcagaga 1200

accaaggaaa aactccaaga cattgcatct gaggctggtc aatacagtac agagaaggcc 1260

agagaaatga aggatgcagc agcagagaag gctagtgaca ttgcaaaggc tgcaaagcaa 1320

aaatcacaag agctgaaaga caaattaggt ggacaacacc gggatgctga gctt 1374

<210> 637
<211> 458
<212> PRT
<213> Glycine max

<400> 637

Met Ala Ser Arg Val Val Ser Val Leu Val Ile Ala Met Met Leu Phe
1               5                   10                  15

Ala Met Asn Cys Asn Cys Thr Ser Val Gly His Met Pro Ser Thr Lys
            20                  25                  30

Glu Glu Gly His Asp Phe Gln Glu Ala Lys Ala Lys Thr Thr Gln Thr
        35                  40                  45

Ala Asn Lys Ala Met Glu Thr Gly Lys Asp Gly Lys Glu Ala Ala Glu
    50                  55                  60

Ser Trp Thr Glu Trp Ala Lys Glu Lys Leu Thr Glu Gly Leu Gly Phe
65                  70                  75                  80

Lys His Asp Gln Glu Ser Thr Thr Lys Lys Val Ser Asp Tyr Ala Gly
                85                  90                  95

Asp Ala Ala Gln Lys Ser Lys Asp Tyr Ala Gly Asp Thr Ala Gln Lys
            100                 105                 110

Thr Lys Asp Tyr Ala Gly Asp Thr Ala Gln Lys Thr Lys Asp Tyr Ala
        115                 120                 125

Gly Asp Thr Ala Gln Thr Ser Lys Asp Tyr Ala Gly Asp Ala Ala Gln
    130                 135                 140

Lys Ser Lys Gly Tyr Val Gly Asp Ala Ala Gln Lys Thr Lys Glu Tyr
145                 150                 155                 160

Val Gly Asp Ala Ala Gln Lys Thr Met Asp Tyr Ala Thr Asp Ala Ala
                165                 170                 175

Gln Lys Thr Lys Asp Tyr Ala Thr Gln Lys Thr Lys Asp Tyr Ala Ser
            180                 185                 190

Asp Ala Thr Asp Ala Ala Lys Lys Thr Lys Asp Tyr Ala Ala Gln Lys
        195                 200                 205

Thr Lys Asp Tyr Ala Ser Asp Ala Thr Asp Ala Ala Lys Lys Thr Lys
    210                 215                 220

Asp Tyr Ala Ala Gln Lys Thr Lys Asp Tyr Ala Ser Gly Gly Ala Gln
225                 230                 235                 240

Lys Thr Lys Asp Tyr Ala Ser Gly Ala Ala Gln Lys Thr Lys Asp Tyr
                245                 250                 255

Ala Ser Asp Ala Ala Gln Arg Thr Lys Asp His Ala Ser Asp Gly Ala
            260                 265                 270

Gln Lys Ser Lys Glu Tyr Ala Gly Asp Val Ala Gln Asn Ala Lys Asp
        275                 280                 285

Tyr Ala Gln Lys Ser Lys Asp Tyr Ala Gly Asp Ala Val Gln Asn Ile
    290                 295                 300

Lys Asp Tyr Ala Asn Asp Ala Ala His Lys Ser Lys Asp Tyr Ala Gly
305                 310                 315                 320

Asp Ala Ser His Lys Ser Lys Glu Ala Ser Asp Tyr Ala Ser Glu Thr

325                 330                 335

Ala Gln Lys Thr Lys Asp Tyr Val Gly Asp Ala Ala Gln Lys Ser Lys
            340                 345                 350

Glu Ala Ser Glu Tyr Ala Ser Asp Ala Ala Gln Arg Thr Lys Glu Tyr
        355                 360                 365

Ala Gly Asp Ala Thr Lys Arg Ser Lys Glu Ala Ser Asp His Ala Asn
    370                 375                 380

Asp Met Ala Arg Lys Thr Lys Asp Tyr Ala Ser Asp Thr Ala Gln Arg
385                 390                 395                 400

Thr Lys Glu Lys Leu Gln Asp Ile Ala Ser Glu Ala Gly Gln Tyr Ser
                405                 410                 415

Thr Glu Lys Ala Arg Glu Met Lys Asp Ala Ala Ala Glu Lys Ala Ser
            420                 425                 430

Asp Ile Ala Lys Ala Ala Lys Gln Lys Ser Gln Glu Leu Lys Asp Lys
        435                 440                 445

Leu Gly Gly Gln His Arg Asp Ala Glu Leu
    450                 455

<210>  638
<211>  11
<212>  PRT
<213>  Glycine max

<400>  638

Asp Tyr Ala Gly Asp Ala Val Gln Asn Ile Lys
1               5                   10

<210>  639
<211>  11
<212>  PRT
<213>  Glycine max

<400>  639

Asp Tyr Ala Ser Asp Ala Ala Gln Arg Thr Lys
1               5                   10

<210>  640
<211>  11
<212>  PRT
<213>  Glycine max

<400>  640

Asp Tyr Ala Ser Asp Ala Thr Asp Ala Ala Lys
1               5                   10

<210>  641
<211>  11
<212>  PRT
<213>  Glycine max

<400>  641

Asp Tyr Ala Ser Asp Thr Ala Gln Arg Thr Lys
1               5                   10

<210>  642
<211>  9
<212>  PRT
<213>  Glycine max

<400>  642

Asp Tyr Val Gly Asp Ala Ala Gln Lys
1               5

<210>  643
<211>  12
<212>  PRT
<213>  Glycine max

<400>  643

Glu Ala Ser Asp Tyr Ala Ser Glu Thr Ala Gln Lys
1               5                   10

<210>  644
<211>  14
<212>  PRT
<213>  Glycine max

<400>  644

Glu Ala Ser Glu Tyr Ala Ser Asp Ala Ala Gln Arg Thr Lys
1               5                   10

<210>  645
<211>  11
<212>  PRT
<213>  Glycine max

<400>  645

Glu Tyr Ala Gly Asp Val Ala Gln Asn Ala Lys
1               5                   10

<210>  646
<211>  9
<212>  PRT

<213>  Glycine max

<400>  646

Gly Tyr Val Gly Asp Ala Ala Gln Lys
1               5


<210>  647
<211>  15
<212>  PRT
<213>  Glycine max

<400>  647

Leu Gln Asp Ile Ala Ser Glu Ala Gly Gln Tyr Ser Thr Glu Lys
1               5                   10                  15


<210>  648
<211>  8
<212>  PRT
<213>  Glycine max

<400>  648

Leu Thr Glu Gly Leu Gly Phe Lys
1               5


<210>  649
<211>  11
<212>  PRT
<213>  Glycine max

<400>  649

Val Ser Asp Tyr Ala Gly Asp Ala Ala Gln Lys
1               5                   10


<210>  650
<211>  8
<212>  PRT
<213>  Artificial Sequence

<220>
<223>  epitope


<220>
<221>  misc_feature
<222>  (1)..(2)
<223>  Xaa can be any amino acid

<220>
<221>  misc_feature
<222>  (4)..(4)
<223>  Xaa can be any amino acid

<220>
<221>  misc_feature
<222>  (6)..(8)

<223>  Xaa can be any amino acid

<400>  650

Xaa Xaa Lys Xaa Glu Xaa Xaa Xaa
1               5


<210>  651
<211>  8
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (4)..(4)
<223>  Xaa can be any amino acid

<220>
<221>  misc_feature
<222>  (6)..(8)
<223>  Xaa can be any amino acid

<400>  651

Xaa Thr Lys Xaa Glu Xaa Xaa Xaa
1               5


<210>  652
<211>  8
<212>  PRT
<213>  Artificial Sequence

<220>
<223>  epitope

<400>  652

Ser Thr Lys Ser Glu Gly Lys Leu
1               5


<210>  653
<211>  9
<212>  PRT
<213>  Artificial Sequence

<220>
<223>  epitope


<220>
<221>  misc_feature

<222>  (1)..(3)
<223>  Xaa can be any amino acid

<220>
<221>  misc_feature
<222>  (8)..(9)
<223>  Xaa can be any amino acid

<400>  653

Xaa Xaa Xaa Lys Asp Asn Val Xaa Xaa
1               5


<210>  654
<211>  9
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (3)..(3)
<223>  Xaa can be any amino acid

<220>
<221>  misc_feature
<222>  (8)..(9)
<223>  Xaa can be any amino acid

<400>  654

Xaa Leu Xaa Lys Asp Asn Val Xaa Xaa
1               5


<210>  655
<211>  9
<212>  PRT
<213>  Artificial Sequence

<220>
<223>  epitope

<400>  655

Ala Leu Ser Lys Asp Asn Val Asp Asp
1               5


<210>  656
<211>  15
<212>  PRT
<213>  Artificial Sequence

<220>
<223>  epitope


<220>
<221>  misc_feature
<222>  (6)..(10)
<223>  Xaa can be any amino acid

<220>
<221>  misc_feature
<222>  (12)..(15)
<223>  Xaa can be any amino acid

<400>  656

Glu Glu Glu Asp Asp Xaa Xaa Xaa Xaa Xaa Glu Xaa Xaa Xaa Xaa
1               5                   10                  15


<210>  657
<211>  15
<212>  PRT
<213>  Artificial Sequence

<220>
<223>  epitope

<400>  657

Glu Glu Glu Asp Asp Thr Glu Val Glu Ala Glu Ser Asp Ala Lys
1               5                   10                  15


<210>  658
<211>  15
<212>  PRT
<213>  Artificial Sequence

<220>
<223>  epitope

<400>  658

Leu His Leu Lys Glu Glu Ala Gly Glu Tyr Leu Glu Glu Ser Lys
1               5                   10                  15


<210>  659
<211>  15
<212>  PRT
<213>  Artificial Sequence

<220>
<223>  epitope

<400>  659

Ser Leu Asp Ile Ser Pro Glu Ala Thr Val Glu Glu Glu Asp Asp
1               5                   10                  15


<210>  660

<211>  10
<212>  PRT
<213>  Artificial Sequence

<220>
<223>  epitope

<400>  660

Glu Ser Thr Asp Lys Glu Lys Lys Asp Ala
1               5                   10


<210>  661
<211>  9
<212>  PRT
<213>  Artificial Sequence

<220>
<223>  epitope


<220>
<221>  misc_feature
<222>  (2)..(3)
<223>  Xaa can be any amino acid

<220>
<221>  misc_feature
<222>  (6)..(9)
<223>  Xaa can be any amino acid

<400>  661

Asp Xaa Xaa Asp Lys Xaa Xaa Xaa Xaa
1               5


<210>  662
<211>  9
<212>  PRT
<213>  Artificial Sequence

<220>
<223>  epitope


<220>
<221>  misc_feature
<222>  (2)..(3)
<223>  Xaa can be any amino acid

<220>
<221>  misc_feature
<222>  (6)..(6)
<223>  Xaa can be any amino acid

<220>
<221>  misc_feature
<222>  (8)..(9)
<223>  Xaa can be any amino acid

<400>  662

Asp Xaa Xaa Asp Lys Xaa Arg Xaa Xaa
1               5

<210>  663
<211>  9
<212>  PRT
<213>  Artificial Sequence

<220>
<223>  epitope

<400>  663

Asp Ala Leu Asp Lys Ile Arg Phe Glu
1               5

<210>  664
<211>  15
<212>  PRT
<213>  Artificial Sequence

<220>
<223>  epitope

<220>
<221>  misc_feature
<222>  (1)..(4)
<223>  Xaa can be any amino acid

<220>
<221>  misc_feature
<222>  (9)..(15)
<223>  Xaa can be any amino acid

<400>  664

Xaa Xaa Xaa Xaa Lys Ser Lys Leu Xaa Xaa Xaa Xaa Xaa Xaa Xaa
1               5                   10                  15

<210>  665
<211>  15
<212>  PRT
<213>  Artificial Sequence

<220>
<223>  epitope

<220>
<221>  misc_feature
<222>  (1)..(4)
<223>  Xaa can be any amino acid

<220>
<221>  misc_feature
<222>  (9)..(11)
<223>  Xaa can be any amino acid

<220>
<221>  misc_feature
<222>  (13)..(15)
<223>  Xaa can be any amino acid

<400>  665

Xaa Xaa Xaa Xaa Lys Ser Lys Leu Xaa Xaa Xaa Pro Xaa Xaa Xaa
1               5                   10                  15


<210>  666
<211>  15
<212>  PRT
<213>  Artificial Sequence

<220>
<223>  epitope

<400>  666

Ser Leu Thr Asp Lys Ser Lys Leu Asp Ala Gln Pro Glu Leu Phe
1               5                   10                  15


<210>  667
<211>  5
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (5)..(5)
<223>  Xaa can be any amino acid

<400>  667

Xaa Pro Asp Lys Xaa
1               5


<210>  668
<211>  5
<212>  PRT
<213>  Artificial Sequence

<220>
<223>  epitope


<220>
<221>  misc_feature
<222>  (5)..(5)
<223>  Xaa can be any amino acid

<400>  668

Ile Pro Asp Lys Xaa
1               5


<210>  669
<211>  5
<212>  PRT
<213>  Artificial Sequence

<220>
<223>  epitope

<400>  669

Ile Pro Asp Lys Thr
1               5


<210>  670
<211>  15
<212>  PRT
<213>  Artificial Sequence

<220>
<223>  epitope

<400>  670

Asp Asp Glu Asp Glu Glu Glu Lys Lys Asp Glu Glu Gly Lys Val
1               5                   10                  15


<210>  671
<211>  15
<212>  PRT
<213>  Artificial Sequence

<220>
<223>  epitope

<400>  671

Pro Glu Tyr Leu Ser Phe Val Lys Gly Ile Val Asp Ser Glu Asp
1               5                   10                  15


<210>  672
<211>  15
<212>  PRT
<213>  Artificial Sequence

<220>
<223>  epitope

<400>  672

Val Asp Ser Glu Asp Leu Pro Leu Asn Ile Ser Arg Glu Met Leu
1               5                   10                  15

<210>  673
<211>  15
<212>  PRT
<213>  Artificial Sequence

<220>
<223>  epitope


<220>
<221>  misc_feature
<222>  (1)..(2)
<223>  Xaa can be any amino acid

<220>
<221>  misc_feature
<222>  (6)..(12)
<223>  Xaa can be any amino acid

<220>
<221>  misc_feature
<222>  (15)..(15)
<223>  Xaa can be any amino acid

<400>  673

Xaa Xaa Phe Ser Lys Xaa Xaa Xaa Xaa Xaa Xaa Xaa Glu Asp Xaa
1               5                   10                  15


<210>  674
<211>  15
<212>  PRT
<213>  Artificial Sequence

<220>
<223>  epitope


<220>
<221>  misc_feature
<222>  (1)..(2)
<223>  Xaa can be any amino acid

<220>
<221>  misc_feature
<222>  (6)..(12)
<223>  Xaa can be any amino acid

<400>  674

Xaa Xaa Phe Ser Lys Xaa Xaa Xaa Xaa Xaa Xaa Xaa Glu Asp Ser
1               5                   10                  15


<210>  675
<211>  15
<212>  PRT
<213>  Artificial Sequence

<220>
<223>  epitope

<400>  675

Glu Ala Phe Ser Lys Asn Leu Lys Leu Gly Ile His Glu Asp Ser
1               5                   10                  15


<210>  676
<211>  15
<212>  PRT
<213>  Artificial Sequence

<220>
<223>  epitope

<400>  676

Leu Gly Asp Lys Val Glu Lys Val Val Val Ser Asp Arg Val Val
1               5                   10                  15


<210>  677
<211>  15
<212>  PRT
<213>  Artificial Sequence

<220>
<223>  epitope

<400>  677

Asp Glu Asp Ala Gly Glu Ala Asp Ala Asp Met Pro Pro Leu Glu
1               5                   10                  15


<210>  678
<211>  15
<212>  PRT
<213>  Artificial Sequence

<220>
<223>  epitope

<400>  678

Met Pro Pro Leu Glu Asp Ala Asp Ala Asp Ala Glu Gly Ser Lys
1               5                   10                  15


<210>  679
<211>  15
<212>  PRT
<213>  Artificial Sequence

<220>
<223>  epitope


<220>
<221>  misc_feature
<222>  (1)..(4)
<223>  Xaa can be any amino acid

<220>

<221>  misc_feature
<222>  (6)..(9)
<223>  Xaa can be any amino acid

<220>
<221>  misc_feature
<222>  (11)..(13)
<223>  Xaa can be any amino acid

<220>
<221>  misc_feature
<222>  (15)..(15)
<223>  Xaa can be any amino acid

<400>  679

Xaa Xaa Xaa Xaa Asp Xaa Xaa Xaa Xaa Glu Xaa Xaa Xaa Asp Xaa
1               5                   10                  15


<210>  680
<211>  15
<212>  PRT
<213>  Artificial Sequence

<220>
<223>  epitope


<220>
<221>  misc_feature
<222>  (1)..(4)
<223>  Xaa can be any amino acid

<220>
<221>  misc_feature
<222>  (9)..(9)
<223>  Xaa can be any amino acid

<220>
<221>  misc_feature
<222>  (11)..(13)
<223>  Xaa can be any amino acid

<400>  680

Xaa Xaa Xaa Xaa Asp Lys Ile Arg Xaa Glu Xaa Xaa Xaa Asp Lys
1               5                   10                  15


<210>  681
<211>  15
<212>  PRT
<213>  Artificial Sequence

<220>
<223>  epitope

<400>  681

Ser Asp Ala Leu Asp Lys Ile Arg Phe Glu Ser Leu Thr Asp Lys
1               5                   10                  15

<210>  682
<211>  15
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (3)..(4)
<223>  Xaa can be any amino acid

<220>
<221>  misc_feature
<222>  (6)..(9)
<223>  Xaa can be any amino acid

<220>
<221>  misc_feature
<222>  (12)..(15)
<223>  Xaa can be any amino acid

<400>  682

Xaa Tyr Xaa Xaa Arg Xaa Xaa Xaa Xaa Gln Asn Xaa Xaa Xaa Xaa
1               5                   10                  15


<210>  683
<211>  15
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (3)..(4)
<223>  Xaa can be any amino acid

<220>
<221>  misc_feature
<222>  (6)..(9)
<223>  Xaa can be any amino acid

<220>
<221>  misc_feature
<222>  (12)..(15)

<223>  Xaa can be any amino acid

<400>  683

Xaa Tyr Xaa Xaa Arg Xaa Xaa Xaa Xaa Gln Asn Xaa Xaa Xaa Xaa
1               5                   10                  15


<210>  684
<211>  15
<212>  PRT
<213>  Artificial Sequence

<220>
<223>  epitope

<400>  684

Asp Tyr Val Thr Arg Met Lys Glu Gly Gln Asn Asp Ile Tyr Tyr
1               5                   10                  15


<210>  685
<211>  15
<212>  PRT
<213>  Artificial Sequence

<220>
<223>  epitope


<220>
<221>  misc_feature
<222>  (2)..(4)
<223>  Xaa can be any amino acid

<220>
<221>  misc_feature
<222>  (6)..(9)
<223>  Xaa can be any amino acid

<220>
<221>  misc_feature
<222>  (12)..(15)
<223>  Xaa can be any amino acid

<400>  685

Asp Xaa Xaa Xaa Arg Xaa Xaa Xaa Xaa Gln Asn Xaa Xaa Xaa Xaa
1               5                   10                  15


<210>  686
<211>  15
<212>  PRT
<213>  Artificial Sequence

<220>
<223>  epitope


<220>
<221>  misc_feature

<222>  (2)..(4)
<223>  Xaa can be any amino acid

<220>
<221>  misc_feature
<222>  (6)..(9)
<223>  Xaa can be any amino acid

<220>
<221>  misc_feature
<222>  (12)..(15)
<223>  Xaa can be any amino acid

<400>  686

Asp Xaa Xaa Xaa Arg Xaa Xaa Xaa Xaa Gln Asn Xaa Xaa Xaa Xaa
1               5                   10                  15


<210>  687
<211>  15
<212>  PRT
<213>  Artificial Sequence

<220>
<223>  epitope

<400>  687

Asp Tyr Val Thr Arg Met Lys Glu Gly Gln Asn Asp Ile Tyr Tyr
1               5                   10                  15


<210>  688
<211>  15
<212>  PRT
<213>  Artificial Sequence

<220>
<223>  epitope


<220>
<221>  misc_feature
<222>  (4)..(11)
<223>  Xaa can be any amino acid

<220>
<221>  misc_feature
<222>  (14)..(15)
<223>  Xaa can be any amino acid

<400>  688

Arg Lys Pro Xaa Xaa Xaa Xaa Xaa Xaa Xaa Xaa Ala Ala Xaa Xaa
1               5                   10                  15


<210>  689
<211>  15
<212>  PRT
<213>  Artificial Sequence

<220>
<223>  epitope

<400>  689

Arg Lys Pro Glu Glu Ile Thr Lys Glu Glu Tyr Ala Ala Phe Tyr
1               5                   10                  15


<210>  690
<211>  9
<212>  PRT
<213>  Artificial Sequence

<220>
<223>  epitope

<400>  690

Ser Leu Thr Asp Lys Ser Lys Leu Asp
1               5


<210>  691
<211>  8
<212>  PRT
<213>  Artificial Sequence

<220>
<223>  epitope

<400>  691

Phe Ile His Ile Ile Pro Asp Lys
1               5


<210>  692
<211>  8
<212>  PRT
<213>  Artificial Sequence

<220>
<223>  epitope

<400>  692

Val Thr Arg Met Lys Glu Gly Gln
1               5


<210>  693
<211>  15
<212>  PRT
<213>  Artificial Sequence

<220>
<223>  epitope


<220>
<221>  misc_feature
<222>  (1)..(10)

<223>  Xaa can be any amino acid

<220>
<221>  misc_feature
<222>  (13)..(13)
<223>  Xaa can be any amino acid

<220>
<221>  misc_feature
<222>  (15)..(15)
<223>  Xaa can be any amino acid

<400>  693

Xaa Xaa Xaa Xaa Xaa Xaa Xaa Xaa Xaa Xaa Arg Leu Xaa Glu Xaa
1               5                   10                  15


<210>  694
<211>  15
<212>  PRT
<213>  Artificial Sequence

<220>
<223>  epitope


<220>
<221>  misc_feature
<222>  (1)..(3)
<223>  Xaa can be any amino acid

<400>  694

Xaa Xaa Xaa Leu Lys Arg Glu Asp Glu Glu Arg Leu Asp Glu Val
1               5                   10                  15


<210>  695
<211>  15
<212>  PRT
<213>  Artificial Sequence

<220>
<223>  epitope

<400>  695

Gly Glu Pro Leu Lys Arg Glu Asp Glu Glu Arg Leu Asp Glu Val
1               5                   10                  15


<210>  696
<211>  15
<212>  PRT
<213>  Artificial Sequence

<220>
<223>  epitope


<220>
<221>  misc_feature

<222>  (1)..(13)
<223>  Xaa can be any amino acid

<400>  696

Xaa Xaa Xaa Xaa Xaa Xaa Xaa Xaa Xaa Xaa Xaa Xaa Xaa Asp Glu
1               5                   10                  15


<210>  697
<211>  15
<212>  PRT
<213>  Artificial Sequence

<220>
<223>  epitope

<400>  697

Ile Leu Glu Arg Lys Lys Ser Pro Asn Arg Leu Val Val Asp Glu
1               5                   10                  15


<210>  698
<211>  7
<212>  PRT
<213>  Artificial Sequence

<220>
<223>  epitope


<220>
<221>  misc_feature
<222>  (1)..(4)
<223>  Xaa can be any amino acid

<400>  698

Xaa Xaa Xaa Xaa Gly Asp Arg
1               5


<210>  699
<211>  7
<212>  PRT
<213>  Artificial Sequence

<220>
<223>  epitope

<400>  699

Phe Pro Glu Ser Gly Asp Arg
1               5


<210>  700
<211>  7
<212>  PRT
<213>  Artificial Sequence

<220>

<223>  epitope

<400>  700

Ser Asp Pro Lys Ser Gly Lys
1               5


<210>  701
<211>  8
<212>  PRT
<213>  Artificial Sequence

<220>
<223>  epitope

<400>  701

Gln Thr Met Glu Lys Leu Gln Leu
1               5


<210>  702
<211>  7
<212>  PRT
<213>  Artificial Sequence

<220>
<223>  epitope

<400>  702

Leu Gly Asp Val Val Ser Val
1               5


<210>  703
<211>  15
<212>  PRT
<213>  Artificial Sequence

<220>
<223>  epitope

<400>  703

Gly Gly Met Arg Ser Val Glu Phe Lys Val Val Glu Thr Asp Pro
1               5                   10                  15


<210>  704
<211>  15
<212>  PRT
<213>  Artificial Sequence

<220>
<223>  epitope

<400>  704

Arg Lys Ala Phe Glu Glu Ala Glu Lys Asn Ala Pro Ser Ile Ile
1               5                   10                  15

<210>  705
<211>  8
<212>  PRT
<213>  Artificial Sequence

<220>
<223>  epitope

<400>  705

Lys Arg Glu Lys Thr His Gly Glu
1               5


<210>  706
<211>  7
<212>  PRT
<213>  Artificial Sequence

<220>
<223>  epitope

<400>  706

Arg Ile Val Ser Gln Leu Leu
1               5


<210>  707
<211>  15
<212>  PRT
<213>  Artificial Sequence

<220>
<223>  epitope

<400>  707

Ser Arg Ala His Val Ile Val Ile Gly Ala Thr Asn Arg Pro Asn
1               5                   10                  15


<210>  708
<211>  9
<212>  PRT
<213>  Artificial Sequence

<220>
<223>  epitope

<400>  708

Leu Arg Arg Phe Gly Arg Phe Asp Arg
1               5


<210>  709
<211>  15
<212>  PRT
<213>  Artificial Sequence

<220>

<223>  epitope

<400>  709

Met Asp Val Ile Asp Leu Glu Asp Glu Thr Ile Asp Ala Glu Val
1               5                   10                  15


<210>  710
<211>  15
<212>  PRT
<213>  Artificial Sequence

<220>
<223>  epitope

<400>  710

Val Thr Asn Glu His Phe Gln Thr Ala Leu Gly Thr Ser Asn Pro
1               5                   10                  15


<210>  711
<211>  15
<212>  PRT
<213>  Artificial Sequence

<220>
<223>  epitope

<400>  711

Val Glu His Pro Glu Lys Phe Glu Lys Phe Gly Met Ser Pro Ser
1               5                   10                  15


<210>  712
<211>  8
<212>  PRT
<213>  Artificial Sequence

<220>
<223>  epitope

<400>  712

Glu Ile Phe Asp Lys Ala Arg Gln
1               5


<210>  713
<211>  15
<212>  PRT
<213>  Artificial Sequence

<220>
<223>  epitope

<400>  713

Asp Ile Ile Asp Pro Ala Leu Leu Arg Pro Gly Arg Leu Asp Gln
1               5                   10                  15

<210>  714
<211>  10
<212>  PRT
<213>  Artificial Sequence

<220>
<223>  epitope

<400>  714

Ile Arg Glu Asn Ile Glu Lys Asp Ile Glu
1               5                   10


<210>  715
<211>  15
<212>  PRT
<213>  Artificial Sequence

<220>
<223>  epitope

<400>  715

Ile Glu Arg Glu Arg Lys Ser Arg Glu Asn Pro Glu Ala Met Asp
1               5                   10                  15


<210>  716
<211>  15
<212>  PRT
<213>  Artificial Sequence

<220>
<223>  epitope

<400>  716

Pro Glu Ala Met Asp Glu Asp Thr Val Asp Asp Glu Val Ala Glu
1               5                   10                  15


<210>  717
<211>  10
<212>  PRT
<213>  Artificial Sequence

<220>
<223>  epitope


<220>
<221>  misc_feature
<222>  (1)..(7)
<223>  Xaa can be any amino acid

<400>  717

Xaa Xaa Xaa Xaa Xaa Xaa Xaa Lys Glu Asp
1               5                   10

<210>  718
<211>  10
<212>  PRT
<213>  Artificial Sequence

<220>
<223>  epitope


<220>
<221>  misc_feature
<222>  (1)..(5)
<223>  Xaa can be any amino acid

<220>
<221>  misc_feature
<222>  (7)..(7)
<223>  Xaa can be any amino acid

<400>  718

Xaa Xaa Xaa Xaa Xaa Glu Xaa Lys Glu Asp
1               5                   10


<210>  719
<211>  10
<212>  PRT
<213>  Artificial Sequence

<220>
<223>  epitope

<400>  719

Leu Asp Lys Phe Ala Glu Asn Lys Glu Asp
1               5                   10


<210>  720
<211>  7
<212>  PRT
<213>  Artificial Sequence

<220>
<223>  epitope


<220>
<221>  misc_feature
<222>  (2)..(3)
<223>  Xaa can be any amino acid

<220>
<221>  misc_feature
<222>  (7)..(7)
<223>  Xaa can be any amino acid

<400>  720

Lys Xaa Xaa Leu Gly Asp Xaa
1               5

<210>  721
<211>  7
<212>  PRT
<213>  Artificial Sequence

<220>
<223>  epitope


<220>
<221>  misc_feature
<222>  (3)..(3)
<223>  Xaa can be any amino acid

<220>
<221>  misc_feature
<222>  (7)..(7)
<223>  Xaa can be any amino acid

<400>  721

Lys Ile Xaa Leu Gly Asp Xaa
1               5


<210>  722
<211>  7
<212>  PRT
<213>  Artificial Sequence

<220>
<223>  epitope

<400>  722

Lys Ile Arg Leu Gly Asp Thr
1               5


<210>  723
<211>  10
<212>  PRT
<213>  Artificial Sequence

<220>
<223>  epitope


<220>
<221>  misc_feature
<222>  (1)..(3)
<223>  Xaa can be any amino acid

<220>
<221>  misc_feature
<222>  (5)..(5)
<223>  Xaa can be any amino acid

<220>
<221>  misc_feature
<222>  (7)..(7)
<223>  Xaa can be any amino acid

<220>
<221> misc_feature
<222> (9)..(9)
<223> Xaa can be any amino acid

<400> 723

Xaa Xaa Xaa Asp Xaa Phe Xaa Lys Xaa Glu
1               5                   10


<210> 724
<211> 10
<212> PRT
<213> Artificial Sequence

<220>
<223> epitope


<220>
<221> misc_feature
<222> (1)..(3)
<223> Xaa can be any amino acid

<220>
<221> misc_feature
<222> (5)..(5)
<223> Xaa can be any amino acid

<220>
<221> misc_feature
<222> (7)..(7)
<223> Xaa can be any amino acid

<400> 724

Xaa Xaa Xaa Asp Xaa Phe Xaa Lys Ile Glu
1               5                   10


<210> 725
<211> 10
<212> PRT
<213> Artificial Sequence

<220>
<223> epitope

<400> 725

Gly Asp Thr Asp Leu Phe Ala Lys Ile Glu
1               5                   10


<210> 726
<211> 15
<212> PRT
<213> Artificial Sequence

<220>
<223> epitope

<400>  726

Ile Asp Ser Cys Leu Thr Val Ala Asp Gln Tyr Asp Ile Gln Ile
1               5                   10                  15


<210>  727
<211>  15
<212>  PRT
<213>  Artificial Sequence

<220>
<223>  epitope

<400>  727

Asn Pro Thr Arg Pro Leu Thr Leu Asn Thr Ile Asp Glu His Leu
1               5                   10                  15


<210>  728
<211>  15
<212>  PRT
<213>  Artificial Sequence

<220>
<223>  epitope

<400>  728

Cys His His Leu Asn Arg Glu Ile Pro Glu Asp Leu Ala Phe Ala
1               5                   10                  15


<210>  729
<211>  15
<212>  PRT
<213>  Artificial Sequence

<220>
<223>  epitope


<220>
<221>  misc_feature
<222>  (1)..(3)
<223>  Xaa can be any amino acid

<220>
<221>  misc_feature
<222>  (5)..(5)
<223>  Xaa can be any amino acid

<220>
<221>  misc_feature
<222>  (7)..(9)
<223>  Xaa can be any amino acid

<220>
<221>  misc_feature
<222>  (12)..(13)
<223>  Xaa can be any amino acid

<400>  729

Xaa Xaa Xaa His Xaa Leu Xaa Xaa Xaa Asn Lys Xaa Xaa Glu Ala
1               5                   10                  15


<210>  730
<211>  15
<212>  PRT
<213>  Artificial Sequence

<220>
<223>  epitope


<220>
<221>  misc_feature
<222>  (1)..(2)
<223>  Xaa can be any amino acid

<220>
<221>  misc_feature
<222>  (5)..(5)
<223>  Xaa can be any amino acid

<220>
<221>  misc_feature
<222>  (7)..(9)
<223>  Xaa can be any amino acid

<220>
<221>  misc_feature
<222>  (12)..(13)
<223>  Xaa can be any amino acid

<400>  730

Xaa Xaa Val His Xaa Leu Xaa Xaa Xaa Asn Lys Xaa Xaa Glu Ala
1               5                   10                  15


<210>  731
<211>  15
<212>  PRT
<213>  Artificial Sequence

<220>
<223>  epitope

<400>  731

Gln Arg Val His Ala Leu Tyr Gly Leu Asn Lys Arg Val Glu Ala
1               5                   10                  15


<210>  732
<211>  15
<212>  PRT
<213>  Artificial Sequence

<220>
<223>  epitope

<220>
<221>  misc_feature
<222>  (1)..(6)
<223>  Xaa can be any amino acid

<220>
<221>  misc_feature
<222>  (9)..(9)
<223>  Xaa can be any amino acid

<220>
<221>  misc_feature
<222>  (11)..(11)
<223>  Xaa can be any amino acid

<220>
<221>  misc_feature
<222>  (13)..(13)
<223>  Xaa can be any amino acid

<220>
<221>  misc_feature
<222>  (15)..(15)
<223>  Xaa can be any amino acid

<400>  732

Xaa Xaa Xaa Xaa Xaa Xaa Gly Leu Xaa Lys Xaa Arg Xaa Glu Xaa
1               5                   10                  15


<210>  733
<211>  15
<212>  PRT
<213>  Artificial Sequence

<220>
<223>  epitope



<220>
<221>  misc_feature
<222>  (1)..(6)
<223>  Xaa can be any amino acid

<220>
<221>  misc_feature
<222>  (9)..(9)
<223>  Xaa can be any amino acid

<220>
<221>  misc_feature
<222>  (11)..(11)
<223>  Xaa can be any amino acid

<220>
<221>  misc_feature
<222>  (13)..(13)
<223>  Xaa can be any amino acid

<400>  733

Xaa Xaa Xaa Xaa Xaa Xaa Gly Leu Xaa Lys Xaa Arg Xaa Glu Gly
1               5                   10                  15

<210>  734
<211>  15
<212>  PRT
<213>  Artificial Sequence

<220>
<223>  epitope

<400>  734

Lys Ala His Glu Ile Ala Gly Leu Arg Lys Glu Arg Ala Glu Gly
1               5                   10                  15

<210>  735
<211>  15
<212>  PRT
<213>  Artificial Sequence

<220>
<223>  epitope

<400>  735

Glu Arg Ala Glu Gly Lys Phe Val Pro Asp Pro Arg Phe Glu Glu
1               5                   10                  15

<210>  736
<211>  15
<212>  PRT
<213>  Artificial Sequence

<220>
<223>  epitope

<400>  736

Arg Ser Gly Ile Phe Gly Ser Tyr Asn Tyr Asp Glu Leu Met Gly
1               5                   10                  15

<210>  737
<211>  15
<212>  PRT
<213>  Artificial Sequence

<220>
<223>  epitope

<400>  737

Asp Glu Leu Met Gly Ser Leu Glu Gly Asn Glu Gly Phe Gly Arg
1               5                   10                  15

<210>  738
<211>  15

<212>  PRT
<213>  Artificial Sequence

<220>
<223>  epitope

<400>  738

Leu Asn Thr Ala Gly Ser Tyr Lys Phe Ser Ser Asp Arg Thr Ile
1               5                   10                  15


<210>  739
<211>  15
<212>  PRT
<213>  Artificial Sequence

<220>
<223>  epitope

<400>  739

Ala Ala Glu Lys Tyr Lys Ser Glu Gly His Asp Met Ile Ile Leu
1               5                   10                  15


<210>  740
<211>  15
<212>  PRT
<213>  Artificial Sequence

<220>
<223>  epitope

<400>  740

Asp Ser Leu Gly Leu Thr Gly His Glu Arg Tyr Thr Ile Asp Leu
1               5                   10                  15


<210>  741
<211>  15
<212>  PRT
<213>  Artificial Sequence

<220>
<223>  epitope

<400>  741

Ile Arg Lys Val Val Glu Leu Gln Glu Glu Leu Asp Ile Asp Val
1               5                   10                  15


<210>  742
<211>  15
<212>  PRT
<213>  Artificial Sequence

<220>
<223>  epitope

<220>
<221>  misc_feature
<222>  (1)..(7)
<223>  Xaa can be any amino acid

<220>
<221>  misc_feature
<222>  (10)..(10)
<223>  Xaa can be any amino acid

<220>
<221>  misc_feature
<222>  (14)..(14)
<223>  Xaa can be any amino acid

<400>  742

Xaa Xaa Xaa Xaa Xaa Xaa Xaa Lys Asp Xaa Val Glu Asp Xaa Glu
1               5                   10                  15


<210>  743
<211>  15
<212>  PRT
<213>  Artificial Sequence

<220>
<223>  epitope


<220>
<221>  misc_feature
<222>  (1)..(7)
<223>  Xaa can be any amino acid

<220>
<221>  misc_feature
<222>  (14)..(14)
<223>  Xaa can be any amino acid

<400>  743

Xaa Xaa Xaa Xaa Xaa Xaa Xaa Lys Asp Glu Val Glu Asp Xaa Glu
1               5                   10                  15


<210>  744
<211>  15
<212>  PRT
<213>  Artificial Sequence

<220>
<223>  epitope

<400>  744

Tyr Gln Ile Ala Leu Ser Ile Lys Asp Glu Val Glu Asp Leu Glu
1               5                   10                  15


<210>  745
<211>  10
<212>  PRT

<213>  Artificial Sequence

<220>
<223>  epitope



<220>
<221>  misc_feature
<222>  (1)..(2)
<223>  Xaa can be any amino acid

<220>
<221>  misc_feature
<222>  (4)..(7)
<223>  Xaa can be any amino acid

<220>
<221>  misc_feature
<222>  (10)..(10)
<223>  Xaa can be any amino acid

<400>  745

Xaa Xaa Glu Xaa Xaa Xaa Xaa Lys Asp Xaa
1               5                   10


<210>  746
<211>  10
<212>  PRT
<213>  Artificial Sequence

<220>
<223>  epitope



<220>
<221>  misc_feature
<222>  (2)..(2)
<223>  Xaa can be any amino acid

<220>
<221>  misc_feature
<222>  (4)..(7)
<223>  Xaa can be any amino acid

<220>
<221>  misc_feature
<222>  (10)..(10)
<223>  Xaa can be any amino acid

<400>  746

Asn Xaa Glu Xaa Xaa Xaa Xaa Lys Asp Xaa
1               5                   10


<210>  747
<211>  10
<212>  PRT
<213>  Artificial Sequence

<220>

<223>  epitope

<400>  747

Asn Pro Glu Thr Tyr Ile Leu Lys Asp Gln
1               5                   10


<210>  748
<211>  8
<212>  PRT
<213>  Artificial Sequence

<220>
<223>  epitope


<220>
<221>  misc_feature
<222>  (6)..(6)
<223>  Xaa can be any amino acid

<400>  748

Leu Asp Phe Leu Glu Xaa Glu Thr
1               5


<210>  749
<211>  8
<212>  PRT
<213>  Artificial Sequence

<220>
<223>  epitope

<400>  749

Leu Asp Phe Leu Glu Val Glu Thr
1               5


<210>  750
<211>  9
<212>  PRT
<213>  Artificial Sequence

<220>
<223>  epitope

<400>  750

Gln Tyr Thr Glu Gly Asp Lys Arg Val
1               5


<210>  751
<211>  10
<212>  PRT
<213>  Artificial Sequence

<220>
<223>  epitope

<220>
<221>  misc_feature
<222>  (3)..(3)
<223>  Xaa can be any amino acid

<220>
<221>  misc_feature
<222>  (6)..(10)
<223>  Xaa can be any amino acid

<400>  751

Pro Leu Xaa Gln Gln Xaa Xaa Xaa Xaa Xaa
1               5                   10


<210>  752
<211>  10
<212>  PRT
<213>  Artificial Sequence

<220>
<223>  epitope


<220>
<221>  misc_feature
<222>  (3)..(3)
<223>  Xaa can be any amino acid

<220>
<221>  misc_feature
<222>  (7)..(10)
<223>  Xaa can be any amino acid

<400>  752

Pro Leu Xaa Gln Gln Gly Xaa Xaa Xaa Xaa
1               5                   10


<210>  753
<211>  10
<212>  PRT
<213>  Artificial Sequence

<220>
<223>  epitope

<400>  753

Pro Leu Val Gln Gln Gly Gln Gln Gly Gln
1               5                   10


<210>  754
<211>  8
<212>  PRT
<213>  Artificial Sequence

<220>

<223>  epitope

<400>  754

Leu Gly Glu Met Asn Gly Lys Met
1               5


<210>  755
<211>  15
<212>  PRT
<213>  Artificial Sequence

<220>
<223>  epitope

<400>  755

Lys Val Ile Thr Leu Ser Val Glu Asp Gln Asp Tyr Met Gly Arg
1               5                   10                  15


<210>  756
<211>  15
<212>  PRT
<213>  Artificial Sequence

<220>
<223>  epitope


<220>
<221>  misc_feature
<222>  (1)..(2)
<223>  Xaa can be any amino acid

<220>
<221>  misc_feature
<222>  (4)..(14)
<223>  Xaa can be any amino acid

<400>  756

Xaa Xaa Gly Xaa Xaa Xaa Xaa Xaa Xaa Xaa Xaa Xaa Xaa Xaa Glu
1               5                   10                  15


<210>  757
<211>  15
<212>  PRT
<213>  Artificial Sequence

<220>
<223>  epitope


<220>
<221>  misc_feature
<222>  (1)..(2)
<223>  Xaa can be any amino acid

<220>
<221>  misc_feature

<222>  (4)..(4)
<223>  Xaa can be any amino acid

<220>
<221>  misc_feature
<222>  (6)..(9)
<223>  Xaa can be any amino acid

<220>
<221>  misc_feature
<222>  (11)..(11)
<223>  Xaa can be any amino acid

<220>
<221>  misc_feature
<222>  (14)..(14)
<223>  Xaa can be any amino acid

<400>  757

Xaa Xaa Gly Xaa Leu Xaa Xaa Xaa Xaa Gly Xaa Arg Thr Xaa Glu
1               5                   10                  15


<210>  758
<211>  15
<212>  PRT
<213>  Artificial Sequence

<220>
<223>  epitope

<400>  758

Ala Ser Gly Lys Leu Ser Gln Tyr Glu Gly Gly Arg Thr Lys Glu
1               5                   10                  15


<210>  759
<211>  15
<212>  PRT
<213>  Artificial Sequence

<220>
<223>  epitope


<220>
<221>  misc_feature
<222>  (1)..(5)
<223>  Xaa can be any amino acid

<220>
<221>  misc_feature
<222>  (8)..(11)
<223>  Xaa can be any amino acid

<400>  759

Xaa Xaa Xaa Xaa Xaa Leu Leu Xaa Xaa Xaa Xaa Glu Glu Asp Asn
1               5                   10                  15

<210>  760
<211>  15
<212>  PRT
<213>  Artificial Sequence

<220>
<223>  epitope


<220>
<221>  misc_feature
<222>  (1)..(5)
<223>  Xaa can be any amino acid

<220>
<221>  misc_feature
<222>  (9)..(11)
<223>  Xaa can be any amino acid

<400>  760

Xaa Xaa Xaa Xaa Xaa Leu Leu Glu Xaa Xaa Xaa Glu Glu Asp Asn
1               5                   10                  15


<210>  761
<211>  15
<212>  PRT
<213>  Artificial Sequence

<220>
<223>  epitope

<400>  761

Glu Lys Ser Asp Asn Leu Leu Glu Gly Gly Gly Glu Glu Asp Asn
1               5                   10                  15


<210>  762
<211>  15
<212>  PRT
<213>  Artificial Sequence

<220>
<223>  epitope


<220>
<221>  misc_feature
<222>  (3)..(11)
<223>  Xaa can be any amino acid

<220>
<221>  misc_feature
<222>  (13)..(13)
<223>  Xaa can be any amino acid

<220>
<221>  misc_feature
<222>  (15)..(15)
<223>  Xaa can be any amino acid

<400>  762

Arg Lys Xaa Xaa Xaa Xaa Xaa Xaa Xaa Xaa Xaa Tyr Xaa Asn Xaa
1               5                   10                  15


<210>  763
<211>  15
<212>  PRT
<213>  Artificial Sequence

<220>
<223>  epitope


<220>
<221>  misc_feature
<222>  (6)..(10)
<223>  Xaa can be any amino acid

<220>
<221>  misc_feature
<222>  (15)..(15)
<223>  Xaa can be any amino acid

<400>  763

Arg Lys Asp His Ser Xaa Xaa Xaa Xaa Xaa Leu Tyr Ala Asn Xaa
1               5                   10                  15


<210>  764
<211>  15
<212>  PRT
<213>  Artificial Sequence

<220>
<223>  epitope

<400>  764

Arg Lys Asp His Ser Asp Val Ser Asn Gln Leu Tyr Ala Asn Tyr
1               5                   10                  15


<210>  765
<211>  15
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (4)..(15)
<223>  Xaa can be any amino acid

<400>  765

Xaa Gly Lys Xaa Xaa Xaa Xaa Xaa Xaa Xaa Xaa Xaa Xaa Xaa Xaa
1               5                   10                  15


<210>  766
<211>  15
<212>  PRT
<213>  Artificial Sequence

<220>
<223>  epitope

<400>  766

Gln Gly Lys Glu Tyr Val Phe Val Ala Asn Ser Asp Asn Leu Gly
1               5                   10                  15


<210>  767
<211>  15
<212>  PRT
<213>  Artificial Sequence

<220>
<223>  epitope

<400>  767

Ile Ala Lys Tyr Gly Leu Asp Asp Asn Thr Ile Asp Phe Ile Gly
1               5                   10                  15


<210>  768
<211>  15
<212>  PRT
<213>  Artificial Sequence

<220>
<223>  epitope

<400>  768

Glu Glu Thr Arg Glu Lys Lys Glu Glu Glu Ser Arg Glu Arg Glu
1               5                   10                  15


<210>  769
<211>  15
<212>  PRT
<213>  Artificial Sequence

<220>
<223>  epitope


<220>
<221>  misc_feature
<222>  (1)..(7)
<223>  Xaa can be any amino acid

<220>
<221>  misc_feature
<222>  (9)..(10)
<223>  Xaa can be any amino acid

<400>  769

Xaa Xaa Xaa Xaa Xaa Xaa Xaa Asn Xaa Xaa Ile Phe Glu Glu Asp
1               5                   10                  15


<210>  770
<211>  15
<212>  PRT
<213>  Artificial Sequence

<220>
<223>  epitope


<220>
<221>  misc_feature
<222>  (1)..(7)
<223>  Xaa can be any amino acid

<220>
<221>  misc_feature
<222>  (9)..(9)
<223>  Xaa can be any amino acid

<400>  770

Xaa Xaa Xaa Xaa Xaa Xaa Xaa Asn Xaa Tyr Ile Phe Glu Glu Asp
1               5                   10                  15


<210>  771
<211>  15
<212>  PRT
<213>  Artificial Sequence

<220>
<223>  epitope

<400>  771

Gln His Glu Glu Gln Asp Glu Asn Pro Tyr Ile Phe Glu Glu Asp
1               5                   10                  15


<210>  772
<211>  15
<212>  PRT
<213>  Artificial Sequence

<220>
<223>  epitope

<400>  772

Ser Ile Phe Ala Ile Ser Arg Glu Gln Val Arg Ala Leu Ala Pro
1               5                   10                  15

<210>  773
<211>  10
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (7)..(7)
<223>  Xaa can be any amino acid

<220>
<221>  misc_feature
<222>  (9)..(10)
<223>  Xaa can be any amino acid

<400>  773

Xaa Glu Leu Pro Arg Glu Xaa Arg Xaa Xaa
1               5                   10


<210>  774
<211>  10
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (9)..(10)
<223>  Xaa can be any amino acid

<400>  774

Xaa Glu Leu Pro Arg Glu Glu Arg Xaa Xaa
1               5                   10


<210>  775
<211>  10
<212>  PRT
<213>  Artificial Sequence

<220>
<223>  epitope

<400>  775

Phe Glu Leu Pro Arg Glu Glu Arg Gly Arg
1               5                   10


<210>  776
<211>  15
<212>  PRT
<213>  Artificial Sequence

<220>
<223>  epitope


<220>
<221>  misc_feature
<222>  (1)..(5)
<223>  Xaa can be any amino acid

<220>
<221>  misc_feature
<222>  (9)..(12)
<223>  Xaa can be any amino acid

<220>
<221>  misc_feature
<222>  (15)..(15)
<223>  Xaa can be any amino acid

<400>  776

Xaa Xaa Xaa Xaa Xaa Pro Leu Tyr Xaa Xaa Xaa Xaa Asp Glu Xaa
1               5                   10                  15


<210>  777
<211>  15
<212>  PRT
<213>  Artificial Sequence

<220>
<223>  epitope


<220>
<221>  misc_feature
<222>  (1)..(5)
<223>  Xaa can be any amino acid

<220>
<221>  misc_feature
<222>  (10)..(12)
<223>  Xaa can be any amino acid

<220>
<221>  misc_feature
<222>  (15)..(15)
<223>  Xaa can be any amino acid

<400>  777

Xaa Xaa Xaa Xaa Xaa Pro Leu Tyr Ile Xaa Xaa Xaa Asp Glu Xaa
1               5                   10                  15

<210>  778
<211>  15
<212>  PRT
<213>  Artificial Sequence

<220>
<223>  epitope

<400>  778

Ile Pro Ala Gly Thr Pro Leu Tyr Ile Val Asn Arg Asp Glu Asn
1               5                   10                  15

<210>  779
<211>  15
<212>  PRT
<213>  Artificial Sequence

<220>
<223>  epitope

<220>
<221>  misc_feature
<222>  (1)..(8)
<223>  Xaa can be any amino acid

<220>
<221>  misc_feature
<222>  (10)..(10)
<223>  Xaa can be any amino acid

<220>
<221>  misc_feature
<222>  (13)..(15)
<223>  Xaa can be any amino acid

<400>  779

Xaa Xaa Xaa Xaa Xaa Xaa Xaa Xaa Arg Xaa Arg Arg Xaa Xaa Xaa
1               5                   10                  15

<210>  780
<211>  15
<212>  PRT
<213>  Artificial Sequence

<220>
<223>  epitope

<220>
<221>  misc_feature
<222>  (1)..(5)
<223>  Xaa can be any amino acid

<220>

<221>  misc_feature
<222>  (7)..(8)
<223>  Xaa can be any amino acid

<220>
<221>  misc_feature
<222>  (10)..(10)
<223>  Xaa can be any amino acid

<220>
<221>  misc_feature
<222>  (13)..(15)
<223>  Xaa can be any amino acid

<400>  780

Xaa Xaa Xaa Xaa Xaa Ser Xaa Xaa Arg Xaa Arg Arg Xaa Xaa Xaa
1               5                   10                  15


<210>  781
<211>  15
<212>  PRT
<213>  Artificial Sequence

<220>
<223>  epitope

<400>  781

Pro Phe Glu Leu Pro Ser Glu Glu Arg Gly Arg Arg Ala Val Ala
1               5                   10                  15


<210>  782
<211>  15
<212>  PRT
<213>  Artificial Sequence

<220>
<223>  epitope


<220>
<221>  misc_feature
<222>  (1)..(10)
<223>  Xaa can be any amino acid

<220>
<221>  misc_feature
<222>  (14)..(15)
<223>  Xaa can be any amino acid

<400>  782

Xaa Xaa Xaa Xaa Xaa Xaa Xaa Xaa Xaa Xaa Glu Glu Glu Xaa Xaa
1               5                   10                  15


<210>  783
<211>  15
<212>  PRT
<213>  Artificial Sequence

<220>
<223>  epitope

<220>
<221>  misc_feature
<222>  (1)..(1)
<223>  Xaa can be any amino acid

<220>
<221>  misc_feature
<222>  (9)..(10)
<223>  Xaa can be any amino acid

<220>
<221>  misc_feature
<222>  (15)..(15)
<223>  Xaa can be any amino acid

<400>  783

Xaa Lys Glu Glu Glu His Gln Glu Xaa Xaa Glu Glu Glu Gln Xaa
1               5                   10                  15

<210>  784
<211>  15
<212>  PRT
<213>  Artificial Sequence

<220>
<223>  epitope

<400>  784

Glu Lys Glu Glu Glu His Gln Glu Gln His Glu Glu Glu Gln Asp
1               5                   10                  15

<210>  785
<211>  15
<212>  PRT
<213>  Artificial Sequence

<220>
<223>  epitope

<220>
<221>  misc_feature
<222>  (1)..(2)
<223>  Xaa can be any amino acid

<220>
<221>  misc_feature
<222>  (5)..(15)
<223>  Xaa can be any amino acid

<400>  785

Xaa Xaa Gly Lys Xaa Xaa Xaa Xaa Xaa Xaa Xaa Xaa Xaa Xaa Xaa
1               5                   10                  15

<210>  786
<211>  15
<212>  PRT
<213>  Artificial Sequence

<220>
<223>  epitope

<400>  786

Phe Ala Gly Lys Asp Asn Ile Val Ser Ser Leu Asp Asn Val Ala
1               5                   10                  15


<210>  787
<211>  10
<212>  PRT
<213>  Artificial Sequence

<220>
<223>  epitope

<400>  787

Ser Asn Leu Ala Leu Gly Lys Leu Lys Glu
1               5                   10


<210>  788
<211>  15
<212>  PRT
<213>  Artificial Sequence

<220>
<223>  epitope

<400>  788

Glu Gly Gly Glu Lys Gly Asn Lys Asp Arg Pro Glu Leu Lys Thr
1               5                   10                  15


<210>  789
<211>  8
<212>  PRT
<213>  Artificial Sequence

<220>
<223>  epitope

<400>  789

Leu Asn Thr Thr Thr Gln Thr Ala
1               5


<210>  790
<211>  15
<212>  PRT
<213>  Artificial Sequence

<220>
<223>  epitope

<400>  790

Gln Gln Gly Tyr Ala Val Thr Lys Asp Thr Ile Ser Ser Ala Ala
1               5                   10                  15


<210>  791
<211>  8
<212>  PRT
<213>  Artificial Sequence

<220>
<223>  epitope

<400>  791

Lys Lys Pro Ser Gln Pro Gln Glu
1               5


<210>  792
<211>  15
<212>  PRT
<213>  Artificial Sequence

<220>
<223>  epitope

<400>  792

Ile Gly Glu Lys Leu Gly Asp Ala Lys Gln Arg Glu Glu Leu Leu
1               5                   10                  15


<210>  793
<211>  5
<212>  PRT
<213>  Artificial Sequence

<220>
<223>  epitope

<400>  793

Glu Thr Lys Asp Thr
1               5


<210>  794
<211>  8
<212>  PRT
<213>  Artificial Sequence

<220>
<223>  epitope

<400>  794

Val Ala Tyr Pro Leu Asp Leu Phe
1               5

<210>  795
<211>  9
<212>  PRT
<213>  Artificial Sequence

<220>
<223>  epitope

<400>  795

Lys Asp Asp Glu Asn Val Asn Ser Gln
1               5


<210>  796
<211>  15
<212>  PRT
<213>  Artificial Sequence

<220>
<223>  epitope

<400>  796

Cys Ala Glu Ala Ile Phe Lys Ser Gln Ala Glu Thr Gly Glu Ile
1               5                   10                  15


<210>  797
<211>  9
<212>  PRT
<213>  Artificial Sequence

<220>
<223>  epitope

<400>  797

Gly His Tyr Leu Asn Ala Thr Ala Gly
1               5


<210>  798
<211>  5
<212>  PRT
<213>  Artificial Sequence

<220>
<223>  epitope

<400>  798

Leu His Ile His Arg
1               5


<210>  799
<211>  15
<212>  PRT
<213>  Artificial Sequence

<220>
<223>  epitope

<400>  799

Asp Asp Phe Val Glu Lys Asp Arg Ser Arg Gly Ile Tyr Phe Thr
1               5                   10                  15


<210>  800
<211>  7
<212>  PRT
<213>  Artificial Sequence

<220>
<223>  epitope

<400>  800

Trp Gly Asn Ala Pro Gly Ala
1               5


<210>  801
<211>  15
<212>  PRT
<213>  Artificial Sequence

<220>
<223>  epitope

<400>  801

Arg Asn Glu Gly Arg Asp Leu Ala Arg Glu Gly Asn Glu Ile Ile
1               5                   10                  15


<210>  802
<211>  15
<212>  PRT
<213>  Artificial Sequence

<220>
<223>  epitope

<400>  802

Val Trp Lys Glu Ile Lys Phe Glu Phe Glu Ala Met Asp Thr Leu
1               5                   10                  15


<210>  803
<211>  15
<212>  PRT
<213>  Artificial Sequence

<220>
<223>  epitope

<400>  803

His Pro Gly Asp Gly Ala Ser Gly Arg Leu Ser Asn Ala Thr Gly
1               5                   10                  15

<210>  804
<211>  15
<212>  PRT
<213>  Artificial Sequence

<220>
<223>  epitope

<400>  804

Val Ile Arg Ile Val Ser Glu Gln Thr Asp Ile Leu Glu Pro Phe
1               5                   10                  15


<210>  805
<211>  15
<212>  PRT
<213>  Artificial Sequence

<220>
<223>  epitope

<400>  805

Ala Thr Ser Gly Asp Arg Asp Gly Lys Thr Phe Val Ser Thr Ala
1               5                   10                  15


<210>  806
<211>  15
<212>  PRT
<213>  Artificial Sequence

<220>
<223>  epitope

<400>  806

Pro Val Thr Val Asn Leu Trp Gln Pro Glu Lys Asn Ala Phe Glu
1               5                   10                  15


<210>  807
<211>  7
<212>  PRT
<213>  Artificial Sequence

<220>
<223>  epitope

<400>  807

Ala Ile Arg Val Thr Gln Ser
1               5


<210>  808
<211>  15
<212>  PRT
<213>  Artificial Sequence

<220>
<223>  epitope

<400>  808

Ser Glu Ala Arg Lys Ser Thr Asn Thr Pro Asp Ala Gln Lys Val
1               5                   10                  15


<210>  809
<211>  15
<212>  PRT
<213>  Artificial Sequence

<220>
<223>  epitope

<400>  809

Ile Leu Trp His Leu Thr Lys Glu Asp Lys Thr Tyr Gly Val Pro
1               5                   10                  15


<210>  810
<211>  15
<212>  PRT
<213>  Artificial Sequence

<220>
<223>  epitope

<400>  810

Val Ala Phe Ser Ile Asp Asn Arg Gln Ile Val Ser Ala Ser Arg
1               5                   10                  15


<210>  811
<211>  9
<212>  PRT
<213>  Artificial Sequence

<220>
<223>  epitope

<400>  811

Arg Leu Tyr Ser Leu Asp Ala Gly Ser
1               5


<210>  812
<211>  9
<212>  PRT
<213>  Artificial Sequence

<220>
<223>  epitope

<400>  812

Asp Leu Lys Val Asp Leu Lys Thr Glu
1               5

<210>  813
<211>  15
<212>  PRT
<213>  Artificial Sequence

<220>
<223>  epitope

<400>  813

Lys Ser Gly Glu Glu Arg Ala Gln Glu Glu Ala Ile Asp Ile Asp
1               5                   10                  15


<210>  814
<211>  15
<212>  PRT
<213>  Artificial Sequence

<220>
<223>  epitope

<400>  814

Asp Asn Gln Leu Val Ala Ala Asp Ala Lys Leu Asn Phe Asp Asp
1               5                   10                  15


<210>  815
<211>  15
<212>  PRT
<213>  Artificial Sequence

<220>
<223>  epitope

<400>  815

Phe Leu Asp Val Gly Gly Asn Ala Ser Glu Asn Gln Val Val Glu
1               5                   10                  15


<210>  816
<211>  15
<212>  PRT
<213>  Artificial Sequence

<220>
<223>  epitope

<400>  816

Val Arg Phe Tyr Lys Glu Glu Glu Lys Asn Asp Pro Glu Phe Ala
1               5                   10                  15


<210>  817
<211>  7
<212>  PRT
<213>  Artificial Sequence

<220>
<223>  epitope


<220>
<221>  misc_feature
<222>  (4)..(7)
<223>  Xaa can be any amino acid

<400>  817

Phe Asp Lys Xaa Xaa Xaa Xaa
1               5


<210>  818
<211>  7
<212>  PRT
<213>  Artificial Sequence

<220>
<223>  epitope


<220>
<221>  misc_feature
<222>  (5)..(7)
<223>  Xaa can be any amino acid

<400>  818

Phe Asp Lys Phe Xaa Xaa Xaa
1               5


<210>  819
<211>  7
<212>  PRT
<213>  Artificial Sequence

<220>
<223>  epitope

<400>  819

Phe Asp Lys Phe Ala Thr Gly
1               5


<210>  820
<211>  15
<212>  PRT
<213>  Artificial Sequence

<220>
<223>  epitope

<400>  820

Gly Lys Pro Leu Val Ile Glu Glu Val Glu Val Ala Pro Pro Gln
1               5                   10                  15

<210> 821
<211> 10
<212> PRT
<213> Artificial Sequence

<220>
<223> epitope


<220>
<221> misc_feature
<222> (1)..(1)
<223> Xaa can be any amino acid

<220>
<221> misc_feature
<222> (3)..(6)
<223> Xaa can be any amino acid

<400> 821

Xaa Gln Xaa Xaa Xaa Xaa Glu Asn Lys Pro
1               5                   10


<210> 822
<211> 10
<212> PRT
<213> Artificial Sequence

<220>
<223> epitope


<220>
<221> misc_feature
<222> (3)..(3)
<223> Xaa can be any amino acid

<220>
<221> misc_feature
<222> (5)..(6)
<223> Xaa can be any amino acid

<400> 822

Glu Gln Xaa Gln Xaa Xaa Glu Asn Lys Pro
1               5                   10


<210> 823
<211> 10
<212> PRT
<213> Artificial Sequence

<220>
<223> epitope

<400> 823

Glu Gln His Gln His Asp Glu Asn Lys Pro
1               5                   10

<210>  824
<211>  15
<212>  PRT
<213>  Artificial Sequence

<220>
<223>  epitope



<220>
<221>  misc_feature
<222>  (1)..(1)
<223>  Xaa can be any amino acid

<220>
<221>  misc_feature
<222>  (3)..(6)
<223>  Xaa can be any amino acid

<220>
<221>  misc_feature
<222>  (9)..(15)
<223>  Xaa can be any amino acid

<400>  824

Xaa Lys Xaa Xaa Xaa Xaa Glu Lys Xaa Xaa Xaa Xaa Xaa Xaa Xaa
1               5                   10                  15


<210>  825
<211>  15
<212>  PRT
<213>  Artificial Sequence

<220>
<223>  epitope



<220>
<221>  misc_feature
<222>  (1)..(1)
<223>  Xaa can be any amino acid

<220>
<221>  misc_feature
<222>  (4)..(6)
<223>  Xaa can be any amino acid

<220>
<221>  misc_feature
<222>  (9)..(13)
<223>  Xaa can be any amino acid

<220>
<221>  misc_feature
<222>  (15)..(15)
<223>  Xaa can be any amino acid

<400>  825

Xaa Lys Asp Xaa Xaa Xaa Glu Lys Xaa Xaa Xaa Xaa Xaa Asp Xaa

1               5                   10                  15


<210>  826
<211>  15
<212>  PRT
<213>  Artificial Sequence

<220>
<223>  epitope

<400>  826

Tyr Lys Asp Tyr Thr Ala Glu Lys Ala Ile Glu Gly Lys Asp Ser
1               5                   10                  15


<210>  827
<211>  15
<212>  PRT
<213>  Artificial Sequence

<220>
<223>  epitope


<220>
<221>  misc_feature
<222>  (1)..(3)
<223>  Xaa can be any amino acid

<220>
<221>  misc_feature
<222>  (5)..(6)
<223>  Xaa can be any amino acid

<220>
<221>  misc_feature
<222>  (10)..(13)
<223>  Xaa can be any amino acid

<220>
<221>  misc_feature
<222>  (15)..(15)
<223>  Xaa can be any amino acid

<400>  827

Xaa Xaa Xaa Asp Xaa Xaa Arg Glu Glu Xaa Xaa Xaa Xaa Arg Xaa
1               5                   10                  15


<210>  828
<211>  15
<212>  PRT
<213>  Artificial Sequence

<220>
<223>  epitope


<220>
<221>  misc_feature

<222>  (1)..(3)
<223>  Xaa can be any amino acid

<220>
<221>  misc_feature
<222>  (5)..(6)
<223>  Xaa can be any amino acid

<220>
<221>  misc_feature
<222>  (12)..(13)
<223>  Xaa can be any amino acid

<220>
<221>  misc_feature
<222>  (15)..(15)
<223>  Xaa can be any amino acid

<400>  828

Xaa Xaa Xaa Asp Xaa Xaa Arg Glu Glu Glu Glu Xaa Xaa Arg Xaa
1               5                   10                  15


<210>  829
<211>  15
<212>  PRT
<213>  Artificial Sequence

<220>
<223>  epitope

<400>  829

Gly Asn Lys Asp Gln Thr Arg Glu Glu Glu Glu Glu Thr Arg Arg
1               5                   10                  15


<210>  830
<211>  15
<212>  PRT
<213>  Artificial Sequence

<220>
<223>  epitope

<400>  830

Lys Glu Thr Lys Met Gly Glu Tyr Ser Asp Tyr Ala Thr Gln Lys
1               5                   10                  15


<210>  831
<211>  15
<212>  PRT
<213>  Artificial Sequence

<220>
<223>  epitope

<400>  831

Gln Lys Ala Gly Glu Tyr Thr Asp Tyr Ala Ala Gln Lys Ala Lys

1               5                   10                  15


<210>  832
<211>  15
<212>  PRT
<213>  Artificial Sequence

<220>
<223>  epitope

<400>  832

Thr Thr Val Gln Lys Ala Gly Glu Tyr Lys Asp Tyr Thr Ala Glu
1               5                   10                  15


<210>  833
<211>  15
<212>  PRT
<213>  Artificial Sequence

<220>
<223>  epitope

<400>  833

Lys Asp Thr Thr Val Gln Lys Ala Gly Glu Tyr Lys Asp Tyr Thr
1               5                   10                  15


<210>  834
<211>  15
<212>  PRT
<213>  Artificial Sequence

<220>
<223>  epitope

<400>  834

Asp Gly Asp Met Glu Glu Glu Gly Val Leu His Val Glu Arg Arg
1               5                   10                  15


<210>  835
<211>  15
<212>  PRT
<213>  Artificial Sequence

<220>
<223>  epitope

<400>  835

Glu Glu Glu Gly Val Leu His Val Glu Arg Arg Arg Glu Lys Met
1               5                   10                  15


<210>  836
<211>  10
<212>  PRT
<213>  Artificial Sequence

<220>
<223>  epitope

<220>
<221>  misc_feature
<222>  (1)..(7)
<223>  Xaa can be any amino acid

<400>  836

Xaa Xaa Xaa Xaa Xaa Xaa Xaa Asp Lys Pro
1               5                   10

<210>  837
<211>  10
<212>  PRT
<213>  Artificial Sequence

<220>
<223>  epitope

<220>
<221>  misc_feature
<222>  (1)..(5)
<223>  Xaa can be any amino acid

<220>
<221>  misc_feature
<222>  (7)..(7)
<223>  Xaa can be any amino acid

<400>  837

Xaa Xaa Xaa Xaa Xaa Phe Xaa Asp Lys Pro
1               5                   10

<210>  838
<211>  10
<212>  PRT
<213>  Artificial Sequence

<220>
<223>  epitope

<400>  838

Glu Lys Thr Leu Leu Phe Gly Asp Lys Pro
1               5                   10

<210>  839
<211>  15
<212>  PRT
<213>  Artificial Sequence

<220>
<223>  epitope

<400>  839

Lys Leu Lys Gly Ile Leu Gly Tyr Thr Glu Asp Asp Val Val Ser
1               5                   10                  15


<210>  840
<211>  15
<212>  PRT
<213>  Artificial Sequence

<220>
<223>  epitope


<220>
<221>  misc_feature
<222>  (1)..(11)
<223>  Xaa can be any amino acid

<220>
<221>  misc_feature
<222>  (14)..(14)
<223>  Xaa can be any amino acid

<400>  840

Xaa Xaa Xaa Xaa Xaa Xaa Xaa Xaa Xaa Xaa Xaa Asp Asp Xaa Glu
1               5                   10                  15


<210>  841
<211>  15
<212>  PRT
<213>  Artificial Sequence

<220>
<223>  epitope


<220>
<221>  misc_feature
<222>  (2)..(11)
<223>  Xaa can be any amino acid

<220>
<221>  misc_feature
<222>  (14)..(14)
<223>  Xaa can be any amino acid

<400>  841

Leu Xaa Xaa Xaa Xaa Xaa Xaa Xaa Xaa Xaa Xaa Asp Asp Xaa Glu
1               5                   10                  15


<210>  842
<211>  15
<212>  PRT
<213>  Artificial Sequence

<220>
<223>  epitope

<400>  842

Leu Glu Phe Thr Gln Gly Asp Leu Arg Asn Arg Asp Asp Leu Glu
1               5                   10                  15


<210>  843
<211>  15
<212>  PRT
<213>  Artificial Sequence

<220>
<223>  epitope

<400>  843

Ser Val Ser Ile Ile Asp Asn Phe Asp Asn Ser Val Val Glu Ala
1               5                   10                  15


<210>  844
<211>  15
<212>  PRT
<213>  Artificial Sequence

<220>
<223>  epitope

<400>  844

Glu Lys Ile Pro Cys Glu Glu Asp Phe Arg Leu Gln Ala Met Asn
1               5                   10                  15


<210>  845
<211>  15
<212>  PRT
<213>  Artificial Sequence

<220>
<223>  epitope

<400>  845

Pro Glu Leu Asn Val Tyr Gly His Asp Tyr Pro Thr Arg Asp Gly
1               5                   10                  15


<210>  846
<211>  15
<212>  PRT
<213>  Artificial Sequence

<220>
<223>  epitope

<400>  846

Pro Thr Arg Asp Gly Ser Ala Ile Arg Asp Tyr Ile His Val Met
1               5                   10                  15

<210>  847
<211>  15
<212>  PRT
<213>  Artificial Sequence

<220>
<223>  epitope


<220>
<221>  misc_feature
<222>  (1)..(4)
<223>  Xaa can be any amino acid

<220>
<221>  misc_feature
<222>  (7)..(9)
<223>  Xaa can be any amino acid

<220>
<221>  misc_feature
<222>  (12)..(14)
<223>  Xaa can be any amino acid

<400>  847

Xaa Xaa Xaa Xaa Asp Lys Xaa Xaa Xaa Glu Arg Xaa Xaa Xaa Glu
1               5                   10                  15


<210>  848
<211>  15
<212>  PRT
<213>  Artificial Sequence

<220>
<223>  epitope


<220>
<221>  misc_feature
<222>  (1)..(4)
<223>  Xaa can be any amino acid

<220>
<221>  misc_feature
<222>  (9)..(9)
<223>  Xaa can be any amino acid

<220>
<221>  misc_feature
<222>  (12)..(14)
<223>  Xaa can be any amino acid

<400>  848

Xaa Xaa Xaa Xaa Asp Lys Arg Val Xaa Glu Arg Xaa Xaa Xaa Glu
1               5                   10                  15


<210>  849
<211>  15
<212>  PRT

<213>  Artificial Sequence

<220>
<223>  epitope

<400>  849

Leu Gly Gly Ile Asp Lys Arg Val Ile Glu Arg Phe Glu Lys Glu
1               5                   10                  15


<210>  850
<211>  15
<212>  PRT
<213>  Artificial Sequence

<220>
<223>  epitope

<400>  850

Gly Gly Phe Glu Ala Gly Ile Ser Lys Asp Gly Gln Thr Arg Glu
1               5                   10                  15


<210>  851
<211>  9
<212>  PRT
<213>  Artificial Sequence

<220>
<223>  epitope


<220>
<221>  misc_feature
<222>  (4)..(4)
<223>  Xaa can be any amino acid

<220>
<221>  misc_feature
<222>  (7)..(9)
<223>  Xaa can be any amino acid

<400>  851

Pro Ile Ser Xaa Phe Glu Xaa Xaa Xaa
1               5


<210>  852
<211>  9
<212>  PRT
<213>  Artificial Sequence

<220>
<223>  epitope


<220>
<221>  misc_feature
<222>  (7)..(9)
<223>  Xaa can be any amino acid

<400>  852

Pro Ile Ser Gly Phe Glu Xaa Xaa Xaa
1               5

<210>  853
<211>  9
<212>  PRT
<213>  Artificial Sequence

<220>
<223>  epitope

<400>  853

Pro Ile Ser Gly Phe Glu Gly Asp Asn
1               5

<210>  854
<211>  10
<212>  PRT
<213>  Artificial Sequence

<220>
<223>  epitope

<220>
<221>  misc_feature
<222>  (1)..(2)
<223>  Xaa can be any amino acid

<220>
<221>  misc_feature
<222>  (4)..(4)
<223>  Xaa can be any amino acid

<220>
<221>  misc_feature
<222>  (7)..(10)
<223>  Xaa can be any amino acid

<400>  854

Xaa Xaa Glu Xaa Leu Asp Xaa Xaa Xaa Xaa
1               5                   10

<210>  855
<211>  10
<212>  PRT
<213>  Artificial Sequence

<220>
<223>  epitope

<220>
<221>  misc_feature
<222>  (4)..(4)

<223>  Xaa can be any amino acid

<220>
<221>  misc_feature
<222>  (7)..(10)
<223>  Xaa can be any amino acid

<400>  855

Leu Leu Glu Xaa Leu Asp Xaa Xaa Xaa Xaa
1               5                   10


<210>  856
<211>  10
<212>  PRT
<213>  Artificial Sequence

<220>
<223>  epitope

<400>  856

Leu Leu Glu Ala Leu Asp Gln Ile Asn Glu
1               5                   10


<210>  857
<211>  15
<212>  PRT
<213>  Artificial Sequence

<220>
<223>  epitope

<400>  857

Gly Thr Val Glu Asp Ile Asp Glu Pro Arg Leu Glu Arg Val Phe
1               5                   10                  15


<210>  858
<211>  15
<212>  PRT
<213>  Artificial Sequence

<220>
<223>  epitope

<400>  858

Trp Thr Pro Leu Ile Pro Ala Ser Phe Lys Glu Glu Glu Thr Ala
1               5                   10                  15


<210>  859
<211>  8
<212>  PRT
<213>  Artificial Sequence

<220>
<223>  epitope

<220>
<221>  misc_feature
<222>  (2)..(5)
<223>  Xaa can be any amino acid

<400>  859

Lys Xaa Xaa Xaa Xaa Gln Gln Gln
1               5


<210>  860
<211>  8
<212>  PRT
<213>  Artificial Sequence

<220>
<223>  epitope


<220>
<221>  misc_feature
<222>  (2)..(4)
<223>  Xaa can be any amino acid

<400>  860

Lys Xaa Xaa Xaa Gln Gln Gln Gln
1               5


<210>  861
<211>  8
<212>  PRT
<213>  Artificial Sequence

<220>
<223>  epitope

<400>  861

Lys Phe Pro Pro Gln Gln Gln Gln
1               5


<210>  862
<211>  7
<212>  PRT
<213>  Artificial Sequence

<220>
<223>  epitope


<220>
<221>  misc_feature
<222>  (4)..(7)
<223>  Xaa can be any amino acid

<400>  862

Arg Gly Lys Xaa Xaa Xaa Xaa

1                   5


<210>  863
<211>  7
<212>  PRT
<213>  Artificial Sequence

<220>
<223>  epitope



<220>
<221>  misc_feature
<222>  (4)..(7)
<223>  Xaa can be any amino acid

<400>  863

Arg Gly Lys Xaa Xaa Xaa Xaa
1                   5


<210>  864
<211>  7
<212>  PRT
<213>  Artificial Sequence

<220>
<223>  epitope

<400>  864

Arg Gly Lys Val Glu Ala Glu
1                   5


<210>  865
<211>  10
<212>  PRT
<213>  Artificial Sequence

<220>
<223>  epitope



<220>
<221>  misc_feature
<222>  (1)..(1)
<223>  Xaa can be any amino acid

<220>
<221>  misc_feature
<222>  (5)..(10)
<223>  Xaa can be any amino acid

<400>  865

Xaa Arg Gly Lys Xaa Xaa Xaa Xaa Xaa Xaa
1                   5                   10


<210>  866

<211>  10
<212>  PRT
<213>  Artificial Sequence

<220>
<223>  epitope


<220>
<221>  misc_feature
<222>  (5)..(10)
<223>  Xaa can be any amino acid

<400>  866

Arg Arg Gly Lys Xaa Xaa Xaa Xaa Xaa Xaa
1               5                   10


<210>  867
<211>  10
<212>  PRT
<213>  Artificial Sequence

<220>
<223>  epitope

<400>  867

Arg Arg Gly Lys Glu Ser Gly Gly Gly Gly
1               5                   10


<210>  868
<211>  15
<212>  PRT
<213>  Artificial Sequence

<220>
<223>  epitope


<220>
<221>  misc_feature
<222>  (2)..(2)
<223>  Xaa can be any amino acid

<220>
<221>  misc_feature
<222>  (4)..(14)
<223>  Xaa can be any amino acid

<400>  868

Arg Xaa Lys Xaa Xaa Xaa Xaa Xaa Xaa Xaa Xaa Xaa Xaa Xaa Glu
1               5                   10                  15


<210>  869
<211>  15
<212>  PRT
<213>  Artificial Sequence

<220>
<223>  epitope

<400>  869

Arg Asn Lys Gln Ala Asn Lys Gly Asn Glu Glu Gly Arg Arg Glu
1               5                   10                  15


<210>  870
<211>  15
<212>  PRT
<213>  Artificial Sequence

<220>
<223>  epitope


<220>
<221>  misc_feature
<222>  (1)..(7)
<223>  Xaa can be any amino acid

<220>
<221>  misc_feature
<222>  (10)..(14)
<223>  Xaa can be any amino acid

<400>  870

Xaa Xaa Xaa Xaa Xaa Xaa Xaa Arg Arg Xaa Xaa Xaa Xaa Xaa Arg
1               5                   10                  15


<210>  871
<211>  15
<212>  PRT
<213>  Artificial Sequence

<220>
<223>  epitope

<400>  871

Met Ala Ser Glu Gln Met Pro Arg Arg Glu Asn Asn Ser Asn Arg
1               5                   10                  15


<210>  872
<211>  15
<212>  PRT
<213>  Artificial Sequence

<220>
<223>  epitope


<220>
<221>  misc_feature
<222>  (1)..(3)
<223>  Xaa can be any amino acid

<220>

<221>  misc_feature
<222>  (9)..(9)
<223>  Xaa can be any amino acid

<220>
<221>  misc_feature
<222>  (11)..(15)
<223>  Xaa can be any amino acid

<400>  872

Xaa Xaa Xaa Lys Gly Arg Val Val Xaa Glu Xaa Xaa Xaa Xaa Xaa
1               5                   10                  15


<210>  873
<211>  15
<212>  PRT
<213>  Artificial Sequence

<220>
<223>  epitope


<220>
<221>  misc_feature
<222>  (9)..(9)
<223>  Xaa can be any amino acid

<400>  873

Leu Pro Asp Lys Gly Arg Val Val Xaa Glu Ser Glu Lys Lys Glu
1               5                   10                  15


<210>  874
<211>  15
<212>  PRT
<213>  Artificial Sequence

<220>
<223>  epitope

<400>  874

Leu Pro Asp Lys Gly Arg Val Val Ile Glu Ser Glu Lys Lys Glu
1               5                   10                  15


<210>  875
<211>  15
<212>  PRT
<213>  Artificial Sequence

<220>
<223>  epitope


<220>
<221>  misc_feature
<222>  (7)..(8)
<223>  Xaa can be any amino acid

<220>
<221>  misc_feature
<222>  (11)..(15)
<223>  Xaa can be any amino acid

<400>  875

Ala Thr Lys Ala Lys Asp Xaa Xaa Arg Glu Xaa Xaa Xaa Xaa Xaa
1               5                   10                  15


<210>  876
<211>  15
<212>  PRT
<213>  Artificial Sequence

<220>
<223>  epitope


<220>
<221>  misc_feature
<222>  (11)..(15)
<223>  Xaa can be any amino acid

<400>  876

Ala Thr Lys Ala Lys Asp Val Thr Arg Glu Xaa Xaa Xaa Xaa Xaa
1               5                   10                  15


<210>  877
<211>  15
<212>  PRT
<213>  Artificial Sequence

<220>
<223>  epitope

<400>  877

Ala Thr Lys Ala Lys Asp Val Thr Arg Glu Lys Gly Gln Gln Gly
1               5                   10                  15


<210>  878
<211>  15
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (3)..(3)
<223>  Xaa can be any amino acid

<220>
<221> misc_feature
<222> (7)..(7)
<223> Xaa can be any amino acid

<220>
<221> misc_feature
<222> (10)..(15)
<223> Xaa can be any amino acid

<400> 878

Xaa Gly Xaa Lys Gly Gln Xaa Lys Lys Xaa Xaa Xaa Xaa Xaa Xaa
1               5                   10                  15


<210> 879
<211> 15
<212> PRT
<213> Artificial Sequence

<220>
<223> epitope


<220>
<221> misc_feature
<222> (1)..(1)
<223> Xaa can be any amino acid

<220>
<221> misc_feature
<222> (3)..(3)
<223> Xaa can be any amino acid

<220>
<221> misc_feature
<222> (10)..(11)
<223> Xaa can be any amino acid

<400> 879

Xaa Gly Xaa Lys Gly Gln Thr Lys Lys Xaa Xaa Gly Glu Glu Gln
1               5                   10                  15


<210> 880
<211> 15
<212> PRT
<213> Artificial Sequence

<220>
<223> epitope

<400> 880

Ala Gly Thr Lys Gly Gln Thr Lys Lys Gly Gly Gly Glu Glu Gln
1               5                   10                  15


<210> 881
<211> 10

<212>  PRT
<213>  Artificial Sequence

<220>
<223>  epitope


<220>
<221>  misc_feature
<222>  (2)..(4)
<223>  Xaa can be any amino acid

<220>
<221>  misc_feature
<222>  (10)..(10)
<223>  Xaa can be any amino acid

<400>  881

Arg Xaa Xaa Xaa Glu Thr Lys Glu Gly Xaa
1               5                   10


<210>  882
<211>  10
<212>  PRT
<213>  Artificial Sequence

<220>
<223>  epitope

<400>  882

Arg Glu Lys Ala Glu Thr Lys Glu Gly Gly
1               5                   10


<210>  883
<211>  10
<212>  PRT
<213>  Artificial Sequence

<220>
<223>  epitope


<220>
<221>  misc_feature
<222>  (1)..(4)
<223>  Xaa can be any amino acid

<400>  883

Xaa Xaa Xaa Xaa Asn Lys Gln Ser Ser Leu
1               5                   10


<210>  884
<211>  10
<212>  PRT
<213>  Artificial Sequence

<220>

<223>  epitope

<400>  884

Ile Gly Glu Glu Asn Lys Gln Ser Ser Leu
1               5                   10


<210>  885
<211>  15
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (3)..(3)
<223>  Xaa can be any amino acid

<220>
<221>  misc_feature
<222>  (6)..(13)
<223>  Xaa can be any amino acid

<220>
<221>  misc_feature
<222>  (15)..(15)
<223>  Xaa can be any amino acid

<400>  885

Xaa Lys Xaa Val Ala Xaa Xaa Xaa Xaa Xaa Xaa Xaa Xaa Glu Xaa
1               5                   10                  15


<210>  886
<211>  15
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (3)..(3)
<223>  Xaa can be any amino acid

<220>

<221>  misc_feature
<222>  (7)..(9)
<223>  Xaa can be any amino acid

<220>
<221>  misc_feature
<222>  (11)..(11)
<223>  Xaa can be any amino acid

<400>  886

Xaa Lys Xaa Val Ala Glu Xaa Xaa Xaa Gln Xaa Ala Ser Glu Leu
1               5                   10                  15


<210>  887
<211>  15
<212>  PRT
<213>  Artificial Sequence

<220>
<223>  epitope

<400>  887

Val Lys Gly Val Ala Glu Tyr Ala Gly Gln Lys Ala Ser Glu Leu
1               5                   10                  15


<210>  888
<211>  10
<212>  PRT
<213>  Artificial Sequence

<220>
<223>  epitope


<220>
<221>  misc_feature
<222>  (1)..(2)
<223>  Xaa can be any amino acid

<220>
<221>  misc_feature
<222>  (8)..(10)
<223>  Xaa can be any amino acid

<400>  888

Xaa Xaa Lys Val Glu Ala Glu Xaa Xaa Xaa
1               5                   10


<210>  889
<211>  10
<212>  PRT
<213>  Artificial Sequence

<220>
<223>  epitope

<400>  889

Arg Lys Lys Val Glu Ala Glu Met Asp Leu
1               5                   10

<210>  890
<211>  10
<212>  PRT
<213>  Artificial Sequence

<220>
<223>  epitope

<220>
<221>  misc_feature
<222>  (1)..(4)
<223>  Xaa can be any amino acid

<400>  890

Xaa Xaa Xaa Xaa Ile Ala Glu Ile Ala Glu
1               5                   10

<210>  891
<211>  10
<212>  PRT
<213>  Artificial Sequence

<220>
<223>  epitope

<220>
<221>  misc_feature
<222>  (1)..(2)
<223>  Xaa can be any amino acid

<400>  891

Xaa Xaa Glu Thr Ile Ala Glu Ile Ala Glu
1               5                   10

<210>  892
<211>  10
<212>  PRT
<213>  Artificial Sequence

<220>
<223>  epitope

<400>  892

Ile Gly Glu Thr Ile Ala Glu Ile Ala Glu
1               5                   10

<210>  893
<211>  15
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (3)..(4)
<223>  Xaa can be any amino acid

<220>
<221>  misc_feature
<222>  (6)..(6)
<223>  Xaa can be any amino acid

<220>
<221>  misc_feature
<222>  (8)..(11)
<223>  Xaa can be any amino acid

<220>
<221>  misc_feature
<222>  (15)..(15)
<223>  Xaa can be any amino acid

<400>  893

Xaa Tyr Xaa Xaa Ser Xaa Lys Xaa Xaa Xaa Xaa Glu Lys Val Xaa
1               5                   10                  15


<210>  894
<211>  15
<212>  PRT
<213>  Artificial Sequence

<220>
<223>  epitope


<220>
<221>  misc_feature
<222>  (1)..(1)
<223>  Xaa can be any amino acid

<220>
<221>  misc_feature
<222>  (4)..(4)
<223>  Xaa can be any amino acid

<220>
<221>  misc_feature
<222>  (6)..(6)
<223>  Xaa can be any amino acid

<220>
<221>  misc_feature
<222>  (8)..(11)

<223>  Xaa can be any amino acid

<220>
<221>  misc_feature
<222>  (15)..(15)
<223>  Xaa can be any amino acid

<400>  894

Xaa Tyr Ile Xaa Ser Xaa Lys Xaa Xaa Xaa Xaa Glu Lys Val Xaa
1               5                   10                  15


<210>  895
<211>  15
<212>  PRT
<213>  Artificial Sequence

<220>
<223>  epitope

<400>  895

Leu Tyr Ile Ala Ser Val Lys Ala Asn Ala Val Glu Lys Val Glu
1               5                   10                  15


<210>  896
<211>  5
<212>  PRT
<213>  Artificial Sequence

<220>
<223>  epitope


<220>
<221>  misc_feature
<222>  (3)..(3)
<223>  Xaa can be any amino acid

<400>  896

Tyr Glu Xaa Gly Val
1               5


<210>  897
<211>  5
<212>  PRT
<213>  Artificial Sequence

<220>
<223>  epitope


<220>
<221>  misc_feature
<222>  (3)..(3)
<223>  Xaa can be any amino acid

<400>  897

Tyr Glu Xaa Gly Val
1               5


<210>  898
<211>  5
<212>  PRT
<213>  Artificial Sequence

<220>
<223>  epitope

<400>  898

Tyr Glu Ala Gly Val
1               5


<210>  899
<211>  15
<212>  PRT
<213>  Artificial Sequence

<220>
<223>  epitope

<400>  899

Ala Arg Glu Ala Ala Ala Arg Asp Ala Arg Glu Ala Ala Ala Arg
1               5                   10                  15


<210>  900
<211>  9
<212>  PRT
<213>  Artificial Sequence

<220>
<223>  epitope


<220>
<221>  misc_feature
<222>  (1)..(5)
<223>  Xaa can be any amino acid

<220>
<221>  misc_feature
<222>  (9)..(9)
<223>  Xaa can be any amino acid

<400>  900

Xaa Xaa Xaa Xaa Xaa Val Thr Ala Xaa
1               5


<210>  901
<211>  9
<212>  PRT
<213>  Artificial Sequence

<220>

<223>  epitope

<220>
<221>  misc_feature
<222>  (2)..(5)
<223>  Xaa can be any amino acid

<220>
<221>  misc_feature
<222>  (9)..(9)
<223>  Xaa can be any amino acid

<400>  901

Val Xaa Xaa Xaa Xaa Val Thr Ala Xaa
1               5

<210>  902
<211>  9
<212>  PRT
<213>  Artificial Sequence

<220>
<223>  epitope

<400>  902

Val Lys Arg Thr Thr Val Thr Ala Thr
1               5

<210>  903
<211>  15
<212>  PRT
<213>  Artificial Sequence

<220>
<223>  epitope

<400>  903

Ala Trp Gly Val Glu Pro Tyr Asp Leu Asp Asp Ala Asp Arg Asn
1               5                   10                  15

<210>  904
<211>  15
<212>  PRT
<213>  Artificial Sequence

<220>
<223>  epitope

<220>
<221>  misc_feature
<222>  (1)..(2)
<223>  Xaa can be any amino acid

<220>
<221>  misc_feature

<222>  (4)..(10)
<223>  Xaa can be any amino acid

<220>
<221>  misc_feature
<222>  (14)..(15)
<223>  Xaa can be any amino acid

<400>  904

Xaa Xaa Trp Xaa Xaa Xaa Xaa Xaa Xaa Xaa Glu Lys Glu Xaa Xaa
1               5                   10                  15


<210>  905
<211>  15
<212>  PRT
<213>  Artificial Sequence

<220>
<223>  epitope


<220>
<221>  misc_feature
<222>  (1)..(2)
<223>  Xaa can be any amino acid

<220>
<221>  misc_feature
<222>  (4)..(7)
<223>  Xaa can be any amino acid

<220>
<221>  misc_feature
<222>  (9)..(10)
<223>  Xaa can be any amino acid

<220>
<221>  misc_feature
<222>  (15)..(15)
<223>  Xaa can be any amino acid

<400>  905

Xaa Xaa Trp Xaa Xaa Xaa Xaa Glu Xaa Xaa Glu Lys Glu Ser Xaa
1               5                   10                  15


<210>  906
<211>  15
<212>  PRT
<213>  Artificial Sequence

<220>
<223>  epitope

<400>  906

Ile Asn Trp Lys Tyr Ala Ser Glu Val Tyr Glu Lys Glu Ser Ser
1               5                   10                  15

<210>  907
<211>  15
<212>  PRT
<213>  Artificial Sequence

<220>
<223>  epitope


<220>
<221>  misc_feature
<222>  (1)..(4)
<223>  Xaa can be any amino acid

<220>
<221>  misc_feature
<222>  (12)..(12)
<223>  Xaa can be any amino acid

<400>  907

Xaa Xaa Xaa Xaa Glu Glu Glu Arg Glu His Ala Xaa Gln Leu Ile
1               5                   10                  15


<210>  908
<211>  15
<212>  PRT
<213>  Artificial Sequence

<220>
<223>  epitope

<400>  908

Lys Glu Ser Ser Glu Glu Glu Arg Glu His Ala Glu Gln Leu Ile
1               5                   10                  15


<210>  909
<211>  15
<212>  PRT
<213>  Artificial Sequence

<220>
<223>  epitope

<400>  909

Lys Glu Ser Ser Glu Glu Glu Arg Glu His Ala Glu Gln Leu Ile
1               5                   10                  15


<210>  910
<211>  15
<212>  PRT
<213>  Artificial Sequence

<220>
<223>  epitope

<400>  910

Pro Asp Gly Ile Leu Ala Tyr Leu Asp Glu Glu Asn Lys Pro Gln
1               5                   10                  15

<210>  911
<211>  15
<212>  PRT
<213>  Artificial Sequence

<220>
<223>  epitope

<400>  911

Cys Ser Leu Lys His Val Pro Gly Phe Ile Glu Arg Ala Glu Glu
1               5                   10                  15

<210>  912
<211>  15
<212>  PRT
<213>  Artificial Sequence

<220>
<223>  epitope

<400>  912

Ala Leu Gly Leu Glu Leu Asp Leu Thr Asp Lys Gly Leu Gly Val
1               5                   10                  15

<210>  913
<211>  9
<212>  PRT
<213>  Artificial Sequence

<220>
<223>  epitope

<220>
<221>  misc_feature
<222>  (1)..(1)
<223>  Xaa can be any amino acid

<220>
<221>  misc_feature
<222>  (3)..(3)
<223>  Xaa can be any amino acid

<220>
<221>  misc_feature
<222>  (5)..(7)
<223>  Xaa can be any amino acid

<220>
<221>  misc_feature
<222>  (9)..(9)
<223>  Xaa can be any amino acid

<400>  913

Xaa Asp Xaa Asp Xaa Xaa Xaa Asp Xaa
1               5

<210>  914
<211>  9
<212>  PRT
<213>  Artificial Sequence

<220>
<223>  epitope

<220>
<221>  misc_feature
<222>  (1)..(1)
<223>  Xaa can be any amino acid

<220>
<221>  misc_feature
<222>  (5)..(7)
<223>  Xaa can be any amino acid

<220>
<221>  misc_feature
<222>  (9)..(9)
<223>  Xaa can be any amino acid

<400>  914

Xaa Asp Ile Asp Xaa Xaa Xaa Asp Xaa
1               5

<210>  915
<211>  9
<212>  PRT
<213>  Artificial Sequence

<220>
<223>  epitope

<400>  915

Trp Asp Ile Asp Tyr Gln Leu Asp Leu
1               5

<210>  916
<211>  15
<212>  PRT
<213>  Artificial Sequence

<220>
<223>  epitope

<400>  916

Lys Ala Asp Ala Gly Ala Ser Lys Thr Tyr Pro Gln Gln Ala Gly
1               5                   10                  15

<210>  917

<211>  15
<212>  PRT
<213>  Artificial Sequence

<220>
<223>  epitope

<400>  917

Cys Asp Val Pro His Val Asn Arg Thr Asp Tyr Gln Leu Ile Asp
1               5                   10                  15


<210>  918
<211>  15
<212>  PRT
<213>  Artificial Sequence

<220>
<223>  epitope

<400>  918

Phe Ala Glu Gly Lys Asp Leu Val Val Ser Val Met Ser Ala Met
1               5                   10                  15


<210>  919
<211>  9
<212>  PRT
<213>  Artificial Sequence

<220>
<223>  epitope


<220>
<221>  misc_feature
<222>  (1)..(6)
<223>  Xaa can be any amino acid

<400>  919

Xaa Xaa Xaa Xaa Xaa Xaa Phe Asp Lys
1               5


<210>  920
<211>  9
<212>  PRT
<213>  Artificial Sequence

<220>
<223>  epitope


<220>
<221>  misc_feature
<222>  (1)..(4)
<223>  Xaa can be any amino acid

<220>
<221>  misc_feature

<222>  (6)..(6)
<223>  Xaa can be any amino acid

<400>  920

Xaa Xaa Xaa Xaa Pro Xaa Phe Asp Lys
1               5


<210>  921
<211>  9
<212>  PRT
<213>  Artificial Sequence

<220>
<223>  epitope

<400>  921

Lys Ala Glu Phe Pro Ile Phe Asp Lys
1               5


<210>  922
<211>  15
<212>  PRT
<213>  Artificial Sequence

<220>
<223>  epitope

<400>  922

Trp Asn Phe Ser Lys Phe Leu Val Asp Lys Asp Gly Asn Val Val
1               5                   10                  15


<210>  923
<211>  5
<212>  PRT
<213>  Artificial Sequence

<220>
<223>  epitope

<400>  923

Lys Ser Arg Asp Asp
1               5


<210>  924
<211>  15
<212>  PRT
<213>  Artificial Sequence

<220>
<223>  epitope

<400>  924

Pro Ser Gln Pro Ala Glu Ser Lys Gly Glu Glu Ser Gly Gly Gly
1               5                   10                  15

<210>  925
<211>  9
<212>  PRT
<213>  Artificial Sequence

<220>
<223>  epitope


<220>
<221>  misc_feature
<222>  (1)..(4)
<223>  Xaa can be any amino acid

<220>
<221>  misc_feature
<222>  (7)..(9)
<223>  Xaa can be any amino acid

<400>  925

Xaa Xaa Xaa Xaa Lys Gln Xaa Xaa Xaa
1               5


<210>  926
<211>  9
<212>  PRT
<213>  Artificial Sequence

<220>
<223>  epitope


<220>
<221>  misc_feature
<222>  (1)..(2)
<223>  Xaa can be any amino acid

<220>
<221>  misc_feature
<222>  (4)..(4)
<223>  Xaa can be any amino acid

<220>
<221>  misc_feature
<222>  (7)..(9)
<223>  Xaa can be any amino acid

<400>  926

Xaa Xaa Asp Xaa Lys Gln Xaa Xaa Xaa
1               5


<210>  927
<211>  9
<212>  PRT
<213>  Artificial Sequence

<220>

<223>  epitope

<400>  927

Lys Leu Asp Asp Lys Gln Gly Ile Gly
1               5


<210>  928
<211>  15
<212>  PRT
<213>  Artificial Sequence

<220>
<223>  epitope

<400>  928

Ser Ile Glu Gln Lys Glu Glu Ser Arg Gly Asn Glu Asp His Val
1               5                   10                  15


<210>  929
<211>  15
<212>  PRT
<213>  Artificial Sequence

<220>
<223>  epitope

<400>  929

Phe Lys Thr Gly Ala Glu Arg Lys Glu Ala Ala Glu Ser Thr Leu
1               5                   10                  15


<210>  930
<211>  15
<212>  PRT
<213>  Artificial Sequence

<220>
<223>  epitope

<400>  930

Glu Leu Asp Thr Leu Gly Glu Glu Ser Tyr Lys Asp Ser Thr Leu
1               5                   10                  15


<210>  931
<211>  15
<212>  PRT
<213>  Artificial Sequence

<220>
<223>  epitope


<220>
<221>  misc_feature
<222>  (1)..(6)
<223>  Xaa can be any amino acid

<220>
<221>  misc_feature
<222>  (8)..(9)
<223>  Xaa can be any amino acid

<220>
<221>  misc_feature
<222>  (11)..(11)
<223>  Xaa can be any amino acid

<220>
<221>  misc_feature
<222>  (13)..(15)
<223>  Xaa can be any amino acid

<400>  931

Xaa Xaa Xaa Xaa Xaa Xaa Ser Xaa Xaa Glu Xaa Leu Xaa Xaa Xaa
1               5                   10                  15


<210>  932
<211>  15
<212>  PRT
<213>  Artificial Sequence

<220>
<223>  epitope


<220>
<221>  misc_feature
<222>  (1)..(2)
<223>  Xaa can be any amino acid

<220>
<221>  misc_feature
<222>  (4)..(6)
<223>  Xaa can be any amino acid

<220>
<221>  misc_feature
<222>  (8)..(9)
<223>  Xaa can be any amino acid

<220>
<221>  misc_feature
<222>  (11)..(11)
<223>  Xaa can be any amino acid

<220>
<221>  misc_feature
<222>  (13)..(15)
<223>  Xaa can be any amino acid

<400>  932

Xaa Xaa Gly Xaa Xaa Xaa Ser Xaa Xaa Glu Xaa Leu Xaa Xaa Xaa
1               5                   10                  15


<210>  933

<211>  15
<212>  PRT
<213>  Artificial Sequence

<220>
<223>  epitope

<400>  933

Gly Thr Gly Leu Ile Val Ser Phe Gly Glu Met Leu Ile Asp Phe
1               5                   10                  15


<210>  934
<211>  15
<212>  PRT
<213>  Artificial Sequence

<220>
<223>  epitope

<400>  934

Asp Glu Phe Gly His Met Leu Ala Gly Ile Leu Lys Glu Asn Gly
1               5                   10                  15


<210>  935
<211>  15
<212>  PRT
<213>  Artificial Sequence

<220>
<223>  epitope

<400>  935

Ser Ala Asp Met Leu Leu Lys Pro Glu Glu Leu Asn Leu Glu Leu
1               5                   10                  15


<210>  936
<211>  15
<212>  PRT
<213>  Artificial Sequence

<220>
<223>  epitope

<400>  936

Asp Leu Ile Lys Val Ser Asp Ala Glu Leu Glu Phe Leu Thr Gly
1               5                   10                  15


<210>  937
<211>  15
<212>  PRT
<213>  Artificial Sequence

<220>
<223>  epitope

<400>  937

Glu Phe Leu Thr Gly Ser Asp Lys Ile Asp Asp Glu Ser Ala Leu
1               5                   10                  15


<210>  938
<211>  15
<212>  PRT
<213>  Artificial Sequence

<220>
<223>  epitope

<400>  938

Val Asp Asp Gln Ser Ile Leu Glu Asp Glu Pro Arg Leu Arg Glu
1               5                   10                  15


<210>  939
<211>  15
<212>  PRT
<213>  Artificial Sequence

<220>
<223>  epitope

<400>  939

Lys Gly Gly Ala Tyr Thr Gly Glu Val Ser Ala Glu Met Leu Val
1               5                   10                  15


<210>  940
<211>  15
<212>  PRT
<213>  Artificial Sequence

<220>
<223>  epitope

<400>  940

Gly Gly Gly Lys Pro Ser Gly Glu Leu Leu Glu Leu Ile Glu Arg
1               5                   10                  15


<210>  941
<211>  7
<212>  PRT
<213>  Artificial Sequence

<220>
<223>  epitope


<220>
<221>  misc_feature
<222>  (2)..(5)
<223>  Xaa can be any amino acid

<220>

<221>  misc_feature
<222>  (7)..(7)
<223>  Xaa can be any amino acid

<400>  941

Lys Xaa Xaa Xaa Xaa Thr Xaa
1               5


<210>  942
<211>  7
<212>  PRT
<213>  Artificial Sequence

<220>
<223>  epitope


<220>
<221>  misc_feature
<222>  (2)..(5)
<223>  Xaa can be any amino acid

<400>  942

Lys Xaa Xaa Xaa Xaa Thr Gln
1               5


<210>  943
<211>  7
<212>  PRT
<213>  Artificial Sequence

<220>
<223>  epitope

<400>  943

Lys Ala Ala Ala Ala Thr Gln
1               5


<210>  944
<211>  9
<212>  PRT
<213>  Artificial Sequence

<220>
<223>  epitope


<220>
<221>  misc_feature
<222>  (3)..(5)
<223>  Xaa can be any amino acid

<220>
<221>  misc_feature
<222>  (8)..(9)
<223>  Xaa can be any amino acid

<400>  944

Thr Lys Xaa Xaa Xaa Ser Asp Xaa Xaa
1               5


<210>  945
<211>  9
<212>  PRT
<213>  Artificial Sequence

<220>
<223>  epitope


<220>
<221>  misc_feature
<222>  (4)..(5)
<223>  Xaa can be any amino acid

<220>
<221>  misc_feature
<222>  (8)..(9)
<223>  Xaa can be any amino acid

<400>  945

Thr Lys Asp Xaa Xaa Ser Asp Xaa Xaa
1               5


<210>  946
<211>  9
<212>  PRT
<213>  Artificial Sequence

<220>
<223>  epitope

<400>  946

Thr Lys Asp Tyr Ala Ser Asp Ala Ala
1               5


<210>  947
<211>  8
<212>  PRT
<213>  Artificial Sequence

<220>
<223>  epitope


<220>
<221>  misc_feature
<222>  (4)..(6)
<223>  Xaa can be any amino acid

<400>  947

Val Lys Asp Xaa Xaa Xaa Asp Ala
1               5

<210>  948
<211>  8
<212>  PRT
<213>  Artificial Sequence

<220>
<223>  epitope


<220>
<221>  misc_feature
<222>  (5)..(6)
<223>  Xaa can be any amino acid

<400>  948

Val Lys Asp Tyr Xaa Xaa Asp Ala
1               5


<210>  949
<211>  8
<212>  PRT
<213>  Artificial Sequence

<220>
<223>  epitope

<400>  949

Val Lys Asp Tyr Ala Ser Asp Ala
1               5


<210>  950
<211>  9
<212>  PRT
<213>  Artificial Sequence

<220>
<223>  epitope


<220>
<221>  misc_feature
<222>  (3)..(8)
<223>  Xaa can be any amino acid

<400>  950

Lys Asp Xaa Xaa Xaa Xaa Xaa Xaa Gln
1               5


<210>  951
<211>  9
<212>  PRT
<213>  Artificial Sequence

<220>
<223>  epitope

<220>
<221>  misc_feature
<222>  (3)..(7)
<223>  Xaa can be any amino acid

<400>  951

Lys Asp Xaa Xaa Xaa Xaa Xaa Ala Gln
1               5


<210>  952
<211>  9
<212>  PRT
<213>  Artificial Sequence

<220>
<223>  epitope

<400>  952

Lys Asp Tyr Ala Ser Asp Thr Ala Gln
1               5


<210>  953
<211>  15
<212>  PRT
<213>  Artificial Sequence

<220>
<223>  epitope


<220>
<221>  misc_feature
<222>  (1)..(7)
<223>  Xaa can be any amino acid

<220>
<221>  misc_feature
<222>  (9)..(9)
<223>  Xaa can be any amino acid

<220>
<221>  misc_feature
<222>  (11)..(11)
<223>  Xaa can be any amino acid

<220>
<221>  misc_feature
<222>  (13)..(13)
<223>  Xaa can be any amino acid

<400>  953

Xaa Xaa Xaa Xaa Xaa Xaa Xaa Glu Xaa Tyr Xaa Leu Xaa His Pro
1               5                   10                  15


<210>  954

<211>  15
<212>  PRT
<213>  Artificial Sequence

<220>
<223>  epitope


<220>
<221>  misc_feature
<222>  (1)..(6)
<223>  Xaa can be any amino acid

<400>  954

Xaa Xaa Xaa Xaa Xaa Xaa Ile Glu Ala Tyr Leu Leu Ala His Pro
1               5                   10                  15


<210>  955
<211>  15
<212>  PRT
<213>  Artificial Sequence

<220>
<223>  epitope

<400>  955

Asp Ala Leu Phe Lys Ala Ile Glu Ala Tyr Leu Leu Ala His Pro
1               5                   10                  15


<210>  956
<211>  9
<212>  PRT
<213>  Artificial Sequence

<220>
<223>  epitope

<400>  956

Gln Lys Glu Ser Glu Glu Arg Lys Gln
1               5


<210>  957
<211>  8
<212>  PRT
<213>  Artificial Sequence

<220>
<223>  epitope

<400>  957

Ser Tyr Arg Leu Gln Ser Gly Asp
1               5


<210>  958
<211>  9

<212>  PRT
<213>  Artificial Sequence

<220>
<223>  epitope

<400>  958

Asp Ala Leu Arg Val Pro Ser Gly Thr
1               5


<210>  959
<211>  9
<212>  PRT
<213>  Artificial Sequence

<220>
<223>  epitope

<400>  959

Arg Lys Thr Ile Ser Ser Glu Asp Lys
1               5


<210>  960
<211>  15
<212>  PRT
<213>  Artificial Sequence

<220>
<223>  epitope

<400>  960

Gln His Pro Glu Arg Glu Pro Gln Gln Pro Gly Glu Lys Glu Glu
1               5                   10                  15


<210>  961
<211>  15
<212>  PRT
<213>  Artificial Sequence

<220>
<223>  epitope

<400>  961

Gly His Gln Ser Gln Lys Gly Lys His Gln Gln Glu Glu Glu Asn
1               5                   10                  15


<210>  962
<211>  15
<212>  PRT
<213>  Artificial Sequence

<220>
<223>  epitope

<400>  962

Leu Leu Asn Ala Leu Pro Glu Glu Val Ile Gln His Thr Phe Asn
1               5                   10                  15


<210>  963
<211>  15
<212>  PRT
<213>  Artificial Sequence

<220>
<223>  epitope

<400>  963

Met Arg Gly Arg Ile Asn Tyr Ile Arg Arg Asn Glu Gly Lys Asp
1               5                   10                  15


<210>  964
<211>  20
<212>  PRT
<213>  Artificial Sequence

<220>
<223>  epitope candidate

<400>  964

Ser Ser Pro Arg Gly Lys Val Glu Ala Glu Lys Ala Arg Pro Met Glu
1               5                   10                  15


Glu Thr Lys Val
            20


<210>  965
<211>  10
<212>  PRT
<213>  Artificial Sequence

<220>
<223>  epitope candidate

<400>  965

Ser Ser Pro Arg Gly Lys Val Glu Ala Glu
1               5                   10


<210>  966
<211>  10
<212>  PRT
<213>  Artificial Sequence

<220>
<223>  epitope candidate

<400>  966

Ser Pro Arg Gly Lys Val Glu Ala Glu Lys
1               5                   10

<210>  967
<211>  10
<212>  PRT
<213>  Artificial Sequence

<220>
<223>  epitope candidate

<400>  967

Pro Arg Gly Lys Val Glu Ala Glu Lys Ala
1               5                   10


<210>  968
<211>  10
<212>  PRT
<213>  Artificial Sequence

<220>
<223>  epitope candidate

<400>  968

Arg Gly Lys Val Glu Ala Glu Lys Ala Arg
1               5                   10


<210>  969
<211>  10
<212>  PRT
<213>  Artificial Sequence

<220>
<223>  epitope candidate

<400>  969

Gly Lys Val Glu Ala Glu Lys Ala Arg Pro
1               5                   10


<210>  970
<211>  10
<212>  PRT
<213>  Artificial Sequence

<220>
<223>  epitope candidate

<400>  970

Lys Val Glu Ala Glu Lys Ala Arg Pro Met
1               5                   10


<210>  971
<211>  10
<212>  PRT
<213>  Artificial Sequence

<220>
<223>  epitope candidate

<400>  971

Val Glu Ala Glu Lys Ala Arg Pro Met Glu
1               5                   10


<210>  972
<211>  10
<212>  PRT
<213>  Artificial Sequence

<220>
<223>  epitope candidate

<400>  972

Glu Ala Glu Lys Ala Arg Pro Met Glu Glu
1               5                   10


<210>  973
<211>  10
<212>  PRT
<213>  Artificial Sequence

<220>
<223>  epitope candidate

<400>  973

Ala Glu Lys Ala Arg Pro Met Glu Glu Thr
1               5                   10


<210>  974
<211>  10
<212>  PRT
<213>  Artificial Sequence

<220>
<223>  epitope candidate

<400>  974

Glu Lys Ala Arg Pro Met Glu Glu Thr Lys
1               5                   10


<210>  975
<211>  10
<212>  PRT
<213>  Artificial Sequence

<220>
<223>  epitope candidate

<400>  975

Lys Ala Arg Pro Met Glu Glu Thr Lys Val
1               5                   10

<210>  976
<211>  8
<212>  PRT
<213>  Artificial Sequence

<220>
<223>  modified epitope

<400>  976

Ala Arg Gly Lys Val Glu Ala Glu
1               5


<210>  977
<211>  8
<212>  PRT
<213>  Artificial Sequence

<220>
<223>  modified epitope

<400>  977

Pro Ala Gly Lys Val Glu Ala Glu
1               5


<210>  978
<211>  8
<212>  PRT
<213>  Artificial Sequence

<220>
<223>  modified epitope

<400>  978

Pro Arg Ala Lys Val Glu Ala Glu
1               5


<210>  979
<211>  8
<212>  PRT
<213>  Artificial Sequence

<220>
<223>  modified epitope

<400>  979

Pro Arg Gly Ala Val Glu Ala Glu
1               5


<210>  980
<211>  8
<212>  PRT
<213>  Artificial Sequence

<220>
<223>  modified epitope

<400>  980

Pro Arg Gly Lys Ala Glu Ala Glu
1               5


<210>  981
<211>  8
<212>  PRT
<213>  Artificial Sequence

<220>
<223>  modified epitope

<400>  981

Pro Arg Gly Lys Val Ala Ala Glu
1               5


<210>  982
<211>  8
<212>  PRT
<213>  Artificial Sequence

<220>
<223>  modified epitope

<400>  982

Pro Arg Gly Lys Val Glu Ala Glu
1               5


<210>  983
<211>  8
<212>  PRT
<213>  Artificial Sequence

<220>
<223>  modified epitope

<400>  983

Pro Arg Gly Lys Val Glu Ala Ala
1               5


<210>  984
<211>  15
<212>  PRT
<213>  Artificial Sequence

<220>
<223>  epitope

<400>  984

Met Leu Ser Lys Gln Met Arg Arg Arg Leu Ser Ala Asp Glu Arg
1               5                   10                  15

<210>  985
<211>  14
<212>  PRT
<213>  Artificial Sequence

<220>
<223>  epitope

<400>  985

Ala Ser Asp His Val Pro Arg Arg Ser Lys Thr Ser Leu Arg
1               5                   10